# EUROPEAN PATENT APPLICATION

(11) **EP 1 782 811 A1**
(43) Date of publication of application: **09.05.2007**
(21) Application number: 05768893.9
(22) Date of filing: 08.08.2005
(51) Int. Cl.: A61K 31/465, A61K 31/47, A61K 31/4709, A61P 33/06, C07D 213/82, C07D 405/12, C07D 409/12

(54) **NOVEL ANTIMALARIA AGENT CONTAINING HETEROCYCLIC COMPOUND**

(30) Priority: 09.08.2004 JP 2004232617; 27.09.2004 WO PCT/JP2004/014063; 22.03.2005 JP 2005082760
(71) Applicant: Eisai R&D Management Co., Ltd., Tokyo 112-8088 (JP)
(72) Inventor: NAKAMOTO, Kazutaka, C/O Tsukuba Research Lab., Tsukuba-shi, Ibaraki 300-2635 (JP); MATSUKURA, Masayuki, C/O Tsukuba Research Lab., Tsukuba-shi, Ibaraki 300-2635 (JP); TANAKA, Keigo, C/O Tsukuba Research Laboratories, Tsukuba-shi, Ibaraki 300-2635 (JP); INOUE, Satoshi, C/O Tsukuba Research Laboratories, Tsukuba-shi, Ibaraki 300-2635 (JP); TSUKADA, Itaru, C/O Tsukuba Research Laboratories, Tsukuba-shi, Ibaraki 300-2635 (JP); HANEDA, Toru, C/O Kashima Plant, Eisai Co., Ltd, Kamisu-shi, Ibaraki 314-0255 (JP); UEDA, Norihiro, C/O Tsukuba Research Laboratories, Tsukuba-shi, Ibaraki 300-2635 (JP); ABE, Shinya, C/O Tsukuba Research Laboratories, Tsukuba-shi, Ibaraki 300-2635 (JP); SAGANE, Koji, C/O Tsukuba Research Laboratories, Tsukuba-shi, Ibaraki 300-2635 (JP)
(74) Representative: HOFFMANN EITLE
(86) International application number: PCT/JP2005/014505
(87) International publication number: WO 2006/016548

(57) **Abstract**

Disclosed is an antimalarial agent containing a compound represented by the formula: 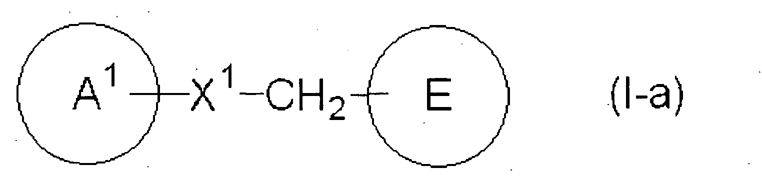 [wherein A¹ represents a 3-pyridyl group that may have a substituent, a 6-quinolyl group that may have a substituent, or the like; X¹ represents a group represented by the formula -C(=O)-NH- or the like; E represents a furyl group, a thienyl group or a phenyl group;
with the proviso that A¹ may have one to three substituents, and E has one of two substituents] or a salt thereof or hydrates thereof.

## Description

### Technical Field

The present invention relates to a novel antimalarial agent containing a heterocyclic compound.

### Background Art

Malaria is the greatest protozoan parasitic infectious disease in humans, whereby a malaria protozoa infect humans via the Anopheles mosquito; the estimated annual number of infected cases is said to be 500 million people, and the annual number of death is said to reach 2 million people or more (See Non-Patent Reference 1). The current situation is that 40 percent of the world population is in malaria epidemic areas, and it is said that one in three infants succumbs to malaria in highly epidemic regions.
It has been already known that in parasites containing malaria protozoa, glycosylphosphatidylinositol (GPI) plays an important role for its growth and infection, and that numerous GPI anchored proteins were present on the surface of malaria protozoa cells. In addition, it has been reported recently that acylation of the inositol ring of glucosaminylphosphatidylinositol, which is a GPI intermediate, was essential for the biosynthesis of GPI, and that maturation of the tropical malaria protozoa *P*. *falciparum* was inhibited if acylation of the inositol ring was prevented by an excess of glucosamine (See Non-Patent Reference 2). Therefore, a compound that inhibits selectively the biosynthesis of GPI, and in particular the acylation of the inositol ring, may be an extremely useful antimalarial agent.
Patent Reference 1 is the prior art related to antimalarial agent based on such a mechanism. Described in Patent Reference 1 are heterocyclic compounds having antimalarial activity by inhibition of the biosynthesis of GPI via inhibition of the activity of the GWT1 gene product from malaria protozoa. However, the compounds disclosed in Patent Reference 1 have 2-benzyl pyridine as a common structure, and clearly differ structurally from the compound according to the present invention.
Meanwhile, Patent References 2 to 6 are the prior arts that are most similar structurally to the heterocyclic compound (I-a) according to the present invention. Described in Patent References 2 to 5 are 2-aryloxynicotinamide derivatives having phosphodiesterase 4 (PDE 4) inhibitory action, and described in Patent Reference 6 are 6-(arylamino)nicotinamide derivatives having cannabinoid receptor regulatory action. However, not only the compound according to the present invention, but also the antimalarial action, are not at all disclosed in Patent References 2 to 6.
[Patent Reference 1] International Publication No. 2004/048567, pamphlet
[Patent Reference 2] European Patent Application No. 1229034, Specification
[Patent Reference 3] International Publication No. 02/060875, pamphlet
[Patent Reference 4] International Publication No. 02/060896, pamphlet
[Patent Reference 5] International Publication No. 03/068232, pamphlet
[Patent Reference 6] International Publication No. 2004/029027, pamphlet
[Non-Patent Reference 1] Gardner, et al, Nature 2003, 419: 498-511
[Non-Patent Reference 2] Naik, R. S. et al., J. Biol. Chem. 278: 2036-2042, 2003

### Disclosure of Invention

### Problems to be solved by the Invention

It is an object of the present invention to provide an excellent antimalarial agent based on GWT1 inhibitory action of malaria protozoa.

### Means for solving the Problems

As a result of earnest studies to address the above problems, the present inventors succeeded in synthesizing novel heterocycle-containing compounds represented by the following formula (I-a):

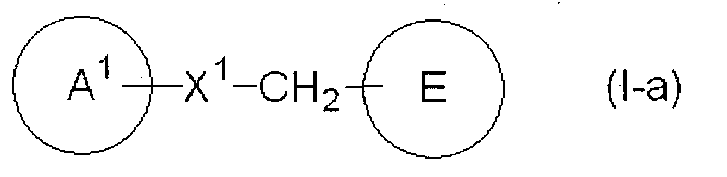

with the chemical structural characteristic that a heterocyclic group A¹ and a heterocyclic group or a phenyl group E are bonded through a linker X¹-CH₂, and discovered that these compounds had excellent antimalarial action to complete the present invention.
That is to say, the present invention provides:

[1] an antimalarial agent containing a compound represented by the formula: 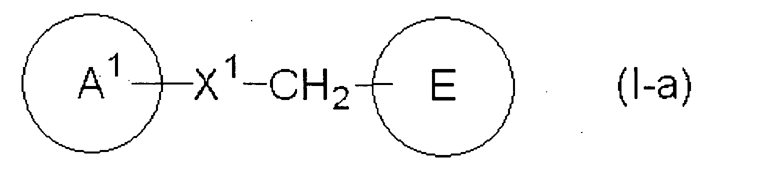
   [wherein A¹ represents a 3-pyridyl group or a 6-quinolyl group;
   X¹ represents a group represented by the formula -C(=Y¹)-NH-;
   Y¹ represents an oxygen atom;
   E represents a furyl group, a thienyl group or a phenyl group; with the proviso that A¹ may have one to three substituents selected from the following Substituent Groups a-1 and a-2, and E has one or two substituents selected from the following Substituent Groups a-1 and a-2] or a salt thereof, or hydrates thereof,
   [Substituent Group a-1]
      a halogen atom, a hydroxyl group, a mercapto group, a cyano group, a carboxyl group, an amino group, a carbamoyl group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₃₋₈ cycloalkylydene C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkoxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₁₋₆ alkylthio group, a C₂₋₆ alkenylthio group, a C₂₋₆ alkynylthio group, a C₃₋₈ cycloalkylthio group, a C₆₋₁₀ arylthio group, a C₃₋₈ cycloalkyl C₁₋₆ alkylthio group, a C₆₋₁₀ aryl C₁₋₆ alkylthio group, a 5 to 10-membered heterocyclic C₁₋₆ alkylthio group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a di-C₁₋₆ alkylamino group, a N-C₂₋₆ alkenyl-N-C₁₋₆ alkylamino group, a N-C₂₋₆ alkynyl-N-C₁₋₆ alkylamino group, a N-C₃₋₈ cycloalkyl-N-C₁₋₆ alkylamino group, a N-C₆₋₁₀ aryl-N-C₁₋₆ alkylamino group, a N-C₃₋₈ cycloalkyl C₁₋₆ alkyl-N-C₁₋₆ alkylamino group, a N-C₆₋₁₀ aryl C₁₋₆ alkyl-N-C₁₋₆ alkylamino group, a N-5 to 10-membered heterocyclic C₁₋₆ alkyl-N-C₁₋₆ alkylamino group, a C₁₋₆ alkylcarbonyl group, a C₁₋₆ alkoxycarbonyl group, a C₁₋₆ alkylsulfonyl group and a group represented by the formula -C(=N-R^{a1})R^{a2} (wherein R^{a1} represents a hydroxyl group or a C₁₋₆ alkoxy group; and R^{a2} represents a C₁₋₆ alkyl group);
   [Substituent Group a-2]
      a C₁₋₆alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkoxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₁₋₆ alkylthio group, a C₂₋₆ alkenylthio group, a C₂₋₆ alkynylthio group, a C₃₋₈ cycloalkylthio group, a C₆₋₁₀ arylthio group, a C₃₋₈ cycloalkyl C₁₋₆ alkylthio group, a C₆₋₁₀ aryl C₁₋₆ alkylthio group, a 5 to 10-membered heterocyclic C₁₋₆ alkylthio group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a di-C₁₋₆ alkylamino group, a N-C₂₋₆ alkenyl-N-C₁₋₆ alkylamino group, a N-C₂₋₆ alkynyl-N-C₁₋₆ alkylamino group, a N-C₃₋₈ cycloalkyl-N-C₁₋₆ alkylamino group, a N-C₆₋₁₀ aryl-N-C₁₋₆ alkylamino group, a N-C₃₋₈ cycloalkyl C₁₋₆ alkyl-N-C₁₋₆ alkylamino group, a N-C₆₋₁₀ aryl C₁₋₆ alkyl-N-C₁₋₆ alkylamino group and a N-5 to 10-membered heterocyclic C₁₋₆ alkyl-N-C₁₋₆ alkylamino group;
      with the proviso that each group mentioned in Substituent Group a-2 has one to three substituents selected from the following Substituent Group b;
   [Substituent Group b]
      a halogen atom, a hydroxyl group, a mercapto group, a cyano group, a carboxyl group, an amino group, a carbamoyl group, a nitro group, a C₁₋₆ alkyl group, a C₃₋₈ cycloalkyl group, a C₆₋₁₀ aryl group, a 5 to 10-membered heterocyclic group, a C₁₋₆ alkoxy group, a C₆₋₁₀ aryloxy group, a 5 to 10-membered heterocyclic oxy group, a C₁₋₆ alkylcarbonyl group, a C₁₋₆ alkoxycarbonyl group, a C₁₋₆ alkylsulfonyl group, a trifluoromethyl group, a trifluoromethoxy group, a mono-C₁₋₆ alkylamino group, a di-C₁₋₆ alkylamino group, a mono-C₆₋₁₀ arylamino group that may have one amino group, a N-C₆₋₁₀ aryl C₁₋₆ alkyl-N-C₁₋₆ alkylamino group that may have one amino group, a trifluoromethyl group, a trifluoromethoxy group and a C₁₋₆ alkyl group,
[2] the antimalarial agent according to item [1], wherein A¹ represents a 3-pyridyl group (with the proviso that A¹ may have one to three substituents selected from the following Substituent Groups c-1 and c-2),
   [Substituent Group c-1]
      a halogen atom, an amino group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a C₁₋₆ alkylcarbonyl group and a group represented by the formula -C(=N-OH)R^{a2} (wherein R^{a2} has the same meaning as defined above);
   [Substituent Group c-2]
      a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group and a mono-5 to 10-membered heterocyclic C₁₋₆ alkylamino group;
      with the proviso that each group mentioned in Substituent Group c-2 has one to three substituents selected from the following Substituent Group d;
   [Substituent Group d]
      a halogen atom, a hydroxyl group, a carboxyl group, an amino group, a carbamoyl group, a C₁₋₆ alkoxy group, a mono-C₁₋₆ alkylamino group, a di-C₁₋₆ alkylamino group, a mono-C₆₋₁₀ arylamino group that may have one amino group and a N-C₆₋₁₀ aryl C₁₋₆ alkyl-N-C₁₋₆ alkylamino group that may have one amino group,
[3] the antimalarial agent according to item[1], wherein A¹ represents a 3-pyridyl group (with the proviso that A¹ may have one to three substituents selected from the following Substituent Groups c'-1 and c'-2),
   [Substituent Group c'-1]
      a halogen atom, an amino group, a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group, a mono-C₂₋₆ alkynylamino group and a C₁₋₆ alkylcarbonyl group;
   [Substituent Group c'-2]
      a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group and a mono-C₂₋₆ alkynylamino group;
      with the proviso that each group mentioned in Substituent Group c'-2 has one to three substituents selected from the following Substituent Group d'; [Substituent Group d']
      a halogen atom, a hydroxyl group, a carboxyl group, an amino group, a carbamoyl group and a C₁₋₆ alkoxy group,
[4] the antimalarial agent according to item [1], wherein A¹ represents a group represented by the formula: 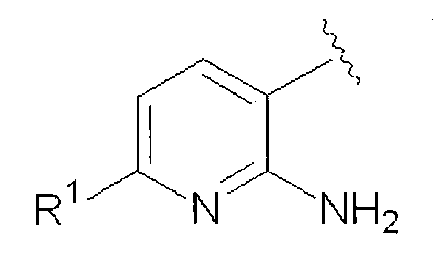 (wherein R¹ represents a hydrogen atom, a substituent selected from the above Substituent Group c-1 or a substituent selected from the above Substituent Group c-2),
[5] the antimalarial agent according to item [1], wherein A¹ represents a group represented by the formula: 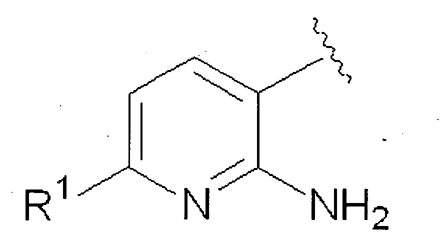 (wherein R¹ represents a hydrogen atom, a substituent selected from the above Substituent Group c'-1 or a substituent selected from the above Substituent Group c'-2),
[6] the antimalarial agent according to item [1], wherein A¹ represents a group represented by the formula: 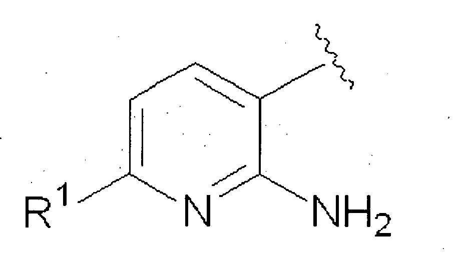 (wherein R¹ represents a hydrogen atom, an amino group, a C₁₋₆ alkyl group that may have one C₁₋₆ alkoxy group, a C₂₋₆ alkenyl group, a mono-C₁₋₆ alkylamino group that may have one C₁₋₆ alkoxy group or a C₁₋₆ alkylcarbonyl group),
[7] the antimalarial agent according to item [1], wherein A¹ represents a group represented by the formula: 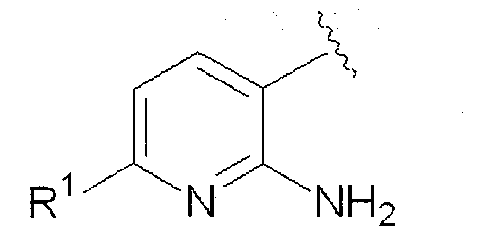 (wherein R¹ represents a C₁₋₆ alkyl group having one halogen atom), [8] the antimalarial agent according to item [1], wherein A¹ represents a group represented by the formula: 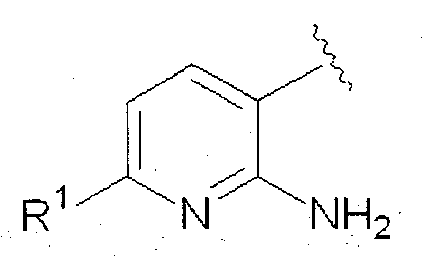 (wherein R¹ represents a hydrogen atom, an amino group, a methoxymethyl group, an ethenyl group, an ethoxyethylamino group or a methylcarbonyl group),
[9] the antimalarial agent according to item [1], wherein A¹ represents a group represented by the formula: 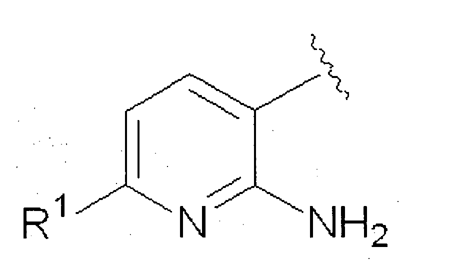 (wherein R¹ represents a fluoroethyl group),
[10] the antimalarial agent according to item [1], wherein A¹ represents a 6-quinolyl group (with the proviso that A¹ may have one to three substituents selected from the following Substituent Groups c-1 and c-2),
   [Substituent Group c-1]
      a halogen atom, an amino group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkyl amino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a C₁₋₆ alkylcarbonyl group and a group represented by the formula -C(=N-OH)R^{a2} (wherein R^{a2} has the same meaning as defined above);
   [Substituent Group c-2]
      a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group and a mono-5 to 10-membered heterocyclic C₁₋₆ alkylamino group;
      with the proviso that each group mentioned in Substituent Group c-2 has one to three substituents selected from the following Substituent Group d; [Substituent Group d]
      a halogen atom, a hydroxyl group, a carboxyl group, an amino group, a carbamoyl group, a C₁₋₆ alkoxy group, a mono-C₁₋₆ alkylamino group, a di-C₁₋₆ alkylamino group, a mono-C₆₋₁₀ arylamino group that may have one amino group and a N-C₆₋₁₀ aryl C₁₋₆ alkyl-N-C₁₋₆ alkylamino group that may have one amino group,
[11] the antimalarial agent according to item [1], wherein A¹ represents a 6-quinolyl group (with the proviso that A¹ may have one to three substituents selected from the following Substituent Groups c'-1 and c'-2),
   [Substituent Group c'-1]
      a halogen atom, an amino group, a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group, a mono-C₂₋₆ alkynylamino group and a C₁₋₆ alkylcarbonyl group;
   [Substituent Group c'-2]
      a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group and a mono-C₂₋₆ alkynylamino group;
      with the proviso that each group mentioned in Substituent Group c'-2 has one to three substituents selected from the following Substituent Group d'; [Substituent Group d']
      a halogen atom, a hydroxyl group, a carboxyl group, an amino group, a carbamoyl group and a C₁₋₆ alkoxy group,
[12] the antimalarial agent according to item [1], wherein A¹ represents a 6-quinolyl group,
[13] the antimalarial agent according to any one of items [1] to [12], wherein E represents a furyl group, a thienyl group or a phenyl group (with the proviso that E has one or two substituents selected from the following Substituent Groups e-1 and e-2),
   [Substituent Group e-1]
      a halogen atom, a hydroxyl group, a C₁₋₆ alkyl group, a C₂₋₆ alkenyl group, a C₂₋₆ alkynyl group, a C₆₋₁₀ aryl group, a C₃₋₈ cycloalkyl C₁₋₆ alkyl group, a C₃₋₈ cycloalkylydene C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₆₋₁₀ arylthio group, a C₆₋₁₀ aryl C₁₋₆ alkylthio group, a mono-C₆₋₁₀ arylamino group, a mono-C₆₋₁₀ aryl C₁₋₆ alkylamino group, a N-C₆₋₁₀ aryl-N-C₁₋₆ alkylamino group and a N-C₆₋₁₀ aryl C₁₋₆ alkyl-N-C₁₋₆ alkylamino group;
   [Substituent Group e-2]
      a C₁₋₆ alkyl group, a C₂₋₆ alkenyl group, a C₂₋₆ alkynyl group, a C₆₋₁₀ aryl group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₆₋₁₀ arylthio group, a C₆₋₁₀ aryl C₁₋₆ alkylthio group, a mono-C₆₋₁₀ arylamino group, a mono-C₆₋₁₀ aryl C₁₋₆ alkylamino group, a N-C₆₋₁₀ aryl-N-C₁₋₆ alkylamino group and a N-C₆₋₁₀ aryl C₁₋₆ alkyl-N-C₁₋₆ alkylamino group;
      with the proviso that each group mentioned in Substituent Group e-2 has one to three substituents selected from the following Substituent Group f; [Substituent Group f]
      a halogen atom, a hydroxyl group, a cyano group, an amino group, a nitro group, a C₃₋₈ cycloalkyl group, a C₁₋₆ alkoxy group, a C₆₋₁₀ aryloxy group, a 5 to 10-membered heterocyclic oxy group, a C₁₋₆ alkylcarbonyl group, a C₁₋₆ alkoxycarbonyl group, a C₁₋₆ alkylsulfonyl group, a mono-C₆₋₁₀ arylamino group, a trifluoromethyl group, a trifluoromethoxy group and a C₁₋₆ alkyl group,
[14] the antimalarial agent according to any one of items [1] to [12], wherein E represents a furyl group, a thienyl group or a phenyl group (with the proviso that E has one substituent selected from the following Substituent Groups g-1 and g-2)
   [Substituent Group g-1]
      a C₃₋₈ cycloalkyl C₁₋₆alkyl group, a phenyl C₁₋₆ alkyl group, a furyl C₁₋₆ alkyl group, a thienyl C₁₋₆ alkyl group, a benzofuryl C₁₋₆ alkyl group, a benzothienyl C₁₋₆ alkyl group, a phenoxy group, a C₃₋₈cycloalkyl C₁₋₆ alkoxy group, a phenyl C₁₋₆ alkoxy group, a furyl C₁₋₆ alkoxy group, a thienyl C₁₋₆ alkoxy group and a pyridyl C₁₋₆ alkoxy group;
   [Substituent Group g-2]
      a C₃₋₈ cycloalkyl C₁₋₆ alkyl group, a phenyl C₁₋₆ alkyl group, a furyl C₁₋₆ alkyl group, a thienyl C₁₋₆ alkyl group, a benzofuryl C₁₋₆ alkyl group, a benzothienyl C₁₋₆ alkyl group, a C₁₋₆ alkoxy group, a phenoxy group, a C₃₋₈ cycloalkyl C₁₋₆ alkoxy group, a phenyl C₁₋₆ alkoxy group, a furyl C₁₋₆ alkoxy group, a thienyl C₁₋₆ alkoxy group and a pyridyl C₁₋₆ alkoxy group;
      with the proviso that each group mentioned in Substituent Group g-2 has one to three substituents selected from the following Substituent Group h;
   [Substituent Group h]
      a halogen atom, a hydroxyl group, a cyano group and a C₁₋₆ alkyl group,
[15] the antimalarial agent according to any one of items [1] to [12], wherein E represents a furyl group, a thienyl group or a phenyl group (with the proviso that E has one C₁₋₆ alkoxy C₁₋₆ alkyl group or one C₁₋₆ alkoxy group),
[16] the antimalarial agent according to any one of items [1] to [12], wherein E represents a 2-furyl group, a 2-thienyl group or a phenyl group (with the proviso that E has one substituent selected from the above Substituent Group g-1 and g-2),
[17] the antimalarial agent according to any one of items [1] to [12], wherein E represents a 2-furyl group, a 2-thienyl group or a phenyl group (with the proviso that E has one phenyl C₁₋₆ alkyl group that may have one halogen atom or one C₁₋₆ alkyl group, or one phenoxy group that may have one halogen atom or one C₁₋₆ alkyl group),
[18] the antimalarial agent according to any one of items [1] to [12], wherein E represents a 2-furyl group, a 2-thienyl group or a phenyl group (with the proviso that E has one C₁₋₆ alkoxy C₁₋₆ alkyl group or one C₁₋₆ alkoxy group),
[19] the antimalarial agent according to any one of items [1] to [12], wherein E represents a 2-furyl group, a 2-thienyl group or a phenyl group (with the proviso that E has one benzyl group that may have one fluorine atom, one chlorine atom or one methyl group, or one phenoxy group that may have one fluorine atom, one chlorine atom or one methyl group),
[20] the,antimalarial agent according to any one of items [1] to [12], wherein E represents a 2-furyl group, a 2-thienyl group or a phenyl group (with the proviso that E has one *n*-butoxy methyl group or one *n*-butoxy group),
[21] the antimalarial agent according to any one of items [1] to [12], wherein E represents a 2-furyl group or a 2-thienyl group (with the proviso that E has one phenyl C₁₋₆ alkyl group that may have one halogen atom or one C₁₋₆ alkyl group, or one phenoxy group that may have one halogen atom or one C₁₋₆ alkyl group),
[22] the antimalarial agent according to any one of items [1] to [1.2], wherein E represents a 2-furyl group or a 2-thienyl group (with the proviso that E has one benzyl group that may have one fluorine atom, one chlorine atom or one methyl group, or one phenoxy group that may have one fluorine atom, one chlorine atom or one methyl group),
[23] the antimalarial agent according to any one of items [1] to [12], wherein E represents a phenyl group (with the proviso that E has one *n*-butoxymethyl group or one *n*-butoxy group),
[24] a method for treating malaria by administering a pharmacologically effective dose of the compound according to [1], or a salt thereof or hydrates thereof, and
[25] use of the compound according to [1], or a salt thereof or hydrates thereof to prepare an antimalarial agent.

In the following, the definitions of symbols, terms and the like which are used in the present specification, the embodiments of the present invention and the like, will be given to describe the present invention in detail.

Herein, although the structural formulae of the compounds may sometimes represent given isomers for the sake of convenience, the present invention includes all isomers such as geometric isomers generated from the structure of the compound, optical isomers based on an asymmetric carbon, stereoisomers, tautomers, and isomer mixtures, are not limited to the formulae given for the sake of convenience, and may be one of the isomers or a mixture. Consequently, although the compounds of the present invention may have an asymmetric carbon atom within the structure such that an optically active compound and a racemic mixture may exist, in the present invention, both are included, without limitation. In addition, although crystal polymorphism may sometimes exist, there is also no limitation, and either crystal form may be single or a crystal form mixture. Also, the compounds according to the present invention include anhydrides and hydrates.

In addition, the compounds generated when the compounds according to the present invention are metabolized such as by oxidation, reduction, hydrolysis or conjugation *in vivo* (a so-called metabolite), and compounds generating the compounds according to the present invention when metabolized such as by oxidation, reduction, hydrolysis or conjugation *in vivo* (a so-called prodrug) are also included in the scope of the present invention.

The term "C₁₋₆ alkyl group" used in the present specification means a linear or branched alkyl group having 1 to 6 carbons, which is a monovalent group derived by removing any one hydrogen atom from an aliphatic hydrocarbon having 1 to 6 carbons. Examples of "C₁₋₆ alkyl group" include a methyl group, an ethyl group, a *n*-propyl group, an isopropyl group, a *n*-butyl group, an isobutyl group, a *sec*-butyl group, a *tert*-butyl group, a *n*-pentyl group, an isopentyl group, a *sec*-pentyl group, a neopentyl group, a 1-methylbutyl group, a 2-methylbutyl group, a 1,1-dimethylpropyl group, a 1,2-dimethylpropyl group, a *n*-hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, and the like, and preferably a methyl group, an ethyl group, a *n*-propyl group, an isopropyl group, a *n*-butyl group, an isobutyl group, a *sec*-butyl group, a *tert*-butyl group and the like.

The term "C₂₋₆ alkenyl group" used in the present specification means a linear or branched alkenyl group having 2 to 6 carbons that may contain one to two double bonds. Examples of "C₂₋₆ alkenyl group" include an ethenyl group, a 1-propenyl group, a 2-propenyl group, a 1-butenyl group, a 2-butenyl group, a 3-butenyl group, a 2-methyl-1-propenyl group, a pentenyl group, a 3-methyl-2-butenyl group, a hexenyl group, a hexane dienyl group, and the like, preferably an ethenyl group, a 1-propenyl group, a 2-propenyl group, a 1-butenyl group, a 2-butenyl group, a 3-butenyl group, a 2-methyl-1-propenyl group, a 3-methyl-2-butenyl group, and the like.

The term "C₂₋₆ alkynyl group" used in the present specification means a linear or a branched alkynyl group having 2 to 6 carbons that may contain one to two triple bonds. Examples of "C₂₋₆ alkynyl group" include an ethynyl group, a 1-propynyl group, a 2-propynyl group, a 1-butynyl group, a 2-butynyl group, a 3-butynyl group, a pentynyl group, a hexynyl group, a hexanediynyl group, and the like, preferably an ethynyl group, a 1-propynyl group, a 2-propynyl group, a 1-butynyl group, a 2-butynyl group, a 3-butynyl group, and the like.

The term "C₃₋₈ cycloalkyl group" used in the present specification means a cyclic aliphatic hydrocarbon group having 3 to 8 carbons. Examples of "C₃₋₈ cycloalkyl group" include a cyclopropyl group, a cyclobutyl group, a cyclopentyl group, a cyclohexyl group, a cycloheptyl group, a cyclooctyl group, and the like, preferably a cyclopropyl group, a cyclobutyl group, a cyclopentyl group, a cyclohexyl group, and the like.

The term "C₁₋₆ alkoxy group" used in the present specification means a group with an oxygen atom bonded to the terminus of the previously defined "C₁₋₆ alkyl group". Examples of "C₁₋₆ alkoxy group" include a methoxy group, an ethoxy group, a *n*-propoxy group, an isopropoxy group, a *n*-butoxy group, an isobutoxy group, a *sec*-butoxy group, a *tert*-butoxy group, a *n*-pentyloxy group, an isopentyloxy group, a *sec*-pentyloxy group, a neopentyloxy group, a 1-methylbutoxy group, a 2-methylbutoxy group, a 1,1-dimethylpropoxy group, a 1,2-dimethylpropoxy group, a *n*-hexyloxy group, an isohexyloxy group, a 1-methylpentyloxy group, a 2-methylpentyloxy group, a 3-methylpentyloxy group, a 1,1-dimethylbutoxy group, a 1,2-dimethylbutoxy group, a 2,2-dimethylbutoxy group, a 1,3-dimethylbutoxy group, a 2,3-dimethylbutoxy group, a 3,3-dimethylbutoxy group, a 1-ethyl butoxy group, a 2-ethyl butoxy group, a 1,1,2-trimethylpropoxy group, a 1,2,2-trimethylpropoxy group, a 1-ethyl-1-methylpropoxy group, a 1-ethyl-2-methylpropoxy group, and the like, preferably a methoxy group, an ethoxy group, a *n*-propoxy group, an isopropoxy group, a *n*-butoxy group, an isobutoxy group, a *sec*-butoxy group, a *tert*-butoxy group, and the like.

The term "C₁₋₆ alkylthio group" used in the present specification means a group with a sulfur atom bonded to the terminus of the previously defined "C₁₋₆ alkyl group". Examples of "C₁₋₆ alkylthio group" include a methylthio group, an ethylthio group, a *n*-propylthio group, an isopropylthio group, a *n*-butylthio group, an isobutylthio group, a *sec*-butylthio group, a *tert*-butylthio group, a *n*-pentylthio group, an isopentylthio group, a *sec*-pentylthio group, an neopentylthio group, a 1-methylbutylthio group, a 2-methylbutylthio group, a 1,1-dimethylpropylthio group, a 1,2-dimethylpropylthio group, a *n*-hexylthio group, an isohexylthio group, a 1-enethylpentylthio group, a 2-methylpentylthio group, a 3-methylpentylthio group, a 1,1-dimethylbutylthio group, a 1,2-dimethylbutylthio group, a 2,2-dimethylbutylthio group, a 1,3-dimethylbutylthio group, a 2,3-dimethylbutylthio group, a 3,3-dimethylbutylthio group, a 1-ethylbutylthio group, a 2-ethylbutylthio group, a 1,1,2-trimethylpropylthio group, a 1,2,2-trimethylpropylthio group, a 1-ethyl-1-methylpropylthio group, a 1-ethyl-2-methylpropylthio group, and the like, preferably a methylthio group, an ethylthio group, a *n*-propylthio group, an isopropylthio group, a *n*-butylthio group, an isobutylthio group, a sec-butylthio group, a *tert*-butylthio group, and the like.

The term "C₁₋₆ alkylcarbonyl group" used in the present specification means a group with a carbonyl group bonded to the terminus of the previously defined "C₁₋₆ alkyl group". Examples of "C₁₋₆ alkyl carbonyl group" include a methylcarbonyl group, an ethylcarbonyl group, a *n*-propylcarbonyl group, an isopropylcarbonyl group, and the like.

The term "C₁₋₆ alkoxycarbonyl group" used in the present specification means a group with a carbonyl group bonded to the terminus of the previously defined "C₁₋₆ alkoxy group". Examples of "C₁₋₆ alkoxycarbonyl group" include a methoxy carbonyl group, an ethoxy carbonyl group, a *n*-propoxy carbonyl group, an isopropoxy carbonyl group, and the like.

The term "C₁₋₆ alkylsulfonyl group" used in the present specification means a group with a sulfonyl group bonded to the terminus of the previously defined "C₁₋₆ alkyl group". Examples of "C₁₋₆ alkylsulfonyl group" include a methylsulfonyl group, an ethylsulfonyl group, a *n*-propylsulfonyl group, an isopropylsulfonyl group, and the like.

The term "C₂₋₆ alkenyloxy group" used in the present specification means a group with an oxygen atom bonded to the terminus of the previously defined "C₂₋₆ alkenyl group". Examples of "C₂₋₆ alkenyloxy group" include an ethenyloxy group, a 1-propenyloxy group, a 2-propenyloxy group, a 1-butenyloxy group, a 2-butenyloxy group, a 3-butenyloxy group, a 2-methyl-1-propenyloxy group, a pentenyloxy group, a 3-methyl-2-butenyloxy group, a hexenyloxy group, a hexanedienyloxy group, and the like, preferably an ethenyloxy group, a 1-propenyloxy, group, a 2-propenyloxy group, a 1-butenyloxy group, a 2-butenyloxy group, a 3-butenyloxy group, a 2-methyl-1-propenyloxy group, a 3-methyl-2-butenyloxy group, and the like.

The term "C₂₋₆ alkenyl thio group" used in the present specification means a group with a sulfur atom bonded to the terminus of the previously defined "C₂₋₆ alkenyl group". Examples of "C₂₋₆ alkenyl thio group" include an ethenylthio group, a 1-propenylthio group, a 2-propenylthio group, a 1-butenylthio group, a 2-butenylthio group, a 3-butenylthio group, a 2-methyl-1-propenylthio group, a pentenylthio group, a 3-methyl-2-butenylthio group, a hexenylthio group, a hexanedienylthio group, and the like, preferably an ethenylthio group, a 1-propenylthio group, a 2-propenylthio group, a 1-butenylthio group, a 2-butenylthio group, a 3-butenylthio group, a 2-methyl-1-propenylthio group, a 3-methyl-2-butenylthio group, and the like.

The term "C₂₋₆ alkynyloxy group" used in the present specification means a group with an oxygen atom bonded to the terminus of the previously defined "C₂₋₆ alkynyl group". Examples of "C₂₋₆ alkynyloxy group" include an ethynyloxy group, a 1-propynyloxy group, a 2-propynyloxy group, a 1-butynyloxy group, a 2-butynyloxy group, a 3-butynyloxy group, a pentynyloxy group, a hexynyloxy group, a hexanediynyloxy group, and the like, preferably an ethynyloxy group, a 1-propynyloxy group, a 2-propynyloxy group, a 1-butynyloxy group, a 2-butynyloxy group, a 3-butynyloxy group, and the like.

The term "C₂₋₆ alkynylthio group" used in the present specification means a group with a sulfur atom bonded to the terminus of the previously defined "C₂₋₆ alkynyl group". Examples of "C₂₋₆ alkynyl thio group" include an ethynylthio group, a 1-propynylthio group, a 2-propynylthio group, a 1-butynylthio group, a 2-butynylthio group, a 3-butynylthio group, a pentynylthio group, a hexynylthio group, a hexanediynyl thio group, and the like, preferably an ethynylthio group, a 1-propynylthio group, a 2-propynylthio group, a 1-butynylthio group, a 2-butynylthio group, a 3-butynylthio group, and the like.

The term "C₃₋₈ cycloalkoxy group" used in the present specification means a group with an oxygen atom bonded to the terminus of the previously defined "C₃₋₈ cycloalkyl group". Examples of "C₃₋₈ cycloalkoxy group" include a cyclopropoxy group, a cyclobutoxy group, a cyclopentyloxy group, a cyclohexyloxy group, a cycloheptyloxy group, a cyclooctyloxy group, and the like, preferably a cyclopropoxy group, a cyclobutoxy group, a cyclopentyloxy group, a cyclohexyloxy group, and the like.

The term "C₃₋₈ cycloalkylthio group" used in the present specification means a group with a sulfur atom bonded to the terminus of the previously defined "C₃₋₈ cycloalkyl group". Examples of "C₃₋₈ cycloalkylthio group" include a cyclopropylthio group, a cyclobutylthio group, a cyclopentylthio group, a cyclohexylthio group, a cycloheptylthio group, a cyclooctylthio group, and the like, preferably a cyclopropylthio group, cyclobutylthio group, cyclopentylthio group, cyclohexylthio group, and the like.

Theb tem "C₃₋₈ cycloalkyl C₁₋₆ alkyl group" used in the present specification means a group with any hydrogen atom in the previously defined "C₁₋₆ alkyl group" substituted by the previously defined "C₃₋₈ cycloalkyl group". Examples of "C₃₋₈ cycloalkyl C₁₋₆ alkyl group"include a cyclopropylmethyl group, a cyclobutylmethyl group, a cyclopentylmethyl group, a cyclohexylmethyl group, a cyclopropylethyl group, a cyclobutylethyl group, a cyclopentylethyl group, a cyclohexyl ethyl group, and the like.

The term "C₃₋₈ cycloalkyl C₁₋₆ alkoxy group" in the present specification means a group with any hydrogen atom in the previously defined "C₁₋₆ alkoxy group" substituted by the previously defined "C₃₋₈ cycloalkyl group". Examples of "C₃₋₈ cycloalkyl C₁₋₆ alkoxy group" include a cyclopropylmethoxy group, a cyclobutylmethoxy group, a cyclopentylmethoxy group, a cyclohexylmethoxy group, a cyclopropylethoxy group, a cyclobutylethoxy group, a cyclopentylethoxy group, a cyclohexylethoxy group, and the like.

The term "C₃₋₈ cycloalkyl C₁₋₆ alkylthio group" used in the present specification means a group with any hydrogen atom in the previously defined "C₁₋₆ alkyl thio group" substituted by the previously defined "C₃₋₈ cycloalkyl group". Examples of "C₃₋₈ cycloalkyl C₁₋₆ alkylthio group" include a cyclopropylmethylthio group, a cyclobutylmethylthio group, a cyclopentylmethylthio group, a cyclohexylmethylthio group, a cyclopropylethylthio group, a cyclobutylethylthio group, a cyclopentylethylthio group, a cyclohexylethylthio group, and the like.

The term "C₃₋₈ cycloalkylydene C₁₋₆ alkyl group" used in the present specification means a group with any carbon atom from a cyclic saturated aliphatic hydrocarbon having 3 to 8 carbons and the previously defined "C₁₋₆ alkyl group" are bonded through a double bond. Examples of "C₃₋₈ cycloalkylydene C₁₋₆ alkyl group" include a cyclopropylydenemethyl group, a cyclobutylydenemethyl group, a cyclopentylydenemethyl group, a cyclohexylydenemethyl group, a cyclopropylydeneethyl group, a cyclobutylydeneethyl group, a cyclopentylydeneethyl group, a cyclohexylydeneethyl group, and the like.

The term "C₆₋₁₀ aryl group" used in the present specification refers to an aromatic hydrocarbon cyclic group having 6 to 10 carbons. Examples of "C₆₋₁₀ aryl group" include a phenyl group, a 1-naphthyl group, a 2-naphthyl group, an indenyl group, an azulenyl group, a heptalenyl group, and the like, preferably a phenyl group, a 1-naphthyl group, a 2-naphthyl group, and the like.

The term "C₆₋₁₀ aryloxy group" used in the present specification means a group with an oxygen atom bonded to the terminus of the previously defined "C₆₋₁₀ aryl group". Examples of "C₆₋₁₀ aryloxy group" include a phenoxy group, a 1-naphthyloxy group, a 2-naphthyloxy group, an indenyloxy group, an azulenyloxy group, a heptalenyloxy group, and the like, preferably a phenoxy group, a 1-naphthyloxy group, a 2-naphthyloxy group, and the like.

The term "C₆₋₁₀ arylthio group" used in the present specification means a group with a sulfur atom bonded to the terminus of the previously defined "C₆₋₁₀ aryl group". Examples of "C₆₋₁₀ arylthio group" include a phenylthio group, a 1-naphthylthio group, a 2-naphthylthio group, an indenylthio group, an azulenylthio group, a heptalenylthio group, and the like, preferably a phenylthio group, a 1-naphthylthio group, a 2-naphthylthio group, and the like.

The term "C₆₋₁₀ aryl C₁₋₆ alkyl group" used herein means a group with any hydrogen atom in the previously defined "C₁₋₆ alkyl group" substituted by the previously defined "C₆₋₁₀ aryl group". Examples of "C₆₋₁₀ aryl C₁₋₆ alkyl group" include a benzyl group, a 1-naphthylmethyl group, a 2-naphthylmethyl group, a phenetyl group, a 1-naphthylethyl group, a 2-naphthylethyl group, a 3-phenyl-1-propyl group, and the like.

The term "phenyl C₁₋₆ alkyl group" used in the present specification means a group with any hydrogen atom in the previously defined "C₁₋₆ alkyl group" substituted by a phenyl group. Examples of "phenyl C₁₋₆ alkyl group" include a benzyl group, a phenetyl group, a 3-phenyl-1-propyl group, and the like.

The term "C₆₋₁₀ aryl C₁₋₆ alkoxy group" used in the present specification means a group with any hydrogen atom in the previously defined "C₁₋₆ alkoxy group" substituted by the previously defined "C₆₋₁₀ aryl group". Examples of "C₆₋₁₀ aryl C₁₋₆ alkoxy group" include a benzyloxy group, a 1-naphthylmethoxy group, a 2-naphthylmethoxy group, a phenetyloxy group, a 1-naphthylethoxy group, a 2-naphthylethoxy group, a 3-phenyl-1-propoxy group, and the like.

The term "phenyl C₁₋₆ alkoxy group" used in the present specification means a group with any hydrogen atom in the previously defined "C₁₋₆ alkoxy group" substituted by a phenyl group. Examples of "phenyl C₁₋₆ alkoxy group" include a benzyloxy group, a phenetyloxy group, a 3-phenyl-1-propoxy group, and the like.

The term "C₆₋₁₀ aryl C₁₋₆ alkylthio group" used in the present specification means a group with any hydrogen atom in the previously defined "C₁₋₆ alkylthio group" substituted by the previously defined "C₆₋₁₀ aryl group". Examples of "C₆₋₁₀ aryl C₁₋₆ alkylthio group" include a benzylthio group, a phenetylthio group, a 3-phenyl-1-propylthio group, and the like.

The term "mono-C₁₋₆ alkylamino group" used in the present specification means a group with one hydrogen atom in an amino group substituted by the previously defined "C₁₋₆ alkyl group". Examples of "mono-C₁₋₆ alkyl amino group" include a methylamino group, an ethylamino group, a *n*-propylamino group, an isopropylamino group, a *n*-butyl amino group, an isobutylamino group, a *sec*-butylamino group, a *tert*-butylamino group, a *n*-pentylamino group, an isopentylamino group, a *sec*-pentylamino group, a neopentylamino group, a 1-methylbutylamirio group, a 2-methylbutylamino group, a 1,1-dimethylpropylamino group, a 1,2-dimethylpropylamino group, a *n*-hexylamino group, an isohexylamino group, a 1-methylpentylamino group, a 2-methylpentylamino group, a 3-methylpentylamino group, a 1,1-dimethylbutylamino group, a 1,2-dimethylbutylamino group, a 2,2-dimethylbutylamino group, a 1,3-dimethylbutylamino group, a 2,3-dimethylbutylamino group, a 3,3-dimethylbutylamino group, a 1-ethylbutylamino group, a 2-ethylbutylamino group, a 1,1,2-trimethylpropylamino group, a 1,2,2-trimethylpropylamino group, a 1-ethyl-1-methylpropylamino group, a 1-ethyl-2-methylpropylamino group, preferably a methylamino group, an ethylamino group, a n-propylamino group, an isopropylamino group, a *n*-butylamino group, an isobutylamino group, a *sec*-butylamino group, a *tert*-butyl amino group, and the like.

The term "mono-C₂₋₆ alkenylamino group" used in the present specification means a group with one hydrogen in an amino group substituted by the previously defined "C₂₋₆ alkenyl group". Examples of "mono-C₂₋₆ alkenylamino group" include an ethenylamino group, a 1-propenylamino group, a 2-propenylamino group, a 1-butenylamino group, a 2-butenylamino group, a 3-butenylamino group, a 2-methyl-1-propenylamino group, a pentenylamino group, a 3-methyl-2-butenylamino group, a hexenylamino group, a hexanedienylamino group, and the like, preferably an ethenylamino group, a 1-propenylamino group, a 2-propenylamino group, a 1-butenylamino group, a 2-butenylamino group, a 3-butenylamino group, a 2-methyl-1-propenylamino group, a 3-methyl-2-butenylamino group, and the like.

The term "mono-C₂₋₆ alkynylamino group" used in the present specification means a group with one hydrogen in an amino group substituted by the previously defined "C₂₋₆ alkynyl group". Examples of "mono-C₂₋₆ alkynylamino group" include an ethynylamino group, a 1-propynylamino group, a 2-propynylamino group, a 1-butynylamino group, a 2-butynylamino group, a 3-butynylamino group, a pentynylamino group, a hexynylamino group, a hexanediynylamino group, and the like, preferably an ethynylamino group, a 1-propynylamino group, a 2-propynylamino group, a 1-butynylamino group, a 2-butynylamino group, a 3-butynylamino group, and the like.

The term "mono-C₃₋₈ cycloalkylamino group" used in the present specification means a group with one hydrogen in an amino group substituted by the previously defined "C₃₋₈ cycloalkyl group". Examples of "mono-C₃₋₈ cycloalkylamino group" include a cyclopropylamino group, a cyclobutylamino group, a cyclopentylamino group, a cyclohexylamino group, a cycloheptylamino group, a cyclooctylamino group, and the like, preferably a cyclopropylamino group, a cyclobutylamino group, a cyclopentylamino group, a cyclohexylamino group, and the like.

The term "mono-C₆₋₁₀ arylamino group" used in the present specification means a group with one hydrogen in an amino group substituted by the previously defined "C₆₋₁₀ aryl group". Examples of "mono-C₆₋₁₀ arylamino group" include a phenylamino group, a 1-naphthylamino group, a 2-naphthylamino group, an indenylamino group, an azulenylamino group, a heptalenylamino group, and the like, preferably a phenylamino group, a 1-naphthylamino group, a 2-naphthylamino group, and the like.

The term "mono-C₃₋₈ cycloalkyl C₁₋₆ alkylamino group" used in the present specification means a group with one hydrogen in an amino group substituted by the previously defined "C₃₋₈ cycloalkyl C₁₋₆ alkyl group". Examples of "mono-C₃₋₈ cycloalkyl C₁₋₆ alkylamino group" include a cyclopropylmethylamino group, a cyclobutylmethylamino group, a cyclopentylmethylamino group, a cyclohexylmethylamino group, a cyclopropylethylamino group, a cyclobutylethylamino group, a cyclopentylethylamino group, a cyclohexylethylamino group, and the like.

The term "mono-C₆₋₁₀ aryl C₁₋₆ alkylamino group" used in the present specification means a group with one hydrogen in an amino group substituted by the previously defined "C₆₋₁₀ aryl C₁₋₆ alkyl group". Examples of "mono-C₆₋₁₀ aryl C₁₋₆ alkylamino group" include a benzylamino group, a 1-naphthylmethylamino group, a 2-naphthylmethylamino group, a phenetylamino group, a 1-naphthylethylamino group, a 2-naphthylethylamino group, and the like.

The term "di-C₁₋₆ alkylamino group" used in the present specification means a group with two hydrogen atoms in an amino group respectively substituted by an identical or different previously defined "C₁₋₆ alkyl group". Examples of "di-C₁₋₆ alkylamino group" include a N,N-dimethylamino group, a N,N-diethylamino group, a N,N-di-n-propylamino group, a N,N-di-isopropylamino group, a N,N-di-*n*-butylamino group, a N,N-di-isobutylamino group, a N,N-di-*sec*-butylamino group, a N,N-di-*tert*-butylamino group, a N-ethyl-N-methyl amino group, a N-*n*-propyl-N-methylamino group, a N-isopropyl-N-methylamino group, a N-*n*-butyl-N-methylamino group, a N-isobutyl-N-methylamino group, a N-*sec*-butyl-N-methylamino group, a N-*tert*-butyl-N-methylamino group, and the like, preferably a N,N-dimethylamino group, a N,N-diethylamino group, a N-ethyl-N-methyl amino group, and the like.

The term "N-C₂₋₆ alkenyl-N-C₁₋₆ alkylamino group" used in the present specification means a group with one hydrogen atom in an amino group substituted by the previously defined "C₂₋₆ alkenyl group", and the other hydrogen atom substituted by the previously defined "C₁₋₆ alkyl group". Examples of "N-C₂₋₆ alkenyl-N-C₁₋₆ alkylamino group" include a N-ethenyl-N-methylamino group, a N-1-propenyl-N-methylamino group, a N-2-properiyl-N-methylamino group, a N-1-butenyl-N-methylamino group, a N-2-butenyl-N-methylamino group, a N-3-butenyl-N-methylamino group, a N-2-methyl-1-propenyl-N-methylamino group, a N-pentenyl-N-methylamino group, a N-3-methyl-2-butenyl-N-methylamino group, a N-hexenyl-N-methylamino group, a N-hexanedienyl-N-methylamino group, and the like, preferably a N-ethenyl-N-methylamino group, a N-1-propenyl-N-methylamino group, a N-2-propenyl-N-methylamino group, a N-1-butenyl-N-methylamino group, a N-2-butenyl-N-methylamino group, a N-3-butenyl-N-methylamino group, a N-2-methyl-1-propenyl-N-methylamino group, a N-3-methyl-2-butenyl-N-methylamino group, and the like.

The term "N-C₂₋₆ alkynyl-N-C₁₋₆ alkylamino group" used in the present specification means a group with one hydrogen atom in an amino group substituted by the previously defined "C₂₋₆ alkynyl group" and the other hydrogen atom substituted by the previously defined "C₁₋₆ alkyl group". Examples of "N-C₂₋₆ alkynyl-N-C₁₋₆ alkylamino group" include a N-ethynyl-N-methylamino group, a N-1-propynyl-N-methylamino group, a N-2-propynyl-N-methylamino group, a N-1-butynyl-N-methylamino group, a N-2-butynyl-N-methylamino group, a N-3-butynyl-N-methylamino group, a N-pentynyl-N-methylamino group, a N-hexynyl-N-methylamino group, a N-hexanediynyl-N-methylamino group, and the like, preferably a N-ethynyl-N-methylamino group, a N-1-propynyl-N-methylamino group, a N-2-propynyl-N-methylamino group, a N-1-butynyl-N-methylamino group, a N-2-butynyl-N-methylamino group, a N-3-butynyl-N-methylamino group, and the like.

The term "N-C₃₋₈ cycloalkyl-N-C₁₋₆ alkylamino group" used in the present specification means a group with one hydrogen atom in an amino group substituted by the previously defined "C₃₋₈ cycloalkyl group" and the other hydrogen atom substituted by the previously defined "C₁₋₆ alkyl group". Examples of "N-C₃₋₈ cycloalkyl-N-C₁₋₆ alkylamino group" include a N-cyclopropyl-N-methylamino group, a N-cyclobutyl-N-methylamino group, a N-cyclopentyl-N-methylamino group, a N-cyclohexyl-N-methylamino group, a N-cycloheptyl-N-methylamino group, a N-cyclooctyl-N-methylamino group, and the like, preferably a N-cyclopropyl-N-methylamino group, a N-cyclobutyl-N-methylamino group, a N-cyclopentyl-N-methylamino group, a N-cyclohexyl-N-methylamino group, and the like.

The term "N-C₆₋₁₀ aryl-N-C₁₋₆ alkylamino group" used in the present specification means a group with one hydrogen atom in an amino group substituted by the previously defined "C₆₋₁₀ aryl group" and the other hydrogen atom substituted by the previously defined "C₁₋₆ alkyl group". Examples of "N-C₆₋₁₀ aryl-N-C₁₋₆ alkylamino group" include a N-phenyl-N-methylamino group, a N-1-naphthyl-N-methylamino group, a N-2-naphthyl-N-methylamino group, a N-indenyl-N-methylamino group, a N-azulenyl-N-methylamino group, a N-heptalenyl-N-methylamino group, and the like, preferably a N-phenyl-N-methylamino group, a N-1-naphthyl-N-methylamino group, a N-2-naphthyl-N-methylamino group, and the like.

The term "N-C₃₋₈ cycloalkyl C₁₋₆ alkyl-N-C₁₋₆ alkylamino group" used in the present specification means a group with one hydrogen atom in an amino group substituted by the previously defined "C₃₋₈ cycloalkyl C₁₋₆ alkyl group" and the other hydrogen atom substituted by the previously defined "C₁₋₆ alkyl group". Examples of "N-C₃₋₈ cycloalkyl C₁₋₆ alkyl-N-C₁₋₆ alkylamino group" include a N-cyclopropylmethyl-N-methylamino group, a N-cyclobutylmethyl-N-methylamino group, a N-cyclopentylmethyl-N-methylamino group, a N-cyclohexylmethyl-N-methylamino group, a N-cyclopropylethyl-N-amino group, a N-cyclobutylethyl-N-methylamino group, a N-cyclopentylethyl-N-methylamino group, a N-cyclohexylethyl-N-methylamino group, and the like.

The term "N-C₆₋₁₀ aryl C₁₋₆ alkyl-N-C₁₋₆ alkylamino group" used in the present specification means a group with one hydrogen atom in an amino group substituted by the previously defined "C₆₋₁₀ aryl C₁₋₆ alkyl group" and the other hydrogen atom substituted by the previously defined "C₁₋₆ alkyl group". Examples of "N-C₆₋₁₀ aryl C₁₋₆ alkyl-N-C₁₋₆ alkylamino group" include a N-benzyl-N-methylamino group, a N-1-naphthylmethyl-N-methylamino group, a N-2-naphthylmethyl-N-methylamino group, a N-phenetyl-N-methylamino group, a N-1-naphthylethyl-N-methylamino group, a N-2-naphthylethyl-N-methylamino group, and the like.

The term "halogen atom" used in the present specification means a fluorine atom, a chlorine atom, a bromine atom or an iodine atom, preferably a fluorine atom, a chlorine atom or a bromine atom.

The term "heteroatom" used in the present specification means a nitrogen atom, a sulfur atom or an oxygen atom

The term "5 to 10-membered heterocyclic group" means a monovalent group derived by removing one hydrogen atom from any position on an aromatic or non-aromatic ring having one to a plurality of heteroatoms among the atoms constituting the ring, the number of atoms constituting the ring being 5 to 10. Examples of "5 to 10-membered heterocyclic group" include a furyl group (for instance, a 2-furyl group, a 3-furyl group, and the like), a thienyl group (for instance, a 2-thienyl group, a 3-thienyl group, and the like), a pyrrolyl group (for instance, a 1-pyrrolyl group, a 2-pyrrolyl group, a 3-pyrrolyl group, and the like), a pyridyl group (for instance, a 2-pyridyl group, a 3-pyridyl group, a 4-pyridyl group, and the like), a pyrazinyl group, a pyridazinyl group (for instance, a 3-pyridazinyl group, a 4-pyridazinyl group, and the like), a pyrimidinyl group (for instance, a 2-pyrimidinyl group, a 4-pyrimidinyl group, a 5-pyrimidinyl group, and the like), a triazolyl group (for instance, a 1,2,3-triazolyl group, a 1,2,4-triazolyl group, and the like), a tetrazolyl group (for instance, a 1*H*-tetrazolyl group, a 2H-tetrazolyl group, and the like), a thiazolyl group (for instance, a 2-thiazolyl group, a 4-thiazolyl group, a 5-thiazolyl group, and the like), a pyrazolyl group (for instance, a 3-pyrazolyl group, a 4-pyrazolyl group, and the like), an oxazolyl group (for instance, a 2-oxazolyl group, a 4-oxazolyl group, a 5-oxazolyl group, and the like), an isooxazolyl group (for instance, a 3-isooxazolyl group, a 4-isooxazolyl group, a 5-isooxazolyl group, and the like), an isothiazolyl group (for instance, a 3-isothiazolyl group, a 4-isothiazolyl group, a 5-isothiazolyl group, and the like), a quinolyl group (for instance, a 5-quinolyl group, a 6-quinolyl group, a 7-quinolyl group, a 8-quinolyl group, and the like), an isoquinolyl group (for instance, a 5-isoquinolyl group, a 6-isoquinolyl group, a 7-isoquinolyl group, a 8-isoquinolyl group, and the like), a naphthylidinyl group (for instance, a [1,5]naphthyridin-2-yl group, a [1,5]naphthyridin-3-yl group, a [1,8]naphthyridin-2-yl group, a [1,8]naphthyridin-3-yl group, and the like), a quinoxalinyl group (for instance, a 5-quinoxalinyl group, a 6-quinoxalinyl group, a 7-quinoxalinyl group, a 8-quinoxalinyl group, and the like), a cinnolinyl group (for instance, a 5-cinnolinyl group, a 6-cinnolinyl group, a 7-cinnolinyl group, a 8-cinnolinyl group, and the like), a quinazolinyl group (for instance, a 4-quinazolinyl group, a 5-quinazolinyl group, a 6-quinazolinyl group, a 7-quinazolinyl group, a 8-quinazolinyl group, and the like), an imidazopyridyl group (for instance, an imidazo[1,2-a]pyridin-6-yl group, and the like), a benzothiazolyl group (for instance, a benzothiazol-4-yl group, a benzothiazol-5-yl group, a benzothiazol-6-yl group, a benzothiazol-7-yl group, and the like), a benzooxazolyl group (for instance, a benzoxazol-4-yl group, a benzoxazole-5-yl group, a benzoxazole-6-yl group, a benzoxazole-7-yl group, and the like), a benzoimidazolyl group (for instance, a benzoimidazol-4-yl group, a benzoimidazol-5-yl group, a benzoimidazol-6-yl group, a benzoimidazol-7-yl group, and the like), an indolyl group (for instance, an indol-4-yl group, an indol-5-yl group, an indol-6-yl group, an indol-7-yl group, and the like), a pyrrolopyridyl group (for instance, a 1*H*-pyrrolo[2,3-b]pyridin-5-yl group, a pyrrolo[3,2-b]pyridin-1-yl group, and the like), a thienopyridyl group (for instance, a thieno[2,3-b]pyridin-5-yl group, a thieno[3,2-b]pyridin-6-yl group, and the like), a furopyridyl group (for instance, a furo[2,3-b]pyridin-5-yl group, a furo[3,2-b]pyridin-6-yl group, and the like), a 2,3-dihydro-1*H*-pyrrolo[2,3-b]pyridin-5-yl group, a benzothiadiazolyl group (for instance, a benzo[1, 2, 5]thiadiazol-5-yl group, and the like), a benzoxadiazolyl group (for instance, a benzo[1, 2, 5]oxadiazol-5-yl group, and the like), a pyridopyrimidinyl group (for instance, a pyrido[2,3-d]pyrimidin-4-yl group, and the like), a benzofuryl group (for instance, a benzofuran-4-yl group, a benzofuran-5-yl group, a benzofuran-6-yl group, a benzofuran-7-yl group, and the like), a benzothienyl group (for instance, a benzothiophen-4-yl group, a benzothiophen-5-yl group, a benzothiophen-6-yl group, a benzothiophen-7-yl group, and the like), a benzo[1,3]dioxol group (for instance, a benzo[1,3]dioxol-5-yl group, and the like) and the like. Examples of non-aromatic "5 to 10-membered heterocyclic group" include a pyrrolidinyl group, a piperidinyl group, a homopiperidinyl group, a piperazinyl group, a homopiperazinyl group, a morpholinyl group, a thiomorpholinyl group, a tetrahydrofuryl group, a tetrahydropyranyl group, and the like.

The term "5 to 10-membered heterocyclic group containing at least one nitrogen atom" used in the present specification means a monovalent group derived by removing one hydrogen atom from any position on an aromatic or non-aromatic ring containing one to a plurality of heteroatoms among the atoms constituting the ring, the number of atoms constituting the ring being 5 to 10, and containing at least one nitrogen atom. Examples of "5 to 10-membered heterocyclic group containing at least one nitrogen atom" include a pyrrolyl group, a pyridyl group, a pyrazinyl group, a pyridazinyl group, a pyrimidinyl group, a tetrazolyl group, a thiazolyl group, a pyrazolyl group, an oxazolyl group, an isooxazolyl group, an isothiazolyl group, a quinolyl group, an isoquinolyl group, a naphthylidinyl group, a quinoxalinyl group, a cinnolinyl group, a quinazolinyl group, an imidazopyridyl group, a benzothiazolyl group, a benzoxazolyl group, a benzoimidazolyl group, an indolyl group, a pyrrolopyridyl group, an thienopyridyl group, a furopyridyl group, a 2,3-dihydro-1*H*-pyrrolo[2,3-b]pyridin-5-yl group, a benzothiadiazolyl group, a benzoxadiazolyl group, a pyridopyrimidinyl group, and the like.

The term "5 to 10-membered heterocyclic C₁₋₆ alkyl group" used in the present specification means a group with any hydrogen atom in the previously defined "C₁₋₆ alkyl group" substituted by the previously defined "5 to 10-membered heterocyclic group". Examples of "5 to 10-membered heterocyclic C₁₋₆alkyl group" include a furylmethyl group, a thienylmethyl group, a pyrrolylmethyl group, a pyridylmethyl group, a triazolylmethyl group, a tetrazolylmethyl group, a thiazolylmethyl group, a pyrazolylmethyl group, an oxazolylmethyl group, a benzo[1,3]dioxolmethyl group, a tetrahydrofurylmethyl group, a furylethyl group, a thienylethyl group, a pyrrolylethyl group, a pyridylethyl group, a triazolylethyl group, a tetrazolylethyl group, a thiazolylethyl group, a pyrazolylethyl group, an oxazolylethyl group, a benzo[1,3]dioxolethyl group, a tetrahydrofurylethyl group, and the like.

The term "furyl C₁₋₆ alkyl group" used in the present specification means a group with any hydrogen atom in the previously defined "C₁₋₆ alkyl group" substituted by a furyl group. Examples of "furyl C₁₋₆ alkyl group" include a furylmethyl group, a furylethyl group, and the like.

The term "thienyl C₁₋₆ alkyl group" used in the present specification means a group with any hydrogen atom in the previously defined "C₁₋₆ alkyl group" substituted by a thienyl group. Examples of "thienyl C₁₋₆ alkyl group" include a thienylmethyl group, a thienylethyl group, and the like.

The term "benzofuryl C₁₋₆ alkyl group" used in the present specification means a group with any hydrogen atom in the previously defined "C₁₋₆ alkyl group" substituted by a benzofuryl group. Examples of "benzofuryl C₁₋₆ alkyl group" include a benzofurylmethyl group, a benzofurylethyl group, and the like.

The term "benzothienyl C₁₋₆ alkyl group" used in the present specification means a group with any hydrogen atom in the previously defined "C₁₋₆ alkyl group" substituted by a benzothienyl group. Examples of "benzothienyl C₁₋₆ alkyl group" include a benzothienylmethyl group, a benzothienylethyl group, and the like.

The tem "5 to 10-membered heterocyclic oxy group" used in the present specification means a group with an oxygen atom bonded to the terminus of the previously defined "5 to 10-membered heterocyclic group". Examples of "5 to 10-membered heterocyclic oxy group" include a furyloxy group, a thienyloxy group, a pyrrolyloxy group, a pyridyloxy group, a triazolyloxy group, a tetrazolyloxy group, a thiazolyloxy group, a pyrazolyloxy group, an oxazolyloxy group, a benzo[1,3]dioxoloxy group, a tetrahydrofuryloxy group, and the like.

The term "5 to 10-membered heterocyclic C₁₋₆ alkoxy group" used in the present specification means a group with any hydrogen atom in the previously defined "C₁₋₆ alkoxy group" substituted by the previously defined "5 to 10-membered heterocyclic group". Examples of "5 to 10-membered heterocyclic C₁₋₆alkoxy group" include a furylmethoxy group, a thienylmethoxy group, a pyrrolylmethoxy group, a pyridylmethoxy group, a triazolylmethoxy group, a tetrazolylmethoxy group, a thiazolylmethoxy group, a pyrazolylmethoxy group, an oxazolylmethoxy group, a benzo[1,3]dioxolmethoxy group, a tetrahydrofurylmethoxy group, a furylethoxy group, a thienylethoxy group, a pyrrolylethoxy group, a pyridylethoxy group, a triazolylethoxy group, a tetrazolylethoxy group, a thiazolylethoxy group, a pyrazolylethoxy group, an oxazolylethoxy group, a benzo[1,3]dioxolethoxy group, a tetrahydrofurylethoxy group, and the like.

The term "furyl C₁₋₆ alkoxy group" used in the present specification means a group with any hydrogen atom in the previously defined "C₁₋₆ alkoxy group" substituted by a furyl group. Examples of "furyl C₁₋₆ alkoxy group" include a furylmethoxy group, a furylethoxy group, and the like.

The term "thienyl C₁₋₆ alkoxy group" used in the present specification means a group with any hydrogen atom in the previously defined "C₁₋₆ alkoxy group" substituted by a thienyl group. Examples of "thienyl C₁₋₆ alkoxy group" include a thienylmethoxy group, a thienylethoxy group, and the like.

The term "pyridyl C₁₋₆ alkoxy group" used in the present specification means a group with any hydrogen atom in the previously defined "C₁₋₆ alkoxy group" substituted by a pyridyl group. Examples of "pyridyl C₁₋₆ alkoxy group" include a pyridylmethoxy group, a pyridylethoxy group, and the like.

The term "5 to 10-membered heterocyclic C₁₋₆ alkylthio group" used in the present specification means a group with any hydrogen atom in the previously defined "C₁₋₆ alkyl thio group" substituted by the previously defined "5 to 10-membered heterocyclic group". Examples of "5 to 10-membered heterocyclic C₁₋₆ alkylthio group" include a furylmethylthio group, a thienylmethylthio group, a pyrrolylmethylthio group, a pyridylmethylthio group, a triazolylmethylthio group, a tetrazolylmethylthio group, a thiazolylmethylthio group, a pyrazolylmethylthio group, an oxazolylmethylthio group, a benzo[1,3]dioxolmethylthio group, a tetrahydrofurylmethylthio group, a furylethylthio group, a thienylethylthio group, a pyrrolylethylthio group, a pyridylethylthio group, a triazolylethylthio group, a tetrazolylethylthio group, a thiazolylethylthio group, a pyrazolylethylthio group, an oxazolylethylthio group, a benzo[1,3]dioxolethylthio group, a tetrahydrofurylethylthio group, and the like.

The term "mono-5 to 10-membered heterocyclic C₁₋₆ alkylamino group" used in the present specification means a group with one hydrogen in an amino group substituted by the previously defined "5 to 10-membered heterocyclic C₁₋₆ alkyl group". Examples of "mono-5 to 10-membered heterocyclic C₁₋₆ alkylamino group" include a furylmethylamino group, a thienylmethylamino group, a pyrrolylmethylamino group, a pyridylmethylamino group, a triazolylmethylamino group, a tetrazolylmethylamino group, a thiazolylmethylamino group, a pyrazolylmethylamino group, an oxazolylmethylamino group, a tetrahydrofurylmethylamino group, a furylethylamino group, a thienylethylamino group, a pyrrolylethylamino group, a pyridylethylamino group, a triazolylethylamino group, a tetrazolylethylamino group, a thiazolylethylamino group, a pyrazolylethylamino group, an oxazolylethylamino group, a tetrahydrofurylethylamino group, a triazolyl-1-propylaminogroup, and the like.

The term "N-5 to 10-membered heterocyclic C₁₋₆ alkyl-N-C₁₋₆ alkylamino group" used in the present specification means a group with one hydrogen atom in an amino group substituted by the previously defined "5 to 10-membered heterocyclic C₁₋₆ alkyl group" and the other hydrogen atom substituted by the previously defined "C₁₋₆ alkyl group". Examples of "N-5 to 10-membered heterocyclic C₁₋₆ alkyl-N-C₁₋₆ alkylamino group" include a N-furylmethyl-N-methylamino group, a N-thienylmethyl-N-methylamino group, a N-pyrrolylmethyl-N-methylamino group, a N-pyridylmethyl-N-methylamino group, a N-triazolylmethyl-N-methylamino group, a N-tetrazolylmethyl-N-methylamino group, a N-thiazolylmethyl-N-methylamino group, a N-pyrazolylmethyl-N-methylamino group, a N-oxazolylmethyl-N-methylamino group, a N-tetrahydrofurylmethyl-N-methylamino group, a N-furylethyl-N-methylamino group, a N-thienylethyl-N-methylamino group, a N-pyrrolylethyl-N-methylamino group, a N-pyridylethyl-N-methylamino group, a N-triazolylethyl-N-methylamino group, a N-tetrazolylethyl-N-methylamino group, a N-thiazolylethyl-N-methylamino group, a N-pyrazolylethyl-N-methylamino group, a N-oxazolylethyl-N-methylamino group, a N-tetrahydrofurylethyl-N-methylamino group, and the like.

The term "may have a substituent" used in the present specification means may have one to a plurality of substituents in any combination at substitutable sites.

The term "having a substituent" used in the present specification means to have one to a plurality of substituents in any combination at substitutable sites.

The term "A¹" used in the present specification means a 3-pyridyl group or a 6-quinolyl group (with the proviso that each above-mentioned group may have one to three substituents selected from the above Substituent Groups a-1 and a-2).

Preferences for the term "A¹" are shown below in descending order when "A¹" is a 3-pyridyl group.
1) a 3-pyridyl group (with the proviso that the 3-pyridyl group may have one to three substituents selected from the above Substituent Groups c-1 and c-2)
2) a 3-pyridyl group (with the proviso that the 3-pyridyl group may have one to three substituents selected from the above Substituent Groups c'-1 and c'-2)
3) a group represented by the formula: 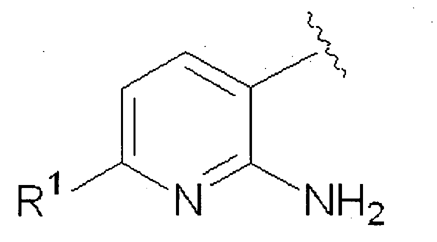 (wherein R¹ represents a hydrogen atom or a substituent selected from the above Substituent Groups c-1 and c-2)
4) a group represented by the formula: 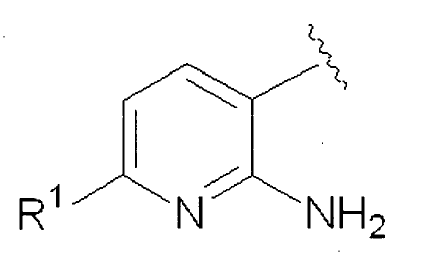 (wherein R¹ represents a hydrogen atom or a substituent selected from the above Substituent Groups c'-1 and c'-2)
5-1) a group represented by the formula: 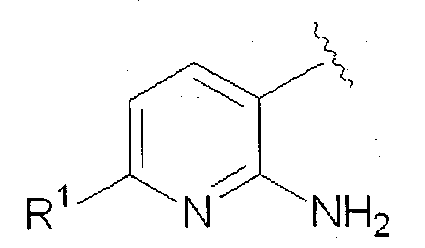 (wherein R¹ represents a hydrogen atom, an amino group, a C₁₋₆ alkyl group that may have one C₁₋₆ alkoxy group, C₂₋₆ alkenyl group, a mono-C₁₋₆ alkylamino group that may have one C₁₋₆ alkoxy group, or an C₁₋₆ alkylcarbonyl group)
5-2) a group represented by the formula: 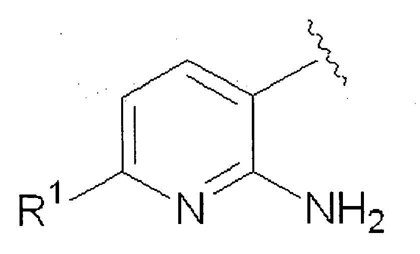 (wherein R¹ represents a C₁₋₆ alkyl group having one halogen atom)
6-1) a group represented by the formula: 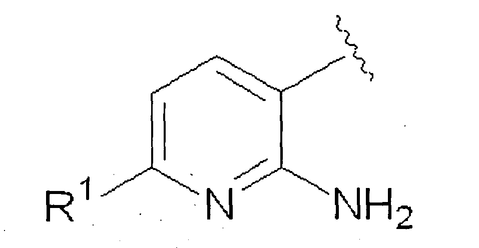 (wherein R¹ represents a hydrogen atom, an amino group, a methoxymethyl group, an ethenyl group, an ethoxyethylamino group or a methylcarbonyl group)
6-2) a group represented by the formula: 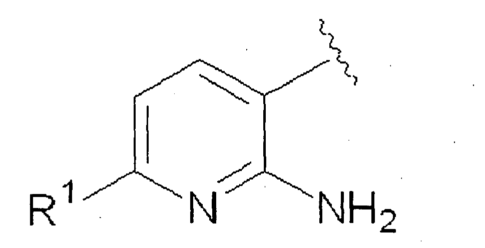 (wherein R¹ represents a fluoroethyl group)

Preferences for "A¹" are shown below in descending order when "A¹" is a 6-quinolyl group.
1) a 6-quinolyl group (with the proviso that the 6-quinolyl group may have one to three substituents selected from the above Substituent Groups c-1 and c-2)
2) a 6-quinolyl group (with the proviso that the 6-quinolyl group may have one to three substituents selected from the above Substituent Groups c'-1 and c'-2)
3) a 6-quinolyl group
   Examples of the "group represented by the formula -C(=N-R^{a1})R^{a2}" (wherein R^{a1} and R^{a2} have the same meanings as defined above) used in the present specification preferably include a group represented by the formula -C(=N-OH)R^{a2} (wherein R^{a2} has the same meaning as defined above), and a group represented by the formula -C(=N-OH)CH₃ being more preferably.

The term "X¹" used in the present specification means a group represented by the formula -C(=O)-NH-.

The term "E" used in the present specification means a furyl group, a thienyl group or a phenyl group (with the proviso that each above-mentioned group has one or two substituents selected from the above Substituent Groups a-1 and a-2).
Preferences for "E" are shown below in descending order.
1) a furyl group, a thienyl group or a phenyl group (with the proviso that each above-mentioned group has one or two substituents selected from the above Substituent Groups e-1 and e-2)
2-1) a furyl group, a thienyl group or a phenyl group (with the proviso that each above-mentioned group has one substituent selected from the above Substituent Groups g-1 and g-2)
2-2) a furyl group, a thienyl group or a phenyl group (with the proviso that each above-mentioned group has one C₁₋₆ alkoxy C₁₋₆ alkyl group or one C₁₋₆ alkoxy group)
3) a 2-furyl group, a 2-thienyl group or a phenyl group (with the proviso that each above-mentioned group has one substituent selected from the above Substituent Groups g-1 and g-2)
4-1) a 2-furyl group, a 2-thienyl group or a phenyl group (with the proviso that each above-mentioned group has one phenyl C₁₋₆ alkyl group that may have one halogen atom or one C₁₋₆ alkyl group, or one phenoxy group that may have one halogen atom or one C₁₋₆ alkyl group)
4-2) a 2-furyl group, a 2-thienyl group or a phenyl group (with the proviso that each above-mentioned group has one C₁₋₆ alkoxy C₁₋₆ alkyl group or one C₁₋₆ alkoxy group)
5-1) a 2-furyl group, a 2-thienyl group or a phenyl group (with the proviso that each above-mentioned group has one benzyl group that may have one fluorine atom, one chlorine atom or one methyl group, or has one phenoxy group that may have one fluorine atom, one chlorine atom or one methyl group)
5-2) a 2-furyl group, a 2-thienyl group or a phenyl group (with the proviso that each above-mentioned group has one n-butoxy methyl group or one *n*-butoxy group)
6) a 2-furyl group or a 2-thienyl group (with the proviso that each above-mentioned group has one phenyl C₁₋₆ alkyl group that may have one halogen atom or one C₁₋₆ alkyl group, or one phenoxy group that may have one halogen atom or one C₁₋₆ alkyl group)
7-1) a 2-furyl group or a 2-thienyl group (with the proviso that each above-mentioned group has one benzyl group that may have one fluorine atom, one chlorine atom or one methyl group, or has one phenoxy group that may have one fluorine atom, one chlorine atom or one methyl group)
7-2) a phenyl group (with the proviso that each above-mentioned group has one *n*-butoxy methyl group or one *n*-butoxy group).

Examples of compound (I-a) include compounds in which the previously defined "A¹" and the previously defined "E" are arbitrary combined, preferably
(1) a compound wherein A¹ is a 3-pyridyl group and E is a 2-furyl group,
(2) a compound wherein A¹ is a 3-pyridyl group and E is a 2-thienyl group,
(3) a compound wherein A¹ is a 3-pyridyl group and E is a phenyl group ,
(4) a compound wherein A¹ is a 6-quinolyl group and E is a 2-furyl group,
(5) a compound wherein A¹ is a 6-quinolyl group and E is a 2-thienyl group or
(6) a compound wherein A¹ is a 6-quinolyl group and E is a phenyl group, more preferably

(1) a compound wherein A¹ is a 3-pyridyl group and E is a 2-furyl group,
(2) a compound wherein A¹ is a 3-pyridyl group and E is a 2-thienyl group,
(3) a compound wherein A¹ is a 3-pyridyl group and E is a phenyl group ,
(4) a compound wherein A¹ is a 6-quinolyl group and E is a 2-furyl group or
(5) a compound wherein A¹ is a 6-quinolyl group and E is a 2-thienyl group and still more preferably

(1) a compound wherein A¹ is a 3-pyridyl group and E is a 2-furyl group,
(2) a compound wherein A¹ is a 3-pyridyl group and E is a 2-thienyl group or
(3) a compound wherein A¹ is a 3-pyridyl group and E is a phenyl group.

Examples of the term "salt" used in the present specification include a salt with an inorganic acid, a salt with an organic acid, a salt with an inorganic base, a salt with an organic base, a salt with an acidic or basic amino acid or the like. Among these salts, it is preferable that a salt used herein be a pharmaceutically acceptable.
Preferable examples of the salt with the inorganic acid include salts with hydrochloric acid, hydrobromic acid, sulfuric acid, nitric acid, phosphoric acid or the like. Preferable examples of the salt with the organic acid include salts with acetic acid, succinic acid, fumaric acid, maleic acid, tartaric acid, citric acid, lactic acid, stearic acid, benzoic acid, methanesulfonic acid, ethanesulfonic acid, *p*-toluenesulfonic acid or the like.
Preferable examples of the salt with the inorganic base include alkaline metal salts such as sodium salt and potassium salt, alkaline earth metal salts such as calcium salt and magnesium salt, aluminum salt, ammonium salt or the like. Preferable examples of the salt with the organic base include salts with diethyl amine, diethanolamine, meglumine, N,N-dibenzyl ethylenediamine or the like.
Preferable examples of the salt with the acidic amino acid include salts with aspartic acid, glutamic acid or the like. Preferable examples of the salt with the basic amino acid include salts with arginine, lysine, ornithine or the like.

The term "Antimalarial agent" used in the present specification means a prophylactic agent and / or therapeutic agent for tropical malaria, tertian malaria, quartan malaria and/or ovale malaria.

### Advantageous Effect of the Invention

The heterocyclic compound (I-a) according to the present invention or salts thereof or hydrates thereof demonstrate excellent antimalarial activity based on the GWT1 inhibitory action of malaria protozoa, and is useful as a prophylactic agent and / or therapeutic agent for tropical malaria, tertian malaria, quartan malaria and / or ovale malaria.

### Best Mode for Carrying Out the Invention

Methods for preparing the compound represented by formula (I-a) (hereinafter referred to as compound (I-a)) according to the present invention will be described. Although the compound according to the present invention can be synthesized using conventional organic synthesis means, it can be satisfactorily synthesized by the methods shown in the following [Preparation Method 1]. In addition, conversion of the substituents on A¹ and E in compound (I-a) according to the present invention can be carried out by the methods shown in the following [Preparation Method 4-1] to [Preparation Method 4-5], and the like.

### [General synthesis method]

### [Preparation Method 1] Representative preparation method for compound (1 a)

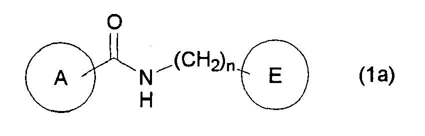

[wherein each symbol has the same meaning as defined above.]

### [Preparation Method 1-1] Amidation

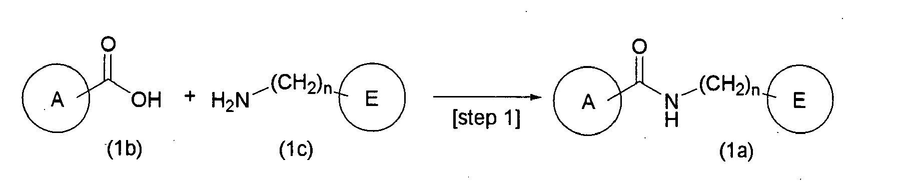

Compound (1b) which is a commercially available product can be used, or compound (1b) can also be prepared from a commercially available product by the well known method. In addition, compound (1b) can also be prepared by the methods described in the preparation examples among the following examples.
Compound (1 c) which is a commercially available product can be used, or compound (1 c) can also be prepared from a commercially available product by the well known method. In addition, compound (1c) can also be prepared by the method described in the preparation examples among the following examples or [Preparation Method 1-2-1], or the like.

### [Step 1]

This step is a step wherein compound (1 b) and compound (1 c) are condensed in a solvent using a condensing agent to obtain compound (1a). The solvent used is not limited in particular. Examples of the solvent in this step include halogenated hydrocarbons such as dichloromethane and chloroform; sulfoxides such as dimethylsulfoxide; esters such as ethyl acetate; ethers such as tetrahydrofuran and 1,4-dioxane; amides such as N,N-dimethylformamide, and N,N-dimethylacetamide. Examples of the condensing agent include Bop (1*H*-1,2,3-benzotriazole-1-yloxy(tri(dimethylamino))phosphonium hexafluorophosphate), WSC (1-ethyl-3-(3-dimethylaminopropyl)carbodiimide hydrochloride), DCC (N,N-dicyclohexylcarbodiimide), CDI (carbonyldiimidazole), diethylphosphoryl cyanide or the like. Compound (1 c) can be used in amounts of 1 equivalent to 1.5 equivalents based on compound (1b). The condensing agent can be used in amounts of 1 equivalent to 1.5 equivalents based on compound (1 b). In addition, it is adequate to add from 1 equivalent to excess amount of an organic base, for instance, triethylamine or the like, as necessary. The reaction temperature is from room temperature to 80°C, and the reaction time is from 10 minutes to 30 hours.
Furthermore, compound (1 a) can also be prepared from compound (1b) and compound (1c) by the method described in the other method (1), (2) or (3) below.
Other method (1): Compound (1 a) can be obtained by converting compound (1b) into an acid chloride, then, reacting the acid chloride and compound (1c). The step for obtaining the acid chloride is carried out by reacting from 1 equivalent to excess amount of acid chloride synthesis reagent based on compound (1b), without solvent or in the presence of a solvent such as, dichloromethane, benzene, toluene or the like. Catalytic amounts of N,N-dimethylformamide may be added in the reaction system. Examples of the acid chloride synthesis reagent include, for instance, thionyl chloride, oxalyl chloride, phosphorus trichloride, phosphorus pentachloride or the like. The reaction temperature is from room temperature to reflux temperature, and the reaction time is from 10 minutes to 24 hours.
The step for condensing the acid chloride and compound (1 c) is carried out by reacting the acid chloride and compound (1 c) in a solvent such as, for instance, dichloromethane, tetrahydrofuran, N,N-dimethylformamide or the like, in the presence of 1 equivalent to 3 equivalents of base based on the acid chloride. Examples of the base include, for instance, an organic base such as triethylamine, pyridine and the like or an inorganic base such as potassium carbonate, cesium carbonate or the like. Compound (1 c) is used in the amounts of 1 equivalent to 1.5 equivalents based on the acid chloride. The reaction time is from 10 minutes to 24 hours, and the reaction temperature is from 0°C to reflux temperature.
Other method (2): Compound (1a) can be obtained by converting compound (1b) into mixed acid anhydride, then, reacting the mixed acid anhydride with compound (1c). The step for obtaining the mixed acid anhydride is carried out by reacting compound (1b) and, for instance, chloroformates such as ethyl chloroformate or the like, in the presence of a base such as, for instance, triethylamine or the like. Chloroformates and base are used in the amounts of 1 equivalent to 2 equivalents based on compound (1b). The reaction time is from 10 minutes to 5 hours. The reaction temperature is from 0°C to room temperature.
The step for condensing the mixed acid anhydride and compound (1 c) is carried out by reacting the mixed acid anhydride and compound (1 c) in a solvent such as, for instance, dichloromethane, tetrahydrofuran, N,N-dimethylformamide or the like. Compound (1c) is used in the amounts of 1 equivalent to 1.5 equivalents based on the mixed acid anhydride. The reaction time is from 10 minutes to 24 hours, and the reaction temperature is from 0°C to 50°C.
Other method (3): Compound (1a) can be obtained by converting compound (1b) into an active ester, then, reacting the active ester and compound (1c). The step for obtaining the active ester is carried out by reacting compound (1b) and an active ester synthesis reagent in a solvent such as, for instance, 1,4-dioxane, tetrahydrofuran or the like, in the presence of a condensing agent such as, for instance, DCC or the like. Examples of the active ester synthesis reagent include, for instance, N-hydroxysuccinimide or the like. The active ester synthesis reagent and the condensing agent are used in the amounts of 1 equivalent to 1.5 equivalents based on compound (1b). The reaction temperature is from 0°C to room temperature. The reaction time is from 2 hours to 24 hours.
The step for condensing the active ester and compound (1c) is carried out by reacting the active ester and compound (1c) in a solvent such as, for instance, dichloromethane, tetrahydrofuran, N,N-dimethylformamide or the like. Compound (1c) is used in the amounts of 1 equivalent to 1.5 equivalents based on the active ester. The reaction temperature is from 0°C to 50°C, and the reaction time is from 10 minutes to 24 hours.
Note that, after [Step 1], a substituent on A and E of compound (1a) can be converted using a well known method. Furthermore, a substituent on A of compound (1a) can also be converted using a method described in [Preparation Method 4-1] or [Preparation Method 4-4], and a substituent on E of compound (1a) can also be converted using a method described in [Preparation Method 4-2], [Preparation Method 4-3], [Preparation Method 4-5] or the like.

### [Preparation Method 1-2-1] Preparation method for compound (1 c)

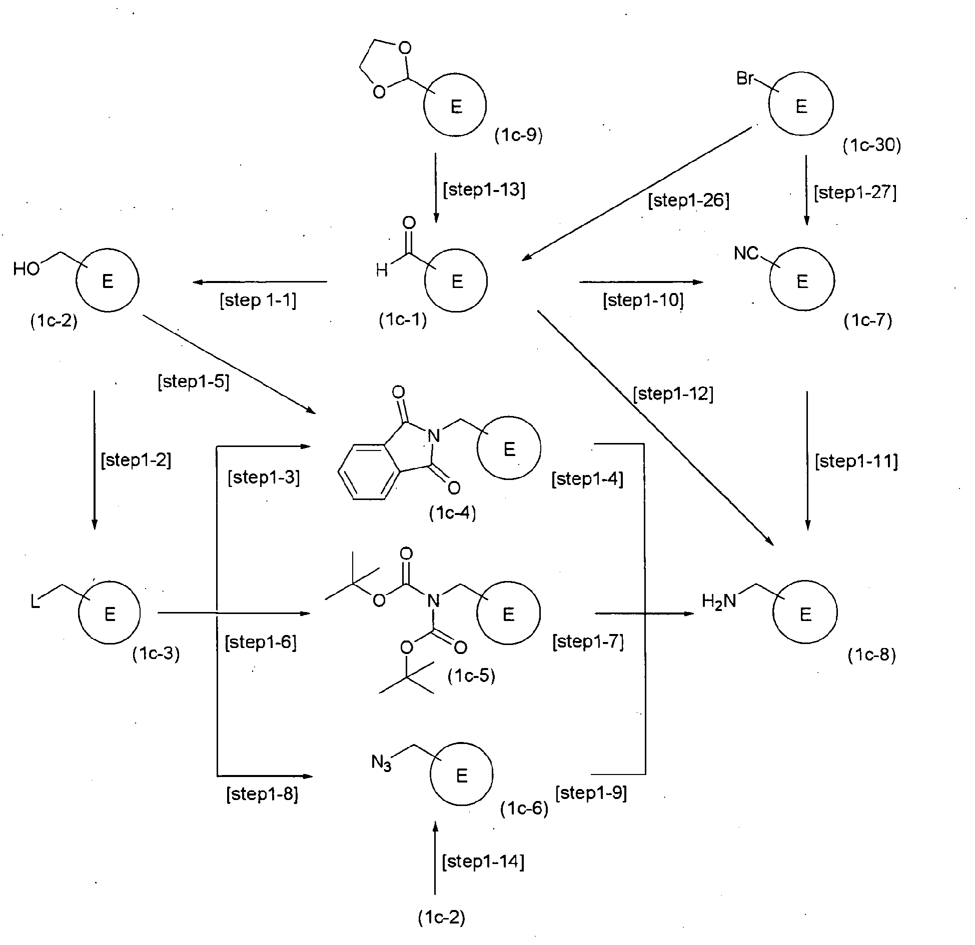

[wherein E has the same meaning as defined above; L represents a leaving group such as a halogen atom, a methanesulfonyloxy group or a *p*-toluenesulfonyloxy group]

For each compound in the above step figure, a commercially available product can be used as is, or it can also be prepared from a commercially available product by a well known method. In addition, it can be prepared using the method described in the preparation examples among the examples and in [Preparation Method 1-2-2] to [Preparation Method 1-2-6]. Further, each compound in the above step figure can also be prepared by converting a substituent on E using the method described in [Preparation Method 4-2] to [Preparation Method 4-5] or the like.

### [Step 1-1]

The present step is a step wherein compound (1c-1) is reduced to obtain compound (1c-2). Examples of the reducing agent include, for instance, sodium borohydride, lithium borohydride, lithium aluminum hydride or the like. Examples of the solvent include alcohols such as, for instance, methanol, ethanol or the like, and ethers such as, for instance, tetrahydrofuran, diethyl ether or the like. The reducing agent is used in the amounts of 1 equivalent to 10 equivalents based on compound (1c-1). The reaction temperature is from 0°C to reflux temperature, and the reaction time is from 5 minutes to 24 hours.

### [Step 1-2]

The present step is a step wherein a hydroxyl group of compound (1c-2) is converted into a leaving group to obtain compound (1 c-3).
When L represents a methanesulfonyloxy group or *p*-toluenesulfonyloxy group, compound (1c-3) can be obtained by reacting compound (1c-2) and methanesulfonyl chloride or *p*-toluenesulfonyl chloride in a solvent such as, for instance, dichloromethane or the like, in the presence of an organic base such as, for instance, triethylamine or the like. The organic base is used in the amounts of 2 equivalents to 6 equivalents based on compound (1c-2). Methanesulfonyl chloride or *p*-toluenesulfonyl chloride is used in the amounts of 1 equivalent to 3 equivalents based on compound (1c-2). The reaction temperature is from 0°C to room temperature, and the reaction time is from 10 minutes to 24 hours.
When L represents a chlorine atom, compound (1c-3) can be obtained by action of a chlorination reagent such as, for instance, thionyl chloride, oxalyl chloride or the like on compound (1c-2). The chlorination reagent is used in the amounts of 1 equivalent to excess amount based on compound (1c-2). The reaction temperature is from 0°C to room temperature, and the reaction time is from 10 minutes to 24 hours.

### [Step 1-3]

The present step is a step wherein compound (1c-3) and phthalimide potassium salt are reacted to obtain compound (1c-4). Compound (1c-4) can be obtained by reacting compound (1c-3) and phthalimide potassium salt in a solvent such as, for instance, N,N-dimethylformamide or the like. Phthalimide is used in the amounts of 1 equivalent to 2 equivalents based on compound (1c-3). The reaction temperature is from room temperature to 160°C. The reaction time is from 10 minutes to 48 hours.

### [Step 1-4]

The present step is a step for obtaining compound (1c-8) from compound (1c-4). Compound (1c-8) can be obtained by adding from 1 equivalent to excess amount of hydrazine hydrate based on compound (1c-4) in a solvent such as, for instance, ethanol or the like. The reaction temperature is from 80°C to reflux temperature, and the reaction time is from 10 minutes to 24 hours.

### [Step 1-5]

The present step is a step wherein compound (1c-2) and phthalimide are reacted to obtain compound (1c-4). Compound (1c-4) can be obtained by reacting compound (1c-2), phthalimide, triphenylphosphine and diethyl azodicarboxylate or diisopropyl azodicarboxylate, in a solvent such as, for instance, dichloromethane, tetrahydrofuran or the like. Phthalimide, triphenylphosphine and diethyl azodicarboxylate or diisopropyl azodicarboxylate are used in the amounts of 1 equivalent to 2 equivalents based on compound (1c-2). The reaction temperature is from -20°C to 80°C, and the reaction time is from 5 minutes to 48 hours.

### [Step 1-6]

The present step is a step wherein compound (1 c-3) and an amine protected with a *tert*-butoxycarbonyl group are reacted to obtain compound (1c-5). Compound (1c-5) can be obtained by reacting compound (1c-3) and the amine protected with the *tert*-butoxycarbonyl group in a solvent such as, for instance, N,N-dimethylformamide or the like, in the presence of a base such as, for instance, sodium hydride or the like. The base is used in the amounts of 1 equivalent to 2 equivalents based on compound (1c-3). The amine protected with the *tert*-butoxycarbonyl group is used in the amounts of 1 equivalent to 2 equivalents based on compound (1c-3). The reaction temperature is from room temperature to 80°C, and the reaction time is from 1 hour to 24 hours.

### [Step 1-7]

The present step is a step wherein the *tert*-butoxycarbonyl group of compound (1c-5) is deprotected to obtain compound (1c-8). Compound (1c-8) can be obtained by deprotecting the *tert*-butoxycarbonyl group of compound (1c-5) without a solvent or in a solvent such as, for instance, dichloromethane or the like, in the presence of an acid such as trifluoroacetic acid or the like in the amounts of 2 equivalents to excess amount based on compound (1c-5). The reaction temperature is from 0°C to 60°C, and the reaction time is from 10 minutes to 24 hours.

### [Step 1-8]

The present step is a step wherein the leaving group of compound (1c-3) is converted into an azide group to obtain compound (1 c-6). Compound (1 c-6) can be obtained by reacting compound (1 c-3) and an azidation reagent such as, for instance, sodium azide, potassium azide or the like in a solvent such as, for instance, N,N-dimethylformamide or the like. The azidation reagent is used in the amounts of 1 equivalent to 5 equivalents based on compound (1c-3). The reaction temperature is from room temperature to 80°C, and the reaction time is from 10 minutes to 48 hours.

### [Step 1-9]

The present step is a step wherein the azide group of compound (1c-6) is reduced to obtain compound (1c-8). Compound (1c-8) can be obtained by carrying out catalytic hydrogenation using Lindlar catalyst in a solvent such as, for instance, ethanol or the like. Lindlar catalyst is used in catalytic amount to excess amount based on compound (1 c-6). The reaction temperature is from room temperature to 80°C, the reaction time is from 30 minutes to 36 hours, and the reaction pressure is from 1 atm to 4 atm.
As other method, compound (1c-8) can be obtained by action of triphenylphosphine in a solvent such as, for instance, dichloromethane, tetrahydrofuran or the like. Triphenylphosphine is used in the amounts of 1.0 equivalent to 2.0 equivalents based on compound (1c-6).

### [Step 1-10]

The present step is a step wherein a formyl group of compound (1c-1) is converted into a cyano group to obtain compound (1c-7). Compound (1c-7) can be obtained by reacting 1 equivalent to 3 equivalent of hydroxyamine hydrochloride based on compound (1c-1) in a solvent such as, for instance, ethanol or the like, to obtain an oxime, then, carrying out dehydration reaction by the action of CDI on the oxime. CDI is used in the amounts of 1 equivalent to 5 equivalents based on the oxime. The reaction temperature is from room temperature to reflux temperature, and the reaction time is from 30 minutes to 24 hours.

### [Step 1-11]

The present step is a step wherein a cyano group of compound (1c-7) is reduced to obtain compound (1c-8). Compound (1c-8) can be obtained either by carrying out a reduction reaction using a reducing agent such as, for instance, lithium aluminum hydride, diisobutylaluminum hydride or the like, or, by carrying out catalytic hydrogenation under hydrogen atmosphere using a catalyst such as, for instance, Raney nickel, palladium-carbon or the like. The solvent used is not limited, however, when carrying out reduction reaction using the reducing agent, ethers such as, for instance, tetrahydrofuran, diethyl ether or the like, hydrocarbons such as, for instance, toluene or the like, are used. When carrying out catalytic hydrogenation, alcohols such as, for instance, methanol, ethanol, propanol or the like are used. The reducing agent is used in the amounts of 1 equivalent to 10 equivalents based on compound (1c-7). The reaction temperature is from room temperature to 80°C, and the reaction time is from 10 minutes to 24 hours. The reaction pressure when carrying out catalytic hydrogenation is from 1 atm to 4 atm.

### [Step 1-12]

The present step is a step for obtaining compound (1c-8) from compound (1c-1). Compound (1c-8) can be obtained by carrying out catalytic hydrogenation using a catalyst such as Raney nickel or the like, in a solvent containing ammonia, under hydrogen atmosphere. Examples of the solvent include, but are not limited to, alcohols such as methanol, ethanol, propanol or the like. The reaction temperature is from room temperature to 80°C, the reaction time is from 1 hour to 36 hour, and the reaction pressure is from 1 atm to 4 atm.

### [Step 1-13]

The present step is a step wherein an acetal group on compound (1 c-9) is deprotected to obtain compound (1c-1). Compound (1c-1) can be obtained by dissolving compound (1c-9) in an organic solvent, and by action of an aqueous solution of acid such as, for instance, hydrochloric acid, sulfuric acid, citric acid or the like. Examples of the solvent include, but are not limited to, for instance, methanol, ethanol, acetonitrile, tetrahydrofuran or the like. The reaction temperature is from room temperature to 60°C, and the reaction time is from 5 minutes to 24 hours.

### [Step 1-14]

The present step is a step wherein an hydroxyl group of compound (1 c-2) is converted into an azide group to obtain compound (1c-6). Compound (1c-6) can be obtained by reacting compound (1c-2) and diphenylphosphoryl azide in a solvent such as, for instance, benzene, toluene or the like, in the presence of an organic base such as, for instance, 1,8-diazabicyclo[5,4,0]undec-7-ene. The organic base is used in the amounts of 1 equivalent to 1.5 equivalents based on compound (1c-2). Diphenylphosphoryl azide is used in the amounts of 1 equivalent to 1.5 equivalents based on compound (1c-2). The reaction temperature is from room temperature to 80°C, and the reaction time is from 10 minutes to 48 hours.

### [Step 1-26]

The present step is a step wherein compound (1c-30) is formylated to obtain compound (1c-1). Compound (1c-1) can be obtained by action of 1 equivalent to 1.5 equivalents of a strong base based on compound (1c-30) for anionization, then, reacting a formylation agent. Examples of the solvent include, for instance, tetrahydrofuran, diethyl ether or the like. Examples of the strong base include, for instance, *n*-butyl lithium or the like. Examples of the formylation agent include, for instance, N,N-dimethylformamide, N-formylmorpholine or the like. The formylation agent is used in the amounts of 1. equivalent to 2 equivalents based on compound (1c-30). The reaction temperature is from -80°C to room temperature, and the reaction time is from 5 minutes to 12 hours.

### [Step 1-27]

The present step is a step wherein compound (1c-30) is cyanated to obtain compound (1c-7). Compound (1c-7) can be obtained by reacting compound (1c-30) and zinc cyanide in a solvent such as, for instance, N,N-dimethylformamide, N-methylpyrrolidinone or the like, under nitrogen atmosphere, in the presence of a catalyst. Examples of the catalyst include, for instance, tetrakis(triphenylphosphine)palladium(0) or the like. Zinc cyanide is used in the amounts of 1 equivalent to 2 equivalents based on compound (1c-30). The catalyst is used in the amounts of 0.01 equivalents to 0.1 equivalents based on compound (1c-30). The reaction temperature is from 50°C to reflux temperature, and the reaction time is from 5 minutes to 24 hours.
Other method: Compound (1c-7) can be obtained by reacting compound (1c-30) and copper cyanide in a solvent such as, for instance, N,N-dimethylformamide and N-methylpyrrolidinone, under nitrogen atmosphere. Copper cyanide is used in the amounts of 1 equivalent to excess amount based on compound (1c-30). The reaction temperature is from 50°C to reflux temperature, and the reaction time is from 10 minutes to 72 hours.

### [Preparation Method 1-2-2] Preparation method for compound (1c-11), which is compound (1c-1)

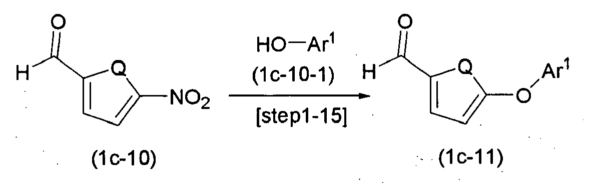

[wherein Q represents an oxygen atom or a sulfur atom; Ar¹ represents a C₆₋₁₀ aryl group that may have 1 or 2 substituents selected from the following substituent group i or an aromatic 5-10 membered heterocyclic group that may have 1 to 3 substituents selected from the following substituent group i.
Substituent group i: a halogen atom, a cyano group, an amino group, a carbamoyl group, a C₁₋₆ alkyl group, a C₃₋₈ cycloalkyl group, a C₁₋₆ alkoxy group, a C₁₋₆ alkylcarbonyl group, a C₁₋₆ alkoxy-carbonyl group, a C₁₋₆ alkylsulfonyl group, a mono-C₆₋₁₀ arylamino group, a trifluoromethyl group and a trifluoromethoxy group]
Compound (1c-10) which is a commercially available product can be used as is. Compound (1c-10-1) which is a commercially available product can be used as is, or it can also be prepared from a commercially available product by a well known method.

### [Step 1-15]

The present step is a step wherein compound (1c-10) and compound (1c-10-1) are reacted to obtain compound (1c-11). Compound (1c-11) can be obtained by reacting compound (1c-10) and compound (1c-10-1) in a solvent such as, for instance, N,N-dimethylformamide, dimethylsulfoxide or the like, in the presence of a base such as, for instance, sodium hydride, potassium carbonate, cesium carbonate or the like. Compound (1c-10-1) is used in the amounts of 1 equivalent to 2 equivalents based on compound (1c-10). The base is used in the amounts of 1 equivalent to 2 equivalents based on compound (1c-10). The reaction temperature is from 0°C to 80°C, and the reaction time is from 5 minutes to 1 hour.

### [Preparation Method 1-2-4] Preparation method for compound (1c-15), which is compound (1c-7)

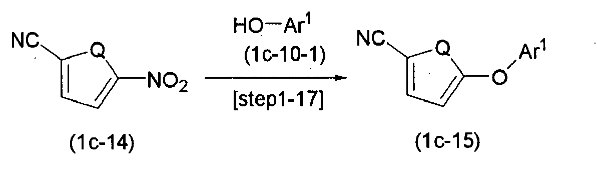

[wherein Q and Ar¹ have the same meanings as defined above.]
Compound (1c-14) which is a commercially available product can be used as is. Compound (1c-10-1) which is a commercially available product can be used as is, or it can also be prepared from a commercially available product by a well known method.

### [Step 1-17]

The present step is a step wherein compound (1c-14) and compound (1c-10-1) are reacted to obtain compound (1c-15). Compound (1c-15) can be obtained by reacting compound (1c-14) and compound (1c-10-1) in a solvent such as, for instance, N,N-dimethylformamide, dimethylsulfoxide or the like, in the presence of a base such as, for instance, sodium hydride, potassium carbonate, cesium carbonate or the like. Compound (1 c-10-1) is used in the amounts of 1 equivalent to 2 equivalents based on compound (1c-14). The base is used in the amounts of 2 equivalent to 3 equivalents based on compound (1 c-14). The reaction temperature is from room temperature to 80°C, and the reaction time is from 1 hour to 72 hours.

### [Preparation Method 1-2-5] Preparation method for compound (1c-17), compound (1c-19), compound (1c-21), compound (1c-23) and compound (1c-25), which are compound (1c-7)

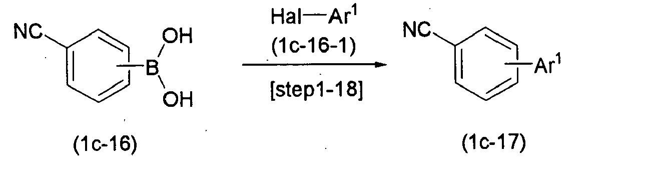

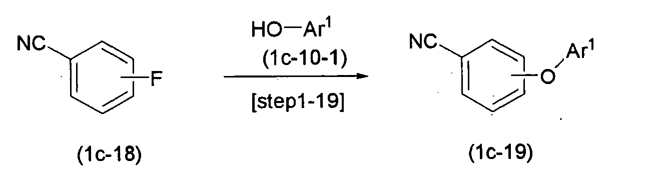

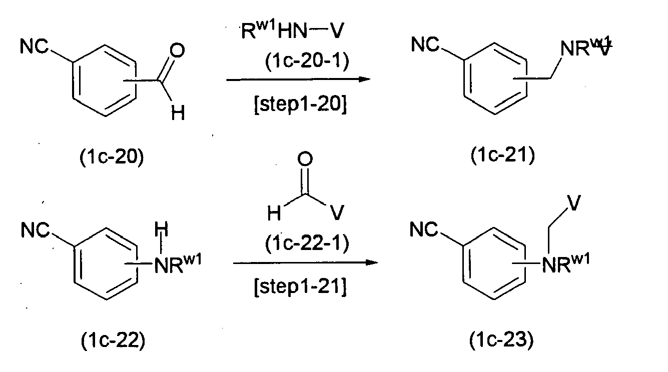

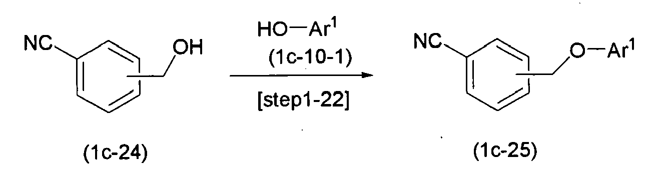

[wherein Ar¹ has the same meaning as defined above; V represent a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group or a 5 to 10-membered heterocyclic C₁₋₆ alkyl group; R^{w1} represents a hydrogen atom or a C₁₋₆ alkyl group; Hal represents a chlorine atom, a bromine atom or an iodine atom.]
Compound (1c-16), compound (1c-18), compound (1c-20), compound (1c-22), compound (1c-24), compound (1c-16-1), compound (1c-10-1), compound (1c-20-1) and compound (1 c-22-1) which are commercially available products can be used as is, or they can also be prepared from commercially available products by a well known method.

### [Step 1-18]

The present step is a step wherein compound (1c-16) and compound (1c-16-1) are subjected to a coupling reaction to obtain compound (1c-17). Compound (1c-17) can be obtained by reacting compound (1c-16) and compound (1c-16-1) in a solvent such as, for instance, tetrahydrofuran, 1,4-dioxane, methanol, ethanol, toluene, N,N-dimethylformamide or the like, in the presence of a base such as, for instance, potassium carbonate, cesium carbonate, potassium phosphate or the like, and a catalyst such as, for instance, palladium(II) acetate, tetrakis(triphenylphosphine)palladium(0), dichlorobis(triphenylphosphine)palladium(II), tris(dibenzylideneacetone)dipalladium(0), dichloro(1,1'-bis(diphenylphosphino)ferrocene)nickel(II) or the like. The base is used in the amounts of 1.5 equivalents to excess amount based on compound (1c-16). The catalyst is used in the amounts of 0.05 equivalents to 0.3 equivalents based on compound (1c-16).

### [Step 1-19]

The present step is a step wherein compound (1c-18) and compound (1c-10-1) are reacted to obtain compound (1c-19). Compound (1c-19) can be obtained by reacting compound (1 c-18) and compound (1c-10-1) in a solvent such as dimethylsulfoxide or the like, in the presence of a base such as potassium *tert*-butoxide or the like. The base is used in the amounts of 1 equivalent to excess amount based on compound (1c-18). The reaction temperature is from 80°C to 220°C, and the reaction time is from 30 minutes to 48 hours.

### [Step 1-20]

The present step is a step wherein compound (1c-20) and compound (1c-20-1) are reacted to carry out reductive amination and to obtain compound (1c-21). Compound (1c-21) can be obtained by reacting compound (1c-20) and compound (1c-20-1) in a solvent such as, for instance, tetrahydrofuran, methanol, ethanol or the like, in the presence of a reducing agent such as, for instance, lithium aluminum hydride, sodium borohydride, sodium cyanoborohydride, triacetoxy sodium borohydride or the like, and acetic acid. The reducing agent is used in the amounts of 1 equivalent to 2 equivalents based on compound (1c-20). The reaction temperature is from room temperature to 60°C, and the reaction time is from 10 minutes to 24 hours.

### [Step 1-21]

The present step is a step wherein compound (1c-22) and compound (1c-22-1) are reacted to carry out reductive amination and to obtain compound (1c-23). Compound (1c-23) can be prepared by a method similar to [Step 1-20].

### [Step 1-22]

The present step is a step wherein compound (1c-24) and compound (1c-10-1) are reacted to obtain compound (1c-25). Compound (1c-25) can be obtained by reacting compound (1 c-24) and compound (1c-10-1) in a solvent such as, for instance, dimethylsulfoxide or the like, and in the presence of a base such as, for instance, potassium carbonate, cesium carbonate or the like. The base is used in the amounts of 1 equivalent to 3 equivalents based on compound (1c-24). The reaction temperature is from room temperature to 80°C, and the reaction time is from 10 minutes to 24 hours.

### [Preparation Method 1-2-6] Preparation method for compound (1c-27), which is compound (1 c-9), and compound (1 c-29), which is compound (1c-1)

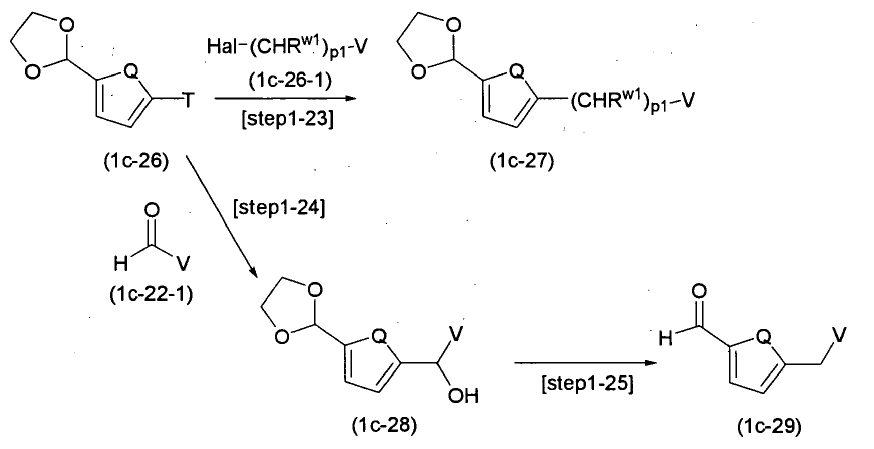

[wherein R^{w1}, V, Q and Hal have the same meanings as defined above; p1 is an integer of 1 or 2; T represents a hydrogen atom when Q represents an oxygen atom, and a bromine atom when Q represents a sulfur atom.]
Compound (1 c-26) which is a commercially available product can be used as is. Compound (1c-26-1) and compound (1c-22-1) which are commercially available products can be used as is, or they can also be prepared from commercially available products by a well known method.

### [Step 1-23]

The present step is a step wherein compound (1c-26) and compound (1c-26-1) are reacted to obtain compound (1c-27). Compound (1c-27) can be obtained by action of 1 equivalent of a strong base based on compound (1c-26) for anionization, then, reacting with compound (1c-26-1). Examples of the solvent include, for instance, tetrahydrofuran, diethyl ether or the like.. Examples of the strong base include, for instance, *n*-butyl lithium or the like. The reaction temperature is from -80°C to room temperature.

### [Step 1-24] .

The present step is a step wherein compound (1c-26) and compound (1c-22-1) are reacted to obtain compound (1c-28). Compound (1c-28) can be obtained by action of 1 equivalent of a strong base based on compound (1c-26) for anionization, then, reacting with compound (1c-22-1). Examples of the solvent include, for instance, tetrahydrofuran, diethyl ether or the like. Examples of the strong base include, for instance, *n*-butyl lithium or the like. In addition, 0.1 equivalent to 1 equivalent of copper(l) iodide or copper(l) bromide based on compound (1c-26) may be added in the reaction system. The reaction temperature is from -80°C to room temperature.

### [Step 1-25]

The present step is a step wherein elimination of a hydroxyl group and deprotection of an acetal group of compound (1 c-28) are carried out simultaneously to obtain compound (1c-29). When compound (1c-28) is treated with trimethylsilyliodide, elimination of the hydroxyl group and deprotection of the acetal group occur simultaneously, to give compound (1c-29). Trimethylsilyliodide is used in the amounts of 2 equivalents to 6 equivalents based on compound (1c-28). In addition, trimethylsilyliodide may be prepared from trimethylsilyl chloride and sodium iodide in the reaction solution and used as is. Examples of the solvent include, for instance, acetonitrile or the like. The reaction temperature is from 0°C to 60°C, and the reaction time is from 5 minutes to 6 hours.
Other method: Compound (1c-29) can be obtained by converting the hydroxyl group of compound (1 c-28) into the acetyl group, then, eliminating the acetyl group, and carrying out deprotection of an acetal group. Conversion of the hydroxyl group into the acetyl group can be carried out by using an acetylating reagent such as, for instance, acetic anhydride, acetyl chloride or the like. A base such as, for instance, N,N-dimethylaminopyridine, triethylamine, pyridine or the like, in the amounts of 1 equivalent to excess amount based on compound (1c-28) may be added in the reaction system. Examples of the solvent include, for instance, dichloromethane, ethyl acetate, N,N-dimethylformamide or the like. In addition, pyridine, which is added as a base, may be used directly as a solvent. An acetylating reagent is used in the amounts of 1 equivalent to excess amount based on compound (1c-28). The reaction temperature is from 0°C to 60°C, and the reaction time is from 1 hour to 36 hours. Elimination of the acetyl group can be carried out, for instance, under hydrogen atmosphere, in a solvent such as, for instance, ethanol, methanol or the like, and using a catalyst such as for instance, palladium-carbon, Raney nickel or the like. The reaction temperature is from room temperature to 80°C, and the reaction time is from 5 hours to 36 hours. Deprotection of the acetal group can be carried out by an analogous method to [Step 1-13].

### [Preparation Method 4-1] Conversion of substituent on A in compound (I-a)-1

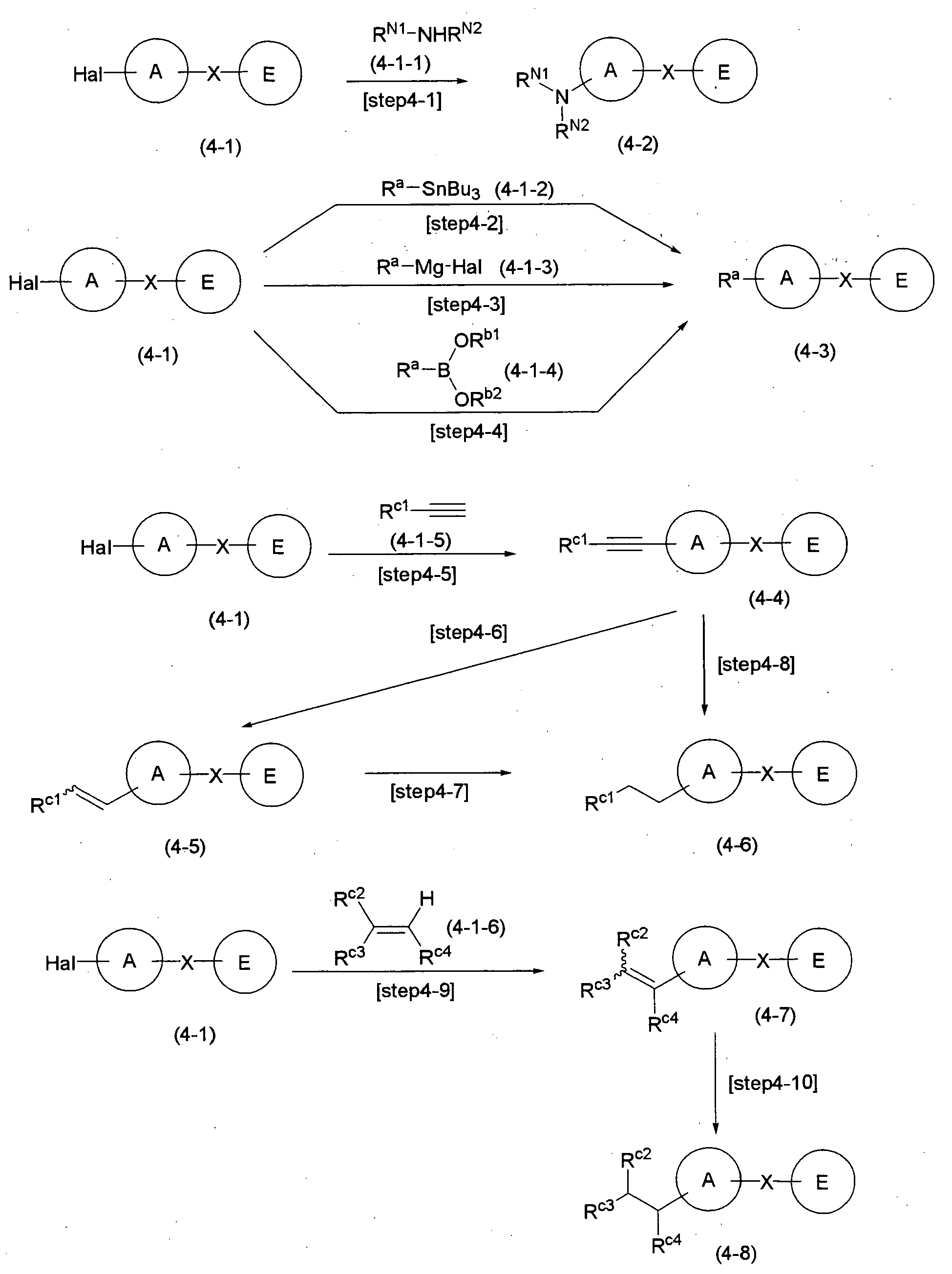

[wherein A¹, E and Hal have the same meanings as defined above; X represents a group represented by the formula -C(=O)-NH-CH₂-; R^{N1} represents a C₁₋₆ alkyl group, a C₂₋₆ alkenyl group or a C₂₋₆ alkynyl group; R^{N2} represents a hydrogen atom or a C₁₋₆ alkyl group; R^{a} represents a C₂₋₆ alkenyl group, a C₆₋₁₀ aryl group or an aromatic 5 to 10-membered heterocyclic group; R^{b1} and R^{b2} are the same or different form each other, and represent a hydrogen atom or a C₁₋₆ alkyl group, or together form a cyclic boric acid ester; R^{c1}, R^{c2}, R^{c3} and R^{c4} are the same or different form each other, and represent a hydrogen atom, a C₁₋₆ alkyl group, a C₂₋₆ alkenyl group, a C₂₋₆ alkynyl group, a C₃₋₈ cycloalkyl group, a C₆₋₁₀ aryl group or a 5 to 10-membered heterocyclic group.]
Compound (4-1-1), compound (4-1-2), compound (4-1-3), compound (4-1-4), compound (4-1-5) and compound (4-1-6) which are commercially available products can be used as is, or they can also be prepared from commercially available products by a well known method.

### [Step 4-1]

The present step is a step wherein compound (4-1) and compound (4-1-1) are reacted to obtain compound (4-2). Examples of the solvent include, for instance, dimethylsulfoxide, tetrahydrofuran, toluene, acetonitrile, N,N-dimethylformamide or the like. In addition, the reaction can also be carried out without the solvent. The reaction is preferably carried out in a sealed tube; the reaction time is from 1 hour to 60 hours, and the reaction temperature is from 50°C to 200°C. Note that, an organic base such as, for instance, N,N-diisopropylethylamine, triethylamine, pyridine, 1,8-diazabicyclo[5,4,0]undec-7-ene, inorganic base such as, for instance, potassium carbonate, sodium carbonate, can be added in the amounts of 2 equivalents to excess amount based on compound (4-1).

### [Step 4-2]

The present step is a step wherein compound (4-1) and compound (4-1-2) are reacted to obtain compound (4-3). Compound (4-3) can be obtained by reacting compound (4-1) and compound (4-1-2) in the presence of a catalyst. Examples of the catalyst include tetrakis(triphenylphosphine)palladium(0), dichlorobis(triphenylphosphine)palladium(II) or the like. Examples of the solvent include, toluene, 1,4-dioxane, xylene or the like. Compound (4-1-2) is used in the amounts of 2 equivalent to 3 equivalents based on compound (4-1). The catalyst is used in the amounts of 0.05 equivalents to 0.3 equivalents based on compound (4-1).
The reaction temperature is from 100°C to 140°C, and the reaction time is from 1 hour to 24 hours.

### [Step 4-3]

The present step is a step wherein compound (4-1) and compound (4-1-3) are reacted to obtain compound (4-3). Compound (4-3) can be obtained by reacting compound (4-1) and compound (4-1-3) in the presence of a catalyst. Examples of the catalyst include, for instance, dichloro(1,1'-bis(diphenylphosphino)propane)nickel(II), dichloro(1,1'-bis(diphenylphosphino)ferrocene)nickel(II), tetrakis(triphenylphosphine)palladium(0) or the like. Examples of the solvent include, for instance, tetrahydrofuran, 1,4-dioxane or the like. Compound (4-1-3) is used in the amounts of 3 equivalents to excess amount based on compound (4-1). The catalyst is used in the amounts of 0.05 equivalents to 0.3 equivalents based on compound (4-1). The reaction temperature is from 0°C to reflux temperature, and the reaction time is from 5 minutes to 24 hours.

### [Step 4-4]

The present step is a step wherein compound (4-1) and compound (4-1-4) are reacted to obtain compound (4-3). Compound (4-3) can be obtained by reacting compound (4-1) and compound (4-1-4) in the presence of a catalyst and a base. Examples of the catalyst include, for instance, palladium(II) acetate, tetrakis(triphenylphosphine)palladium(0), dichlorobis(triphenylphosphine)palladium(II), tris(dibenzylideneacetone) dipalladium(0) or the like. To obtain satisfactory results, a phosphorus ligand (for instance, triphenylphosphine, tri-*tert*-butylphosphine or the like) may be added in the amounts of 0.25 equivalents to 1.5 equivalents based on compound (4-1). Examples of the basic include, for instance, potassium carbonate, sodium carbonate, cesium carbonate, potassium fluoride, cesium fluoride, potassium phosphate, sodium hydroxide, barium hydroxide, potassium hydroxide or the like. The present reaction is preferably carried out under an inert gas atmosphere such as, for instance, nitrogen gas and argon gas. Examples of the solvent include, for instance, tetrahydrofuran, 1,4-dioxane, methanol, ethanol, toluene, water or the like. Depending on the reagent used, quaternary ammonium salt such as tetrabutylammonium bromide can be added. The catalyst is used in the amounts of 0.05 equivalents to 0.3 equivalents based on compound (4-1). The base is used in the amounts of 2 equivalents to excess amount based on compound (4-1). The reaction temperature is from room temperature to reflux temperature, and the reaction time is from 30 minutes to 24 hours.

### [Step 4-5]

The present step is a step wherein compound (4-1) and compound (4-1-5) are reacted to obtain compound (4-4). Compound (4-4) can be obtained by reacting compound (4-1) and compound (4-1-5) in the presence of a catalyst and a base. Examples of the catalyst include, for instance, palladium(II) acetate, tetrakis(triphenylphosphine)palladium(0), dichlorobis(triphenylphosphine)palladium(II), tris(dibenzylideneacetone) dipalladium (0) or the like. Examples of the base include, for instance, triethylamine, N,N-diisopropylethylamine, pyridine or the like. Examples of the solvent include, for instance, tetrahydrofuran, acetonitrile, 1,4-dioxane, N,N-dimethylformamide, N-methylpyrrolidinone, dimethylsulfoxide, toluene or the like. In addition, to obtain satisfactory results, 0.1 equivalents to 0.3 equivalents of copper (I) iodide or tetrabutylammonium fluoride may be added based on compound (4-1). Compound (4-1-5) is used in the amounts of 1 equivalent to 5 equivalents based on compound (4-1). The catalyst is used in the amounts of 0.05 equivalents to 0.3 equivalents based on compound (4-1). The base is used in the amounts of 2 equivalents to 5 equivalents based on compound (4-1). The reaction temperature is from room temperature to 150°C, and the reaction time is from 30 minutes to 24 hours.

### [Step 4-6]

The present step is a step wherein a triple bond in compound (4-4) is reduced into a double bond to obtain compound (4-5). Compound (4-5) can be obtained using a catalyst such as, for instance, Lindlar catalyst, palladium-barium sulfate or the like, in a solvent such as, for instance, tetrahydrofuran, ethyl acetate, acetonitrile, methanol, ethanol or the like, under hydrogen atmosphere. The preferable solvent is ethyl acetate. To obtain satisfactory results, 0.1 equivalents to 1 equivalents of quinoline may be added based on compound (4-4). The catalyst is used in catalytic amount to excess amount based on compound (4-4). The reaction temperature is room temperature, the reaction time is from 15 minutes to 24 hours, and reaction pressure is from 1 atm to 4 atm.

### [Step 4-7]

The present step is a step wherein compound (4-5) is reduced to obtain compound (4-6). Compound (4-6) can be obtained using a catalyst such as, for instance, palladium-carbon, Raney nickel, platinum dioxide or the like, in a solvent such as, for instance, tetrahydrofuran, ethyl acetate, acetonitrile, methanol, ethanol or the like, under hydrogen atmosphere. The catalyst is used in catalytic amount to excess amount based on compound (4-5). The reaction temperature is room temperature, the reaction time is from 5 minutes to 24 hours, and the reaction pressure is from 1 atm to 4 atm.

### [Step 4-8]

The present step is a step wherein compound (4-4) is reduced to obtain compound (4-6). Compound (4-6) can be prepared by an analogous method to [Step 4-7].

### [Step 4-9]

The present step is a step wherein compound (4-1) and compound (4-1-6) are reacted to obtain compound (4-7). Compound (4-7) can be obtained by reacting compound (4-1) and compound (4-1-6) in the presence of a catalyst and a base. Examples of the catalyst include, for instance, palladium(II) acetate , tetrakis(triphenylphosphine)palladium(0), dichlorobis(triphenylphosphine)palladium(II), tris(dibenzylideneacetone)dipalladium (0) or the like. Examples of the base include, for instance, triethylamine, N,N-diisopropylethylamine, N,N-dicyclohexylmethylamine or the like. Examples of the solvent include, for instance, acetonitrile, tetrahydrofuran, 1,4-dioxane, benzene, toluene, xylene, N,N-dimethylformamide, N-methylpyrrolidinone or the like. In addition, to obtain satisfactory results, 0.25 equivalents to 1.5 equivalents of a phosphorus ligand (for instance, triphenylphosphine, tri-*tert*-butylphosphine, 2-(di-*tert*-butylphosphino)biphenyl or the like) may be added based on compound (4-1). Compound (4-1-6) is used in the amounts of 1 equivalent to 4 equivalents based on compound (4-1). The catalyst is used in the amounts of 0.05 equivalents to 0.3 equivalents based on compound (4-1). The base is used in the amounts of 2 equivalents to 5 equivalents based on compound (4-1). The reaction temperature is from room temperature to 150°C, the reaction time is from 5 minutes to 24 hours.

### [Step 4-10]

The present step is a step wherein compound (4-7) is reduced to obtain compound (4-8). Compound (4-8) can be prepared by an analogous method to [Step 4-7].

### [Preparation Method 4-2] Conversion of substituent on E in compound (I-a) -1

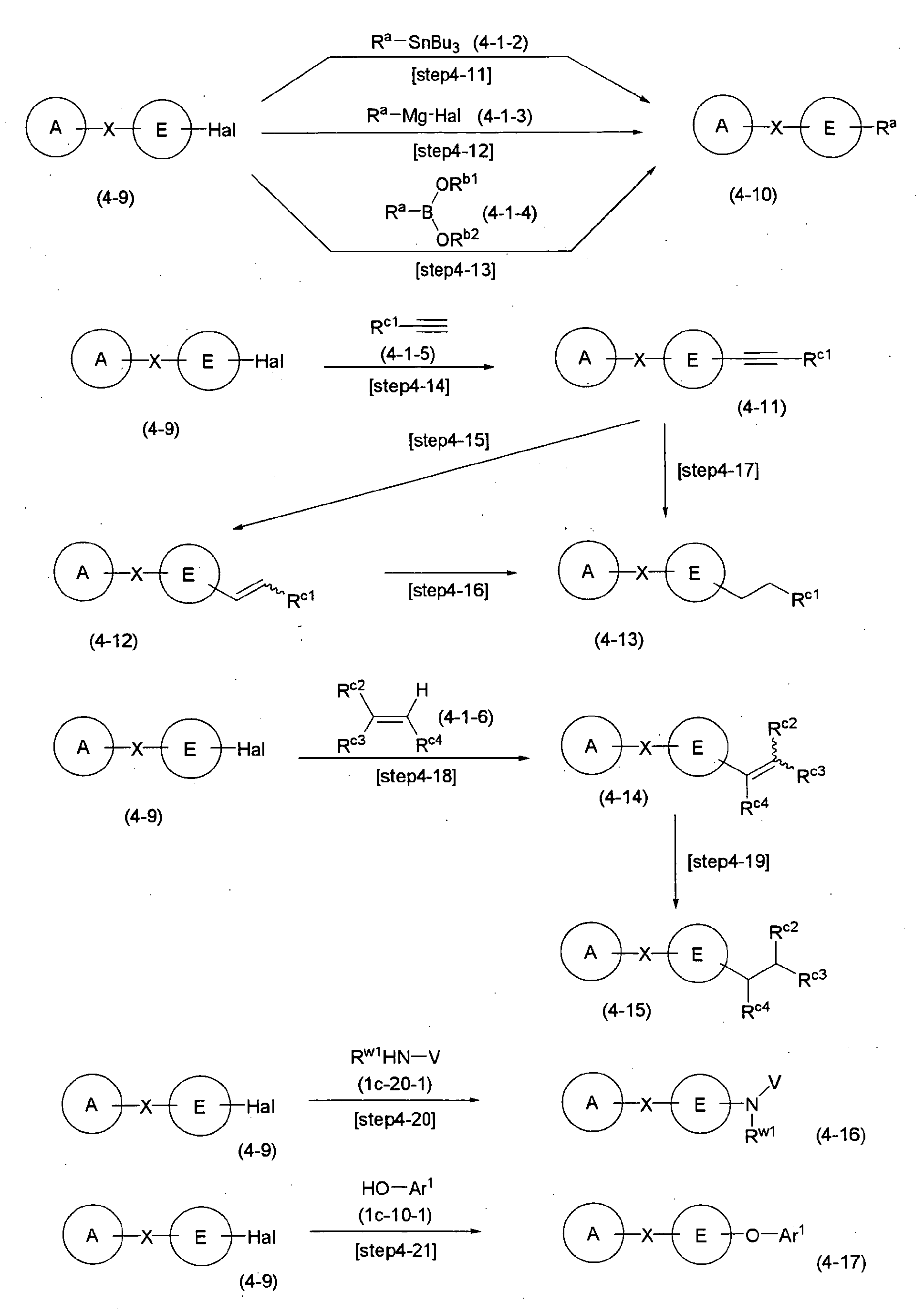

[wherein A, X E, Hal, R^{a}, R^{b1}, R^{b2}, R^{c1}, R^{c2}, R^{c3}, R^{c4}, R^{w1}, V and Ar¹ have the same meanings as defined above.]
Compound (4-1-2), compound (4-1-3), compound (4-1-4), compound (4-1-5), compound (4-1-6), compound (1c-20-1) and compound (1c-10-1) which are commercially available products can be used as is, or they can also be prepared from commercially available products by a well known method.

### [Step 4-11]

The present step is a step wherein compound (4-9) and compound (4-1-2) are reacted to obtain compound (4-10). Compound (4-10) can be prepared by an analogous method to [Step 4-2].

### [Step 4-12]

The present step is a step wherein compound (4-9) and compound (4-1-3) are reacted to obtain compound (4-10). Compound (4-10) can be prepared by an analogous method to [Step 4-3].

### [Step 4-13]

The present step is a step wherein compound (4-9) and compound (4-1-4) are reacted to obtain compound (4-10). Compound (4-10) can be prepared by an analogous method to [Step 4-4].

### [Step 4-14]

The present step is a step wherein compound (4-9) and compound (4-1-5) are reacted to obtain compound (4-11). Compound (4-11) can be prepared by an analogous method to [Step 4-5].

### [Step 4-15]

The present step is a step wherein a triple bond of compound (4-11) is reduced into a double bond to obtain compound (4-12). Compound (4-12) can be prepared by an analogous method to [Step 4-6].

### [Step 4-16]

The present step is a step wherein compound (4-12) is reduced to obtain compound (4-13). Compound (4-13) can be prepared by an analogous method to [Step 4-7].

### [Step 4-17]

The present step is a step wherein compound (4-11) is reduced to obtain compound (4-13). Compound (4-13) can be prepared by an analogous method to [Step 4-8].

### [Step 4-18]

The present step is a step wherein compound (4-9) and compound (4-1-6) are reacted to obtain compound (4-14). Compound (4-14) can be prepared by an analogous method to [Step 4-9].

### [Step 4-19]

The present step is a step wherein compound (4-14) is reduced to obtain compound (4-15). Compound (4-15) can be prepared by an analogous method to [Step 4-10].

### [Step 4-20]

The present step is a step wherein compound (4-9) and compound (1c-20-1) are reacted to obtain compound (4-16). Compound (4-16) can be obtained by reacting compound (4-9) and compound (1c-20-1) in a solvent such as, for instance, tetrahydrofuran, benzene, toluene, xylene or the like, in the presence of a catalyst such as for instance, tris(dibenzylideneacetone)dipalladium(0), dichloro(1,1'-bis(diphenylphosphino)ferrocene)palladium(II), palladium(II) acetate, a phosphorus ligand such as, for instance, 2,2-bis(diphenylphosphino)-1,1'-binaphthyl, and a base such as, for instance, sodium *tert*-butoxide. Compound (1c-20-1) is used in the amounts of 1 equivalent to 3 equivalents based on compound (4-9). Catalyst is used in the amounts of 0.05 equivalents to 0.3 equivalents based on compound (4-9). The base is used in the amounts of 1.5 equivalent to excess amount based on compound (4-9). The phosphorus ligand is used in the amounts of 0.25 equivalents to 1.5 equivalents based on compound (4-9). The reaction temperature is 50°C to reflux temperature, and the reaction time is from 1 hour to 48 hours.

### [Step 4-21]

The present step is a step wherein compound (4-9) and compound (1c-10-1) are reacted to obtain compound (4-17). Compound (4-17) can be obtained by reacting compound (4-9) and compound (1c-10-1) in a solvent such as, for instance, tetrahydrofuran, toluene or the like, in the presence of a catalyst such as, for instance, copper(I) chloride, copper(I) iodide or the like, and a base such as, for instance, potassium carbonate, cesium carbonate, potassium phosphate, pyridine or the like. Compound (1c-10-1) is used in the amounts of 1 equivalent to 3 equivalents based on compound (4-9). Catalyst is used in the amounts of 0.5 equivalents to 3 equivalents based on compound (4-9). The base is used in the amounts of 2 equivalents to 10 equivalents based on compound (4-9). The reaction temperature is 50°C to reflux temperature, and the reaction time is from 1 hour to 48 hours.
Other method for [Step 4-20] and [Step 4-21]
When E represent a furyl group or a thienyl group, compound (4-16) or compound (4-17) can be obtained respectively by reacting compound (4-9) and compound (1c-20-1) or compound (1c-10-1) in a solvent such as, for instance, dimethylsulfoxide, N-methylpyrrolidone or the like, in the presence of a catalyst such as, for instance, copper(I) chloride or the like, a base such as, for instance, cesium carbonate and 2,2,6,6-tetramethyl-3,5-heptanedione. Compound (1c-20-1) or compound (1c-10-1) is used in the amounts of 1 equivalent to 3 equivalents based on compound (4-9). The catalyst is used in the amounts of 0.5 equivalents to 3 equivalents based on compound (4-9). The base is used in the amounts of 2 equivalents to 10 equivalents based on compound (4-9). The reaction temperature is 80°C to reflux temperature, and the reaction time is from 1 hour to 24 hours.

### [Preparation Method 4-3] Conversion of substituent on E in compound (I-a) -2

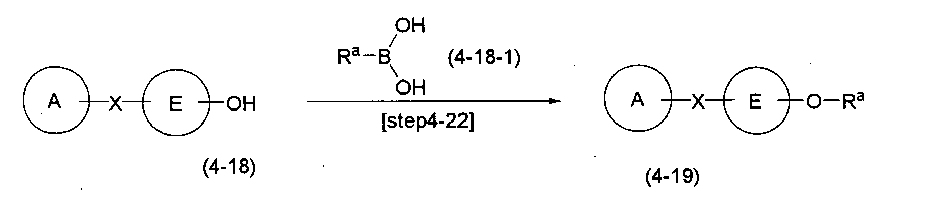

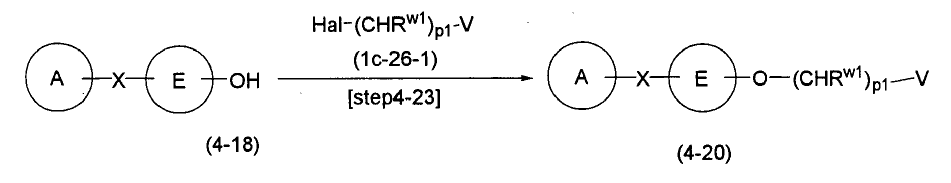

[wherein A, X, E, R^{a}, Hal, R^{W1}, p1 and V have the same meanings as defined above.]
Compound (4-18-1) and compound (1c-26-1) which are commercially available products can be used as is, or they can also be prepared from commercially available products by a well known method.

### [Step 4-22]

The present step is a step wherein compound (4-18) and compound (4-18-1) are reacted to obtain compound (4-19). Compound (4-19) can be obtained by reacting compound (4-18) and compound (4-18-1) in the presence of a catalyst and a base. Examples of the catalyst include a cuprous catalyst such as, for instance, copper (II) acetate. Examples of the base include, for instance, triethylamine, N,N-diisopropylethylamine or the like. Examples of the solvent include, for instance, dichloromethane, tetrahydrofuran, toluene or the like. It is preferable to use dichloromethane. The present reaction is preferably carried out in the presence of oxygen. To obtain satisfactory results, molecular sieves 4A may be added. Compound (4-18-1) is used in the amounts of 1 equivalent to 4 equivalents based on compound (4-18). The catalyst is used in the amounts of 0.1 equivalents to 0.3 equivalents based on compound (4-18). The base is used in the amounts of 2 equivalents to excess amount based on compound (4-18). The reaction temperature is from room temperature to 50°C, the reaction time is from 24 hours to 5 days.

### [Step 4-23]

The present step is a step wherein compound (4-18) and compound (1c-26-1) are reacted to obtain compound (4-20). Compound (4-20) can be obtained by reacting compound (4-18) and compound (1c-26-1) in a solvent such as, for instance, N,N-dimethylformamide, N-methylpyrrolidinone, tetrahydrofuran or the like, in the presence of a base such as, for instance, potassium carbonate, cesium carbonate, sodium hydride or the like. To obtain satisfactory results, a catalytic amount of sodium iodide or potassium iodide may be added. The reaction temperature is from room temperature to 160°C, and the reaction time is from 10 minutes to 48 hours.
Another method is based on the technique that uses the Mitsunobu reaction. Compound (4-20) can be obtained by reacting compound (4-18), compound (1c-26-1), triphenylphosphine and diethyl azodicarboxylate or diisopropyl azodicarboxylate in a solvent such as, for instance, dichloromethane, tetrahydrofuran or the like. Compound (1c-26-1) is used in the amounts of 1 equivalent to 1.5 equivalents based on compound (4-18). Triphenylphosphine is used in the amounts of 1 equivalent to 1.5 equivalents based on compound (4-18). Diethyl azodicarboxylate or diisopropyl azodicarboxylate is used in the amounts of 1 equivalent to 1.5 equivalents based on compound (4-18). The reaction temperature is from room temperature to reflux temperature, and the reaction time is from 5 minutes to 24 hours.

### [Preparation Method 4-4] Conversion of substituent on A in compound (I-a) - 2

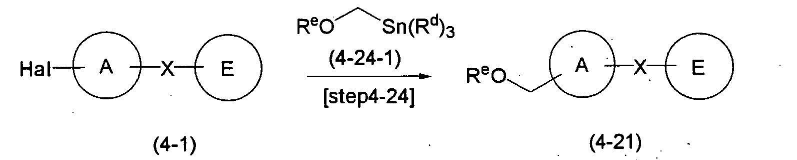

[wherein A, X, E and Hal have the same meanings as defined above; R^{d} and R^{e} are the same or different from each other, and represent C₁₋₆ alkyl groups.]
Compound (4-24-1) which is commercially available products can be used as is, or compound (4-24-1) can also be prepared from commercially available products by a well known method.

### [Step 4-24]

The present step is a step wherein compound (4-1) and compound (4-24-1) are reacted to obtain compound (4-21). The present reaction is preferably carried out under an inert gas atmosphere; the solvent for use varies depending on the starting materials and reagents used; for instance, N-methylpyrrolidinone, 1,4-dioxane or the like can be used. Examples of the catalyst include, for instance, palladium(II) acetate, tetrakis(triphenylphosphine)palladium(0), dichlorobis(triphenylphosphine)palladium(II) or tris(dibenzylideneacetone)dipalladium(0) or the like. To obtain satisfactory results, of phosphorus ligand, preferably, for instance, triphenylphosphine, tri-*tert*-butylphosphine, diphenylphosphino ferrocene or the like may be added. Compound (4-24-1) is used in the amounts of 1 equivalent to 10 equivalents based on compound (4-1). The catalyst is used in the amounts of 0.001 equivalents to 0.2 equivalents based on compound (4-1). The phosphorus ligand is used in the amounts of 0.001 equivalents to 0.4 equivalents based on compound (4-1). The reaction temperature is from room temperature to reflux temperature, and the reaction time is from 10 minutes to 24 hours.

### [Preparation Method 4-5] Conversion of substituent on E in compound (I-a) -3

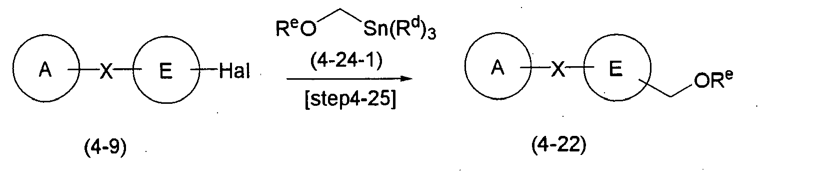

[wherein A, X, E, Hal, R^{d} and R^{e} have the same meanings as defined above.]
Compound (4-24-1) which is commercially available products can be used as is, or compound (4-24-1) can also be prepared from commercially available products by a well known method.

### [Step 4-25]

The present step is a step wherein compound (4-9) and compound (4-24-1) are reacted to obtain compound (4-22). Compound (4-22) can be prepared by an analogous method to [Step 4-24].

### (Preparation method for compound (4-24-1))

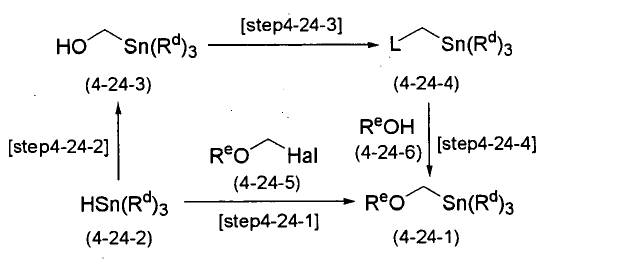

[wherein L, R^{d}, R^{e} and Hal have the same meanings as defined above.]
Compound (4-24-2), compound (4-24-5) and compound (4-24-6) which are commercially available products can be used as is, or they can also be prepared from commercially available products by a well known method.

### [Step 4-24-1]

The present step is a step wherein compound (4-24-2) and compound (4-24-5) are reacted to obtain compound (4-24-1). Compound (4-24-1) can be obtained in a solvent such as, for instance, tetrahydrofuran, by abstracting a hydrogen atom from compound (4-24-2) with a strong base such as for instance, lithium diisopropyl amide, and then reacting with compound (4-24-5). Examples of compound (4-24-5) include, for instance, chloromethyl ethyl ether, chloromethyl benzyl ether or the like. The strong base is used in the amounts of 1 equivalent to 2 equivalents based on compound (4-24-2). Compound (4-24-5) is used in the amounts of 1 equivalent to 2 equivalents based on compound (4-24-2). The reaction temperature is from -78°C to reflux temperature, and the reaction time is from 1 hour to 24 hours.

### [Step 4-24-2]

The present step is a step wherein compound (4-24-2) and a formaldehyde equivalent are reacted to obtain compound (4-24-3). Compound (4-24-3) can be obtained in a solvent such as, for instance, tetrahydrofuran, by abstracting an hydrogen atom from compound (4-24-2) with a base such as, for instance, lithium diisopropyl amide, and then reacting with paraformaldehyde. The strong base is used in the amounts of 1 equivalent to 2 equivalents based on compound (4-24-2). The formaldehyde equivalent is used in the amounts of 1 equivalent to 2 equivalents based on compound (4-24-2). The reaction temperature is -78°C to reflux temperature, and the reaction time is from 1 hour to 24 hours.

### [Step 4-24-3]

The present step is a step wherein a hydroxyl group of compound (4-24-3) is converted into a leaving group to obtain compound (4-24-4).
When L represents a methane sulfonyloxy group, a *p*-toluenesulfonyloxy group, or the like, compound (4-24-4) can be obtained in a solvent such as, for instance, dichloromethane, by reacting compound (4-24-3) and a sulfonyl halide such as methane sulfonyl chloride or *p*-toluenesulfonyl chloride, in the presence of an organic base such as, for instance, triethylamine. The organic base is used in the amounts of 1 equivalent to 3 equivalents based on compound (4-24-3). The sulfonyl halide is used in the amounts of 1 equivalent to 2 equivalents based on compound (4-24-3). The reaction temperature is from 0°C to reflux temperature, and the reaction time is from 10 minutes to 24 hours.
When L represents a bromine atom or an iodine atom, compound (4-24-4) can be obtained in a solvent such as, for instance, dichloromethane, by action of a halogenation agent such as, for instance, carbon tetrabromide, N-bromosuccinimide or N-iodosuccinimide on compound (4-24-3), in the presence of triphenylphosphine. Triphenylphosphine is used in the amounts of 1 equivalent to 2 equivalents with based on compound (4-24-3). The halogenation agent is used in the amounts of 1 equivalent to 2 equivalents with respect to compound (4-24-3). The reaction temperature is from 0°C to room temperature, and the reaction time is from 10 minutes to 24 hours.

### [Step 4-24-4]

The present step is a process wherein compound (4-24-4) and compound (4-24-6) are reacted to obtain compound (4-24-1). Compound (4-24-1) can be obtained in a solvent such as for instance, N,N-dimethylformamide, abstracting a hydrogen atom from compound (4-24-4) using a base such as, for instance, sodium hydride, and reacting with compound (4-24-6). Compound (4-24-6) is used in the amounts of 1 equivalent to 10 equivalents based on compound (4-24-4). The base is used in the amounts of 1 equivalent to 10 equivalents based on compound (4-24-4). The reaction temperature is from 0°C to reflux temperature, and the reaction time is from 10 minutes to 24 hours.

The compounds according to the present invention, or salts or hydrates thereof, can be formulated into tablets, powders, fine granules, granules, coated tablets, capsulates, syrups, troches, inhalants, suppositories, injections, ointments, eye ointments, tapes, eye drops, nose drops, ear drops, cataplasms, lotions or the like, by the conventional methods. Such formulation can be achieved by using typical diluents, binders, lubricants, colorants, flavorants, and, as necessary, stabilizers, emulsifiers, absorbefacients, surfactants, pH modulators, preservatives, antioxidants or the like, and materials commonly used as ingredients of pharmaceutical preparations according to the conventional methods. For example, an oral preparation can be produced by combining a compound according to the present invention or a pharmaceutically acceptable salt thereof with a diluent, and if required, a binder, a disintegrating agent, a lubricant, a colorant, a flavorant or the like, and formulating the mixture into powders, fine granules, granules, tablets, coated tablets, capsules or the like according to the conventional methods. Examples of the materials include animal and vegetable oils such as soya bean oil, beef tallow, and synthetic glyceride; hydrocarbons such as liquid paraffin, squalane, and solid paraffin; ester oils such as octyldodecyl myristate and *iso*-propyl myristate; higher alcohols such as cetostearyl alcohol and behenyl alcohol; silicone resins; silicone oils; surfactants such as polyoxyethylene fatty acids ester, sorbitan fatty acid ester, polyoxyethylene sorbitan fatty acid ester, polyoxyethylene hydrogenated castor oil, and polyoxyethylene polyoxypropylene block co-polymer; water-soluble polymers such as hydroxyethyl cellulose, polyacrylic acid, carboxyvinyl polymer, polyethylene glycol, polyvinylpyrrolidone, and methytl cellulose; lower alcohols such as ethanol and isopropanol; polyhydric alcohols such as glycerol, propylene glycol, dipropylene glycol, and sorbitol; sugars such as glucose and sucrose; inorganic powder such as anhydrous silicic acid, magnesium aluminum silicate, and aluminum silicate; and pure water. Examples of the diluents include lactose, corn starch, white sugar, glucose, mannitol, sorbitol, crystalline cellulose, silicon dioxide or the like. Examples of the binders include polyvinyl alcohol, polyvinyl ether, methylcellulose, ethylcellulose, gum Arabic, tragacanth, gelatin, shellac, hydroxypropyl methylcellulose, hydroxypropyl cellulose, polyvinylpyrrolidone, polypropylene glycol-polyoxyethylene block co-polymer, and meglumine or the like. Examples of disintegrating agents include starch, agar, gelatin powder, crystalline cellulose, calcium carbonate, sodium hydrogencarbonate, calcium citrate, dextrin, pectin, calcium carboxymethyl cellulose or the like. Examples of lubricants include magnesium stearate, talc, polyethylene glycol, silica, hydrogenated vegetable oil or the like. Examples of colorants include those pharmaceutically acceptable. Examples of flavorants include cocoa powder, peppermint camphor, aromatic powder peppermint oil, Borneo camphor, cinnamon powder or the like. Tablets and granules may be coated with sugar, or if required, other appropriate coatings can be made. Solutions, such as syrups or injectable preparations, to be administered can be formulated by combining a compound according to the present invention or a pharmaceutically acceptable salt thereof with a pH modulator, a solubilizing agent, an isotonizing agent or the like, and if required, with an auxiliary solubilizing agent, a stabilizer or the like, according to the conventional methods. Methods for manufacturing an external preparations are not limited and such preparations can be manufactured by the conventional methods. Specifically, various materials typically used for manufacturing pharmaceuticals, quasi drugs, cosmetics or the like can be used as base materials for the external formulation. More specifically, examples of base materials to be used include animal and vegetable oils, minerals oils, ester oils, wax, higher alcohols, fatty acids, silicone oil, surfactants, phospholipids, alcohols, polyhydric alcohols, water-soluble polymers, clay minerals, pure water or the like. Furthermore, external preparations of the present invention can contain, as required, pH modulators, antioxidants, chelating agents, antibacterial/antifungal agents, colorants, odoriferous substances or the like. But this does not limit the type of base materials that are to be used in the external preparations of the present invention. If required, the preparation may contain differentiation inducers, blood flow improving agents, antimicrobial agents, antiphologistics, cell activators, vitamins, amino acids, humectants, keratolytic agents or the like. The amount of the base materials listed above is adjusted within a concentration range used for producing typical external preparations.

When administering a compound according to the present invention or a salt thereof, or a hydrate thereof, the forms of the compounds are not limited in particular, and the compound can be given orally or parenterally by the conventional method. For instance, the compound can be administered as a dosage form such as tablets, powders, granules, capsules, syrups, troches, inhalants, suppositories, injections, ointments, eye ointments, tapes, eye drops, nasal drops, ear drops, cataplasms and lotions.

Dose of a pharmaceutical of the present invention can be selected appropriately according to symptom severity, age, sex, body weight, forms of administration, type of salts, specific type of disease or the like.
The does varies remarkably depending on the patient's disease, symptom severity, age and sex, drug susceptibility or the like. An oral preparation of the present invention can be generally administered once or several time at a dose of from 1 to 10000 mg/adult/day, preferably from 10 to 2000 mg/adult/day. An injection of the present invention can be generally administered at a dose of from 0.1 to 10000 mg/adult/day, preferably from 1 to 2000 mg/adult/day.

The fact that the compound according to the present invention has antimalarial activity is tested, as described in WO 2004/048567, by introducing into a GWT1 gene deficient fungus an expression vector into which a degenerate mutant DNA with a decreased AT content compared to the malaria protozoa gene coding for the GWT1 (PfGWT1) protein of the tropical malaria protozoa Plasmodium falciparum (P. falciparum) has been inserted, and subjecting the test compound act. A specific description is as follows:
A degenerate mutant DNA sequence with a decreased AT content compared to the malaria protozoa gene coding for the GWT1 protein of the malaria protozoa is designed, as described in WO 2004/048567, by reverse translating based on the amino acid sequence of the GWT1 protein, listing up the codon sequences that each amino acid may adopt, and, among these, selecting the codons with low AT content and high frequency of use in the expression host. This is preferably the sequence described in SEQ. ID No. 5 (optimized PfGWT1 (opfGWT1)) of WO 2004/048567.
The designed degenerate mutant DNA can be synthesized by the methods widely known to those skilled in the art. The degenerate mutant DNA of the present invention is, for instance, synthesized based on the designed base sequence, using a commercial DNA synthesizer.
The GWT1 gene deficient fungus can be prepared by, for instance, using a homologous recombination technique to insert into the GWT1 gene an unrelated DNA, for instance, a selection marker or the like, and deleting the GWT1 gene of fungal origin. Specifically, it can be prepared by introducing into a yeast a selective marker cassette obtained by amplifying the his 5 gene or the kanamycin resistance gene of Schizosaccharomyces pombe (S. pombe) (Longtine et al, Yeast, 14: 953-961, 1998) with primers containing homologous base sequences to the GWT1 gene (50 bases or more and 70 bases or less). Note that the fungal GWT1 gene is described in WO 02/04626.
The GWT1 gene deficient fungus into which the above-mentioned degenerate mutant DNA has been introduced can be prepared by introducing into the fungus an expression vector with the degenerate mutant DNA inserted. pRS316, YEp351 and the like can be used as vectors for S. cerevisiae, and pcL, pALSK or the like for S. pombe.
The inhibitory activity of the compound according to the present invention on the PfGWT1 protein is tested by culturing the above fungus, adding the test compound and measuring the growth of the above fungus. Specifically, under conventional culture conditions, that is to say, a fungus is inoculated into a liquid culture medium such as the YPD culture medium (Yeast extract-polypeptone-dextrose culture medium) or onto an agar medium, cultured for on the order of 4 hours to 72 hours at 25°C to 37°C, and the growth of the GWT1 deficient fungus with the degenerate mutant DNA introduced is measured. In addition, the growth can be measured with the turbidity of the fungal culture solution, the number of colonies formed on the agar medium or the spot size or density as indicators. A compound that inhibits fungal growth is determined to have inhibitory activity on the PfGWT1 protein.
The effect of the compound according to the present invention on the malaria protozoa growth using malaria protozoa can be carried out by the methods described in Brobey R. K. B., Sano G., Itoh F., Aso K., Kimura M., Mitamura T. and Horii T. Recombinant Plasmodium falciparum dihydrofolate reductase-based in vitro screen for antifolate antimalarials. Mol. Biochem. Parasitol. 81: 225-237, 1996.

### Examples

### Example 1. Measurement of antimalarial agent activity using opfGWT1-expressing yeast

Compounds having antimalarial activity were screened, using the opfGWT1 expressing yeast, which expresses the malaria protozoa GWT1 gene. The opfGWT1 expressing yeast was prepared according to the methods described in Example 1 to Example 3 of WO 2004/048567.
The test compound was serially diluted twice in SD culture medium (Sabouraud-dextrose culture medium) so that the final highest concentration was 25 µg / ml and dispensed into a 96 well plate so as to obtain 50 µl / well. An overnight culture solution of the opfGWT1 expressing yeast diluted to 1 / 1000 was added therein in the amount of 50 µl / well addition, the plate was incubated at 30°C for 2 days, then turbidity at 660 nm was measured.
The minimum concentration of test compound, at which the test compound inhibited the growth of yeast cells and turbidity (660 nm) was 0.1 or less, was calculated as the minimum growth inhibitory concentration (MIC: Minimum inhibitory concentration), and reported in Table 1.

### [Table 1]

**TABLE 1**

| REFERENCE EXAMPLES | MIC (µg / m1) |
|---|---|
| | opfGWT1 EXPRESSING YEAST |
| A-4 | 6.25 |
| A-73 | 6.25 |
| A-182 | 6.25 |
| A-188 | 6.25 |
| A-66 | 12.5 |
| A-72 | 12.5 |
| A-98 | 12.5 |
| A-67 | 12.5 |
| A-68 | 12.5 |
| A-64 | 12.5 |
| A-168 | 12.5 |
| A-173 | 12.5 |
| E-65 | 25.0 |
| A-123 | 25.0 |
| A-69 | 25.0 |
| A-6 | 25.0 |
| A-54 | 25.0 |
| A-105 | 25.0 |
| A-177 | 25.0 |

The compounds shown in Table 1 all gave a MIC of 25 µg /ml or lower against the opfGWT1 expressing yeast.
From the above results, these compounds inhibiting the growth of the opfGWT1 expressing yeast were suggested to inhibit tropical malaria protozoa GWT1. From the fact that compounds thought to inhibit the growth of opfGWT1 expressing yeast and inhibit tropical malaria protozoa GWT1 demonstrated antimalarial activity in a red blood cell culture system of tropical malaria protozoa as shown in Example 4 of WO 2004/048567, similarly, compounds that inhibit the growth of opfGWT1 expressing yeast were thought to have an antimalarial activity.
The heterocyclic compound (I-a) according to the present invention or a salt thereof or hydrates thereof is extremely useful as an antimalarial agent.

### Reference Examples

The compounds according to the present invention can be prepared by a method described in, for instance, the following Preparation Examples and Reference Examples. With the proviso that these are illustrative, and that the compounds according to the present invention is not to be limited in any way to the following specific examples.

### Preparation Example A-1. 2-Amino-6-chloro-nicotinic acid

To liquid ammonia (approximately 20mL) was added 2,6-dichloro-nicotinic acid (0.38g, 2mmol) and copper(l) iodide (720mg, 3.8mmol) at -78°C in a sealed tube, and the solution was heated for 25 hours (the temperature of the oil bath was 115°C). The temperature of the oil bath was raised to 125°C, which was further heated for 14 hours 30 minutes. The reaction mixture was allowed to room temperature, and ammonia was evaporated. Methanol was added, insoluble matter was removed by filtration, and the filtrate was concentrated to obtain the title compound (0.25g, 1.45mmol, 72%) as a solid.
¹H-NMR Spectrum (DMSO-d₆) δ (ppm) : 6.63 (1H, d, J=8.0 Hz), 7.55 (2H, brs), 8.02 (1 H, d, J=8.0 Hz).

### Preparation Example A-2. 2-Amino-nicotinic acid methyl ester

2-Amino-nicotinic acid (10.0g, 72.4mmol) was dissolved in a mixed solution of methanol (200mL) and sulfuric acid (10mL), and the solution was stirred under reflux for 35 hours. A saturated aqueous solution of sodium bicarbonate was added to the reaction solution at 0°C, which was extracted with ethyl acetate, the organic layer was washed with brine and dried over anhydrous magnesium sulfate. The solvent was evaporated *in vacuo,* the residue was purified by NH silica gel column chromatography to obtain the title compound (5.26g, 34.6mmol, 48%) as a white solid.
¹H-NMR Spectrum (CDCl₃) δ (ppm) : 3.89 (3H, s), 6.63 (1H, ddd, J=1.1, 4.8, 7.7Hz), 8.13 (1 H, dd, J=1.6, 7.7Hz), 8.22 (1 H, dd, J=1.8, 4.8Hz).

### Preparation Example A-3 2-Amino-5-nitro-nicotinic acid methyl ester

2-Amino-nicotinic acid methyl ester (1.00g, 6.57mmol) described in Preparation Example A-2 was dissolved at 0°C in a mixed solution of nitric acid (0.7mL) and sulfuric acid (2.6mL), which was stirred at 0°C for 40 minute and at room temperature for 19 hours, then, further stirred at 70°C for 4 hours. A saturated aqueous solution of sodium bicarbonate was added to the reaction solution at 0°C, which was extracted with ethyl acetate and tetrahydrofuran, the organic layer was washed with brine and dried over anhydrous magnesium sulfate. The solvent was evaporated *in vacuo.* Methanol was added to the residue, the precipitated solid was filtered to obtain the title compound (459mg, 2.33mmol, 35%) as a white solid.
¹H-NMR Spectrum (DMSO-d₆) δ (ppm) : 3.86 (3H, s), 8.14 (1 H, brs), 8.62 (1 H, brs), 8.68 (1 H, d, J=2.7Hz), 9.04 (1 H, d, J=2.9Hz).

### Preparation Example A-4. 2-Amino-6-chloronicotinic acid

Tris(2-(2-methoxyethoxy)ethyl)amine (3.0mL, 9.4mmol) was added to a mixture of 2,6-dichloronicotinic acid (40g (90%purity), 0.19 mol), acetamide (80g, 1.4mol), potassium carbonate (78g, 0.56mol), copper(I) chloride (0.93g, 9.4mmol) and xylene (80mL), which was stirred overnight at 145°C. After cooling, copper(I) chloride (0.46g, 4.6mmol) was added to the reaction solution, which was stirred overnight at 145°C. After cooling the reaction solution to 105°C, water (1 00mL) was added, the solution was stirred for 1 hour at the same temperature, and cooled down to room temperature. 5N hydrochloric acid (150mL) was added, the solution was neutralized with a citric acid aqueous solution, then, ethyl acetate was added, and the solution was filtered through Celite pad. The organic layer was washed with brine, then, the solvent was evaporated *in vacuo.* The residue was purified by silica gel column chromatography (ethyl acetate), recrystallization by the ethyl acetate-hexane was carried out to obtain the title compound (1.4g, 8.3mmol, 4.5%) as white crystal.
¹H-NMR Spectrum (DMSO-d₆) δ (ppm) : 6.61 (1H, d, J=8.1 Hz), 7.53 (2H, brs), 8.01 (1 H, d, J=8.1 Hz).

### Preparation Example A-5. 2-Amino-6-(2-hydroxy-ethoxy)-nicotinic acid

To ethyleneglycol (0.50mL) was added sodium hydride (70mg, 1.7mmol, 60% in oil), catalytic amount of copper(I) iodide and 2-amino-6-chloronicotinic acid (30mg, 0.17mmol), which was stirred for 3 hours at 110°C, then, further stirred overnight at 80°C. After cooling, water, diethyl ether and aqueous ammonia was added to the reaction solution, which was then partitioned, the aqueous layer was neutralized with citric acid, then, extracted with dichloromethane. The organic layer was washed with brine, then dried over anhydrous magnesium sulfate. The solvent was evaporated *in vacuo* to obtain the title compound (14mg).

### Preparation Example A-6. 2-Amino-6-ethoxy-nicotinic acid

The title compound (35mg) was obtained from ethanol (0.50mL) and 2-amino-6-chloro-nicotinic acid (30mg, 0.17mmol) according to an analogous method to Preparation Example A-5.

### Preparation Example A-7. 2-Amino-6-isopropoxy-nicotinic acid

The title compound (60mg) was obtained from isopropanol (0.50mL) and 2-amino-6-chloro-nicotinic acid (30mg, 0.17mmol) according to an analogous method to Preparation Example A-5.

### Preparation Example A-8. 2-Amino-6-chloro-nicotinic acid methyl ester

To methanol (50mL) were added concentrated sulfuric acid (25mL) and 2-amino-6-chloro-nicotinic acid (4.3g, 25mmol) described in Preparation Example A-1 (or A-4) on an ice bath, which was stirred at 70°C for 5 hours. After cooling, an aqueous solution of sodium bicarbonate (90g) was added to neutralize. The resulting solid was filtered to obtain the title compound (3.2g, 17mmol, 68%) as a light brown solid.
¹H-NMR Spectrum (CDCl₃) δ (ppm) : 3.88(3H, s), 6.62(1 H, d, J=8.2Hz), 8.05(1 H, d, J=8.1 Hz).

### Preparation Example A-9. Tributyl-methoxymethyl-stannane

To a mixture of diisopropylamine (9.4mL,67mmol) and tetrahydrofuran (150mL) was added n-butyl lithium (2.4M n-hexane solution, 25mL, 61 mmol) dropwise at -78°C, which was stirred for 30 minutes at the same temperature. Tributyltin hydride (16mL, 61 mmol) was added dropwise at the same temperature, the solution was then stirred for 30 minutes on ice. The reaction solution was brought to -78°C, chloromethyl methyl ether (4.6mL, 61mmol) was added dropwise thereto, the solution was then gradually warmed to room temperature. Water (100mL) was added to the reaction solution, which was then extracted with diethyl ether (300mL). The organic layer was washed with brine, then, evaporated *in vacuo.* The residue was purified by neutral silica gel column chromatography (heptane /ethyl acetate = 30 / 1), and the title compound (18g, 0.52mmol, 86%) was obtained as a colorless oil.
¹H-NMR Spectrum (CDCl₃) δ (ppm) : 0.88-0.93(15H, m), 1.26-1.35(6H, m), 1.47-1.55(6H, m), 3.30(3H, s), 3.71 (2H, t, J=6.8Hz).

### Preparation Example A-10. 2-Amino-6-methoxymethyl-nicotinic acid methyl ester

A mixture of 2-amino-6-chloro-nicotinic acid methyl ester described in Preparation Example A-8 (1.4g, 7.6mmol), tributyl-methoxymethyl-stannane (3.1 g, 9.1mmol) described in Preparation Example A-9, tetrakis(triphenylphosphine)palladium (440mg, 0.38mmol) and N-methylpyrrolidinone (20mL) was stirred at 130°C for 3.5 hours. The reaction solution was allowed to cool, an aqueous solution of potassium fluoride and ethyl acetate were added thereto on an ice bath, followed by filtering through Celite pad. The organic layer was washed with brine, then, evaporated *in vacuo.* The residue was purified by silica gel column chromatography (heptane / ethyl acetate=2/1), and the title compound (0.93g, 4.7mmol, 63%) was obtained as a light brown oil.
¹H-NMR Spectrum (CDCl₃) δ (ppm) : 3.47(3H, s), 3.88(3H, s), 4.41 (2H, s), 6.74(1 H, d, J=7.9Hz), 8.14(1 H, d, J=7.9Hz).

### Preparation Example A-11. 2-Amino-6-methoxymethyl-nicotinic acid

Lithium hydroxide monohydrate (1.2g, 29mmol) was added to a mixture of 2-amino-6-methoxymethyl-nicotinic acid methyl ester described in Preparation Example A-10 (2.9g, 15mmol), tetrahydrofuran (30mL), methanol (7.5mL), and water (7.5mL), which was then stirred overnight at room temperature. Acetic acid (1.7mL, 29mmol) was added to the reaction solution and the solvent was evaporated *in vacuo.* After filtration using silica gel (methanol/ ethyl acetate=1/3) and the solvent was evaporated *in vacuo,* the residue was washed with water, and the title compound (2.1 g, 12mmol,80%) was obtained as a pale yellow solid.
¹H-NMR Spectrum (DMSO-d₆) δ (ppm) : 3.32(3H, s), 4.29(2H, s), 6.61 (1 H, d, J=7.9Hz), 7.16(2H, br s), 8.02(1H, d, J=7.9Hz).

### Preparation Example A-12. 2-(2-Cyanoethyl)-3,3-diaminopropenoic acid ethyl ester

(1-Ethoxyformimidoyl) 1-acetic acid ethyl ester hydrochloride (50g, 0.26mol) was suspended in an ammonia-ethanol solution (300mL; prepared by saturating ethanol with ammonia gas at room temperature), which was then stirred at room temperature for 4 hours. After the reaction was completed, the precipitated salt was removed by filtration, and the filtrate was concentrated *in vacuo* at room temperature to reach 1/3 of the amount. Hydrochloric acid-methanol (130mL; hydrochloric acid content:7.5%) was added to this filtrate, the solution was then concentrated under a reduced pressure to obtain 3,3-diamino-acrylic acid ethyl ester hydrochloride (40g, 0.24mol, 92%) as a solid.
The resulting 3,3-diamino-acrylic acid ethyl ester hydrochloride (2.2g, 13.2mmol) was suspended in tetrahydrofuran (40mL), triethylamine (2mL, 14.3mmol) and acrylonitrile (1.2mL, 19.3mmol) were added thereto, and the solution was refluxed for 6 hours. After the reaction was completed, the resulting triethylamine hydrochloride was filtered, and the filtrate was concentrated to obtain the title compound (0.6g, 3.3mmol, 25%).
¹H-NMR Spectrum (CDCl₃) δ (ppm) :1.26 (3H, t, J=7.2Hz), 2.42-2.49 (2H, m), 2.50-2.57 (2H, m), 4.12 (2H, q, J=7.2Hz), 4.22 (2H, brs), 6.45 (2H, brs).

### Preparation Example A-13. 2,6-Diamino-4,5-dihydronicotinic acid ethyl ester

A solution of 2-(2-cyanoethyl) 3,3-diaminopropenoic acid ethyl ester described in Preparation Example A-12 (0.55g, 3mmol) in tetrahydrofuran (7mL) was added dropwise to a suspension of sodium hydride (208mg, 5.2mmol, 60% in oil) in tetrahydrofuran (7mL), and the solution was stirred for 19 hours 20 minutes under reflux. After the reaction was completed, the reaction solution was poured into an ice water, which was then extracted with ethyl acetate. The organic layer was dried over anhydrous magnesium sulfate, then, concentrated to obtain the title compound as a crude product (0.188g, 1mmol, 34%).
¹H-NMR Spectrum (CDCl₃) δ (ppm) :1.27 (3H, t, J=7.2Hz), 2.28-2.34 (2H, m), 2.46-2.52 (2H, m), 4.14 (2H, q, J=7.2Hz).

### Preparation Example A-14. 2,6-Diamino-nicotinic acid ethyl ester

To a solution of 2,6-diamino-4,5-dihydronicotinic acid ethyl ester described in Preparation Example A-13 (4.5g, 24.6mmol) in tetrahydrofuran (300mL) was added 2,3-Dichloro-5,6-dicyano-1,4-benzoquinone (5.6g, 24.7mmol), which was then stirred for 40 minutes at room temperature. The residue obtained by concentrating the reaction solution was then purified by NH silica gel column chromatography (ethyl acetate) to obtain a solid of the target compound. This solid was washed with hexane and dried to obtain the title compound (3.1 g, 17.1 mmol, 69.5%).
¹H-NMR Spectrum (CDCl₃) δ (ppm) :1.35 (3H, t, J=7.2Hz), 4.27 (2H, q, J=7.2Hz), 4.60 (2H, brs), 5.82 (1 H, d, J=8.4Hz), 7.90 (1 H, d, J=8.4Hz).

### Preparation Example A-15. 2,6-Diamino-nicotinic acid

2,6-Diamino-nicotinic acid ethyl ester described in Preparation Example A-14 (2g, 11 mmol) was dissolved in ethanol (15mL), an aqueous solution of 1 N sodium hydroxide (15mL) was added thereto, followed by stirring for 2 hours under reflux. The reaction solution was allowed to room temperature, ethanol was then removed by evaporation, the residue was cooled on an ice bath, then neutralized with 1 N hydrochloric acid. The precipitated solid was collected by filtration, washed with water, then, dried to obtain the title compound (1.72g, 11 mmol, quantitatively).
¹H-NMR Spectrum (DMSO-d₆) δ (ppm) : 5.70 (1H, d, J=8.4Hz), 6.31 (2H, brs), 6.58-7.12 (1 H, brs), 7.62 (1 H, d, J=8.4Hz).

### Preparation Example A-16. 2-Amino-6-vinyl-nicotinic acid methyl ester

2-Amino-6-chloro-nicotinic acid methyl ester (2.95g, 15.8mmol), vinyl tri-*n*-butyltin (5.01g, 15.8mmol) and tetrakis(triphenylphosphine)palladium(0) (1.83g, 1.58mmol) were suspended in xylene (15mL), and heated for 1 hour at 130°C. The reaction mixture was allowed to room temperature, water and ethyl acetate were added thereto, this mixture was filtered through Celite pad, then, the filtrate thereof was partitioned. The organic layer was separated, the solvent was evaporated in *vacuo,* and the residue was purified by silica gel column chromatography to obtain the title compound (1.87g, 10.5mmol, 66%) as a white solid.
¹H-NMR Spectrum (DMSO-d₆) δ (ppm) : 3.81 (3H, s), 5.54 (1H, dd, J=1.6, 10.4 Hz), 6.24 (1 H, dd, J=1.6, 17.2 Hz), 6.65 (1 H, dd, J=10.4, 17.2 Hz), 6.76 (1 H, d, J=8.0 Hz), 7.16 (1 H, brs), 8.04 (1 H, d, J=8.0 Hz).

### Preparation Example A-17. 2-Amino-6-(2-cyanoethy)-nicotinic acid methyl ester

To a solution of 2-amino-6-vinyl-nicotinic acid methyl ester (760mg, 4.26mmol) in tetrahydrofuran (76mL) was added a solution of diethyl aluminum cyanide in toluene (12.8mL, 12.8mmol) dropwise under sodium chloride - an ice bath cooling, at an internal temperature of -5°C or below, then, the solution was gradually allowed to room temperature and stirred overnight. The reaction solution was partitioned into an aqueous solution of saturated ammonium chloride and ethyl acetate. This organic layer was separated, washed with brine and dried over anhydrous magnesium sulfate. The solvent was evaporated *in vacuo,* the residue was purified by silica gel column chromatography, and the title compound (180mg, 0.878mmol, 21 %) was obtained as a yellow oil.
¹H-NMR Spectrum (CDCl₃) δ (ppm) : 2.80 (2H, t, J=7.2 Hz), 2.97 (2H, t, J=7.2 Hz), 3.88 (3H, s), 6.53 (1 H, d, J=8.0 Hz) 8.07 (1 H, d, J=8.0 Hz).

### Preparation Example A-18. 2-Amino-6-(2-cyanoethyl)-nicotinic acid

To a solvent mixture of 2N-sodium hydroxide aqueous solution (5mL) and methanol (5mL) was added 2-amino-6-(2-cyanoethyl)-nicotinic acid methyl ester (90mg, 0.439mmol), and the solution was stirred for 18 hours at room temperature. This mixed solution was neutralized with 5N-hydrochloric acid, then, extracted with ethyl acetate. This organic layer was dried over anhydrous magnesium sulfate, then, the solvent was evaporated *in vacuo,* and the title compound (68mg, 0.355mmol, 81 %) was obtained as a yellowish brown solid.
¹H-NMR Spectrum (DMSO-d₆) δ (ppm) : 2.86 (4H, bs), 6.54 (1H, d, J=8.0Hz), 7.18 (2H, bs), 7.98 (1 H, d, J=8.0Hz).

### Preparation Example A-19. 2-Amino-6-(2-ethoxy-vinyl)-nicotinic acid methyl ester

To a solution of ethyl ethynyl ether (3.6g, 25.7mmol) in tetrahydrofuran (10mL) was added catechol borane (3.08g, 25.7mmol) on an ice bath. Immediately, the cold bath was removed, the reaction mixture was allowed to room temperature, and then, the solution was stirred for 1.5 hours under reflux. This reaction mixture was allowed to room temperature. To this mixture solution were added 2-amino-6-chloro-nicotinic acid methyl ester (1.6g, 8.57mmol), sodium hydroxide powder (1.13g, 28.3mmol), tetrakis(triphenylphosphine)palladium(0) (0.99g, 0.857mmol) and dioxane (20mL), which was then stirred for 2 hours under reflux. The reaction mixture was allowed to room temperature, and partitioned into water and ethyl acetate. The organic layer was separated, the solvent was evaporated in *vacuo,* the residue was purified by silica gel column chromatography, and the title compound (1.30g, 5.85mmol, 68%) was obtained as a brown solid.
¹H-NMR Spectrum (DMSO-d₆) δ (ppm) :1.27 (3H, t, J=7.2 Hz), 3.77 (3H, s), 3.95 (2H, q, J=7.2 Hz), 5.75 (1 H, d, J=12.8 Hz), 6.49 (1 H, d, J=8.4 Hz), 7.02 (2H, brs), 7.63 (1 H, d, J=12.8Hz), 7.89 (1H, d, J=8.4Hz).

### Preparation Example A-20. 2-Amino-6-(2-hydroxy-ethyl)-nicotinic acid methyl ester

2-Amino-6-(2-ethoxy-vinyl)-nicotinic acid methyl ester (1.07g, 4.81 mmol) was dissolved in 5N-hydrochloric acid (25mL), ethanol (20mL) and tetrahydrofuran (5mL), and the solution was stirred for 3 hours under reflux. This reaction mixture was allowed to room temperature, and neutralized with a aqueous solution of saturated sodium bicarbonate, then, sodium borohydride (1g, 26.5mmol) was added to this reaction mixture, which was then stirred for 20 minutes at room temperature. Ethyl acetate was added thereto, followed by filtrating through Celite pad. This filtrate was partitioned. This organic layer was separated, the solvent was evaporated *in vacuo,* the residue was purified by silica gel column chromatography, and the title compound (350mg, 1.92mmol, 40%) was obtained as a brown oil.
¹H-NMR Spectrum (DMSO-d₆) δ (ppm) : 2.72 (2H, t, J=6.8 Hz), 3.71 (2H, q, J=6.8Hz), 3.79 (3H, s), 4.66 (1 H, t, J=6.8Hz), 6.53 (1 H, d, J=8.0 Hz), 7.12 (2H, brs), 7:95 (1 H, d, J=8.0Hz).

### Preparation Example A-21. 2-Amino-6-(2-fluoro-ethyl)-nicotinic acid methyl ester

A solution of (bis(2-methoxyethyl)amino)sulfur trifluoride (2.39g, 10.8mmol) in dichloromethane (30mL) was cooled with a dry ice-methanol bath, and 2-amino-6-(2-hydroxyethyl)-nicotinic acid methyl ester (50mg, 0.255mmol) was added thereto dropwise. After dropwise addition the cold bath was immediately removed, and the solution was gradually allowed to room temperature. Water and ethyl acetate were added to the reaction solution, which was then partitioned. The organic layer was separated, the solvent was evaporated *in vacuo,* the residue was purified by silica gel column chromatography, and the title compound (4mg, 0.020mmol, 7.9%) was obtained as a white solid.
¹H-NMR Spectrum (DMSO-d₆) δ (ppm) : 2.97 (2H, td, J=6.0, 26.0 Hz), 3.80 (3H, s), 4.77 (2H, td, J=6.0, 47.2 Hz), 6.58 (1 H, d, J=8.0 Hz), 7.16 (2H, bs), 8.00 (1 H, d, J=8.0 Hz).

### Preparation Example A-22. 2-Amino-6-(2-fluoro-ethyl)-nicotinic acid

2-Amino-6-(2-fluoro-ethyl)-nicotinic acid methyl ester (77mg, 0.387mmol) was dissolved in an aqueous solution of 2N sodium hydroxide (5mL) and methanol (5mL), and the solution was stirred for 20 minutes at room temperature. This mixture solution was neutralized with 5N hydrochloric acid, then, extracted with ethyl acetate. The organic layer was separated, the solvent was evaporated *in vacuo,* and the title compound (64mg, 0.348mmol ,90%/65% purity) was obtained as a white solid.
¹H-NMR Spectrum (DMSO-d₆) δ (ppm) : 2.97 (2H, td, J=6.0, 26.0 Hz), 4.77 (2H, td, J=6.0, 47.2 Hz), 6.57 (1 H, d, J=8.0 Hz), 7.98 (1 H, d, J=8.0 Hz).

### Preparation Example A-23. Tributyl-ethoxymethyl-stannane

The title compound (2.8g, 8.0mmol, 67%) was obtained as a colorless oil from chloromethyl ethyl ether (1.1 mL, 12mmol) according to an analogous method to Preparation Example A-9.
¹H-NMR Spectrum (CDCl₃) δ (ppm) : 0.87-0.92(15H, m), 1.16(3H, t, J=7.0Hz), 1.26-1.35(6H, m), 1.43-1.55(6H, m), 3.36(2H, q, J=7.0Hz), 3.74(2H, t, J=6.5Hz).

### Preparation Example A-24. 2-Amino-6-ethoxymethyl-nicotinic acid methyl ester

The title compound (0.35g, 1.7mmol, 39%) was obtained as a pale yellow solid from tributyl-ethoxymethyl-stannane (2.0g, 6.3mmol) described in Preparation Example A-23 and 2-amino-6-chloro-nicotinic acid methyl ester described in Preparation Example A-8 (0.80g, 4.3mmol) according to an analogous method to Preparation Example A-10.
¹H-NMR Spectrum (CDCl₃) δ (ppm) :1.28(3H, t, J=7.0Hz), 3.61(2H, q, J=7.0Hz), 3.88(3H, s), 4.45(2H, s), 6.41 (2H, br s), 6.78(1 H, d, J=7.9Hz), 8.13(1 H, d, J=8.1Hz).

### Preparation Example A-25. 2-Amino-6-ethoxymethyl-nicotinic acid

The title compound (180mg, 0.92mmol, 57%) was obtained as a pale yellow solid from 2-amino-6-ethoxymethyl-nicotinic acid methyl ester (330mg, 1.6mmol) described in Preparation Example A-24 according to an analogous method to Preparation Example A-11.
¹H-NMR Spectrum (DMSO-d₆) δ (ppm) :1.15(3H, t, J=7.1Hz), 3.51 (2H, q, J=7.0Hz), 4.33(2H, s), 6.64(1 H, d, J=7.9Hz), 8.02(1 H, d, J=7.9Hz).

### Preparation Example A-26. Tributyl-isopropoxymethyl-stannane

To a mixture of isopropanol (2mL) and tetrahydrofuran (2mL) was added sodium hydride (66%, 58mg, 1.6mmol) on an ice bath, then, the solution was stirred for 20 minutes at room temperature. To the reaction solution was added a solution of tributyl-iodomethyl-stannane (230mg, 0.53mmol), synthesized according to the document (Synthetic Communications, Vol.24, No.8, pp. 1117-1120), in tetrahydrofuran (1mL) dropwise on an ice bath, then, N,N-dimethylformamide (0.5mL) was added thereto, followed by stirring overnight at room temperature. The reaction solution was partitioned into water (20mL) and diethyl ether (50mL). The organic layer was separated, washed with brine, and then, evaporated *in vacuo.* The residue was purified by neutral silica gel column chromatography (heptane : ethyl acetate = 30 : 1), and the title compound (63mg, 0.17mmol, 32%) was obtained as a colorless oil.
¹H-NMR Spectrum (CDCl₃) δ (ppm) : 0.87-0.91(15H, m), 1.11(6H, d, J=6.0Hz), 1.26-1.35(6H, m), 1.47-1.53(6H, m), 3.28-3.31(1H, m), 3.69(2H, t, J=7.6Hz).

### Preparation Example A-27. Butoxymethyl-tributyl-stannane

The title compound (220mg, 0.58mmol, 99%) was obtained as a colorless oil from tributyl-iodomethyl-stannane (250mg, 0.58mmol) according to an analogous method to Preparation Example A-26.
¹H-NMR Spectrum (CDCl₃) δ(ppm) : 0.87-0.93(18H, m), 1.26-1.38(8H, m), 1.47-1.55(8H, m), 3.30(2H, t, J=6.5Hz), 3.73 (2H, t, J=6.5Hz).

### Preparation Example A-28. Tributyl-propoxymethyl-stannane

The title compound (230mg, 0.63mmol, 97%) was obtained as a colorless oil from tributyl-iodomethyl-stannane (280mg, 0.65mmol) according to an analogous method to Preparation Example A-26.
¹H-NMR Spectrum (CDCl₃) δ (ppm) : 0.87-0.91(18H, m), 1.26-1.35(6H, m), 1.47-1.58(8H, m), 3.27(2H, t, J=6.5Hz), 3.73 (2H, t, J=6.5Hz).

### Preparation Example A+-1. Sodium 4-(((2-Aminopyridine-3-carbonyl)-amino)-methyl)-phenolate

To a solution of 4-hydroxybenzaldehyde (10g, 81.9mmol) in methanol (45mL) was added Raney nickel (3g) and 7N aqueous ammonia solution (45mL), and the solution was stirred under hydrogen atmosphere (1 atm) at room temperature for 21 hours. The reaction solution was filtered through Celite pad to remove the catalyst, the filtrate was concentrated, and 4-aminomethyl-phenol (10g, quantitatively) was obtained as a pale green solid.
Then, a solution of 2-aminonicotinic acid (3.0g, 21.7mmol) in N,N-dimethylformamide (30mL) was cooled with an ice water, 1-hydroxybenzotriazole (3.51 g, 26mmol), (3-dimethylaminopropyl)-ethyl-carbodiimide (4.04g, 26mmol) and a solution of the resulting 4-aminomethyl-phenol (3.0g, 21.7mmol) in N,N-dimethylformamide (20mL) were added, and the solution was stirred for 18 hours at this temperature. The reaction solution was partitioned into brine, the organic layer was dried over anhydrous magnesium sulfate, and the solvent was evaporated.
The residue was dissolved in ethyl acetate, filtration was carried out using NH silica gel, and the filtrate was concentrated. The residue was dissolved in methanol (90mL), 1 N sodium hydroxide (17.8mL, 17.8mmol) was added thereto, followed by stirring at room temperature for an hour and a half. The reaction solution was concentrated *in vacuo,* and the title compound (5.66g) was obtained as a pale yellow solid.

### Preparation Example A+-2. (6-Amino-5-((5-(3-fluoro-phenoxy)-thiophene-2-ylmethyl)-carbamoyl)-pyridine-2-yl)-carbamic acid tert-butyl ester

To a solution of 6-amino-nicotinic acid (270mg, 2.0mmol) and C-(5-(3-fluorophenoxy)thiophen-2-yl)methylamine (400mg, 1.8mmol) in N,N-dimethylformamide (10mL) were added benzotriazole-1-yl-tris(dimethylamino)phosphonium hexafluorophosphate (870mg, 2.0mmol) and triethylamine (0.50mL, 3.6mmol), and the solution was stirred for 30 minutes at 60°C. Water and ethyl acetate were added to the reaction solution, which was then partitioned, and the organic layer was washed twice with water. Silica gel was added to the organic layer, the solvent was evaporated *in vacuo* for adsorption, purification was carried out with NH silica gel column chromatography (ethyl acetate, then ethyl acetate : methanol = 10 : 1), and 6-amino-N-(5-(3-fluoro-phenoxy)-thiophen-2-yl)-methylnicotinamide (270mg, 0.79mmol, 43.9%) was obtained.
To the resulting 6-amino-N-(5-(3-fluoro-phenoxy)-thiophen-2-yl)-methylnicotinamide (270mg, 0.79mmol) were added di-*tert*-butyldicarbonate (210mg, 0.94mmol) and *tert*-butyl alcohol (15mL), and the solution was stirred for 16.5 hours at room temperature. NH silica gel was added to the reaction solution, solvent was evaporated *in vacuo* for adsorption, purification was carried out by NH silica gel column chromatography (hexane : ethyl acetate = 4:1 then 1:1), and the title compound (250mg, 0.54mmol, 68.3%) was obtained as a white solid.
¹H-NMR Spectrum (DMSO-d₆) δ (ppm) :1.46 (9H, s), 4.52 (2H, d, J=5.6Hz), 6.55-6.59 (1 H, m), 6.78-6.82 (1 H, m), 6.88-7.00 (4H, m), 7.36-7.44 (1 H, m), 7.85 (1H, d, J=8.8Hz), 8.15 (1H, d, J=8.8Hz), 8.70 (1H,s), 9.14 (1H, t, J=5.6Hz), 10.1 (1H, s).

### Preparation Example A+-3. (6-Amino-5-((5-(3-fluoro-phenoxy)-thiophen-2-ylmethyl)-carbamoyl)-pyridin-2-yl)-methyl-carbamic acid tert-butyl ester

To a solution of (6-amino-5-((5-(3-fluoro-phenoxy)-thiophen-2-ylmethyl)-carbamoyl)-pyridine-2-yl)-carbamic acid *tert*-butyl ester described in Preparation Example A+-2 (125mg, 0.27mmol) and methyl iodide (43mg, 0.29mmol) in N,N-dimethylformamide (5mL) was added sodium hydride (12mg,0.29mmol,60% in oil) on an ice bath, and the solution was stirred for 1 hour at room temperature. NH silica gel was added to the reaction solution, the solvent was evaporated *in vacuo* for adsorption, purification was carried out by NH silica gel column chromatography (hexane : ethyl acetate = 4 : 1 then 2 : 1), and the title compound (87mg, 0.19mmol, 70.5%) was obtained as a colorless oil.
¹H-NMR Spectrum (DMSO-d₆) δ (ppm) :1.46 (9H, s), 3.31 (3H, s), 4.53 (2H, d, J=5.6Hz), 6.57 (1 H, d, J=3.6Hz), 6.81 (1 H, d, J=3.6Hz), 6.86-7.00 (3H, m), 7.36-7.44 (1H, m), 7.76 (1 H, d, J=8.8Hz), 8.14 (1 H, dd, J=2.0, 8.8Hz), 8.80 (1 H, d, J=2.0Hz), 9.22 (1 H, t, J=5.6Hz).

### Preparation Example A+-4. (6-Amino-5-((5-benzyl-thiophen-2-ylmethyl)-carbamoyl)-pyridine-2-yl)-carbamoylmethyl-carbamic acid tert-butyl ester

To a solution of 6-aminonicotinic acid (340mg, 2.4mmol) and C-(5-benzyl-thiophen-2-yl)-methylamine described in Preparation Example 42 (450mg, 2.2mmol) in N,N-dimethylformamide (5mL) were added benzotriazol-1-yl-tris(dimethylamino)phosphonium hexafluorophosphate (1.1g, 2.4mmol) and triethylamine(0.62mL, 4.4mmol), and the solution was stirred at 60°C for 1 hour. Water and ethyl acetate were added to the reaction solution, which was then partitioned, and the organic layer was washed twice with water. Silica gel was added to the organic layer, the solvent was evaporated *in vacuo* adsorption, purification was carried out by NH silica gel column chromatography (hexane : ethyl acetate = 1:1, then ethyl acetate, then ethyl acetate : methanol = 10 : 1), and 6-amino-N-(5-benzyl-thiophen-2-ylmethyl)-nicotinamide (210mg, 0.65mmol,29.5%) was obtained.
Then, according to an analogous method to Preparation Example A+-2, from the resulting 6-amino-N-(5-benzyl-thiophen-2-ylmethyl)-nicotinamide (210mg,0.65mmol),
(5-((5-benzyl-thiophen-2-ylmethyl)-carbamoyl)-pyridin-2-yl)-carbamic acid *tert*-butyl ester (120mg, 0.28mmol, 43.0%) was obtained as a colorless oil.
To a solution of this oil (60mg, 0.14mmol) in N,N-dimethylformamide (5mL) were added sodium hydride (6.8mg, 0.14mmol, 60% in oil) and bromoacetamide (23mg, 0.16mmol), and the solution was stirred for 25 hours room temperature. Water and ethyl acetate were added to the reaction solution, which was then partitioned, the organic layer was evaporated *in vacuo,* the residue was purified by NH silica gel column chromatography (hexane : ethyl acetate = 1:1, then ethyl acetate, then ethyl acetate : methanol = 20 : 1), and the title compound (23mg, 0.047mmol, 33.5%) was obtained as a colorless oil.
¹H-NMR Spectrum (DMSO-d₆) δ (ppm) :1.43 (9H, s), 4.04 (2H, s), 4.49-4.54 (4H, m), 6.69 (1H, d, J=3.6Hz), 6.79 (1H, d, J=3.6Hz), 6.94 (1H, s), 7.16-7.30 (5H, m), 7.38 (1H, s), 7.87 (1H, d, J=8.8Hz), 8.10-8.16 (1H, m), 8.68-8.73 (1H, m), 9.13 (1H, t, J=5.6Hz).

### Preparation ExampIeA+-5. 6-Chloro-N-(1-(3-fluoro-benzyl)-1H-pyrrol-3-ylmethyl)-nicotinamide

6-chloro-nicotinic acid (100mg, 0.58mmol), triethylamine (0.194mL, 1.39mmol) and benzotriazol-1-yloxytris(dimethylamino)phosphonium hexafluorophosphate (308mg, 0.696mmol) were dissolved in N,N-dimethylformamide (3mL), C-(1-(3-fluoro-benzyl)-1*H*-pyrrol-3-yl)-methylamine (142mg, 0.695mmol) described in Preparation Example 59 was added, and the solution was stirred for 15 hours 10 minutes at room temperature. After the reaction was completed, the reaction solution was poured into brine, and the solution was extracted with ethyl acetate. The organic layer was dried over anhydrous magnesium sulfate, then the solution was concentrated, the resulting residue was purified by NH silica gel column chromatography (ethyl acetate : hexane = 1 : 1), and the title compound (0.14g, 0.39mmol, 67%) was obtained.
¹H-NMR Spectrum (CDCl₃) δ (ppm) : 4.42 (2H, d, J=4.8Hz), 5.02 (2H, s), 5.99-6.09 (1 H, m), 6.16-6.18 (1 H, m), 6.56 (1 H, d, J=8.0 Hz), 6.57 (1 H, brs), 6.64-6.68 (2H, m), 6.78-6.83 (1 H, m), 6.91-6.94(1H, m), 6.96-7.02 (1H, m), 7.27-7.33 (1 H, m), 7.49 (1H, d, J=8.0 Hz).

### Preparation Example A+-6. 2-(Ethoxymethyl-amino)-N-(5-phenoxy-thiophen-2-ylmethyl)-nicotinamide

To a solution of 2-aminonicotinic acid (3245mg, 23.49mmol) in N,N-dimethylformamide (200mL) were added C-(5-phenoxy-thiophen-2-yl)methylamine (5305mg, 25.84mmol) described in Preparation Example 24, benzotriazol-1-yloxytris(dimethylamino)phosphonium hexafluorophosphate (12.49g, 28.19mmol) and triethylamine (7.86mL, 56.38mmol), and the solution was stirred for 2 days at room temperature. Water was added to the reaction mixture, which was then extracted with ethyl acetate, the organic layer was sequentially washed with an aqueous solution of saturated sodium bicarbonate, water, then brine, the solvent was evaporated *in vacuo,* and 2-amino-N-(5-phenoxy-thiophen-2-ylmethyl)-nicotinamide (4999mg, 15.36mmol, 65%) was obtained as crude product.
To a solution of the resulting 2-amino-N-(5-phenoxy-thiophen-2-ylmethyl)-nicotinamide (602mg, 1.85mmol) in ethanol (40mL) were added 5,5-dimethylhydantoin (260mg, 2.04mmol), 37% formic acid aqueous solution (3.00mL, 23.6mmol), and the solution was stirred under reflux for 1 hour. Water was added to the reaction mixture, which was then extracted with ethyl acetate, the organic layer was sequentially washed with water and brine, dried over anhydrous sodium sulfate, and then, evaporated *in vacuo.* The resulting residue was purified by silica gel chromatography (hexane-ethyl acetate), and the title compound (430mg, 1.12mmol, 61 %) was obtained.
¹H-NMR Spectrum (DMSO-d₆) δ (ppm):1.03 (3H, t, J=6.8Hz), 3.41 (2H, q, J=6.8Hz), 4.48 (2H, d, J=5.6Hz), 4.90 (2H, d, J=7.2Hz), 6.49 (1H, d, J=3.6Hz), 6.68 (1H, dd, J=4.8, 7.6Hz), 6.77 (1 H, d, J=3.6Hz), 7.06-7.14 (3H, m), 7.33-7.38(2H, m), 7.94 (1 H, dd, J=1.6, 7.6Hz), 8.19 (1 H, dd, J=1.6, 4.8Hz), 8.91 (1 H, t, J=7.2Hz), 9.16-9.20 (1 H, m).

### Preparation Example A+-7. 2-Chloro-N-(5-phenoxy-thiophen-2-ylmethyl)-nicotinamide

To a solution of 2-chloro nicotinic acid (1182mg, 7.50mmol) in N,N-dimethylformamide (3mL) were added C-(5-phenoxy-thiophen-2-yl)methylamine described in Preparation Example 24 (1693mg, 8.25mmol), benzotriazol-1-yloxy tris(dimethylamino)phosphonium hexafluorophosphate (3987mg, 9.0mmol) and triethylamine (2.5mL, 18.0mmol), and the solution was stirred at 60°C for 2 days. Water was added to the reaction mixture, which was then extracted with ethyl acetate, and then concentrated. The residue was purified by silica gel column chromatography, and the title compound (1181mg, 3.43mmol, 46%) was obtained.
¹H-NMR Spectrum (DMSO-d₆) δ (ppm) : 4.51 (2H, d, J=5.2Hz), 6.52 (1H, d, J=3.6Hz), 6.80 (1 H, d, J=3.6Hz), 7.07-7.16 (3H, m), 7.30-7.45 (2H, m), 7.47 (1 H, dd, J=4.8, 7.6Hz), 7.87 (1H, dd, J=1.6, 7.6Hz), 8.46 (1H, dd, J=1.6, 4.8Hz), 9.20 (1H, t, J=5.2Hz).

### Preparation Example A+-8. N-(4-Benzyloxy-benzyl)-6-(ethoxymethyl-amino)-nicotinamide

To a solution of 6-aminonicotinic acid (130mg, 0.941 mmol) and 4-benzyloxy-benzylamine (201 mg, 0.941 mmol) described in Preparation Example 1 in N,N-dimethylformamide (5mL), was added benzotriazol-1-yl-tris(dimethylamino)phosphonium hexafluorophosphate (624mg, 1.41mmol) and triethylamine (394µl, 2.82mmol), and the solution was stirred for 40 minutes at 80°C. Water and ethyl acetate were added to the reaction solution, which was then partitioned, and the organic layer was washed twice with water. The solvent was evaporated *in vacuo,* ethyl acetate was added to the residue, a white insoluble matter was collected by filtration, and 6-amino-N-(4-benzyloxy-benzyl)-nicotinamide (202mg, 0.606mmol, 64%) was obtained.
To a solution of the resulting 6-amino-N-(4-benzyloxy-benzyl)-nicotinamide (200mg, 0.556mmol) in ethanol (10mL) were added 5,5-dimethylimidazophospho-2,4-dione (85mg, 0.66mmol) and 37% formaldehyde aqueous solution (1mL), and the solution was stirred for 1 hour under reflux. Water and ethyl acetate were added to the reaction solution, which was then partitioned, and the organic layer was washed twice. NH silica gel was added to the organic layer, the solvent was evaporated *in vacuo* for adsorption, purification was carried out by NH silica gel column chromatography (ethyl acetate), and the title compound (95mg, 0.243mmol, 40%) was obtained as a white solid.
¹H-NMR Spectrum (CDCl₃) δ (ppm) :1.20 (3H, t, J=7.0Hz), 3.55 (2H, q, J=7.0Hz), 4.56 (2H, d, J=5.5Hz), 4.84 (2H, s), 5.07 (2H, s), 5.68 (1 H, brs), 6.14 (1 H, brs), 6.62 (1 H, d, J=8.8Hz), 6.96 (2H, d, J=8.8Hz), 7.22-7.35 (4H, m), 7.27-7.44 (3H, m), 7.93 (1 H, dd, J=2.4, 8.8Hz), 8.54 (1 H, d, J=2.4Hz).

### Preparation Example A+-9. 2-Amino-5-nitro-N-(5-phenoxy-thiophen-2-ylmethyl)-nicotinamide

2-Amino-5-nitro-nicotinic acid methyl ester (48.4mg, 0.245mmol) described in Preparation Example A-3 and lithium hydroxide monohydrate (10.3mg, 0.245mmol) were dissolved in a solvent mixture of tetrahydrofuran (1mL), methanol (0.1mL) and water (0.1mL), and the solution was stirred for 17 hours at room temperature. The solvent was evaporated *in vacuo,* and 2-amino-5-nitro-nicotinic acid was obtained as a lithium salt.
Then, the resulting lithium salt of 2-amino-5-nitro-nicotinic acid, C-(5-phenoxy-thiophen-2-yl)-methylamine (60mg, 0.29mmol), benzotriazol-1-yl-tris(dimethylamino)phosphonium hexafluorophosphate (162mg, 0.367mmol) and triethylamine (103µl, 0.735mmol) were dissolved in N,N-dimethylformamide (2.0mL), and the solution was stirred for 6 hours at room temperature. Water was added to the reaction solution, which was then extracted with ethyl acetate, and the organic layer was washed with water and brine. The solvent was evaporated *in vacuo,* the residue was purified by silica gel column chromatography (hexane : ethyl acetate = 1 : 1), and the title compound (87mg,0.24mmol,96%) was obtained as a pale yellow solid.
¹H-NMR Spectrum (DMSO-d₆) δ (ppm) : 4.49 (2H, d, J=5.5Hz), 6.50 (1 H, d, J=3.7Hz), 6.80 (1H, d, J=3.1Hz), 7.08 (2H, d, J=7.7Hz), 7.13 (1H, t, J=7.5Hz), 7.37 (2H, t, J=7.5Hz), 8.76 (1 H, d, J=2.2Hz), 8.96 (1 H, d, J=1.7Hz), 9.51 (1 H, t, J=5.5Hz).

### Preparation Example A+-10. 2,5-Diamino-N-(5-phenoxy-thiophen-2-ylmethyl)-nicotinamide

2-Amino-5-nitro-N-(5-phenoxy-thiophen-2-ylmethyl)-nicotinamide (74mg, 0.20mmol) described in Preparation Example A+-9, an iron powder (56mg, 1.0mmol) and ammonium chloride (21 mg, 0.40mmol) were suspended in a solvent mixture of ethanol (2mL) and water (0.5mL), the solution was stirred at 60°C for 3 hours, then, it was stirred at 90°C for 6 hours. The reaction solution was cooled to room temperature, then, it was filtered through Celite pad. The filtrate was evaporated in *vacuo,* the residue was purified by reverse phase high performance liquid chromatography (acetonitrile-water: mobile phase (containing 0.1% trifluoroacetic acid) was used), and the title compound (54.4mg) was obtained as a trifluoroacetic acid salt.
MS m/e (ESI) 341.26(MH⁺)

### Preparation Example A+-11. 2-Amino-6-chloro-N-(5-phenoxy-thiophen-2-ylmethyl)-nicotinamide

2-Amino-6-chloro-nicotinic acid (400mg, 2.31 mmol) described in Preparation Example A-1 was dissolved in N,N-dimethylformamide (10mL), triethylamine (0.78mL, 5.6mmol), benzotriazol-1-yloxytris(dimethylamino)phosphonium hexafluorophosphate (1.23g, 2.8mmol) and C-(5-phenoxy-thiophen-2-yl)-methylamine (572mg, 2.8mmol) described in Preparation Example 35 were added thereto, and the solution was stirred for 13 hours 30 minutes at room temperature. After the reaction was completed, the reaction solution was poured into brine, and the solution was extracted with ethyl acetate. The organic layer was dried over anhydrous magnesium sulfate, then concentrated, the resulting residue was purified by silica gel column chromatography (ethyl acetate : hexane = 1 : 1), and the title compound (380mg, 1.05mmol, 46%) was obtained.
¹H-NMR Spectrum (DMSO-d₆) δ (ppm) : 4.47 (2H, d, J=6.0Hz), 6.50 (1H, d, J=4.0Hz), 6.64 (1 H, d, J=8.0Hz), 6.78 (1 H, d, J=4.0Hz), 7.07-7.17 (3H, m), 7.36-7.41 (2H, m), 7.53 (2H, brs), 7.93 (1 H, d, J=8.0Hz), 9.11 (1H, t, J=6.0Hz).

### Preparation Example A+-12. 2-Amino-6-(2-amino-ethylamino)-N-(5-phenoxythiophen-2-ylmethyl)-nicotinamide

2-Amino-6-chloro-N-(5-phenoxy-thiophen-2-ylmethyl)-nicotinamide (150mg, 0.417mmol) described in Preparation Example A+-11, ethane-1,2-diamine (418µl, 6.25mmol) were dissolved in a mixture solution of dimethylsulfoxide (2mL) and N, N-diisopropylethylamine (1 mL), and the solution was stirred at 120°C for 15 hours. The reaction solution was cooled to room temperature, water was added, then extracted with ethyl acetate and tetrahydrofuran, the organic layer was washed with brine and dried over anhydrous magnesium sulfate. The solvent was evaporated in *vacuo,* tetrahydrofuran and NH silica gel were added to the residue, the solvent was evaporated *in vacuo* adsorption, purification was carried out by NH silica gel column chromatography (ethyl acetate : methanol = 10 : 1), and the title compound (95mg,0.25mmo1,59%) was obtained as a white solid.
¹H-NMR Spectrum (DMSO-d₆) δ (ppm) : 2.64 (2H, t, J=6.4Hz), 3.16-3.22 (2H, m), 4.41 (2H, d, J=5.7Hz), 5.70 (1 H, d, J=8.6Hz), 6.48 (1 H, d, J=3.8Hz), 6.67 (1H, brs), 6.72 (1 H, d, J=3.8Hz), 7.02 (2H, s), 7.08 (2H, d, J=8.6Hz), 7.14 (1 H, t, J=7.3Hz), 7.38 (2H, dd, J=7.3, 8.6Hz), 7.59 (1 H, d, J=8.6Hz), 8.38 (1 H, t, J=5.7Hz).

### Preparation Example A+-13. 2-Amino-6-(2-(4-nitro-phenylamino)-ethylamino)-N-(5-phenoxy-thiophen-2-ylmethyl)-nicotinamide

2-Amino-6-(2-amino-ethylamino)-N-(5-phenoxy-thiophen-2-ylmethyl)-nicotinamide (25mg, 65µmol) described in Preparation Example A+-12 , 4-fluoronitrobenzene (7.6µl, 71µmol) and N,N-diisopropylethylamine (22.7µl, 130µmol) were dissolved in dimethylsulfoxide (0.5mL), the solution was stirred for 3. 5 hours at room temperature, and then, the solution was stirred for 15.5 hours at 70°C. The reaction solution was cooled to room temperature, water was added, the solution was extracted with ethyl acetate, the organic layer was washed with brine and dried over anhydrous magnesium sulfate. The solvent was evaporated in *vacuo,* residue was purified by reverse phase high performance liquid chromatography (mobile phase:acetonitrile-water (containing 0.1% trifluoroacetic acid) was used), and the title compound (23mg) was obtained as a trifluoroacetic acid salt.
MS m/e (ESI) 505.37(MH⁺)

### Preparation Example A+-14. 2-Amino-6-(1-ethoxyvinyl)-N-(5-phenoxy-thiophen-2-ylmethyl)-nicotinamide

1-Ethoxyvinyl(tri-*n*-butyl)tin (0.47mL, 1.4mmol) was added to a mixture of 2-amino-6-chloro-N-(5-phenoxy-thiophen-2-ylmethyl)-nicotinamide (170mg, 0.46mmol) described in Preparation Example A+-11, tetrakis(triphenylphosphine)palladium(0) (54mg, 0.046mmol) and xylene (5mL), and the solution was stirred for 2.5 hours at 130°C. After cooling, water and ethyl acetate were added to the reaction solution for extraction, and the solution was washed with brine. The solvent was evaporated *in vacuo,* then, the residue was purified by NH silica gel column chromatography (hexane ethyl acetate = 2 : 1), and the title compound (150mg, 0.38mmol, 82%) was obtained as a pale yellow solid.
¹H-NMR Spectrum (DMSO-d₆) δ (ppm) :1.42 (3H, t, J=7.0Hz), 3.93 (2H, q, J=7.0Hz), 4.35 (1H, d, J=1.8Hz), 4.65 (2H, d, J=5.3Hz), 5.37 (1H, d, J=1.8Hz), 6.30-6.32 (3H, m), 6.39 (1H, d, J=3.7Hz), 6.74 (1H, d, J=3.8Hz), 7.00 (1H, d, J=8.1 Hz), 7.08-7.13 (3H, m), 7.30-7.35 (2H, m), 7.59 (1 H, d, J=8.1Hz).

### Preparation Example A+-15. 2-Amino-N-(5-phenoxy-thiophen-2-ylmethyl)-6-tributylstanyl-nicotinamide

To a mixture of 2-amino-6-chloro-N-(5-phenoxy-thiophen-2-ylmethyl)-nicotinamide (1.1g, 3.0mmol) described in Preparation Example A+-11, tetrakis(triphenylphosphine)palladium(0) (170mg; 0.15mmol) and xylene (5mL) was added bis(tri *n*-butyl tin) (9.1 mL, 18mmol), and the solution was stirred at 135°C for 2 hours. After cooling, the reaction solution was directly purified by neutral silica gel column chromatography (hexane : ethyl acetate = 3 : 1), then, the resulting crude product was washed with hexane cooled to 0°C to obtain the title compound (600mg, 0.98mmol, 33%) as a colorless oil.
¹H-NMR Spectrum (CDCl₃) δ (ppm) : 0.86-0.90 (9H, m), 1.05-1.09 (6H, m), 1.27-1.36 (6H, m), 1.50-1.58 (6H, m), 4.64 (1 H, d, J=5.5Hz), 6.26-6.30 (4H, m), 6.38 (1H, d, J=3.8Hz), 6.73-6.74 (2H, m), 7.08-7.12 (3H, m), 7.31-7.36 (3H, m).

### Preparation Example A+-16. 2-Amino-5-iodo-N-(5-phenoxy-thiophene-2-ylmethyl)-nicotinamide

2-Amino-N-(5-phenoxy-thiophen-2-ylmethyl)-nicotinamide (250mg, 0.768mmol) obtained from 2-aminonicotinic acid and C-(5-phenoxy-thiophen-2-yl)methylamine described in Preparation Example 24 according to an analogous method to Preparation Example A+-5, and N-iodosuccinimide (190mg, 0.845mmol) were dissolved in tetrahydrofuran (5mL), and the solution was stirred for 16 hours at room temperature. An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* residue was purified by NH silica gel column chromatography (hexane : ethyl acetate = 1 : 1), and title compound (45mg, 0.10mmol, 13%) was obtained as a pale yellow solid.
¹H-NMR Spectrum (DMSO-d₆) δ (ppm) : 4.45 (2H, d, J=5.7Hz), 6.49 (1H, d, J=3.7Hz), 6.77 (1H, d, J=3.8Hz), 7.08 (2H, d, J=7.7Hz), 7.13 (1H, t, J=7.3Hz), 7.23 (2H, s), 7.37 (2H, t, J=7.3Hz), 8.15 (1 H, d, J=2.2Hz), 8.21 (1 H, d, J=1.8Hz), 9.13 (1 H, d, J=5.7Hz).

### Preparation Example A+-17. 2-Amino-N-(3-hydroxvbenzyl)-nicotinamide

The title compound (0.63g, 2.6mmol, 53%) was obtained as a white solid from 3-aminomethylphenol (0.60g, 4.9mmol) described in Preparation Example 130 and 2-aminonicotinic acid (0.67g, 4.9mmol) according to an analogous method to Preparation Example Q+-1.
¹H-NMR Spectrum (DMSO-d₆) δ (ppm) : 4.34 (2H, d, J=5.9Hz), 6.56-6.61 (2H, m), 6.69-6.71 (2H, m), 7.06-7.10 (3H, m), 7.93 (1H, dd, J=1.8, 7.8Hz), 8.06 (1H, dd, J=1.8, 4.8Hz), 8.91 (1 H, t, J=6.0Hz), 9.30 (1 H, s).

### Preparation Example A+-18. 2-Amino-N-(4-benzyloxy-benzyl)-6-chloro-nicotinamide

The title compound (0.43g, 1.2mmol, 28%) was obtained as a white solid from 14-benzyloxy-benzylamine (0.90g, 4.2mmol) described in Preparation Example and 2-amino-6-chloro-nicotinic acid (1.5g, 8.4mmol) described in Preparation Example A-1 (or A-4) according to an analogous method to Preparation Example Q+-1.
¹H-NMR Spectrum (DMSO-d₆) δ (ppm) : 4.33 (2H, d, J=5.7Hz), 5.06 (2H, s), 6.61 (1 H, d, J=8.1 Hz), 6.94 (2H, d, J=8.6Hz), 7.20 (2H, d, J=8.4Hz), 7.28-7.31 (1 H, m), 7.34-7.38 (2H, m), 7.41 (2H, d, J=7.5Hz), 7.49 (2H, brs), 7.95 (1 H, d, J=8.1Hz), 8.92-8.95 (1 H, m).

### Preparation Example A+-19. 5-Bromo-thiophene-2-carbaldehyde oxime

To a mixture of 2-bromo-5-formylthiophene (2.5mL, 21 mmol) and pyridine (25mL) was added hydroxylamine hydrochloride (2.2g, 32mmol) on an ice bath, then, the solution was stirred overnight at room temperature. This reaction mixture was concentrated *in vacuo,* then, partitioned with water (50mL), ethyl acetate (50mL) then 1 N hydrochloric acid solution (50mL). The organic layer was separated, sequentially washed with an aqueous solution of saturated sodium bicarbonate and brine, and then, dried over anhydrous magnesium sulfate. The solvent was evaporated *in vacuo,* the residue was washed with heptane-ethyl acetate (30:1), and the title compound (4.3g, 21 mmol, 98%) was obtained as a light brown solid.
¹H-NMR Spectrum (CDCl₃) δ (ppm) : 7.07(1H, d, J=4.0Hz), 7.11(1H, d, J=4.0Hz), 7.62(1 H, s), 8.35(1 H, br s).

### Preparation Example A+-20. 5-Bromo-thiophene-2-carbonitrile

To a mixture of 5-bromo-thiophene-2-carbaldehyde oxime described in Preparation Example A+-19 (1.3g, 6.2mmol) and tetrahydrofuran (15mL) were acetic acid (1.4mL, 25mmol) and acetic anhydride (1.5mL, 15mmol) at room temperature, then, stirred at 50°C for 2 hours, and further stirred at 70°C for 8 hours. After cooling this reaction mixture, the solution was concentrated *in vacuo.* The residue was purified by NH-silica gel column chromatography (heptane : ethyl acetate = 8 : 1), and the title compound (1.0g, 5.4mmol, 88%) was obtained as a colorless solid.
¹H-NMR Spectrum (CDCl₃) δ(ppm) : 7.11 (1 H, d, J=4.0Hz), 7.40(1 H, d, J=4.0Hz).

### Preparation Example A+-21.C-(5-Bromo-thiophen-2-yl)-methylamine

To a mixture of lithium aluminum hydride (1.6g, 41 mmol) and tetrahydrofuran (45mL) was aluminum chloride (6.1 g, 46mmol) on an ice bath, then, the solution was stirred for 1 hour at room temperature. The reaction solution was cooled to -20°C, a solution of 5-bromo-thiophene-2-carbonitrile described in Preparation Example A+-20 (4.3g, 25mmol) in tetrahydrofuran (5mL) was added dropwise at the same temperature. After stirring at 2°C for 20 minutes, the reaction solution was cooled to -10°C, and while maintaining the internal temperature at 0°Cor lower, tetrahydrofuran (300mL) and 28% aqueous ammonia solution (5mL) were added thereto. Anhydrous magnesium sulfate was added to the reaction solution, which was then filtered using a filter paper, and then, concentrated *in vacuo.* The residue was purified by NH-silica gel column chromatography (ethyl acetate), and the title compound (840mg, 4.4mmol, 85%) was obtained as a colorless oil.
¹H-NMR Spectrum (CDCl₃) δ (ppm) : 3.99(2H, s), 6.67(1 H, d, J=3.7Hz), 6.88(1 H, d, J=3.7Hz).

### Preparation Example A+-22. 2-Amino-N-(5-bromo-thiophen-2-ylmethyl)-6-methoxymethyl-nicotinamide

To a solution of C-(5-bromo-thiophen-2-yl)-methylamine (250mg, 1.3mmol) in N,N-dimethylformamide (5mL) were added tiethylamine (0.54mL, 3.9mmol), 2-amino-6-methoxymethyl-nicotinic acid (240mg, 1.3mmol) described in Preparation Example A-11, and benzotriazol-1-yloxytris(dimethylamino)phosphonium hexafluorophosphate (690mg, 1.6mmol) sequentially on an ice bath, then, the solution was stirred for 2 days at room temperature. The reaction solution was partitioned in water and ethyl acetate. The organic layer was separated, the solvent was evaporated *in vacuo.* The residue was purified by silica gel column chromatography (heptane : ethyl acetate = 4 : 1), and the title compound (370mg, 1.0mmol, 79%) was obtained as a white solid.
¹H-NMR Spectrum (CDCl₃) δ (ppm) : 3.46(3H, s), 4.40(2H, s), 4.67(2H, d, J=5.7Hz), 6.33(1 H, br s), 6.38(2H, br s), 6.71 (1 H, d, J=7.9Hz), 6.79(1 H, d, J=3.7Hz), 6.91 (1 H, d, J=3.9Hz), 7.59(1 H, d, J=7.9Hz).

### Preparation Example A+-23. 2-Amino-N-(4-bromo-benzyl)-6-methoxymethyl-nicotinamide

The title compound (1.9g, 5.4mmol, 86%) was obtained as a pale yellow solid from 2-amino-6-methoxymethyl-nicotinic acid (1.2g, 6.3mmol) described in Preparation Example A-11 and 4-bromobenzylamine hydrochloride (1.5g, 6.9mmol) according to an analogous method to Preparation Example A+-22.
¹H-NMR Spectrum (CDCl₃) δ (ppm) : 3.46(3H, s), 4.40(2H, s), 4.55(2H, d, J=5.7Hz), 6.30(1 H, br s), 6.39(2H, br s), 6.70(1 H, d, J=7.9Hz), 7.22(2H, d, J=8.2Hz), 7.48(2H, d, J=8.4Hz), 7.60(1 H, d, J=7.9Hz).

### Preparation Example A+-24. 2-Amino-N-(4-((Z)-2-ethoxy-vinyl)-benzyl)-6-methoxymethyl-nicotinamide

A mixture of (2-ethoxy-vinyl)-tributyl-stannane (37mg, 0.10mmol) synthesized according to WO02/018368, 2-amino-N-(4-bromo-benzyl)-6-methoxymethyl-nicotinamide (30mg, 0.086mmol) described in Preparation Example A+-23, tri-o-tolylphosphine (6.5mg, 0.021 mmol), palladium acetate (0.96mg, 0.0043mmol), tetrabutylammonium chloride (24mg, 0.086mmol) and N-methylpyrrolidinone (1mL) was stirred at 125°C for 1 hour. The reaction solution was allowed to cool, an aqueous solution of potassium fluoride was added on an ice bath, the solution was filtered through Celite pad. The filtrate was partitioned with ethyl acetate. The organic layer was separated, washed with brine, then, evaporated *in vacuo.* The residue was purified by silica gel column chromatography (heptane : ethyl acetate = 1 : 2), and the title compound (12mg, 0.035mmol, 35%) was obtained as a colorless solid.
¹H-NMR Spectrum (CDCl₃) δ (ppm) :1.36(3H, d, J=7.1Hz), 3.45(3H, s), 3.99(2H, q, J=7.1Hz), 4.39(2H, s), 4.55(2H, d, J=5.5Hz), 5.21 (1 H, d, J=7.0Hz), 6.21-6.23(2H, m), 6.40(2H, br s), 6.68(1H, d, J=7.9Hz), 7.26(2H, d, J=8.3Hz), 7.57-7.59(3H, m).

### Preparation Example A+-25. 2-Amino-6-methoxymethyl-N-(3-hydroxybenzyl)-nicotinamide

2-Amino-6-methoxymethylcotinic acid (500mg, 2.74mmol), 3-hydroxybenzylamine (405mg, 3.29mmol), benzotriazol-1-yloxytris(dimethylamino)phosphonium hexafluorophosphate (1.33mg, 3.01mmol) and triethylamine (555mg, 5.48mmol) were added to dimethylsulfoxide (20mL), and the solution was stirred for 15 minutes at 60°C. Water and ethyl acetate were added to the reaction mixture, which was then partitioned. The aqueous layer was extracted once with ethyl acetate, the ethyl acetate layers were combined and the solvent was evaporated *in vacuo.* The residue was purified by silica gel column chromatography (hexane:ethyl acetate=1:1, then ethyl acetate). The resulting residue was purified by NH silica gel column chromatography (ethyl acetate). The resulting residue was solidified from hexane-ethyl acetate, and the title compound (490mg, 1.71 mmol, 62.2%) was obtained as a pale yellow solid.
¹H-NMR Spectrum (DMSO-d₆) δ (ppm) : 3.34 (3H, s), 4.30 (2H, s), 4.35 (2H, d, J=6.0 Hz), 6.61 (2H, d, J=8.0 Hz), 6.70 (1 H, s), 6.71 (1 H, d, J=8.0 Hz), 7.10 (1 H, dd, J=8.0, 8.0 Hz), 7.13 (2H, brs), 7.99 (1 H, d, J=8.0 Hz), 8.91 (1 H, t, J=6.0 Hz), 9.31 (1 H, s).

### Preparation Example A+-26. 2-Amino-6-methoxymethyl-N-(4-hydroxybenzyl)-nicotinamide

The title compound (506mg, 1.76mmol, 64.3%) was obtained as a pale yellow solid from 2-amino-6-methoxymethylcotinic acid (500mg, 2.74mmol) and 4-hydroxybenzylamine (506mg, 4.11mmol) according to an analogous method to Preparation Example A+-25.
¹H-NMR Spectrum (DMSO-d₆) δ (ppm) : 3.34 (3H, s), 4.29 (2H, s), 4.30 (2H, d, J=6.0), 6.59 (1 H, d, J=7.6 Hz), 6.70 (2H, d, J=7.4 Hz), 7.10 (2H, d, J=7.4 Hz), 7.12 (2H, brs), 7.95 (1 H, d, J=7.6 Hz), 8.84 (1 H, t, J=6.0 Hz), 9.27 (1 H, s).

### Preparation Example AA-1. 3,5-Diamino-pyrazine-2-carboxylic acid methyl ester

To a solution of 3,5-diamino-6-chloro-pyrazine-2-carboxylic acid methyl ester (8.00g, 39.5mmol) in tetrahydrofuran (150mL) were added tetrakis(triphenylphosphine)palladium(0) (2.28g, 1.98mmol), formic acid (2.24mL, 59.3mmol) and triethylamine (16.5mL, 119mmol) at 0°C under nitrogen atmosphere, then, the solution was stirred at 125°C for 12 hours. The reaction solution was cooled to room temperature, and a solid precipitated. This solid was collected by filtration, and the title compound (10.7g, quantitatively) was obtained as a crude product of a white solid.
¹H-NMR Spectrum (DMSO-d₆) δ (ppm) : 3.70(3H, s), 6.95(2H, brs), 7.21 (1H, s).

### Preparation Example AA-2. 3,5-Diamino-pyrazine-2-carboxylic acid

Lithium hydroxide monohydrate (2.50g, 59.5mmol) was added to 3,5-diamino-pyrazine-2-carboxylic acid methyl ester (10.0g, 59.5mmol) described in Preparation Example AA-1 in a mixture solvent of tetrahydrofuran (100mL), methanol (10mL) and water (10mL) at room temperature. The solution was stirred at room temperature for 17 hours, then, an aqueous solution of 5N sodium hydroxide (15mL) was added thereto, followed by further stirring for 4.5 hours under reflux. The reaction solution was cooled to room temperature, and partitioned in 5N hydrochloric acid solution and ethyl acetate. The organic layer was separated, washed with brine, and then, dried over anhydrous magnesium sulfate. The organic layer was filtered, then, the solvent was evaporated *in vacuo,* and the title compound (3.34g, 36%) was obtained as a crude product of a white solid.
¹H-NMR Spectrum (DMSO-d₆) δ (ppm) : 6.93(2H, brs), 7.20(1 H, s).

### Preparation Example B-1 4-Amino-5-hydroxy-carbonyl-2-n-propylamino-pyrimidine

4-Amino-2-chloro-5-cyano-pyrimidine (300mg, 1.94mmol) and *n*-propyl amine (5g, 84.6mmol) were mixed and stirred for 10 minutes at 60°C. The reaction solution was directly purified by silica gel column chromatography, and a 2-propylamino compound (300mg, 1.69mmol, 101 %) was obtained as a brown solid. This solid was suspended in concentrated sulfuric acid (3mL) and water (3mL), and the solution was stirred for 1.5 hours at 130°C. This mixture was alkalinized with an aqueous solution of saturated sodium bicarbonate, then, the aqueous layer was washed with ethyl acetate. Then, this aqueous layer was neutralized with citric acid, and extracted with a mixture solvent of acetate-methanol. This organic layer was separated, then, the solvent was evaporated, and the title compound (44mg, 0.224mmol, 12%) was obtained as a white solid.
¹H-NMR Spectrum (DMSO-d₆) δ (ppm) : 0.87 (3H, t, J=7.2 Hz), 1.49 (2H, qt, J=7.2, 7.2 Hz), 3.18 (2H, q, J=7.2Hz), 6.98 (2H, bs), 8.14 (1 H, bs), 8.35 (1 H, s).

### Preparation Example C-1 Potassium salt of 2-Cyano-3-hydroxy-acrylic acid ethyl ester

To a solution of potassium ethoxide (9.8g, 116mmol) in ethanol (180mL) were added a solution of cyano ethyl acetate ester (13.2g, 117mmol) and formic acid ethyl ester (30g, 405mmol) in ethanol (20mL), and the solution was stirred for 2 hours under reflux. The reaction solution was allowed to room temperature, the precipitated solid was collected by filtration and dried, and the title compound (18g, 100mmol, 85%) was obtained.
¹H-NMR Spectrum (DMSO-d₆) δ (ppm) :1.13 (3H, t, J=7.2 Hz), 3.96 (2H, q, J=7.2Hz), 9.18 (1H, s).

### Preparation Example C-2. 1-(Pyrrolidino)-2-(2-carbethoxy-2-cyanoethylene)cyclopentene

Potassium salt of 2-cyano-3-hydroxy-acrylic acid ethyl ester described in Preparation Example C-1 (18g, 0.1mol) was dissolved in dichloromethane (80mL), phosphorus pentachloride (20.9g, 0.1mol) was added, and the solution was stirred for 2 hours under reflux. After the reaction was completed, the residue obtained by evaporating dichloromethane was subjected to distillation *in vacuo,* and ethyl(chloromethylene)cyanoacetate (9.5g, 56mmol) was obtained.
To a solution of 1-pyrrolidinocyclopentene (10.2g, 74mmol) and triethylamine (10mL, 72mmol) in dichloromethane (200mL) was added a solution of the resulting ethyl(chloromethylene)cyanoacetate (9.5g, 56mmol) in dichloromethane (20mL) dropwise while stirring at from -20°C to -25°C. The solution was stirred at room temperature for 50 minutes, water (20mL) was added thereto, followed by further stirring for 5 minutes. The reaction mixture was partitioned, the organic layer was dried over anhydrous magnesium sulfate, then concentrated, and the title compound (6g, 23mmol, 23%) was obtained.
¹H-NMR Spectrum (DMSO-d₆) δ (ppm) :1.19 (3H, t, J=7.2 Hz), 1.76-1.86 (2H, m), 1.86-2.04 (4H, m), 2.73 (2H, t, J=7.6 Hz), 2.88 (2H, t, J=7.2 Hz), 3.60-3.71 (4H, m), 4.09 (2H, q, J=7.2Hz), 7.97(1H, brs).

### Preparation Example C-3. 1-Amino-2-(2-carbethoxy-2-cyanoethylene)cyclopentene

1-(Pyrrolidino)-2-(2-carbethoxy-2-cyanoethylene)cyclopentene (6g, 23mmol) described in Preparation Example C-2 was dissolved in ethanol saturated with ammonia (75mL; was saturated at room temperature using ammonia gas), and the solution was stirred for 19 hours at room temperature. The reaction solution was concentrated and the title compound (4.8g, 23mmol, quantitatively) was obtained.
¹H-NMR Spectrum (DMSO-d₆) δ (ppm) :1.21 (3H, t, J=7.2 Hz), 1.74-1.83 (2H, m), 2.48-2.54 (2H, m), 2.72-2.78 (2H, m), 4.12 (2H, q, J=7.2Hz), 8.09-8.47 (1 H, brs).

### Preparation Example C-4. 2-Amino-6,7-dihydro-5H-cyclopenta[b]pyridine-3-carboxylic acid ethyl ester

1-Amino-2-(2-carboethoxy-2-cyanoethylene)cyclopentene (0.8g, 3.9mmol) described in Preparation Example C-3 was dissolved in ethanol (27mL), sodium ethoxide (0.12g, 1.8mmol) was added thereto, followed by stirring for 16 hours under reflux. The reaction mixture was allowed to room temperature, poured into brine, and the solution was extracted with ethyl acetate. The organic layer was dried over anhydrous magnesium sulfate, then, the residue obtained by concentration was purified by silica gel column chromatography (ethyl acetate : hexane = 1 : 2), and the title compound (0.63g, 3.1mmol, 79%) was obtained.
¹H-NMR Spectrum (CDCl₃) δ (ppm) : 1.38 (3H, t, J=7.2Hz), 2.04-2.13 (2H, m), 2.79-2.88 (4H, m), 4.32 (2H, q, J=7.2 Hz), 6.16-6.32 (2H, brs), 7.96 (1 H, s).

### Preparation Example C-5. 2-Amino-6,7-dihydro-5H-cyclopenta[b]pyridine-3-carboxylic acid

2-Amino-6,7-dihydro-5*H*-cyclopenta[b]pyridine-3-carboxylic acid ethyl ester (0.2g, 0.97mmol) described in Preparation Example C-4 was dissolved in ethanol (15mL), an aqueous solution of 1 N sodium hydroxide (7.5mL) was added thereto, followed by heating at 100°C for 30 minutes. The reaction solution was allowed to room temperature, then cooled on an ice bath, and neutralized with 1 N hydrochloric acid. The precipitated solid was collected by filtration, rinsed with water, then dried, and the title compound (143mg, 0.8mmol, 83%) was obtained. ¹H-NMR Spectrum (DMSO-d₆) δ (ppm) :1.94-2.03 (2H, m), 2.71-2.76 (4H, m), 7.84 (1H, s).

### Preparation Example D-1. [1,5]Naphthylidine-2-carboxylic acid

5-Amino-2-chloro pyridine (10g, 0.078mol) and oxalacetic acid diethyl ester (14.7g, 0.078mol) were stirred at 90°C for 7 hours. The reaction solution was allowed to room temperature, ethyl acetate was added thereto, the precipitated yellow solid was removed by filtration, and the filtrate was concentrated. The residue was purified by silica gel column chromatography (hexane : ethyl acetate), and 2-(6-chloro-pyridin-3-ylamino)-buto-2-enedioic acid diethyl ester (4.8g, 21 %) was obtained as a yellow oil.
The resulting 2-(6-chloro-pyridin-3-ylamino)-buto-2-enedioic acid diethyl ester (4.8g, 16.1 mmol) was added to Dowtherm A (Dowtherm A; trademark) (300mL), and the solution was stirred at 210°C for 5 hours. The reaction solution was allowed to room temperature, hexane was added thereto, the precipitated solid was collected, washed with hexane, then, dried *in vacuo* to obtain 6-chloro-4-hydroxy-[1,5]naphthylidine-2-carboxylic acid ethyl ester (1.38g, 34%) as a pale brown solid.
To the resulting 6-chloro-4-hydroxy-[1,5]naphthylidine-2-carboxylic acid ethyl ester (502mg, 1.99mmol) was added thionyl chloride (10mL), and the solution was stirred for 7 hours under reflux. Excess thionyl chloride was evaporated *in vacuo,* 4,6-dichloro-[1,5]naphthylidine-2-carboxylic acid ethyl ester (522mg, 97%) was obtained as a pale brown solid. Under nitrogen atmosphere, a portion of the resulting solid (200mg, 0.738mmol) was dissolved in dimethylsulfoxide (30mL), tetrakis(triphenylphosphine)palladium(0) (171mg, 0.148mmol) and formic acid sodium (251 mg, 3.69mmol) were added thereto, followed by stirring at 100°C for 4 hours. The reaction solution was allowed to room temperature, ethyl acetate and water were added for partitioning, the organic layer was washed with water and dried over anhydrous magnesium sulfate. The solvent was evaporated, then, the residue was purified by silica gel column chromatography (hexane : ethyl acetate), and [1,5]naphthylidine-2-carboxylic acid ethyl ester (49mg, 33%) was obtained as a colorless solid.
The resulting solid was dissolved in methanol (1.0mL), an aqueous solution of 1N sodium hydroxide (0.3mL) was added thereto, followed by stirring at room temperature for 30 minutes. Water was added to the reaction solution, the pH was adjusted from 3 to 4 using 1 N hydrochloric acid, the solution was extracted with ethyl acetate, dried over anhydrous magnesium sulfate, and the solvent was evaporated to obtain the title compound (29mg, 69%) as a white solid.

### Preparation Example E-1 Quinoline-6-carboxylic acid cyanomethyl-amide

To a solution of quinoline-6-carboxylic acid (500mg, 2.9mmol) and amino acetonitrile hydrochloride (320mg, 3.4mmol) in N,N-dimethylformamide (10mL) were added benzotriazol-1-yl-tris(dimethylamino)phosphonium hexafluorophosphate (1.5g, 3.48mmol) and triethylamine (1.2mL, 8.7mmol), and the solution was stirred at 60°C for 10 minutes. Ethyl acetate and water was added to the reaction solution, which was then partitioned, the organic layer was washed twice with water. Silica gel was added to the organic layer, the solvent was evaporated *in vacuo* for adsorption, purified by NH silica gel column chromatography (ethyl acetate), and the title compound (420mg, 2.0mmol, 68.9%) was obtained as a light brown solid.
¹H-NMR Spectrum (DMSO-d₆) δ (ppm) : 4.43 (2H, d, J=5.6Hz), 7.65 (1H, dd, J=4.0, 8.4Hz), 8.14 (1H, d, J=8.8Hz), 8.18-8.22 (1H, m), 8.30-8.35 (1H, m), 8.58 (1H, d, J=1.2Hz), 9.02-9.05 (1 H, m), 9.49 (1 H, t, J=5.6Hz).

### Preparation Example E+-1 Quinoline-6-carboxylic acid 4-hydroxybenzylamide

Trifluoroacetic acid (5mL) and thioanisole (3 drops) were added to quinoline-6-carboxylic acid 4-benzyloxybenzylamide (2.67g, 7.25mmol) synthesized in Reference Example E-8, the solution was stirred at room temperature for 14 hours, followed by stirring at 50°C for 4 hours, lastly, and the solution was stirred at 70°C for 3 hours. The reaction solution was allowed to room temperature, then, was neutralized with an aqueous solution of saturated sodium bicarbonate, extracted with ethyl acetate and dried over anhydrous magnesium sulfate. The solvent was evaporated, the residue was purified by NH silica gel column chromatography (ethyl acetate), and the title compound (433mg, 22%) was obtained as a colorless solid.

### Preparation Example E+-2. Quinoline-6-carboxylic acid (5-bromo-furan-2-ylmethyl)-amide

The title compound (1.0g, 3.0mmol, 75.5%) was obtained as a white solid from C-(5-bromo-furan-2-yl)-methylamine (700mg, 4.0mmol) and quinoline-6-carboxylic acid (700mg, 4.0mmol) according to an analogous method to Reference Example Q-6.
¹H-NMR Spectrum (DMSO-d₆) δ (ppm) : 4.49 (2H, d, J=5.6Hz), 6.38-6.41 (1H, m), 6.50 (1 H, d, J=3.6Hz), 7.60 (1 H, dd, J=4.0, 8.4Hz), 8.06 (1 H, d, J=8.8Hz), 8.17 (1 H, dd, J=2.0, 8.8Hz), 8.44-8.48 (1H, m), 8.52 (1H, d, J=2.0Hz), 8.97 (1H, dd, J=1.6, 4.0Hz), 9.23 (1 H, t, J=5.6Hz).

### Preparation Example E+-3 Quinoline-6-carboxylic acid 4-benzylamino-benzylamide

4-Benzylamino-benzonitrile (472mg, 2.27mmol) described in Preparation Example 89 was dissolved at 0°C in tetrahydrofuran (20mL), and lithium aluminum hydride (430mg, 11.3mmol) was added thereto. The solution was stirred overnight at room temperature, then, at 0°C, water (430µl), an aqueous solution of 5N sodium hydroxide (430µl) and water (1.29mL) were sequentially added to the solution. The reaction solution was filtered through Celite pad, the solvent was then evaporated in *vacuo,* (4-aminomethyl-phenyl)-benzylamine (475mg, 2.24mmol, 99%) was obtained as an oil.
The resulting (4-aminomethyl-phenyl)-benzylamine (162mg, 0.763mmol), quinoline-6-carboxylic acid (132mg, 0.736mmol), benzotriazol-1-yl-tris(dimethylamino)phosphonium hexafluorophosphate (506mg,1.14mmol) and triethylamine (319µl, 2.29mmol) were dissolved in N,N-dimethylformamide (4.0mL), and the solution was stirred for 2 hours at room temperature. Water was added to the reaction solution, which was then extracted with ethyl acetate, the organic layer was washed with water and brine and dried over anhydrous magnesium sulfate. The solvent was evaporated *in vacuo,* the residue was purified by NH silica gel column chromatography (hexane : ethyl acetate = 1 : 1), and the title compound (224mg, 0.610mmol, 80%) was obtained as a white solid.
¹H-NMR Spectrum (DMSO-d₆) δ (ppm) : 4.23 (2H, d, J=6.0Hz), 4.33 (2H, d, J=6.0Hz), 6.18 (1 H, t, J=6.1 Hz), 6.51 (2H, d, J=8.6Hz), 7.03 (2H, d, J=8.6Hz), 7.18 (1H, t, J=7.0Hz), 7.25-7.34 (4H, m), 7.58 (1H, dd, J=4.1, 8.3Hz), 8.04 (1H, d, J=8.8Hz), 8.16 (1H, dd, J=1.8, 9.0Hz), 8.43 (1 H, d, J=7.0Hz), 8.49 (1H, d, J=2.0Hz), 8.95 (1 H, dd, J=1.8, 5.0Hz), 9.04 (1 H, t, J=5.5Hz).

### Preparation Example E+-4 Quinoline-6-carboxylic acid 3-hydroxybenzylamide

Thioanisole (1.7mL, 14mmol) was added to a mixture of quinoline-6-carboxylic acid 3-benzyloxybenzylamide (1.3g, 3.6mmol) and trifluoroacetic acid(8mL) on an ice bath, and the solution was stirred for 4 hours at room temperature. The solvent was evaporated *in vacuo,* then water, an aqueous solution of saturated sodium bicarbonate, ethyl acetate and tetrahydrofuran were added to the residue for extraction, the organic layer was washed with brine, then, dried over anhydrous magnesium sulfate. The solvent was evaporated *in vacuo,* recrystallized from ethyl acetate-methanol, and the title compound (0.64g, 2.3mmol, 64%) was obtained as a white crystal.
¹H-NMR Spectrum (DMSO-d₆) δ (ppm) : 4.45 (2H, d, J=5.9Hz), 6.60-6.63 (1 H, m), 6.75-6.77 (2H, m), 7.10 (1H, t, J=8.1Hz), 7.60 (1H, dd, J=4.2, 8.2Hz), 8.07 (1H, d, J=8.8Hz), 8.20 (1H, dd, J=2.0, 8.8Hz), 8.45-8.47 (1H, m), 8.54 (1H, d, J=1.8Hz), 8.97 (1H, dd, J=1.7, 4.2Hz), 9.23 (1H, t, J=5.8Hz), 9.33 (1H, s).

### Preparation Example E+-5. Quinoline-6-carboxylic acid 4-phenylethynyl-benzylamide

Quinoline-6-carboxylic acid 4-bromobenzylamide(1.3g, 68%) was obtained from quinoline-6-carboxylic acid (1.0g, 5.8mmol) and 4-bromobenzylamine hydrochloride (1.3g, 5.8mmol) according to an analogous method to Preparation Example A+-5 (with the proviso that reaction was carried out at 80°C).
N,N-diisopropylethylamine (0.31mL, 1.8mmol) was added to a mixture of the resulting quinoline-6-carboxylic acid 4-bromobenzylamide (200mg, 0.59mmol), ethynylbenzene(0.077mL, 0.70mmol), copper(I) iodide (catalytic amount), tetrakis(triphenylphosphine)palladium(0) (68mg, 0.059mmol) and N-methylpyrrolidinone (4mL), and the solution was stirred at 100°C for 30 minutes, and at 120°C for 50 minutes. After cooling, water, ethyl acetate, tetrahydrofuran and 29% aqueous ammonia solution were added to the reaction mixture for extraction, the organic layer was washed with brine, then, the solvent was evaporated *in vacuo.* The residue was purified by silica gel column chromatography (hexane : ethyl acetate = 1 : 4), then, the resulting crudely purified product was washed with diethyl ether to obtain the title compound (50mg, 0.14mmol, 24%) as a white solid.
¹H-NMR Spectrum (DMSO-d₆) δ (ppm) : 4.57 (2H, d, J=5.7Hz), 7.39-7.41 (5H, m), 7.52 (4H, d, J=6.8Hz), 7.60 (1 H, dd, J=3.8, 8.1 Hz), 8.08 (1 H, d, J=8.6Hz), 8.21 (1 H, d, J=8.8Hz), 8.47 (1 H, d, J=7.9Hz), 8.56 (1 H, s), 8.97 (1H, d, J=4.0Hz), 9.33 (1 H, brs).

### Preparation Example E+-6. Quinoline-6-carboxylic acid 4-[1,3]dioxolan-2-yl-benzylamide

The title compound (1.31g, 3.92mmol, 77%) was obtained as a white solid from 4-[1,3]dioxolan-2-yl-benzylamine described in Preparation Example 120 (970mg, 5.60mmol) and quinoline-6-carboxylic acid (913mg, 5.09mmol) according to an analogous method to Preparation Example Q+-1.
¹H-NMR Spectrum (DMSO-d₆) δ (ppm) : 3.90-4.02 (4H, m), 4.54 (2H, d, J=5.9Hz), 5.69 (1 H, s), 7.35-7.40 (4H, m), 7.60 (1 H, dd, J=4.2, 8.2Hz), 8.07 (1 H, d, J=9.0Hz), 8.2 (1 H, dd, J=1.9, 9.0Hz), 8.46 (1 H, d, J=8.1Hz), 8,54 (1 H, d, J=1.5Hz), 8.97 (1 H, dd, J=1.7, 4.2Hz), 9.29 (1 H, t, J=5.7Hz).

### Preparation Example E+-7. Quinoline-6-carboxylic acid 4-formyl-benzylamide

Quinoline-6-carboxylic acid 4-[1,3]dioxolan-2-yl-benzylamide described in Preparation Example E+-6 (1.30g, 3.89mmol) was dissolved in a mixture solution of tetrahydrofuran (20mL), water (10mL) and sulfuric acid (3mL), the solution was stirred for 2 hours under reflux. An aqueous solution of saturated sodium bicarbonate was added to the reaction solution at 0°C, the solution was extracted with ethyl acetate and tetrahydrofuran. The organic layer was washed with brine, dried over anhydrous magnesium sulfate, and the solvent was evaporated *in vacuo.* Ethyl acetate was added to the residue, the precipitated solid was filtered, and the title compound (700mg, 2.41 mmol, 62%) was obtained as a white solid.
¹H-NMR Spectrum (DMSO-d₆) δ (ppm) : 4.63 (2H, d, J=5.7Hz), 7.57 (2H, d, J=7.9Hz), 7.61 (1H, t, J=4.1 Hz), 7.88 (2H, dd, J=1.8, 8.4Hz), 8.09 (1H, d, J=8.8Hz), 8.21 (1H, dd, J=2.0, 8.8Hz), 8.48 (1H, d, J=7.9Hz), 8.56 (1H, d, J=1.5Hz), 8.98 (1H, dd, J=1.8, 4.2Hz), 9.39 (1 H, t, J=6.0Hz), 9.97 (1 H, s).

### Preparation Example E+-8. Quinoline-6-carboxylic acid 3-bromobenzylamide

The title compound (1.4g, 4.0mmol, 70%) was obtained as a white solid from 3-bromobenzylamine hydrochloride (1.3g, 5.8mmol) and quinoline-6-carboxylic acid (1.0g, 5.8mmol) according to an analogous method to Preparation Example Q+-1.
¹H-NMR Spectrum (CDCl₃) δ (ppm) : 4.68 (2H, d, J=5.7Hz), 6.75 (1H, brs), 7.23 (1H, t, J=7.8Hz), 7.31-7.34 (1H, m), 7.43-7.45 (1H, m), 7.47 (1H, dd, J=4.2, 8.2Hz), 7.53-7.54 (1 H, m), 8.07 (1H, dd, J=2.0, 8.8Hz), 8.16 (1 H, d, J=8.8Hz), 8.22-8.24 (1 H, m), 8.34 (1 H, d, J=1.8Hz), 8.99 (1 H, dd, J=1.8, 4.2Hz).

### Preparation Example E+-9. Quinoline-6-carboxylic acid 4-bromobenzylamide

The title compound (1.3g, 3.9mmol, 68%) was obtained as a white solid from 4-bromobenzylamine hydrochloride (1.3g, 5.8mmol) and quinoline-6-carboxylic acid (1.0g, 5.8mmol) according to an analogous method to Preparation Example Q+-1.
¹H-NMR Spectrum (CDCl₃) δ (ppm) : 4.67 (2H, d, J=5.9Hz), 6.63 (1H, brs), 7.26-7.29 (2H, m), 7.47-7.51 (3H, m), 8.06 (1H, dd, J=2.0, 8.8Hz), 8.16 (1H, d, J=8.8Hz), 8.22-8.25 (1 H, m), 8.33 (1 H, d, J=2.0Hz), 8.99 (1 H, dd, J=1.7, 4.2Hz).

### Preparation Example E+-10 Quinoline-6-carbothioic acid 4-benzyloxy-benzylamide

To a solution of 6-quinoline carboxylic acid (100mg, 0.577mmol) in tetrahydrofuran (50mL) was added N,N'-dicyclohexylcarbodiimide (1.90g, 11.7mmol), and the solution was stirred for 1 hour at room temperature. Then, a solution of 4-benzyloxybenzylamine described in Preparation Example 1 (2.49g, 11.7mmol) in tetrahydrofuran was added thereto, followed by stirring overnight at room temperature. The solvent was evaporated, the residue was purified by NH silica gel column chromatography (hexane:ethyl acetate), and quinoline-6-carboxylic acid 4-benzyloxy-benzylamide (4.31 g, quantitatively) was obtained as a white solid.
A mixture of the resulting quinoline-6-carboxylic acid 4-benzyloxy-benzylamide (310mg, 0.84mmol), 2,4-bis (4-methoxyphenyl)-1,3-dithia-2,4-diphosphetane-2,4-disulphide (Lawesson's reagent) (1.4g, 3.4mmol) and tetrahydrofuran (10mL) was refluxed for 1 hour. After cooling, the solvent was evaporated *in vacuo,* dichloromethane was added to the residue, which was purified by NH silica gel column chromatography (hexane : ethyl acetate = 1 : 1), and the title compound (55mg, 0.14mmol, 17%) was obtained as a pale yellow solid.
¹H-NMR Spectrum (DMSO-d₆) δ (ppm) : 4.93 (2H, d, J=4.6Hz), 5.09 (2H, s), 6.99 (2H,d, J=8.8Hz), 7.26-7.44 (7H, m), 7.58, (1H, dd, J=4.2, 8,2Hz), 8.01 (1H, d, J=9.0Hz), 8.13 (1 H, dd, J=2.1, 8.9Hz), 8.29 (1 H, d, J=1.8Hz), 8.46 (1 H, d, J=8.2Hz), 8.94 (1 H, dd, J=1.6, 4.2Hz), 10.9(1 H, brs).

### Preparation Example F-1. 3-Acetyl-4-amino-benzoic acid methyl ester

To a solution of 4-amino-3-iodo-benzoic acid methyl ester (11.30g, 40.77mmol) in toluene (300mL) were added tributyl(1-ethoxyvinyl)tin (16.5mL, 48.9mmol) and tetrakis(triphenylphosphine)palladium(0) (9422mg, 8.154mmol) under nitrogen atmosphere, and the solution was stirred at 105°C for 7 hours. After cooling to room temperature, water was added, the mixture was extracted with ethyl acetate-tetrahydrofuran, the organic layer was washed with water, and then, evaporated. The residue was dissolved in 280mL of tetrahydrofuran, 2N hydrochloric acid (80mL) was added thereto, followed by stirring for 3 hours at room temperature. The reaction mixture was cooled on an ice bath, an aqueous solution of 2N sodium hydroxide (80mL) was added, an aqueous solution of saturated sodium bicarbonate was further added, and the solution was extracted with ethyl acetate. An aqueous solution of 10% potassium fluoride was added to the organic layer, and the solution was stirred for 3 hours at room temperature. The organic layer was separated, washed with brine, then evaporated, the residue was purified by silica gel column chromatography (hexane-ethyl acetate), and title compound (6.42g, 33.2mmol, 81.4%) was obtained.
¹H-NMR Spectrum (CDCl₃) δ (ppm) : 2.64 (3H, s), 3.89 (3H, s), 6.63 (1H, d, J=8.8Hz), 7.91 (1 H, dd, J=2.0, 8.8Hz), 8.47 (1 H, d, J=2.0Hz).

### Preparation Example F-2. 4-Oxo-1,4-dihydro-cinnoline-6-carboxylic acid methyl ester

To a solution of 3-acetyl-4-amino-benzoic acid methyl ester (2063mg, 10.68mmol) in acetic acid (39mL) was added sulfuric acid (6.5mL) on an ice bath, then, an aqueous solution (6.5mL) of sodium nitrite (922mg, 13.35mmol) was added thereto, followed by stirring on the ice for 1 hour, and at room temperature for 2 days. The reaction mixture was concentrated until it reached half of the amount, water was added, then an aqueous solution of 2N sodium hydroxide was added on an ice bath to adjust the pH to 5. The insoluble matter was separated by filtration, then, the filtrate was extracted with ethyl acetate, the organic layer was dried over sodium sulfate and then evaporated. Diethyl ether was added to the resulting residue for solidification, the resulting solid was washed with diethyl ether, and 365mg of the title compound (1.78mmol, 16.6%) was obtained.
¹H-NMR Spectrum (DMSO-d₆) δ (ppm) : 3.88 (3H, s), 7.66 (1H, d, J= 8.8Hz), 7.82 (1 H, s), 8.24 (1 H, d, J=8.8Hz), 8.58 (1H, s), 13.7(1 H, brs).

### Preparation Example F-3. 4-Chloro-cinnoline-6-carboxylic acid methyl ester

Thionyl chloride (5mL, 68.5mmol) and N,N-dimethylformamide (3 drops) were added to 4-oxo-1,4-dihydro-cinnoline-6-carboxylic acid methyl ester (212mg, 1.04mol), and the solution was stirred for 1.15 hours under reflux. Toluene was added to the reaction mixture, which was then evaporated *in vacuo.* Ethyl acetate was added to the residue the organic layer was washed with an ice water, dried over anhydrous magnesium sulfate, then, evaporated *in vacuo,* and the title compound (192mg, 0.862mmol, 82.9%) was obtained. This was used in the next reaction without purification.
¹H-NMR Spectrum (DMSO-d₆) δ (ppm) : 3.97 (3H, s), 8.43 (1 H, d, J=8.8Hz), 8.66 (1 H, d, J=8.8Hz), 8.72 (1 H, s), 9.73 (1 H, s).

### Preparation Example F-4. Cinnoline-6-carboxylic acid methyl ester

To a solution of 4-chloro-cinnoline-6-carboxylic acid methyl ester (192mg, 0.863mmol) in dimethylsulfoxide (30mL) were added sodium formate (70mg, 1.04mmol), tetrakis(triphenylphosphine)palladium(0) (198mg, 0.702mmol) and N,N-diisopropylethylamine (0.21 mL, 1.21 mmol), and the mixture was stirred for 1.5 hours at 90°C. After cooling to room temperature, water was added, ethyl acetate extraction was carried out, the organic layer was washed with water and brine, then, after drying over anhydrous sodium sulfate, the organic layer was evaporated. The resulting solid was washed with diethyl ether, then, purified by silica gel column chromatography (hexane-ethyl acetate), and the title compound (16mg, 0.089mmol, 10%) was obtained.
¹H-NMR Spectrum (CDCl₃) δ (ppm) : 4.03 (3H, s), 7.97 (1 H, dd, J=0.8, 6.0Hz), 8.42 (1 H, J=0.8, 8.0Hz), 8.59-8.63 (2H, m), 9.43(1H, dd, J= 0.8, 6.0Hz).

### Preparation Example G-1. Isoquinoline-6-carboxylic acid

A solution prepared by adding (4-bromobenzylydene)-(2,2-diethoxyethyl) amine (synthesized from 4-bromobenzaldehyde, according to the method described in J. Org. Chem., vol. 48, 3344-3346 (1983)) (51.4g, 0.189mmol) to an ice-cold concentrated sulfuric acid (20g) was added to a solution prepared by adding diphosphorus pentoxide (40g) to an ice-cold concentrated sulfuric acid (360g), and the solution was stirred at 160°C for 2 hours. The reaction solution was gradually cooled to 0°C, the solution was filtered through Celite pad, the filtrate was neutralized with sodium carbonate. This solution was further filtrated through Celite pad, this filtrate was extracted with ethyl acetate and dried over anhydrous magnesium sulfate. The solvent was evaporated, and the residue was purified by silica gel column chromatography (hexane:ethyl acetate), and 6-bromoisoquinoline (482mg, 1.2%) was obtained as an orange oil.
Next, to a solution of 6-bromoisoquinoline (382mg, 1.84mmol) in N,N-dimethylformamide (3.8mL) were added zinc cyanide (431 mg, 3.67mmol) and tetrakis(triphenylphosphine)palladium(0) (42mg, 0.0367mmol) under nitrogen atmosphere, and the mixture was stirred at 100°C for 1 hour. Tetrakis(triphenylphosphine)palladium(0) (42mg, 0.0367mmol) was further added, and the mixture was stirred for 2.5 hours at 100°C. The reaction mixture was allowed to room temperature, ethyl acetate and water were added for extraction, the organic layer was washed with water and dried over anhydrous magnesium sulfate. The residue was purified by silica gel column chromatography (hexane : ethyl acetate), and isoquinoline-6-carbonitrile (234mg, 83%) was obtained as a yellow solid.
Lastly, isoquinoline-6-carbonitrile (51mg, 0.331 mmol) was dissolved in diethyleneglycol (1.0mL), potassium hydroxide (9mg, 0.166mmol) was added thereto, followed by stirring at 160°C for 3 hours. The reaction mixture was allowed to room temperature, neutralized using hydrochloric acid, extracted with ethyl acetate, dried over anhydrous magnesium sulfate, then, the solvent was evaporated. Water was added to the residue, the precipitated solid was collected, washed with water, dried *in vacuo,* so as to obtain the title compound (12mg, 21 %) as a yellow solid.

### Preparation Example H-1. 4-Chloro-quinazoline-6-carboxylic acid ethyl ester

The title compound (380mg, 1.61 mmol, 88%) was obtained from 4-oxo-dihydroquinazoline-6-carboxylic acid ethyl ester (396mg, 1.81mmol) according to an analogous method to Preparation Example F-3.
¹H-NMR Spectrum (DMSO-d₆) δ (ppm) :1.34(3H, t, J=7.2Hz), 4.35 (2H, q, J=7.2Hz), 7.78 (1 H, d, J=8.4Hz), 8.29 (1 H, dd, J=2.0, 8.4Hz), 8.37 (1 H, s), 8.64 (1 H, d, J=2.0Hz).

### Preparation Example H-2. Quinazoline-6-carboxylic acid ethyl ester

The title compound (79mg, 0.39mmol, 24%) was obtained from 4-chloro-quinazoline-6-carboxylic acid ethyl ester (380mg, 1.61 mmol) according to an analogous method to Preparation Example F-4.
¹H-NMR Spectrum (CDCl₃) δ (ppm) :1.47 (3H, t, J=7.6Hz), 4.48 (2H, t, J=7.6Hz), 8.11 (1 H, d, J=8.8Hz), 8.53 (1 H, dd, J=2.0, 8.8Hz), 8.71 (1 H, d, J=2.0Hz), 9.42 (1 H, s), 9.52 (1H, s).

### Preparation Example H-3. Quinazoline-6-carboxylic acid

To a solution of quinazoline-6-carboxylic acid ethyl ester (79mg, 0.391 mmol) in ethanol (4mL) was added an aqueous solution of 1 N sodium hydroxide (4mL), and the solution was stirred for 1 hour at room temperature. 1 N Hydrochloric acid was added to the reaction mixture to adjust the pH to 4, and the solution was evaporated *in vacuo.* Ethanol was added to the residue, and the organic layer was concentrated. The residue was dissolved in an ethyl acetate-tetrahydrofuran mixture solvent, dried over anhydrous magnesium sulfate, then, evaporated *in vacuo,* and quinazoline-6-carboxylic acid (15mg, 0.086mmol, 22%) was obtained. This was used in the next reaction without purification.
¹H-NMR Spectrum (DMSO-d₆) δ (ppm) : 8.09 (1 H, d, J=8.8Hz), 8.44 (1 H, dd, J=2.0, 8.8Hz), 8.83 (1 H, d, J=2.0Hz), 9.39 (1 H, s), 9.79 (1 H, s).

### Preparation Example I-1. Quinoxaline-6-carboxylic acid

To a solution of quinoxaline-6-carboxylic acid methyl ester (2084mg, 11.07mmol) in ethanol (25mL) was added an aqueous solution of 1 N sodium hydroxide (25mL), and the solution was stirred for 4 hours under reflux. 1 N Hydrochloric acid was added to the reaction mixture to adjust the pH to 4, then, the precipitated solid was collected by filtration, washed with water and isopropanol, then dried to obtain the title compound (1477mg, 8.479mmol, 76.6%) as a solid.
¹H-NMR Spectrum (DMSO-d₆) δ (ppm) : 8.18 (1 H, d, J=8.4Hz), 8.29 (1H, dd, J=8.4, 1.2Hz), 8.61 (1 H, d, J=1.2Hz), 9.00-9.07 (2H, m).

### Preparation Example J-1. 2,2-Dimethyl-N-pyridin-2-yl-propionamide

2-Aminopyridine (3.1g, 33mmol) and triethylamine(6.9mL, 49mmol) was dissolved in dichloromethane (40mL), 2,2-dimethylpropionyl chloride (4.5mL, 36mmol) was added on an ice bath, and the solution was stirred for 2 hours at the same temperature. Water was added thereto for extraction, the organic layer was sequentially washed with an aqueous solution of saturated sodium bicarbonate and brine, then, dried over anhydrous magnesium sulfate. The solvent was evaporated *in vacuo,* and the title compound (6.0g, 34mmol, 102%) was obtained as a white solid.
¹H-NMR Spectrum (CDCl₃) δ (ppm) :1.27 (9H, s), 7.03 (1 H, ddd, J=1.1, 4.9, 7.3Hz), 7.68-7.72 (1 H, m), 8.02 (1 H, s), 8.23-8.27 (2H, m).

### Preparation Example J-2. N-(3-formylpyridin-2-yl)-2,2-dimethylpropionamide

To a mixture solution of *tert*-butyl lithium (1.5M pentane solution, 10mL, 15mmol) and diethyl ether (50mL) was added a solution of 2,2-dimethyl-N-pyridin-2-yl-propionamide described in Preparation Example J-1 (900mg, 5.0mmol) in diethyl ether (10mL) dropwise at -78°C, and the solution was stirred for 90 minutes at the same temperature. At the same temperature, morpholine-4-carbaldehyde (1.0mL, 10mmol) was added dropwise, and the solution was warmed gradually to room temperature. Water and tetrahydrofuran were added to the reaction mixture for extraction, the organic layer was washed with brine, then, the solvent was evaporated *in vacuo.* The residue was purified by silica gel column chromatography (hexane : ethyl acetate = 1 : 4), and the title compound (880mg, 4.3mmol, 85%) was obtained as a white solid.
¹H-NMR Spectrum (CDCl₃) δ (ppm) :1.38 (9H, s), 7.21 (1H, dd, J=4.9, 7.6Hz), 8.05 (1 H, dd, J=2.0, 7.5Hz), 8.69 (1 H, dd, J=2.0, 4.9Hz), 9.94 (1 H, s), 10.9 (1 H, brs).

### Preparation Example J-3. (2-Aminopyridin-3-yl)-methanol

A mixture solution of N-(3-formylpyridin-2-yl)-2,2-dimethylpropionamide described in Preparation Example J-2 (500mg, 2.4mmol) and an aqueous solution of 5N sodium hydroxide (7mL) was refluxed for 90 minutes. After cooling, ethyl acetate and tetrahydrofuran were added for extraction, the organic layer was washed with brine, then, the solvent was evaporated *in vacuo.* The residue was purified by silica gel column chromatography (methanol : ethyl acetate = 1 : 5), and the title compound (160mg, 1.2mmol, 53%) was obtained as a pale yellow solid.
¹H-NMR Spectrum (DMSO-d₆) δ (ppm) : 4.31 (2H, s), 5.13 (1 H, brs), 5.62 (2H, s), 6.51,(1 H, dd, J=5.0, 7.3Hz), 7.34-7.36 (1 H, m), 7.81-7.82 (1 H, m).

### Preparation Example J-4. 2-Aminopyridine-3-carbaldehyde

To a mixture of (2-aminopyridin-3-yl)-methanol described in Preparation Example J-3 (130mg,1.1mmol) and dichloromethane (10mL) was added manganese dioxide (1.3g, 15mmol) at room temperature, and the solution was stirred overnight. The reaction solution was filtered through Celite pad, then, the solvent was evaporated *in vacuo,* and the title compound (108mg, 0.88mmol, 83%) was obtained as a white solid.
¹H-NMR Spectrum (CDCl₃) δ (ppm) : 6.75 (1 H, dd, J=4.9, 7.5Hz), 7.83 (1H, dd, J=1.9, 7.5Hz), 8.23 (1 H, dd, J=1.9, 4.9Hz), 9.86 (1 H, s).

### Preparation Example J-5. 2-Hydroxy-[1,8]naphthylidine-3-carboxylic acid ethyl ester

To a mixture of 2-aminopyridine-3-carbaldehyde described in Preparation Example J-4 (8.0mg, 0.066mmol) and ethanol (2mL) were added diethylmalonate (0.50mL, 3.3mmol) and piperidine (0.20mL, 2.0mmol), and the solution was stirred overnight at 70°C. The solvent was evaporated *in vacuo,* the residue was purified by silica gel column chromatography (methanol : ethyl acetate =1:10), and the title compound (9.2mg, 0.042mmol, 64%) was obtained as a white solid.
¹H-NMR Spectrum (CDCl₃) δ (ppm) :1.43 (3H, t, J=7.1 Hz), 4.45 (2H, q, J=7.1 Hz), 7.28 (1 H, dd, J=4.9, 7.8Hz), 8.04 (1 H, dd, J=1.7, 7.9Hz), 8.48 (1 H, s), 8.87-8.88 (1 H, m), 12.16 (1 H, brs).

### Preparation Example J-6. 2-Trifluoromethane sulfonyloxy-[1.8]naphthylidine-3-carboxylic acid ethyl ester

To a mixture of 2-hydroxy-[1,8]naphthylidine-3-carboxylic acid ethyl ester described in Preparation Example J-5 (95mg, 0.44mmol), dichloromethane(4mL) and N,N-dimethylformamide (0.5mL) were added N-phenyl-bis(trifluoromethanesulfonimide) (230mg, 0.65mmol), triethylamine (0.18mL,1.3mmol) and catalytic amount of 4-(dimethylamino)pyridine, and the solution was stirred for 2.5 hours at room temperature. The solvent was evaporated *in vacuo,* the residue was purified by silica gel column chromatography (hexane : ethyl acetate = 1 : 1), and the title compound (22mg, 0.063mmol, 14%) was obtained as a white solid.
¹H-NMR Spectrum (CDCl₃) δ (ppm) :1.49 (3H, t, J=7.1 Hz), 4.54 (2H, q, J=7.1 Hz), 7.69 (1 H, dd, J=4.3, 8.2Hz), 8.41 (1 H, dd, J=2.0, 8.4Hz), 9.09 (1 H, s), 9.28 (1 H, dd, J=2.0, 4.2Hz).

### Preparation Example J-7. [1.8]Naphthylidine-3-carboxylic acid ethyl ester

To a mixture of 2-trifluoromethanesulfonyloxy-[1,8]naphthylidine-3-carboxylic acid ethyl ester described in Preparation Example J-6 (22mg, 0.063mmol), tetrakis(triphenylphosphine)palladium(0) (7.3mg, 0.0063mmol), and 1-methyl-2-pyrrolidinone (1.5mL) were added N,N-diisopropylethylamine (0.033mL, 0.19mmol) and formic acid (0.0036mL, 0.094mmol), and the solution was stirred for 45 minutes at 100°C. After cooling, filtration was carried out using NH silica gel, and the filtrate was evaporated *in vacuo.* Water, ethyl acetate and tetrahydrofuran were added to the residue for extraction, the organic layer was washed with brine, the solvent was then evaporated *in vacuo.* The residue was purified by silica gel column chromatography (ethyl acetate), and the title compound (8.1mg, 0.040mmol, 64%) was obtained as a white solid.
¹H-NMR Spectrum (CDCl₃) δ (ppm) :1.49 (3H, t, J=7.1Hz), 4.51 (2H, q, J=7.1Hz), 7.61 (1 H, dd, J=4.3, 8.2Hz), 8.34 (1 H, dd, J=2.0, 8.2Hz), 8.91 (1 H, d, J=2.2Hz), 9.25 (1H, dd, J=2.0, 4.2Hz), 9.69 (1H, d, J=2.4Hz).

### Preparation Example K-1. 2-Methyl-benzoxazole-6-carboxylic acid methyl ester

To a solution of 4-amino-3-hydroxy-benzoic acid methyl ester (2085mg, 12.47mmmol) in xylene (200mL) were added acetyl chloride (1.06mL, 14.96mmol), pyridinium *p*-toluenesulfonate (940mg, 3.74mmol) and triethylamine (2.09mL, 14.96mmol), and the solution was stirred for 8.5 hours under reflux. Ethyl acetate was added to the reaction mixture, which was then washed with water, dried over anhydrous magnesium sulfate, then, evaporated *in vacuo,* the resulting residue was purified by silica gel chromatography (hexane-ethyl acetate), and the title compound (1917mg, 10.02mmol, 80.4%) was obtained.
¹H-NMR Spectrum (CDCl₃) δ (ppm) : 2.69 (3H, s), 3.96 (3H, s), 7.68 (1H, d, J=8.4Hz), 8.05 (1 H, dd, J=1.2, 8.4Hz), 8.17 (1 H, d, J= 1.2Hz).

### Preparation Example K-2. 2-Methyl-benzoxazole-6-carboxylic acid

To a solution of 2-methyl-benzoxazole-6-carboxylic acid methyl ester (301 mg, 1.57mmol) in ethanol (10mL) was added an aqueous solution of 2N sodium hydroxide (10mL), and the mixture was stirred for 2 hours at room temperature. 2N Hydrochloric acid was added to the reaction mixture to adjust the pH to 4, and the solution was extracted with ethyl acetate. The organic layer was washed with brine, dried over anhydrous sodium sulfate, then, evaporated *in vacuo,* and the title compound (270mg, 1.52mmol, 97%) was obtained. This was used in the next reaction without purification.
¹H-NMR Spectrum (DMSO-d₆) δ (ppm) : 2.64 (3H, s), 7.73 (1H, d, J=8.0Hz), 7.93(1 H, dd, J=1.2, 8.0Hz), 8.15 (1 H, d, J= 1.2Hz).

### Preparation Example O-1. 2,3-Dihydro-1H-pyrrolo[2,3-b]pyridine-5-carbonitrile

1*H*-Pyrrolo[2,3-b]pyridine (1.0g, 8.46mmol) and 10% palladium-carbon (500mg) were dissolved in a mixture of formic acid (10mL) and triethylamine (10mL), and the solution was stirred at 70°C for 17 hours. To this reaction mixture was further added 10% palladium-carbon (270mg), and the mixture was stirred at 70°C for 2 hours. The reaction mixture was cooled to room temperature, then an aqueous solution of 5N sodium hydroxide was added thereto, the solution was extracted with ethyl acetate and tetrahydrofuran, and the organic layer was dried over anhydrous magnesium sulfate. The solvent was evaporated *in vacuo,* the residue was purified by silica gel column chromatography (ethyl acetate : methanol = 10 : 1), and 2,3-dihydro-1*H*-pyrrolo[2,3-b]pyridine (614mg, 5.11mmol, 60%) was obtained as a pale yellow solid.
The resulting 2,3-dihydro-1*H*-pyrrolo[2,3-b]pyridine (614mg, 5.11mmol) and N-bromosuccinimide (1.09g, 6.13mmol) were dissolved in N,N-dimethylformamide (12mL), and the solution was stirred for 2.5 hours at room temperature. An aqueous solution of saturated sodium bicarbonate was added to the reaction solution at 0°C, which was then extracted with ethyl acetate, the organic layer was washed with brine and dried over anhydrous magnesium sulfate. The solvent was evaporated in *vacuo,* the residue was purified by NH silica gel column chromatography (hexane : ethyl acetate = 3 : 1), and 5-bromo-2,3-dihydro-1*H*-pyrrolo[2,3-b]pyridine (370mg, 1.86mmol, 36%) was obtained as a white solid.
The resulting 5-bromo-2,3-dihydro-1*H*-pyrrolo[2,3-b]pyridine (345mg, 1.73mmol), zinc cyanide (305mg, 2.60mmol) and tetrakis(triphenylphosphine)palladium(0) (200mg, 0.173mmol) were dissolved in dimethylsulfoxide (7mL), and the solution was stirred at 120°C for 4 hours under nitrogen atmosphere. The reaction solution was cooled to room temperature, water and ethyl acetate were added to the reaction solution, the organic layer was separated, the organic layer was washed with brine and dried over anhydrous magnesium sulfate. The solvent was evaporated *in vacuo,* the residue was purified by silica gel column chromatography (ethyl acetate), and the title compound (167mg, 1.15mmol, 66%) was obtained as a light brown solid.
¹H-NMR Spectrum (DMSO-d₆) δ (ppm) : 3.01 (2H, t, J=8.6Hz), 3.58 (2H, t, J=8.6Hz), 7.46 (1 H, s), 7.63 (1 H, s), 8.10 (1 H, s).

### Preparation Example O-2. 2,3-Dihydro-1H-pyrrolo[2,3-b]pyridine-5-carboxylic acid

The title compound (259mg, quantitatively) was obtained as a white solid from 2,3-dihydro-1H-pyrrolo[2,3-b]pyridine-5-carbonitrile described in Preparation Example O-1 (167mg,1.15mmol) according to an analogous method to Preparation Example T-6.
¹H-NMR Spectrum (DMSO-d₆) δ (ppm) : 3.00 (2H, t, J=8.6Hz), 3.56 (2H, t, J=8.6Hz), 7.25 (1 H, s), 7.59 (1 H, s), 8.30 (1 H, s).

### Preparation Example P-1. 6-Oxo-1,6-dihydro-pyridine-3-carboxylic acid ethyl ester

To a suspension of 6-hydroxy-nicotinic acid (5.00g, 35.9mmol) in ethanol (60mL) was added 1N hydrochloric acid (20mL), which was then stirred at 110°C for 3 hours. The reaction solution was cooled to 0°C, then, an aqueous solution of saturated sodium bicarbonate was added, the solution was extracted with ethyl acetate and tetrahydrofuran, and the organic layer was dried over anhydrous magnesium sulfate. The solvent was evaporated *in vacuo,* the residue was purified by silica gel column chromatography (ethyl acetate), and the title compound (3.90g, 23.3mmol, 65%) was obtained as a white solid.
¹H-NMR Spectrum (DMSO-d₆) δ (ppm) :1.27 (3H, t, J=7.1 Hz), 4.23 (2H, q, J=7.1 Hz), 6.36 (1 H, d, J=9.7Hz), 7.79 (1 H, dd, J=2.6, 9.7Hz), 8.03 (1 H, d, J=2.6Hz).

### Preparation Example P-2. 5-lodo-6-oxo-1,6-dihydro-pyridine-3-carboxylic acid ethyl ester

The title compound (2.82g, 9.62mmol, 80%) was obtained as a white solid from 6-oxo-1,6-dihydro-pyridine-3-carboxylic acid ethyl ester described in Preparation Example P-1 (2.00g, 12.0mmol) according to an analogous method to Preparation Example A+-16.
¹H-NMR Spectrum (DMSO-d₆) δ (ppm) :1.28 (3H, t, J=7.1Hz), 4.23 (2H, q, J=7.1Hz), 8.09 (1 H, s), 8.36 (1H, d, J=2.4Hz).

### Preparation Example P-3. 6-Oxo-5-trimethylsilanylethynyl-1,6-dihydro-pyridine-3-carboxylic acid ethyl ester

5-lodine-6-oxo-1,6-dihydro-pyridine-3-carboxylic acid ethyl ester described in Preparation Example P-2 (1.00g, 3.41mmol), trimethylsilylacetylene (626µl, 4.43mmol), palladium(II) acetate (7.66mg, 34µmol), triphenylphosphine (17.9mg, 68µmol), copper(I) iodide (13mg, 68µmol) and butylamine (674µl, 6.82mmol) were suspended in tetrahydrofuran (6mL), and the mixture was stirred at 40°C for 16 hours under nitrogen atmosphere. The reaction mixture was cooled to room temperature, then, water was added thereto, the solution was extracted with ethyl acetate, and the organic layer was washed with brine. The organic layer was dried over anhydrous magnesium sulfate, then, the solvent was evaporated *in vacuo,* the residue was purified by silica gel column chromatography (hexane : ethyl acetate = 1 : 1), and the title compound (551 mg, 2.09mmol, 61 %) was obtained as a light brown solid.
¹H-NMR Spectrum (DMSO-d₆) δ (ppm) : 0.22 (9H, s), 1.27 (3H, t, J=7.1Hz), 4.23 (2H, q, J=7.1Hz), 7.91 (1 H, d, J=2.4Hz), 8.07 (1 H, d, J=2.4Hz).

### Preparation Example P-4. Furo[2,3-b]pyridine-5-carboxylic acid ethyl ester

6-Oxo-5-trimethylsilanylethynyl-1,6-dihydro-pyridine-3-carboxylic acid ethyl ester described in Preparation Example P-3 (545mg, 2.07mmol) and copper(I) iodide (5.9mg, 31µmol) were suspended in ethanol (7mL) and triethylamine (3mL), and the mixture was stirred at 75°C for 20 hours. The reaction mixture was cooled to room temperature, then, potassium carbonate (572mg, 4.14mmol) was added to the reaction mixture, which was further stirred at 75°C for 5 hours. The reaction mixture was cooled to 0°C, then, water was added, the precipitated solid was filtered, and the title compound (303mg) was obtained as a brown solid. In addition, ethyl acetate was added to the mother liquor for extraction, the organic layer was washed with brine, then, dried over anhydrous magnesium sulfate, the solvent was evaporated in *vacuo,* the residue was purified by silica gel column chromatography (hexane : ethyl acetate = 1 : 1), and the title compound (33mg, 0.17mmol) was obtained as a white solid.
¹H-NMR Spectrum (DMSO-d₆) δ (ppm) :1.36 (3H, t, J=7.1Hz), 4.38 (2H, q, J=7.1Hz), 7.16 (1 H, d, J=2.4Hz), 8.25 (1 H, d, J=2.2Hz), 8.69 (1 H, d, J=1.8Hz), 8.87 (1 H, d, J=2.0Hz).

### Preparation Example Q+-1 Imidazo[1,2-a]pyridine-6-carboxylic acid (5-(3-fluorophenoxy)thiophene-2-ylmethyl) amide

To a solution of imidazo[1,2-a]pyridine-6-carboxylic acid (87mg, 0.54mmol) and C-(5-(3-fluorophenoxy)thiophen-2-yl)methylamine (120mg, 0.54mmol) in N,N-dimethylformamide (5mL) were added benzotriazol-1-yl-tris(dimethylamino)phosphonium hexafluorophosphate (240mg, 0.54mmol) and triethylamine(0.15mL, 1.08mmol), and the solution was stirred for 40 minutes at 80°C. Water and ethyl acetate were added to the reaction mixture for extraction, and the organic layer was washed twice with water. Silica gel was added to the organic layer, solvent was evaporated *in vacuo* for adsorption, purification was carried out by silica gel column chromatography (hexane : ethyl acetate = 1 : 1, then ethyl acetate), and the title compound (90mg, 0.25mmol, 45.4%) was obtained as a light brown oil.
¹H-NMR Spectrum (DMSO-d₆) δ (ppm) : 4.55 (2H, d, J=5.6Hz), 6.58 (1H, d, J=4.0Hz), 6.83 (1 H, d, J=4.0Hz), 6.90-7.00 (3H, m), 7.40 (1 H, ddd, J=8.0, 8.0, 8.0Hz), 7.57-7.66 (3H, m), 8.04 (1 H, s), 9.12 (1 H, d, J=0.8Hz), 9.20 (1 H, t, J=5.6Hz).

### Preparation Example R-1. 2.6-Diamino-5-iodo-nicotinic acid ethyl ester

To a solution of 2,6-diamino-nicotinic acid ethyl ester described in Preparation Example A-14 (1.4g, 7.7mmol) in N,N-dimethylformamide (15mL) was added N-iodosuccinimide (2.09g, 9.3mmol), and the solution was stirred for 1 hour at room temperature. The reaction mixture was poured into an aqueous solution of saturated sodium thiosulfate pentahydrate, and the solution was extracted with ethyl acetate. The organic layer was washed with brine, dried over anhydrous magnesium sulfate, and then evaporated. The resulting residue was purified by silica gel column chromatography (ethyl acetate : hexane = 1 : 1) and the title compound (0.84g, 2.7mmol, 35.5%) was obtained.
¹H-NMR Spectrum (CDCl₃) δ (ppm) :1.36 (3H, t, J=7.2Hz), 4.27 (2H, q, J=7.2Hz), 5.10 (2H, brs), 8.23 (1 H, s).

### Preparation Example R-2. 6-Amino-1H-pyrrolo[2,3-b]pyridine-5-carboxylic acid ethyl ester

Catechol borane (2.7mL, 1 M tetrahydrofuran solution, 2.7mmol) was added dropwise to ethoxyacetylene (0.7mL, 40% hexane solution, 2.83mmol) on an ice bath, and the mixture was stirred for 1 hour at room temperature. The mixture was further heated at 70°C and stirred for 2 hours, and allowed to room temperature. A solution of 2,6-diamino-5-iodo-nicotinic acid ethyl ester described in Preparation Example R-1 (415mg, 1.35mmol) in tetrahydrofuran (5.5mL), tetrakis(triphenylphosphine)palladium (0) (48mg, 0.042mmol) and sodium hydroxide (160mg, 4mmol, powder) were added thereto, followed by stirring for 7 hours 30 minutes under reflux. The reaction mixture was allowed to room temperature, 2N hydrochloric acid (4.7mL, 9.4mmol) was added thereto, followed by stirring for 60 hours at room temperature. After the reaction was completed, the reaction mixture was evaporated, and extracted using diethyl ether. The aqueous layer was fractionated, neutralized on an ice bath with an aqueous solution of 5N sodium hydroxide, then, was extracted with ethyl acetate. The organic layer was dried over anhydrous magnesium sulfate, then evaporated, the resulting residue was purified by silica gel column chromatography (ethyl acetate : hexane = 2 : 1), and the title compound (97mg, 0.47mmol, 35%) was obtained.
¹H-NMR Spectrum (CDCl₃) δ (ppm) :1.41(3H, t, J=7.2Hz), 4.36(2H, q, J=7.2Hz), 6.28-6.42(3H, m), 6.99-7.02(1 H, m), 8.49(1 H, s), 9.19(1 H, brs).

### Preparation Example R-3. 2,3-Dihydro-1H-pyrrolo[2,3-b]pyridine

1*H*-Pyrrolo[2,3-b]pyridine (1.00g, 8.46mmol) and 10% palladium-carbon (500mg) were dissolved in a mixture of formic acid (10mL) and triethylamine (10mL), and the mixture was stirred at 70°C for 87 hours. To this reaction mixture was further added 10% palladium-carbon (400mg), and the solution was stirred for 9.5 hours at 70°C. The reaction mixture was cooled to room temperature, an aqueous solution of 5N sodium hydroxide was added thereto, the solution was extracted with ethyl acetate and tetrahydrofuran, and the organic layer was dried over anhydrous magnesium sulfate. The solvent was evaporated *in vacuo,* the residue was purified by silica gel column chromatography (ethyl acetate : methanol = 10 : 1), and the title compound (219mg, 1.82mmol, 22%) was obtained as a pale yellow solid.
¹H-NMR Spectrum (DMSO-d₆) δ (ppm) : 2.94 (2H, t, J=8.4Hz), 3.43 (2H, t, J=8.4Hz), 6.27 (1H, s), 6.39 (1H, dd, J=5.3, 7.0Hz), 7.22 (1H, d, J=7.0Hz), 7.66 (1H, d, J=4.9Hz).

### Preparation Example R-4. 5-Bromo-2,3-dihydro-1H-pyrrolo[2,3-b]pyridine

2,3-Dihydro-1*H*-pyrrolo[2,3-b]pyridine described in Preparation Example R-3 (15mg, 0.13mmol) and N-bromosuccinimide (24mg, 0.14mmol) were dissolved in N,N-dimethylformamide (0.5mL), and the solution was stirred for 15 hours at room temperature. An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 5 : 1), and the title compound (12mg; 60µmol, 48%) was obtained as a white solid.
¹H-NMR Spectrum (DMSO-d₆) δ (ppm) : 2.98 (2H, t, J=8.8Hz), 3.48 (2H, t, J=8.8Hz), 6.60 (1 H, s), 7.37 (1 H, d, J=1.1 Hz), 7.71 (1 H, d, J=2.4Hz).

### Preparation Example R-5. 5-Bromo-1H-pyrrolo[2,3-b]pyridine

5-Bromo-2,3-dihydro-1H-pyrrolo[2,3-b]pyridine (600mg, 3.01 mmol) and 2,3-dichloro-5,6-dicyano-1,4-benzoquinone described in Preparation Example R-4 (753mg, 3.31 mmol) was dissolved in toluene (15mL), and the solution was refluxed for 40 minutes under nitrogen atmosphere. The reaction solution was cooled to room temperature, water and ethyl acetate were added thereto, the organic layer was partitioned, the organic layer was washed with brine and dried over anhydrous magnesium sulfate. The solvent was evaporated *in vacuo,* the residue was purified by silica gel column chromatography (hexane : ethyl acetate = 5 : 1), and the title compound (260mg, 1.32mmol, 44%) was obtained as a white solid.
¹H-NMR Spectrum (DMSO-d₆) δ (ppm) : 6.40-6.48 (1 H, m), 7.50-7.60 (1 H, m), 8.20 (1 H, s), 8.30 (1 H, s), 11.9 (1H, s).

### Preparation Example R-6. 1H-Pyrrolo[2,3-b]pyridine-5-carbonitrile

5-Bromo-1*H*-pyrrolo[2,3-b]pyridine described in Preparation Example R-5 (90mg, 0.46mmol), zinc cyanide (80mg, 0.69mmol) and tetrakis(triphenylphosphine)palladium(0) (53mg, 46µmol) were dissolved in N-methyl-2-pyrrolidinone (2mL), and the mixture was stirred for 4.5 hours at 110°C under nitrogen atmosphere. The reaction mixture was cooled to room temperature, water and ethyl acetate were added to the reaction mixture, the organic layer was partitioned, the organic layer was washed with brine and dried over anhydrous magnesium sulfate. The solvent was evaporated *in vacuo,* the residue was purified by silica gel column chromatography (hexane : ethyl acetate = 5 : 1), and the title compound (48mg, 0.34mmol, 73%) was obtained as a white solid.
¹H-NMR Spectrum (DMSO-d₆) δ (ppm) 6.55-6.68(1 H, m), 7.65-7.78 (1 H, m), 8.52 (1 H, s), 8.60 (1 H, s), 12.3 (1H, brs).

### Preparation Example R-7. 1 H-Pyrrolo[2,3-b]pyridine-5-carboxylic acid

The title compound (47mg, 0.29mmol, 88%) was obtained as a white solid from 1*H*-pyrrolo[2,3-b]pyridine-5-carbonitrile described in Preparation Example R-6 (47mg, 0.33mmol) according to an analogous method to Preparation Example T-6.
¹H-NMR Spectrum (DMSO-d₆) δ (ppm) : 6.57-6.63 (1H, m), 7.55-7.62 (1H, m), 8.51 (1 H, s), 8.79 (1 H, s), 12.0 (1 H, s), 12.7 (1 H, brs).

### Preparation Example S-1. 3-Amino-2-bromopyridine

2-Bromo-3-nitropyridine (3g, 15mmol) was dissolved in a mixture solution of tetrahydrofuran (15mL) and water (5mL), then iron powder (1 g, 18mmol) and ammonium chloride (2g, 37mmol) were added thereto, followed by stirring at from 60°C to 70°C for 5 hours. After the reaction was completed, the reaction mixture was filtered through Celite pad, brine was added, and the solution was extracted with ethyl acetate. The organic layer was dried over anhydrous magnesium sulfate, then evaporated, the resulting residue was purified by silica gel column chromatography (ethyl acetate : hexane =1:4), and the title compound (2.6g, 15mmol, quantitatively) was obtained.
¹H-NMR Spectrum (DMSO-d₆) δ(ppm) : 5.47 (2H, brs), 7.07-7.09 (2H, m), 7.54 (1H, dd, J=2.0, 3.6Hz).

### Preparation Example S-2. (2-Bromo-pyridin-3-yl)carbamic acid ethyl ester

3-Amino-2-bromopyridine described in Preparation Example S-1 (1.4g, 8.1 mmol) was dissolved in pyridine (10mL), ethyl chloroformate (0.93mL, 9.7mmol) was added dropwise on an ice bath, and the solution was stirred for 2 hours at room temperature. After the reaction was completed, the reaction solution was poured into brine, and the solution was extracted with ethyl acetate. The organic layer was dried over anhydrous magnesium sulfate, then evaporated, the resulting residue was purified by silica gel column chromatography (ethyl acetate : hexane = 1 : 4), and the title compound (0.56g, 2.3mmol, 28%) was obtained.
¹H-NMR Spectrum (DMSO-d₆) δ (ppm) :1.22 (3H, t, J=7.2Hz), 4.12 (2H, q, J=7.2Hz), 7.43 (1H, dd, J=4.8, 8.0Hz), 7.92 (1H, dd, J=1.6, 8.0Hz), 8.17 (1H, dd, J=1.6, 4.8Hz), 9.10 (1 H, brs).

### Preparation Example S-3. (2-Trimethylsilanylethynyl-pyridin-3-yl)carbamic acid ethyl ester

A mixture of (2-bromo-pyridin-3-yl)carbamic acid ethyl ester described in Preparation Example S-2 (395mg, 1.6mmol), dichlorobis(triphenylphosphine)palladium(II) (20mg, 0.028mmol), triethylamine (0.25mL, 1.8mmol), copper(I) iodide (10mg, 0.05mmol) and trimethylsilylacetylene (0.131 mL, 2.4mmol) was placed in a sealed tube, and heated at 100°C for 4 hours. After the reaction was completed, the reaction mixture was poured into water, which was then extracted with ethyl acetate. The organic layer was dried over anhydrous magnesium sulfate, then evaporated, the resulting residue was purified by silica gel column chromatography (ethyl acetate : hexane = 1 : 2), and the title compound (0.42g, 1.6mmol, quantitatively) was obtained.
¹H-NMR Spectrum (DMSO-d₆) δ (ppm) : 0.24 (9H, s), 1.21 (3H, t, J=7.2Hz), 4.12 (2H, q, J=7.2Hz), 7.38 (1H, dd, J=4.8, 8.4Hz), 7.88-7.96 (1H, m), 8.29 (1H, dd, J=1.6, 4.8Hz), 8.82 (1 H, brs).

### Preparation Example S-4. 1H-Pyrrolo[3, 2-b]pyridine

(2-Trimethylsilanylethynyl-pyridin-3-yl)carbamic acid ethyl ester described in Preparation Example S-3 (0.42g, 1.6mmol) was dissolved in ethanol (8mL), sodium ethoxide (204mg, 3mmol) was added thereto, followed by stirring for 1 hour under reflux. After the reaction was completed, the reaction mixture was poured into brine, and the solution was extracted with ethyl acetate. The organic layer was dried over anhydrous magnesium sulfate, then evaporated, the resulting solid was washed with solvent (diethylether : hexane = 1 : 2), and the title compound (0.12g, 1 mmol, 63.5%) was obtained.
¹H-NMR Spectrum (DMSO-d₆) δ (ppm) : 6.50-6.54 (1 H, m), 7.06 (1 H, dd, J=4.8, 8.4Hz), 7.58-7.62 (1 H, m), 7.72-7.76 (1 H, m), 8.26-8.30 (1 H, m), 11.2 (1 H, brs).

### Preparation Example T-1. 3-Dichloromethyl-2-nitro-thiophene

To a solution of potassium *tert*-butoxide (23.0mL, 1.0M tetrahydrofuran solution, 23.2mmol) in N,N-dimethyl formamide (20mL) were added a mixture solution of 2-nitro-thiophene (1.00g, 7.74mmol) in chloroform (682µl, 8.51 mmol) and N,N-dimethylformamide (2mL) dropwise at -78°C, the solution was stirred for 5 minutes, and then, methanol and acetic acid were added at 0°C. Brine was added to the reaction solution, which was then extracted with ethyl acetate. The solvent was evaporated *in vacuo,* the residue was purified by silica gel column chromatography (hexane : ethyl acetate = 20 : 1), and the title compound (1.54g, 7.26mmol, 94%) was obtained as a light brown solid.
¹H-NMR Spectrum (CDCl₃) δ (ppm) : 7.54 (1H, d, J=5.7Hz), 7.57 (1H, d, J=5.7Hz), 7.64 (1H, s).

### Preparation Example T-2. 2-nitro-thiophene-3-carbaldehyde

3-Dichloromethyl-2-nitro-thiophene described in Preparation Example T-1 (1.54g, 7.26mmol) was dissolved in formic acid (10mL), and the solution was refluxed for 24 hours under nitrogen atmosphere. An aqueous solution of 5N sodium hydroxide was added to the reaction solution at 0°C, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 10 : 1), and the title compound (472mg,3.00mmol,41 %) was obtained as a light brown solid.
¹H-NMR Spectrum (CDCl₃) δ (ppm) : 7.49 (1 H, d, J=5.5Hz), 7.54 (1 H, d, J=5.7Hz), 10.62 (1H, s).

### Preparation Example T-3. 2-(2-Nitro-thiophen-3-yl)-[1,3]dioxolane

2-Nitro-thiophene-3-carbaldehyde described in Preparation Example T-2 (367mg, 2.33mmol), ethane-1,2-diol (651µl, 11.7mmol) and toluene-4-sulfonic acid monohydrate (40mg, 0.233mmol) were dissolved in toluene (8mL), and the solution was stirred for 2.5 hours under reflux. An aqueous solution of saturated sodium bicarbonate was added to the reaction solution at 0°C, which was then extracted with ethyl acetate, the organic layer was washed with brine, and the organic layer was dried over anhydrous magnesium sulfate. The solvent was evaporated *in vacuo,* the residue was purified by NH silica gel column chromatography (hexane : ethyl acetate = 5 : 1), and the title compound (304mg, 1.51 mmol, 65%) was obtained as a white solid.
¹H-NMR Spectrum (CDCl₃) δ (ppm) : 4.07-4.15 (4H, m), 6.51 (1 H, s), 7.25 (1 H, d, J=5.5Hz), 7.45 (1 H, d, J=5.5Hz).

### Preparation Example T-4. 2-Amino-thiophene-3-carbaldehyde

2-(2-Nitro-thiophen-3-yl)-[1,3]dioxolane described in Preparation Example T-3 (150mg, 0.746mmol), iron powder (208mg, 3.73mmol) and ammonium chloride (80mg, 1.49mmol) were suspended in a mixture solvent of ethanol (3mL) and water (0.75mL), and the mixture was stirred at 90°C for 5 hours. The reaction mixture was cooled to room temperature, then, filtered through Celite pad. The filtrate was evaporated *in vacuo,* the residue was purified by silica gel column chromatography (hexane : ethyl acetate = 5 : 1), and the title compound (38mg, 0.30mmol, 40%) was obtained as a red oily substance.
¹H-NMR Spectrum (CDCl₃) δ (ppm) : 6.19 (1H, d, J=5.7Hz), 6.67 (2H, brs), 6.90 (1H, d, J=5.7Hz), 9.69 (1 H, s).

### Preparation Example T-5. 6-Amino-thieno[2,3-b]pyridine-5-carbonitrile

2-Amino-thiophene-3-carbaldehyde described in Preparation Example T-4 (38mg, 0.30mmol) and malononitrile (20mg, 0.30mmol) were dissolved in ethanol (1mL) to which piperidine (several drops) had been added, and the solution was stirred for 1 hour under reflux. The reaction solution was cooled to room temperature, and the solvent was evaporated *in vacuo.* The residue was purified by NH silica gel column chromatography (hexane : ethyl acetate = 10 : 1), and the title compound (50mg, 0.29mmol, 96%) was obtained as a white solid.
¹H-NMR Spectrum (DMSO-d₆) δ (ppm) : 7.00 (2H, s), 7.18 (1 H, d, J=6.0Hz), 7.42 (1 H, d, J=6.OHz), 8.40 (1 H, s).

### Preparation Example T-6. 6-Amino-thieno[2,3-b]pyridine-5-carboxylic acid

6-Amino-thieno[2,3-b]pyridine-5-carbonitrile described in Preparation Example T-5 (104mg, 0.594mmol) was dissolved in a mixture solution of water (1.5mL) and sulfuric acid (1.5mL), and the solution was stirred for 3 hours under reflux. An aqueous solution of 5N sodium hydroxide was added to the reaction solution at 0°C, to neutralize the solution. The precipitated solid was filtered, and title compound (65mg, 0.33mmol, 56%) was obtained as a pale yellow solid.
¹H-NMR Spectrum (DMSO-d₆) δ (ppm) : 7.19 (1H, d, J=5.9Hz), 7.25 (1H, d, J=6.0Hz), 8.48 (1 H, s).

### Preparation Example T-7. 6-Amino-thieno[2,3-b]pyridine-5-carboxylic acid methyl ester

6-Amino-thieno[2,3-b]pyridine-5-carboxylic acid (44mg, 0.23mmol) was dissolved in a mixture solution of methanol (1mL) and sulfuric acid (0.5mL), and the solution was stirred for 24 hours under reflux. An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* the residue was purified by NH silica gel column chromatography (hexane : ethyl acetate = 2 : 1), and the title compound (34mg, 0.16mmol, 72%) was obtained as a white solid.
¹H-NMR Spectrum (CDCl₃) δ (ppm) : 3.93 (3H, s), 7.09 (1 H, d, J=6.0Hz), 7.11 (1 H, d, J=6.0Hz), 8.54 (1 H, s).

### Preparation Example T-8. 6-Oxo-6,7-dihydro-thieno[2,3-b]pyridine-5-carboxylic acid methyl ester

6-Amino-thieno[2,3-b]pyridine-5-carboxylic acid methyl ester described in Preparation Example T-7 (10mg, 48µmol) and sodium nitrite (10mg, 144µmol) were dissolved in phosphinic acid (0.5mL), and the solution was stirred at 0°C for 1 hour. An aqueous solution of saturated sodium bicarbonate was added to the reaction solution at 0°C, which was then extracted with ethyl acetate, and the organic layer was dried over anhydrous magnesium sulfate. The solvent was evaporated in *vacuo,* the residue was purified by silica gel column chromatography (hexane : ethyl acetate = 1 : 1), and the title compound (10mg, 48µmol, quantitatively) was obtained as a white solid.
¹H-NMR Spectrum (CDCl₃) δ (ppm) : 4.03 (3H, s), 7.21 (1 H, d, J=5.9Hz), 7.34 (1 H, d, J=6.0Hz), 8.61 (1 H, s), 11.4 (1 H, s).

### Preparation Example T-9. 6-trifluoromethanesulfonyloxy-thieno[2,3-b]pyridine-5-carboxylic acid methyl ester

6-Oxo-6,7-dihydro-thieno[2,3-b]pyridine-5-carboxylic acid methyl ester described in Preparation Example T-8 (9mg, 43µmol), N-phenyltrifluoromethanesulfonimide (23mg, 65µmol) and dimethyl-pyridin-4-yl-amine (catalytic amount) were dissolved in dichloromethane (0.5mL), and the solution was stirred for 18.5 hours at room temperature. Water was added to the reaction solution, which was then extracted with ethyl acetate, the organic layer was washed with brine and dried over anhydrous magnesium sulfate. The solvent was evaporated *in vacuo,* the residue was purified by NH silica gel column chromatography (hexane : ethyl acetate = 3 : 1), and the title compound (10mg, 29µmol, 68%) was obtained as a white solid.
¹H-NMR Spectrum (CDCl₃) δ (ppm) : 4.03 (3H, s), 7.43 (1 H, d, J=5.9Hz), 7.73 (1H, d, J=5.9Hz), 8.87 (1 H, s).

### Preparation Example T-10. Thieno[2,3-b]pyridine-5-carboxylic acid methyl ester

6-Trifluoromethanesulfonyloxy-thieno[2,3-b]pyridine-5-carboxylic acid methyl ester described in Preparation Example T-9 (10mg, 29µmol), tetrakis(triphenylphosphine)palladium(0) (3.4mg, 2.9µmol), formic acid (1.7µl, 44µmol) and N,N-diisopropylethylamine (15µl, 87µmol) were dissolved in 1-methyl-2-pyrrolidone (0.5mL), and the mixture was stirred for 1.5 hours at 100°C. The reaction mixture was cooled to room temperature, water and ethyl acetate were added, the organic layer was partitioned, the organic layer was washed with brine and dried over anhydrous magnesium sulfate. The solvent was evaporated in *vacuo,* the residue was purified by NH silica gel column chromatography (hexane : ethyl acetate = 5 : 1), and the title compound (7mg, quantitatively) was obtained as a white solid.
¹H-NMR Spectrum (CDCl₃) δ (ppm) : 3.99 (3H, s), 7.36 (1 H, d, J=6.4Hz), 7.62 (1 H, d, J=6.0Hz), 8.70 (1H, d, J=1.6Hz), 9.17 (1 H, d, J=2.0Hz).

### Preparation Example U-1. Thiophen-3-yl-carbamic acid tert-butyl ester

Thiophene-3-carboxylic acid (2.50g, 19.5mmol), diphenylphosphoryl azide (4.62mL, 21.5mmol), triethylamine (3.26mL ,23.4mmol) were dissolved in *tert*-butanol (50mL), and the solution was stirred for 3.5 hours under reflux. Water was added to the reaction solution at 0°C, which was then extracted with ethyl acetate, the organic layer was washed with brine and dried over anhydrous magnesium sulfate. The solvent was evaporated *in vacuo,* the residue was purified by NH silica gel column chromatography (hexane : ethyl acetate = 10 : 1), and the title compound (3.33g, 16.7mmol,86%) was obtained as a white solid.
¹H-NMR Spectrum (DMSO-d₆) δ (ppm) :1.46 (9H, s), 6.97 (1H, d, J=5.2Hz), 7.16 (1H, s), 7.38 (1 H, m), 9.61 (1H, s).

### Preparation Example U-2. (2-Formyl-thiophen-3-yl)-carbamic acid tert-butyl ester

Thiophen-3-yl-carbamic acid *tert*-butyl ester described in Preparation Example U-1 (1.00g,5.02mmol) was dissolved in tetrahydrofuran (20mL), to which *n*-butyl lithium (2.47M hexane solution, 4.47mL, 11.0mmol) was added at -78°C, and the mixture was stirred at -78°C for 1 hour. N,N-dimethylformamide (466µl, 6.02mmol) was added to the reaction mixture at -78°C, and the solution was stirred for 1 hour at room temperature. Water was added to the reaction solution, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 10 : 1), and the title compound (1.14g, quantitatively) was obtained as a colorless oil.
¹H-NMR Spectrum (DMSO-d₆) δ (ppm) :1.50 (9H, s), 7.60 (1H, d, J=5.3Hz), 8.02 (1H, d, J=5.3Hz), 9.94 (1 H, s), 10.1 (1H, s).

### Preparation Example U-3. 5-Amino-thieno[3,2-b]pyridine-6-carbonitrile

(2-Formyl-thiophen-3-yl)-carbamic acid *tert*-butyl ester described in Preparation Example U-2 (500mg, 2.20mmol) and malononitrile (153mg, 2.31 mmol) were dissolved in a solution of ethanol (10mL) to which piperidine (catalytic amount) had been added, and the mixture was stirred for 1 hour under reflux. The reaction mixture was cooled to room temperature, the precipitated solid was filtered, and title compound (215mg, 1.23mmol, 56%) was obtained as a white solid.
¹H-NMR Spectrum (DMSO-d₆) δ (ppm) : 6.76 (2H, s), 7.22 (1H, dd, J=0.73, 5.5Hz), 8.22 (1 H, d, J=5.5Hz), 8.64 (1 H, s).

### Preparation Example U-4. 5-Amino-thieno[3.2-b]pyridine-6-carboxylic acid

The title compound (200mg) was obtained as a white solid from 5-amino-thieno[3,2-b]pyridine-6-carbonitrile described in Preparation Example U-3 (208mg, 1.19mmol) according to an analogous method to Preparation Example T-6.
¹H-NMR Spectrum (DMSO-d₆) δ (ppm) : 7.27 (1H, dd, J=0.73, 5.5Hz), 8.28 (1H, d, J=5.5Hz), 8.92 (1 H, s).

### Preparation Example U+-1. 5-Oxo-4,5-dihydro-thieno[3,2-b]pyridine-6-carboxylic acid (5-phenoxy-thiophen-2-ylmethyl)-amide

The title compound (17mg, 44µmol, 46%) was obtained as a white solid from 5-amino-thieno[3,2-b]pyridine-6-carboxylic acid (5-phenoxy-thiophen-2-ylmethyl)-amide described in Preparation Example U-4 (37mg, 97µmol) according to an analogous method to Preparation ExampleT-8.
¹H-NMR Spectrum (DMSO-d₆) δ (ppm) : 4.59 (2H, d, J=5.9Hz), 6.49 (1H, d, J=3.8Hz), 6.79 (1 H, d, J=3.7Hz), 7.07-7.15 (4H, m), 7.37 (2H, t, J=7.7Hz), 8.15 (1 H, d, J=5.5Hz), 8.94 (1 H, s), 10.3 (1 H, m), 13.0 (1 H, s).

### Preparation Example U+-2. Trifluoromethanesulfonic acid 6-((5-phenoxy-thiophen-2-ylmethyl)-carbamoyl)-thieno[3,2-b]pyridin-5-yl ester

The title compound (11mg, 21µmol, 68%) was obtained as a white solid from 5-oxo-4,5-dihydro-thieno[3,2-b]pyridine-6-carboxylic acid (5-phenoxy-thiophen-2-ylmethyl)-amide described in Preparation Example U+-1 (12mg, 31µmol) according to an analogous method to Preparation Example T-9 (with the proviso that N,N-dimethylformamide was used instead of dichloromethane).
¹H-NMR Spectrum (DMSO-d₆) δ (ppm) : 4.53 (2H, d, J=5.5Hz), 6.53 (1H, d, J=3.8Hz), 6.83 (1H, d, J=4.4Hz), 7.09 (2H, d, J=8.6Hz), 7.13 (1H, t, J=7.7Hz), 7.37 (2H, t, J=7.7Hz), 7.65 (1 H, d, J=5.5Hz), 8.50 (1 H, d, J=5.7Hz), 8.97 (1 H, s), 9.39-9.44(1 H, m).

### Preparation Example 1. 4-Benzyloxybenzylamine

Potassium phthalimide (20g, 0.108mol) was added to a solution obtained by dissolving 4-benzyloxybenzyl chloride (25g, 0.107mol) in N,N-dimethylformamide (75mL), and the solution was stirred for 3 hours under reflux. The reaction solution was allowed to room temperature, then, ethyl acetate and water were added for partitioning, the organic layer was washed with water and dried over anhydrous magnesium sulfate. The solvent was evaporated, and 2-(4-benzyloxybenzyl)-isoindol-1,3-dione (37g, quantitatively) was obtained as a pale brown solid.
Next, to a solution of the resulting 2-(4-benzyloxybenzyl)-isoindol-1,3-dione (37g,0.107mol) in ethanol (1 L) was added hydrazine monohydrate (8.04g, 0.161 mol), and the solution was stirred for 8 hours under reflux. The reaction solution was allowed to room temperature, then, water was added, and ethanol was evaporated in *vacuo.* Ethyl acetate and water were added to the residue for partitioning, the organic layer was washed with water, an aqueous solution of 2N sodium hydroxide and water, in this order, and dried over anhydrous magnesium sulfate. The solvent was evaporated, the residue was purified by NH silica gel column chromatography (hexane : ethyl acetate = 2 : 1; hereinafter, the NH silica gel used was manufactured by Fuji Silysia), and the title compound (15g, 64%) was obtained as a white solid.

### Preparation Example 2. 3-Benzyloxybenzylamine

To a solution of 3-benzyloxybenzyl alcohol (3.0g, 14.0mmol) in dichloromethane (30mL) was added methanesulfonyl chloride (1.39mL, 16.8mmol) and triethylamine (2.34mL, 16.8mmol) on an ice bath, and the solution was stirred overnight. The reaction solution was diluted with dichloromethane, washed with aqueous solution of 5% sodium bicarbonate, and then, dried over anhydrous magnesium sulfate. The solvent was evaporated, then, the residue was purified by silica gel column chromatography (hexane : ethyl acetate = 10 : 1), and 1-benzyloxy-3-chloromethyl-benzene (2.2g, 67%) was obtained as a colorless oil.
Next, to a solution of iminodicarboxylic acid di-*tert*-butyl ester (2.12g, 8.76mmol) in N,N-dimethylformamide (13mL) was added sodium hydride (0.39g, 9.86mmol, 60% in oil), the solution was stirred at 60°C for 6 hours, 1-benzyloxy-3-chloromethyl-benzene (1.0g, 4.30mmol) was added, and the solution was further stirred at 60°C for 4 hours. The reaction solution was allowed to room temperature, then, dichloromethane and water were added for partitioning, and the organic layer was dried over anhydrous magnesium sulfate. The solvent was evaporated, then, the residue was purified by silica gel column chromatography (hexane : ethyl acetate = 10 : 1), and (3-benzyloxybenzyl) iminodicarboxylic acid di-*tert*-butyl ester (691 mg, 39%) was obtained as a pale yellow oil.
Lastly, (3-benzyloxybenzyl) iminodicarboxylic acid di-*tert*-butyl ester (691 mg, 1.67mmol) was cooled on an ice bath, trifluoroacetic acid (3mL) was added, and the solution was stirred for 30 minutes. An aqueous solution of saturated sodium bicarbonate was added to the reaction solution, which was then extracted with ethyl acetate and dried over anhydrous magnesium sulfate. The solvent was evaporated, and the title compound (292mg, 82%) was obtained as a white waxy solid. This was used in the next reaction without further purification.

### Preparation Example 3. 4-Phenoxybenzylamine

A solution of sodium borohydride (2.20g, 58.3mmol) and concentrated sulfuric acid in diethyl ether (1.6mL) was added to a solution of 4-phenoxybenzoic acid (5.0g, 23.3mmol) in tetrahydrofuran (20mL) that had been cooled on an ice bath, and the solution was stirred for 4 hours at room temperature. The reaction solution was cooled on an ice bath, methanol was added, then, the solution was allowed to room temperature and stirred for 30 minutes. This reaction solution was cooled again, ethyl acetate and an aqueous solution of 2N sodium hydroxide were added for partitioning, the organic layer was washed with 10% sodium chloride water, dried over anhydrous magnesium sulfate, and the solvent was evaporated to obtain 4-phenoxybenzyl alcohol (4.66g, quantitatively) as a colorless solid. This 4-phenoxybenzyl alcohol was used to carry out an analogous reaction to Preparation Example 2, and the title compound (886mg) was obtained as a pale brown solid.

### Preparation Example 4. 3-Phenoxybenzylamine

The title compound was obtained as a pale brown solid from 3-phenoxybenzyl alcohol according to a similar method to Preparation Example 2.

### Preparation Example 5. C-Biphenyl-3-yl-methylamine

To a solution of 3-cyanophenylboronic acid (1.0g, 6.81 mmol) and bromobenzene (1.07g, 6.81mmol) in N,N-dimethylformamide (100mL) were added tetrakis(triphenylphosphine)palladium(0) (0.393g, 0.341 mmol) and cesium carbonate (2.77g, 8.51mmol) under nitrogen atmosphere, and the mixture was stirred for 4 hours under reflux. The reaction mixture was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate = 10 : 1), and biphenyl-3-carbonitrile (821 mg, 67%) was obtained as a yellow solid.
Next, a solution of the resulting biphenyl-3-carbonitrile (821 mg, 4.58mmol) in tetrahydrofuran (5mL) was added to a solution of lithium aluminum hydride (0.435g, 11.5mmol) in tetrahydrofuran (5mL) that had been cooled on an ice bath, and the solution was stirred for 6 hours at room temperature. The reaction solution was cooled on an ice bath, a mixture solution of methanol and water (9:1) was added thereto, an aqueous solution of saturated ammonium chloride was further added, filtration was carried out through Celite pad and insoluble matter was removed. The filtrate was partitioned, the organic layer was dried over anhydrous magnesium sulfate, and the title compound (527mg, 63%) was obtained as a brown oil. This was used in the next reaction without further purification.

### Preparation Example 6. 4-(3-Fluorobenzyloxy)-benzylamine

To a solution of 4-cyanophenol (3.0g, 25.2mmol) and 3-fluorobenzyl bromide (3.1mL, 25.2mmol) in N,N-dimethylformamide (30mL) was added potassium carbonate (8.71g, 63.0mmol), and the mixture was stirred for 1 hour at room temperature. Ethyl acetate and water were added to the reaction mixture, which was then partitioned, the organic layer was washed with water, then, dried over anhydrous magnesium sulfate, and the solvent was evaporated to obtain 4-(3-fluorobenzyloxy)-benzonitrile (5.31 g, 93%) as a colorless solid.
Next, to a solution of lithium aluminum hydride (1.25g, 133.0mmol) in tetrahydrofuran (15mL) was added a solution of 4-(3-fluorobenzyloxy)-benzonitrile (218mg, 0.615mmol) in tetrahydrofuran (12mL) on an ice bath, and the solution was stirred at room temperature for 19 hours. A mixture solvent of methanol and water (9:1) was added to the reaction solution, an aqueous solution of saturated ammonium chloride was further added, and the solution was stirred on an ice bath for 30 minutes. This solution was filtered through Celite pad, and insoluble matter was removed. The filtrate was partitioned, and the organic layer was dried over anhydrous magnesium sulfate. The solvent was evaporated, and the title compound (1.33g, 44%) was obtained as a yellow solid. This was used in the next reaction without further purification.

### Preparation Example 7. C-(4-Phenoxy-pyridin-2-yl)-methylamine

To a solution of 4-phenoxypyridine (3.0g, 17.5mmol) in dichloromethane (500mL) was added 3-chloro-perbenzoic acid (5.18g, 21.0mmol) on an ice bath, and the solution was stirred for 22 hours. An aqueous solution of saturated sodium thiosulfate and an aqueous solution of saturated sodium bicarbonate were added to the reaction solution, the solution was stirred at room temperature for 10 minutes, then, the organic layer was separated, washed with brine and dried over anhydrous magnesium sulfate. The solvent was evaporated, and 4-phenoxy-pyridine N-oxide (3.3g, quantitatively) was obtained as a pale yellow solid.
The resulting solid (3.3g, 17.6mmol) was dissolved in acetonitrile (18mL), trimethylsilyl cyanide (6.6mL, 52.8mmol) and triethylamine (4.9mL, 35.2mmol) were added, and the solution was stirred for 5 hours under reflux. The solvent was evaporated, the residue was purified by silica gel column chromatography (hexane : ethyl acetate = 4 : 1), and 4-phenoxy-pyridine-2-carbonitrile (2.5g, 73%) was obtained as a pale yellow solid.
Next, to a solution of lithium aluminum hydride (725mg,19.1mmol) in tetrahydrofuran (6.0mL) was portionwise added a solution of the resulting 4-phenoxy-pyridine-2-carbonitrile (1.5g, 7.65mmol) in tetrahydrofuran (3mL) on an ice bath, and the solution was stirred at room temperature for 15 hours. A mixture solvent of methanol and water (9:1) was added to the reaction solution, an aqueous solution of saturated ammonium chloride was further added, the solution was extracted with ethyl acetate, dried over anhydrous magnesium sulfate, then, the solvent was evaporated to obtain the title compound (730mg, 48%) as a pale brown oil. This was used in the next reaction without further purification.

### Preparation Example 8. 3-(4-Fluorophenoxy)-benzylamine

The title compound (790mg, quantitatively) was obtained as a pale yellow solid from 3-(4-fluorophenoxy)benzyl bromide (944mg, 3.36mmol) according to a similar technique to Preparation Example 1.

### Preparation Example 9. 3-(4-Methoxyphenoxy)benzylamine

To a solution of 3-(4-methoxyphenoxy)benzaldehyde (5.0g, 21.9mmol) in methanol (35mL) was added a solution of sodium borohydride (0.86g, 22.8mmol) in an aqueous solution of 2N sodium hydroxide (2.5mL), and the mixture was stirred at room temperature for 30 minutes. Water was added to the reaction mixture, which was neutralized with acetic acid, then, extracted with ethyl acetate, washed with brine, then, dried over anhydrous magnesium sulfate, and the solvent was evaporated to obtain (3-(4-methoxy-phenoxy)-phenyl)-metanol (5.3g, quantitatively) as a colorless oil.
To a solution of the obtained (3-(4-methoxy-phenoxy)-phenyl)-metanol (2.0g, 8.73mmol) in dichloromethane (20mL) was added methanesulfonyl chloride (0.81 mL, 10.5mmol) and triethylamine (1.46mL, 10.5mmol) on an ice bath, and the solution was stirred for 19 hours. The reaction solution was diluted with dichloromethane, washed with an aqueous solution of 5% sodium bicarbonate, dried over anhydrous magnesium sulfate, and the solvent was evaporated to obtain methanesulfonic acid 3-(4-methoxy-phenoxy)benzyl ester (2.4g, 89%) as a pale brown oil.
Next, the title compound (859mg, 89%) was obtained as a pale yellow solid from the resulting methanesulfonic acid 3-(4-methoxy-phenoxy)benzyl ester (2.4g, 7.78mmol) according to a similar technique to Preparation Example 1.

### Preparation Example 10. 3-(3-Trifluoromethyl-phenoxy)-benzylamine

The title compound (2.63g) was obtained as a brown oil from 3-(3-(trifluoromethyl)phenoxy)benzaldehyde (5.01 g, 18.8mmol) according to an analogous method to Preparation Example 9.

### Preparation Example 11. 3-(3-Fluoro-phenoxy)-benzylamine

To a solution of 3-fluoro-phenol (500mg, 4.46mmol) and 3-fluoro-benzonitrile (540mg, 4.46mmol) in dimethylsulfoxide (1.0mL) was added potassium *tert*-butoxide (500mg, 4.46mmol), and the solution was stirred at 140°C for 3 hours. The reaction solution was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate = 4 : 1), and 3-(3-fluoro-phenoxy)-benzonitrile (313mg, 33%) was obtained as a yellow solid.
Next, to a solution of lithium aluminum hydride (139mg,3.68mmol) in tetrahydrofuran (3.0mL) was added a solution of the resulting 3-(3-fluoro-phenoxy)-benzonitrile (313mg, 1.47mmol) in tetrahydrofuran (1mL) on an ice bath, and the solution was stirred at room temperature for 18 hours. A mixture solvent of methanol and water (9:1) was added to the reaction solution, an aqueous solution of saturated ammonium chloride was further added thereto, followed by stirring at room temperature for 10 minutes, then, extracted with ethyl acetate, dried over anhydrous magnesium sulfate, then, the solvent was evaporated to obtain the title compound (285mg, 89%) as a yellow oil.

### Preparation Example 12. 4-(Furan-2-ylmethoxy)-benzylamine

To a solution of 4-cyanophenol (2.0g, 16.8mmol) in dichloromethane (20mL) were added triphenyl phosphine (6.6g, 25.2mmol), furfuryl alcohol (1.65g, 16.8mmol) and diethyl azodicarboxylate (3.97mL, 25.2mmol) on an ice bath, and the solution was stirred at room temperature for 16 hours. The reaction solution was directly purified by silica gel column chromatography (hexane : ethyl acetate), the obtained crudely purified product was further purified by NH silica gel column chromatography (hexane : ethyl acetate), 4-furan-2-ylmethoxy)-benzonitrile (106mg, 3%) was obtained as a pale yellow solid.
Next, to a solution of lithium aluminum hydride (50mg,1.33mmol) in tetrahydrofuran (1.0mL) was added a solution of the resulting 4-furan-2-ylmethoxy)-benzonitrile (106mg, 0.532mmol) in tetrahydrofuran (1mL) on an ice bath, and the solution was stirred at room temperature for 4 hours. A mixture solvent of methanol and water (9 : 1) was added to the reaction solution, an aqueous solution of saturated ammonium chloride was further added, the solution was extracted with ethyl acetate, dried over anhydrous magnesium sulfate, then, the solvent was evaporated to obtain the title compound (76mg, 70%) as a yellow solid.

### Preparation Example 13. 4-(Thiophen-2-ylmethoxy)-benzylamine

To a solution of 2-thiophenemethanol (2.0g, 17.5mmol) in dichloromethane (20mL) were added methanesulfonyl chloride (1.63mL, 21.0mmol) and triethylamine (2.93mL, 21.0mmol) on an ice bath, and the solution was stirred for 13 hours. The reaction solution was diluted with dichloromethane, washed with an aqueous solution of 5% sodium bicarbonate, dried over anhydrous magnesium sulfate, and the solvent was evaporated to obtain methanesulfonic acid 2-thiophen-2-ylmethyl ester (2.4g) as a brown oil.
Next, to a solution of the resulting methanesulfonic acid 2-thiophen-2-ylmethyl ester (2.4g, 12.6mmol) and *p*-cyanophenol (1.50g, 12.6mmol) in N,N-dimethylformamide (25mL) was added potassium carbonate (4.35g, 32.5mmol), and the solution was stirred at room temperature for 13 hours. Ethyl acetate and water were added to the reaction solution, which was then separated, the organic layer was washed with water and dried over anhydrous magnesium sulfate. The solvent was evaporated, the residue was purified by NH silica gel column chromatography (hexane : ethyl acetate = 4 : 1), and 4-(thiophen-2-ylmethoxy)-benzonitrile (1.88g) was obtained as a white solid.
In addition, to a solution of lithium aluminum hydride (220mg, 5.80mmol) in tetrahydrofuran (2.5mL) was added a solution of the resulting 4-(thiophene-2-ylmethoxy)-benzonitrile (500mg, 2.32mmol) in tetrahydrofuran (1mL) on an ice bath, and the solution was stirred at room temperature for 4 hours. A mixture solvent of methanol and water (9:1) was added to the reaction solution, an aqueous solution of saturated ammonium chloride was further added, the solution was extracted with ethyl acetate, dried over anhydrous magnesium sulfate, then, the solvent was evaporated to obtain the title compound (415mg, 82%) as a colorless solid.

### Preparation Example 14. 4-(Thiophen-3-ylmethyl)-benzylamine

The title compound (419mg) was obtained as a pale brown solid from 3-thiophenemethanol according to an analogous method to Preparation Example 13.

### Preparation Example 15. 4-((S)-1-Phenyl-ethoxy)-benzylamine

To a solution of 4-bromobenzonitrile (500mg, 2.75mmol) and S-(-)-α-phenylethylalcohol (403mg, 3.30mmol) in toluene (5mL) were added sodium hydride (220mg, 5.49mmol; 60% in oil), tris(dibenzylideneacetone)dipalladium(0) (38mg, 0.0413mmol) and 2,2-bis (di-*p*-tolylphosphino)-1,1-binaphthyl (67mg, 0.099mmol), and the solution was stirred at 70°C for 4 hours. The reaction solution was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate), and 4-(1-phenyl-ethoxy)-benzonitrile (159mg, 26%) was obtained as a colorless oil.
Next, to a solution of lithium aluminum hydride (68mg, 1.78mmol) in tetrahydrofuran (5.0mL) was added a solution of the resulting 4-(1-phenyl-ethoxy)-benzonitrile (159mg, 0.712mmol) in tetrahydrofuran (1mL) on an ice bath, which was stirred under reflux for 2 hours. The reaction solution was allowed to room temperature, a mixture solvent of methanol and water (9:1) was added, an aqueous solution of saturated ammonium chloride was further added, then, ethyl acetate and water were added for partitioning, the organic layer was dried over anhydrous magnesium sulfate, then, the solvent was evaporated to obtain the title compound (172mg, quantitatively) as a yellow oily substance.

### Preparation Example 16. C-(6-Phenoxy-pyridin-2-yl)-methylamine

To a solution of 2,6-dibromopyridine (20g, 84.4mmol) and phenol (7.94g, 84.4mmol) in dimethylsulfoxide (200mL) was added potassium *tert*-butoxide (9.47g, 84.4mmol), and the solution was stirred at 160°C for 7 hours. The reaction solution was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate = 10 : 1), and 2-bromo-6-phenoxy-pyridine (19.6g, 93%) was obtained as a yellow solid.
Next, to a solution of the resulting 2-bromo-6-phenoxy-pyridine (1.0g, 4.0mmol) in N,N-dimethylformamide (30mL) were added zinc cyanide (940mg, 8.0mmol) and tetrakis(triphenylphosphine)palladium(0) (924mg, 0.8mmol) under nitrogen atmosphere, and the mixture was stirred at 100°C for 1 hour. The reaction mixture was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate = 10 : 1), and 6-phenoxy-pyridine-2-carbonitrile (524mg, 67%) was obtained as a white solid.
10% Palladium-carbon (50mg) was added to a solution of the resulting 6-phenoxy-pyridine-2-carbonitrile (100mg, 0.51mmol) in methanol (5.0mL), the mixture was stirred at room temperature for 24 hours under hydrogen atmosphere (1 atm). The catalyst was removed by filtration, and the filtrate was concentrated to obtain the title compound (65mg, 64%) as a colorless oil.

### Preparation Example 17. C-(5-(3-bromophenoxy)-thiophen-2-yl)-methylamine

To a solution of 5-nitrothiophene-2-carbonitrile (1.79g, 11.6mmol) and 3-bromophenol (2.00g, 11.6mmol) in dimethylsulfoxide (22mL) was added potassium carbonate (1.76g, 12.8mmol), and the solution was stirred at 70°C for 3 hours. The reaction solution was allowed to room temperature, ethyl acetate and water were added for partitioning, the organic layer was washed with brine and dried over anhydrous magnesium sulfate. The solvent was evaporated, the residue was purified by silica gel column chromatography (hexane : ethyl acetate = 10 : 1), and 5-(3-bromophenoxy)-thiophene-2-carbonitrile (2.00g, 62%) was obtained as a yellow oil.
Next, to a solution of lithium aluminum hydride (204mg, 5.39mmol) in tetrahydrofuran (10mL) was added a solution of the resulting 5-(3-bromophenoxy)-thiophene-2-carbonitrile (1.01 g, 3.59mmol) in tetrahydrofuran (10mL), and the solution was stirred at room temperature for 2 hours. Then, lithium aluminum hydride (68mg, 1.80mmol) was added, and the solution was further stirred at room temperature for 1 hour. Water was added to the reaction solution, which was then extracted with ethyl acetate, dried over anhydrous magnesium sulfate, and the solvent was evaporated to obtain a mixture of the title compound and debrominated compound (740mg) as a pale brown oil. As this mixture cannot be purified, it was used in the next reaction without purification.

### Preparation Example 18. C-(5-(3-Benzyloxy-phenoxy)-thiophen-2-yl)-methylamine

To a solution of resorcinol (10g, 90.8mmol) in N,N-dimethylformamide (100mL) was added potassium carbonate (12.6g, 90.8mmol) and benzyl bromide (10.8mL, 90.8mmol), and the mixture was stirred at 60°C for 2 hours. The reaction mixture was allowed to room temperature, ethyl acetate and water were added for partitioning, the organic layer was washed with water and dried over anhydrous magnesium sulfate. The solvent was evaporated, then, the residue was purified by silica gel column chromatography (hexane : ethyl acetate), and 3-benzyloxy-phenol (6.0g, 33%) was obtained as a pale brown oil.
To a solution of the resulting 3-benzyloxy-phenol (2.6g, 13.0mmol) and a 5-nitrothiophene-2-carbonitrile (2.0g, 13.0mmol) in dimethylsulfoxide (25mL) was added potassium carbonate (1.98g, 14.0mmol), and the solution was stirred at 70°C for 3 hours. The reaction solution was allowed to room temperature, ethyl acetate and water were added for partitioning, the organic layer was washed with water and dried over anhydrous magnesium sulfate. The solvent was evaporated, the residue was purified by silica gel column chromatography (hexane : ethyl acetate = 10 : 1), and 5-(3-benzyloxy-phenoxy)-thiophene-2-carbonitrile (110mg, 2.8%) was obtained as a pale brown solid.
Next, to a solution of lithium aluminum hydride (27mg,0.716mmol) in tetrahydrofuran (2.0mL) was added a solution of 5-(3-benzyloxy-phenoxy)-thiophene-2-carbonitrile obtained above (110mg, 0.358mmol) in tetrahydrofuran (1mL), and the solution was stirred at room temperature for 3 hours. Water was added to the reaction solution, which was then extracted with ethyl acetate, dried over anhydrous magnesium sulfate, and the solvent was evaporated to obtain the title compound (80mg, 72%) as a red solid. This compound was used in the next reaction without purification.

### Preparation Example 19. (4-Aminomethylphenyl)-benzyl-amine

To a mixture of sodium *tert*-butoxide (7.44g, 77.4mmol), tris(dibenzylideneacetone)dipalladium(0) (0.38g, 0.415mmol) and rac-2,2-bis(diphenylphosphino)-1,1-binaphthyl (0.172g, 0.277mmol) was added a solution of 4-bromobenzonitrile (10g, 55.3mmol) and benzylamine (11.8g, 0.11mol) in toluene (100mL) under nitrogen atmosphere, and the solution was stirred at 80°C for 5 hours. The reaction solution was allowed to room temperature, then, filtrated through Celite pad to remove insoluble matter, and the filtrate was concentrated. The residue was purified by silica gel column chromatography (hexane : ethyl acetate), and 4-benzyl amino-benzonitrile (11.1g, 96%) was obtained as a yellow solid.
Next, to a solution of lithium aluminum hydride (911mg, 24.0mmol) in tetrahydrofuran (60mL) was added a solution of the resulting 4-benzyl amino-benzonitrile (2.0g, 9.61 mmol) in tetrahydrofuran (5mL), and the solution was stirred at room temperature for 3 hours. Water was added to the reaction solution, which was then extracted with ethyl acetate, dried over anhydrous magnesium sulfate, and the solvent was evaporated to obtain the title compound (2.0g, quantitatively) as an orange oil. This compound was used in the next reaction without purification.

### Preparation Example 20. (4-Aminomethyl-phenyl)-phenyl-amine

To a mixture of sodium *tert*-butoxide (7.44g, 77.4mmol), tris(dibenzylideneacetone)dipalladium(0) (0.38g, 0.415mmol) and rac-2,2-bis(diphenylphosphino)-1,1-binaphthyl (0.172g, 0.277mmol) was added a solution of 4-bromobenzonitrile (10g, 55.3mmol) and benzylamine (6.5mL, 0.11 mol) in toluene (100mL) under nitrogen atmosphere, and the solution was stirred at 80°C for 5 hours. The reaction mixture was allowed to room temperature, then, filtered through Celite pad to remove insoluble matter, and the filtrate was concentrated. The residue was purified by silica gel column chromatography (hexane : ethyl acetate), and 4-phenylamino-benzonitrile (6.7g, 63%) was obtained as a yellow solid.
Next, to a solution of lithium aluminum hydride (1.17g, 30.9mmol) in tetrahydrofuran (60mL) was added a solution of the obtained 4-phenylamino-benzonitrile (2.0g, 10.3mmol) in tetrahydrofuran (5mL), and the solution was stirred at room temperature for 22 hours. Water was added to the reaction solution, which was then extracted with ethyl acetate, dried over anhydrous magnesium sulfate, and the solvent was evaporated to obtain the title compound (2.0g, 98%) as an orange oil. This compound was used in the next reaction without purification.

### Preparation Example 21. (4-Aminomethyl-benzyl)-phenylamine

To a solution of 4-cyanobenzaldehyde (10g, 76.3mmol) and aniline (4.48mL, 76.3mmol) in tetrahydrofuran (370mL) were added acetic acid (21.9mL, 0.383mol) and triacetoxy sodium borohydride (32.3g, 0.153mol), and the solution was stirred at room temperature for 1 hour, Ethyl acetate and water were added to the reaction solution, which was then partitioned, the organic layer was washed with water and dried over anhydrous magnesium sulfate. The solvent was evaporated, the residue was purified by silica gel column chromatography (hexane : ethyl acetate), and 4-phenylaminomethyl-benzonitrile) (5.1g, 32%) was obtained as a pale yellow solid.
Next, to a solution of lithium aluminum hydride (0.91g, 24mmol) in tetrahydrofuran (60mL) was added a solution of the resulting 4-phenylaminomethyl-benzonitrile (2.0g, 9.61 mmol) in tetrahydrofuran (5mL), and the solution was stirred at room temperature for 2 hours. Water was added to the reaction solution, which was then extracted with ethyl acetate, dried over anhydrous magnesium sulfate, and the solvent was evaporated to obtain the title compound (1.98g, 97%) as a yellow oil. This compound was used in the next reaction without purification.

### Preparation Example 22. 5-(3-Fluorophenoxy)thiophene-2-carbonitrile

5-Nitrothiophene-2-carbonitrile (2g, 13mmol), 3-fluorophenol (1.75g, 15.6mmol) and potassium carbonate (3.6g, 26mmol) were suspended in dimethylsulfoxide (15mL), and the mixture was stirred at room temperature for 16 hours. Water and ethyl acetate were added for partitioning, silica gel was added to the organic layer, and the solvent was evaporated *in vacuo* for adsorption. Purification was carried out by silica gel column chromatography (hexane : ethyl acetate =10:1), and the title compound (670mg, 3.1mmol, 23.5%) was obtained as an oil.
¹H-NMR Spectrum (DMSO-d₆) δ (ppm) : 6.83 (1H, d, J=4.0Hz), 7.08-7.26 (2H, m), 7.18-7.24 (1 H, m), 7.49 (1 H, ddd, J=8.0, 8.0, 8.0Hz), 7.81 (1 H, d, J=4.0Hz).

### Preparation Example 23. C-(5-(3-Fluorophenoxy)thiophen-2-yl)methylamine

To a solution of 5-(3-fluorophenoxy)thiophene-2-carbonitrile described in Preparation Example 22 (670mg, 3mmol) in tetrahydrofuran (30mL) was added lithium aluminum hydride (460mg, 12mmol), and the solution was stirred at room temperature for 16 hours. Water and ethyl acetate were added to the reaction solution, which was then partitioned, the organic layer was filtered with NH silica gel, the filtrate was evaporated *in vacuo,* and the title compound (570mg, 2.42mmol, 80.7%) was obtained as a brown oil.
¹H-NMR Spectrum (DMSO-d₆) δ (ppm) : 2.08 (2H, brs), 3.80 (2H, s), 6.54 (1H, d, J=3.6Hz), 6.66-6.70 (1 H, m), 6.88-6.99 (3H, m), 7.39 (1 H, ddd, J=8.0, 8.0, 8.0Hz).

### Preparation Example 24. C-(5-Phenoxy thiophen-2-yl)methylamine

5-Nitrothiophene-2-carbonitrile (0.80g, 5.2mmol), phenol (1.0g, 10.4mmol) and potassium carbonate (2.2g, 15.6mmol) were suspended in dimethylsulfoxide (30mL), and the mixture was stirred at room temperature for 15.5 hours. Water and ethyl acetate were added for partitioning, and the organic layer was washed three times with water. NH silica gel was added to the organic layer, the solvent was evaporated *in vacuo* for adsorption, purification was carried out by NH silica gel column chromatography (hexane : ethyl acetate = 8 : 1), and 5-phenoxythiophene-2-carbonitrile (720mg, 3.6mmol, 69.2%) was obtained as a colorless oil.
To a solution of this oil in tetrahydrofuran (40mL) was added lithium aluminum hydride (540mg,14.4mmol), and the mixture was stirred for 30 minutes at room temperature. Water and ethyl acetate were added to the reaction mixture, which was then partitioned, silica gel was added to the organic layer, the solvent was evaporated *in vacuo* for adsorption, purification was carried out by silica gel column chromatography (ethyl acetate, then ethyl acetate : methanol = 4 : 1), and the title compound (570mg, 2.8mmol,77.2%) was obtained as a light brown oil.
¹H-NMR Spectrum (DMSO-d₆) δ (ppm) : 2.34 (2H, brs), 3.78-3.82(2H, m), 6.47 (1H, d, J=4.0Hz), 6.65-6.68 (1 H, m), 7.04-7.14 (3H, m), 7.34-7.40(2H, m).

### Preparation Example 25. 5-Phenoxy thiophene-2-carbonitrile

5-Nitro-thiophene-2-carbonitrile(1.5g, 9.7mmol), phenol (1.8g, 19.4mmol) and potassium carbonate(4.0g, 29.1mmol) were suspended in dimethylsulfoxide (20mL), and the mixture was stirred for 50 minutes at 60°C, followed by further stirring overnight at room temperature. Water and ethyl acetate were added to the reaction mixture, which was then partitioned, the organic layer was washed 4 times with water, then, NH silica gel was added to the organic layer, the solvent was evaporated in *vacuo* for adsorption, purification was carried out by NH silica gel column chromatography (hexane, then hexane : ethyl acetate = 20 : 1, then 10 : 1), and the title compound (1.4g, 7.0mmol, 72.1 %) was obtained as a pale yellow oil.
¹H-NMR Spectrum (DMSO-d₆) δ (ppm) : 6.75 (1 H, d, J=4.0Hz), 7.23-7.31 (3H, m), 7.42-7.49 (2H, m), 7.78 (1 H, d, J=4.0Hz).

### Preparation Example 26. C-(5-Phenoxy thiophen-2-yl)methylamine

To a solution of 5-phenoxythiophene-2-carbonitrile described in Preparation Example 25 (1.4g, 7.0mmol) in tetrahydrofuran (30mL) was added lithium aluminum hydride (1.1g,28mmol), and the solution was stirred at room temperature for 25 minutes. Water and ethyl acetate were added to the reaction solution, this mixed solution was filtered through Celite pad, furthermore, the organic layer was partitioned. The solvent was evaporated *in vacuo,* and the title compound (1.29g, 6.3mmol, 89.9%) was obtained as a brown oil.
¹H-NMR Spectrum (DMSO-d₆) δ (ppm) :1.92 (2H, brs), 3.74-3.80(2H, m), 6.46 (1H, d, J=3.6Hz), 6.62-6.66(1 H, m), 7.02-7.14 (3H, m), 7.32-7.39 (2H, m).

### Preparation Example 27. 5-(4-Fluorophenoxy)thiophene-2-carbonitrile

5-Nitrothiophene-2-carbonitrile (2.0g, 13mmol), 4-fluorophenol (2.9g, 26mmol) and potassium carbonate (5.4g, 39mmol) were suspended in dimethylsulfoxide (30mL), and the mixture was stirred for 30 minutes at 60°C. Water and ethyl acetate were added to the reaction mixture, which was then separated, the organic layer was washed with water twice, then, NH silica gel was added to the organic layer, the solvent was evaporated *in vacuo* for adsorption, purification was carried out by NH silica gel column chromatography (hexane : ethyl acetate = 20 : 1), and the title compound (3.7g, containing 4-fluorophenol) was obtained as a brown oil.
¹H-NMR Spectrum (DMSO-d₆) δ (ppm) : 6.68-6.76 (2H, m), 7.26-7.38 (3H, m), 7.74-7.80 (1 H, m).

### Preparation Example 28. C-(5-(4-Fluorophenoxy)thiophen-2-yl)methylamine

To a solution of 5-(4-fluorophenoxy)thiophene-2-carbonitrile described in Preparation Example 27 (containing 4-fluorophenol) (3.7g) in tetrahydrofuran (40mL) was added lithium aluminum hydride (1.3g,34mmol), and the solution was stirred at room temperature for 30 minutes. Water and ethyl acetate were added to the reaction solution, which was then partitioned, the solvent was evaporated *in vacuo,* the residue was purified by silica gel column chromatography (ethyl acetate, then ethyl acetate : methanol = 4 : 1), and the title compound (1.2g, 5.4mmol) was obtained as a brown oil.
¹H-NMR Spectrum (DMSO-d₆) δ (ppm) : 2.00 (2H, brs), 3.75-3.80 (2H, m), 6.44-6.48 (1 H, m), 6.62-6.67 (1 H, m), 7.08-7.14 (2H, m), 7.16-7.24 (2H, m).

### Preparation Example 29. 5-m-Tolyloxy-thiophene-2-carbonitrile

The title compound (960mg, 4.47mmol, 68.7%) was obtained as a yellow oil from 5-nitro-thiophene-2-carbonitrile (1.0g, 6.5mmol) and 3-methylphenol (1.4g, 13mmol) according to an analogous method to Preparation Example 27.
¹H-NMR Spectrum (DMSO-d₆) δ (ppm) : 2.31 (3H, s), 6.73 (1 H, dd, J=0.8, 4.0Hz), 7.03-7.06 (1 H, m), 7.07-7.12 (2H, m), 7.33 (1H, dd, J=8.0, 8.0Hz), 7.77 (1 H, dd, J=0.8, 4.0Hz).

### Preparation Example 30. C-(5-m-Tolyloxy-thiophen-2-yl)-methylamine

The title compound (900mg, 4.10mmol, 91.7%) was obtained as a reddish brown oil from 5-*m*-tolyloxy thiophene-2-carbonitrile described in Preparation Example 29 (960mg, 4.47mmol) according to an analogous method to Preparation Example 28.
¹H-NMR Spectrum (DMSO-d₆) δ (ppm) : 2.03 (2H, brs), 2.34 (3H, s), 3.85 (2H, s), 6.51-6.54 (1 H, m), 6.71-6.74 (1 H, m), 6.90-7.03 (3H, m), 7.31 (1 H, dd, J=8.0, 8.0Hz).

### Preparation Example 31. 5-p-Tolyloxy-thiophene-2-carbonitrile

The title compound (1.0g, 4.65mmol, 71.5%) was obtained as a yellow oil from 5-nitro-thiophene-2-carbonitrile (1.0g, 6.5mmol) and 4-methylphenol (1.4g, 13mmol) according to an analogous method to Preparation Example 27.
¹H-NMR Spectrum (DMSO-d₆) δ (ppm) : 2.30 (3H, s), 6.69-6.71 (1H, m), 7.15-7.18 (2H, m), 6.24-6.28 (2H, m), 7.15-7.78 (1 H, m).

### Preparation Example 32. C-(5-p-Tolyloxy thiophen-2-yl)methylamine

The title compound (780mg, 3.56mmol, 76.5%) was obtained as a reddish brown oil from 5-*p*-tolyloxy thiophene-2-carbonitrile described in Preparation Example 31 (1.0g,4.65mmol) according to an analogous method to Preparation Example 28
¹H-NMR Spectrum (DMSO-d₆) δ (ppm) : 2.06 (2H, br), 2.22 (3H, s), 3.76 (2H, s), 6.41 (1 H, d, J=3.6Hz), 6.62 (1 H, d, J=3.6Hz), 6.90-6.98 (2H, m), 7.15-7.18 (2H, m).

### Preparation Example 33. 2-(4-(3-Fluoro-phenoxy)-thiophen-2-yl)-[1,3]dioxolane

2-(4-Bromo-thiophen-2-yl)-[1,3]dioxolane (1.0g, 4.3mmol), 3-fluorophenol (0.95g, 8.6mmol), 2,2,6,6-tetramethyl-3,5-heptanedione (0.078g,0.43mmol), copper(I) chloride (0.21g,2.7mmol) and cesium carbonate (2.8g,8.6mmol) were suspended in N-methylpyrrolidone (10mL) under a nitrogen stream, and the mixture was stirred for 4.5 hours at 120°C. To this suspension was added 2,2,6,6-tetramethyl-3,5-heptanedione (0.12g, 0.65mmol), and the solution was further stirred for 8 hours at 140°C. The reaction mixture was filtered through Celite pad, then, water and ethyl acetate were added for partitioning, and the organic layer was washed with water twice. NH silica gel was added to the organic layer, the solvent was evaporated *in vacuo* for adsorption to NH silica gel, purification was carried out by NH silica gel column chromatography (hexane, then hexane : ethyl acetate = 30 : 1), and the title compound (280mg,1.05mmo1,24.4%) was obtained as a colorless oil.
¹H-NMR Spectrum (DMSO-d₆) δ (ppm) : 3.88-3.96 (2H, m), 3.96-4.04 (2H, m), 5.98 (1H, s), 6.82-6.88 (2H, m), 6.91-6.97 (1H, m), 7.04-7.05 (1H, m), 7.09 (1H, d, J=2.0Hz), 7.35-7.42 (1H, m).

### Preparation Example 34. 4-(3-Fluorophenoxy)thiophene-2-carbaldehvde

To a solution of 2-(4-(3-fluoro-phenoxy)-thiophen-2-yl)-[1,3]dioxolane described in Preparation Example 33 (280mg,1.05mmol) in methanol (10mL) was added citric acid aqueous solution (10mL), and the solution was stirred at room temperature for 30 minutes. The reaction solution was neutralized with an aqueous solution of sodium bicarbonate, the solution was extracted with ethyl acetate, the organic layer was dried over anhydrous sodium sulfate, the solvent was evaporated *in vacuo,* and the title compound (210mg, 0.95mmol, 90%) was obtained as a colorless oil.
¹H-NMR Spectrum (DMSO-d₆) δ (ppm) : 6.88-7.03 (3H, m), 7.38-7.46 (1H, m), 7.67(1 H, d, J=1.6Hz), 7.88 (1 H, d, J=1.6Hz), 9.86 (1 H, s).

### Preparation Example 35. (C-(4-(3-Fluorophenoxy)thiophen-2-yl)methylamine

4-(3-Fluorophenoxy)thiophene-2-carbaldehyde described in Preparation Example 34 (210mg, 0.95mmol) was dissolved in 7N ammonia/methanol (30mL), Raney nickel (500mg) was added thereto, followed by stirring for 19 hours under hydrogen atmosphere at room temperature. The reaction mixture was filtered through Celite pad, Raney nickel was removed, silica gel was added to the filtrate, then, the solvent was evaporated *in vacuo,* for adsorption on silica gel, purification by silica gel chromatography (ethyl acetate, then ethyl acetate : methanol = 4 : 1) was carried out, and the title compound (70mg, 0.32mmol) was obtained as a pale yellow oil.
¹H-NMR Spectrum (DMSO-d₆) δ (ppm) : 2.11 (2H, brs), 3.82(2H, s), 6.75(1H, s), 6.80-6.95 (4H, m), 7.33-7.41 (1 H, m).

### Preparation Example 36. 2-(5-(4-Fluoro-benzyl)-thiophen-2-yl)-[1,3]dioxolane

*n*-Butyl lithium (2.6N hexane solution, 3.3mL, 8.47mmol) was added dropwise to a solution of 2-(5-bromo-thiophen-2-yl)-[1,3]dioxolane (1.8g, 7.7mmol) in tetrahydrofuran (20mL) that had been cooled to from -75° to -70°C, and the solution was stirred for 30 minutes. 4-Fluorobenzyl bromide (1.1mL, 8.47mmol) was added dropwise to this reaction solution while keeping it at -70°C or less. After completion of dropwise addition, the reaction solution was gradually allowed to room temperature. Water and ethyl acetate were added to the reaction solution, which was then partitioned, silica gel was added to the organic layer, which was then evaporated *in vacuo* for adsorption, purification by silica gel column chromatography (hexane, then hexane : ethyl acetate = 20 : 1, then 10 : 1) was carried out, and the title compound (560mg, 2.04mmol, 26.4%) was obtained as a brown oil.
¹H-NMR Spectrum (DMSO-d₆) δ (ppm) : 3.84-3.98 (4H, m), 4.08 (2H, s), 5.90 (1H, s), 6.75-6.78 (1 H, m), 7.00 (1 H, d, J=3.6Hz), 7.08-7.15 (2H, m), 7.25-7.30 (2H, m).

### Preparation Example 37. 5-(4-Fluoro-benzyl)-thiophene-2-carbaldehyde

To a solution of 2-(5-(4-fluoro-benzyl)-thiophen-2-yl)-[1,3]dioxolane described in Preparation Example 36 (560mg, 2.04mmol) in methanol (20mL)was added citric acid aqueous solution (20mL), and the solution was stirred at room temperature for 30 minutes. Ethyl acetate and water were added to the reaction solution, which was then partitioned, the organic layer was washed with water twice, dried over anhydrous sodium sulfate, the solvent was evaporated *in vacuo,* and title compound (460mg, 2.09mmol) was obtained as a brown oil.
¹H-NMR Spectrum (DMSO-d₆) δ (ppm) : 4.22 (2H, s), 7.08-7.18 (3H, m), 7.29-7.36 (2H, m), 7.83-7.87 (1 H, m), 9.79 (1 H, s).

### Preparation Example 38. C-(5-(4-Fluorobenzyl)thiophen-2-yl)methylamine

7N ammonia/methanol(30mL) and Raney nickel (500mg) were added to 5-(4-fluorobenzyl)thiophene-2-carbaldehyde described in Preparation Example 37 (460mg, 2.09mmol), and the mixture was stirred for 14 hours under hydrogen atmosphere at room temperature. The catalyst was removed by filtering through Celite pad, then, purification by silica gel column chromatography (ethyl acetate, then ethyl acetate : methanol = 4 : 1) was carried out, and the title compound (70mg, 0.316mol, 15.1 %) was obtained as an oil.
¹H-NMR Spectrum (DMSO-d₆) δ (ppm) : 2.20 (2H, brs), 3.78 (2H, s), 4.03 (2H, s), 6.58-6.80 (2H, m), 7.00-7.38 (4H, m).

### Preparation Example 39. 5-Benzyl-furan-2-carbaldehyde

*n*-Butyl lithium (2.44N hexane solution, 15mL, 39.6mmol) was added dropwise to a solution of 2-furan-2-yl-[1,3]dioxolane (5g, 36mmol) in tetrahydrofuran (30mL) that had been cooled to from -75°C to -70°C, and the solution was stirred for 1 hour. Benzyl bromide (4.7mL, 39.6mmol) was added dropwise to this solution at from -75°C to -70°C. After completion of dropwise addition, the cold bath was removed, and the solution was gradually allowed to room temperature. Water and ethyl acetate were added to the reaction mixture for partitioning, silica gel was added to the organic layer, which was then evaporated *in vacuo* to adsorb the reaction mixture, purification by silica gel column chromatography (hexane : ethyl acetate = 50 : 1, then 6 : 1) was carried out, and 2-(5-benzyl-furan-2-yl)-[1,3]dioxolane (3.8g, 16.5mmol, 45.9%) was obtained as a yellow oil.
The resulting 2-(5-benzyl-furan-2-yl)-[1,3]dioxolane (3.8g, 16.5mmol) was suspended in a mixture solution of methanol (15mL), tetrahydrofuran (10mL) and 2N hydrochloric acid (15mL), and the solution was stirred at room temperature for 2 hours. Ethyl acetate and an aqueous solution of sodium bicarbonate were added to the reaction solution, which was then partitioned, the organic layer was washed with water twice and dried over anhydrous sodium sulfate. The solvent was evaporated, and the title compound (2.5g, 13mmol) was obtained as a brown oil.
¹H-NMR Spectrum (DMSO-d₆) δ (ppm) : 4.09 (2H, s), 6.45-6.48 (1H, m), 7.20-7.35 (5H, m), 7.45 (1 H, d, J=3.6Hz), 9.46 (1 H, s).

### Preparation Example 40. 2-(5-Benzyl-thiophen-2-yl)-[1,3]dioxolane

The title compound (520mg, 2.1mmol, 41.4%) was obtained as a colorless oil from benzyl bromide according to an analogous method to Preparation Example 36.
¹H-NMR Spectrum (DMSO-d₆) δ (ppm) : 3.84-3.90 (2H, m), 3.90-3.98 (2H, m), 4.08 (2H, s), 5.90 (1 H, s), 6.75-6.78 (1 H, m), 7.00 (1 H, d, J=3.6Hz), 7.18-7.32 (5H, m).

### Preparation Example 41. 5-Benzyl-thiophene-2-carbaldehyde

The title compound (containing impurity, 460mg) was obtained as a colorless oil from 2-(5-benzyl-thiophen-2-yl)-[1,3]dioxolane described in Preparation Example 40 (520mg, 2.1 mmol) according to an analogous method to Preparation Example 37.
¹H-NMR Spectrum (DMSO-d₆) δ (ppm) : 4.22 (2H, s), 7.11 (1H, d, J=3.6Hz), 7.20-7.34 (5H, m), 7.85 (1 H, d, J=3.6Hz), 9.79 (1 H, s).

### Preparation Example 42. C-(5-benzyl-thiophen-2-yl)-methylamine

The title compound (270mg) was obtained as a brown oil from 5-benzyl-thiophene-2-carbaldehyde described in Preparation Example 41 (containing impurity, 460mg, 2.27mmol) according to an analogous method to Preparation Example 38.
¹H-NMR Spectrum (DMSO-d₆) δ (ppm) :1.85 (2H, brs), 3.75 (2H, s), 4.01 (2H, s), 6.65-6.72 (2H, m), 7.15-7.30 (5H, m).

### Preparation Example 43. 2-(5-(3-Chloro-benzyl)-thiophen-2-yl)-[1,3]dioxolane

*n*-Butyl lithium (2.6N hexane solution, 15.6mL, 39mmol) was added dropwise to a solution of 2-(5-bromo-thiophen-2-yl)-[1,3]dioxolane (7.0g,30mmol) in tetrahydrofuran (40mL) at from -75° to -68°C, and the solution was stirred for 20 minutes. 3-Chlorobenzyl bromide (4.3mL,33mmol) was added dropwise to this reaction solution at from -75°C to -68°C, and the solution was stirred for 20 minutes. The cold bath was removed, and the reaction solution was gradually allowed to room temperature. Water and ethyl acetate were added to the reaction solution, which was then partitioned, the solvent was evaporated, the residue was purified by NH silica gel column chromatography (hexane : ethyl acetate = 20 : 1), and the title compound (1.6g, 5.7mmol, 19.0%) was obtained as a yellow oil.
¹H-NMR Spectrum (DMSO-d₆) δ (ppm): 3.82-4.00 (4H, m), 4.11 (2H, s), 5.91 (1 H, s), 6.78-6.80 (1 H, m), 7.01 (1 H, d, J=3.6Hz), 7.19-7.36 (4H, m).

### Preparation Example 44. 5-(3-Chloro-benzyl)-thiophene-2-carbaldehyde

To a solution of 2-(5-(3-chloro-benzyl)-thiophen-2-yl)-[1,3]dioxolane described in Preparation Example 43 (1.6g,5.7mmol) in methanol (20mL) was added citric acid aqueous solution (20mL), and the solution was stirred at room temperature for 20 minutes. An aqueous solution of sodium bicarbonate and ethyl acetate were added to the reaction solution, which was then partitioned, the organic layer was washed with water twice and dried over anhydrous magnesium sulfate. The solvent was evaporated, and the title compound (1.2g, 5.08mmol ,89.2%) was obtained as a yellow oil.
¹H-NMR Spectrum (DMSO-d₆) δ (ppm) : 4.25 (2H, s), 7.12-7.15 (1H, m), 7.24-7.40 (4H, m), 7.86 (1 H, d, J=3.6Hz), 9.80 (1 H, s).

### Preparation Example 45. C-(5-(3-Chloro-benzyl)-thiophen-2-yl)-methylamine

To a solution of 5-(3-chloro-benzyl)thiophene-2-carbaldehyde described in Preparation Example 44 (1.2g, 5.08mmol) in 7N ammonia/methanol (40mL) was added Raney nickel (2g), and the solution was stirred at room temperature for 17 hours under hydrogen atmosphere. The reaction solution was filtered through Celite pad, the catalyst was removed, then, this filtrate was evaporated *in vacuo,* the residue was purified by silica gel column chromatography (ethyl acetate, then ethyl acetate : methanol = 5 : 1), and the title compound (740mg, 3.12mmol, 61.4%) was obtained as a brown oil.
¹H-NMR Spectrum (DMSO-d₆) δ (ppm) :1.94 (2H, brs), 3.76 (2H, s), 4.06 (2H, s), 6.69-6.72 (2H, m), 7.18-7.34 (4H, m).

### Preparation Example 46. 5-(4-Chloro-phenoxy)-furan-2-carbaldehyde

To a solution of 4-chlorophenol (4.4g, 33.6mmol) in dimethylsulfoxide (30mL) was added sodium hydride (1.34g, 33.6mmol, 60% in oil), and the solution was stirred at room temperature for 10 minutes. 5-Nitrofuran-2-carbaldehyde (4.0g, 28mmol) was added to this reaction solution, and the solution was stirred at room temperature for 5 minutes. Water and ethyl acetate were added to the reaction solution, which was then partitioned, and the organic layer was washed with water 6 times. The solvent was evaporated *in vacuo,* the residue was purified by silica gel column chromatography (hexane, then hexane : ethyl acetate = 10 : 1, then 4 : 1), and the title compound (3.3g, 14.9mmol, 53.0%) was obtained as a yellow oil.
¹H-NMR Spectrum (DMSO-d₆) δ (ppm) : 5.89-5.93 (1 H, m), 7.30-7.36 (2H, m), 7.50-7.60 (3H, m), 9.35-9.38 (1 H, m).

### Preparation Example 47. C-(5-(4-Chloro-phenoxy)-furan-2-yl)-methylamine

The title compound (200mg, 0.90mmol, 8.7%) was obtained as a brown oil from 5-(4-chloro-phenoxy)-furan-2-carbaldehyde described in Preparation Example 46 (2.3g, 10.3mmol) according to an analogous method to Preparation Example 38.
¹H-NMR Spectrum (DMSO-d₆) δ (ppm) : 3.56 (2H, s), 5.73 (1H, d, J=3.2Hz), 6.18 (1 H, d, J=3.2Hz), 7.03-7.08 (2H, m), 7.40-7.45 (2H, m).

### Preparation Example 48. 5-Phenoxy-furan-2-carbaldehyde

The title compound (2.3g, 12.2mmol, 43.5%) was obtained as a light brown oil from phenol (3.2g,33.6mmol) and 5-nitro-furan-2-carbaldehyde (4.0g, 28mmol) according to an analogous method to Preparation Example 46.
¹H-NMR Spectrum (DMSO-d₆) δ (ppm) : 5.85 (1H, d, J=4.0Hz), 7.25-7.33 (3H, m), 7.45-7.50 (2H, m), 7.57 (1 H, d, J=4.0Hz), 9.34 (1 H, s).

### Preparation Example 49. C-(5-Phenoxy-furan-2-yl)-methylamine

The title compound (250mg, 1.32mmol, 24.9%) was obtained as a yellow oil from 5-phenoxy-furan-2-carbaldehyde described in Preparation Example 48 (1.0g, 5.3mmol) according to an analogous method to Preparation Example 38.
¹H-NMR Spectrum (DMSO-d₆) δ (ppm) :1.98 (2H, brs), 3.56 (2H, s), 5.67 (1H, d, J=3.2Hz), 6.16-6.18 (1H, m), 6.99-7.04 (2H, m), 7.10-7.16 (1H, m), 7.34-7.40 (2H, m).

### Preparation Example 50. 5-(3-Fluoro-phenoxy)-furan-2-carbaldehyde

The title compound (1.5g, 7.3mmol, 52.1 %) was obtained as a yellow oil from 3-fluorophenol (1.9g, 16.8mmol) and 5-nitro-furan-2-carbaldehyde (2.0g,14mmol) according to an analogous method to Preparation Example 46.
¹H-NMR Spectrum (DMSO-d₆) δ (ppm) : 5.97-6.00 (1H, m), 7.10-7.20 (2H, m), 7.24-7.30 (1 H, m), 7.48-7.55 (1 H, m), 7.59 (1 H, d, J=3.6Hz) 9.37 (1 H, s).

### Preparation Example 51. (5-(3-Fluoro-phenoxy)-furan-2-yl)-methanol

To a solution of 5-(3-fluoro-phenoxy)-furan-2-carbaldehyde described in Preparation Example 50 (1.5g, 7.3mmol) in tetrahydrofuran (20mL) was added sodium borohydride (280mg, 7.3mmol), and the solution was stirred at room temperature for 1 hour. Water and ethyl acetate were added to the reaction solution, which was then partitioned, the organic layer was washed with water twice, and the organic layer was filtered through a glass filter lined with silica gel. The solvent was evaporated *in vacuo,* and the title compound (1.5g, 7.2mmol) was obtained as a pale yellow oil.
¹H-NMR Spectrum (DMSO-d₆) δ (ppm) : 4.29 (2H, d, J=6.0Hz), 5.17 (1H, t, J=6.0Hz), 5.78-5.82 (1H, m), 6.78-6.82 (1H, m), 6.85-6.95 (2H, m), 6.98-7.04 (1H, m), 7.40-7.46 (1 H, m).

### Preparation Example 52. 2-(5-(3-Fluoro-benzyl)-thiophen-2-yl)-[1,3]dioxolane

*n*-Butyl lithium (2.44N hexane solution, 6.4mL, 16.9mmol) was added dropwise to a solution of 2-(5-bromo-thiophen-2-yl)-[1,3]dioxolane (3.0g,13mmol) in tetrahydrofuran (30mL) at from -75°C to -69°C, and the solution was stirred for 17 minutes. 3-Fluorobenzyl bromide (1.7mL, 14.3mmol) was added dropwise to this reaction solution at from -75°C to -69°C. After dropwise addition was completed, the reaction solution was gradually allowed to room temperature. Water and ethyl acetate were added to the reaction solution, which was then partitioned, the organic layer was concentrated *in vacuo,* the residue was purified by silica gel column chromatography (hexane : ethyl acetate = 20 : 1), and the title compound (478mg, 1.81 mmol, 13.9%) was obtained as a brown oil.
¹H-NMR Spectrum (DMSO-d₆) δ (ppm) : 3.82-4.00 (4H, m), 4.13 (2H, s), 5.91 (1H, s), 6.78-6.80 (1 H, m), 7.00-7.10 (4H, m), 7.30-7.37 (1 H, m).

### Preparation Example 53. 5-(3-Fluoro-benzyl)-thiophene-2-carbaldehyde

An aqueous solution of saturated citric acid (20mL) was added to a solution of 2-(5-(3-fluoro-benzyl)thiophen-2-yl)-[1,3]dioxolane described in Preparation Example 52 (670mg, 2.53mmol) in methanol (20mL), and the solution was stirred at room temperature for 30 minutes. Water and ethyl acetate were added to the reaction solution, which was then partitioned, and the organic layer was dried over anhydrous magnesium sulfate. The solvent was evaporated *in vacuo,* and the title compound (485mg, 2.2mmol, 87.0%) was obtained as a brown oil.
¹H-NMR Spectrum (DMSO-d₆) δ (ppm) : 4.25 (2H, s), 7.03-7.18 (4H, m), 7.30-7.40 (1 H, m), 7.86 (1 H, dd, J=1.6, 3.6Hz), 9.80 (1 H, d, J=1.6Hz).

### Preparation Example 54. 2-(5-(3-Chloro-benzyl)-furan-2-yl)-[1,3]dioxolane

The title compound (1.34g, 5.07mmol, 14.1%) was obtained as a yellow oil from 2-furan-2-yl-[1,3]dioxolane (5.0g,36mol) and 3-chlorobenzyl bromide (5.2mL,39.6mmol) according to an analogous method to Preparation Example 36.
¹H-NMR Spectrum (DMSO-d₆) δ (ppm) : 3.84-4.02 (6H, m), 5.76 (1H, s), 6.10-6.12 (1H, m), 6.41 (1 H, d, J=3.2Hz), 7.16-7.20 (1 H, m), 7.26-7.36 (3H, m).

### Preparation Example 55. 5-(3-Chloro-benzyl)furan-2-carbaldehyde

The title compound (1.03g, 4.68mmol, 82.1 %) was obtained as a yellow oil from 2-(5-(3-chloro-benzyl)-furan-2-yl)-[1,3]dioxolane described in Preparation Example 54 (1.34g, 5.07mmol) according to an analogous method to Preparation Example 37.
¹H-NMR Spectrum (DMSO-d₆) δ (ppm) : 4.11 (2H, s), 6.48-6.51 (1H, m), 7.20-7.24 (1 H, m), 7.28-7.37 (3H, m), 7.46 (1 H, d, J=3.2Hz), 9.46-9.49 (1 H, m).

### Preparation Example 56. C-(5-(3-Chloro-benzyl)-furan-2-yl)-methylamine

The title compound (690mg, 3.12mmol, 66.6%) was obtained as a yellow oil from 5-(3-Chloro-benzyl)-furan-2-carbaldehyde described in Preparation Example 55 (1.03g, 4.68mmol) according to an analogous method to Preparation Example 38.
¹H-NMR Spectrum (DMSO-d₆) δ (ppm) :1.90 (2H, brs), 3.57 (2H, s), 3.92 (2H, s), 6.01 (1 H, d, J=2.8Hz), 6.07 (1 H, d, J=2.8Hz), 7.16-7.20 (1 H, m), 7.24-7.34 (3H, m).

### Preparation Example 57. 1-Benzyl-1H-pyrrole-3-carbaldehyde

Benzylamine (540mg, 5.00mmol) and acetic acid (10mL) were added to 2,5-dimethoxy-tetrahydrofuran-3-carbaldehyde (1g, 6.25mmol), and the solution was stirred for 20 minutes at 90°C. Water and ethyl acetate were added to the reaction solution, which was then partitioned, the organic layer was washed with an aqueous solution 2N sodium hydroxide once and with water twice, filtered with a glass filter lined with silica gel, the filtrate was evaporated *in vacuo,* and title compound (800mg, 4.3mmol, 68.8%) was obtained as a brown oil.
¹H-NMR Spectrum (DMSO-d₆) δ (ppm) : 5.18 (2H, s), 6.46 (1 H, dd, J=2.0, 2.0Hz), 6.97 (1 H, dd, J=2.0, 2.0Hz), 7.24-7.37 (5H, m), 7.71 (1 H, dd, J=2.0, 2.0Hz), 9.63 (1 H, s).

### Preparation Example 58. 1-(3-Fluoro-benzyl)-1H-pyrrole-3-carbaldehyde

The title compound (2.33g, 11.4mmol, 71.7%) was obtained as an oil from 2,5-dimethoxy-tetrahydrofuran-3-carbaldehyde (2.6g, 16mmol) and 3-fluorobenzylamine (2.0g, 16mmol) according to an analogous method to Preparation Example 57.
¹H-NMR Spectrum (DMSO-d₆) δ (ppm) : 5.20 (2H, s), 6.44-6.48 (1H, m), 6.98-7.02 (1 H, m), 7.07-7.17 (3H, m), 7.36-7.42 (1 H, m), 7.74 (1 H, d, J=1.6Hz), 9.63 (1 H, s).

### Preparation Example 59. C-(1-(3-Fluoro-benzyl)-1H-pyrrol-3-yl)-methylamine

7N Ammonia/methanol(40mL) and Raney nickel(2g) were added to 1-(3-fluoro-benzyl)-1*H*-pyrrole-3-carbaldehyde described in Preparation Example 58 (1.0g,4.9mmol), and the solution was stirred for 18 hours under hydrogen atmosphere at room temperature. The catalyst was removed by filtering through Celite pad, then, NH silica gel was added to the filtrate, the solvent was evaporated in *vacuo* for adsorption, purification by NH silica gel column chromatography (hexane : ethyl acetate = 2 : 1, then 1 : 1, then ethyl acetate) was carried out, and the title compound (530mg, 2.5mmol, 53.0%) was obtained as a brown oil.
¹H-NMR Spectrum (DMSO-d₆) δ (ppm) : 3.50 (2H, s), 5.01 (2H, s), 5.95 (1H, d, J=2.0Hz), 6.64 (1 H, d, J=1.2Hz), 6.69-6.74 (1 H, m), 6.92-7.10 (3H, m), 7.32-7.38 (1 H, m).

### Preparation Example 60. 1-Benzo[1,3]dioxol-5-ylmethyl-1H-pyrrole-3-carbaldehyde

The title compound (2.0g, 8.7mmol, 69.8%) was obtained as a brown oil from 2,5-dimethoxy-tetrahydrofuran-3-carbaldehyde (2.0g, 12.5mmol) and C-benzo[1,3]dioxol-5-ylmethylamine) (1.9g, 12.5mmol) according to an analogous method to Preparation Example 57.
¹H-NMR Spectrum (DMSO-d₆) δ (ppm) : 5.04 (2H, s), 5.97 (2H, s), 6.42-6.45 (1H, m), 6.80-7.00 (4H, m), 7.69-7.72 (1 H, m), 9.61 (1 H, s).

### Preparation Example 61. C-(1-benzo[1,3]dioxol-5-ylmethyl-1H-pyrrol-3-yl)-methylamine

The title compound (1.5g, 6.5mmol, 74.7%) was obtained as a light green oil from 1-benzo[1,3]dioxol-5-ylmethyl-1*H*-pyrrole-3-carbaldehyde described in Preparation Example 60 (2.0g, 8.7mmol) according to an analogous method to Preparation Example 59.
¹H-NMR Spectrum (DMSO-d₆) δ (ppm) :1.37 (2H, brs), 3.48 (2H, brs), 4.87 (2H, s), 5.90 (1 H, s), 5.95 (2H, s), 6.60 (1 H, s), 6.66-6.72 (2H, m), 6.75 (1 H, s), 6.83 (1 H, d, J=7.6Hz).

### Preparation Example 62. 1-Phenethyl-1H-pyrrol-3-carbaldehyde

The title compound (840mg, 4.2mmol, 84%) was obtained as a brown oil from 2,5-dimethoxy-tetrahydrofuran-3-carbaldehyde (1.0g, 6.25mmol) and phenethyl amine (600mg, 5.0mmol) according to an analogous method to Preparation Example 57.
¹H-NMR Spectrum (DMSO-d₆) δ (ppm) : 3.03 (2H, t, J=7.2Hz), 4.19 (2H, t, J=7.2Hz), 6.38-6.42 (1 H, m), 6.37-6.82 (1 H, m), 7.15-7.30 (5H, m), 7.54-7.60 (1 H, m), 9.57 (1 H, s).

### Preparation Example 63. 1-Benzyloxy-1H-pyrrole-3-carbaldehyde

The title compound (500mg, 2.5mmol, 13.1 %) was obtained as a yellow oil from 2,5-dimethoxy-tetrahydrofuran-3-carbaldehyde (3.0g, 19mmol) and O-benzyl hydroxylamine (2.3g, 19mmol) according to an analogous method to Preparation Example 57.
¹H-NMR Spectrum (DMSO-d₆) δ (ppm) : 5.24 (2H, s), 6.35-6.48 (1H, m), 7.05-7.08 (1 H, m), 7.28-7.43 (5H, m), 7.74-7.77 (1 H, m), 9.55 (1 H, s).

### Preparation Example 64. (1-Benzyloxy-1H-pyrrol-3-yl)-methanol

To a solution of 1-benzyloxy-1*H*-pyrrole-3-carbaldehyde described in Preparation Example 63 (500mg, 2.5mmol) in tetrahydrofuran (10mL) was added lithium aluminum hydride (75mg,1.97mmol), and the solution was stirred at room temperature for 10 minutes. Water and ethyl acetate were added to the reaction solution, which was then partitioned, silica gel was added to the organic layer, the solvent was evaporated *in vacuo* for adsorption, purification by silica gel column chromatography (hexane : ethyl acetate = 2 : 1) was carried out, and the title compound (168mg, 0.828mmol, 33.1 %) was obtained as a colorless oil.
¹H-NMR Spectrum (DMSO-d₆) (ppm) : 4.22 (2H, d, J=5.6Hz), 4.60 (1 H, t, J=5.6Hz), 5.10 (2H, s), 5.78-5.81 (1 H, m), 6.75-6.78 (1 H, m), 6.78-6.81 (1 H, m), 7.37-7.42 (5H, m).

### Preparation Example 65. (5-[1,3]Dioxotan-2-yl-thiophen-2-yl)-(5-methyl-thiophen-2-yl)-methano)

*n*-Butyl lithium (2.44N hexane solution, 7.4mL, 17.9mmol) was added dropwise to a solution of 2-(5-bromo-thiophen-2-yl)-[1,3]dioxolane (4.0g, 17mmol) in tetrahydrofuran (50mL) at from -75°C to -70°C, and the solution was stirred for 10 minutes. 5-Methylthiophene-2-carbaldehyde (2.4g, 18.7mmol) was further added dropwise to the reaction solution at from -75°C to -70°C. After dropwise addition was completed, the reaction solution was gradually allowed to room temperature. Water and ethyl acetate were added to the reaction mixture, which was then partitioned, and the solvent was evaporated. The residue was purified by NH silica gel column chromatography (hexane : ethyl acetate = 4 : 1, then 2 : 1), and the title compound (2.0g, 7.09mmol, 41.7%) was obtained as a brown oil.
¹H-NMR Spectrum (DMSO-d₆) δ (ppm) : 2.38 (3H, s), 3.88-4.04 (4H, m), 5.95 (1 H, s), 6.04-6.08 (1H, m), 6.43-6.46 (1H, m), 6.60-6.63 (1H, m), 6.75 (1H, d, J=3.6Hz), 6.83 (1 H, d, J=3.6Hz), 7.02 (1 H, d, J=3.6Hz).

### Preparation Example 66. 5-(5-Methyl-thiophen-2-ylmethyl)-thiophene-2-carbaldehyde

Sodium iodide (6.4g, 42.6mmol) and trimethylsilyl chloride (4.6g, 42.6mmol) were suspended in acetonitrile (100mL) on an ice bath, and (5-[1,3]dioxolane-2-ylthiophen-2-yl)-(5-methyl-thiophen-2-yl)-methanol described in Preparation Example 65 (2.0g,7.09mmol) was added dropwise. After dropwise addition was completed, the reaction solution was gradually allowed to room temperature. A solution obtained by dissolving an aqueous solution of 2N sodium hydroxide (10.6mL) and sodium thiosulfate pentahydrate (530mg, 2.13mmol) in water (5mL) was added to the reaction solution that had been cooled on an ice bath, and the solution was stirred. Water and ethyl acetate were added to the reaction solution, which was then partitioned, and the organic layer was concetrated *in vacuo.* The residue was purified by silica gel column chromatography (hexane : ethyl acetate = 20 : 1), and the title compound (790mg, 3.56mmol, 50.2%) was obtained as a brown oil.
¹H-NMR Spectrum (DMSO-d₆) δ (ppm) : 2.38 (3H, s), 4.38 (2H, s), 6.62-6.67 (1H, m), 6.76-6.80 (1 H, m), 7.12-7.16 (1 H, m), 7.85-7.90 (1 H, m), 9.83 (1 H, s).

### Preparation Example 67 (5-(5-Methyl-thiophen-2-ylmethyl)-thiophen-2-yl)-methanol

To a solution of 5-(5-methyl-thiophen-2-ylmethyl)-thiophene-2-carbaldehyde described in Preparation Example 66 (790mg,3.56mmol) in tetrahydrofuran (5mL) was added lithium aluminum hydride (41 mg, 1.06mmol), and the mixture was stirred at room temperature for 10 minutes. Water and ethyl acetate were added to the reaction mixture, which was then partitioned, the organic layer was filtered with a glass filter lined with silica gel, the filtrate was evaporated *in vacuo,* and the title compound (640mg, 2.86mmol, 80.3%) was obtained as a light brown oil.
¹H-NMR Spectrum (DMSO-d₆) δ (ppm) : 2.36 (3H, s), 4.20 (2H, s), 4.52 (2H, d, J=6.0Hz), 5.33 (1 H, t, J=6.0Hz), 6.56-6.63 (1 H, m), 6.66-6.76 (3H, m).

### Preparation Example 68. (5-[1,3]Dioxolan-2-yl-thiophen-2-yl)-(5-methyl-furan-2-yl)-methanol

The title compound (4.2g, 16mmol) was obtained as a reddish brown oil from 2-(5-bromo-thiophen-2-yl)-[1,3]dioxolane (4.0g, 17mmol) and 5-methyl-furan-2-carbaldehyde (1.9g, 17mmol) according to an analogous method to Preparation Examples 65.
¹H-NMR Spectrum (DMSO-d₆) δ (ppm) : 2.22 (3H, s), 3.89-3.96 (2H, m), 3.98-4.06 (2H, m), 5.83 (1 H, d, J=4.8Hz), 5.96-6.02 (2H, m), 6.11-6.13 (1 H, m), 6.22-6.24 (1 H, m), 6.82-6.84 (1 H, m), 7.02-7.05 (1 H, m).

### Preparation Example 69. 5-(5-Methyl-furan-2-ylmethyl)-thiophene-2-carbaldehyde

The title compound (400mg, 1.9mmol, 11.8%) was obtained as a brown oil from 5-[1,3]dioxolan-2-yl-thiophen-2-yl)-(5-methyl-furan-2-yl)-methanol described in Preparation Example 68 (4.2g,16mmol) according to an analogous method to Preparation Example 66.
¹H-NMR Spectrum (DMSO-d₆) δ (ppm) : 2.21 (3H, s), 4.24 (2H, s), 5.98-6.02 (1 H, m), 6.12-6.14 (1 H, m), 7.13 (1 H, d, J=3.6Hz), 7.89 (1 H, d, J=3.6Hz), 9.82 (1 H, s).

### Preparation Example 70. (5-(5-Methyl-furan-2-ylmethyl)-thiophen-2-yl)-methanol

The title compound (210mg, 1.0mmol, 52.6%) was obtained as a brown oil from 5-(5-methyl-furan-2-ylmethyl)-thiophene-2-carbaldehyde described in Preparation Example 69 (400mg, 1.9mmol) according to an analogous method to Preparation Example 67.
¹H-NMR Spectrum (DMSO-d₆) δ (ppm) : 2.20 (3H, s), 4.05 (2H, s), 4.53 (2H, d, J=5.6Hz), 5.33 (1H, t, J=5.6Hz), 5.94-5.98 (1H, m), 6.02 (1H, d, J=2.8Hz), 6.70-6.77 (2H, m).

### Preparation Example 71. Benzofuran-2-yl-(5-[1,3]dioxolan-2-yl-thiophen-2-yl)-methanol

The title compound (7.2g, 23.8mmol, 91.5%) was obtained as a yellow oil from 2-(5-bromo-thiophen-2-yl)-[1,3]dioxolane (6.0g, 26mmol) and benzofuran-2-carbaldehyde (3.8g, 26mmol) according to an analogous method to Preparation Example 65.
¹H-NMR Spectrum (DMSO-d₆) δ (ppm) : 3.88-4.06 (4H, m), 5.98 (1H, s), 6.08-6.11 (1 H, m), 6.59 (1 H, d, J=5.2Hz), 6.78 (1 H, s), 6.95 (1 H, d, 3.6Hz), 7.07 (1 H, d, J=3.6Hz), 7.20-7.30 (2H, m), 7.52 (1 H, d, J=8.0Hz), 7.61 (1 H, d, J=7.2Hz).

### Preparation Example 72. (5-Benzofuran-2-ylmethyl-thiophene)-methanol

(Benzofuran-2-ylmethyl)-thiophene-2-carbaldehyde (1.3g, 5.4mmol, 54.5%) was obtained as a brown oil from benzofuran-2-yl-(5-[1,3]dioxolan-2-yl-thiophen-2-yl)-methanol described in Preparation Example 71 (3.0g, 9.9mmol) according to an analogous method to Preparation Example 66, Using this oil (1.2g) according to an analogous method to Preparation Example 67, the title compound (900mg, 3.7mmol, 68.5%) was obtained as a brown oil.
¹H-NMR Spectrum (DMSO-d₆) δ (ppm) : 4.32 (2H, s), 4.55 (2H, d, J=5.6Hz), 5.36 (1 H, t, J=5.6Hz), 6.67 (1 H, s), 6.80 (1 H, d, J=3.2Hz), 6.83 (1 H, d, J=3.2Hz), 7.17-7.27 (2H, m), 7.50 (1 H, d, J=8.0Hz), 7.56 (1 H, d, J=7.2Hz).

### Preparation Example 73. 1-Phenyl-1H-pyrrole-3-carbaldehyde

The title compound (1.2, 7.0mmol, 70%) was obtained as a brown oil from 2,5-dimethoxy-tetrahydrofuran-3-carbaldehyde (2.0g, 12.5mmol) and aniline (930mg, 10mmol) according to an analogous method to Preparation Example 57.
¹H-NMR Spectrum (DMSO-d₆) δ (ppm) : 6.67-6.70 (1 H, m), 7.32-7.39 (1 H, m), 7.48-7.55 (3H, m), 7.65-7.70 (2H, m), 8.22-8.26 (1 H, m), 9.77 (1 H, s).

### Preparation Example 74. C-(1-Phenyl-1H-pyrrol-3-yl)-methylamine

The title compound (580mg, 3.37mmol, 48.1%) was obtained as a light green oil from 1-phenyl-1*H*-pyrrole-3-carbaldehyde described in Preparation Example 73 (1.2g, 7.0mmol) according to an analogous method to Preparation Example 59.
¹H-NMR Spectrum (DMSO-d₆) δ (ppm) : 3.58 (2H, s), 6.19-6.22 (1 H, m), 7.16-7.22 (2H, m), 7.24-7.28 (1 H, m), 7.38-7.44 (2H, m), 7.47-7.52 (2H, m).

### Preparation Example 75. (3-Phenoxy-benzyl)-carbamic acid phenyl ester

To a solution of phenyl chloroformate (0.29mL, 2.3mmol) in tetrahydrofuran (10mL) were added 3-phenoxybenzylamine described in Preparation Example 4 (0.5g, 2.5mmol) and triethylamine (0.35mL, 2.5mmol) dropwise on an ice bath, and then, the solution was stirred at room temperature for 4 hours. The reaction solution was poured into brine, the solution was extracted with ethyl acetate and concentrated, and the title compound (0.7g, 2.2mmol, 88%) was obtained.
¹H-NMR Spectrum (DMSO-d₆) δ (ppm) : 4.24 (2H, d, J=6.0 Hz), 6.87-7.49 (14H, m), 8.29 (1 H, t, J=6.0 Hz).

### Preparation Example 76. 5-(2,4-Difluoro-phenoxy)-furan-2-carbaldehyde

To a solution of 2,4-difluorophenol (6.54mL, 68.0mmol) in dimethylsulfoxide (70mL) was sodium hydride (4534mg, 68.0mmol, 60% in oil), which was then stirred for 40 minutes. 5-Nitro-2-furaldehyde (8000mg, 56.7mmol) was added to the reaction solution, and the solution was stirred at room temperature for 2 hours. Water was added to the reaction mixture, which was then extracted with ethyl acetate. The organic layer was washed with water and brine, dried over anhydrous magnesium sulfate, and then, concentrated *in vacuo.* The resulting residue was purified by silica gel chromatography (hexane : ethyl acetate), and the title compound (4134mg, 18.44mmol, 33%) was obtained.
¹H-NMR Spectrum (CDCl3) δ (ppm) : 5.54 (1H, d, J=3.6Hz), 6.90-7.03 (2H, m), 7.21 (1 H, d, J=3.6Hz), 7.24-7.31 (1 H, m), 9.40 (1 H, s).

### Preparation Example 77. C-(5-(2,4-Difluoro-phenoxy)-furan-2-yl)-methylamine

To a solution of 5-(2,4-difluoro-phenoxy)-furan-2-carbaldehyde (2060mg, 9.19mmol) in 7N ammonia/methanol solution (100mL) was added Raney nickel (5.9g), and the mixture was stirred for 24 hours at room temperature under hydrogen atmosphere. The reaction mixture was filtered through Celite pad, and the filtrate was concentrated *in vacuo.* The residue was purified by NH silica gel column chromatography (hexane : ethyl acetate), and the title compound (1814mg, 88.06mmol, 87.7%) was obtained as a yellow oil.
¹H-NMR Spectrum (DMSO-d₆) δ (ppm) :1.70 (2H, brs), 3.55 (2H, s), 5.55 (1H, d, J=3.2Hz), 6.13 (1H, d, J=3.2Hz), 7.05-7.22 (1H, m), 7.21-7.28(1H, m), 7.43-7.50(1H, m).

### Preparation Example 78. 5-(2,5-Difluoro-phenoxy)-furan-2-carbaldehyde

To a solution of 2,5-difluorophenol (3360mg, 25.83mmol) in N,N-dimethylformamide (60mL) was sodium hydride (1 032mg, 25.83mmol,60% in oil), which was then stirred for 1 hour. 5-Bromo-2-furaldehyde (3826mg, 21.52mmol) was added to the reaction solution, and the solution was stirred at 60°C for 12 hours. Water was added to the reaction mixture, which was then extracted with ethyl acetate. The organic layer was washed with water and brine, dried over anhydrous magnesium sulfate, and then, concentrated *in vacuo.* The resulting residue was purified by silica gel chromatography (hexane-ethyl acetate), and the title compound (1104mg, 4.92mmol, 22.9%) was obtained.
¹H-NMR Spectrum (CDCl₃) δ (ppm) : 5.67 (1H, d, J=3.6Hz), 6.93-7.04 (2H, m), 7.15-7.22(1 H, m), 7.24 (1 H, d, J=3.6Hz), 9.43 (1 H, s).

### Preparation Example 79. C-(5-(2,5-Difluoro-phenoxy)-furan-2-yl)-methylamine

The title compound (2353mg, 10.50mmol, 97%) was obtained from 5-(2,5-difluoro-phenoxy)-furan-2-carbaldehyde (2402mg, 11.65mmol) according to an analogous method to Preparation Example 77.
¹H-NMR Spectrum (DMSO-d₆) δ (ppm) :1.72 (2H, brs), 3.56 (2H, s), 5.71 (1H, d, J=3.2Hz), 6.17 (1 H, d, J=3.2Hz), 7.01-7.12 (2H, m), 7.41-7.50 (1 H, m).

### Preparation Example 80. 2-Benzyloxy-thiophene

To a solution of benzyl alcohol (3.45mL, 33.3mmol) in 1,2-dimethoxyethane (80mL) was added *n*-butyl lithium (2.6M hexane solution, 13.5mL, 33.3mmol) dropwise, which was then stirred for 10 minutes. Copper(I) chloride (5210mg, 49.45mmol) was added thereto, followed by stirring for 10 minutes on an ice bath, then, it was stirred for 2.5 hours at room temperature. 2-lodothiophene (4995mg, 23.78mmol) and pyridine 320mL were further added, and the solution was stirred for 13 hours under reflux. Ethyl acetate was added to the reaction mixture, which was sequentially washed with 1 N hydrochloric acid, an aqueous solution of sodium hydrogen sulfate and brine, dried over anhydrous magnesium sulfate, and then, concentrated *in vacuo.* The resulting residue was purified by silica gel chromatography (hexane-ethyl acetate), and the title compound (420mg, 2.21 mmol, 9.5%) was obtained.
¹H-NMR Spectrum (CDCl3) δ (ppm) : 5.08 (2H, s), 6.28 (1H, dd, J=1.6, 4.0Hz), 6.57 (1 H, dd, J=1.6, 5.6Hz), 6.71 (1 H, dd, J=4.0, 5.6Hz), 7.30-7.47 (5H, m).

### Preparation Example 81. 5-Benzvloxy-thiophene-2-carbonitrile

To a solution of 2-benzyloxy-thiophene (184mg, 0.967mmol) in diethyl ether (4mL) was added *n*-butyl lithium (2.47M hexane solution,0.47mL, 1.16mmol) at -78°C under nitrogen atmosphere, then, the solution was stirred for 1.5 hours on an ice bath. The solution was cooled again to -78°C, N,N-dimethylformamide (487µl, 4.84mmol) was added, and the solution was stirred for 45 minutes while warming to room temperature. An aqueous solution of saturated ammonium chloride was added to the reaction mixture, which was then extracted with ethyl acetate. The organic layer was washed with brine, dried over anhydrous magnesium sulfate, then, filtered with silica gel, the filtrate was concentrated *in vacuo,* and 5-benzyloxy-thiophene-2-carbaldehyde (171 mg) was obtained.
The resulting 5-benzyloxy-thiophene-2-carbaldehyde (171 mg) was dissolved in N,N-dimethylformamide (4mL), pyridine (82µl, 1.02mmol) and hydroxylamine hydrochloride (65mg, 0.94mmol) were added thereto, followed by stirring for 30 minutes at 60°C, then, cooled on an ice bath. 1,1'-Carbonyldiimidazole (635mg, 3.92mmol) was added, the solution was warmed again to 60°C and stirred for 35 minutes, triethylamine (272µl, 1.96mmol) was added, and the solution was further stirred for 30 minutes. The reaction mixture was cooled to room temperature, water was added, and the solution was extracted with ethyl acetate. The organic layer was sequentially washed with an aqueous solution of oxalic acid, an aqueous solution of saturated sodium bicarbonate and brine, dried over anhydrous sodium sulfate, and then, concentrated *in vacuo.* The resulting residue was purified by silica gel chromatography (hexane-ethyl acetate), and the title compound (30mg, 0.14mmol, 14%) was obtained.
¹H-NMR Spectrum (CDCl3) δ (ppm) : 5.15 (2H, s), 6.28 (1 H, d, J=4.0Hz), 7.32 (1 H, d, J=4.0Hz), 7.38-7.48 (5H, m).

### Preparation Example 82. 2-5-(3-Fluoro-benzyl)-furan-2-yl)-[1,3]dioxolane

*n*-Butyl lithium (2.66M hexane solution,25.5mL, 67.88mmol) was added dropwise to a solution of 2-(1,3-dioxolan-2-yl)furan (8272mg, 59.03mmol) in tetrahydrofuran (160mL) that had been cooled to -78°C solution under nitrogen atmosphere, which was then stirred for 10 minutes. To this solution was added a solution of 3-fluorobenzyl bromide (14.50g, 76.73mmol) in tetrahydrofuran (60mL) dropwise, which was then stirred for 1 hour at -78°C, and for 1.25 hours at room temperature. An aqueous solution of saturated ammonium chloride was added to the reaction mixture, which was extracted with diethyl ether. The organic layer was washed with brine, dried over anhydrous sodium sulfate, and then, concentrated in *vacuo.* The obtained residue was purified by silica gel chromatography (hexane-ethyl acetate), and a mixture of the title compound and 2-(1,3-dioxolane-2-yl)furan (8033mg), which is the starting material, was obtained.

### Preparation Example 83. 5-(3-Fluoro-benzyl)-furan-2-carbaldehyde

To a solution of a mixture of 2-(5-(3-fluoro-benzyl)-furan-2-yl)-[1,3]dioxolane and 2-(1,3-dioxolan-2-yl)furan (8033mg) in methanol (80mL) was added an aqueous solution of oxalic acid (22g, 115mmol) (80mL), and the solution was stirred at room temperature for 1 hour. Water was added to the reaction mixture, which extracted with diethyl ether, the organic layer was sequentially washed with an aqueous solution of saturated sodium bicarbonate, water and brine, dried over anhydrous sodium sulfate, and then, concentrated *in vacuo.* The resulting residue was purified by silica gel chromatography (hexane-ethyl acetate), and the title compound (4084mg, 20.0mmol, 34%) was obtained.
¹H-NMR Spectrum (CDCl3) δ (ppm) :4.07 (2H, s), 6.23 (1H, d, J=4.0Hz), 6.90-7.10 (3H, m), 7.18 (1 H, d, J=4.0Hz), 7.25-7.37 (1 H, m), 9.56 (1 H, s).

### Preparation Example 84. C-(5-(3-Fluoro-benzyl)-furan-2-yl)-methylamine

The title compound (4104mg, 20.0mmol, 100%) was obtained from 5-(3-fluoro-benzyl)-furan-2-carbaldehyde (4084mg ,20.0mmol) according to an analogous method to Preparation Example 77.
¹H-NMR Spectrum (DMSO-d₆) δ (ppm) :1.60 (2H, brs), 3.31 (2H, s), 3.93 (2H, s), 5.97-6.11 (2H, m), 6.82-7.15 (3H, m), 7.20-7.41 (1H, m).

### Preparation Example 85. 2-(3-[1.2.3] Triazol-2-yl-propyl)-isoindol-1.3-dione

To a solution of 1 H-1,2,3-triazole (2000mg, 28.96mmol) in N,N-dimethylformamide (60mL) was added sodium hydride (1159mg, 28.96mmol, 60% in oil), which was stirred for 30 minutes. N-(3-Bromopropyl)phthalimide (7057mg, 26.32mmol) and potassium iodide (431 mg,2.63mmol) were added thereto, and the solution was stirred for 3 hours at 70°. Water was added to the reaction mixture, which was then extracted with ethyl acetate. The organic layer was washed with water and brine, dried over anhydrous magnesium sulfate, and then, concentrated *in vacuo.* The resulting residue was purified by silica gel chromatography (dichloromethane-ethyl acetate), and the title compound (3526mg, 13.75mmol, 47%) was obtained.
¹H-NMR Spectrum (DMSO-d₆) δ (ppm) : 2.19-2.28 (2H, m), 3.65 (2H, t, J=6.8Hz), 4.51 (2H, t, J=6.8Hz), 7.74 (2H, s), 7.80-7.89 (4H, m).

### Preparation Example 86. 3-[1,2,3] Triazol-2-yl-propyl amine

To a mixture solution of 2-(3-[1,2,3]triazol-2-yl-propyl)-isoindol-1,3-dione (1782mg, 6.95mmol) in methanol-tetrahydrofuran (5:4, 27mL) was added hydrazine monohydrate (371µl, 7.65mmol), and the solution was stirred for 5 days at room temperature. Methanol (8mL) was added thereto, followed by further stirring for 3.25 hours under reflux. The reaction mixture was filtered, and the filtrate was concentrated. The resulting residue was dissolved in tetrahydrofuran, adsorbed onto NH silica gel, purified by NH silica gel chromatography (ethyl acetate-methanol), and the title compound (491 mg, 1.36mmol, 19.6%) was obtained.
¹H-NMR Spectrum (DMSO-d₆) δ (ppm) :1.85-1.93 (2H, m), 2.46-2.51 (2H, m), 4.42-4.52 (2H, m), 7.75 (2H, s).

### Preparation Example 87. 3-Benzyl amino-benzonitrile

3-Bromo-benzonitrile (500mg, 2.75mmol), benzylamine (360µl, 3.30mmol), 2,2-bis(diphenylphosphino)-1,1'-binaphthyl (8.6mg, 14µmol), tris(dibenzylideneacetone)dipalladium(0) (19mg, 21µmol) and sodium *tert*-butoxide (370mg, 3.85mmol) were suspended in toluene (10mL), and the mixture was stirred at 80°C for 22 hours under nitrogen atmosphere. The reaction mixture was filtered through Celite pad, then, the filtrate was evaporated *in vacuo,* the residue was purified by NH silica gel column chromatography (hexane : ethyl acetate = 15 : 1), and the title compound (331 mg, 1.59mmol, 58%) was obtained as a pale yellow oil.
¹H-NMR Spectrum (CDCl₃) δ (ppm) : 4.33 (2H, m), 4.34 (1H, s), 6.79-6.83 (2H, m), 6.97 (1 H, dt, J=1.2, 7.7Hz), 7.22 (1 H, t, J=8.1Hz), 7.29-7.39 (5H, m).

### Preparation Example 88. 4-Phenylamino-benzonitrile

The title compound (460mg, 2.37mmol, 86%) was obtained as a white solid from 4-bromo-benzonitrile (500mg, 2.75mmol) and phenylamine (300µl, 3.30mmol) according to an analogous method to Preparation Example 87.
¹H-NMR Spectrum (CDCl₃) δ (ppm) : 6.04 (1H, s), 6.97 (2H, d, J=8.8Hz), 7.12 (1H, t, J=7.3Hz), 7.17 (2H, d, J=7.7Hz), 7.36 (2H, t, J=7.5Hz), 7.48 (2H, d, J=8.8Hz).

### Preparation Example 89. 4-Benzylamino-benzonitrile

The title compound (472mg, 2.27mmol, 83%) was obtained as a pale yellow solid from 4-bromo-benzonitrile (500mg, 2.75mmol) and benzylamine (360µl, 3.30mmol) according to an analogous method to Preparation Example 87.
¹H-NMR Spectrum (CDCl₃) δ (ppm) : 4.38 (2H, d, J=5.5Hz), 4.58 (1 H, s), 6.59 (2H, d, J=8.8Hz), 7.29-7.39 (5H, m), 7.42 (2H, d, J=8.8Hz).

### Preparation Example 90. 2-(3-Bromo-phenyl)-[1,3]dioxolane

3-Bromobenzaldehyde (4.00g, 21.6mmol), ethane-1,2-diol (6.03mL, 108mmol) and toluene-4-sulfonic acid monohydrate (186mg,1.08mmol) were dissolved in toluene (80mL), and the solution was stirred under reflux for 4 hours. An aqueous solution of saturated sodium bicarbonate was added to the reaction solution at 0°C, which was then extracted with ethyl acetate, the organic layer was washed with brine, and the organic layer was dried over anhydrous magnesium sulfate. The solvent was evaporated *in vacuo,* the residue was purified by NH silica gel column chromatography (hexane : ethyl acetate = 20 : 1), and the title compound (4.79g, 20.9mmol, 97%) was obtained as a colorless oil.
¹H-NMR Spectrum (CDCl₃) δ (ppm) : 4.02-4.05 (2H, m), 4.07-4.13 (2H, m), 5.79 (1 H, s), 7.23-7.27 (1 H, m), 7.40 (1 H, d, J=7.7Hz), 7.49 (1 H, dt, J=1.1, 7.1 Hz), 7.64 (1 H, s).

### Preparation Example 91. 2-(3-Phenylsulfanyl-phenyl)-[1,3]dioxolane

2-(3-Bromo-phenyl)-[1,3]dioxolane described in Preparation Example 90 (515mg,2.25mmol) was dissolved in tetrahydrofuran (10mL) under nitrogen atmosphere, *n*-butyl lithium (2.47M hexane solution,1.64mL,4.05mmol) was added at -78°C, the solution was stirred for 15 minutes, then, diphenyldisulphide (540mg, 2.48mmol) was added thereto, followed by stirring for 3 hours. The reaction solution was cooled to 0°C, water was added, the solution was extracted with ethyl acetate, the organic layer was washed with brine and dried over anhydrous magnesium sulfate. The solvent was evaporated *in vacuo,* the residue was purified by silica gel column chromatography (hexane : ethyl acetate = 20 : 1), and the title compound (402mg, 1.56mmol, 69%) was obtained as a colorless oil. ¹H-NMR Spectrum (CDCl₃) δ (ppm) : 4.01-4.06 (2H, m), 4.09-4.14 (2H, m), 5.76 (1H, s), 7.22-7.39 (8H, m), 7.48 (1 H, s).

### Preparation Example 92. 3-Phenylsulfanyl-benzaldehyde

2-(3-Phenylsulfanyl-phenyl)-[1,3]dioxolane described in Preparation Example 91 (396mg, 1.53mmol) was dissolved in a mixture solution of ethanol (5mL), water (5mL), tetrahydrofuran (5mL) and sulfuric acid (1 mL), and the solution was stirred for 2.5 hours under reflux. The reaction solution was cooled to 0°C, an aqueous solution of saturated sodium bicarbonate was added thereto, the solution was extracted with ethyl acetate, the organic layer was washed with brine and dried over anhydrous magnesium sulfate. The solvent was evaporated *in vacuo,* the residue was purified by silica gel column chromatography (hexane : ethyl acetate = 20 : 1), and the title compound (323mg, 1.51 mmol, 98%) was obtained as a colorless oil.
¹H-NMR Spectrum (CDCl₃) δ (ppm) : 7.31-7.39 (3H, m), 7.41-7.50 (3H, m), 7.52 (1H, d, J=7.9Hz), 7.71 (1 H, d, J=7.3Hz), 7.76 (1 H, s), 9.94 (1 H, s).

### Preparation Example 93. (3-Phenylsulfanyl-phenyl)-methanol

3-Phenylsulfanyl-benzaldehyde described in Preparation Example 92 (321 mg, 1.49mmol) was dissolved in ethanol (6mL), sodium borohydride (113mg, 2.98mmol) was added thereto at 0°C, followed by stirring at room temperature for 3 hours. Water was added to the reaction solution, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 5 : 1), and the title compound (220mg, 1.02mmol, 68%) was obtained as a colorless oil.
¹H-NMR Spectrum (CDCl₃) δ (ppm) : 3.73 (2H, s), 4.66 (1H, s), 7.18-7.37 (9H, m).

### Preparation Example 94. 2-(3-Phenylsulfanyl-benzyl)-isoindol-1,3-dione

(3-Phenylsulfanyl-phenyl)-methanol described in Preparation Example 93 (212mg, 0.980mmol), phthalimide (144mg, 0.980mmol), diethylazodicarboxylate (170µl, 1.08mmol) and triphenylphosphine (308mg, 1.18mmol) were dissolved in tetrahydrofuran (4mL), and the solution was stirred overnight at room temperature. Water was added to the reaction solution, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 10 : 1), and the title compound (124mg, 0.359mmol, 37%) was obtained as a solid.
¹H-NMR Spectrum (CDCl₃) δ (ppm) : 4.80 (2H, s), 7.17-7.36 (9H, m), 7.73 (2H, dd, J=2.9, 5.3Hz), 7.86 (2H, dd, J=2.9, 5.3Hz).

### Preparation Example 95. 3-Phenylsulfanyl-benzylamine

2-(3-Phenylsulfanyl-benzyl)-isoindole-1,3-dione described in Preparation Example 94 (123mg, 0.356mmol) was dissolved in ethanol (3mL), hydrazine monohydrate (518µl, 10.7mmol) was added at 0°C, and the solution was stirred under reflux for 2 hours. Water was added to the reaction solution at 0°C, which was then extracted with ethyl acetate, the organic layer was washed with brine and dried over anhydrous magnesium sulfate. The solvent was evaporated *in vacuo,* the residue was purified by NH silica gel column chromatography (ethyl acetate), and the title compound (75mg, 0.35mmol, 98%) was obtained as a colorless oil.
¹H-NMR Spectrum (CDCl₃) δ (ppm): 3.83 (2H, s), 7.19-7.36 (9H, m).

### Preparation Example 96. 2-(4-Bromo-phenyl)-[1,3]dioxolane

4-Bromo-benzaldehyde (4.00g, 21.6mmol), ethane-1,2-diol (6.03mL, 108mmol) and toluene-4-sulfonic acid monohydrate (186mg, 1.08mmol) were dissolved in toluene (80mL), and the solution was stirred under reflux for 4 hours. An aqueous solution of saturated sodium bicarbonate was added to the reaction solution at 0°C, which was then extracted with ethyl acetate, the organic layer was washed with brine, and the organic layer was dried over anhydrous magnesium sulfate. The solvent was evaporated *in vacuo,* the residue was purified by NH silica gel column chromatography (hexane : ethyl acetate = 20 : 1), and the title compound (4.66g, 20.3mmol, 94%) was obtained as a colorless oil.
¹H-NMR Spectrum (CDCl₃) δ (ppm) : 4.01-4.05 (2H, m), 4.07-4.13 (2H, m), 5.77 (1H, s), 7.35 (2H, d, J=8.2Hz), 7.51 (2H, d, J=8.4Hz).

### Preparation Example 97. 2-(4-Benzylsulfanyl-phenyl)-[1,3]dioxolane

The title compound (568mg, 2.09mmol, 48%) was obtained as a solid from 2-(4-bromo-phenyl)-[1,3]dioxolane described in Preparation Example 96 (1.00g, 4.37mmol) and benzyl disulphide (1.18g, 4.81 mmol) according to an analogous method to Preparation Example 91.
¹H-NMR Spectrum (CDCl₃) δ (ppm) : 4.02-4.04 (2H, m), 4.10-4.13 (2H, m), 4.13 (2H, s), 5.76 (1 H, s), 7.23-7.27 (1 H, m), 7.28-7.32 (6H, m), 7.37 (2H, d, J=8.2Hz).

### Preparation Example 98. 4-Benzylsulfanyl-benzaldehyde

The title compound (462mg, 2.02mmol, 97%) was obtained as a white solid from 2-(4-benzyl sulfanyl-phenyl)-[1,3]dioxolane described in Preparation Example 97 (568mg,2.09mmol) according to an analogous method to Preparation Example 92.
¹H-NMR Spectrum (CDCl₃) δ (ppm) : 4.24 (2H, s), 7.26-7.40 (7H, m), 7.75 (2H, d, J=8.6Hz), 9.92 (1 H, s).

### Preparation Example 99. (4-Benzylsulfanyl-phenyl)-methanol

The title compound (406mg, 1.76mmol, 87%) was obtained as a white solid from 4-benzylsulfanyl-benzaldehyde described in Preparation Example 98 (462mg, 2.02mmol) according to an analogous method to Preparation Example 93.
¹H-NMR Spectrum (CDCl₃) δ (ppm) : 4.11 (2H, s), 4.65 (2H, d, J=4.4Hz), 7.20-7.35 (8H, m), 7.37 (1 H, d, J=4.4Hz).

### Preparation Example 100. 2-(4-Benzylsulfanyl-benzyl)-isoindol-1.3-dione

The title compound (563mg, 1.57mmol, 89%) was obtained as a white solid from (4-benzylsulfanyl)-methanol described in Preparation Example 99 (406mg, 1.76mmol) according to an analogous method to Preparation Example 94.
¹H-NMR Spectrum (CDCl₃) δ (ppm) : 4.09 (2H, s), 4.79 (2H, s), 7.20-7.35 (5H, m), 7.24 (2H, d, J=8.2Hz), 7.32 (2H, d, J=8.1 Hz), 7.71 (2H, dd, J=2.9, 5.3Hz), 7.84 (2H, dd, J=2.9, 5.3Hz).

### Preparation Example 101. 4-Benzylsulfanyl-benzylamine

The title compound (260mg,1.13mmo1,72%) was obtained as a white solid from 2-(4-benzylsulfanyl-benzyl)-isoindol-1,3-dione described in Preparation Example 100 (563mg,1.57mmol) according to an analogous method to Preparation Example 95.
¹H-NMR Spectrum (CDCl₃) δ (ppm) : 3.83 (2H, s), 4.10 (2H, s), 7.20 (2H, d, J=8.6Hz), 7.19-7.32 (7H, m).

### Preparation Example 102. (5-Phenylaminomethyl-furan-2-yl)-methanol

Acetic acid 5-formyl-furan-2-ylmethyl ester (2.00g, 11.9mmol), aniline (1.63mL, 17.9mmol) and triacetoxy sodium borohydride (5.04g, 23.8mmol) were suspended in a mixture solution of tetrahydrofuran (40mL) and acetic acid (1mL) at 0°C, and the mixture was stirred at room temperature for 7 hours. An aqueous solution of saturated sodium bicarbonate was added to the reaction solution at 0°C, which was then extracted with ethyl acetate and tetrahydrofuran, the organic layer was washed with brine and dried over anhydrous magnesium sulfate. The solvent was evaporated *in vacuo,* the residue was purified by silica gel column chromatography (hexane : ethyl acetate =1: 1), and a mixture of acetic acid 5-phenylaminomethyl-furan-2-ylmethyl ester and aniline (2.91g) was obtained as a pale yellow oil.
Then, the resulting mixture of acetic acid 5-phenylaminomethyl-furan-2-ylmethyl ester and aniline (2.91 g) as well as potassium carbonate (3.28g ,23.7mmol) were suspended in methanol (60mL), and the solution was stirred overnight at room temperature. The reaction solution was evaporated in *vacuo,* water and ethyl acetate were added to the residue, the organic layer was partitioned, washed with brine and dried over anhydrous magnesium sulfate. The solvent was evaporated *in vacuo,* the residue was purified by NH silica gel column chromatography (hexane : ethyl acetate = 1 : 1), and the title compound (1.99g, 9.79mmol, 82%) was obtained as a colorless oil.
¹H-NMR Spectrum (CDCl₃) δ (ppm) : 4.04 (1H, brs), 4.31 (2H, s), 4.59 (2H, d, J=5.9Hz), 6.19 (1H, d, J=3.1Hz), 6.23 (1 H, d, J=3.1Hz), 6.68 (2H, d, J=7.5Hz), 6.75 (1 H, t, J=7.3Hz), 7.19 (2H, t, J=7.3Hz).

### Preparation Example 103. 2-(5-phenylaminomethyl-furan-2-ylmethyl)-isoindol-1,3-dione

The title compound (603mg, 1.81 mmol, 23%) was obtained as a pale yellow solid from (5-phenylaminomethyl-furan-2-yl)-methanol described in Preparation Example 102 (1.58g, 7.77mmol) according to an analogous method to Preparation Example 94.
¹H-NMR Spectrum (CDCl₃) δ (ppm) : 4.25 (2H, s), 4.82 (2H, s), 6.14 (1 H, d, J=3.1Hz), 6.28 (1 H, d, J=3.1Hz), 6.63 (2H, d, J=7.5Hz), 6.70 (1 H, t, J=7.3Hz), 7.14 (2H, t, J=7.3Hz), 7.72 (2H, dd, J=3.1,5.3Hz), 7.87 (2H, dd, J=3.1,5.3Hz).

### Preparation Example 104. (5-Aminomethyl-furan-2-ylmethyl)-phenyl-amine

The title compound (92mg, 0.46mmol, 60%) was obtained as a pale yellow oil from 2-(5-phenylaminomethyl-furan-2-ylmethyl)-isoindol-1,3-dione described in Preparation Example 103 (251 mg, 0.755mmol) according to an analogous method to Preparation Example 95.
¹H-NMR Spectrum (CDCl₃) δ (ppm) : 3.79 (2H, s), 4.28 (2H, s), 6.06 (1H, d, J=3.1Hz), 6.15 (1H, d, J=3.1Hz), 6.68 (2H, d, J=7.7Hz), 6.74 (1H, t, J=7.3Hz), 7.19 (2H, t, J=7.3Hz).

### Preparation Example 105. (2-(5-[1,3]Dioxolan-2-yl-furan-2-yl)-ethyl)-phenylamine

5-[1,3]Dioxolan-2-yl-furan-2-carbaldehyde (2.03g, 12.1mmol), trimethylsulfonium bromide (1.90g, 12.1 mmol) and potassium hydroxide (779mg, 13.9mmol) were suspended in acetonitrile (75mL), and the solution was stirred overnight at room temperature. Water was added to the reaction solution, which was then extracted with ethyl acetate, the organic layer was washed with brine and dried over anhydrous magnesium sulfate. The solvent was evaporated *in vacuo,* 2-(5-oxiranyl-furan-2-yl)-[1,3]dioxolane (2.25g) was obtained as a pale yellow oil.
The resulting 2-(5-oxiranyl-furan-2-yl)-[1,3]dioxolane (2.25g) and silica gel (5.00g) were suspended in ethyl acetate (40mL), and the solution was stirred for 6.5 hours at room temperature. The reaction solution was filtered, then, the filtrate was evaporated *in vacuo,* and (5-[1,3]dioxolan-2-yl-furan-2-yl)-acetaldehyde (1.57g) was obtained as a yellow oil.
Next, the resulting (5-[1,3]dioxolan-2-yl-furan-2-yl)-acetaldehyde (1.57g), aniline (0.94mL, 10.3mmol) and triacetoxy sodium borohydride (3.76g, 17.2mmol) were suspended in a mixture solution of tetrahydrofuran (30mL) and acetic acid (1mL) at 0°C, and the mixture was stirred at room temperature for 19 hours. An aqueous solution of saturated sodium bicarbonate was added to the reaction mixture at 0°C, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 5 : 1), and the title compound (453mg, 1.75mmol, 14%) was obtained as a brown oil.
¹H-NMR Spectrum (CDCl₃) δ (ppm) : 2.95 (2H, t, J=6.8Hz), 3.43 (2H, t, J=6.8Hz), 3.99-4.06 (2H, m), 4.10-4.15 (2H, m), 5.88 (1 H, s), 6.06 (1 H, d, J=3.3Hz), 6.37 (1 H, d, J=3.1 Hz), 6.62 (2H, dd, J=1.1, 8.6Hz), 6.71 (1H, tt, J=1.1, 7.3Hz), 7.18 (2H, dd, J=7.3, 8.6Hz).

### Preparation Example 106. 5-(2-Phenylamino-ethyl)-furan-2-carbaldehyde

The title compound (314mg, 1.46mmol) was obtained as a light brown oil from (2-(5-[1,3]dioxolan-2-yl-furan-2-yl)-ethyl)-phenylamine described in Preparation Example 105 (453mg, 1.75mmol) according to an analogous method to Preparation Example 44.
¹H-NMR Spectrum (CDCl₃) δ (ppm) : 3.05 (2H, t, J=6.8Hz), 3.53 (2H, t, J=6.8Hz), 6.33 (1H, d, J=3.5Hz), 6.62 (2H, dd, J=1.1, 8.6Hz), 6.73 (1H, t, J=7.3Hz), 7.17-7.21 (3H, m), 9.55 (1 H, s).

### Preparation Example 107. (2-(5-Aminomethyl-furan-2-yl)-ethyl)-phenylamine

The title compound (117mg,0.541mmol,78%) was obtained as a pale yellow oil from 5-(2-phenylamino-ethyl)-furan-2-carbaldehyde described in Preparation Example 106 (150mg,0.697mmol) according to an analogous method to Preparation Example 45.
¹H-NMR Spectrum (CDCl₃) δ (ppm) : 2.92 (2H, t, J=6.8Hz), 3.41 (2H, t, J=6.8Hz), 3.78 (2H, s), 6.00 (1H, d, J=3.1Hz), 6.04 (1H, d, J=2.9Hz), 6.62 (2H, dd, J=1.1, 8.6Hz), 6.71 (1 H, t, J=7.3Hz), 7.18 (2H, dd, J=7.3, 8.6Hz).

### Preparation Example 108. 2-(4-Bromo-thiophen-2-yl)-[1,3]dioxolane

4-Bromo-thiophene-2-carbaldehyde (9.24g,48.4mmol), ethane-1,2-diol (13.5mL,242mmol), toluene-4-sulfonic acid monohydrate (416mg,2.42mmol) were dissolved in toluene (100mL), and the solution was stirred for 1.5 hours under reflux.
An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* the residue was purified by NH silica gel column chromatography (hexane : ethyl acetate = 20 : 1), and the title compound (11.8g, quantitatively) was obtained as a white solid.
¹H-NMR Spectrum (CDCl₃) δ (ppm) : 4.02-4.04 (2H, m), 4.09-4.11 (2H, m), 6.07 (1H, s), 7.08 (1 H, dd, J=0.73, 1.5Hz), 7.22 (1 H, d, J=1.5Hz).

### Preparation Method 109. 2-(4-Phenoxy-thiophen-2-yl)-[1.3]dioxolane

The title compound (5.40g, 21.7mmol, 73%) was obtained as a pale yellow oil from 2-(4-bromo-thiophen-2-yl)-[1,3]dioxolane described in Preparation Example 108 (6.96g, 29.6mmol) and phenol (6.60g, 71.0mmol) according to an analogous method to Preparation Example 33.
¹H-NMR Spectrum (CDCl₃) δ (ppm) : 4.00-4.04 (2H, m), 4.12-4.17 (2H, m), 6.04 (1H, s), 6.60 (1H, d, J=1.7Hz), 6.94 (1 H, d, J=1.7Hz), 7.04 (2H, dd, J=1.1, 8.6Hz), 7.09 (1H, tt, J=1.1, 7.3Hz), 7.32 (2H, dd, J=7.3, 8.6Hz).

### Preparation Example 110. 4-Phenoxy-thiophene-2-carbaldehyde

The title compound (183mg, 0.896mmol, 44%) was obtained as a colorless oil from 2-(4-phenoxy-thiophen-2-yl)-[1,3]dioxolane described in Preparation Example 109 (500mg, 2.01mmol) according to an analogous method to Preparation Example 34.
¹H-NMR Spectrum (CDCl₃) δ (ppm) : 7.04 (1H, dd, J=1.3, 1.6Hz), 7.07 (2H, dd, J=1.1, 8.8Hz), 7.16 (1H, tt, J=1.1, 7.3Hz), 7.35 (2H, dd, J=7.3, 8.6Hz), 7.51 (1H, d, J=1.7Hz), 9.84 (1H, s).

### Preparation Example 111. C-(4-Phenoxy-thiophen-2-yl)-methylamine

The title compound (94mg, 0.458mmol, 51 %) was obtained as a pale yellow oil from 4-phenoxy-thiophene-2-carbaldehyde described in Preparation Example 110 (183mg, 0.896mmol) according to an analogous method to Preparation Example 35.
¹H-NMR Spectrum (CDCl₃) δ (ppm) : 3.99 (2H, s), 6.46 (1H, d, J=1.7Hz), 6.69-6.70 (1H, m), 7.05 (2H, dd, J=1.1, 7.7Hz), 7.09 (1H, t, J=7.5Hz), 7.33 (2H, dd, J=7.5, 8.6Hz).

### Preparation Example 112. 5-Oxo-2,5-dihydro-isoxazole-4-carboxylic acid ethyl ester

2-Ethoxymethylene-malonic acid diethyl ester (5.00g, 23.1mmol), hydroxylamine hydrochloride (4.01 g, 57.8mmol) and triethylamine (8.06mL, 57.8mmol) were dissolved in ethanol (100mL), the solution was stirred at room temperature for 17 hours, then, the solution was stirred for 4.5 hours under reflux. The reaction solution was cooled to room temperature, water was added, and 1 N hydrochloric acid was added until a salt precipitated. The precipitated salt was collected by filtration, and a hydrochloride salt of the title compound (2.39g, 15.2mmol, 66%) was obtained as a white solid.
¹H-NMR Spectrum (CDCl₃) δ (ppm) :1.19 (3H, t, J=7.1Hz), 4.08 (2H, q, J=7.1Hz), 4.90 (1H, brs), 8.51 (1H, d, J=6.8Hz).

### Preparation Example 113. 5-Oxo-2-phenoxy thiocarbonyl-2,5-dihydro-isoxazole-4-carboxylic acid ethyl ester

5-Oxo-2,5-dihydro-isoxazole-4-carboxylic acid ethyl ester described in Preparation Example 112 (1.00g, 5.17mmol), phenyl chlorothionoformate (786µl, 5.69mmol) and pyridine (919µl, 11.4mmol) were dissolved at 0°C in toluene (20mL), and the solution was stirred at room temperature for 17 hours under nitrogen atmosphere. Water was added to the reaction solution at 0°C, which was then extracted with ethyl acetate, and the organic layer was washed with brine. The solvent was evaporated *in vacuo,* the residue was purified by silica gel column chromatography (hexane : ethyl acetate = 1 : 1), and the title compound (1.66g, 5.66mmol, quantitatively) was obtained as a pale yellow solid.
¹H-NMR Spectrum (CDCl₃) δ (ppm) :1.39 (3H, t, J=7.1 Hz), 4.39 (2H, q, J=7.1Hz), 7.16 (2H, dd, J=1.3, 8.6Hz), 7.38 (1 H, t, J=7.1 Hz), 7.49 (2H, dd, J=7.1, 8.8Hz), 9.30 (1 H, s).

### Preparation Example 114. 2-Phenoxy-thiazole-5-carboxylic acid ethyl ester

5-Oxo-2-phenoxythiocarbonyl-2,5-dihydro-isoxazole-4-carboxylic acid ethyl ester described in Preparation Example 113 (500mg, 2.01mmol) was dissolved in acetone (500mL), and the solution was irradiated with light (300nm) for 30 minutes at room temperature. The reaction solution was evaporated *in vacuo,* the residue was purified by silica gel column chromatography (hexane : ethyl acetate = 2 : 1), and the title compound (384mg, 1.54mmol, 90%) was obtained as a pale yellow oil.
¹H-NMR Spectrum (CDCl₃) δ (ppm) :1.35 (3H, t, J=7.1 Hz), 4.33 (2H, q, J=7.1 Hz), 7.26-7.35 (3H, m), 7.47 (2H, dd, J=7.5, 8.4Hz), 7.90 (1 H, s).

### Preparation Example 115. (2-Phenoxy-thiazol-5-yl)-methanol

2-Phenoxy-thiazole-5-carboxylic acid ethyl ester described in Preparation Example 114 (384mg, 1.54mmol) was dissolved in tetrahydrofuran (5mL), and lithium aluminum hydride (292mg, 7.70mmol) was added at 0°C. The solution was stirred at room temperature for 1 hour, then, at 0°C, water (292µl), an aqueous solution of 5N sodium hydroxide (292µl) and water (876µl) were sequentially added thereto. The reaction solution was filtered through Celite pad, then, the filtrate was evaporated *in vacuo,* the residue was purified by silica gel column chromatography (hexane : ethyl acetate = 1 : 4), and the title compound (270mg, 1.30mmol, 85%) was obtained as a white solid.
¹H-NMR Spectrum (CDCl₃) δ (ppm) : 4.73 (2H, d, J=5.9Hz), 7.13 (1H, s), 7.25-7.29 (3H, m), 7.41-7.45 (2H, m).

### Preparation Example 116. 2-(2-Phenoxy-thiazol-5-ylmethy)-isoindol-1,3-dione

The title compound (131mg, 0.389mmol, 30%) was obtained as a colorless oil from (2-phenoxy-thiazole-5-yl)-methanol described in Preparation Example 115 (270mg,1.30mmol) according to an analogous method to Preparation Example 94.
¹H-NMR Spectrum (CDCl₃) δ (ppm) : 4.89 (2H, s), 7.21-7.28 (4H, m), 7.40 (2H, t, j=8.0Hz), 7.73 (2H, dd, J=3.1,5.3Hz), 7.86 (2H, dd, J=2.9, 5.5Hz).

### Preparation Example 117. C-(2-Phenoxy-thiazol-5-yl)-methylamine

The title compound (63mg, 0.31 mmol, 78%) was obtained as a colorless oil from 2-(2-phenoxy-thiazol-5-ylmethyl)-isoindol-1,3-dione described in Preparation Example 116 (131 mg, 0.389mmol) according to an analogous method to Preparation Example 95.
¹H-NMR Spectrum (CDCl₃) δ (ppm) : 3.95 (2H, s), 7.03 (1 H, t, J=1.1Hz), 7.25-7.28 (3H, m), 7.39-7.43 (2H, m).

### Preparation Example 118. 4-Benzyloxy-2-fluoro-benzonitrile

To a solution of 4-hydroxy-2-fluoro-benzonitrile (1.0g, 7.3mmol) in N,N-dimethylformamide (15mL) was added potassium carbonate (2.0g, 15mmol) and benzyl bromide (0.87mL, 7.3mmol), and the solution was stirred at room temperature for 5 hours. Water and ethyl acetate were added to the reaction solution, which was then extracted, washed with brine, then, the solvent was evaporated *in vacuo.* The residue was purified by silica gel column chromatography (hexane : ethyl acetate = 5 : 1), and the title compound (1.5g, 6.7mmol, 92%) was obtained as a white solid.
¹H-NMR Spectrum (CDCl₃) δ (ppm): 5.11 (2H, s), 6.78 (1 H, dd, J=2.4, 11.0Hz), 6.83 (1 H, ddd, J=0.6, 2.4, 8.8Hz), 7.37-7.43 (5H, m), 7.52 (1 H, dd, J=7.5, 8.6Hz).

### Preparation Example 119. 4-[1.3]Dioxolane-2-yl-benzonitrile

4-Formyl-benzonitrile (3.00g, 22.9mmol), ethane-1,2-diol (6.38mL, 115mmol) and toluene-4-sulfonic acid monohydrate (197mg, 1.15mmol) were dissolved in toluene (60mL), and the solution was stirred under reflux for 10 hours. An aqueous solution of saturated sodium bicarbonate was added to the reaction solution at 0°C, which was then extracted with ethyl acetate, the organic layer was washed with brine, and the organic layer was dried over anhydrous magnesium sulfate. The solvent was evaporated *in vacuo,* the residue was purified by NH silica gel column chromatography (hexane : ethyl acetate = 10 : 1), and the title compound (3.78g, 21.6mmol, 94%) was obtained as a white solid.
¹H-NMR Spectrum (CDCl₃) δ (ppm) : 4.04-4.13 (4H, m), 5.85 (1H, s), 7.59 (2H, d, J=8.1 Hz), 7.68 (2H, d, J=8.4Hz).

### Preparation Example 120. 4-[1,3]Dioxolan-2-yl-benzylamine

4-[1,3]Dioxolan-2-yl-benzonitrile described in Preparation Example 119 (3.78g,21.6mmol) was dissolved in tetrahydrofuran (76mL), lithium aluminum hydride (4.09g, 108mmol) was added at 0°C, and the solution was stirred overnight at room temperature. Water (4.09mL), an aqueous solution of 5N sodium hydroxide (4.09mL) and water (12.3mL) were sequentially added to the reaction solution. The reaction solution was filtered through Celite pad, then, the filtrate was evaporated in *vacuo,* and the title compound (3.92g, quantitatively) was obtained as a pale yellow oil.
¹H-NMR Spectrum (CDCl₃) δ (ppm) : 3.89 (2H, s), 4.03-4.16 (4H, m), 5.81 (1H, s), 7.34 (2H, d, J=7.9Hz), 7.46 (2H, d, J=8.1Hz).

### Preparation Example 121. 5-(3-Chloro-phenoxy)-thiophene-2-carbonitrile

5-Nitro-thiophene-2-carbonitrile (5.00g, 32.5mmol), 3-chloro-phenol (6.90mL, 65.0mmol) and potassium carbonate (13.4g, 97.5mmol) were suspended in dimethylsulfoxide (50mL), and the mixture was stirred at 60°C for 4 hours. Water was added to the reaction mixture at 0°C, which was then extracted with ethyl acetate, the organic layer was washed with water twice, and further washed with brine. The organic layer was dried over anhydrous magnesium sulfate. The solvent was evaporated *in vacuo,* the residue was purified by NH silica gel silica gel column chromatography (hexane : ethyl acetate = 20 : 1), and the title compound (5.56g, 23.6mmol, 73%) was obtained as a pale yellow oil.
¹H-NMR Spectrum (CDCl₃) δ (ppm) : 6.49 (1H, d, J=4.2Hz), 7.04 (1H, ddd, J=0.92, 2.4, 8.2Hz), 7.15 (1 H, t, J=2.2Hz), 7.22 (1 H, ddd, J=0.92, 2.0, 8.1 Hz), 7.33 (1 H, t, J=8.2Hz), 7.4 (1 H, d, J=4.2Hz).

### Preparation Example 122. 2-(5-(2-Fluoro-benzyl)-thiophen-2-yl)-[1.3]dioxolane

The title compound (4.33g, 16.4mmol, 54%) was obtained as a pale yellow oil from 2-(5-bromo-thiophen-2-yl)-[1,3]dioxolane (8.00g, 30.4mmol) and 1-bromo methyl-2-fluoro-benzene (4.48mL, 36.5mmol) according to an analogous method to Preparation Example 36.
¹H-NMR Spectrum (CDCl₃) δ (ppm) : 3.97-4.03 (2H, m), 4.06-4.13 (2H, m), 4.14 (2H, s), 6.01 (1H, s), 6.71 (1H, d, J=3.7Hz), 6.98 (1H, d, J=3.9Hz), 7.01-7.08 (2H, m), 7.19-7.23 (2H, m).

### Preparation Example 123. 5-(2-Fluoro-benzyl)-thiophene-2-carbaldehyde

2-(5-(2-Fluoro-benzyl)-thiophen-2-yl)-[1,3]dioxolane described in Preparation Example 122 (4.33g, 16.4mmol) was dissolved in a mixture solvent of methanol (40mL) and water (10mL), 1 N hydrochloric acid (20mL) was added thereto, followed by stirring at room temperature for 1 hour. An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* the residue was purified by silica gel column chromatography (hexane : ethyl acetate = 10 : 1), and the title compound (3.54g, 16.1 mmol, 98%) was obtained as a light yellow oil.
¹H-NMR Spectrum (CDCl₃) δ (ppm) : 4.22 (2H, s), 6.94 (1H, d, J=3.8Hz), 7.05-7.13 (2H, m), 7.21-7.28 (2H, m), 7.60 (1 H, d, J=3.8Hz), 9.81 (1H, s).

### Preparation Example 124. (5-(2-fluoro-benzyl)-thiophene-2-yl)-methanol

5-(2-Fluoro-benzyl)-thiophene-2-carbaldehyde described in Preparation Example 123 (2.81g, 12.7mmol) was dissolved in ethanol (40mL), sodium borohydride (964mg, 25.4mmol) was added at 0°C, and the solution was stirred at room temperature for 1 hour. Water was added to the reaction solution at 0°C, which was then extracted with ethyl acetate, the organic layer was washed with brine and dried over anhydrous magnesium sulfate. The solvent was evaporated in *vacuo,* the residue was purified by silica gel column chromatography (hexane : ethyl acetate = 5 : 1), and the title compound (2.10g, 9.45mmol, 74%) was obtained as a colorless oil.
¹H-NMR Spectrum (CDCl₃) δ (ppm) : 4.14 (2H, s), 4.73 (2H, s), 6.69 (1H, d, J=3.5Hz), 6.82 (1 H, d, J=3.7Hz), 7.02-7.10 (2H, m), 7.19-7.26 (2H, m).

### Preparation Example 125. 2-(5-(2-fluoro-benzyl)-thiophen-2-ylmethyl)-isoindol-1.3-dione

The title compound (1.49g, 4.24mmol, 45%) was obtained as a pale yellow solid from (5-(2-fluoro-benzyl)-thiophen-2-yl)-methanol described in Preparation Example 124 (2.10g, 9.44mmol) according to an analogous method to Preparation Example 94.
¹H-NMR Spectrum (CDCl₃) δ (ppm) : 4.08 (2H, s), 4.92 (2H, s), 6.63 (1H, d, J=3.5Hz), 6.94 (1 H, d, J=3.5Hz), 6.99-7.08 (2H, m), 7.12-7.23 (2H, m), 7.70 (2H, dd, J=3.1,5.5Hz), 7.84 (2H, dd, J=3.1,5.5Hz).

### Preparation Example 126. C-(5-(2-Fluoro-benzyl)thiophen-2-yl)-methylamine

The title compound (901 mg, 4.07mmol, 96%) was obtained as a pale yellow oil from 2-(5-(2-fluoro-benzyl)-thiophen-2-ylmethyl)-isoindol-1,3-dione described in Preparation Example 125 (1.49g, 4.24mmol) according to an analogous method to Preparation Example 95.
¹H-NMR Spectrum (CDCl₃) δ (ppm) : 3.95 (2H, s), 4.12 (2H, s), 6.65 (1H, d, J=3.5Hz), 6.71 (1H, d, J=3.5Hz), 7.01-7.09 (2H, m), 7.18-7.25 (2H, m).

### Preparation Example 127. (5-Bromo-4-phenoxy-thiophen-2-yl)-methanol

2-(4-Phenoxy-thiophen-2-yl)-[1,3]dioxolane described in Preparation Example 109 (4.88g,19.7mmol) and N-bromosuccinimide (3.85g,21.7mmol) were dissolved in tetrahydrofuran (100mL), and the solution was stirred for 4.5 hours at room temperature. An aqueous solution of saturated sodium bicarbonate was added to the reaction solution at 0°C, which was then extracted with ethyl acetate, the organic layer was washed with brine and dried over anhydrous magnesium sulfate. The solvent was evaporated *in vacuo,* and the residue was silica gel-filtered to obtain 2-(5-bromo-4-phenoxy-thiophen-2-yl)-[1,3]dioxolane (5.48g) as a pale yellow oil.
Then, 5-bromo-4-phenoxy-thiophene-2-carbaldehyde (3.11g) was obtained as a colorless oil from 2-(5-bromo-4-phenoxy-thiophen-2-yl)-[1,3]dioxolane (5.48g) according to an analogous method to Preparation Example 34.
Then, the title compound (2.76g, 9.68mmol, 88%) was obtained as a colorless oil from 5-bromo-4-phenoxy-thiophene-2-carbaldehyde (3.11g, 11.0mmol) according to an analogous method to Preparation Example 93.
¹H-NMR Spectrum (CDCl₃) δ (ppm) : 4.70 (2H, s), 6.62 (1 H, s), 6.98 (2H, dd, J=1.1, 8.8Hz), 7.09 (1 H, tt, J=1.1, 7.5Hz), 7.29-7.34 (2H, m).

### Preparation Example 128. 2-(5-Bromo-4-phenoxy-thiophen-2-ylmethyl)-isoindol-1,3-dione

The title compound (2.66g, 6.42mmol, 68%) was obtained as a white solid from (5-bromo-4-phenoxy-thiophen-2-yl)-methanol described in Preparation Example 127 (2.71 g, 9.50mmol) according to an analogous method to Preparation Example 94.
¹H-NMR Spectrum (CDCl₃) δ (ppm) : 4.86 (2H, s), 6.76 (1H, s), 6.95 (2H, dd, J=1.1, 8.8Hz), 7.08 (1H, t, J=7.5Hz), 7.30 (2H, dd, J=7.3, 8.8Hz), 7.70-7.76 (2H, m), 7.83-7.88 (2H, m).

### Preparation Example 129. C-(5-Bromo-4-phenoxy-thiophen-2-yl)-methylamine

The title compound (1.62g, 5.70mmol, 89%) was obtained as a colorless oil from 2-(5-bromo-4-phenoxy-thiophen-2-ylmethyl)-isoindole-1,3-diene described in Preparation Example 128 (2.66g, 6.42mmol) according to an analogous method to Preparation Example 95.
¹H-NMR Spectrum (CDCl₃) δ (ppm) : 3.94 (2H, s), 6.53 (1H, t, J=1.1Hz), 6.97 (2H, dd, J=1.1, 8.6Hz), 7.08 (1H, tt, J=1.1, 7.5Hz), 7.31 (2H, dd, J=7.5, 8.8Hz).

### Preparation Example 130. 3-Aminomethylphenol

The title compound (2.9g, 24mmol, 97%) was obtained as a white solid from 3-hydroxybenzaldehyde (3.0g, 24mmol) according to an analogous method to Preparation Example 38.
¹H-NMR Spectrum (DMSO-d₆) δ (ppm) : 3.60 (2H, s), 6.55 (1H, d, J=7.5Hz), 6.68-6.70 (2H, m), 7.03-7.07 (1 H, m).

### Preparation Example 131. 1-Quinolin-6-yl-ethanone

6-Bromoquinoline (4.4g, 21.3mmol), 1-ethoxyvinyl (tri-*n*-butyl)tin (10g, 27.69mmol) and dichlorobis(triphenylphosphine)palladium(II) (1.2g, 1.7 mmol) were dissolved in toluene (120mL) under nitrogen atmosphere, and the solution was stirred at 80°C for 7 hours. At room temperature, 5M hydrochloric acid (30mL) and tetrahydrofuran (150 mL) were added to the reaction solution, which was then stirred for 15 hours. The reaction solution was poured into an aqueous solution of saturated sodium bicarbonate containing finely ground ice (200mL), the solution was adjusted to pH 8-9, and filtered through Celite pad. The filtrate was extracted with ethyl acetate twice, washed with brine twice, dried over anhydrous magnesium sulfate, and then, filtered. The organic layer was evaporated *in vacuo,* then, residue of reddish brown oil (16.8g) was obtained. Ethyl acetate (20mL) was added thereto, which was dissolved, then, silica gel(80mL) was added, the solvent was evaporated *in vacuo* for adsorption, purification was carried out by silica gel column chromatography (hexane : ethyl acetate = 95 : 5, then hexane : ethyl acetate = 60 : 40), and a yellow solid (4.16 g) was obtained. This yellow solid was dissolved in ethyl acetate (100mL), then, the solution was extracted with 1 M hydrochloric acid (70, 50mL) twice, the aqueous layer was adjusted to pH 8 with sodium bicarbonate, then, the solution was extracted with ethyl acetate twice. The organic layer was washed with brine, dried over anhydrous magnesium sulfate, then, filtered, organic layer was evaporated *in vacuo,* then, the title compound (2.98g, 17.4mmol, 77%) was obtained as a yellow solid.
¹H-NMR Spectrum (Acetone-d₆) δ (ppm) : 2.73 (3H, s), 7.07 (1H, dd, J=8.4, 4.4 Hz), 8.11 (1 H, d, J=9.2 Hz), 8.27 (1 H, dd, J=8.8, 2.0 Hz), 8.50-8.53 (1 H, m), 8.70 (1 H, m), 9.02 (1 H, m).

### Preparation Example 132. 4-Benzyloxy-3-methoxymethoxy-benzonitrile

To a solution of 3,4-dihydroxy-benzonitrile (1.36g, 10mmol) and potassium *tert*-butoxide (1.5g, 13mmol) in dimethylsulfoxide (15mL) was added benzyl chloride (1.5mL, 13mmol) under nitrogen atmosphere at room temperature, and the solution was stirred for 24 hours. Silica gel (100mL) was added portionwise to the reaction solution for adsorption, a pale yellow oil of 4-benzyloxy-3-hydroxy-benzonitrile (2.38g) was obtained by silica gel column chromatography (hexane : ethyl acetate = 7 : 3), in addition, this was purified by NH silica gel column chromatography (hexane : ethyl acetate = 1 : 1, then ethyl acetate, then ethyl acetate : methanol = 90 : 10, then 85 : 15), and 4-benzyloxy-3-hydroxy-benzonitrile (0.588g, 2.61 mmol, 24.8%) was obtained as a pale yellow solid.
To a solution of the resulting 4-benzyloxy-3-hydroxy-benzonitrile (300mg, 1.33mmol) and potassium *tert*-butoxide (300mg, 2.66mmol) in dimethylsulfoxide (4mL) was added chloromethyl methyl ether (0.204mL, 2.66mmol) portionwise under nitrogen atmosphere stirring on an ice bath, and the solution was stirred for 2 days. NH silica gel (25mL) was added to the reaction solution, purification was carried out by NH silica gel column chromatography (hexane : ethyl acetate = 7 : 3) then silica gel column chromatography (hexane : ethyl acetate = 7 : 3), and the title compound (306mg, 0.829mmol, 85.3%) was obtained as a pale yellow solid.
¹H-NMR Spectrum (Acetone-d₆) δ (ppm) :3.46(3H, s), 5.26(2H, s), 5.27(2H,s), 7.26(1 H, d, J=8.4 Hz), 7.36-7.44(4H, m), 7.45(1 H, d, J=2.4 Hz), 7.50-7.53(2H, m).

### Preparation Example 133. 3-(3-Methyl-2-butenyloxy)-benzonitrile

3-Hydroxybenzonitrile (1.19g, 10mmol) and 4-bromo-2-methyl-2-butene (1.66g, 10mmol) were dissolved in N,N-dimethylformamide (5mL), potassium carbonate (1.66g, 12mmol) was added, and the solution was stirred at room temperature for 2 hours. Water (50mL) was added to the reaction solution, which was then extracted with ethyl acetate (50mL). The extract was washed, then, dried over anhydrous magnesium sulfate, and the solvent was evaporated *in vacuo.* The residue was purified by silica gel column chromatography (hexane : ethyl acetate = 95 : 5), and the title compound (1.71g, 10mmol, 99.4%) was obtained as a colorless oil.
¹H-NMR Spectrum (CDCl₃) δ (ppm) :1.73(3H, s), 1.78(3H, s), 4.51(2H, d, J=6.8Hz), 5.48(1H, t, J=6.8Hz), 7.13(1H, dd, J=2.4, 8.4Hz), 7.15(1H, d, J=2.4Hz), 7.23(1H, d, J=8.4Hz), 7.28(1 H, t, J=8.4Hz).

### Preparation Example 134. 3-(3-Methyl-2-butenyloxy)-benzylamine

3-(3-Methyl-2-butenyloxy)-benzonitrile described in Preparation Example 133 (1.71g,10mmol) was dissolved in tetrahydrofuran (20mL), lithium aluminum hydride (0.57g, 15mmol) was added under stirring at room temperature, the solution was heated to 70°C, then stirring continued for 2 hours. The reaction solution was cooled on an ice bath, then, water (0.6mL), an aqueous solution of 15% sodium hydroxide (0.6mL) and water (1.8mL) were added in this order, then, the solid was filtered, and washed with tetrahydrofuran (20mL). The filtrate and the washings were combined, and dried over anhydrous sodium sulfate. The solvent was evaporated *in vacuo,* and the title compound (1.50g, 8.52mmol, 85.2%) was obtained as a colorless oil.
¹H-NMR Spectrum (CDCl₃) δ (ppm) :1.73(3H, s), 1.78(3H, s), 4.51 (2H, d, J=6.8Hz), 4.68(2H, d, J=5.6Hz), 5.48(1H, t, J=6.8Hz), 6.77(1H, dd, J=2.4, 8.4Hz), 6.83(1H, d, J=2.4Hz), 6.87(1 H, s), 7.23(1 H, t, J=8.4Hz).

### Preparation Example 135. (3-Cyanobenzyl)phosphonic acid diethyl ester

3-Cyanobenzyl bromide (9.8g,50mmol) and triethylphosphite (9.97g, 60mmol) were stirred at 140°C for 3 hours. The resulting residue was evaporated, the distillate which has 145°C/1mmHg was collected, and the title compound (10g, 39.5mmol, 79.1 %) was obtained as a colorless oil.

### Preparation Example 136. 3-(2-Methylpropenyl) benzonitrile

Sodium hydride (0.40g, 10mmol, 60% in oil) was suspended in tetrahydrofuran (5mL), (3-cyanobenzyl)phosphonic acid diethyl ester obtained in Preparation Example 135 (2.53g, 10mmol) was added dropwise under stirring at room temperature. After stirring for 1 hour at 60°C, the solution was allowed to room temperature, acetone (0.92g, 20mmol) was added dropwise, and the solution was further stirred for 30 minutes at room temperature. Water (100mL) was added to the reaction solution, which was then extracted with ethyl acetate (50mL). The organic layer was washed with water, then, dried over anhydrous magnesium sulfate, and the solvent was evaporated *in vacuo.* The residue was purified by silica gel column chromatography (hexane : ethyl acetate = 97 : 3), and the title compound (0.44g, 2.80mmol, 28%) was obtained as a colorless oil.
¹H-NMR Spectrum (CDCl₃) δ (ppm) :1.85(3H, s), 1.93(3H, s), 6.23(1H, s), 7.40-7.50(4H, m).

### Preparation Example 137. (3-(2-Methylpropenyl)-benzylamine)

3-(2-Methylpropenyl)benzonitrile described in Preparation Example 136 (0.44g, 2.8mmol) was dissolved in tetrahydrofuran (5mL), lithium aluminum hydride (0.16g,4.2mmol) was added under stirring at room temperature, the solution was heated to 70°C, followed by stirring for 2 hours. The reaction solution was cooled on an ice bath, then, water (0.16mL), an aqueous solution of 15% sodium hydroxide (0.16mL) and water (0.48mL) were sequentially added, then, the solid was filtered, and washed with tetrahydrofuran (10mL). The filtrate and the washings were combined, and dried over anhydrous sodium sulfate. The solvent was evaporated *in vacuo,* and the title compound (0.40g, 2.48mmol, 88.7%) was obtained as a colorless oil.
¹H-NMR Spectrum (CDCl₃) δ (ppm) :1.87(3H, s), 1.90(3H, s), 3.85(2H, s), 6.27(1H, s), 7.11-7.28(4H, m).

### Preparation Example 138. 3-Cyclopentylydenemethylbenzonitrile

Potassium *tert*-butoxide (1.12g,10mmol) was suspended in N,N-dimethylformamide (10mL), and (3-cyanobenzyl)phosphonic acid diethyl ester (2.53g,10mmol) was added dropwise under stirring at room temperature. The solution was stirred at room temperature for 1 hour, then, cyclopentanone (0.84g,10mmol) was added, and the solution was stirred at room temperature for 2 hours. Water (100mL) was added to the reaction solution, which was then extracted with ethyl acetate (50mL). The organic layer was washed with water, then, dried over anhydrous magnesium sulfate, and the solvent was evaporated *in vacuo.* The residue was purified by silica gel column chromatography (hexane : ethyl acetate = 97 : 3), and the title compound (1.32g, 7.21 mol, 72.3%) was obtained as a colorless oil.
¹H-NMR Spectrum (CDCl₃) δ(ppm) : 1.65-1.79(4H, m), 2.47-2.58(4H, m), 6.33(1H, s), 7.40-7.57(4H, m).

### Preparation Example 139. 3-Cyclopentylydenemethyl-benzylamine

3-Cyclopentylydenemethylbenzonitrile described in Preparation Example 138 (1.32g, 7.21 mmol) was dissolved in tetrahydrofuran (10mL), lithium aluminum hydride (0.41 g, 10.8mmol) was added under stirring at room temperature, the solution was heated to 70°C, followed by stirring for 2 hours. The reaction solution was cooled on an ice bath, then, water (0.41 mL), an aqueous solution of 15% sodium hydroxide (0.41 mL) and water (1.23mL) were sequentially added, then, the solid was filtered, and washed with tetrahydrofuran (20mL). The filtrate and the washings were combined, and dried over anhydrous sodium sulfate. The solvent was evaporated *in vacuo,* and the title compound (1.30g, 6.95mmol, 96.4%) was obtained as a colorless oil.

### Preparation Example 140. (5-Bromothiophen-2-yl)methanol

5-Bromo-2-thiophene carboxy aldehyde (25g,131mmol) was dissolved in a mixture solvent of ethanol-tetrahydrofuran (1:1) (200mL), sodium borohydride (1.86g, 49mmol) was added in small amounts under ice-cold stirring, and stirring continued for 30 minutes. An aqueous solution of saturated ammonium chloride (20mL) was added to the reaction solution, which was then stirred for 30 minutes. The reaction solution was extracted with ethyl acetate (100mL), washed with water, dried over anhydrous magnesium sulfate, then, the solvent was evaporated *in vacuo.* The residue was purified by silica gel column chromatography (hexane : ethyl acetate = 90 : 10), and the title compound (6.7g, 34.7mmol, 26.5%) was obtained as a colorless oil.

### Preparation Example 141. 2-Bromo-5-chloromethylthiophene

(5-Bromothiophen-2-yl)methanol described in Preparation Example 140 (6.7g,34.7mmol) was dissolved in diethyl ether (40mL), 10mL of concentrated hydrochloric acid was added thereto, and the solution was vigorously stirred at room temperature for 8 hours. An ice water (200mL) was added to the reaction solution, an aqueous solution of sodium bicarbonate was further added for neutralization, then, the solution was extracted with ethyl acetate (100mL). The organic layer was washed with water, dried over anhydrous magnesium sulfate, then, the solvent was evaporated *in vacuo,* and the title compound (7.3g, 34.5mmol, 99.4%) was obtained as a colorless oil.

### Preparation Example 142. (5-Bromothiophen-2-ylmethyl)phosphonic acid diethyl ester

2-Bromo-5-chloromethylthiophene described in Preparation Example 141 (7.3g, 34.5mmol) and triethylphosphite (6.35g, 38.2mmol) were stirred at 140°C for 3 hours. The resulting residue was purified by silica gel column chromatography (hexane : ethyl acetate = 50 : 50), and the title compound (8.82g, 31.2mmol, 90.3%) was obtained as a reddish brown oil.
¹H-NMR Spectrum (CDCl₃) δ (ppm) :1.27-1.36(6H, m), 3.25(2H, d, J=24Hz), 4.05-4.16(4H, m), 6.72(1 H, d, J=3.6Hz), 6.92(1 H, d, J=3.6Hz).

### Preparation Example 143. 2-Bromo-5-(2-methylpropenyl)-thiophene

(5-Bromothiophen-2-ylmethyl)phosphonic acid diethyl ester described in Preparation Example 142 (3.13g, 10mmol) was dissolved in tetrahydrofuran (20mL), sodium hydride (0.40g, 10mmol, 60% in oil) was added to this solution under stirring at room temperature. The solution was stirred for 30 minutes at 60°C, then, acetone (1 g, 17.2mmol) was added, and the solution was further stirred for 30 minutes. Water (100mL) was added to the reaction solution, which was then extracted with hexane (50mL), the organic layer was washed with water, dried over anhydrous magnesium sulfate, then, the solvent was evaporated *in vacuo.* The residue was purified by silica gel column chromatography (hexane), and the title compound (60mg, 0.27mmol, 2.7%) was obtained as a colorless oil.
¹H-NMR Spectrum (CDCl₃) δ (ppm) :1.90(3H, s), 1.93(3H, s), 6.27(1 H, s), 6.62(1H, d, J=3.6Hz), 6.93(1 H, d, J=3.6Hz).

### Preparation Example 144. 5-(2-Methylpropenyl)-thiophene-2-carbonitrile

2-Bromo-5-(2-methylpropenyl)-thiophene described in Preparation Example 143 (60mg,0.27mmol) was dissolved in N,N-dimethylformamide (1mL), copper cyanide (62mg,0.69mmol) was added at 160°C, and the solution was stirred for 2 hours. The reaction solution was cooled, then, concentrated aqueous ammonia (5mL) was added, and the solution was extracted with diethyl ether (10mL). The organic layer was washed, dried over anhydrous magnesium sulfate, then, the solvent was evaporated *in vacuo.* The residue was purified by silica gel column chromatography (hexane : ethyl acetate = 95 : 5), and the title compound (15mg, 0.092mmol ,34%) was obtained as a colorless oil.
¹H-NMR Spectrum (CDCl₃) δ (ppm):1.97(3H, s), 2.00(3H, s), 6.39(1H, s), 6.85(1H, d, J=4.0Hz), 7.50(1 H, d, J=4.0Hz).

### Preparation Example 145. C-(5-(2-Methylpropenyl)-thiophen-2-yl)-methylamine

5-(2-Methylpropenyl)-thiophene-2-carbonitrile described in Preparation Example 144 (15mg, 0.092mmol) was dissolved in tetrahydrofuran (2mL), lithium aluminum hydride (10mg,0.26mmol) was added under stirring at room temperature, the solution was heated to 70°C, followed by stirring for 2 hours. The reaction solution was cooled on an ice bath, then, water (0.01 mL), an aqueous solution of 15% sodium hydroxide (0.01 mL) and water (0.03mL) were sequentially added, then, the solid was filtered, and washed with tetrahydrofuran (5mL). The filtrate and the washings were combined, and dried over anhydrous sodium sulfate. The solvent was evaporated *in vacuo,* and the title compound (14mg, 0.083mmol, 91.1 %) was obtained as a colorless oil.
¹H-NMR Spectrum (CDCl₃) δ (ppm) :1.91 (3H, s), 1.96(3H, s), 4.09(2H, s), 6.33(1H, s), 6.75(1H, d, J=3.6Hz), 6.95(1H, d, J=3.6Hz).

### Preparation Example 146. 3-lsobutylbenzylamine

3-(2-Methylpropenyl)-benzylamine described in Preparation Example 137 (100mg, 0.621mmol) was dissolved in ethanol (5mL), 10% palladium-carbon (50% water wet, 20mg) was added thereto, and the mixture was stirred for 2 hours at room temperature under hydrogen atmosphere. The catalyst was filtered, then, the solvent was evaporated, and the title compound (58mg, 56.6%) was obtained as a colorless oil.
¹H-NMR Spectrum (CDCl₃) δ (ppm) : 0.90(6H, d, J=6.8Hz), 1.87(1H, dq, J=7.6, 6,8Hz), 2.48(2H, d, J=7.6Hz), 3.84(2H, s), 7.02-7.28(4H, m).

### Preparation Example 147. 2-(2-cyclopropyl vinyl)thiophene

Potassium *tert*-butoxide (1.81 g, 16.2mmol) was suspended in N,N-dimethylformamide (50mL), thiophen-2-ylmethyl triphenylphosphonium chloride (6.38g,16.2mmol) was added while stirring at room temperature under a nitrogen stream, and the mixture was stirred at room temperature for 30 minutes. Then, cyclopropanecarboxyaldehyde (1.13g, 16.2mmol) was added dropwise to the reaction solution, and the solution was stirred for 1 hour. Water (100mL) was added to the reaction solution, which was then extracted with hexane (50mL). The organic layer was passed through silica gel (10g) for filtration, the filtrate was evaporated in *vacuo,* and the title compound (1.27g, 8.47mmol, 52.3%) was obtained as a colorless oil.
¹H-NMR Spectrum (CDCl₃) δ (ppm) : 0.44-0.52(2H, m), 0.76-0.84(2H, m), 1.451.55(1H, m), 5.60(1H, dd, J=8.8, 15.6Hz), 6.83(1H, d, J=3.2Hz), 6.92(1H, dd, J=3.2, 5.2Hz), 7.00(1H, dd, J=3.2, 5.2Hz), 7.05(1 H, J=5.2Hz).

### Preparation Example 148. 5-(2-Cyclopropylyinyl)thiophene-2-carboxy aldehyde

2-(2-Cyclopropylyinyl)thiophene described in Preparation Example 147 (1.27g,8.47mmol) was dissolved in anhydrous diethyl ether (20mL), *n*-butyl lithium (2.47M hexane solution, 4.1mL, 10.2mmol) was added dropwise under stirring on an ice bath, and the solution was stirred for 30 minutes. The reaction solution was cooled on a dry ice-acetone bath, N, N-dimethylformamide (2g,27.4mmol) was added thereto, and the solution was stirred as is for 30 minutes. Acetic acid (1mL) and water (10mL) were sequentially added to the reaction solution, which was then allowed to room temperature. The reaction solution was extracted with ethyl acetate (50mL), then, the organic layer was washed with brine and dried over anhydrous magnesium sulfate. The solvent was evaporated *in vacuo,* the residue was purified by silica gel column chromatography (hexane : ethyl acetate = 95 : 5), and the title compound (960mg, 5.39mmol, 63.7%) was obtained as a colorless oil.
¹H-NMR Spectrum (CDCl₃) δ (ppm) : 0.57-0.63(2H, m), 0.88-0.94(2H, m), 1.53-1.60(1H, m), 5.84(1H, dd, J=9.2, 15.6Hz), 6.59(1H, d, J=15.6Hz), 6.91(1H, d, J=3.6Hz), 7.59(1 H, d, J=3.6Hz), 9.80(1H, s).

### Preparation Example 149. (5-(2-Cyclopropylvinyl)thiophen-2-yl)methanol

5-(2-Cyclopropylvinyl)thiophen-2-aldehyde described in Preparation Example 148 (960mg, 5.39mmol) was dissolved in a mixture solvent of tetrahydrofuran-ethanol (2:1) (30mL), sodium borohydride (100mg,2.64mmol) was added under stirring on an ice bath, and the solution was stirred for 30 minutes. Acetic acid (0.5mL) and water (10mL) were added sequentially to the reaction solution, which was then extracted with ethyl acetate (50mL). The organic layer was washed with an aqueous solution of saturated sodium bicarbonate and brine, and dried over anhydrous magnesium sulfate. The solvent was evaporated *in vacuo,* and the title compound (930mg, 5.19mmol, 96.2%) was obtained as a colorless oil.

### Preparation Example 150. 2-(5-(2-cyclopropylvinyl)thiophen-2-ylmethyl) isoindol-1,3-dione

(5-(2-Cyclopropylvinyl)thiophen-2-yl)methanol described in Preparation Example 149 (930mg, 5.19mmol), triphenylphosphine (2040mg, 7.78mmol) and phthalimide (1140mg, 7.78mmol) were dissolved in tetrahydrofuran (50mL), azodicarboxylic acid dimethyl ester (1140mg, 7.78mmol) was added under stirring at room temperature, and the solution was stirred for 1 hour. Water (50mL) was added to the reaction solution, which was then extracted with ethyl acetate (50mL). The organic layer was washed with water, dried over anhydrous magnesium sulfate, and the solvent was evaporated *in vacuo.* The residue was purified by NH silica gel column chromatography (hexane : ethyl acetate = 9 : 1), and the title compound (330mg, 1.07mmol, 20.6%) was obtained as a white solid.
¹H-NMR Spectrum (CDCl₃) δ (ppm) : 0.44-0.48(2H, m), 0.76-0.80(2H, m), 1.42-1.50(1 H, m), 4.92(2H, s), 5.52(1H, dd, J=8.8, 15.6Hz), 6.47(1H, d, J=15.6Hz), 6.62(1 H, d, J=3.6Hz), 6.93(1H, d, J=3.6Hz), 7.67-7.73(2H, m), 7.82-7.86(2H, m).

### Preparation Example 151. C-(5-(2-Cyclopropylvinyl)thiophen-2-yl)methylamine

2-(5-(2-Cyclopropylvinyl)thiophen-2-ylmethyl)isoindol-1,3-dione described in Preparation Example 150 (330mg,1.02mmol) was dissolved in ethanol (50mL), hydrazine monohydrate (500mg,10mmol) was added thereto, and the solution was stirred under reflux for 2 hours. After cooling to room temperature, 2N sodium hydroxide solution (10mL) and water (100mL) were added to the reaction solution, which was then washed twice with hexane (50mL). The organic layer was dried over anhydrous sodium sulfate, then, the solvent was evaporated *in vacuo,* and the title compound (180mg, 1.01 mmol, 98.6%) was obtained as a colorless oil.
¹H-NMR Spectrum (CDCl₃) δ (ppm) : 0.44-0.48(2H, m), 0.76-0.80(2H, m), 1.42-1.50(1H, m), 3.96(2H, s), 5.52(1H, dd, J=8.8, 15.6Hz), 6.52(1H, d, J=15.6Hz), 6.65(1 H, d, J=3.6Hz), 6.71 (1 H, d, J=3.6Hz).

### Preparation Example 152. C-(5-(2,2-Dicyclopropylvinyl)thiophen-2-yl)methylamine

The title compound was synthesized according to an analogous method to Preparation Example 147 to 151.
¹H-NMR Spectrum (CDCl₃) δ (ppm) : 0.42-0.46(2H, m), 0.61-0.66(2H, m), 0.83-0.88(4H, m), 1.16-1.23(1H, m), 1.96-2.03(1H, m), 3.96(2H, s), 6.30(1H, s), 6.85(1H, d, J=3.2Hz), 6.90(1 H, d, J=3.2Hz).

### Preparation Example 153. Methanesulfonic acid 2-fluoro-benzyl ester

To a solution of 2-fluorobenzyl alcohol (4.40g, 34.9mmol) in dichloromethane (40mL) was added methanesulfonyl chloride (3.24mL, 41.9mmol) and triethylamine (5.84mL, 41.9mmol) on an ice bath, the solution was warmed to room temperature and stirred overnight. The reaction solution was diluted with dichloromethane, washed with an aqueous solution of 5% sodium bicarbonate, then, dried over anhydrous magnesium sulfate, the solvent was evaporated, and the title compound (4.62g, 65%) was obtained as a brown oil. This was used in the next reaction without purification.

### Preparation Example 154. 4-(2-Fluorobenzyloxy)-benzylamine

The title compound (710mg, 14%) was obtained as a yellow oil from *p*-cyanophenol (2.70g, 22.7mmol) and methanesulfonic acid 2-fluoro-benzyl ester described in Preparation Example 153 (4.63g, 22.7mmol) according to an analogous method to Preparation Example 6.

### Preparation Example 155. 4-(4-Fluorobenzyloxy)-benzylamine

4-(4-Fluorobenzyloxy)-benzonitrile (5.89g, quantitatively) was obtained from *p*-cyanophenol (3.00g, 25.2mmol) and 4-fluorobenzyl bromide (4.76g, 25.2mmol) according to an analogous method to Preparation Example 6.
Next, the title compound (1.02g, 67%) was obtained as a yellow solid from the resulting 4-(4-fluorobenzyloxy)-benzonitrile (1.5g, 6.6mmol) according to an analogous method to Preparation Example6.

### Preparation Example 156. 5-(4-chloro-phenoxy)-thiophene-2-carbonitrile

The title compound (770mg, 3.27mmol, 65%) was obtained as a pale yellow oil from 5-nitrothiophene-2-carbonitrile (771 mg, 5mmol) and 4-chlorophenol (643mg, 5mmol) according to an analogous method to Preparation Example 22.
¹H-NMR Spectrum (Acetone-d₆) δ (ppm) :6.72(1H, d, J=4.4Hz), 7.30-7.32(2H, m), 7.50-7.52(2H, m), 7:67(1 H, d, J=4.4Hz)

### Preparation Example 157. C-(5-(4-Chloro-phenoxy)-thiophen-2-yl)-methylamine

The title compound (307mg, 1.28mmol, 86%) was obtained as an orange oil from 5-(4-chloro-phenoxy)-thiophene-2-carbonitrile described in Preparation Example 156 (350mg, 1.49mmol) according to an analogous method to Preparation Example 23.
¹H-NMR Spectrum (DMSO-d₆) δ (ppm) :2.19(2H, brs), 3.81-3.82(2H, m), 6.53-6.54(1 H, m), 6.69-6.70(1H, m), 7.10-7:13(2H, m), 7.42-7.45(2H, m).

### Preparation Example 158. 5-(2-Chloro-phenoxy)-thiophene-2-carbonitrile

The title compound (516mg, 2.19mmol, 44%) was obtained as a white solid from 5-nitrothiophene-2-carbonitrile (771 mg, 5mmol) and 2-chlorophenol (643mg, 5mmol) according to an analogous method to Preparation Example 22.
¹H-NMR Spectrum (Acetone-d₆) δ (ppm) : 6.63(1H, d, J=4.0Hz), 7.35-7.40(1H, m), 7.42-7.50(2H, m), 7.61-7.65(2H, m).

### Preparation Example 159. C-(5-(2-Chloro-phenoxy)-thiophen-2-yl)-methylamine

The title compound (305mg, 1.27mmol, 72%) was obtained as an orange oil from 5-(2-chloro-phenoxy)-thiophene-2-carbonitrile described in Preparation Example 158 (356mg, 1.51mmol) according to an analogous method to Preparation Example 23.
¹H-NMR Spectrum (DMSO-d₆) δ (ppm) :2.10(2H, brs), 3.80(2H, s), 6.48-6.50(1H, m), 6.66-6.72(1 H, m), 7.15-7.23(2H, m), 7.34-7.38(1 H, m), 7.56-7.59(1 H, m).

### Preparation Example 160. 5-(2-Fluoro-phenoxy)-thiophene-2-carbonitrile

The title compound (684mg, 3.12mmol, 77%) was obtained as a colorless oil from 5-nitrothiophene-2-carbonitrile (771 mg, 5mmol) and 2-fluorophenol (673mg, 6mmol) according to an analogous method to Preparation Example 22.
¹H-NMR Spectrum (DMSO-d₆) δ (ppm) :6.76(1H, d, J=4.4Hz), 7.29-7.33(1H, m), 7.35-7.41 (1 H, m), 7.43-7.53(2H, m), 7.79(1H, d, J=4.4Hz).

### Preparation Example 161. C-(5-(2-Fluoro-phenoxy)-thiophen-2-yl)-methylamine

The title compound (298mg, 1.33mmol, 84%) was obtained as a brown oil from 5-(2-fluoro-phenoxy)-thiophene-2-carbonitrile described in Preparation Example 160 (350mg, 1.60mmol) according to an analogous method to Preparation Example 23.
¹H-NMR Spectrum (DMSO-d₆) δ (ppm) :3.79(2H, s), 6.45-6.47(1 H, m), 6.64-6.70(1 H, m), 7.15-7.27(3H, m), 7.34-7.41 (1 H, m).

### Preparation Example 162. 3-Bromo-quinoline-6-carboxylic acid methyl ester

To a mixture of quinoline-6-carboxylic acid methyl ester (0.50g, 2.7mmol) and tetrahydrofuran(10mL) was added 1,3-dibromo-5,5-dimethyl hydantoin (0.76g, 2.7mmol) on an ice bath, and the solution was stirred at 50°C for 4 hours. Water, an aqueous solution of saturated sodium bicarbonate and ethyl acetate were added to the reaction solution, which was then partitioned, the organic layer was washed with brine, the solvent was then evaporated *in vacuo.* The residue was purified by silica gel column chromatography (hexane : ethyl acetate = 4 : 1), and the title compound (69mg, 0.26mmol, 10%) was obtained as a white solid.
¹H-NMR Spectrum (CDCl₃) δ (ppm) : 4.01 (3H, s), 8.13 (1H, d, J=8.8Hz), 8.32 (1H, dd, J=2.0, 8.8Hz), 8.42 (1H, d, J=2.4Hz), 8.52 (1H, d, J=1.8Hz), 9.00 (1H, d, J=2.4Hz).

### Preparation Example 163. 3-Bromo-quinoline-6-carboxylic acid lithium salt

To a mixture of 3-bromo-quinoline-6-carboxylic acid methyl ester described in Preparation Example 162(26mg, 0.098mmol) and tetrahydrofuran (2mL) were added methanol (0.2mL), lithium hydroxide monohydrate (4.1mg, 0.098mmol) and water (0.2mL), and the solution was stirred overnight at room temperature. The solvent was evaporated *in vacuo,* and the title compound (27mg) was obtained.

### Preparation Example 164. Quinoline-6-carboxylic acid methyl ester N-oxide

To a mixture of quinoline-6-carboxylic acid methyl ester (4.7g, 25mmol) and chloroform (80mL) was added 3-chloro-peroxyl benzoic acid (purity 65%, 8.6g, 33mmol) on an ice bath, and the solution was stirred at room temperature for 75 minutes. Water and an aqueous solution of 1 N sodium hydroxide were added to the reaction solution, which was then partitioned, the organic layer was sequentially washed with an aqueous solution of saturated sodium thiosulfate and brine, and dried over anhydrous magnesium sulfate. The solvent was evaporated *in vacuo,* and the title compound (3.8g, 19mmol, 75%) was obtained as a pale yellow solid.
¹H-NMR Spectrum (CDCl₃) δ (ppm) : 4.03 (3H, s), 7.38 (1 H, dd, J=6.0, 8.4Hz), 7.84 (1 H, d, J=8.4Hz), 8.34 (1 H, dd, J=1.8, 9.2Hz), 8.60 (1 H, dd, J=0.9, 6.0Hz), 8.63 (1 H, d, J=1.8Hz), 8.81 (1H, d, J=9.2Hz).

### Preparation Example 165. 2-Chloro-quinoline-6-carboxylic acid methyl ester

Phosphorus oxychloride (10mL) was added to quinoline-6-carboxylic acid methyl ester N-oxide (1.5g, 7.6mmol), and the solution was refluxed for 2 hours. The reaction solution was poured onto an ice, and warmed gradually to room temperature. Ethyl acetate was added to the reaction solution, which was then extracted, and sequentially washed with an aqueous solution of saturated sodium bicarbonate and brine. The solvent was evaporated *in vacuo,* the residue was purified by silica gel column chromatography (hexane : ethyl acetate = 2 : 1), and the title compound (0.47g, 2.1mmol, 28%) was obtained as a white solid.
¹H-NMR Spectrum (CDCl₃) δ (ppm) : 4.01 (3H, s), 7.47 (1H, dd, J=0.6, 8.6Hz), 8.07 (1 H, d, J=8.8Hz), 8.22 (1 H, d, J=8.6Hz), 8.32-8.35 (1 H, m), 8.59 (1 H, s).

### Preparation Example 166. 2-Chloro-quinoline-6-carboxylic acid lithium salt

The title compound (54mg) was obtained as a crude compound from 2-chloro-quinoline-6-carboxylic acid methyl ester described in Preparation Example 165 (40mg, 0.18mmol) according to an analogous method to Preparation Example 163.

### Preparation Example 167. C-(5-(2-Chloro-phenoxy)-thiophen-2-yl)-methylamine

To a solution of lithium aluminum hydride (1.79g, 47.1mmol) in tetrahydrofuran was added aluminum chloride (7.54g, 56.5mmol) on an ice bath, which was then stirred for 10 minutes. To the suspension was 5-(2-chloro-phenoxy)-thiophene-2-carbonitrile described in Preparation Example 158 (2.22g, 9.42mmol) was added on an ice bath, and the solution was stirred for 1 hour. An aqueous ammonia was added to the reaction solution, then, anhydrous magnesium sulfate was added for drying, and the solution was filtered through Celite pad. The filtrate was concentrated *in vacuo,* the residue was purified by NH silica gel column chromatography (heptane / ethyl acetate = 1 /2), and the title compound (2.26g, 9.42mmol, 100%) was obtained as a light brown oil.
¹H-NMR Spectrum (DMSO-d₆) δ (ppm) : 3.81(2H, s), 6.56(1H, d, J=3.6Hz), 6.69(1H, dd, J=1.2, 3.6Hz), 7.05(1 H, dd, J=2.4, 8.4Hz), 7.11 (1 H, t, J=2.0Hz), 7.19(1 H, dt, J=1.2, 8.0Hz), 7.40(1H, t, J=8.0Hz).

### Preparation Example 168. 5-(4-Fluoro-phenoxy)-furan-2-carbaldehyde

To a solution of 4-fluorophenol (2.39g, 21.3mmol) in dimethylsulfoxide (20mL) was added sodium hydride (785mg, 19.6-23.6mmol, 60-72% in oil), and the solution was stirred at room temperature for 20 minutes. Next, a solution of 5-nitro-furan-2-carbaldehyde (3g, 21.3mmol) in dimethylsulfoxide (10mL) was added dropwise, the solution was then stirred for 2 hours at room temperature. The reaction mixture was poured into brine, and the mixture was extracted with ethyl acetate, The fractionated organic layer was dried over anhydrous magnesium sulfate, then concentrated, and the title compound (4.3g, 20.8mmol, 98%) was obtained. ¹H-NMR Spectrum (CDCl₃) δ (ppm) : 5.23 (1H, d, J=4.0 Hz), 7.07-7.24 (5H, m), 9.40 (1 H, s).

### Preparation Example 169. C-(5-(4-Fluoro-phenoxy)-furan-2-yl)methylamine

A suspension of 5-(4-fluoro-phenoxy)-furan-2-carbaldehyde described in Preparation Example 168 (4.3g, 20.9mmol), Raney nickel (1.5g) and 7N ammonia-methanol solution (40mL) was stirred at room temperature for 15 hours under hydrogen atmosphere (1 atm). The reaction solution was filtered through Celite pad to remove the catalyst, and the filtrate was concentrated. The residue was dissolved in ethyl acetate, filtered by NH silica gel lined over a glass filter, and then, this filtrate was concentrated to obtain the title compound (3.5g, 16.9mmol, 81%).
¹H-NMR Spectrum (CDCl₃) δ (ppm) : 3.55 (2H, s), 5.64 (1H, d, J=3.2 Hz), 6.13-6.16 (1 H, m), 7.04-7.10 (2H, m), 7.17-7,24 (2H, m).

### Preparation Example 170. 4-(Pyridin-2-ylmethoxy)-benzonitrile

To a solution of 4-cyanophenol (5g, 42mmol) in N,N-dimethyl formamide (40mL) were added potassium carbonate (17.4g, 126mmol) and 2-picolyl bromide hydrobromide (10.6g, 42mmol), and the solution was stirred at room temperature for 15 hours. The reaction solution was poured into brine, and the solution was extracted with ethyl acetate. The fractionated organic layer was dried over anhydrous magnesium sulfate, then, filtered by NH silica gel lined over a glass filter, and the filtrate was concentrated to obtain the title compound (4.7g, 22.4mmol, 53%). ¹H-NMR Spectrum (CDCl₃) δ (ppm) : 5.25 (2H, s), 7.03-7.07 (2H, m), 7.25-7.29 (1 H, m), 7.48 (1 H, d, J=8.0 Hz), 7.57-7.61 (2H, m), 7.74 (1 H, dt, J=1.6 Hz, 8.0Hz), 8.61-8.63 (1H, m).

### Preparation Example 171. 4-(Pyridin-2-ylmethoxy)-benzylamine

To a solution of 4-(pyridine-2-ylmethoxy)-benzonitrile described in Preparation Example 170 (1.2g, 5.70mmol) in tetrahydrofuran (30mL) was added lithium aluminum hydride (0.22g, 5.80mmol), and the solution was stirred for 2 hours 30 minutes at room temperature. Ice water was added to the reaction solution, which was then stirred for 30 minutes. This mixture was filtered through Celite pad, and washed with ethyl acetate. The filtrate was partitioned, this organic layer was dried over anhydrous magnesium sulfate, then, concentrated, and the title compound (1.1g, 5.13mmol, 90%) was obtained.
¹H-NMR Spectrum (CDCl₃) δ (ppm) : 3.81 (2H, s), 5.21 (2H, s), 6.94-6.98 (2H, m), 7.20-7.26 (3H, m), 7.52 (1 H, d, J=7.8 Hz), 7.74 (1 H, dt, J=1.6 Hz, 7.8 Hz), 8.59-8.62 (1 H, m).

### Preparation Example 172. 4-(Pyridin-2-yloxymethyl)-benzylamine

To a solution of 2-bromopyridine (2.35g, 15.0mmol) and 4-(hydroxymethyl)benzonitrile (3.00g, 22.5mmol) in N,N-dimethylformamide (20mL) was added sodium hydride (0.90g, 22.5mmol; 60% in oil), and the solution was stirred at 70°C for 30 minutes. The reaction solution was allowed to room temperature, then, partitioned in ethyl acetate and water, the organic layer was washed with water, and then, dried over anhydrous magnesium sulfate. The solvent was evaporated, the residue was purified by NH silica gel column chromatography (hexane/ethyl acetate), and a white solid (581mg, 18%) was obtained.
To a solution of the obtained white solid (100mg, 0.476mmol) in tetrahydrofuran (3mL) was added lithium aluminum hydride (45mg, 1.19mmol), and the solution was stirred at room temperature for 1 hour. The reaction solution was partitioned in ethyl acetate and water, and the organic layer was dried over anhydrous magnesium sulfate. The solvent was evaporated, and the title compound (71 mg, 70%) was obtained as a colorless solid, which is a crudely purified product.
¹H-NMR Spectrum (DMSO-d₆) δ (ppm): 3.70(2H, s), 5.31 (2H, s), 6.84-6,87(1H, m), 6.97-7.00(1H, m), 7.32(2H, d, J=8.0Hz), 7.37(2H, d, J=8.0Hz), 7.69-7.74(1H, m), 8.16-8.18(1H, m).

### Preparation Example 173, 5-Bromo-2,3-dihydro-benzofuran

To a solution of 2,3-dihydrobenzofuran (15.0g, 125mmol) in tetrahydrofuran (300mL) was added N-bromosuccinimide (24.5g, 138mmol) at 0°C. The reaction solution was stirred at room temperature for 50 minutes, then, water was added, the solution was extracted with ethyl acetate, and the organic layer was washed with brine. Anhydrous magnesium sulfate was added to the organic layer for drying, which was then filtered, the filtrate was concentrated *in vacuo,* the residue was purified by NH silica gel column chromatography (hexane), and the title compound (24.0g, 97%) was obtained as a colorless oily substance.
¹H-NMR Spectrum (DMSO-d₆) δ (ppm) : 3.19(2H, t, J=8.6Hz), 4.54(2H, t, J=8.6Hz), 6.72(1H, d, J=8.2Hz), 7.22(1H, dd, J=2.2. 8.4Hz), 7.40(1H, s).

### Preparation Example 174 2,3-Dihydro-benzofuran-5-carbaldehyde

To a solution of 5-bromo-2,3-dihydro-benzofuran described in Preparation Example 173 (15.0g, 75.4mmol) in tetrahydrofuran (300mL) was added *n*-butyl lithium (31.2mL, 82.9mmol) at -78°C. The reaction solution was stirred for 85 minutes at -78°C, then, N,N-dimethylformamide (6.42mL, 82.9mmol) was added thereto, followed by stirring at room temperature for 1 hour. 1 N Hydrochloric acid was added to the reaction solution, which was then extracted with ethyl acetate, and the organic layer was washed with brine. Anhydrous magnesium sulfate was added to the organic layer for drying, which was then filtered, the filtrate was concentrated in *vacuo,* the residue was purified by silica gel column chromatography (hexane / ethyl acetate = 5 / 1), and the title compound (10.1 g, 90%) was obtained as a pale yellow oil.
¹H-NMR Spectrum (DMSO-d₆) δ (ppm) : 3.26(2H, t, J=8.6Hz), 4.67(2H, t, J=8.6Hz), 6.96(1 H, d, J=7.9Hz), 7.72(1 H, d, J=8.2Hz), 7.77(1 H, s), 9.82(1 H, s).

### Preparation Example 175. Benzofuran-5-carbaldehyde

To a solution of 2,3-dihydro-benzofuran-5-carbaldehyde described in Preparation Example 174 (6.00g, 40.5mmol) in toluene (120mL) was added 2,3-dichloro-5,6-dicyano-1,4-benzoquinone (18.3g, 81mmol), and the solution was refluxed for 4 hours 30 minutes. The reaction solution was cooled to room temperature, water was added, the solution was extracted with ethyl acetate and tetrahydrofuran, and the organic layer was washed with brine. Anhydrous magnesium sulfate was added to the organic layer for drying, which was then filtered, the filtrate was concentrated *in vacuo,* the residue was purified by silica gel column chromatography (hexane / ethyl acetate =5/1), and the title compound (1.24g, 21 %) was obtained as a pale yellow oil.
¹H-NMR Spectrum (CDCl₃) δ (ppm) : 6.91 (1H, dd, J=0.92, 2.2Hz), 7.63(1H, d, J=8.6Hz), 7.74(1H, d, J=2.4Hz), 7.89(1H, dd, J=1.7, 8.6Hz), 8.16(1H, s), 10.08(1H, s).

### Preparation Example 176. Benzofuran-5-yl-(5-bromo-thiophen-2-yl)-methanol

To a solution of 2,5-dibromothiophene (2.05g, 8.48mmol) in tetrahydrofuran (25mL) was added *n*-butyl lithium (3.48mL, 8.48mmol) at -78°C, and the solution was stirred for 40 minutes. Then, benzofuran-5-carbaldehyde described in Preparation Example 175 (1.24g, 8.48mmol) was added to this reaction solution at -78°C, and the solution was stirred at room temperature for 75 minutes. Water was added to the reaction solution, which was then extracted with ethyl acetate, and the organic layer was washed with brine. Anhydrous magnesium sulfate was added to the organic layer for drying, which was then filtered, the filtrate was concentrated *in vacuo,* the residue was purified by NH silica gel column chromatography (heptane / ethyl acetate = 3 /1), and the title compound (2.11 g, 81 %) was obtained as a light brown oil.
¹H-NMR Spectrum (DMSO-d₆) δ (ppm) : 5.98(1 H, s), 6.40(1 H, d, J=2.9Hz), 6.68(1 H, dd, J=0.92, 3.8Hz), 6.96(1H, dd, J=0.92, 2.2Hz), 7.01(1H, d, J=3.8Hz), 7.34(1H, dd, J=1.5, 8.4Hz), 7.55(1H, d, J=8.4Hz), 7.68(1H, s), 7.99(1H, d, J=2.2Hz).

### Preparation Example 177. 5-(Benzofuran-5-yl-hydroxy-methyl)-thiophene-2-carbonitrile

To a solution of benzofuran-5-yl-(5-bromo-thiophen-2-yl)-methanol described in Preparation Example 176 (755mg, 2.44mmol) in 1-methyl-2-pyrrolidinone (15mL) were added zinc cyanide (344mg, 2.93mmol) and tetrakis(triphenylphosphine)palladium (282mg, 0.244mmol), and the mixture was stirred at 120°C for 3 hours. The reaction mixture was cooled to room temperature, then, aqueous ammonia was added, and the solution was filtered through Celite pad. The mother liquor was extracted with ethyl acetate, and the organic layer was washed with brine. Anhydrous magnesium sulfate was added to the organic layer for drying, which was then filtered, the filtrate was concentrated *in vacuo,* the residue was purified by silica gel column chromatography (heptane / ethyl acetate = 3 / 1), and the title compound (364mg, 58%) was obtained as a pale yellow oil.
¹H-NMR Spectrum (DMSO-d₆) δ (ppm) : 6.13(1H, s), 6.73(1H, s), 6.97(1H, d, J=2.2Hz), 6.99(1H, d, J=3.8Hz), 7.37(1H, dd, J=1.5, 8.4Hz), 7.58(1H, d, J=8.6Hz), 7.72(1 H, s), 7.78(1 H, d, J=3.8Hz), 8.01 (1 H, d, J=2.2Hz).

### Preparation Example 178. C-(5-Benzofuran-5-ylmethyl-thiophen-2-yl)-methylamine

To a solution of lithium aluminum hydride (488mg, 12.9mmol) in tetrahydrofuran (10mL) was added aluminum chloride (1.72g, 12.9mmol) on an ice bath, and the solution was stirred for 10 minutes. 5-(Benzofuran-5-yl-hydroxy-methyl)-thiophene-2-carbonitrile described in Preparation Example 177 (364mg, 1.43mmol) was added to this suspension on an ice bath, and the solution was stirred for 3 hours. Aqueous ammonia was added to the reaction solution, then, anhydrous magnesium sulfate was added for drying, and the solution was filtered through Celite pad. The filtrate was concentrated *in vacuo,* the residue was purified by NH silica gel column chromatography (heptane / ethyl acetate = 1/1), and the title compound (285mg, 82%) was obtained as a light brown oil.
¹H-NMR Spectrum (DMSO-d₆) δ (ppm) : 3.77(2H, s), 4.15(2H, s), 6.70(2H, s), 6.91(1H, d, J=2.2Hz), 7.19(1H, dd, J=1.7, 8.4Hz), 7.50-7.52(2H, m), 7.96(1H, d, J=2.2Hz).

### Preparation Example 179. C-(6-Benzyloxypyridin-3-yl)-methylamine

To a solution of 2,5-dibromopyridine (5.0g, 21.1 mmol) and benzyl alcohol (3.28mL, 31.7mmol) in N,N-dimethylformamide (50mL) was added sodium hydride (1.27g, 31.7mmol; 60% in oil), and the solution was stirred at 70°C for 2 hours. The reaction solution was allowed to room temperature, then, partitioned in ethyl acetate and water, the organic layer was washed with water, and then, dried over anhydrous magnesium sulfate. The solvent was evaporated, the residue was purified by silica gel column chromatography (hexane / ethyl acetate), and a colorless oil (4.60g, 83%) was obtained.
To a solution of the resulting colorless oil (2.0g, 7.60mmol) in N,N-dimethylformamide (20mL) were added zinc cyanide (1.78g, 1.5.2mmol) and tetrakis(triphenylphosphine)palladium (0) (878mg, 0.760mmol) under nitrogen atmosphere, and the solution was stirred at 140°C for 4 hours. The reaction solution was allowed to room temperature, then, partitioned in ethyl acetate and water, the organic layer was washed with water, and then, dried over anhydrous magnesium sulfate. The solvent was evaporated, the residue was purified by silica gel column chromatography (hexane/ethyl acetate), and a pale yellow solid (1.15g, 72%) was obtained.
To a solution of the pale yellow solid (100mg, 0.476mmol) in tetrahydrofuran (3mL) was added lithium aluminum hydride (45mg, 0.120mmol), and the solution was stirred at room temperature for 1 hour. The reaction solution was partitioned in ethyl acetate and water, and the organic layer was dried over anhydrous magnesium sulfate. The solvent was evaporated, and the title compound (75mg, 74%) was obtained as a yellow oil, which was a crudely purified product.
¹H-NMR Spectrum (CDCl₃) δ (ppm) : 4.37-4.38(2H, br d), 5.36(2H, s), 6.83-6.87(1 H, m), 7.30-7.46(4H, m), 7.74-7.76(1 H, m), 8.17(1 H, s), 8.80-8.83(1 H, m).

### Preparation Example 180. C-(6-Benzyl-pyridin-3-yl)-methylamine

A solution of 2,5-dibromopyridine (10g, 42.2mmol) in diethyl ether (260mL) was cooled to -78°C under nitrogen atmosphere, and *n*-butyl lithium (17.8mL, 46.4mmol; 2.6M hexane solution) was added dropwise. This solution was stirred at -78°C for 15 minutes, then, a solution of N,N-dimethylformamide (4.94mL) in diethyl ether solution (10mL) was added dropwise at -78°C. This solution was warmed to 0°C, and further stirred for 2 hours at this temperature. After the reaction was completed, this solution was partitioned in diethyl ether and water. The organic layer was separated, washed with water and dried over anhydrous magnesium sulfate. The solvent was evaporated, the residue was purified by silica gel column chromatography (hexane : ethyl acetate), and an aldehyde derivative (5.82g, 74%) was obtained.
To a solution of the resulting aldehyde derivative (5.82g, 31.3mmol) in toluene (120mL) were added ethyleneglycol (17.5mL, 0.313mol) and D-10-camphor-sulfonic acid (73mg, 0.313mmol), and the solution was refluxed for 7 hours. The reaction mixture was cooled to room temperature, then, washed with an aqueous solution of saturated sodium hydrogen carbonate and brine, and the organic layer was separated. The organic layer was dried over anhydrous magnesium sulfate, then, solvent was evaporated, the residue was purified by silica gel column chromatography (hexane : ethyl acetate), and an acetal (6.65g, 93%) was obtained.
Next, to a suspension of zinc (1.53g, 23.4mmol) in tetrahydrofuran (120mL) was added benzyl bromide (2.1mL, 17.6mmol) dropwise over 15 minutes, at 0°C, and the solution was stirred at this temperature for 4 hours. After 4 hours, bis(triphenylphosphine)nickel(II) chloride (1.58g, 2.42mmol) and a solution of the acetal (3.0g, 13.1 mmol) in tetrahydrofuran (90mL) were added to this suspension, and the solution was stirred at room temperature for 12 hours. After the reaction was completed, this suspension was partitioned in ethyl acetate and an aqueous solution of saturated ammonium chloride. The organic layer was separated, washed with water and brine, and then, dried over anhydrous magnesium sulfate. The solvent was evaporated, the residue was purified by silica gel column chromatography (hexane : ethyl acetate), and a 2-benzyl pyridine derivative (1.08g, 34%) was obtained.
To a mixture solution of the resulting 2-benzyl pyridine derivative (1.08g, 4.48mmol) in methanol and tetrahydrofuran (5mL:4mL) was added 2N hydrochloric acid (5mL), and the solution was stirred at room temperature for 2 hours. Furthermore, to this solution was added 5N hydrochloric acid (8mL) was added in three times, the solution was stirred at room temperature for 24 hours, then, refluxed for 30 minutes. The reaction solution was cooled to room temperature, then, partitioned in ethyl acetate and an aqueous solution of saturated sodium bicarbonate. The organic layer was separated, washed with water, and then, dried over anhydrous magnesium sulfate. The solvent was evaporated, and a formyl derivative (950mg, quantitatively) was obtained.
To a solution of the resulting formyl derivative (950mg, 4.48mmol) in methanol (25mL) was added sodium borohydride (176mg, 4.66mmol), and the solution was stirred at room temperature for 1 hour. The reaction solution was partitioned in ethyl acetate and water. This organic layer was separated, washed with water and dried over anhydrous magnesium sulfate. The solvent was evaporated, and a benzyl alcohol derivative (810mg, 91 %) was obtained.
Methanesulfonyl chloride (0.37mL, 4.83mmol) and triethylamine (0.67mL, 4.84mL) were added to a solution of the resulting benzyl alcohol derivative (810mg, 4.07mmol) in dichloromethane (8mL) that had been cooled to 0°C, and the solution was stirred at room temperature for 14 hours. The reaction mixture was partitioned in dichloromethane and an aqueous solution of saturated sodium bicarbonate. The organic layer was separated, washed with brine, and then, dried over anhydrous magnesium sulfate. The solvent was evaporated, and a methanesulfonate ester derivative (1.09g, 97%) was obtained.
To a solution of the resulting methanesulfonate ester derivative (1.09g, 3.93mmol) in N,N-dimethylformamide (10mL) was added phthalimide potassium salt (757mg, 4.09mmol), and the solution was stirred for 2 hours under reflux. This reaction mixture was cooled to room temperature, and partitioned in ethyl acetate and water. This organic layer was separated, washed with water and brine, and then, dried over anhydrous magnesium sulfate. The solvent was evaporated, the residue was purified by silica gel column chromatography (hexane : ethyl acetate), and a phthalimide derivative (910mg, 71 %) was obtained.
Then, to a solution of the resulting phthalimide derivative (910mg, 2.77mmol) in ethanol (23mL) was added hydrazine monohydrate (144mg, 2.88mmol), and the solution was stirred for 2 hours under reflux. The reaction mixture was cooled to room temperature, then, water was added to this mixture. This mixture was concentrated *in vacuo* until the liquid volume of this mixture became half. This concentrated solution was partitioned in ethyl acetate and water. This organic layer was separated, washed with 2N sodium hydroxide and water, and dried over anhydrous magnesium sulfate. The solvent was evaporated, and the title compound (360mg, 66%) was obtained. This was used in the next reaction without further purification.

### Preparation Example 181. 2-Amino-methyl-5-phenoxy-pyridine

2-Cyano-5-phenoxy-pyridine (150mg, 0.76mmol) and lithium aluminum hydride (58mg, 1.53mmol) were suspended in tetrahydrofuran (5mL) and diethyl ether (5mL), and the solution was stirred for 10 minutes under reflux. The reaction solution was partitioned in water and ethyl acetate. The organic layer was separated, the solvent was evaporated, and the title compound (140mg, brown oil) was obtained as a crude product. ¹H-NMR Spectrum (DMSO-d₆) δ (ppm) : 3.80 (2H, s), 6.70-6.80 (2H, m), 7.00-7.12 (1 H, m), 7.12-7.22 (2H, m), 7.40-7.50 (2H, m), 8.30 (1H, s).

### Preparation Example 182. 5-Benzyloxy-2-methyl-pyridine

To a solution of 3-hydroxy-6-methylpyridine (5.00g, 45.8mmol) in N,N-dimethylformamide (50mL) was added sodium hydride (2.02g, 50.4mmol, 60% in oil) at 0°C, and the solution was stirred for 15 minutes at 0°C. Then, benzyl bromide (5.99mL, 50.4mmol) was added at 0°C, and the solution was stirred for 3.5 hours at room temperature. The reaction solution was partitioned in water and ethyl acetate. This organic layer was separated, washed with water and brine and dried over anhydrous magnesium sulfate. The organic layer was filtered, then, the solvent was evaporated *in vacuo,* the residue was purified by silica gel column chromatography (heptane / ethyl acetate = 2/1), and the title compound (5.99g, 66%) was obtained as a colorless oil.
¹H-NMR Spectrum (CDCl₃) δ (ppm) : 2.49(3H, s), 5.08(2H, s), 7.05(1H, d, J=8.6Hz), 7.17(1 H, dd, J=2.9Hz, 8.4Hz), 7.31-7.44(5H, m), 8.27(1 H, d, J=2.9Hz).

### Preparation Example 183. (5-Benzyloxy-pyridin-2-yl)-methanol

To a solution of 5-benzyloxy-2-methyl pyridine described in Preparation Example 182 (5.99g, 30.1mmol) in methylene chloride (100mL) was added 3-chloroperoxylbenzoic acid (8.79g, 33.1mmol, 65%) at 0°C, and the solution was stirred at room temperature for 2 hours. An aqueous solution of saturated sodium bicarbonate was added to the reaction solution at 0°C, which was partitioned with methylene chloride. This organic layer was separated, washed with an aqueous solution of saturated sodium bicarbonate and brine, and dried over anhydrous magnesium sulfate. The organic layer was filtered, then, the solvent was evaporated *in vacuo,* and 5-benzyloxy-2-methyl-pyridine-1-oxide (7.71g) was obtained as a crude product of a white solid. Next, acetic anhydride (77mL) was added to 5-benzyloxy-2-methyl-pyridin-1-oxide (7.71g), and the solution was stirred for 80 minutes at 120°C. This reaction mixture was cooled to room temperature, then, the solvent was evaporated *in vacuo.* Ethanol (50mL) and an aqueous solution of 5N sodium hydroxide (7mL) were added to the resulting residue, and the solution was stirred at room temperature for 50 minutes. The solvent was evaporated *in vacuo,* this residue was partitioned in brine and ethyl acetate. The organic layer was separated, washed with brine and dried over anhydrous magnesium sulfate. The organic layer was filtered, then, the solvent was evaporated *in vacuo,* the residue was purified by NH silica gel column chromatography (heptane / ethyl acetate = 1 /1), and the title compound (4.17g, 54%) was obtained as a white solid.
¹H-NMR Spectrum (DMSO-d₆) δ (ppm) : 4.46(2H, d, J=5.9Hz), 5.15(2H, s), 5.26(1 H, t, J=5.9Hz), 7.29-7.40(4H, m), 7.42-7.45(3H, m), 8.22(1 H, d, J=2.9Hz).

### Preparation Example 184. 2-(5-Benzyloxy-pyridin-2-ylmethyl)-isoindol-1,3-dione

To a solution of (5-benzyloxy-pyridin-2-yl)-methanol described in Preparation Example 183 (2.00g, 9.29mmol) in tetrahydrofuran (40mL) were added phthalimide (1.50g, 10.2mmol), triphenylphosphine (2.92g, 11.1mmol) and diethyl azodicarboxylate (5.08mL, 11.1mmol, 40% toluene solution) at 0°C, and the solution was stirred at room temperature for 2 hours. The reaction solution was partitioned in water and ethyl acetate. The organic layer was separated, washed with brine and dried over anhydrous magnesium sulfate. The organic layer was filtered, then, the solvent was evaporated *in vacuo,* the residue was purified by silica gel column chromatography (heptane / ethyl acetate = 2 / 1), and the title compound (4.1g, quantitatively) was obtained as a white solid.
¹H-NMR Spectrum (DMSO-d₆) δ (ppm) : 4.84(2H, s), 5.15(2H, s), 7.31-7.45(7H, m), 7.86-7.92(4H, m), 8.20(1 H, d, J=2.9Hz).

### Preparation Example 185. C-(5-Benzyloxy-pyridin-2-yl)-methylamine

2-(5-Benzyloxy-pyridin-2-ylmethyl)-isoindol-1,3-dione described in Preparation Example 184 (4.10g, 11.9mmol) was dissolved in ethanol (40mL) and tetrahydrofuran (40mL). Hydrazine monohydrate (5.77mL, 119mmol) was added to this solution at room temperature, and the solution was stirred under reflux for 50 minutes. The reaction solution was partitioned in water and ethyl acetate. This organic layer was separated, washed with brine and dried over anhydrous magnesium sulfate. The organic layer was filtered, then, the solvent was evaporated *in vacuo,* the residue was purified by NH silica gel column chromatography (ethyl acetate), and the title compound (2.8g, quantitatively) was obtained as a pale yellow solid.
¹H-NMR Spectrum (DMSO-d₆) δ (ppm) : 3.70(2H, s), 5.15(2H, s), 7.30-7.45(7H, m), 8.23(1 H, d, J=3.2Hz).

### Preparation Example 186. 2-Methyl-5-phenoxymethyl-pyridine

Thionyl chloride (1 mL) was added to (6-methyl-pyridin-3-yl)-methanol (300mg, 2.44mmol) at 0°C, and the solution was stirred at room temperature for 20 minutes. The reaction solution was partitioned in an aqueous solution of saturated sodium bicarbonate and ethyl acetate. This organic layer was separated, washed with brine, and then, dried over anhydrous magnesium sulfate. The organic layer was filtered, then, the solvent was evaporated *in vacuo.* N,N-dimethylformamide (5mL), phenol (230mg, 2.44mmol) and potassium carbonate (674mg, 4.88mmol) were added to this residue at room temperature. This reaction mixture was stirred at room temperature for 40 minutes, then, further stirred for at 60°C 40 minutes. The reaction solution was partitioned in water and ethyl acetate. The organic layer was separated, washed with water and brine, and then, dried over anhydrous magnesium sulfate. The organic layer was filtered, then, the solvent was evaporated *in vacuo,* the residue was purified by silica gel column chromatography (heptane / ethyl acetate = 2 / 1), and the title compound (323mg, 66%) was obtained as a white solid.
¹H-NMR Spectrum (CDCl₃) δ (ppm) : 2.58(3H, s), 5.04(2H, s), 6.96-7.00(3H, m), 7.18(1H, d, J=8.2Hz),7.30(2H, t, J=8.8Hz), 7.67(1H, dd, J=2.0Hz, 8.0Hz), 8.56(1H, s).

### Preparation Example 187. (5-Phenoxymethyl-pyridin-2-yl)-methanol

To a solution of 2-methyl-5-phenoxymethyl-pyridine described in Preparation Example 186 (323mg, 1.62mmol) in methylene chloride was added 3-chloroperoxylbenzoic acid (473mg, 1.78mmol, 65%) at 0°C, and the solution was stirred at room temperature for 7 hours. An aqueous solution of saturated sodium bicarbonate was added to the reaction solution at 0°C, which was then partitioned with ethyl acetate. The organic layer was separated, washed with an aqueous solution of saturated sodium bicarbonate and brine, and then, dried over anhydrous magnesium sulfate. The organic layer was filtered, then, the solvent was evaporated *in vacuo.* Acetic anhydride (4mL) was added to this residue, which was then stirred for 30 minutes at 120°C. This reaction solution was cooled to room temperature, then, the solvent was evaporated *in vacuo.* Ethanol (5mL), and an aqueous solution of 5N sodium hydroxide (2mL) were added to this residue, and the solution was stirred at room temperature for 45 minutes. This reaction solution was concentrated *in vacuo,* the residue was partitioned in water and ethyl acetate. The organic layer was separated, washed with brine, and then, dried over anhydrous magnesium sulfate. The organic layer was filtered, then, the solvent was evaporated *in vacuo,* the residue was purified by NH silica gel column chromatography (heptane / ethyl acetate = 1 / 1), and the title compound (167mg, 48%) was obtained as a white solid.
¹H-NMR Spectrum (CDCl₃) δ (ppm) : 3.63(1H, t, J=5.2Hz), 4.78(2H, d, J=4.8Hz), 5.09(2H, s), 6.97-7.02(3H, m), 7.27-7.33(3H, m), 7.78(1H, dd, J=2.2Hz, 8.0Hz), 8.63(1 H, d, J=1.7Hz).

### Preparation Example 188. 2-(5-Phenoxymethyl-pyridin-2-ylmethyl)-isoindol-1,3-dione

To a solution of (5-phenoxymethyl-pyridin-2-yl)-methanol described in Preparation Example 187 (167mg, 0.776mmol) in tetrahydrofuran (4mL) were added phthalimide (126mg, 0.856mmol), triphenylphosphine (244mg, 0.930mmol) and diethyl azodicarboxylate (424µL, 0.931mmol, 40% toluene solution) at 0°C, and the solution was stirred at room temperature for 30 minutes. The reaction solution was partitioned with water and ethyl acetate. The organic layer was separated, washed with brine, and then, dried over anhydrous magnesium sulfate. The organic layer was filtered, then, the solvent was evaporated *in vacuo,* the residue was purified by silica gel column chromatography (heptane / ethyl acetate = 1 / 1), and the title compound (383mg, quantitatively) was obtained as a white solid.
¹H-NMR Spectrum (DMSO-d₆) δ (ppm) : 4.92(2H, s), 5.10(2H, s), 6.94(1H, t, J=7.3Hz), 7.00(2H, dd, J=0.92Hz, 8.8Hz), 7.29(2H, dd, J=7.2Hz, 8.8Hz), 7.44(1H, d, J=7.9Hz), 7.85(1H.dd, J=2.0Hz, 8.0Hz), 7.87-7.94(4H, m), 8.52(1H, d, J=1,8Hz).

### Preparation Example 189. C-(5-Phenoxymethyl-pyridin-2-yl)-methylamine

2-(5-Phenoxymethyl-pyridin-2-ylmethyl)-isoindol-1,3-dione described in Preparation Example 188 (383mg, 1.11mmol) was dissolved in ethanol (3mL) and tetrahydrofuran (3mL). Hydrazine monohydrate (538µL, 11.1mmol) was added to this solution at room temperature, and the solution was stirred under reflux for 1 hour.
The reaction solution was partitioned in water and ethyl acetate. The organic layer was separated, washed with brine, and then, dried over anhydrous magnesium sulfate. The organic layer was filtered, then, the solvent was evaporated *in vacuo,* the residue was purified by NH silica gel column chromatography (ethyl acetate /methanol = 10 / 1), and the title compound (122mg, 51%) was obtained as a colorless oil.
¹H-NMR Spectrum (CDCl₃) δ (ppm) : 4.00(2H, s), 5.07(2H, s), 6.67-7.01(3H, m), 7.29-7.33(3H, m), 7.75(1 H, dd, J=2.4Hz, 8.0Hz), 8.63(1 H, d, J=1.6Hz).

### Preparation Example 190. 4-(6-Fluoro-pyridin-2-yloxymethyl)-benzylamine

To a solution of 2,6-difluoropyridine (500mg, 4.34mmol) and 4-(hydroxymethyl)benzonitrile (867mg,6.51mmol) in N,N-dimethylformamide (3mL) was added sodium hydride (0.26g, 6.51mmol; 60% in oil), and the solution was stirred at 70°C for 7 hours. The reaction solution was allowed to room temperature, then, partitioned in ethyl acetate and water, the organic layer was washed with water, and then, dried over anhydrous magnesium sulfate. The solvent was evaporated, the residue was purified by silica gel column chromatography (hexane / ethyl acetate), and a white solid (734mg, 74%) was obtained.
To a solution of the resulting white solid (734mg, 3.22mmol) in tetrahydrofuran (5mL) was added lithium aluminum hydride (244mg, 6.44mmol), and the solution was stirred at room temperature for 30 minutes. The reaction solution was partitioned in ethyl acetate and water, and the organic layer was dried over anhydrous magnesium sulfate. The solvent was evaporated, and the title compound (662mg, 89%) was obtained as a crude product.
¹H-NMR Spectrum (DMSO-d₆) δ (ppm) : 3.71(2H, s), 5.27(2H, s), 6.71-6.73(1H, m), 6.79-6.81(1H, m), 7.33-7.39(4H, m), 7.86-7.90(1 H, m).

### Preparation Example 191. 4-(3-Chloro-benzyloxy)-benzylamine

To a solution of 4-cyanophenol (2.28g, 19.1mmol) and 3-chlorobenzyl bromide (2.2mL, 16.8mmol) in N,N-dimethylformamide (100mL) was added potassium carbonate (5.88g, 42.5mmol), and the solution was stirred at 50°C for 9 hours. The reaction mixture was partitioned in ethyl acetate and water. This organic layer was separated, washed with 2N sodium hydroxide, water and then brine, and dried over anhydrous magnesium sulfate. The solvent was evaporated, and a crude product (4.20g, quantitatively) was obtained.
To a suspension of aluminum chloride in tetrahydrofuran (40mL) was added lithium aluminum hydride (4.70g, 35.2mmol) while cooling on a water bath. A solution of the crude product (1.15g, 4.71mmol) in tetrahydrofuran (10mL) was added to this suspension, which was then stirred at 0-1 °C for 50 minutes. Concentrated aqueous ammonia (8mL) was added to the reaction mixture, ultrasound was applied, concentrated aqueous ammonia (8mL) was further added, and the solution was stirred for 1 hour at room temperature. This mixture was filtered through Celite pad, and this filtrate was separated. This filtrate was partitioned in tetrahydrofuran, ethyl acetate and water. This organic layer was separated, washed with water and brine, and dried over anhydrous sodium sulfate and anhydrous magnesium sulfate. The solvent was evaporated, and the title compound (1.15g, 99%) was obtained.
¹H-NMR Spectrum (CDCl₃) δ (ppm): 3.03(2H, brs), 3.64(2H, s), 5.11(2H, s), 6.94(2H, d, J=8.0Hz), 7.24(2H, d, J=8.0Hz), 7.37-7.45(3H, m), 7.50(1H, s).

### Preparation Example 192. 4-(3-Methoxy-benzyloxy)-benzylamine

To a solution of 4-cyanophenol (3.31g,27.8mmol) and 3-methoxybenzyl bromide (3.7mL,26.4mmol) in N,N-dimethylformamide (100mL) was added potassium carbonate (8.50g,61.5mmol), and the solution was stirred at 50°C for 5 hours. The reaction mixture was partitioned in diethyl ether and water. This organic layer was separated, washed with 2N sodium hydroxide, water and brine, and dried over anhydrous magnesium sulfate. The solvent was evaporated, and a crude product (6.52g,98%) was obtained.
To a solution of the resulting crude product (3.75g, 15.7mmol) in tetrahydrofuran (80mL) was added lithium aluminum hydride (596mg, 15.7mmol), and the solution was stirred at room temperature for 23 hours. Sodium fluoride (6.6g) was added to the reaction mixture solution at room temperature, which was then cooled with an ice water bath, then, a mixture solution of water (2mL) and tetrahydrofuran (18mL) was added, followed by stirring. This mixture solution was filtered through Celite pad, and what was on Celite was washed with tetrahydrofuran and ethyl acetate. This filtrate was separated, the solvent was evaporated to obtain the title compound (3.84g, quantitatively) as a crude product.
¹H-NMR Spectrum (CDCl₃) δ (ppm) : 3.33(2H, brs), 3.63(2H, s), 3.76(3H, s), 5.06(2H, s), 6.87-6.90(2H, d, J=8.4Hz), 6.93(2H, d, J=8.4Hz), 7.00-7.01(2H, m), 7.23(2H, d, J=8.4Hz), 7.28-7.32(1 H, m).

### Preparation Example 193. 4-(4-Methyl-Dyridin-2-yloxymethyl)-benzylamine

To a solution of 2-fluoro-4-methylpyridine (500mg, 4.50mmol) and 4-(hydroxymethyl)benzonitrile (899mg, 6.75mmol) in N,N-dimethylformamide (3mL) was added sodium hydride (0.27g, 6.75mmol; 60% in oil), and the solution was stirred at 70°C for 1 hour. The reaction solution was allowed to room temperature, then, partitioned in ethyl acetate and water, the organic layer was washed with water, and then, dried over anhydrous magnesium sulfate. The solvent was evaporated, the residue was purified by silica gel column chromatography (hexane / ethyl acetate), and a white solid (833mg, 83%) was obtained.
To a solution of the resulting white solid (200mg, 0.891 mmol) in tetrahydrofuran (3mL) was lithium aluminum hydride (68mg, 1.78mmol), and the solution was stirred at room temperature for 1 hour. The reaction solution was partitioned in ethyl acetate and water, and the organic layer was dried over anhydrous magnesium sulfate. The solvent was evaporated, and the title compound (181 mg, 89%) was obtained as a crude product.
¹H-NMR Spectrum (DMSO-d₆) δ (ppm) : 2.77(3H, s), 3.69(2H, s), 5.29(2H, s), 6.68(1 H, s), 6.82(1 H, d, J=4.4Hz), 7.30-7.39(4H, m), 8.02(1 H, d, J=5.6Hz).

### Preparation Example 194. 4-(5-Methyl-pyridin-2-yloxymethyl)-benzylamine

To a solution of 2-fluoro-5-methylpyridine (1.0g, 9.0mmol) and 4-(hydroxymethyl)benzonitrile (1.8g, 13.5mmol) in N,N-dimethylformamide (5mL) was added sodium hydride (0.54mg, 13.5mmol; 60% in oil), and the solution was stirred for 30 minutes at 70°C. The reaction solution was allowed to room temperature, then, partitioned in ethyl acetate and water, the organic layer was washed with water, and then, dried over anhydrous magnesium sulfate. The solvent was evaporated, the residue was purified by silica gel column chromatography (hexane / ethyl acetate), and a white solid (1.46g, 72%) was obtained.
To a solution of the resulting white solid (500mg, 2.23mmol) in tetrahydrofuran (5mL) was added lithium aluminum hydride (169mg, 4.46mmol), and the solution was stirred at room temperature for 30 minutes. The reaction solution was partitioned in ethyl acetate and water, and the organic layer was dried over anhydrous magnesium sulfate. The solvent was evaporated, and the title compound (457mg, 90%) was obtained as a crude product.
¹H-NMR Spectrum (DMSO-d₆) δ (ppm) : 2.20(3H, s), 3.69(2H, s), 5.27(2H, s), 6.76(1 H, d, J=8.4Hz), 7.30-7.39(4H, m), 7.54(1 H, d, J=7.2Hz), 7.97(1H, s).

### Preparation Example 195. 1-Bromo-4-(2-propoxy-ethyl)-benzene

To a mixture of sodium hydride (66%, 360mg, 15mmol) and tetrahydrofuran (10mL) was added 2-(4-bromophenyl)ethanol (1.5g, 7.5mmol) on an ice bath, the solution was stirred at room temperature for 1 hour. 1-Iodopropane (1.5mL, 15mmol) and N,N-dimethylformamide (10mL) were added to the reaction solution on an ice bath, and the solution was stirred overnight at 45°C. The reaction solution was partitioned with water (100mL) and heptane (200mL). The organic layer was separated, and washed with brine this solvent was evaporated *in vacuo,* the residue was purified by silica gel column chromatography (heptane : ethyl acetate = 30 : 1), and the title compound (0.80g, 3.3mmol, 44%) was obtained as a colorless oil.
¹H-NMR Spectrum (CDCl₃) δ (ppm) : 0.88-0.92(3H, m), 1.55-1.61(2H, m), 2.83(2H, t, J=7.0Hz), 3.36-3.40(2H, m), 3.60(2H, dt, J=1.5, 7.0Hz), 7.10(2H, d, J=8.1 Hz), 7.40(2H, d, J=8.2Hz).

### Preparation Example 196. 4-(2-Propoxy-ethyl)-benzonitrile

1-Bromo-4-(2-propoxy-ethyl)-benzene described in Preparation Example 195 (790mg, 3.2mmol), zinc cyanide (380mg, 3.2mmol) and tetrakis(triphenylphosphine)palladium (190mg, 0.16mmol) were added to N-methylpyrrolidinone (10mL), and this mixture was stirred at 125°C for 4 hours. This reaction mixture was allowed to cool, and water (50mL) and ethyl acetate (50mL) were added thereto. This mixture solution was filtered through Celite pad. The organic layer was separated, then, washed with water (3 times) and brine, and then, concentrated *in vacuo.* The residue was purified by silica gel column chromatography (heptane : ethyl acetate = 8 : 1), and the title compound (120mg, 0.62mmol, 19%) was obtained as a colorless oil.
¹H-NMR Spectrum (CDCl₃) δ (ppm) : 0.89(3H, t, J=7.3Hz), 1.57(2H, tq, J=7.3, 7.3Hz), 2.93(2H, t, J=6.6Hz), 3.38(2H, t, J=6.7Hz), 3.64(2H, t, J=6.6Hz), 7.34(2H, d, J=8.1Hz), 7.58(2H, d, J=8.2Hz).

### Preparation Example 197. 4-(2-Propoxy-ethyl)-benzylamine

To a mixture of lithium aluminum hydride (120mg, 2.5mmol) and tetrahydrofuran (3mL) was added 4-(2-propoxy-ethyl)-benzonitrile described in Preparation Example 196 (120mg, 0.62mmol), the solution was stirred overnight at room temperature. The reaction solution was cooled to 0°C, tetrahydrofuran (30mL), water (0.12mL), an aqueous solution of 5N sodium hydroxide (0.12mL) and water (0.36mL) were sequentially added dropwise. After stirring for 1 hour at room temperature, this reaction mixture was filtered through a filter paper. This filtrate was concentrated *in vacuo,* the residue was filtered using NH-silica gel, and the title compound (123mg, 0.64mmol, 103%) was obtained as a colorless oil.
¹H-NMR Spectrum (CDCl₃) δ (ppm) : 0.91 (3H, t, J=7.4Hz), 1.55-1.62(2H, m), 2.88(2H, t, J=7.3Hz), 3.40(2H, t, J=6.7Hz), 3.63(2H, t, J=7.2Hz), 3.84(2H, s), 7.20(2H, d, J=8.1 Hz), 7.23(2H, d, J=8.2Hz).

### Reference Example A-1. 2.6-Diamino-N-(5-(4-fluoro-phenoxy)-furan-2-ylmethyl)-nicotinamide

2,6-Diamino-nicotinic acid described in Preparation Example A-15 (0.15g, 0.98mmol), triethylamine (0.41 mL, 2.94mmol) and benzotriazol-1-yloxytris(dimethylamino)phosphonium hexafluorophosphate (0.65g, 1.47mmol) were dissolved in N,N-dimethylformamide (5mL), and the solution was stirred at room temperature for 10 minutes. Then, a solution of C-(5-(4-fluoro-phenoxy)-furan-2-yl)methylamine described in Preparation Example 169 (304mg, 1.47mmol) in N,N-dimethylformamide (1mL) was added thereto, followed by stirring at room temperature for 14 hours 50 minutes. After the reaction was completed, reaction solution was poured into brine, the solution was extracted with ethyl acetate, the fractionated organic layer was dried over anhydrous magnesium sulfate and then concentrated. The obtained residue was subjected to silica gel column chromatography, eluted with solvent (ethyl acetate), and the title compound (0.12g, 0.35mmol, 36%) was obtained.
¹H-NMR Spectrum (DMSO-d6) δ (ppm) : 4.28 (2H, d, J=5.2 Hz), 5.64-5.69 (2H, m), 6.10 (2H, br s), 6.22 (1 H, d, J=3.2 Hz), 6.96 (2H, br s), 7.08-7.14 (2H, m), 7.19-7.26 (2H, m), 7.63 (1 H, d, J=8.8 Hz), 8.22 (1 H, t, J=5.2 Hz)

### Reference Example A-2. 2,6-Diamino-N-(5-benzofuran-2-ylmethyl-furan-2-ylmethyl) -nicotinamide

¹H-NMR Spectrum (CDCl₃) δ (ppm) : 4.18(2H, s), 4.31 (2H, d, J=5.6Hz), 5.65 (1H, dd, J=1.2, J=8.4Hz), 6.08 (2H, brs), 6.13-6.20 (2H, m), 6.65 (1H, s), 6.95 (2H, brs), 7.18-7.28 (2H, m), 7.48-7.53 (1H, m), 7.55 (1H, dd, J=0.8, J=5.6Hz), 7.63 (1H, d, J=8.4Hz), 8.23 (1 H, t, J=5.6Hz).

### Reference Example A-3. 2-Amino-N-(5-(4-chloro-phenoxy)-furan-2-ylmethyl)nicotinamide

The title compound (55mg, 0.160mmol, 72.9%) was obtained as a brown oil from 2-aminonicotinic acid (34mg,0.24mmol) and C-(5-(4-chlorophenoxy)furan-2-yl)methylamine described in Preparation Example 47 (50mg, 0.22mmol) according to an analogous method to Reference Example Q-6.
¹H-NMR Spectrum (DMSO-d₆) δ (ppm) : 4.33 (2H, d, J=5.6Hz), 5.73-5.77 (1H, m), 6.29 (1 H, d, J=3.2Hz), 6.54-6.58 (1 H, m), 7.00-7.10 (4H, m), 7.38-7.45 (2H, m), 7.88 (1 H, d, J=7.6Hz), 8.03-8.07 (1 H, m), 8.85 (1 H, t, J=5.6Hz).

### Reference Example A-4. 2-Amino-N-(5-(3-chloro-benzyl)-furan-2-vlmethyl)-nicotinamide

The title compound (110mg, 0.322mmol, 89.4%) was obtained as a white solid from 2-aminonicotinic acid (55mg,0.39mmol) and C-(5-(3-chloro-benzyl)-furan-2-yl)-methylamine described in Preparation Example 56 (80mg, 0.36mmol) according to an analogous method to Reference Example Q-6.
¹H-NMR Spectrum (DMSO-d₆) δ (ppm) : 3.94 (2H, s), 4.34 (2H, d, J=5.6Hz), 6.05 (1H, d, J=3.2Hz), 6.15 (1 H, d, J=3.2Hz), 6.54 (1 H, dd, J=4.0, 8.0Hz), 7.03 (2H, brs), 7.16-7.20 (1H, m), 7.24-7.35 (3H, m), 7.87 (1H, dd, J=1.6, 8.0Hz), 8.05 (1H, dd, J=1.6, 4.0Hz), 8.48 (1 H, t, J=5.6Hz).

### Reference Example A-5. 2-Amino-N-(5-benzyl-furan-2-ylmethyl)-nicotinamide

The title compound (118mg, 0.384mmol, 24%) was obtained from C-(5-benzyl-furan-2-yl)-methylamine (360mg, 1.92mmot) prepared from 5-benzyl-furan-2-carbaldehyde according to an analogous method to Reference Example Q-1, and 2-aminonicotinic acid (221mg, 1.60mmol) according to an analogous method to Example H-1.
¹H-NMR Spectrum (DMSO-d₆) δ (ppm): 3.91 (2H, s), 4.34 (2H, d, J=6.0Hz), 6.00 (1 H, d, J=2.8Hz), 6.14 (1 H, d, J=2.8Hz), 6.54 (1 H, dd, J=4.8, 7.6Hz), 7.04 (2H, brs), 7.13-7.32 (5H, m), 7.87 (1 H, dd, J=1.6, 7.6Hz), 8.05 (1 H, d, J=1.6, 4.8Hz), 8.84 (1 H, t, J=6.0Hz).

### Reference Example A-6. 2-Amino-N-(5-(3-fluoro-benzyl)-furan-2-ylmethyl)-nicotinamide

The title compound (252mg, 0.775mmol, 65%) was obtained from 2-aminonicotinic acid (164mg, 1.19mmol), and C-(5-(3-fluoro-benzyl)-furan-2-yl)-methylamine described in Preparation Example 84 (269mg, 1.31 mmol) according to an analogous method to Reference Example H-1.
¹H-NMR Spectrum (DMSO-d₆) δ (ppm) : 3.95 (2H, s), 4.34 (2H, d, J=5.6Hz), 6.04 (1H, d, J=3.2Hz), 6.15 (1H, d, J=3.2Hz), 6.54 (1H, dd, J=4.8, 7.6Hz), 6.97-7.12 (5H, m), 7.28-7.36 (1H, m), 7.87 (1H, dd, J=2.0, 7.6Hz), 8.04(1H, dd, J=2.0, 4.8Hz), 8.84 (1H, t, J=5.6Hz).

### Reference Example A-7 2-Amino-N-(5-phenylaminomethyl-furan-2-ylmethyl)-nicotinamide

The title compound (49mg, 0.15mmol, 90%) was obtained as a white solid from (5-aminomethyl-furan-2-ylmethyl)-phenyl-amine described in Preparation Example 104 (34mg, 0.17mmol) and 2-amino-nicotinic acid (26mg, 0.19mmol) according to an analogous method to Reference Example A-26.
¹H-NMR Spectrum (DMSO-d₆) δ (ppm) : 4.19 (2H, d, J=6.0Hz), 4.39 (2H, d, J=5.5Hz), 6.02 (1H, t, J=6.0Hz), 6.19 (2H, dd, J=3.1, 11Hz), 6.52 (1H, t, J=7.3Hz), 6.57 (1H, dd, J=4.8, 7.7Hz), 6.62 (2H, d, J=7.7Hz), 7.03-7.07 (4H, m), 7.91 (1H, dd, J=1.7, 7.7Hz), 8.07 (1 H, dd, J=1.7, 4.8Hz), 8.88 (1 H, t, J=5.5Hz).

### Reference Example A-8 2-Amino-N-(5-(2-phenylamino-ethyl)-furan-2-ylmethyl)-nicotinamide

The title compound (29mg,86µmol,89%) was obtained as a white solid from 2-(5-aminomethyl-furan-2-yl)-ethyl)-phenylamine described in Preparation Example 107 (21 mg, 97µmol) and 2-amino-nicotinic acid (16mg, 0.12mmol) according to an analogous method to Reference Example A-26.
¹H-NMR Spectrum (DMSO-d₆) δ (ppm) : 2.83 (2H, t, J=7.3Hz), 3.26 (2H, m), 4.38 (2H, d, J=5.5Hz), 5.61 (1 H, t, J=5.7Hz), 6.12 (1 H, d, J=3.1Hz), 6.17 (1 H, d, J=3.1Hz), 6.52 (1H, t, J=7.3Hz), 6.54-6.59 (3H, m), 7.04-7.08 (4H, m), 7.92 (1H, dd, J=1.8, 7.7Hz), 8.07 (1 H, dd, J=1.8, 4.8Hz), 8.87 (1 H, t, J=5.7Hz).

### Reference Example A-9. 6-Amino-N-(5-(3-fluoro-benzyl)-furan-2-ylmethyl)-nicotinamide

The title compound (265mg, 0.814mmol, 63%) was obtained from 6-aminonicotinic acid (180mg, 1.30mmol) and C-(5-(3-fluoro-benzyl)-furan-2-yl)-methylamine described in Preparation Example 84 (293mg, 1.43mmol) according to an analogous method to Reference Example H-1.
¹H-NMR Spectrum (DMSO-d₆) δ (ppm) : 3.95 (2H, s), 4.33(2H, d, J=5.6Hz), 6.03 (1H, d, J=3.2Hz), 6.12 (1 H, d, J=3.2Hz), 6.38 (1 H, d, J=8.8Hz), 6.45 (2H, brs), 7.00-7.09 (3H, m), 7.28-7.36 (1 H, m), 7.78 (1 H, dd, J=2.4, 8.4Hz), 8.43 (1H, d, J=2.4Hz), 8.56 (1 H, t, J=5.6Hz).

### Reference Example A-10. 2,6-Diamino-N-(4-benzyloxy-benzyl)-nicotinamide

2,6-Diamino-nicotinic acid described in Preparation Example A-15 (0.6g, 3.92mmol), triethylamine (1.64mL, 11.8mmol) and benzotriazole-1-yloxy tris(dimethylamino)phosphonium hexafluorophosphate (2.6g, 5.9mmol) were dissolved in N,N-dimethylformamide (200mL), 4-benzyloxy-benzylamine described in Preparation Example 1 (1.25g, 5.9mmol) was added thereto, and the solution was stirred at room temperature for 16 hours. After the reaction was completed, the reaction solution was poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1, then ethyl acetate), the resulting solid was washed with solvent (chloroform : ethyl acetate = 2 : 1), and the title compound (0.37g, 1.1 mmol, 27%) was obtained.
¹H-NMR Spectrum (DMSO-d₆) δ (ppm) : 4.29 (2H, d, J=6.0Hz), 5.07 (2H, s), 5.66 (1 H, d, J=8.4Hz), 6.06 (2H, br s), 6.91-6.97 (4H, m), 7.19 (2H, d, J=8.8Hz), 7.29-7.45 (5H, m), 7.65 (1 H, d, J=8.4Hz), 8.27 (1 H, t, J=6.0Hz).

### Reference Example A-11. 2,6-Diamino-N-(4-(2-fluoro-benzyloxy)-benzyl)-nicotinamide

2,6-Diamino-nicotinic acid described in Preparation Example A-15 (200mg, 1.3mmol), triethylamine (0.54mL, 3.87mmol) and benzotriazol-1-yloxytris(dimethylamino)phosphonium hexafluorophosphate (862mg, 1.95mmol) were added to N,N-dimethylformamide (20mL), and the solution was stirred at room temperature for 20 minutes. Next, 4-(2-fluoro-benzyloxy)-benzylamine described in Preparation Example 154 (453mg, 1.96mmol) was added thereto, and the solution was stirred at room temperature for 14 hours. After the reaction was completed, the reaction solution was poured into brine, the solution was extracted with ethyl acetate, the fractionated organic layer was dried over anhydrous magnesium sulfate and then concentrated. The obtained residue was subjected to silica gel column chromatography, eluted with solvent (ethyl acetate) and the title compound (147mg, 0.40mmol, 31%) was obtained.
¹H-NMR Spectrum (DMSO-d₆) δ (ppm) : 4.30 (2H, d, J=6.0 Hz), 5.11 (2H, s), 5.67 (1H, d, J=8.8 Hz), 6.06 (2H, br s), 6.89-7.02 (4H, m), 7.16-7.28 (4H, m), 7.37-7.44 (1 H, m), 7.51-7.56 (1 H, m), 7.66 (1 H, d, J=8.8 Hz), 8.28 (1 H, t, J=6.0 Hz).

### Reference Example A-12. 2,6-Diamino-N-(4-(pyridin-2-ylmethoxy)-benzyl)-nicotinamide

¹H-NMR Spectrum (DMSO-d₆) δ (ppm) : 4.29 (2H, d, J=6.0Hz), 5.15 (2H, s), 5.66 (1 H, d, J=8.8 Hz), 6.06 (2H, br s), 6.91-6.98 (4H, m), 7.17-7.23 (2H, m), 7.31-7.35 (1H, m), 7.49 (1 H, d, J=7.6 Hz), 7.65 (1 H, d, J=8.8 Hz), 7.82 (1 H, dt, J=2.0, 7.6 Hz), 8.28 (1 H, t, J=6.0 Hz), 8.55-8.58 (1 H, m).

### Reference Example A-13. 2,6-Diamino-N-(4-phenoxymethyl-benzyl)-nicotinamide

¹H-NMR Spectrum (CDCl₃) δ (ppm) : 4.51(2H, brs), 4.57(2H, d, J=5.6Hz), 5.06(2H, s), 5.77(1H, d, J=8.4Hz), 6.04(1H, brs), 6.45(2H, brs), 6.96-6.98(3H, m), 7.28-7.31 (3H, m), 7.34-7.43(4H, m).

### Reference Example A-14. 2,6-biamino-N-(4-(thiophen-3-ylmethoxy)-benzyl)-nicotinamide

¹H-NMR Spectrum (DMSO-d₆) δ (ppm) : 4.28 (2H, d, J=6.0 Hz), 5.04 (2H, s), 5.65 (1 H, d, J=8.4 Hz), 6.05 (2H, br s), 6.89-6.99 (4H, m), 7.13-7.21 (3H, m), 7.51-7.55 (2H, m), 7.64 (1 H, d, J=8.4 Hz), 8.26.(1H, t, J=6.0 Hz).

### Reference Example A-15. 2-amino-N-(4-(2-nitro benzyloxy)-benzyl)-nicotinamide

To a solution of sodium 4-(((2-aminopyridin-3-carbonyl)-amino)-methyl)-phenolate described in Preparation Example A+-1 (100mg, 0.377mmol) in N,N-dimethylformamide (2.5mL) was added O-Nitrobenzyl chloride (65mg, 0.379mmol), and the solution was stirred at room temperature for 2 hours. Ethyl acetate and water were added to the reaction solution, which was then partitioned, the organic layer was washed with water and dried over anhydrous magnesium sulfate. The residue was purified by silica gel column chromatography (hexane : ethyl acetate), and the title compound (51mg, 37%) was obtained as a white solid.
¹H-NMR Spectrum (CDCl₃) δ (ppm) : 4.55(2H, d, J=5.6Hz), 5.50(2H, s), 6.23(1 H, brs), 6.39(2H, brs), 6.57-6.60(1 H, m), 6.98(2H, d, J=8.6Hz), 7.30(2H, d, J=8.6Hz), 7.50-7.52(1H, m), 7.58-7.60(1H, m), 7.67-7.71(1H, m), 7.87-7.89(1H, m), 8.15-8.18(2H, m).

### Reference Example A-16. 2-Amino-N-(4-(2-amino-benzyloxyl-benzyl)-nicotinamide

2-Amino-N-(4-(2-nitrobenzyloxy)-benzyl)-nicotinamide described in Referece Example A-15 was dissolved in a mixture solvent of ethanol-tetrahydrofuran-water (3:1:1.5), iron powder (4mg) and ammonium chloride (85mg) were added thereto, and the solution was stirred overnight under reflux. In addition, iron powder (10mg) and ammonium chloride (20mg) were added, and the solution was stirred under reflux for 2 hours. The reaction solution was allowed to room temperature, filtered through Celite pad to remove insoluble matter, and the filtrate was concentrated in *vacuo.* The residue was purified by NH silica gel chromatography (hexane : ethyl acetate), and the title compound (9mg, 98%) was obtained as a pale brown solid. ¹H-NMR Spectrum (CDCl₃) δ (ppm) : 4.53(2H, d, J=5.6Hz), 5.04(2H, s), 6.24(1 H, brs), 6.51 (2H, brs), 6.57-6.60(1H, m), 6.72-6.79(2H, m), 6.99(2H, d, J=8.4Hz), 7.16-7.20(2H, m), 7.28(2H, d, J=8.4Hz), 7.58-7.60(1 H, m), 8.13-8.14(1 H, m).

### Reference Example A-17. 2-Amino-N-(4-benzyloxy-benzyl)-nicotinamide

The title compound (257mg, 0.771 mmol, 72%) was obtained from 2-aminonicotinic acid (148mg, 1.07mmol) and 4-benzyloxy-benzylamine described in Preparation Example 1 (251 mg, 1.18mmol) according to an analogous method to Reference Example H-1.
¹H-NMR Spectrum (DMSO-d₆) δ (ppm) : 4.34 (2H, d, J=6.0Hz), 5.06 (2H, s), 6.55 (1 H, dd, J=4.8, 7.6Hz), 6.94 (2H, d, J=8.0Hz), 7.03 (2H, brs), 7.20 (2H, d, J=8.0Hz),7.29 (1H, t, J=6.4Hz), 7.36 (2H, dd, J=6.4,6.8Hz), 7.41 (2H, d, J=6.8Hz), 7.90 (1 H, d, J=7.6Hz), 8.05 (1 H, d, J=4.8Hz), 8.88 (1 H, t, J=6.0Hz).

### Reference Example A-18. 2-Amino-N-(3-phenoxy-benzyl)-nicotinamide

The title compound (87mg, 0.27mmol, 26%) was obtained from 2-aminonicotinic acid (144mg,1.04mmol) and 3-phenoxy-benzylamine described in Preparation Example 4 (228mg, 1.15mmol) according to an analogous method to Example H-1.
¹H-NMR Spectrum (DMSO-d₆) δ (ppm) : 4.40 (2H, d, J=5.6Hz), 6.56 (1H, dd, J=4.8, 7.6Hz), 6.85 (1 H, dd, J=1.2, 8.0Hz), 6.92-7.05 (5H, m), 7.06 (1 H, d, J=7.6Hz), 7.11 (1 H, dd, J=7.6, 8.0Hz), 7.29-7.40 (3H, m), 7.89 (1 H, dd, J=2.0, 7.6Hz), 8.06 (1 H, dd, J=2.0, 4.8Hz), 8.96 (1 H, t, J=5.6Hz).

### Reference Example A-19. 2-Amino-N-(4-(3-fluoro-benzyloxy)-benzyl)-nicotinamide

The title compound (172mg, 0.489mmol, 40%) was obtained from 2-aminonicotinic acid (170mg,1.23mmol) and 4-(3-fluoro-benzyloxy)-benzylamine described in Preparation Example 6 (312mg, 1.35mmol) according to an analogous method to Reference Example H-1.
¹H-NMR Spectrum (DMSO-d₆) δ (ppm) : 4.35 (2H, d, J=6.0Hz), 5.10 (2H, s), 6.45-6.60 (1 H, m), 6.85-7.46 (10H, m), 7.85-7.92 (1 H, m), 8.03-8.07 (1 H, m), 8.75-8.92 (1 H, m).

### Reference Example A-20. 2-Amino-N-(4-(2-fluoro-benzyloxy)-benzyl)-nicotinamide

The title compound (67mg, 0.19mmol, 45%) was obtained from 2-aminonicotinic acid (58mg, 0.42mmol) and 4-(2-fluoro-benzyloxy)-benzylamine described in Preparation Example 154 (117mg, 0.506mmol) according to an analogous method to Reference Example H-1 (with the proviso that only the reaction temperature was changed to 60°C).
¹H-NMR Spectrum (DMSO-d₆) δ (ppm) : 4.34 (2H, d, J=6.0Hz), 5.10 (2H, s), 6.56 (1H, dd, J=4.8, 7.6Hz), 6.90-7.00 (2H, m), 7.04 (2H, brs), 7.15-7.28 (4H, m), 7.35-7.44 (1H, m), 7.50-7.58 (1H, m), 7.90 (1H, dd, J=1.2, 7.6Hz), 8.05 (1H, dd, J=1.2, 4.8Hz), 8.86-8.95 (1H, m).

### Reference Example A-21. 2-Amino-N-(4-(4-fluoro-benzyloxy)-benzyl)-nicotinamide

The title compound (187mg, 0.532mmol, 96%) was obtained from 2-aminonicotinic acid (77mg, 0.56mmol) and 4-(4-fluoro-benzyloxy)-benzylamine described in Preparation Example 155 (155mg, 0.670mmol) according to an analogous method to Reference Example H-1 (with the proviso that only the reaction temperature was changed to 60°C).
¹H-NMR Spectrum (DMSO-d₆) δ (ppm) :4.34 (2H, d, J=5.6Hz), 5.04 (2H, s), 6.56 (1H, dd, J=5.2, 8.0Hz), 6.94 (2H, d, J=8.4Hz), 7.03 (2H, brs), 7.12-7.25 (4H, m), 7.46 (2H, dd, J=6.0, 8.4Hz), 7.90 (1 H, d, J=8.0Hz), 8.05 (1 H, d, J=5.2Hz), 8.88 (1 H, t, J=5.6Hz).

### Reference Example A-22. 2-Amino-N-(4-benzyloxy-benzyl)-thionicotinamide

A mixture of 2-amino-N-(4-benzyloxy-benzyl)-nicotinamide described in Reference Example A-17 (220mg, 0.67mmol), 2,4-bis(4-methoxyphenyl)-1,3-dithia-2,4-diphosphetane-2,4-disulphide (Lawesson's reagent) (670mg, 1.7mmol) and toluene (8mL) was stirred at 80°C for 15 minutes, then, refluxed for 45 minutes. After cooling, the precipitate was filtered, and the filtrate was evaporated *in vacuo.* Purification was carried out by NH silica gel column chromatography (ethyl acetate), and the title compound (28mg, 0.080mmol, 12%) was obtained as a pale yellow oil.
¹H-NMR Spectrum (CDCl₃) δ (ppm) : 4.87 (2H, d, J=4.9Hz), 5.08 (2H, s), 5.88 (2H, brs), 6.62 (1H, dd, J=4.9, 7.5Hz), 6.98 (2H, d, J=8.6Hz), 7.30 (2H, d, J=8.6Hz), 7.32-7.44 (6H, m), 7.71 (1 H, brs), 8.07 (1 H, dd, J=1.7, 4.9Hz).

### Reference Example A-23 2-Amino-N-(3-(2-butynyloxy)benzyl)-nicotinamide

Trifluoroacetic acid salt of the title compound (10mg, 0.024mmol, 49%) was obtained from 2-amino-N-(3-hydroxybenzyl)-nicotinamide described in Preparation Example A+-17 (12mg, 0.050mmol) and 1-bromo-2-butyne (6.6mg, 0.050mmol) according to an analogous method to Reference Example E-43.
MS m/e (ESI) 296.3 (MH⁺)

### Reference Example A-24. 2-Amino-N-(4-benzylamino-benzyl)-6-chloro-nicotinamide

To a solution of (4-aminomethylphenyl)-benzylamine described in Preparation Example 19 (369mg, 1.74mmol) and 2-amino-6-chloro-nicotinic acid (300mg, 1.74mmol) in N,N-dimethylformamide (10mL) were added benzotriazol-1-yloxytris(dimethylamino)phosphonium hexafluorophosphate (924mg, 2.09mmol) and triethylamine (0.49mL, 3.48mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by silica gel column chromatography (hexane : ethyl acetate), and the title compound (310mg, 49%) was obtained as a pale yellow solid.
¹H-NMR Spectrum (CDCl₃) δ (ppm) : 4.32-4.34(3H, m), 4.45(2H, d, J=5.6Hz), 6.07(1H, brs), 6.54-6.63(5H, m), 7.03(1H, dd, J=2.4, J=8.4Hz), 7.14(2H, d, J=8.4Hz), 7.35-7.36(4H, m), 7.48(1H, d, J=8.0Hz).

### Reference Example A-25. 2-Amino-6-chloro-N-(4-phenylamino-benzyl)-nicotinamide

To a solution of (4-aminomethyl-phenyl)-phenylamine described in Preparation Example 20 (345mg, 1.74mmol) and 2-amino-6-chloro-nicotinic acid (300mg, 1.74mmol) in N,N-dimethylformamide (10mL) were benzotriazol-1-yloxytris(dimethylamino)phosphonium hexafluorophosphate (924mg, 2.09mmol) and triethylamine (0.49mL, 3.48mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by silica gel column chromatography (hexane : ethyl acetate), and the title compound (360mg, 59%) was obtained as a pale yellow solid.
¹H-NMR Spectrum (CDCl₃) δ (ppm) : 4.51(2H, d, J=5.2Hz), 5.74(1H, s), 6.16(1H, brs), 6.57(1H, d, J=8.0Hz), 6.58(2H, s), 6.94-6.97(1H, m), 7.04-7.09(4H, m), 7.21-7.30(4H, m), 7.52(1H, d, J=8.0Hz).

### Reference Example A-26 2-Amino-6-chloro-N-(4-phenylaminomethyl-benzyl)-nicotinamide

To a solution of (4-aminomethyl-benzyl)-phenylamine described in Preparation Example 21 (369mg, 1.74mmol) and 2-amino-6-chloro-nicotinic acid (300mg, 1.74mmol) in N,N-dimethylformamide (10mL) were added benzotriazol-1-yloxytris(dimethylamino)phosphonium hexafluorophosphate (924mg, 2.09mmol) and triethylamine (0.49mL, 3.48mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by silica gel column chromatography (hexane : ethyl acetate), and the title compound (479mg, 75%) was obtained as a pale yellow solid.
¹H-NMR Spectrum (CDCl₃) δ (ppm) : 4.08(1 H, brs), 4.31 (2H, s), 4.57(2H, d, J=5.6Hz), 6.21 (1H, brs), 6.57(1H, d, J=8.0Hz), 6.58(2H, s), 6.61-6.63(2H, m), 6.70-6.74(1H, m), 7.15-7.19(2H, m), 7.30(2H, d, J=8.0Hz), 7.37(2H, d, J=8.0Hz), 7.51 (1 H, d, J=8.0Hz).

### Reference Example A-27.2-Amino-6-chloro-N-(4-benzyloxy-benzyl)-nicotinamide

2-Amino-6-chloro-nicotinic acid described in Preparation Example A-1 (220mg, 1.4mmol), triethylamine (0.47mL, 3.37mmol) and benzotriazol-1-yloxytris(dimethylamino)phosphonium hexafluorophosphate (764mg, 1.73mmo1) were dissolved in N,N-dimethylformamide (3mL), 4-benzyloxy-benzylamine described in Preparation Example 1 (399mg, 1.87mmol) was added thereto, followed by stirring at room temperature for 17 hours 30 minutes. After the reaction was completed, the reaction solution was poured into brine, which was then extracted with ethyl acetate. The organic layer was dried over anhydrous magnesium sulfate, then concentrated, the obtained residue was purified by silica gel column chromatography (ethyl acetate : hexane = 1 : 1), and the title compound (115mg, 0.31 mmol, 22%) was obtained.
¹H-NMR Spectrum (DMSO-d₆) δ (ppm): 4.35 (2H, d, J=6.0Hz), 5.08 (2H, s), 6.63 (1 H, d, J=8.0Hz), 6.94-6.98 (2H, m), 7.23 (2H, d, J=8.4Hz), 7.29-7.45 (5H, m), 7.52 (2H, brs), 7.97 (1 H, d, J=8.0Hz), 8.96 (1 H, t, J=6.0Hz).

### Reference Example A-28. 2-Amino-N-(4-benzyloxy-benzyl)-6-cyclopropylaminonicotinamide

2-Amino-6-chloro-N-(4-benzyloxy-benzyl)-nicotinamide described in Reference Example A-27 (80mg, 0.22mmol) was dissolved in tetrahydrofuran (3mL), cyclopropylamine (0.3mL, 4.3mmol) was added thereto, and the solution was heated in a sealed tube for 16 hours (oil bath temperature: 140°C). The reaction solution was allowed to room temperature, the solution was concentrated, the obtained residue was purified by NH silica gel column chromatography (ethyl acetate : hexane = 2 : 1), and the title compound (12mg, 0.031mmol, 14%) was obtained.
¹H-NMR Spectrum (CDCl₃) δ (ppm) : 0.52-0.58 (2H, m), 0.74-0.81 (2H, m), 2.47-2.56 (1 H, m), 4.45 (2H, d, J=5.2 Hz), 5.07 (2H, s), 5,02 (1 H, brs), 5.96-6.01 (1 H, m), 6.04 (1 H, d, J=8.4 Hz), 6.39 (2H, brs), 6.95 (2H, d, J=8.8 Hz), 7.23-7.45 (8H, m).

### Reference Example A-29. 2-Amino-N-(4-benzyloxy-benzyl)-6-ethoxy-nicotinamide

Sodium hydride (70mg, 1.7mmol,60% in oil), catalytic amount of copper(I) iodide, 2-amino-6-chloronicotinic acid described in Preparation Example A-4 (30mg, 0.17mmol) were added sequentially to ethanol (0.5mL), the solution was stirred at 110°C for 3 hours, then, stirred overnight at 80°C. After cooling to room temperature, water, diethyl ether and an aqueous solution of 29% ammonia were added to the reaction solution, which was then partitioned, and the aqueous layer was neutralized with citric acid. Dichloromethane was added to the aqueous layer, the organic layer was partitioned, washed with brine, then, the solvent was evaporated *in vacuo.* Trifluoroacetic acid salt of the title compound (3.4mg, 0.0069mmol, 14%) was obtained from a portion (10mg) of the residue (35mg) and 4-benzyloxybenzylamine (10mg, 0.047mmol) according to an analogous method to Reference Example Q-6.
MS m/e (ESI) 378.5 (MH⁺)

### Reference Example A-30. (6-amino-5-(4-benzyloxy-benzylcarbamoyl)-pyridin-2-vlamino)-acetic acid

Glycine (935mg, 12.5mmol) and 1,8-diazabicyclo[5,4,0]undec-7-ene (1.86mL, 12.5mmol) were added to 2-amino-N-(4-benzyloxy-benzyl)-6-chloro-nicotinamide described in Preparation Example A+-18 (454mg, 1.25mmol) under nitrogen atmosphere, and the solution was stirred for 6 hours at 130°C. Dimethylsulfoxide (35mL) was added to the reaction mixture, which was then filtered with polytetrafluoroethylene membrane filter (Whatman Inc), the filtrate was purified by reverse phase high performance liquid chromatography (acetonitrile-water mobile phase (containing 0.1% trifluoroacetic acid) was used), and the title compound (287mg, 0.551mmol, 44%) was obtained.
MS m/e (ESI) 406.91 (MH⁺)
¹H-NMR Spectrum (DMSO-d₆) δ (ppm) : 3.82-4.00(2H, m), 4.29(2H, d, J=6.0Hz), 5.06(2H, s), 5.77-5.88(1H, m), 6.93(2H, d, J=8.8Hz), 7.18(2H, d, J=8.8Hz), 7.28-7.42(5H, m), 7.68-7.80(1 H, m).

### Reference Example A-31. 2-Amino-6-methoxymethyl-N-(4-ywridin-2-ylmethoxy)-benzyl)-nicotinamide

To a solution of 2-amino-6-methoxymethyl-nicotinic acid described in Preparation Example A-11 (100mg, 0.55mmol) and 4-(pyridin-2-ylmethoxy)-benzylamine described in Preparation Example 171 (170mg, 0.82mmol) in N,N-dimethylformamide (5mL) were added benzotriazol-1-yloxytris(dimethylamino)phosphonium hexafluorophosphate (290mg, 0.66mmol) and triethylamine (0.23mL, 1.7mmol), and the solution was stirred overnight at room temperature. Ethyl acetate and water were added to the reaction solution for extraction, the organic layer was washed with brine, then, the solvent was evaporated *in vacuo.* The residue was purified by silica gel column chromatography (heptane : ethyl acetate = 1 : 2), and the title compound (150mg, 0.40mmol, 73%) was obtained as a colorless solid.
¹H-NMR Spectrum (DMSO-d₆) δ (ppm) : 3.32(3H, s), 4.27(2H, s), 4.33(2H, d, J=5.9Hz), 5.14(2H, s), 6.58(1 H, d, J=7.9Hz), 6.96(2H, d, J=8.6Hz), 7.10(2H, br s), 7.22(2H, d, J=8.6Hz), 7.31 (1 H, ddd, J=7.5, 4.8, 1.1 Hz), 7.47 (1 H, d, J=7.9Hz), 7.80 (1H, td, J=7.6, 1.8Hz), 7.94 (1H, d, J=8.1 Hz), 8.54-8.56 (1H, m), 8.87 (1H, t, J=5.9Hz).

### Reference Example A-32. 2-Amino-N-(4-(4-fluoro-benzyloxy)-benzyl)-6-methoxymethyl-nicotinamide

To a solution of 2-amino-6-methoxymethyl-nicotinic acid described in Preparation Example A-11 (10mg, 0.055mmol) and 4-(4-fluoro-benzyloxy)-benzylamine described in Preparation Example 155 (19mg, 0.082mmol) in N,N-dimethylformamide (1mL) were added benzotriazol-1-yloxytris(dimethylamino)phosphonium hexafluorophosphate (29mg, 0.066mmol) and triethylamine (0.022mL, 0.16mmol), and the solution was stirred overnight at room temperature. Water was added to the reaction solution, the precipitated solid was filtered, and the title compound (13mg, 0.033mmol, 60%) was obtained as a colorless solid.
¹H-NMR Spectrum (DMSO-d₆) δ (ppm) : 3.32(3H, s), 4.27(2H, s), 4.33(2H, d, J=5.9Hz), 5.04(2H, s), 6.58(1 H, d, J=7.9Hz), 6.94(2H, d, J=8.8Hz), 7.09(2H, br s), 7.16-7.22(4H, m), 7.46 (2H, dd, J=8.7, 5.6Hz), 7.93 (1 H, d, J=8.1 Hz), 8.85-8.88 (1 H, m).

### Reference Example A-33. 2-Amino-N-(4-benzyloxy-benzyl)-6-methoxymethylnicotinamide

MS m/e (ESI) 378 (MH⁺)

### Reference Example A-34. 2-Amino-6-methoxymethyl-N-(4-(pyridin-2-yloxymethyl)-benzyl)-nicotinamide

¹H-NMR Spectrum (CDCl₃) δ (ppm) : 3.46(3H, s), 4.40(2H, s), 4.60(2H, d, J=5.6Hz), 5.38(2H, s), 6.25(1H, brs), 6.40(2H, brs), 6.68-6.70(1H, m), 6.77-6.81(1H, m), 6.87-6.91(1H, m), 7.35-7.37(2H, m), 7.46-7.48(2H, m), 7.57-7.61 (2H, m), 8.16-8.18(1 H, m).

### Reference Example A-35. 2-Amino-N-(4-(3-fluoro-benzyloxy)-benzyl)-6-methoxymethyl-nicotinamide

MS m/e (ESI) 396 (MH⁺)

### Reference Example A-36. 2-Amino-N-(4-benzyloxy-benzyl)-6-(3-methoxypropoxy)-nicotinamide

Trifluoroacetic acid salt of the title compound (0.65mg, 0.0012mmol, 2.4%) was obtained from 2-amino-6-chloronicotinic acid described in Preparation Example A-4 (8.6mg, 0.050mmol) and 3-methoxypropanol (0.5mL) according to an analogous method to Example A-29.
MS m/e (ESI) 422.5 (MH⁺)

### Reference Example A-37. 2-Amino-N-(4-benzyloxy-benzyl)-6-methyl-nicotinamide

Trifluoroacetic acid salt of the title compound (0.38mg, 0.00082mmol, 4.6%) was obtained from 2-amino-N-(4-benzyloxy-benzyl)-6-chloro-nicotinamide described in Preparation Example A+-18 (6.5mg, 0.018mmol) and methylmagnesium bromide (0.93M tetrahydrofuran solution, 0.12mL, 0.11mmol) according to an analogous method to Reference Example E-40.
MS m/e (ESI) 348.5 (MH⁺)

### Reference Example A-38. 2-Amino-N-(4-benzyloxy-benzyl)-6-propoxy-nicotinamide

Trifluoroacetic acid salt of the title compound (1.5mg, 0.0030mmol, 5.9%) was obtained from 2-amino-6-chloronicotinic acid described in Preparation Example A-4 (8.6mg, 0.050mmol) and propanol (0.5mL) according to an analogous method to Reference Example A-29.
MS m/e (ESI) 406.6 (MH⁺)

### Reference Example A-39. 6-Amino-N-(4-benzyloxybenzyl)-nicotinamide

Trifluoroacetic acid salt of the title compound (7.1 mg, 0.016mmol, 32%) was obtained from 4-benzyloxybenzylamine described in Preparation Example 1 (11 mg, 0.050mmol) and 6-aminonicotinic acid (6.9mg, 0.050mmol) according to an analogous method to Reference Example Q-6.
MS m/e (ESI) 334.3 (MH⁺)

### Reference Example A-40. 6-Amino-N-(3-phenoxybenzyl)-nicotinamide

Trifluoroacetic acid salt of the title compound (16mg, 0.037mmol, 74%) was obtained from 3-phenoxybenzylamine described in Preparation Example 4 (10mg, 0.050mmol) and 6-aminonicotinic acid (6.9mg, 0.050mmol) according to an analogous method to Reference Example Q-6.
MS m/e (ESI) 320.2 (MH⁺)

### Reference Example A-41. 6-Chloro-N-(3-phenoxy-benzyl)-nicotinamide

The title compound (240mg, 0.71 mmol, 61 %) was obtained as a white solid from 3-phenoxy-benzylamine described in Preparation Example 4 (230mg, 1.1 mmol) and 6-chloronicotinic acid (180mg, 1.1mmol) according to an analogous method to Referece Example Q-6.
¹H-NMR Spectrum (CDCl₃) δ (ppm) : 4.63 (2H, d, J=5.7Hz), 6.42 (1H, brs), 6.94 (1H, dd, J=1.8, 8.1Hz), 6.98-7.03 (3H, m), 7.08(1H, d, J=7.5Hz), 7.11-7.15 (1H, m), 7.30-7.37 (3H, m), 7.41-7.43 (1H, m), 8.09 (1H, dd, J=2.6, 8.2Hz), 8.74 (1H, d, J=2.2Hz).

### Reference Example A-42. N-(4-Benzyloxy-benzyl)-6-methylamino-nicotinamide

The title compound (71 mg, 0.19mmol, 88%) was obtained as a white solid from N-(4-benzyloxy-benzyl)-6-(ethoxymethyl-amino)-nicotinamide described in Preparation Example A+-8 (90mg, 0.22mmol) according to an analogous method to
Reference Example A-163.
¹H-NMR Spectrum (CDCl₃) δ (ppm) : 2.96 (3H, s), 4.56 (2H, d, J=5.5Hz), 5.07 (2H, s), 6.12 (1H, brs), 6.38 (1H, d, J=8.8Hz), 6.96 (2H, d, J=8.8Hz), 7.22-7.34 (4H, m), 7.36-7.44 (3H, m), 7.89 (1 H, dd, J=2.4, 8.6Hz), 8.50 (1 H, d, J=2.4Hz).

### Reference Example A-43. N-(4-benzyloxybenzyl)-nicotinamide

The title compound (8.5mg, 0.027mmol, 33%) was obtained as a white solid from 4-benzyloxybenzylamine described in Preparation Example 1 (26mg, 0.12mmol) and nicotinic acid (10mg, 0.081mmol) according to an analogous method to Reference Example Q-6.
¹H-NMR Spectrum (CDCl₃) δ (ppm) : 4.59 (2H, d, J=5.5Hz), 5.07 (2H, s), 6.41 (1H, brs), 6.97 (2H, d, J=8.6Hz), 7.29 (2H, d, J=8.6Hz), 7.32-7.44 (6H, m), 8.11-8.14 (1H, m), 8.71 (1 H, dd, J=1.5, 4.8Hz), 8.96 (1 H, d, J=1.8Hz).

### Reference Example A-44. 2-Amino-N-(4-benzyloxy-3-hydroxy-benzyl)-nicotinamide

To a solution of 4-benzyloxy-3-methoxymethoxy-benzonitrile obtained in Preparation Example 132 (100mg, 0.371 mmol) in tetrahydrofuran (5mL) was added lithium aluminum hydride (75mg, 1.98mmol) portionwise under nitrogen atmosphere on an ice bath, the solution was stirred for 24 hours. In addition, lithium aluminum hydride (75mg, 1.98mmol) was added portionwise on an ice bath, then, the solution was stirred at 50-60°C for 3 hours. Ethyl acetate (10mL) and methanol (5mL) were added little by little to the reaction solution on an ice bath, then, NH silica gel (50mL) was added, and a pale yellow oily residue (73mg) was obtained by NH silica gel column chromatography (hexane : ethyl acetate = 7 : 3). This residue was purified again by NH silica gel column chromatography (hexane : ethyl acetate = 7 : 3), and 4-benzyloxy-3-methoxymethoxy-benzylamine (30mg, 0.11 mmo1,30%) was obtained as a pale yellow oil.
Next, a solution of 2-amino-nicotinic acid (16mg, 0.116mmol), 4-benzyloxy-3-methoxymethoxy-benzylamine (15mg, 0.0549mmol), benzotriazol-1-yl-tris(dimethylamino)phosphonium hexafluorophosphate (55mg, 0.124mmol) and triethylamine (0.08mL, 0.574mmol) in dimethylsulfoxide (4mL) was stirred under nitrogen atmosphere at room temperature for 24 hours. Water (100mL) and brine (50mL) were added to the reaction solution, which was then extracted with ethyl acetate (100mL) twice, and washed with water twice. The organic layer was dried over anhydrous magnesium sulfate, which was then filtered, the filtrate was evaporated *in vacuo,* and 2-amino-N-(4-benzyloxy-3-methoxymethoxy-benzyl)-nicotinamide was obtained as a pale yellow oil. This was purified by NH silica gel column chromatography (hexane : ethyl acetate = 1 : 1, then hexane : ethyl acetate = 3 : 7), and 2-amino-N-(4-benzyloxy-3-methoxymethoxy-benzyl)-nicotinamide) (9.4mg, 0.0239mmol, 43.5%) was obtained as a pale yellow oil. A solution of the resulting 2-amino-N-(4-benzyloxy-3-methoxymethoxy-benzyl)-nicotinamide (7.8mg, 0.0198mmol) and 2M hydrochloric acid (2mL) in methanol (3mL) was stirred for 21 hour at room temperature. Sodium bicarbonate (600mg, 7.14mmol) was added to the reaction mixture to basify it, which was then filtered, evaporation *in vacuo* was carried out, then, the obtained residue was purified by thin layer NH silica gel chromatography (methanol : ethyl acetate = 5 : 95), and the title compound (2.0mg, 0.0057mmol, 29%) was obtained as a white solid.
MS m/e (ESI) 350(MH⁺)

### Reference Example A-45. 2-Amino-N-(6-benzyloxypyridin-3-ylmethyl)-6-methoxymethyl-nicotinamide

¹H-NMR Spectrum (CDCl₃) δ (ppm) : 3.45(3H, s), 4.39(2H, s), 4.53(2H, d, J=5.6Hz), 5.38(2H, s), 6.23(1H, brs), 6.39(2H, brs), 6.69-6.71 (1H, m), 6.80-6.82(1 H, m), 7.30-7.33(1H, m), 7.36-7.40(2H, m), 7.57-7.59(2H, m), 7.60-7.63(2H, m), 8.15-8.15(1H, m).

### Reference Example A-46, 2,6-Diamino-N-(1-(3-fluoro-benzyl)-1H-pyrrol-3-ylmethyl)-nicotinamide

The title compound (6.2mg, 0.018mmol, 5.5%) was obtained from 2,6-diamino-nicotinic acid ethyl ester described in Preparation Example A-14 (60mg, 0.33mmol) and C-(1-(3-fluoro-benzyl)-1*H*-pyrrol-3-yl)-methylamine described in Preparation Example 59 (159mg, 0.78mmol) according to an analogous method to Reference Example A-54.
¹H-NMR Spectrum (CDCl₃) δ (ppm) : 4.40 (2H, d, J=4.8 Hz), 4.53 (2H, brs), 5.01 (2H, s), 5.76 (1H, d, J=8.4 Hz), 5.84-5.89 (1H, m), 6.17 (1H, t, J=2.0 Hz), 6.48 (2H, brs), 6.62-6.67 (2H, m), 6.78-6.82 (1 H, m), 6.91-6.93 (1 H, m), 6.98 (1 H, dt, J=2.4, 8.4Hz), 7.27-7.33 (1 H, m), 7.36 (1 H, d, J=8.4 Hz).

### Reference Example A-47. 2-Amino-N-(1-(3-fluoro-benzyl)-1H-pyrrol-3-ylmethyl)nicotinamide .

The title compound (106mg, 0.327mmol, 66.7%) was obtained as a white solid from C-(1-(3-fluoro-benzyl)-1*H*-pyrrol-3-yl)methylamine described in Preparation Example 59 (100mg, 0.49mmol) and 2-aminonicotinic acid (68mg, 0.49mmol) according to an analogous method to Reference Example Q-6.
¹H-NMR Spectrum (DMSO-d₆) δ (ppm) : 4.22 (2H, d, J=5.6Hz), 5.03 (2H, s), 5.97-6.01 (1H, m), 6.50-6.55 (1 H, m), 6.73 (2H, s), 6.95-7.10 (5H, m), 7.32-7.38 (1 H, m), 7.83-7.88 (1 H, m), 8.00-8.05 (1 H, m), 8.63 (1 H, t, J=5.6Hz).

### Reference Example A-48. 2-Amino-N-(1-(3-fluoro-benzyl)-1H-prryol-3-ylmethyl)-6-methylamino-nicotinamide

2-Amino-6-chloro-N-(1-(3-fluoro-benzyl)-1*H*-pyrrol-3-ylmethyl)-nicotinamide described in Preparation Example A+-5 (50mg, 0.14mmol) was dissolved in a mixture solution of dimethylsulfoxide (1mL) and N,N-diisopropylethylamine (0.5mL), methylamine (2.0M tetrahydrofuran solution) (1 mL, 2mmol) was added thereto, and the solution was heated in a sealed tube for 15 hours (oil bath temperature: 135°C). The reaction mixture was allowed to room temperature, poured into brine, and the solution was extracted with ethyl acetate. The organic layer was dried over anhydrous magnesium sulfate then concentrated, the obtained residue was purified by NH silica gel column chromatography (ethyl acetate), and the title compound (7.3mg, 0.021 mmol, 15%) was obtained.
¹H-NMR Spectrum (CDCl₃) δ (ppm) : 2.88 (3H, d, J=5.2 Hz), 4.40 (2H, d, J=5.2 Hz), 4.58-4.66 (1H, m), 5.01 (2H, s), 5.66 (1H, J=8.8 Hz), 5.81-5.87 (1H, m), 6.17 (1H, t, J=2.4 Hz), 6.45 (2H, brs), 6.63 (1H, t, J=2.4 Hz), 6.66 (1H, brs), 6.78-6.83 (1H, m), 6.92 (1 H, br d, J=7.2 Hz), 6.97 (1 H, dt, J=2.4, 8.4 Hz), 7.27-7.33 (1 H, m), 7.36 (1 H, d, J=8.8 Hz).

### Reference Example A-49. N-(1-(3-Fluoro-benzyl)-1H-pyrrol-3-ylmethyl)-6-methyl-nicotinamide

The title compound (61 mg, 0.18mmol, 65.1 %) was obtained as a colorless oil from C-(1-(3-fluoro-benzyl)-1*H*-pyrrol-3-yl)methylamine described in Preparation Example 59 (60mg, 0.29mmol) and 6-methylnicotinic acid (40mg, 0.29mmol) according to an analogous method to Reference Example Q-6.
¹H-NMR Spectrum (DMSO-d₆) δ (ppm) : 2.48(3H, s), 4.27 (2H, d, 5.6Hz), 5.03 (2H, s), 5.99-6.02 (1 H, m), 6.74-6.78 (2H, m), 6.96-7.10 (3H, m), 7.28-7.38 (2H, m), 8.06 (1 H, dd, J=2.4, 8.0Hz), 8.80 (1 H, t, J=5.6Hz), 8.87 (1 H, d, J=2.4Hz).

### Reference Example A-50. 2-Amino-N-(2-phenoxy-thiazol-5-ylmethyl)-nicotinamide

The title compound (13.5mg, 41µmol, 87%) was obtained as a white solid from C-(2-phenoxy-thiazol-5-yl)-methylamine described in Preparation Example 117 (9.8mg, 48µmol) and 2-amino-nicotinic acid (7.9mg, 58µmol) according to an analogous method to Reference Example A-26.
¹H-NMR Spectrum (DMSO-d₆) δ (ppm) : 4.44 (2H, d, J=5.7Hz), 6.55 (1H, dd, J=4.8, 7.7Hz), 7.05 (2H, s), 7.18 (1 H, s), 7.27-7.31 (3H, m), 7.45 (2H, t, J=8.2Hz), 7.84 (1 H, d, J=7.5Hz), 8.06 (1 H, d, J=4.6Hz), 9.05 (1 H, t, J=6.0Hz).

### Reference Example A-51. 2-((furan-2-ylmethyl)-amino)-N-(5-phenoxy-thiophen-2-ylmethyl)-nicotinamide

The title compound (2.29mg, 0.0044mmol, 4.4%) was obtained as a trifluoroacetic acid salt from 2-chloro-N-(5-phenoxy-thiophen-2-ylmethyl)-nicotinamide described in Preparation Example A+-7 (35mg, 0.10mmol) and furfurylamine (16µl, 0.18mmol) according to an analogous method to Reference Example A-133.
MS m/e (ESI) 406.15(MH⁺)

### Reference Example A-52. 4-((3-((5-Phenoxy-thiophen-2-ylmethyl)-carbamoyl)-pyridin-2-yl) amino)-methyl) benzoic acid

The title compound (2.75mg, 0.0048mmol, 4.8%) was obtained as a trifluoroacetic acid salt from 2-chloro-N-(5-phenoxy-thiophen-2-ylmethyl)-nicotinamide described in Preparation Example A+-7 (36mg, 0.10mmol) and 4-(aminomethyl)benzoic acid (16mg, 0.11mnol) according to an analogous method to Reference Example A-133.
MS m/e (ESI) 460.17(MH⁺)
¹H-NMR Spectrum (DMSO-d₆) δ (ppm) : 4.50 (2H, d, J=6.0Hz), 4.71 (2H, d, J=4.8Hz), 6.51(1H, d, J=3.6Hz), 6.63 (1 H, dd, J=4.8, 8.0Hz), 6.79 (1 H, d, J=3.6Hz), 7.09 (2H, d,J=8.0Hz), 7.09-7.19 (1 H, m), 7.32-7.47 (4H, m), 7.87 (2H, d, J=7.6Hz), 7.98 (1H, dd, J=0.8, 8.0Hz), 8.13 (1H, dd, J=0.8, 4.8Hz), 8.87 (1H, brs), 9.16-9.24 (1 H, m).

### Reference Example A-53. 2.6-Diamino-N-(5-(4-fluoro-phenoxy)-thiophen-2-ylmethyl)-nicotinamide

The title compound (180mg, 0.50mmol, 38.6%) was obtained as a white solid from C-(5-(4-fluoro-phenoxy)-thiophen-2-yl)-methylamine described in Preparation Example 28 (290mg, 1.3mmol) and 2,6-diaminonicotinic acid described in Preparation Example A-15 (200mg, 1.3mmol) according to an analogous method to Reference Example Q-6.
¹H-NMR Spectrum (DMSO-d₆) δ (ppm) : 4.40 (2H, d, J=5.6Hz), 5.64-5.68 (1 H, m), 6.10 (2H, s), 6.45-6.49 (1 H, m), 6.70-6.74 (1 H, m), 6.95 (2H, s), 7.10-7.18 (2H, m), 7:18-7.26 (2H, m), 7.60 (1 H, d, J=8.0Hz), 8.40 (1 H, t, J=5.6Hz).

### Reference Example A-54. 2,6-diamino-N-(5-phenoxy-thiophene-2-ylmethyl)-nicotinamide

To a solution of 2,6-diamino-nicotinic acid ethyl ester described in Preparation Example A-14 (18mg, 0.1 mmol) in ethanol (10mL) was added 1 N sodium hydroxide aqueous solution (5mL), and the solution was stirred for 1 hour 10 minutes under reflux. After cooling the reaction solution, the solution was neutralized with 1 N hydrochloric acid and concentrated. The resulting crude product was suspended in N,N-dimethylformamide (3mL), triethylamine (0.02mL, 0.15mmol), benzotriazol-1-yloxytris(dimethylamino)phosphonium hexafluorophosphate (65mg, 0.15mmol) and C-(5-phenoxy-thiophen-2-yl)-methylamine described in Preparation Example 26 (30mg, 0.15mmol) were added thereto, followed by stirring at room temperature for 19 hours 40 minutes. After the reaction was completed, reaction solution was poured into brine, and the solution was extracted with ethyl acetate. The organic layer was dried over anhydrous magnesium sulfate then concentrated, the obtained residue was purified by NH silica gel column chromatography (ethyl acetate), and the title compound (8.7mg, 0.025mol, 25%) was obtained.
¹H-NMR Spectrum (CDCl₃) δ (ppm) : 4.58-4.61 (2H, m), 4.62 (2H, brs), 5.75 (1H, d, J=8.4Hz), 6.21-6.27 (1 H, m), 6.36-6.38 (1 H, m), 6.45 (2H, brs), 6.69-6.72 (1 H, m), 7.06-7.12 (3H, m), 7.28-7.34 (2H, m), 7.39 (1 H, d, J=8.4Hz).

### Reference Example A-55. 2,6-Diamino-N-(5-benzyl-thiophen-2-ylmethyl)-nicotinamide

To a solution of 2,6-diaminonicotinic acid described in Preparation Example A-15 (173mg, 1.13mmol) in dimethylsulfoxide (15mL) were added 1-ethyl-3-(3-dimethylaminopropyl)carbodiimide hydrochloride (433mg, 2.26mmol), 1-hydroxybenzotriazole (346mg, 2.26mmol) and C-(5-benzyl-thiophen-2-yl)-methylamine described in Preparation Example 42 (230mg, 1.13mmol), and the solution was stirred at room temperature fro 16 hours 30 minutes. The reaction solution was poured into brine, the solution was extracted with ethyl acetate, the organic layer was dried over anhydrous magnesium sulfate and then concentrated. The obtained residue was subjected to NH silica gel column chromatography, eluted with solvent (ethyl acetate) and the title compound (114mg, 0.34mmol, 30%) was obtained.
¹H-NMR Spectrum (CDCl₃) δ (ppm) : 4.00 (2H, s), 4.53 (2H, d, J=5.6 Hz), 4.70 (2H, br s), 5.69 (1 H, d, J=8.4 Hz), 6.39 (2H, br s), 6.55 (1 H, d, J=3.2 Hz), 6.67-6.95 (2H, m), 7.09-7.25 (5H, m), 7.38 (1 H, d, J=8.4 Hz).

### Reference Example A-56. 2.6-Diamino-N-(5-benzyloxy-thiophen-2-ylmethyl)-nicotinamide

To a solution of 2,6-diaminonicotinic acid described in Preparation Example A-15 (109mg, 0.71 mmol) in dimethylsulfoxide (10mL) were added 1-ethyl-3-(3-dimethylaminopropyl)carbodiimide hydrochloride (272mg, 1.42mmol), 1-hydroxybenzotriazole (217mg, 1.42mmol) and C-(5-benzyloxy-thiophen-2-yl)methylamine described in Reference Example E-76 (156mg, 0.71mmol), and the solution was stirred at room temperature for 14 hours. The reaction solution was poured into brine, extracted with ethyl acetate, the fractionated organic layer was dried over anhydrous magnesium sulfate and then concentrated. The obtained residue was subjected to NH silica gel column chromatography, eluted with solvent (ethyl acetate) and the title compound (157mg, 0.44mmol,62%) was obtained.
¹H-NMR Spectrum (CDCl₃) δ (ppm) : 4.53 (2H, d, J=5.2 Hz), 4.69 (2H. br s), 5.03 (2H, s), 5.76 (1H, d, J=8.8 Hz), 6.08 (1H, d, J=4.0 Hz), 6.46 (3H. br), 6.59 (1H, d, J=4.0 Hz), 7.31-7.44 (6H, m).

### Reference Example A-57. 2,6-Diamino-N-(5-(3-fluoro-phenoxy)-thiophen-2-ylmethyl)-nicotinamide

¹H-NMR Spectrum (CDCl3) δ (ppm) : 4.42(2H, d, J=6.0Hz), 5.66 (1H, d, J=8.4Hz), 6.11 (2H, brs), 6.55-6.59 (1 H, m), 6.75 (1H, d, J=4.0Hz), 6.90-7.02 (5H, m), 7.38-7.45 (1H, m), 7.60 (1H, d, J=8.4Hz), 8.42 (1H, t, J=6.0Hz).

### Reference Example A-58. 2,6-Diamino-N-(5-benzofuran-2-ylmethyl-thiophen-2-yimethyl)-nicotinamide

¹H-NMR Spectrum (CDCl₃) δ (ppm) : 4.29 (2H, s), 4.43 (2H, d, J=6.0Hz), 5.64 (1H, d, J=8.4Hz), 6.09 (2H, brs), 6.66 (1H, d, J=0.8Hz), 6.92 (2H, s), 6.94 (2H, brs), 7.17-7.26 (2H, m), 7.47-7.64 (3H, m), 8.37 (1 H, t, J=6.0Hz).

### Reference Example A-59. 2,6-Diamino-N-(5-benzo[b]thiophen-2-ylmethyl-thiophen-2-ylmethyl)-nicotinamide

¹H-NMR Spectrum (CDCl₃) δ (ppm) : 4.39 (2H, s), 4.44 (2H, d, J=5.6Hz), 5.64 (1H, d, J=8.4Hz), 6.09 (2H, brs), 6.78-6.84 (2H, m), 6.95 (2H, brs), 7.23-7.36 (3H. m), 7.59 (1H, d, J=8.4Hz), 7.74 (1H, dd, J=0.8, J=6.0Hz), 7.85 (1H, dd, J=0.8, J=6.0Hz), 8.38(1 H, t, J=5.6Hz).

### Reference Example A-60. N-(5-(3-Fluorophenoxy)thiophen-2-ylmethyl)-2,6-dimethylnicotinamide

The title compound (56mg, 0.157mmol, 47.6%) was obtained as a light brown solid from 2,6-dimethylnicotinic acid (50mg, 0.33mmol) and C-(5-(3-fluorophenoxy)thiophen-2-yl)methylamine described in Preparation Example 23 (66mg,0.297mmol) according to an analogous method to Reference Example Q-6.
¹H-NMR Spectrum (DMSO-d₆) δ (ppm) : 2.44 (3H, s), 2.46 (3H, s), 4.51 (2H, d, J=5.6Hz, 6.59-6.62 (1 H, m), 6.80-6.84 (1 H, m), 6.90-7.03 (3H, m), 7.12 (1 H, d, J=8.0Hz), 7.40-7.47 (1 H, m), 7.60 (1 H, d, J=8.0Hz), 9.00 (1 H, t, J=5.6Hz).

### Reference Example A-61. 2-Acetylamino-N-(5-phenoxy-thiophen-2-ylmethyl)-nicotinamide

To a mixture of 2-amino-N-(5-phenoxy-thiophen-2-ylmethyl)-nicotinamide described in Reference Example A-67 (50mg, 0.15mmol), acetonitrile(5mL) and tetrahydrofuran (2mL) was added nitronium tetrafluoroborate (0.50M sulfolane solution, 0.46mL, 0.23mmol) on an ice bath, and the solution was stirred overnight at room temperature. Water, ethyl acetate, tetrahydrofuran and an aqueous solution of saturated sodium bicarbonate were added to the reaction solution for extraction, washed with brine, then, the solvent was evaporated *in vacuo.* The residue was purified by silica gel column chromatography (methanol : ethyl acetate = 1 : 30), and the title compound (1.3mg, 0.0035mmol, 2.3%) was obtained as a white solid.
¹H-NMR Spectrum (CDCl₃) δ (ppm) : 2.31 (3H, s), 4.67 (2H, d, J=5.5Hz), 6.40 (1 H, d, J=3.9Hz), 6.60 (1H, brs), 6.77 (1H, d, J=3.7Hz), 7.05 (1H, dd, J=4.9, 7.8Hz), 7.08-7.14 (3H, m), 7.32-7.36 (2H, m), 7.84 (1 H, brs), 8.51 (1 H, brs).

### Reference Example A-62. 2-Amino-N-(5-(3-cyano-phenoxy)-thiophen-2-ylmethyl)-nicotinamide

To a solution of C-(5-(3-bromophenoxy)-thiophen-2-yl)-methylamine described in Preparation Example 17 (366mg, 1.29mmol) and 2-aminopyridine-3-carboxylic acid (178mg, 1.29mmol) in tetrahydrofuran (5mL) were added benzotriazol-1-yloxytris(dimethylamino)phosphonium hexafluorophosphate (685mg, 1.55mmol) and triethylamine (0.36mL, 2.58mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and a mixture of 2-amino-N-(5-(3-bromophenoxy)-thiophen-2-ylmethyl)-nicotinamide and debrominated compound (344mg, 66%) was obtained as a yellow solid.
Next, to a solution of a mixture of 2-amino-N-(5-(3-bromophenoxy)-thiophen-2-ylmethyl)-nicotinamide and debrominated compound (100mg, 0.247mmol) in N,N-dimethylformamide (3.0mL) were added zinc cyanide (58mg, 0.495mmol) and tetrakis(triphenylphosphine)palladium(0) (285mg, 0.247mmol) under nitrogen atmosphere, and the solution was stirred at 140°C fro 3 hours and a half. The reaction solution was allowed to room temperature, ethyl acetate and water were added to the reaction solution, which was then partitioned, the organic layer was washed with water and dried over anhydrous magnesium sulfate. The solvent was evaporated, then, the residue was purified by NH silica gel column chromatography (hexane : ethyl acetate), and the title compound (9mg, 10%) was obtained as a pale yellow oil.
¹H-NMR Spectrum (CDCl₃) δ (ppm) : 4.69(2H, d, J=6.0Hz), 6.36(2H, brs), 6.41 (1H, brs), 6.47-6.48(1H, m), 6.59-6.62(1H, m), 6.79-6.80(1H, m), 7.31-7.34(2H, m), 7.37-7.44(2H, m), 7.61-7.63(1 H, m), 8.17-8.19(1 H, m).

### Reference Example A-64. 2-Amino-N-(5-m-tolyloxy-thiophen-2-ylmethyl)-nicotinamide

The title compound (126mg, 0.37mmol, 80.8%) was obtained as a brown oil from 2-aminonicotinic acid (69mg, 0.51 mmol) and C-(5-*m*-tolyloxy-thiophen-2-yl)-methylamine described in Preparation Example 30 (100mg, 0.46mmol) according to an analogous method to Reference Example Q-6.
¹H-NMR Spectrum (DMSO-d₆) δ (ppm) : 2.26 (3H, s), 4.46 (2H, d, J=5.6Hz), 6.46 (1 H, d, J=3.6Hz), 6.55 (1 H, dd, J=4.8, 8.0Hz), 6.74 (1 H, d, J=3.6Hz), 6.84-6.96 (3H, m), 7.04 (2H, s), 7.22 (1 H, dd, J=8.0, 8.0Hz), 7.86 (1 H, dd, J=1.6, 8.0Hz), 8.05 (1 H, dd, J=1.6, 4.8Hz), 9.02 (1 H, t, J=5.6Hz).

### Reference Example A-65. 2-Amino-N-(5-p-tolyloxy-thiophen-2-ylmethyl)nicotinamide

The title compound (72mg, 0.212mmol, 57.4%) was obtained as a light brown solid from 2-aminonicotinic acid (55mg, 0.41 mmol) and C-(5-*p*-tolyloxythiophen-2-yl)methylamine described in Preparation Example 32 (80mg, 0.37mmol) according to an analogous method to Reference Example Q-6.
¹H-NMR Spectrum (DMSO-d₆) δ (ppm) : 2.24 (3H, s), 4.44 (2H, d, J=5.6Hz), 6.40-6.44 (1 H, m), 6.53-6.58 (1 H, m), 6.72 (1 H, d, J=3.6Hz), 6.95-7.00 (2H, m), 7.04 (2H, s), 7.12-7.18 (2H, m), 7.85 (1H, d, J=7.6Hz), 8.03-8.08 (1H, m), 9.01 (1H, t, J=5.6Hz).

### Reference Example A-66. 2-amino-4-(5-(3-chloro-benzyl)thiophene-2-ylmethyl)-nicotinamide

The title compound (63mg, 0.176mmol, 51.9%) was obtained as a white solid from 2-aminonicotinic acid (51 mg, 0.37mmol) and C-(5-(3-chloro-benzyl)thiophen-2-yl)-methylamine described in Preparation Example 45 (80mg,0.34mmol) according to an analogous method to Reference Example Q-6.
¹H-NMR Spectrum (DMSO-d₆) δ (ppm) : 4.06 (2H, s), 4.47 (2H, d, J=5.6Hz), 6.54 (1 H, dd, J=4.8, 7.6Hz), 6.72 (1 H, d, J=3.6Hz), 6.80 (1 H, d, J=3.6Hz), 7.05 (2H, brs), 7.18-7.34 (4H, m), 7.84 (1 H, dd, J=1.6, 7.6Hz), 8.04 (1 H, dd, J=1.6, 4.8Hz), 8.98 (1H, t, J=5.6Hz).

### Reference Example A-67. 2-Amino-N-(5-phenoxy-thiophen-2-ylmethyl)-nicotinamide

The title compound (148mg, 0.455mmol, 73%) was obtained from 2-aminonicotinic acid (87mg, 0.63mmol) and C-(5-phenoxy-thiophen-2-yl)methylamine described in Preparation Example 24 (143mg, 0.697mmol) according to an analogous method to Reference Example H-1.
¹H-NMR Spectrum (DMSO-d₆) δ (ppm) : 4.46 (2H, d, J=5.6Hz), 6.49 (1H, d, J=4.0Hz), 6.55 (1H, dd, J=4.8, 7.6Hz), 6.76 (1H, d, J=4.0Hz), 7.00-7.17 (5H, m), 7.32-7.40 (2H, m), 7.87 (1H, dd, J=1.6, 7.6Hz), 8.06 (1H, dd, J=1.6, 4.8Hz), 9.00-9.09(1 H, m).

### Reference Example A-68. 2-Amino-N-(5-(3-fluoro-phenoxy)-thiophen-2-vlmeth^{y}l)-nicotinamide

The title compound (112mg, 0.326mmol, 75%) was obtained from 2-aminonicotinic acid (60mg, 0.43mmol) and C-(5-(3-fluoro-phenoxy)-thiophen-2-yl)methylamine described in Preparation Example 23 (106mg, 0.475mmol) according to an analogous method to Reference Example H-1.
¹H-NMR Spectrum (DMSO-d₆) δ (ppm) : 4.48 (2H, d, J=5.2Hz), 6.52-6.59 (2H, m), 6.79 (1 H, d, J=2.8Hz), 6.87-7.00 (3H, m), 7.06 (2H, brs), 7.34-7.45 (1 H, m), 7.87 (1 H, dd, J=2.0, 7.6Hz), 8.06 (1 H, dd, J=2.0, 4.8Hz), 9.05 (1 H, t, J=5.2Hz).

### Reference Example A-69. 2-Amino-N-(5-(4-fluoro-phenoxy)-thiophen-2-ylmethyl)-nicotinamide

The title compound (174mg, 0.507mmol, 65%) was obtained from 2-aminonicotinic acid (107mg, 0.777mmol) and C-(5-(4-fluoro-phenoxy)-thiophen-2-yl)methylamine described in Preparation Example 28 (191mg, 0.856mmol) according to an analogous method to Reference Example H-1.
¹H-NMR Spectrum (DMSO-d₆) δ (ppm) : 4.45 (2H, d, J=6.0Hz), 6.46 (1H, d, J=4.0Hz), 6.55 (1H, dd, J=4.8, 7.6Hz), 6.74 (1H, d, J=4.0Hz), 6.85-7.25 (6H, m), 7.86 (1 H, d, J=7.6Hz), 8.05 (1 H, d, J=4.8Hz), 8.96-9.08 (1 H, m).

### Reference Example A-70. 2-Amino-N-(5-benzyl-thiophen-2-ylmethyl)-nicotinamide

The title compound (67mg, 0.21mmol, 92%) was obtained from 2-aminonicotinic acid (31 mg, 0.224mmol) and C-(5-benzyl-thiophen-2-yl)-methylamine described in Preparation Example 42 (50mg, 0.245mmol) according to an analogous method to Reference Example H-1.
¹H-NMR Spectrum (DMSO-d₆) δ (ppm) : 4.04 (2H, s), 4.46 (2H, d, J=5.2Hz), 6.54 (1H, dd, J=4.8, 8.0Hz), 6.69 (1H, d, J=3.6Hz), 6.78 (1 H, d, J=3.6Hz), 7.05 (2H, brs), 7.15-7.30 (5H, m), 7.85 (1 H, dd, J=2.0, 8.0Hz), 8.04 (1 H, d, J=2.0, 4.8Hz), 8.98 (1 H, t, J=5.2Hz).

### Reference Example A-71. 2-Amino-N-(5-(3-fluoro-benzyl)-thiophen-2-ylmethyl)-nicotinamide

The title compound (13mg, 0.038mmol, 19%) was obtained from 2-aminonicotinic acid (28mg, 0.205mmol) and C-(5-(3-fluoro-benzyl)-thiophen-2-yl)-methylamine obtained by the method described in Example A-146 (50mg, 0.226mmol) according to an analogous method to Reference Example H-1.
¹H-NMR Spectrum (DMSO-d₆) δ (ppm) : 4.07 (2H, s), 4.40-4.53 (2H, m), 6.50-6.58 (1H, m), 6.71 (1H, d, J=3.2Hz), 6.79 (1H, d, J=3.2Hz), 6.94-7.09 (5H, m), 7.22-7.37 (1 H, m), 7.84 (1 H, dd, J=1.6, 8.0Hz), 8.03-8.06 (1 H, m), 8.92-9.03 (1 H, m).

### Reference Example A-72. 2-Amino-N-(4-phenoxy-thiophen-2-ylmethyl)-nicotinamide

The title compound (108mg, 0.331mmol, 74%) was obtained as a white solid from C-(4-phenoxy-thiophen-2-yl)-methylamine described in Preparation Example 111 (92mg, 0.45mmol) and 2-amino-nicotinic acid (68mg, 0.49mmol) according to an analogous method to Reference Example A-26.
¹H-NMR Spectrum (CDCl₃) δ (ppm) : 4.70 (2H, d, J=5.7Hz), 6.35 (3H, brs), 6.48 (1H, d, J=1.7Hz), 6.60 (1 H, dd, J=4.9, 7.7Hz), 6.82 (1 H, d, J=1.6Hz), 7.05 (2H, dd, J=1.1, 8.6Hz), 7.11 (1H, tt, J=1.1, 7.7Hz), 7.34 (2H, t, J=8.6Hz), 7.61 (1H, dd, J=1.7, 7.7Hz), 8.18 (1 H, dd, J=1.8, 4.8Hz).

### Reference Example A-73. 2-Amino-N-(5-(3-chloro-phenoxy)-thiophen-2-ylmethyl)-nicotinamide

C-(5-(3-Chloro-phenoxy)-thiophen-2-yl)-methylamine (1.02g, 4.25mmol, 98%) was obtained as an oil from 5-(3-chloro-phenoxy)-thiophene-2-carbonitrile described in Preparation Example 121 (1.02g, 4.32mmol) according to an analogous method to Reference Example E-24. Then, title compound (12.1mg) was obtained from the resulting C-(5-(3-chloro-phenoxy)-thiophen-2-yl)-methylamine (30mg, 0.13mmol) and 2-amino-nicotinic acid (17mg, 0.13mmol). Trifluoroacetic acid salt of the title compound (12.1mg) was obtained by reverse phase high performance liquid chromatography (acetonitrile-water mobile phase (containing 0.1 % trifluoroacetic acid) was used).
MS m/e (ESI): 360.3(MH⁺)

### Reference Example A-74. 2-Amino-N-(5-(2-fluoro-benzyl)-thiophen-2-ylmethyl)nicotinamide

The title compound was obtained from C-(5-(2-fluoro-benzyl)thiophen-2-yl)-methylamine described in Preparation Example 126 (30mg, 0.14mmol) and 2-amino-nicotinic acid (21 mg, 0.15mmol) according to an analogous method to Reference Example A-26. Trifluoroacetic acid salt of the title compound (16.2mg) was obtained by reverse phase high performance liquid chromatography (acetonitrile-water mobile phase (containing 0.1% trifluoroacetic acid) was used).
MS m/e (ESI) 342.34(MH⁺)

### Reference Example A-75. 2-Amino-N-(5-(4-chloro-ohenoxy)-thiophen-2-ylmethyl)-nicotinamide

A solution of 2-amino-nicotinic acid (21 mg, 0.15mmol), C-(5-(4-chloro-phenoxy)-thiophen-2-yl)-methylamine described in Preparation Example 157 (36mg, 0.15mmol), benzotriazol-1-yl-tris(dimethylamino)phosphonium hexafluorophosphate (86mg, 0.195mmol) and triethylamine (0.065mL, 0.45mmol) in dimethylsulfoxide (1 mL) was stirred under nitrogen atmosphere for 17 hours at room temperature. This reaction solution was purified by reverse phase high performance liquid chromatography (acetonitrile-water mobile phase (containing 0.1% trifluoroacetic acid) was used), and trifluoroacetic acid salt of the title compound (17.7mg, 0.037mmol, 24.9%) was obtained as a pale yellow solid.
MS m/e (ESI) 360(MH⁺)

### Reference Example A-76. 2-Amino(5-(2-chloro-phenoxy)-thiophen-2-ylmethyl)-nicotinamide

Trifluoroacetic acid salt of the title compound (31.1mg, 0.07mmol, 43.7%) was obtained as a light brown oil from 2-amino-nicotinic acid (21 mg, 0.15mmol) and C-(5-(2-chloro-phenoxy)-thiophen-2-yl)-methylamine described in Preparation Example 159 (36mg, 0.15mmol) according to an analogous method to Reference Example A-75.
MS m/e (ESI) 360(MH⁺)

### Reference Example A-77. 2-Amino-N-(5-(2,2-dicyclopropylyinyl)thiophen-2-ylmethyl) nicotinamide

The title compound (25mg, 0.0742mol, 53.8%) was obtained as a white solid from 2-aminonicotinic acid (19mg, 0.138mmol) and C-(5-(2,2-dicyclopropylyinyl)thiophen-2-yl)methylamine described in Preparation Example 152 (30mg, 0.138mmol) according to an analogous method to Reference Example A-149.
¹H-NMR Spectrum (CDCl₃) δ (ppm) : 0.42-0.46(2H, m), 0.61-0.66(2H, m), 0.83-0.88(4H, m), 1.16-1.23(1H, m), 1.96-2.03(1H, m), 4.73(2H, d, J=5.6Hz), 6.27(1H, s), 6.34(3H, s), 6.58(1 H, dd, J=4.8, 7.6Hz), 6.84(1 H, d, J=3.2Hz), 6 92(1 H, d, J=3.2Hz), 7.57(1 H, dd, J=1.6, 7.6Hz), 8.16(1 H, dd, J=1.6, 4.8Hz).

### Reference Example A-78. 2-Amino-5-chloro-N-(5-phenoxy-thiophen-2-ylmethyl)-nicotinamide

Trifluoroacetic acid salt of the title compound (1.7mg, 0.0036mmol, 5.6%) was obtained as a by-product from Reference Example A-171.
MS m/e (ESI) 360.1 (MH⁺)

### Reference Example A-79. 2-Amino-5-methyl-N-(5-phenoxy-thiophen-2-ylmethyl)-nicotinamide

The title compound was obtained from 2-amino-5-iodine-N-(5-phenoxy-thiophen-2-ylmethyl)-nicotinamide described in Preparation Example A+-16 (10mg,22µmol) according to an analogous method to Reference Example A-170. Trifluoroacetic acid salt of the title compound was obtained by reverse phase high performance liquid chromatography (acetonitrile-water mobile phase (containing 0.1% trifluoroacetic acid) was used).
MS m/e (ESI) 340.12(MH⁺)

### Reference Example A-80. 2-Amino-6-(1-pentynyl)-N-(5-ghenoxy-thiophen-2-ylmethyl)-nicotinamide

Trifluoroacetic acid salt of the title compound (0.70mg, 0.00014mmol, 3.3%) was obtained from 2-amino-6-chloro-N-(5-phenoxy-thiophen-2-ylmethyl)-nicotinamide described in Example A-101 (15mg, 0.042mmol) and 1-pentyne (3.4mg, 0.050mmol) according to an analogous method to Reference Example A-91.
MS m/e (ESI) 392.2 (MH⁺)

### Reference Example A-81. 2-Amino-N-(5-phenoxy-thiophen-2-ylmethyl)-6-(3-[1,2,3]-triazol-2-yl-propylamino)-nicotinamide

The title compound (14.96mg, 0.027mmol, 9.2%) was obtained from 2-amino-6-chloro-N-(5-phenoxy-thiophen-2-ylmethyl)-nicotinamide described in Example A-101 (105mg, 0.292mmol) and 3-[1,2,3]triazol-2-yl-propylamine (279mg, 2.21 mmol) according to an analogous method to Reference Example A-126.
MS m/e(ESI) 450.38(MH⁺)

### Reference Example A-82. 2-Amino-6-(furfurylamino)-N-(5-phenoxy-thiophen-2-ylmethyl)-nicotinamide

2-Amino-6-chloro-N-(5-phenoxy-thiophen-2-ylmethyl)-nicotinamide described in Reference Example A-101 (180mg, 0.5mmol) was dissolved in a mixture solution of dimethylsulfoxide (2mL) and diisopropylethylamine (1mL), furfurylamine (0.663mL, 7.5mmol) was added, and the solution was heated in a sealed tube for 13 hours 30 minutes (oil bath temperature: 135°C). The reaction mixture was allowed to room temperature, poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1), and the title compound (144mg, 0.34mmol, 68%) was obtained.
¹H-NMR Spectrum (CDCl₃) δ (ppm) : 4.50 (2H, d, J=5.6 Hz), 4.61 (2H, dd, J=0.8, 5.6 Hz), 4.87-4.94 (1H, m), 5.74 (1 H, d, J=8.8 Hz), 6.04 (1 H, t, J=5.2 Hz), 6.22 (1 H, dd, J=0.8, 3.2 Hz), 6.31 (1H, dd, J=2.0, 3.2Hz), 6.38 (1H, d, J=3.6 Hz), 6.45 (2H, brs), 6.69-6.72 (1 H, m), 7.06-7.12 (3H, m), 7.29-7.38 (4H, m).

### Reference Example A-83. 2-Amino-N-(5-phenoxy-thiophen-2-ylmethyl)-6-(2-pyridin-2-yl-ethylamino)-nicotinamide

2-Amino-6-chloro-N-(5-phenoxy-thiophen-2-ylmethyl)-nicotinamide described in Reference Example A-101 (20mg, 37µmol) and 2-pyridin-2-yl-ethyl amine (66µl, 0.56mmol) were dissolved in a mixture solvent of dimethylsulfoxide (1mL) and N,N-diisopropylethylamine (0.5mL), and the solution was stirred at 130°C for 17 hours. The reaction solution was cooled to room temperature, water and ethyl acetate were added, the organic layer was partitioned, washed with water and brine, and dried over anhydrous magnesium sulfate. The solvent was evaporated in *vacuo,* the residue was purified by reverse phase high performance liquid chromatography (acetonitrile-water mobile phase (containing 0.1 % trifluoroacetic acid) was used), and the title compound (17.7mg) was obtained as a trifluoroacetic acid salt.
MS m/e (ESI) 446.05(MH⁺)

### Reference Example A-84. 2-Amino-N-(5-phenoxy-thiophen-2-ylmethyl)-6-(tetrahydrofuran-2-ylmethoxy)-nicotinamide

Trifluoroacetic acid salt of the title compound (16mg, 0.030mmol, 31%) was obtained from 2-amino-6-chloronicotinic acid described in Preparation Example A-4 (17mg, 0.096mmol), tetrahydrofuran-2-ylmethanol (0.5mL) and C-(5-phenoxy-thiophen-2-yl)-methylamine (20mg, 0.097mmol) according to an analogous method to Reference Example A-29.
MS m/e (ESI) 426.2 (MH⁺)

### Reference Example A-85. 2-Amino-6-N-(5-phenoxy-thiophen-2-ylmethyl)-6-(2-(R)-(-)-tetrahydrofurfurylamino)-nicotinamide

2-Amino-6-chloro-N-(5-phenoxy-thiophen-2-ylmethyl)-nicotinamide described in Reference Example A-101 (30mg, 0.083mmol) was dissolved in a mixture solution of dimethylsulfoxide (1 mL) and diisopropylethylamine (0.5mL), (R)-(-)-tetrahydrofurfuryl amine (0.086mL, 0.83mmol) was added thereto, followed by heating in a sealed tube for 22 hours 30 minutes (oil bath temperature: 130°C). The reaction mixture was allowed to room temperature, poured into brine, and the solution was extracted with ethyl acetate. The organic layer was dried over anhydrous magnesium sulfate, then concentrated, the obtained residue was purified by silica gel column chromatography (ethyl acetate : hexane = 1 : 1), and the title compound (22mg, 0.052mmol, 62%) was obtained.
¹H-NMR Spectrum (CDCl₃) δ (ppm) :1.56-1.67 (1H, m), 1.86-2.04 (3H, m), 3.23-3.31 (1 H, m), 3.54-3.62 (1 H, m), 3.73-3.80 (1 H, m), 3.84-3.91 (1 H, m), 4.02-4.09 (1 H, m), 4.61 (2H, d, J= 5.6 Hz), 4.91-5.02 (1 H, m), 5.71 (1 H, d, J=8.8 Hz), 5.98-6.04 (1 H, m), 6.38 (1H, d, J=3.6 Hz), 6.46 (2H, brs), 6.71 (1H, d, J=3.6 Hz), 7.06-7.12 (3H, m), 7.29-7.37 (3H, m).

### Reference Example A-86. 2-Amino-N-(5-phenoxy-thiophen-2-ylmethyl)-6-thiazol-2-yl-nicotinamide

To a solution of 2-amino-6-chloro-N-(5-phenoxy-thiophen-2-ylmethyl)-nicotinamide described in Reference Example A-101 (101mg, 0.281mmol) in xylene (7mL) were added 2-tributylstanyl thiazole (137mg, 0.365mmol) and tetrakis(triphenylphosphine)palladium(0) (81 mg, 0.070mmol) under nitrogen atmosphere, and the solution was stirred for 12 hours at 120°C. The reaction mixture was concentrated, the obtained residue was purified by silica gel chromatography (toluene-ethyl acetate), then, the obtained residue was washed by hexane-ethyl acetate (20:1), and the title compound (22mg, 0.054mmol, 19%) was obtained.
¹H-NMR Spectrum (DMSO-d₆) δ (ppm) : 4.50 (2H, d, J=6.0Hz), 6.50 (1H, d, J=3.6Hz), 6.78 (1 H, d, J=3.6Hz), 7.00-7.18 (3H, m), 7,22-7.50 (5H, m), 7.85 (1H, d, J=2.8Hz), 7.97 (1H, d, J=2.8Hz), 8.05 (1 H, d, J=8.4Hz), 9.12-9.22 (1 H, m).

### Reference Example A-87. 2-Amino-6-(3-methyl-2-butenyl)-N-(5-phenoxythiophen-2-ylmethyl)-nicotinamide

Trifluoroacetic acid salt of the title compound (0.71 mg, 0.0014mmol, 1.7%) was obtained from 2-amino-6-chloro-N-(5-phenoxy-thiophen-2-ylmethyl)-nicotinamide described in Reference Example A-101 (30mg, 0.083mmol) and tributyl(3-methyl-2-butenyl)tin (0.084mL, 0.25mmol) according to an analogous method to Reference Example A-29, followed by purifying by reverse phase high performance liquid chromatography (acetonitrile-water mobile phase (containing 0.1 % trifluoroacetic acid) was used).
MS m/e (ESI) 394.2 (MH⁺)

### Reference Example A-88. 2-Amino-6-(3-dimethylamino-1-propynyl)-N-(5-phenoxythiophen-2-ylmethyl)-nicotinamide

Trifluoroacetic acid salt of the title compound (1.00mg, 0.00019mmol, 4.6%) was obtained from 2-amino-6-chloro-N-(5-phenoxy-thiophen-2-ylmethyl)-nicotinamide described in Reference Example A-101 (15mg, 0.042mmol) and 1-dimethylamino-2-propyn (4.2mg, 0.050mmol) according to an analogous method to Reference Example A-91.
MS m/e (ESI) 407.2 (MH⁺)

### Reference Example A-89. 2-Amino-6-(3-fluoro-benzylamino)-N-(5-phenoxythiophen-2-ylmethyl)-nicotinamide

The title compound (20.6mg, 0.0365mmol, 43%) was obtained from 2-amino-6-chloro-N-(5-phenoxy-thiophen-2-ylmethyl)-nicotinamide described in Reference Example A-101 (30mg, 0.085mmol) and 3-fluorobenzylamine (146µl, 1.28mmol) according to an analogous method to Reference Example A-94.
MS m/e (ESI) 449.50(MH⁺)

### Reference Example A-90. 2-Amino-6-(3-methoxy-1-(Z)-propenyl)-N-(5-phenoxythiophen-2-ylmethyl)-nicotinamide

To a mixture of 2-amino-6-(3-methoxy-1-propynyl)-N-(5-phenoxy-thiophen-2-ylmethyl)-nicotinamide described in Reference Example A-91 (11 mg, 0.028mmol) and tetrahydrofuran (1 mL) were added quinoline (5.4mg, 0.042mmol) and Lindlar catalyst (5.0mg), and the mixture was stirred under hydrogen atmosphere at room temperature for 15 minutes. The interior of the reaction system was exchanged with nitrogen, then, filtration was carried out through Celite pad, and the solvent was evaporated *in vacuo.* The residue was filtered by NH silica gel, then, purified by reverse phase high performance liquid chromatography (acetonitrile-water mobile phase (containing 0.1% trifluoroacetic acid) was used), and trifluoroacetic acid salt of the title compound (4.4mg, 0.0086mmol, 31 %) was obtained.
MS m/e (ESI) 396.5 (MH⁺)

### Reference Example A-91. 2-Amino-6-(3-methoxy-1-propynyl)-N-(5-phenoxythiophen-2-ylmethyl)-nicotinamide

A mixture of 2-amino-6-chloro-N-(5-phenoxy-thiophen-2-ylmethyl)-nicotinamide described in Reference Example A-101 (15mg, 0.042mmol), methylpropargyl ether (3.5mg, 0.050mmol), diisopropylethylamine (0.023mL, 0.13mmol), pyridine (0.011mL, 0.13mmol), catalytic amount of copper(I) iodide, tetrakis(triphenylphosphine)palladium(0) (9.6mg, 0.0083mmol) and N-methylpyrrolidinone (1 mL) was stirred for 4 hours at 120°C. After cooling, water and dichloromethane were added to the reaction solution for extraction, and the organic layer was filtered through a membrane filter. The solvent was evaporated in *vacuo,* then, the residue was purified by reverse phase high performance liquid chromatography (acetonitrile-water mobile phase (containing 0.1% trifluoroacetic acid) was used), trifluoroacetic acid salt of the title compound (1.5mg, 0.00030mmol, 7.2%) was obtained.
MS m/e (ESI) 394.2 (MH⁺)

### Reference Example A-92. 2-Amino-6-(2-(4-amino-phenylamine)-ethylamino)-N-(5-phenoxy-thiophen-2-ylmethyl)-nicotinamide

2-Amino-6-(2-(4-nitro-phenylamino)-ethylamino)-N-(5-phenoxy-thiophen-2-yl methyl)-nicotinamide described in Preparation Example A+-13 (17mg, 28µmol), iron powder (7.7mg, 138µmol) and ammonium chloride (4.41mg, 83µmol) were suspended in a mixture solvent of ethanol (1mL) and water (250pl), and the solution was stirred at 90°C for 8 hours. The reaction suspension was cooled to room temperature, then, filtered through Celite pad, water was added to the filtrate, which was then extracted with ethyl acetate, the organic layer was washed with brine and dried over anhydrous magnesium sulfate. The solvent was evaporated *in vacuo,* the residue was purified by NH silica gel column chromatography (ethyl acetate : methanol = 10 : 1), and the title compound (10mg,21 µmol,77%) was obtained as a white solid.
¹H-NMR Spectrum (CDCl₃) δ (ppm) : 2.80 (3H, brs), 3.28 (2H, t, J=5.7Hz), 3.53 (2H, q, J=5.7Hz), 4.60 (2H, d, J=4.9Hz), 4.91 (1 H, t, J=5.7Hz), 5.68 (1 H, d, J=8.6Hz), 6.07 (1 H, t, J=5.3Hz), 6.38 (1 H, d, J=3.7Hz), 6.46 (2H, s), 6.52 (2H, d, J=8.8Hz), 6.60 (2H, d, J=8.8Hz), 6.71 (1 H, d, J=3.7Hz), 7.08-7.12 (3H, m), 7.29-7.34 (3H, m).

### Reference Example A-93 2-Amino-N-(5-phenoxy-thiophen-2-ylmethyl)-6-(2-(4-sulfamoyl-phenylamino)-ethylamino)-nicotinamide

MS m/e (ESI) 539.47 (MH⁺)

### Reference Example A-94. 2-Amino-6-(4-chloro-benzylamino)-N-(5-phenoxy-thiophen-2-ylmethyl)-nicotinamide

To a solution of 2-amino-6-chloro-N-(5-phenoxy-thiophen-2-ylmethyl)-nicotinamide described in Reference Example A-101 (35mg,0.10mmol) in dimethylsulfoxide (1mL) were added 4-chlorobenzylamine (234µl, 1.92mmol) and N,N-diisopropylethylamine. (1.0mL, 5.74mmol), and the solution was stirred at 140°C for 2.5 days. Ethanolamine (116µl, 1.92mmol) and N,N-diisopropylethylamine (1.0mL, 5.74mmol) were added to the reaction mixture, which was then further stirred at 140°C for 2.5 days. Water was added to the reaction mixture, which was then extracted with ethyl acetate, the organic layer was sequentially washed with water and brine, dried over anhydrous sodium sulfate, and then, concentrated *in vacuo.* The obtained residue was purified by reverse phase high performance liquid chromatography (acetonitrile-water mobile phase (containing 0.1% trifluoroacetic acid) was used), and the title compound (13.8mg, 0.024mmol, 24%) was obtained as a trifluoroacetic acid salt.
MS m/e (ESI) 465.07(MH⁺)

### Reference Example A-95. 2-Amino-6-(4-fluoro-benzylamino)-N-(5-phenoxythiophen-2-ylmethyl)-nicotinamide

The title compound (10.6mg, 0.0188mmol, 16%) was obtained as a trifluoroacetic acid salt from 2-amino-6-chloro-N-(5-phenoxy-thiophen-2-ylmethyl)-nicotinamide described in Reference Example A-101 (41mg, 0.12mmol) and 4-fluorobenzylamine (200µl, 1.75mmol) according to an analogous method to Reference Example A-94.
MS m/e (ESI) 449.56(MH⁺)

### Reference Example A-96. 2-Amino-6-(4-methoxy-benzylamino)-N-(5-phenoxythiophen-2-ylmethyl)-nicotinamide

The title compound (19.4mg, 0.034mmol, 37%) was obtained as a trifluoroacetic acid salt from 2-amino-6-chloro-N-(5-phenoxy-thiophen-2-ylmethyl)-nicotinamide described in Reference Example A-101 (32mg, 0.091mmol) and 4-methoxybenzylamine (238µl, 1.82mmol) according to an analogous method to Reference Example A-126.
MS m/e (ESI) 461.21 (MH⁺)

### Reference Example A-97. 2-Amino-N-(5-phenoxy-thiophen-2-ylmethyl)-6-(4-trifluoromethyl-benzylamino)-nicotinamide

The title compound (15.0mg, 0.024mmol, 16%) was obtained from 2-amino-6-chloro-N-(5-phenoxy-thiophen-2-ylmethyl)-nicotinamide described in Reference Example A-101 (54mg, 0.15mmol) and 4-(trifluoromethyl)benzylamine (330µl, 2.45mmol) according to an analogous method to Reference Example A-126.
MS m/e(ESI) 499.10(MH⁺)

### Reference Example A-98. 6-Acetyl-2-amino-N-(5-phenoxy-thiophen-2-vlmethyl)-nicotinamide

To a mixture of 2-amino-6-(1-ethoxy vinyl)-N-(5-phenoxy-thiophene-2-ylmethyl)-nicotinamide described in Preparation Example A+-14 (2.0mg, 0.0051 mmol) and acetone (2mL) were added water (1mL) and concentrated sulfuric acid (0.2mL), and the solution was stirred at room temperature for 3 hours. The reaction solution was neutralized with an aqueous solution of saturated sodium bicarbonate, and ethyl acetate was added for extraction. The organic layer was washed with brine, the solvent was then evaporated *in vacuo,* the residue was purified by silica gel column chromatography (hexane : ethyl acetate = 2 : 1), and the title compound (1.0mg, 0.0027mmol, 53%) was obtained as a white solid.
¹H-NMR Spectrum (CDCl₃) δ (ppm) : 2.62 (3H, s), 4.66 (2H, d, J=5.7Hz), 6.39-6.40 (4H, m), 6.75 (1 H, d, J=3.9Hz), 7.08-7.13 (3H, m), 7.29-7.35 (3H, m), 7.71 (1H, d, J=7.9Hz).

### Reference Example A-99. (6-Amino-5-((5-phenoxy-thiophen-2-ylmethyl)-carbamoyl)-pyridin-2-ylamino)-acetic acid

Glycine (610mg, 8.13mmol) and 1,8-diazabicyclo[5,4,0]undec-7-ene (405µl, 2.71 mmol) were added to 2-amino-6-chloro-N-(5-phenoxy-thiophen-2-ylmethyl)-nicotinamide described in Reference Example A-101 (195mg, 0.542mmol) under nitrogen atmosphere, and the solution was stirred at 190°C for 4 hours. Dimethylsulfoxide (5mL) was added to the reaction mixture, which was then filtered with a polytetrafluoroethylene membrane filter (Whatman Inc), the filtrate was purified by reverse phase high performance liquid chromatography (acetonitrile-water mobile phase (containing 0.1% trifluoroacetic acid) was used), title compound (55.36mg, 0.108mmol, 20%) was obtained.
MS m/e(ESI) 399.30(MH⁺)

### Reference Example A-100. 2-amino-6-(1-(Z)-hydroxylmino-ethyl)-N-(5-phenoxy-thiophen-2-ylmethyl)-nicotinamide

To a mixture of 6-acetyl-2-amino-N-(5-phenoxy-thiophen-2-ylmethyl)-nicotinamide described in Reference Example A-98 (9.0mg, 0.024mmol), ethanol (1mL) and water (0.5mL) were added hydroxylamine hydrochloride (2.6mg, 0.037mmol) and sodium acetate (3.0mg, 0.037mmol), and the solution was stirred under reflux for 6 hours. After cooling the reaction solution, water and ethyl acetate were added for extraction. The solvent was evaporated *in vacuo,* the residue was purified by NH silica gel column chromatography (methanol : ethyl acetate = 1 : 50), and the title compound (8.3mg, 0.022mmol, 90%) was obtained as a white solid.
¹H-NMR Spectrum (CDCl₃) δ (ppm) : 2.28 (3H, s), 4.65 (2H, d, J=5.7Hz), 6.36 (1 H, brs), 6.39 (1 H, d, J=3.9Hz), 6.49 (2H, brs), 6.74 (1 H, d, J=3.8Hz), 7.06-7.13 (4H, m), 7.31-7.34 (2H, m), 7.58 (1H, d, J=8.1Hz).

### Reference Example A-101. 2-Amino-6-chloro-N-(5-phenoxy-thiophen-2-ylmethyl)-nicotinamide

2-Amino-6-chloro-nicotinic acid described in Preparation Example A-1 (400mg, 2.31 mmol) was dissolved in N,N-dimethylformamide (10mL), triethylamine (0.78mL, 5.6mmol), benzotriazol-1-yloxytris(dimethylamino)phosphonium hexafluorophosphate (1.23g, 2.8mmol) and C-(5-phenoxy-thiophen-2-yl)-methylamine described in Preparation Example 26 (572mg, 2.8mmol) were added, and the solution was stirred for 13 hours 30 minutes at room temperature. After the reaction was completed, the reaction solution was poured into brine, which was then extracted with ethyl acetate. The organic layer was dried over anhydrous magnesium sulfate, then concentrated, the obtained residue was purified by silica gel column chromatography (ethyl acetate : hexane = 1:1), and the title compound (380mg, 1.05mmol, 46%) was obtained.
¹H-NMR Spectrum (DMSO-d₆) δ (ppm) : 4.47 (2H, d, J=6.0Hz), 6.50 (1 H, d, J=4.0Hz), 6.64 (1 H, d, J=8.0Hz), 6.78 (1 H, d, J=4.0Hz), 7.07-7.17 (3H, m), 7.36-7.41 (2H, m), 7.53 (2H, brs), 7.93 (1 H, d, J=8.0Hz), 9.11 (1 H, t, J=6.0Hz).

### Reference Example A-102. 2-Amino-6-cyclopropyl-N-(5-phenoxy-thiophen-2-ylmethyl)-nicotinamide

To a mixture of 2-amino-N-(5-phenoxy-thiophen-2-ylmethyl)-6-vinyl-nicotinamide described in Reference Example A-168 (6.0mg, 0.017mmol) and toluene (0.5mL) were added diiodine methane (0.0055mL, 0.068mmol) and diethyl zinc (1.1M toluene solution, 0.046mL, 0.051mmol) on an ice bath, and the solution was stirred at room temperature for 30 minutes. Water, ethyl acetate and an aqueous solution of 29% ammonia were added to the reaction solution for extraction, the organic layer was then washed with brine. The organic layer was evaporated *in vacuo*, then, residue was purified by reverse phase high performance liquid chromatography (acetonitrile-water mobile phase (containing 0.1% trifluoroacetic acid) was used), and trifluoroacetic acid salt of the title compound (0.40mg, 0.00083mmol, 4.9%) was obtained.
MS m/e (ESI) 366.1 (MH⁺)

### Reference Example A-103. 2-Amino-6-cyclopropylamino-N-(5-phenoxy-thiophen-2-ylmethyl)-nicotinamide

2-Amino-6-chloro-N-(5-phenoxy-thiophen-2-ylmethyl)-nicotinamide described in Reference Example A-101 (30mg, 0.083mmol) was dissolved in a mixture solution of dimethylsulfoxide (1mL) and N,N-diisopropylethylamine (0.5mL), cyclopropylamine (0.058mL, 0.84mmol) was added thereto, followed by heating in a sealed tube for 15 hours 30 minutes (oil bath temperature: 130°C). The reaction mixture was allowed to room temperature, poured into brine, which was then extracted with ethyl acetate. The organic layer was dried over anhydrous magnesium sulfate, then concentrated, the obtained residue was purified by NH silica gel column chromatography (ethyl acetate : hexane = 2 : 1), and the title compound (15mg, 0.039mmol, 47.5%) was obtained.
¹H-NMR Spectrum (CDCl₃) δ (ppm) : 0.52-0.57 (2H, m), 0.74-0.80 (2H, m), 2.47-2.54 (1H, m), 4.62 (2H, d, J=5.6 Hz), 5.09 (1 H, brs), 6.06 (1 H, d, J=8.4 Hz), 6.08-6.14 (1H, m), 6.34-6.42 (3H, m), 6.72 (1H, d, J=3.6 Hz), 7.06-7.13 (3H, m), 7.29-7.35 (2H, m), 7.45 (1H, d, J=8.4 Hz).

### Reference Example A-104. 2-Amino-6-(cyclopropylmethyl-amino)-N-(5-phenoxy-thiophen-2-ylmethyl)-nicotinamide

MS m/e (ESI) 395.22 (MH⁺)

### Reference Example A-105. 2-Amino-6-(2-ethoxy-ethylamino)-N-(5-phenoxythiophen-2-ylmethyl)-nicotinamide

2-Amino-6-chloro-N-(5-phenoxy-thiophen-2-ylmethyl)-nicotinamide described in Reference Example A-101 (100mg, 0.28mmol) was dissolved in a mixture solution of dimethylsulfoxide (2mL) and N,N-diisopropylethylamine (1mL), 2-ethoxyethylamine (0.051 mL, 0.49mmol) was added thereto, followed by heating in a sealed tube for 32 hours 40 minutes (oil bath temperature: 130°C). The reaction mixture was allowed to room temperature, poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1), and the title compound (37mg, 0.09mmol, 32%) was obtained.
¹H-NMR Spectrum (DMSO-d₆) δ (ppm) :1.11 (3H, t, J=7.2 Hz), 3.35-3.47 (6H, m), 4.41 (2H, d, J=6.0 Hz), 5.72 (1 H, d, J=8.8 Hz), 6.48 (1 H, d, J=3.6 Hz), 6.67-6.77 (2H, m), 7.05 (2H, brs), 7.05-7.16 (3H, m), 7.35-7.41 (2H, m), 7.59 (1 H, d, J=8.8 Hz), 8.39 (1 H, t, J=6.0Hz).

### Reference Example A-106 2-Amino-6-ethylamino-N-(5-phenoxy-thiophen-2-ylmethyl)-nicotinamide

2-Amino-6-chloro-N-(5-phenoxy-thiophen-2-ylmethyl)-nicotinamide described in Reference Example A-101 (200mg, 0.56mmol) was dissolved in a mixture solution of dimethylsulfoxide (1mL) and N,N-diisopropylethylamine (0.5mL), ethylamine (2M tetrahydrofuran solution) (2mL, 4mmol) was added thereto, followed by heating in a sealed tube for 17 hours (oil bath temperature: 135°C). The reaction mixture was allowed to room temperature, poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1), and the title compound (117mg, 0.32mmol, 57%) was obtained.
¹H-NMR Spectrum (CDCl₃) δ (ppm) :1.21 (3H, t, J=7.2 Hz), 3.24-3.32 (2H, m), 4.56-4.63 (3H, m), 5.67 (1 H, d, J=8.8 Hz), 6.06-6.11 (1H, m), 6.37 (1H, d, J=4.0 Hz), 6.42 (2H, brs), 6.71 (1 H, d, J=4.0 Hz), 7.06-7.12 (3H, m), 7.29-7.34 (2H, m), 7.37 (1 H, J=8.8 Hz).

### Reference Example A-107. (±)-2-(6-Amino-5-((5-phenoxy-thiophen-2-ylmethyl)-carbamoyl)-pyridin-2-ylamino)-(R)-3-hydroxy-butyric acid

Trifluoroacetic acid salt of the title compound (12mg, 0.022mmol, 26%) was obtained from 2-amino-6-chloro-N-(5-phenoxy-thiophen-2-ylmethyl)-nicotinamide described in Reference Example A-101 (30mg, 0.083mmol) and (L)-threonine (99mg, 0.83mmol) according to an analogous method to Reference Example A-99.
MS m/e (ESI) 443.1 (MH⁺)

### Reference Example A-108. (±)-2-(6-Amino-5-((5-phenoxy-thiophen-2-ylmethyl)-carbamoyl)-pyridin-2-ylamino)-3-phenyl-propionic acid

Trifluoroacetic acid salt of the title compound (11mg, 0.019mmol, 23%) was obtained from 2-amino-6-chloro-N-(5-phenoxy-thiophen-2-ylmethyl)-nicotinamide described in Reference Example A-101 (30mg, 0.083mmol) and (±)-phenylalanine (140mg, 0.83mmol) according to an analogous method to Reference Example A-99, trifluoroacetic acid salt of the title compound (11 mg, 0.019mmol, 23%) was obtained.
MS m/e (ESI) 489.1 (MH⁺)

### Reference Example A-109.(±)-2-(6-Amino-5-((5-phenoxy-thiophen-2-ylmethyl)-carbamoyl)-pyridin-2-ylamino)-4-methyl-pentanoic acid

Trifluoroacetic acid salt of the title compound (6.8mg, 0.012mmol, 14%) was obtained from 2-amino-6-chloro-N-(5-phenoxy-thiophen-2-ylmethyl)-nicotinamide described in Reference Example A-101 (30mg, 0.083mmol) and (D)-leucine (110mg, 0.83mmol) according to an analogous method to Reference Example A-99.
MS m/e (ESI) 455.2 (MH⁺)

### Reference Example A-110. (±)-2-(6-Amino-5-((5-phenoxy-thiophen-2-ylmethyl)-carbamoyl)-pyridin-2-ylamino)-3-methoxy-propionic acid

Trifluoroacetic acid salt of the title compound (5.5mg, 0.0099mmol, 12%) was obtained from 2-amino-6-chloro-N-(5-phenoxy-thiophen-2-ylmethyl)-nicotinamide described in Reference Example A-101 (30mg, 0.083mmol) and (±)-O-methylserine (99mg, 0.83mmol) according to an analogous method to Reference Example A-99.
MS m/e (ESI) 443.1 (MH⁺)

### Reference Example A-111. (±)-2-(6-Amino-5-((5-phenoxy-thiophene-2-ylmethyl)-carbamoyl)-pyridin-2-ylamino)-pentanedioic acid

Trifluoroacetic acid salt of the title compound (1.7mg, 0.0029mmol, 3.5%) was obtained from 2-amino-6-chloro-N-(5-phenoxy-thiophen-2-ylmethyl)-nicotinamide described in Reference Example A-101 (30mg, 0.083mmol) and (±)-glutamic acid (122mg, 0.83mmol) according to an analogous method to Reference Example A-99.
MS m/e (ESI) 471.4 (MH⁺)

### Reference Example A-112. 2-Amino-6-(2-hydroxyethoxy)-N-(5-phenoxy-thiophen-2-ylmethyl)-nicotinamide

Sodium hydride (3.1mg, 0.078mmol, 60% in oil), catalytic amount of copper(I) iodide, 2-amino-6-chloro-N-(5-phenoxy-thiophen-2-ylmethyl)-nicotinamide described in Reference Example A-102 (4.0mg, 0.011mmol) were added sequentially to ethyleneglycol (0.7mL), and the solution was stirred at 65°C for 2 hours. After further stirring at 90°C, the solution was allowed to cool to room temperature. Water, dichloromethane and an aqueous solution of saturated ammonium chloride were added to the reaction solution for extraction, which was then washed with brine, then, the solvent was evaporated *in vacuo.* The residue was purified by reverse phase high performance liquid chromatography (acetonitrile-water mobile phase (containing 0.1% trifluoroacetic acid) was used), and trifluoroacetic acid salt of the title compound (0.26mg, 0.00052mmol, 4.7%) was obtained.
MS m/e (ESI) 386.2 (MH⁺)

### Reference Example A-113. 2-Amino-6-(2-hydroxy-ethylamino)-N-(5-phenoxythiophen-2-ylmethyl)-nicotinamide

2-Amino-6-chloro-N-(5-phenoxy-thiophen-2-ylmethyl)-nicotinamide described in Reference Example A-101 (170mg, 0.47mmol) was dissolved in a mixture solution of dimethylsulfoxide (2mL) and diisopropylethylamine (1mL), ethanolamine (0.428mL, 7.1mmol) was added thereto, followed by heating in a sealed tube for 15 hours 20 minutes (oil bath temperature: 135°C). The reaction mixture was allowed to room temperature, poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1), and the title compound (138mg, 0.36mmol, 76%) was obtained.
¹H-NMR Spectrum (DMSO-d₆) δ (ppm) : 3.24-3.31 (2H, m), 3.44-3.51 (2H, m), 4.39 (2H, d, J=5.6 Hz), 4.68 (1 H, t, J=5.2 Hz), 5,70 (1 H, d, J= 8.4 Hz), 6.46 (1 H, d, J=4.0 Hz), 6.66 (1H, brs), 6.70 (1H, d, J=4.0Hz), 7.02 (2H, brs), 7.04-7.14 (3H, m), 7.34-7.39 (2H, m), 7.57 (1 H, d, J=8.4 Hz), 8.37 (1 H, t, J=5.6 Hz).

### Reference Example A-114. 2-Amino-6-hydroxymethyl-N-(5-phenoxy-thiophen-2-ylmethyl)-nicotinamide

To a mixture of 2-amino-N-(5-phenoxy-thiophen-2-ylmethyl)-6-tributylstanyl-nicotinamide described in Preparation Example A+-15 (98mg, 0.16mmol) in tetrahydrofuran (1.5mL) was added *n*-butyl lithium (2.4M hexane solution,0.32mL,0.80mmol) dropwise at -78°C, and the solution was stirred for 1 hour 40 minutes at the same temperature. N,N-dimethylformamide (0.037mL, 0.48mmol) was added at the same temperature, the solution was stirred fro 35 minutes, then, a solution of sodium borocyanide (50mg, 0.80mmol) in tetrahydrofuran (1mL) was added dropwise at the same temperature, and the solution was stirred at -3°C for 1 hour. The reaction solution was cooled to -78°C, acetic acid (0.091 mL, 1.6mmol) was added, and the solution was warmed gradually to 0°C. Water, ethyl acetate and tetrahydrofuran were added to the reaction solution for extraction, which was then washed with brine, then, the solvent was evaporated *in vacuo.* The residue was purified by silica gel column chromatography (methanol : ethyl acetate = 1 : 50), and the title compound (19mg, 0.053mmol, 33%) was obtained as a pale yellow oil.
¹H-NMR Spectrum (CDCl₃) δ (ppm) : 4.58 (2H, s), 4.64 (2H, d, J=5.5Hz), 6.38 (1 H, d, J=3.7Hz), 6.43 (1H, brs), 6.48-6.50 (3H, m), 6.74 (1H, d, J=3.7Hz), 7.08-7.13 (3H, m), 7.30-7.34 (2H, m), 7.59 (1 H, d, J=7.9Hz).

### Reference Example A-115. 2-Amino-6-isopropoxy-N-(5-phenoxy-thiophen-2-ylmethyl)-nicotinamide

Trifluoroacetic acid salt of the title compound (4.3mg, 0.0086mmol, 9.0%) was obtained from 2-amino-6-chloronicotinic acid described in Preparation Example A-4 (17mg, 0.096mmol), isopropanol (0.5mL) and C-(5-phenoxy-thiophen-2-yl)-methylamine (20mg, 0.097mmol) according to an analogous method to Reference Example A-29.
MS m/e (ESI) 384.2 (MH⁺)

### Reference Example A-116. 2-Amino-6-methoxy-N-(5-phenoxy-thiophen-2-ylmethyl)-nicotinamide

2,5-Diamino-N-(5-phenoxy-thiophen-2-ylmethyl)-nicotinamide described in Preparation Example A+-10 (27mg,59µmol), sodium nitrite (4.1 mg,59µmol) and sulfuric acid (several drops) were dissolved in methanol (5mL), and the solution was stirred for 30 minutes under reflux. An aqueous solution of saturated sodium bicarbonate was added to the reaction solution at 0°C, which was then extracted with ethyl acetate, and the organic layer was washed with brine. The solvent was evaporated *in vacuo,* the residue was purified by reverse phase high performance liquid chromatography (acetonitrile-water mobile phase (containing 0.1% trifluoroacetic acid) was used), then, further by NH silica gel column chromatography (hexane : ethyl acetate = 5 : 1), and the title compound (0.7mg) was obtained as a white solid.
MS m/e (ESI) 356.32(MH⁺).
¹H-NMR Spectrum (CDCl₃) δ (ppm) : 3.87 (3H, s), 4.63 (2H, d, J=5.7Hz), 6.01 (1 H, d, J=8.6Hz), 6.11 (1 H, brs), 6.39 (1 H, d, J=3.8Hz), 6.51 (1 H, brs), 6.73 (1 H, d, J=3.3Hz), 7.08-7.12 (3H, m), 7.22-7.26 (1 H, m), 7.32 (2H, t, J=8.6Hz), 7.50 (1 H, t, J=8.8Hz).

### Reference Example A-117. 2-Amino-N-(5-benzofuran-5-ylmethyl-thiophen-2-ylmethyl) -6-methoxymethyl-nicotinamide

MS m/e (ESI) 407.85 (MH⁺)

### Reference Example A-118. 2-Amino-N-(5-benzo[1,3]dioxol-5-ylmethyl-thiophen-2-ylmethyl) 6-methoxymethyl-nicotinamide

¹H-NMR Spectrum (CDCl₃) δ (ppm) : 3.33 (3H, s), 3.96 (2H, s), 4.28 (2H, s), 4.47 (2H, d, J=6.0Hz), 5.96 (2H, d, J=1.2Hz), 6.59 (1H, d, J=8.0Hz), 6.68-6.74 (2H, m), 6.77-6.84 (3H, m), 7.12 (2H, brs), 7.90 (1 H, d, J=8.0Hz), 8.97 (1 H, t, J=6.0Hz).

### Reference Example A-119. 2-Amino-6-methoxymethyl-N-(5-phenoxy-thiophen-2-ylmethyl)-nicotinamide

¹H-NMR Spectrum (CDCl₃) δ (ppm) : 3.46(3H, s), 4.41(2H, s), 4.64-4.66(2H, m), 6.32(1 H, br s), 6.39(1 H, d, J=3.8Hz), 6.47(2H, br s), 6.71(1H, d, J=7.9Hz), 6.74(1 H, d, J=3.8Hz), 7.08-7.13(3H; m), 7.31-7.35(2H, m), 7.62 (1 H, d, J=7.9Hz).

### Reference Example A-120. 2-Amino-N-(4-benzylamino-benzyl)-6-methoxymethyl-nicotinamide

MS m/e (ESI) 377 (MH⁺)

### Reference Example A-121. 2-Amino-N-(5-benzyl-thiophen-2-ylmethyl)-6-methoxymethyl-nicotinamide

¹H-NMR Spectrum (CDCl₃) δ (ppm) : 3.44 (3H, s), 4.09 (2H, s), 4.38 (2H, s), 4.66 (2H, d, J=5.2 Hz), 6.25-6.34 (1 H, m), 6.38 (2H, br s), 6.65 (1 H, d, J=3.6 Hz), 6.67 (1 H, d, J=8.0 Hz), 6.82 (1 H, d, J=3.6 Hz), 7.20-7.27 (3H, m), 7.27-7.34 (2H, m), 7.57 (1 H, d, J=8.0 Hz).

### Reference Example A-122. 2-Amino-N-(5-(3-chloro-phenoxy)-thiophen-2-ylmethyl)-6-methoxymethyl-nicotinamide

MS m/e (ESI) 404 (MH⁺)

### Reference Example A-123. 2-Amino-N-(5-(3-fluoro-phenoxy)-thiophen-2-ylmethyl) 6-methoxymethyl-nicotinamide

MS m/e (ESI) 388 (MH⁺)

### Reference Example A-124. 2-Amino-6-(3-methoxy-propyl)-N-(5-phenoxy-thiophen-2-ylmethyl)-nicotinamide

To a mixture of 2-amino-6-(3-methoxy-1-(Z)-propenyl)-N-(5-phenoxy-thiophen-2-ylmethyl)-nicotinamide described in Reference Example A-90 (3.0mg, 0.0059mmol) and tetrahydrofuran (1 mL) were added triethylamine (3.6mg, 0.036mmol) and 10% palladium-carbon (50% water wet, 5mg), and the solution was stirred under hydrogen atmosphere at room temperature for 15 minutes. The interior of the reaction system was exchanged with nitrogen, then, filtration was carried out through Celite pad, the solvent was evaporated *in vacuo.* The residue was purified by reverse phase high performance liquid chromatography (acetonitrile-water mobile phase (containing 0.1% trifluoroacetic acid) was used), and trifluoroacetic acid salt of the title compound (0.48mg, 0.00094mmol, 16%) was obtained.
MS m/e (ESI) 398.3 (MH⁺)

### Reference Example A-125. 2-Amino-6-methylamino-N-(5-phenoxy-thiophen-2-ylmethyl)-nicotinamide

2-Amino-6-chloro-N-(5-phenoxy-thiophen-2-ylmethyl)-nicotinamide described in Reference Example A-101 (200mg, 0.56mmol) was dissolved in a mixture solution of dimethylsulfoxide (1mL) and diisopropylethylamine (0.5mL), methylamine (2M tetrahydrofuran solution) (2mL, 4mmol) was added thereto, followed by heating in a sealed tube for 14 hours (oil bath temperature: 135°C). The reaction mixture was allowed to room temperature, poured into brine, and the solution was extracted with ethyl acetate. The organic layer was dried over anhydrous magnesium sulfate, then concentrated, the obtained residue was purified by NH silica gel column chromatography (ethyl acetate : hexane = 2 : 1), and the title compound (144mg, 0.41 mmol, 73%) was obtained.
¹H-NMR Spectrum (CDCl₃) δ (ppm) : 2.89 (3H, d, J=5.2 Hz), 4.61 (2H, d, J=5.6 Hz), 4.64-4.71 (1 H, m), 5.69 (1 H, d, J=8.4 Hz), 6.03-6.09 (1 H, m), 6.38 (1 H, d, J=4.0 Hz), 6.44 (2H, brs), 6.71 (1 H, d, J=4.0 Hz), 7.06-7.12 (3H, m), 7.29-7.35 (2H, m), 7.39 (1 H, d, J=8.4 Hz).

### Reference Example A-126. 2-Amino-6-benzylamino-N-(5-phenoxy-thiophen-2-ylmethyl)-nicotinamide

To a solution of 2-amino-6-chloro-N-(5-phenoxy-thiophen-2-ylmethyl)-nicotinamide described in Reference Example A-101 (110mg, 0.31 mmol) in dimethylsulfoxide (1mL) were added benzylamine (1.8mL, 16.5mmol) and N,N-diisopropylethylamine (0.5mL, 2.94mmol), and the solution was stirred for 17 hours at 135°C. Water was added to the reaction mixture, which was then extracted with ethyl acetate, the organic layer was sequentially washed with water and brine, dried over anhydrous sodium sulfate, and then, concentrated *in vacuo.* The obtained residue was purified by reverse phase high performance liquid chromatography (acetonitrile-water mobile phase (containing 0.1% trifluoroacetic acid) was used), and the title compound (23.4mg, 0.0429mmol, 14%) was obtained as a trifluoroacetic acid salt.
MS m/e(ESI) 431.27(MH⁺)

### Reference Example A-127. 2-Amino-N-(5-phenoxy-thiophen-2-ylmethyl)-6-aropoxynicotinamide

Trifluoroacetic acid salt of the title compound (5.3mg, 0.011 mmol, 11 %) was obtained from 2-amino-6-chloronicotinic acid described in Preparation Example A-4 (17mg, 0.096mmol), propanol (0.5mL) and C-(5-phenoxy-thiophen-2-yl)-methylamine (20mg, 0.097mmol) according to an analogous method to Reference Example A-29.
MS m/e (ESI) 384.1 (MH⁺)

### Reference Example A-128. 2-Amino-N-(5-phenoxy-thiophen-2-ylmethyl)-6-propylamino-nicotinamide

2-Amino-6-chloro-N-(5-phenoxy-thiophen-2-ylmethyl)-nicotinamide described in Reference Example A-101 (200mg, 0.56mmol) was dissolved in a mixture solution of dimethylsulfoxide (2mL) and N,N-diisopropylethylamine (1mL), propylamine (0.685mL, 8.3mmol) was added thereto, followed by heating in a sealed tube for 13 hours (oil bath temperature: 135°C). The reaction mixture was allowed to room temperature, poured into brine, which was then extracted with ethyl acetate. The organic layer was dried over anhydrous magnesium sulfate, then concentrated, the obtained residue was purified by NH silica gel column chromatography (ethyl acetate : hexane = 2 : 1), and the title compound (89mg, 0.23mmol, 42%) was obtained.
¹H-NMR Spectrum (CDCl₃) δ (ppm) : 0.96 (3H, t, J=7.6Hz), 1.54-1.64 (2H, m), 3.16-3.22 (2H, m), 4.59 (2H, d, J=5.6 Hz), 4.69 (1H, t, J=5.2 Hz), 5.67 (1H, d, J=8.8 Hz), 6.16 (1 H, t, J=5.6 Hz), 6.36 (1 H, d, J=3.6 Hz), 6.42 (2H, brs), 6.69 (1 H, d, J=3.6 Hz), 7.06-7.12 (3H, m), 7.29-7.35 (2H, m), 7.37 (1 H, J=8.8 Hz).

### Reference Example A-129. 2-Amino-N-(5-phenoxy-thiophen-2-ylmethyl)-6-((pyrazin-2-ylmethyl)-amino)-nicotinamide

MS m/e (ESI) 433.15(MH⁺)
¹H-NMR Spectrum (DMSO-d₆) δ (ppm) : 4.33(2H, d, J=5.6Hz), 4.68(2H, s), 5.99(1 H, d, J=8.4Hz), 6.48(1H, d, J=3.6Hz), 6.73(1H, d, J=3.6Hz), 7.06-7.15(3H, m), 7.34-7.39(2H, m), 7.82-7.94 (1H, m), 8.55 (1H, d, J=2.4Hz), 8.60 (1H, dd, J=2.4,1.2Hz), 8.67 (1 H, d, J=1.2Hz), 8.69-8.79(1 H, m).

### Reference Example A-130. 2-Amino-N-(5-phenoxy-thiophen-2-ylmethyl)-6-((pyridin-2-ylmethyl)-amino)-nicotinamide

MS m/e (ESI) 432.17 (MH⁺)

### Reference Example A-131. 3-(3-(5-Phenoxy-thiophen-2-ylmethyl)-carbamoyl)-pyridin-2-ylamino)-propionic acid

To a solution of 2-chloro-N-(5-phenoxy-thiophen-2-ylmethyl)-nicotinamide described in Preparation Example A+-7 (51 mg, 0.15mmol) in dimethylsulfoxide (2mL) were added *tert*-butyl 3-aminopropanoate hydrochloride (32mg, 0.178mmol) and triethylamine (27µl, 0.192mmol), and the solution was stirred for 2.5 hours at 120°C. Potassium carbonate (49mg, 0.36mmol) was added to the reaction mixture, which was then stirred for 20 hours 120°C. Water was added to the reaction mixture, which was then extracted with ethyl acetate and concentrated. To a solution of the resulting residue in dichloromethane (1mL) was added trifluoroacetic acid (500µl, 6.49mmol), and the solution was stirred for 2.5 hours at room temperature. The reaction mixture was concentrated, and the obtained residue was purified by reverse phase high performance liquid chromatography (acetonitrile-water mobile phase (containing 0.1% trifluoroacetic acid) was used), and the title compound (3.44mg, 0.0067mmol, 4.5%) was obtained as a trifluoroacetic acid salt.
MS m/e(ESI) 398.52(MH⁺)

### Reference Example A-132. 2-Chloro-N-(5-(3-fluorophenoxy)thiophen-2-ylmethyl)-6-methylnicotinamide

The title compound (330mg, 0.877mmol, 65.0%) was obtained as a white solid from 2-chloro-6-methylnicotinic acid (230mg, 1.35mmol) and C-(5-(3-fluorophenoxy)thiophen-2-yl)methylamine described in Preparation Example 23 (300mg,1.35mmol) according to an analogous method to Reference Example Q-6.
¹H-NMR Spectrum (DMSO-d₆) δ (ppm) : 2.47 (3H, s), 4.52 (2H, d, J=5.6Hz), 6.60-6.63 (1H, m), 6.84 (1H, d, J=4.0Hz), 6.92-7.04 (3H, m), 7.32-7.35 (1H, m), 7.40-7.47 (1 H, m), 7.77 (1 H, d, J=7.2Hz), 9.15 (1 H, t, J=5.6Hz).

### Reference Example A-133. 2-(Cyclopropylmethyl-amino)-N-(5-phenoxy-thiophen-2-ylmethyl)-nicotinamide

To a solution of 2-chloro-N-(5-phenoxy-thiophen-2-ylmethyl)-nicotinamide described in Preparation Example A+-7 (64mg, 0.186mmol) in dimethylsulfoxide (1 mL) was added (aminomethyl)cyclopropane (48µl, 0.56mmol), and the solution was stirred for 14 hours at 120°C. The reaction mixture was purified by reverse phase high performance liquid chromatography (acetonitrile-water mobile phase (containing 0.1 % trifluoroacetic acid) was used), and the title compound (15.6mg, 0.0316mmol, 17%) was obtained as a trifluoroacetic acid salt.
MS m/e(ESI) 380.43(MH⁺)

### Reference Example A-134. 2-(2-Methoxy-ethylamino)-N-(5-phenoxy-thiophen-2-ylmethyl)-nicotinamide

To a solution of 2-chloro-N-(5-phenoxy-thiophen-2-ylmethyl)-nicotinamide described in Preparation Example A+-7 (16mg, 0.046mmol) in 1-methyl-2-pyrrolidone (2mL) were added 2-methoxyethylamine (6µl, 0.07mmol) and sodium hydride (4mg, 0.092mmol, 60% in oil), and the solution was stirred for 8 hours at 110°C. An aqueous solution of saturated ammonium chloride was added to the reaction mixture, which was then extracted with ethyl acetate, and concentrated. The obtained residue was purified by reverse phase high performance liquid chromatography (acetonitrile-water mobile phase (containing 0.1% trifluoroacetic acid) was used), and the title compound (0.67mg, 0.0013mmol, 2.8%) was obtained as a trifluoroacetic acid salt.
MS m/e(ESI) 384.16(MH⁺)

### Reference Example A-135. 2-Methyl-N-(5-phenoxy-thiophen-2-ylmethyl)-nicotinamide

The title compound (40mg, 0.123mmol, 42.4 %) was obtained as a light brown solid from 2-methylnicotinic acid (40mg,0.29mmol) and C-(5-phenoxy-thiophen-2-yl)-methylamine described in Preparation Example 26 (60mg, 0.29mmol) according to an analogous technique to Reference Example Q-6.
¹H-NMR Spectrum (DMSO-d₆) δ (ppm) : 2.47 (3H, s), 4.49 (2H, d, J=5.6Hz), 6.50-6.63 (1 H, m), 6.77-6.80 (1H, m), 7.06-7.16 (3H, m), 7.23-7.28 (1 H, m), 7.35-7.40 (2H, m), 7.66-7.70 (1 H, m), 8.48 (1 H, dd, J=1.6, 4.8Hz), 9.05 (1H, t, J=5.6Hz).

### Reference Example A-136. 2-Methylamino-N-(5-phenoxy-thiophen-2-ylmethyl)-nicotinamide

To a solution of 2-(ethoxymethyl-amino)-N-(5-phenoxy-thiophene-2-ylmethyl)-nicotinamide described in Preparation Example A+-6 (148mg, 0.385mmol) in dimethylsulfoxide (3mL) was added sodium borohydride (44mg, 1.15mmol), and the solution was stirred for 30 minutes at 100°C, followed by stirring for 20 minutes at 110°C. Furthermore, sodium borohydride (35mg, 0.925mmol) was added thereto, followed by stirring for 20 minutes at 110°C. Water was added to the reaction mixture, which was then extracted with ethyl acetate, the organic layer was sequentially washed with water and brine, dried over anhydrous sodium sulfate, and then, concentrated *in vacuo.* The obtained residue was purified by silica gel chromatography (hexane-ethyl acetate), and the title compound (86mg, 0.26mmol, 67%) was obtained.
¹H-NMR Spectrum (DMSO-d₆) δ (ppm) : 2.87 (3H, d, J=4.8Hz), 4.46 (2H, d, J=5.6Hz), 6.48 (1H, d, J=3.6Hz), 6.53 (1H, dd, J=4.8, 7.6Hz), 6.76 (1H, d, J=3.6Hz), 7.00-7.18(3H, m), 7.30-7.41 (2H, m), 7.88 (1 H, dd, J=2.0, 7.6Hz), 8.10-8.25 (2H, m), 9.07 (1 H, t, J=5.6Hz).

### Reference Example A-137. 6-Amino-N-(5-(3-fluorophenoxy)thiophen-2-ylmethyl)nicotinamide

The title compound (20mg, 0.058mmol, 21.6%) was obtained as a white solid from 6-amino-nicotinic acid (37mg, 0.27mmol) and C-(5-(3-fluorophenoxy)thiophen-2-yl)methylamine described in Preparation Example 23 (60mg, 0.27mmol) according to an analogous method to Reference Example Q-6.
¹H-NMR Spectrum (DMSO-d₆) δ (ppm) : 4.56 (2H, d, 6.0Hz), 6.39 (1H, d, J=8.8Hz), 6.48 (2H, brs), 6.56 (1 H, d, J=3.6Hz), 6.76 (1 H, d, J=3.6Hz), 6.88-7.00 (3H, m), 7.39 (1 H, ddd, J=8.0, 8.0, 8.0), 7.79 (1 H, dd, J=2.0, 8.8Hz), 8.43 (1 H, d, J=2.0Hz), 8.77 (1H, t, J=6.0Hz).

### Reference Example A-138. 6-Amino-N-(5-phenoxy thiophen-2-ylmethyl)-nicotinamide

To a solution of C-(5-phenoxythiophen-2-yl)methylamine described in Preparation Example 24 (170mg, 0.83mmol) and 6-aminonicotinic acid (130mg, 0.91mmol) in N,N-dimethylformamide (10mL) were added benzotriazol-1-yl-tris(dimethylamino)phosphonium hexafluorophosphate (400mg, 0.91 mmol) and triethylamine (0.3mL, 2.2mmol), and the solution was stirred for 35 minutes at 60°C. Water and ethyl acetate were added to the reaction solution, which was then partitioned, the organic layer was washed with water twice, NH silica gel was added to the organic layer, the solvent was evaporated *in vacuo* for adsorption, purification was carried out by NH silica gel column chromatography (ethyl acetate, then ethyl acetate : methanol = 50 : 1), and the title compound (130mg, 0.40mmol, 48.2%) was obtained as a solid.
¹H-NMR Spectrum (DMSO-d₆) δ (ppm) : 4.45 (2H, d, 5.2Hz), 6.39 (1H, d, J=8.8Hz), 6.43-6.54 (3H, m), 6.70-6.77 (1 H, m), 7.20-7.16 (3H, m), 7.31-7.41 (2H, m), 7.78 (1 H, d, J=8.8Hz), 8.43 (1 H, s), 8.76 (1 H, d, J=5.2Hz).

### Reference Example A-139. 6-Amino-N-(5-(4-fluoro-phenoxy)-thiophen-2-ylmethyl)-nicotinamide

To a solution of C-(5-(4-fluorophenoxy)thiophen-2-yl)methylamine described in Preparation Example 28 (500mg, 2.24mmol) and 6-aminonicotinic acid (340mg, 2.46mmol) in N,N-dimethylformamide (10mL) were added benzotriazol-1-yl-tris(dimethylamino)phosphonium hexafluorophosphate (1.1g, 2.46mmol) and triethylamine (0.6mL, 4.48mmol), and the solution was stirred for 30 minutes at 60°C. Water and ethyl acetate were added to the reaction solution, which was then partitioned, and the organic layer was washed with water three times. The organic layer was passed through a glass filter lined with NH silica gel and silica gel (1:1), and eluted with a mixture solvent of ethyl acetate and methanol (20 : 1). The solvent was evaporated *in vacuo,* ethyl acetate and hexane were added to the residue, the generated solid was collected by filtration, and the title compound (560mg, 1.63mmol, 72.8%) was obtained as a slightly yellow solid.
¹H-NMR Spectrum (DMSO-d₆) δ (ppm) : 4.44 (2H, d, J=5.2Hz), 6.39 (1H, d, J=8.8Hz), 6.44-6.50 (3H, m), 6.72 (1 H, d, J=2.4Hz), 7.09-7.16 (2H, m), 7.16-7.24 (2H, m), 7.78 (1 H, d, J=8.8Hz), 8.42 (1 H, s), 8.74 (1 H, t, J=5.2Hz).

### Reference Example A-140. 6-Amino-N-(5-(4-chloro-phenoxy)-thiolohen-2-ylmethyl)-nicotinamide

The title compound (32mg, 0.089mmol, 30.7%) was obtained as a white solid from C-(5-(4-chlorophenoxy)thiophen-2-yl)methylamine obtained according to an analogous method to Reference Example E-66 (70mg, 0.29mmol) and 6-amino-nicotinic acid (40mg, 0.29mmol).
¹H-NMR Spectrum (DMSO-d₆) δ (ppm) : 4.45 (2H, d, 5.2Hz), 6.39 (1H, d, J=8.4Hz), 6.48 (2H, s), 6.51 (1 H, d, J=3.6Hz), 6.74 (1H, d, J=3.6Hz), 7.04-7.14 (2H, m), 7.36-7.46 (2H, m), 7.78 (1 H, d, J=8.4Hz), 8.43 (1 H, s), 8.76 (1 H, t, J=5.2Hz).

### Reference Example A-141. 6-Amino-N-(5-m-tolyloxy-thiophen-2-ylmethyl)nicotinamide

The title compound (243mg, 0.717mmol, 52.7%) was obtained as a light brown solid from C-(5-*m*-tolyloxythiophen-2-yl)methylamine described in Preparation Example 30 (300mg, 1.36mmol) and 6-aminonicotinic acid (210mg, 1.52mmol) according to an analogous method to Reference Example Q-6.
¹H-NMR Spectrum (DMSO-d₆) δ (ppm): 2.26 (3H, s), 4.44 (2H, d, J=5.6Hz), 6.39 (1 H, d, J=8.8Hz), 6.42-6.52 (3H, m), 6.70-6.74 (1 H, m), 6.84-6.95 (3H, m), 7.22 (1 H, dd, J=8.0, 8.0Hz), 7.78 (1 H, dd, J=2.4, 8.8Hz), 8.43 (1 H, d, J=2.4Hz), 8.74 (1 H, t, J=5.6Hz).

### Reference Example A-142. 6-Amino-N-(5-p-tolyloxy-thiophene-2-ylmethyl)-nicotinamide

The title compound (265mg, 0.78mmol, 52.1 %) was obtained as a light brown solid from 6-aminonicotinic acid (210mg, 1.50mmol) and C-(5-*p*-tolyloxy-thiophen-2-yl)-methylamine described in Preparation Example 32 (300mg, 1.37mmol) according to an analogous method to Reference Example Q-6.
¹H-NMR Spectrum (DMSO-d₆) δ (ppm) : 2.24 (3H, s), 4.43 (2H, d, J=5.6Hz), 6.38 (1H, d, J=8.8Hz), 6.41 (1H, d, J=3.6Hz), 6.47 (2H, s), 6.70 (1H, d, J=3.6Hz), 6.94-7.00 (2H, m), 7.13-7.19 (2H, m), 7.78 (1H, dd, J=2.4, 8.8Hz), 8.42 (1H, d, J=2.4Hz), 8.73 (1 H, t, J=5.6Hz).

### Reference Example A-143. 6-Amino-N-(5-(4-fluoro-benzyl)-thiophen-2-ylmethyl)-nicotinamide

The title compound (46mg, 0.13mmol, 81.2%) was obtained as a white solid from C-(5-(4-fluoro-benzyl)-thiophen-2-yl)-methylamine described in Preparation Example 38 (35mg, 0.16mmol) and 6-aminonicotinic acid (24mg, 0.17mmol) according to an analogous method to Reference Example Q-6.
¹H-NMR Spectrum (DMSO-d₆) δ(ppm) : 4.03 (2H, s), 4.45 (2H, d, J=5.6Hz), 6.38 (1 H, d, J=8.8Hz), 6.45 (2H, s), 6.67 (1 H, d, J=3.6Hz), 6.76 (1 H, d, J=3.6Hz), 7.04-7.14 (2H, m), 7.20-7.30 (2H, m), 7.77 (1 H, dd, J=2.4, 8.8Hz), 8.41 (1 H, d, J=2.4Hz), 8.69 (1 H, t, J=5.6Hz).

### Reference Example A-144. 6-Amino-N-(5-benzyl-thiophen-2-ylmethyl)-nicotinamide

The title compound (27mg, 0.082mmol, 31.0 %) was obtained as a white solid from 6-aminonicotinic acid (37mg, 0.27mmol) and C-(5-benzyl-thiophen-2-yl)-methylamine described in Preparation Example 42 (54mg, 0.27mmol) according to an analogous technique to Reference Example Q-6.
¹H-NMR Spectrum (DMSO-d₆) δ (ppm) : 4.03 (2H, s), 4.44 (2H, d, J=5.2Hz), 6.37 (1 H, d, J=8.8Hz), 6.45 (2H, s), 6.67 (1 H, d, J=3.2Hz), 6.75 (1 H, d, J=3.2Hz), 7.15-7.30 (5H, m), 7.76 (1H, dd, J=2.0, 8.8Hz), 8.41 (1H, d, J=2.0Hz), 8.68 (1H, t, J=5.2Hz).

### Reference Example A-145. 6-Amino-N-(5-(3-chloro-benzyl)-thiophen-2-ylmethyl)-nicotinamide

The title compound (42mg, 0.12mmol, 56.0%) was obtained as a white solid from C-(5-(3-chloro-benzyl-2-yl)methylamine described in Preparation Example 45 (50mg, 0.21 mmol) and 6-aminonicotinic acid (32mg, 0.23mmol) according to an analogous method to Reference Example Q-6.
¹H-NMR Spectrum (DMSO-d₆) δ (ppm) : 4.05 (2H, s), 4.45 (2H, d, J=5.6Hz), 6.38 (1H, d, J=8.8Hz), 6.45 (2H, s), 6.71 (1H, d, J=3.2Hz), 6.76 (1H, d, J=3.2Hz), 7.16-7.34 (4H, m), 7.77 (1 H, d, J=8.8Hz), 8.41 (1 H, s), 8.70 (1 H, t, J=5.6Hz).

### Reference Example A-146. 6-Amino-N-(5-(3-fluoro-benzyl)-thiophen-2-ylmethyl)-nicotinamide

To a solution of 5-(3-fluoro-benzyl)-thiophene-2-carbaldehyde described in Preparation Example 53 (485mg, 2.2mmol) in 7N ammonia/methanol (30mL) was added Raney nickel (1g), and the solution was stirred under hydrogen atmosphere at room temperature for 16 hours. The reaction solution was filtered through Celite pad to remove the catalyst, then, the organic layer was concentrated *in vacuo,* the residue was purified by NH silica gel column chromatography (hexane, then hexane : ethyl acetate = 4 : 1), and C-(5-(3-fluoro-benzyl-2-yl)-methylamine (290mg, 1.3mmol, 59.6%) was obtained as a brown oil. Using the resulting C-(5-(3-fluoro-benzyl-2-yl)-methylamine (50mg, 0.226mmol) and 6-aminonicotinic acid (34mg, 0.248mmol) according to an analogous technique to Example Q-6, the title compound (44mg, 0.129mmol, 57.1 %) was obtained as a white solid.
¹H-NMR Spectrum (DMSO-d₆) δ (ppm) : 4.06 (2H, s), 4.45 (2H, d, J=5.6Hz), 6.38 (1H, d, J=8.8Hz), 6.45 (2H, s), 6.70 (1H, d, J=2.0Hz), 6.77 (1H, d, J=2.0Hz), 6.96-7.10 (3H, m), 7.28-7.36 (1H, m), 7.77 (1H, dd, J=2.0, 8.8Hz), 8.41 (1H, d, J=2.0Hz), 8.70 (1H, t, J=5.6Hz).

### Reference Example A-147. 6-Amino-N-(5-(3-chloro-phenoxy)-thiophen-2-ylmethyl)-nicotinamide

The title compound (8.21 mg) was obtained from C-(5-(3-chloro-phenoxy)-thiophen-2-yl)-methylamine described in Reference Example A-73 (30mg, 0.13mmol) and 6-amino-nicotinic acid (17mg, 0.13mmol) according to an analogous method to Reference Example E-24, the title compound (8.21 mg) was obtained. Trifluoroacetic acid salt of the tile compound was obtained by reverse phase high performance liquid chromatography (acetonitrile-water mobile phase (containing 0.1% trifluoroacetic acid) was used).
MS m/e (ESI) 360.3(MH⁺)

### Reference Example A-148. 6-Amino-N-(5-(2-fluoro-phenoxy)-thiophen-2-ylmethyl)-nicotinamide

Trifluoroacetic acid salt of the title compound (26.6mg) was obtained as a pale yellow oil from 6-amino-nicotinic acid (21 mg, 0.15mmol) and C-(5-(2-fluoro-phenoxy)-thiophen-2-yl)-methylamine described in Preparation Example 161 (33.5mg, 0.15mmol) according to an analogous method to Reference Example A-75. Then, the title compound (7.8mg, 0.023mmol, 15.1 %) was obtained as a pale yellow solid by purifying again by thin layer NH silica gel chromatography (ethyl acetate).
MS m/e (ESI) 344(MH⁺)

### Reference Example A-149. 6-Amino-N-(5-(2-cyclopropylyinyl)thiophen-2-ylmethyl) nicotinamide

6-Aminonicotinic acid (35mg, 0.251 mmol), C-(5-(2-cyclopropylvinyl)thiophen-2-yl)methylamine described in Preparation Example 151 (45mg,0.251mmol), benzotriazol-1-yl-tris(dimethylamino)phosphonium hexafluorophosphate (133mg,0.301 mmol) and triethylamine (0.042mL,0.301mmol) were dissolved in N,N-dimethylformamide (3mL), and the solution was stirred at room temperature for 3 hours. Water (10mL) was added to the reaction solution, which was then extracted with ethyl acetate (30mL). The organic layer was washed, dried over anhydrous magnesium sulfate, then, the solvent was evaporated *in vacuo,* the obtained residue was purified by NH silica gel column chromatography (hexane : ethyl acetate = 3 : 1), and the title compound (50mg, 0.167mmol, 66.6%) was obtained as a white solid.
¹H-NMR Spectrum (CDCl₃) δ (ppm) : 0.44-0.52(2H, m), 0.76-0.84(2H, m), 1.45-1.55(1H, m), 4.70(2H, d, J=5.6Hz), 4.76(2H, s), 5.53(1H, dd, J=8.8, 15.6Hz), 6.21(1H, d, J=5.6Hz), 6.49(1H, d, J=8.8Hz), 6.52(1H, d, J=15.6Hz), 6.66(1H, d, J=3.6Hz), 6.83(1 H, d, J=3.6Hz), 7.88(1 H, dd, J=2.4, 8.8Hz), 8.48(1H, d, J=2.4Hz).

### Reference Example A-150. 6-Amino-N-(5-(4-fluoro-phenoxy)-thioiphen-2-ylmethyl)-nicotinamide

¹H-NMR Spectrum (CDCl₃) δ(ppm) : 4.44 (2H, d, J=5.2Hz), 6.39 (1H, d, J=8.8Hz), 6.43-6.52 (3H, m), 6.72 (1 H, d, J=2.4Hz), 7.09-7.16 (2H, m), 7.16-7.24 (2H, m), 7.78 (1 H, dd, J=2.4, 8.8Hz), 8.42 (1 H, s), 8.74 (1 H, t, J=5.2Hz).

### Reference Example A-151. 6-Amino-N-(5-(3-cyano-phenoxy)-thiophen-2-ylmethyl)-nicotinamide

¹H-NMR Spectrum (CDCl₃) δ (ppm) : 4.68(2H, d, J=5.6Hz), 4.78(2H, s), 6.27(1H, brs), 6.38-6.40(1H, m), 6.49-6.52(1H, m), 6.74-6.75(1H, m), 7.08-7.10(2H, m), 7.30-7.33(2H, m), 7.88-7.91 (1 H, m), 8.48-8.49(1 H, m).

### Example A-152. N-(5-Benzyl-thiophen-2-ylmethyl)-6-(carbamoylmethyl-amino)-nicotinamide

5N Hydrochloric acid (1.5mL) and ethanol (10mL) were added to (5-((5-benzyl-thiophen-2-ylmethyl)-carbamoyl)-pyridin-2-yl)-carbamoylmethylcarbamic acid *tert*-butyl ester described in Preparation Example A+-4 (23mg, 0.047mmol), and the solution was stirred for 10 minutes at 80°C. An aqueous solution of sodium bicarbonate and ethyl acetate were added to the reaction solution, which was then partitioned, the organic layer was washed with water twice and dried over anhydrous magnesium sulfate. The solvent was evaporated, and the title compound (12mg, 0.031 mmol, 67.1 %) was obtained as a brown oily substance.
¹H-NMR Spectrum (DMSO-d₆) δ (ppm) : 4.00-4.07 (4H, m), 4.46 (2H, d, J=5.6Hz), 6.55 (1H, d, J=8.8Hz), 6.66-6.69 (1H, m), 6.76 (1H, d, J=3.6Hz), 7.15-7.30 (5H, m), 7.46 (1 H, t, J=6.0Hz), 7.80 (1 H, dd, J=2.4, 8.8Hz), 8.44 (1 H, d, J=2.4Hz), 8.74 (1 H, t, J=5.6Hz).

### Reference Example A-153. 6-(Ethoxymethyl-amino)-N-(5-phenoxy-thiophen-2-ylmethyl)-nicotinamide

To a solution of 6-amino-N-(5-phenoxy-thiophen-2-ylmethyl)-nicotinamide described in Reference Example A-138 (600mg,1.8mmol) in ethanol (40mL) were added 5,5-dimethylimidazo phospho-2,4-dione (260mg,2.0mmol) and 37% formaldehyde aqueous solution (3mL), and the solution was stirred under reflux for 30 minutes. Water and ethyl acetate were added to the reaction solution, which was then partitioned, and the organic layer was washed with water twice. NH silica gel was added to the organic layer, the solvent was evaporated *in vacuo* for adsorption, purification was carried out by NH silica gel column chromatography (ethyl acetate), and the title compound (457mg, 1.19mmol, 66.1 %) was obtained as a white solid.
¹H-NMR Spectrum (DMSO-d₆) δ (ppm) :1.05 (3H, t, J=7.2Hz), 3.42 (2H, q, J=7.2Hz), 4.47 (2H, d, J=5.6Hz), 4.73 (2H, d, J=6.8Hz), 6.44-6.50 (1H, m), 6.55 (1H, d, J=8.8Hz), 6.74 (1 H, d, J=3.6Hz), 7.03-7.15 (3H, m), 7.30-7.40 (2H, m), 7.83-7.92 (2H, m), 8.53 (1H, d, J=2.0Hz), 8.85 (1 H, t, J=5.6Hz).

### Reference Example A-154. 6-(Ethoxymethyl-amino)-N-(5-(4-fluoro-phenoxy)-thiophen-2-ylmethyl)-nicotinamide

The title compound (120mg,0.30mmo1,51.7%) was obtained as a white solid from (6-amino-N-(5-(4-fluoro-phenoxy)-thiophen-2-ylmethyl)-nicotinamide described in Reference Example A-139 (200mg,0.58mmol) according to an analogous method to Reference Example A-153.
¹H-NMR Spectrum (DMSO-d₆) δ (ppm) :1.03-1.10 (3H, m), 3.40-3.46 (2H, m), 4.46 (2H, d, J=5.6Hz), 4.73(2H, d, J=6.8Hz), 6.44-6.49 (1 H, m), 6.55 (1 H, d, J=8.8Hz), 6.73 (1 H, d, J=2.8Hz), 7.08-7.16 (2H, m), 7.16-7.24 (2H, m), 7.83-7.90 (2H, m), 8.52 (1 H, s), 8.84 (1 H, t, J=5.6Hz).

### Reference Example A-155. 6-(Ethoxymethyl-amino)-N-(5-m-tolyloxy-thiophen-2-ylmethyl)-nicotinamide

The title compound (84mg, 2.05mmol, 31.5%) was obtained as a white solid from 6-amino-N-(5-m-tolyloxy-thiophen-2-ylmethyl)-nicotinamide described in Reference Example A-141 (220mg, 0.65mmol) according to an analogous method to Reference Example A-153.
¹H-NMR Spectrum (DMSO-d₆) δ (ppm) :1.03-1.10 (3H, m), 2.25 (3H, s), 3.40-3.46 (2H, m), 4.46 (2H, d, J=5.6Hz), 4.73 (2H, d, J=6.8Hz), 6.44-6.47 (1H, m), 6.54 (1H, d, J=8.8Hz), 6.73 (1H, d, J=4.0Hz), 6.83-6.96 (3H, m), 7.22 (1H, dd, J=8.0, 8.0Hz), 7.84-7.91 (2H, m), 8.52 (1H, d, J=2.4Hz), 8.84 (1H, t, J=5.6Hz).

### Reference Example A-156. N-(5-(3-Fluoro-phenoxy)-thiophen-2-ylmethyl)-6-(2-methoxy-ethylamino)-nicotinamide

To a solution of (5-((5-(3-fluoro-phenoxy)-thiophen-2-ylmethyl)-carbamoyl)-pyridin-2-yl)-carbamic acid *tert*-butyl ester described in Preparation Example A+-2 (100mg, 0.227mmol) and methoxyethyl bromide (38mg, 0.272mmol) in dimethylsulfoxide (5mL) was added sodium hydride (11mg, 0.272mmol, 60% in oil), and the solution was stirred at room temperature for 1 hour. 5N Hydrochloric acid was added to the reaction solution, which was then stirred for 5 minutes at 80°C. Water and ethyl acetate were added to the reaction solution, which was then partitioned, the organic layer was washed with water twice, then, silica gel was added, the solvent was evaporated *in vacuo* for adsorption, purification was carried out by silica gel column chromatography (hexane : ethyl acetate = 1 : 1, then ethyl acetate), and the title compound (39mg, 0.097mmol, 42.8%) was obtained as a white solid.
¹H-NMR Spectrum (DMSO-d₆) δ (ppm) : 3.24 (3H, s), 3.41-3.46 (4H, m), 4.47 (2H, d, J=5.6Hz), 6.48 (1 H, d, J=9.2Hz), 6.54-6.58 (1H, m), 6.76 (1 H, d, J=2.8Hz); 6.88-7.00 (3H, m), 7.12 (1 H, s), 7.36-7.44 (1 H, m), 7.77 (1H, d, J=9.2Hz), 8.48 (1 H, s), 8.77 (1 H, t, J=5.6Hz).

### Reference Example A-157. 6-Methoxymethyl-N-(5-phenoxy-thiophen-2-ylmethyl)-nicotinamide

The title compound (56mg, 0.158mmol, 43.9 %) was obtained as a light brown solid from 6-methoxymethylnicotinic acid (60mg, 0.36mmol) and C-(5-phenoxy-thiophen-2-yl)methylamine described in Preparation Example 26 (64mg, 0.36mmol) according to an analogous technique to Reference Example Q-6.
¹H-NMR Spectrum (DMSO-d₆) δ (ppm) : 3.38 (3H, s), 4.53-4.57 (4H, m), 6.52 (1 H, d, J=3.6Hz), 6.81 (1 H, d, J=3.6Hz), 7.07-7.17 (3H, m), 7.35-7.42 (2H, m), 7.50 (1 H, d, J=8.0Hz), 8.22 (1 H, dd, J=2.0, 8.0Hz), 8.95 (1 H, d, J=2.0Hz), 9.31 (1 H, t, J=5.6Hz).

### Reference Example A-158. N-(5-(3-Chloro-benzyl)-thiophen-2-ylmethyl)-6-(methoxymethyl-amino)-nicotinamide

To a solution of 6-amino-N-(5-(3-chloro-benzyl)-thiophen-2-ylmethyl)-nicotinamide described in Reference Example A-145 (110mg, 0.3mmol) in methanol (10mL) were added 5,5-dimethylimidazo phospho-2,4-dione (43mg,0.33mmol) and an aqueous solution of 37% formaldehyde (0.5mL), and the solution was stirred under reflux for 2 hours. NH silica gel was added to the reaction solution, the solvent was evaporated *in vacuo* for adsorption, purification was carried out by NH silica gel column chromatography (hexane : ethyl acetate = 1 : 1, then ethyl acetate), and the title compound (65mg, 0.16mmol, 54%) was obtained as a slightly yellow solid.
¹H-NMR Spectrum (DMSO-d₆) δ (ppm) : 3.16 (3H, s), 4.06 (2H, s), 4.48 (2H, d, J=6.0Hz), 4.68 (2H, d, J=6.8Hz), 6.54 (1 H, d, J=8.4Hz), 6.69-6.73 (1 H, m), 6.76-6.80 (1 H, m), 7.17-7.34 (4H, m), 7.84-7.92 (2H, m), 8.51 (1 H, s), 8.81 (1 H, t, J=6.0Hz).

### Example A-159. N-(5-benzylthiophen-2-ylmethyl)-6-(methoxymethyl-amino)-nicotinamide

To a solution of 6-amino-N-(5-benzylthiophen-2-ylmethyl)-nicotinamide described in Reference Example A-144 (210mg, 0.65mmol) and 5,5-dimethylimidazo phospho-2,4-dione (92mg,0.71 mmol) in methanol (15mL) was added an aqueous solution of 37% formaldehyde (2.5mL) under reflux in three time, and the solution was stirred for 1 hour. NH silica gel was added to the reaction solution, the solvent was evaporated *in vacuo* for adsorption, purification was carried out by NH silica gel column chromatography (hexane : ethyl acetate = 1 : 1, then ethyl acetate), and the title compound (210mg,0.57mmo1,87.6%) was obtained as a white solid.
¹H-NMR Spectrum (DMSO-d₆) δ (ppm) : 3.14-3.18 (3H, m), 4.03 (2H, s), 4.47 (2H, d, J=4.8Hz), 4.68 (2H, d, J=7.2Hz), 6.54 (1 H, d, J=8.8Hz), 6.68 (1 H, s), 6.76 (1 H, s), 7.14-7.30 (5H, m), 7.84-7.92 (2H, m), 8.51 (1 H, s), 8.79 (1 H, t, J=4.8Hz).

### Reference Example A-160. N-(5-(3-Fluorophenoxy)thiophen-2-ylmethyl)-6-methylnicotinamide

The title compound (53mg, 0.154mmol, 43.0%) was obtained as a white solid from 6-methylnicotinic acid (49mg, 0.36mmol) and C-(5-(3-fluorophenoxy)thiophen-2-yl)methylamine described in Preparation Example 23 (100mg, 0.36mmol) according to an analogous method to Reference Example Q-6.
¹H-NMR Spectrum (DMSO-d₆) δ (ppm) : 2.51 (3H, s), 4.55 (2H, d, J=6.4Hz), 6.58-6.61 (1 H, m), 6.82-6.85 (1 H, m), 6.92-7.03 (3H, m), 7.34-7.46 (2H, m), 8.10 (1 H, dd, J=2.0, 8.0Hz), 8.90 (1 H, d, J=2.0Hz), 9.25 (1 H, t, J=5.6Hz).

### Reference Example A-161. 6-Methyl-N-(5-phenoxy-thiophen-2-ylmethyl)-nicotinamide

The title compound (31 mg, 0.095mmol, 32.9 %) was obtained as a white solid from 6-methylnicotinic acid (40mg, 0.29mmol) and C-(5-phenoxy-thiophen-2-yl)-methylamine described in Preparation Example 26 (49mg, 0.24mmol) according to an analogous method to Reference Example Q-6.
¹H-NMR Spectrum (DMSO-d₆) δ (ppm) : 2.49 (3H, s), 4.51 (2H, d, J=5.6Hz), 6.49 (1H, d, J=4.0Hz), 6.78 (1H, d, J=4.0Hz), 7.05-7.15 (3H, m), 7.32-7.40 (3H, m), 8.07 (1 H, dd, J=2.0, 8.0Hz), 8.87 (1 H, d, J=2.0Hz), 9.21 (1 H, t, J=5.6Hz).

### Reference Example A-162 N-(5-(3-Chloro-benzyl)-thiophen-2-ylmethyl)-6-methylnicotinamide

The title compound (32mg,0.089mmol,26.4%) was obtained as a white solid from C-(5-(3-chloro-benzyl-2-yl)-methylamine described in Preparation Example 45 (80mg,0.34mmol) and 6-methylnicotinic acid (46mg,0.34mmol) according to an analogous method to Reference Example Q-6.
¹H-NMR Spectrum (DMSO-d₆) δ (ppm) : 2.50 (3H, s), 4.08 (2H, s), 4.54 (2H, d, 5.6Hz), 6.75 (1 H, d, J=3.6Hz), 6.84 (1H, d, J=3.6Hz), 7.20-7.36 (5H, m), 8.08 (1 H, dd, J=2.0, 8.0Hz), 8.88 (1 H, d, J=2.0Hz), 9.18 (1 H, t, J=5.6Hz).

### Reference Example A-163. 6-Methylamino-N-(5-phenoxy-thiophen-2-ylmethyl)-nicotinamide

To a solution of 6-(ethoxymethyl-amino)-N-(5-phenoxy-thiophen-2-ylmethyl)-nicotinamide described in Reference Example A-153 (427mg, 0.92mmol) in dimethylsulfoxide (5mL) was added sodium borohydride (100mg, 2.7mmol), and the solution was stirred for 15 minutes at 100°C. Water and ethyl acetate were added to the reaction solution, which was then partitioned, and the organic layer was washed with water three times. Silica gel was added to the organic layer, the solvent was evaporated *in vacuo* for adsorption, and purified by silica gel column chromatography (ethyl acetate). The solvent was evaporated *in vacuo,* hexane, diethyl ether and ethyl acetate were added to the residue for solidification, and the title compound (150mg, 0.44mmol, 48.1 %) was obtained as a white solid.
¹H-NMR Spectrum (DMSO-d₆) δ (ppm) : 2.78 (3H, d, J=4.8Hz), 4.46 (2H, d, J=5.6Hz), 6.40 (1H, d, J=8.8Hz), 6.47 (1H, d, J=3.6Hz), 6.73 (1H, d, J=3.6Hz), 6.98-7.15 (4H, m), 7.32-7.40 (2H, m), 7.79 (1H, dd, J=2.0, 8.8Hz), 8.50 (1H, d, J=2.0Hz), 8.75 (1 H, t, J=5.6Hz).

### Reference Example A-164. N-(5-(4-Fluoro-phenoxy)-thiophen-2-ylmethyl)-6-methylamino-nicotinamide

The title compound (120mg, 0.30mmol, 51.7%) was obtained as a white solid from 6-(ethoxymethyl-amino)-N-(5-(4-fluoro-phenoxy)-thiophen-2-ylmethyl)-nicotinamide described in Example A-154 according to an analogous method to Reference Example A-163.
¹H-NMR Spectrum (DMSO-d₆) δ (ppm) : 2.76-2.81 (3H, m), 4.45 (2H, d, J=5.6Hz), 6.40 (1H, d, J=8.4Hz), 6.44-6.48 (1H, m), 6.70-6.75 (1H, m), 6.98-7.06 (1H, m), 7.08-7.24 (4H, m), 7.79 (1 H, d, J=8.4Hz), 8.50 (1 H, s), 8.75 (1 H, t, J=5.6Hz).

### Reference Example A-165. N-(5-(3-Fluoro-phenoxy)-thiophen-2-ylmethyl)-6-methylamino-nicotinamide

Ethanol (20mL) and 5N hydrochloric acid (0.8mL) were added to (5-((5-(3-fluoro-phenoxy)-thiophen-2-ylmethyl)carbamoyl)pyridin-2-yl)methylcarbamic acid *tert*-butyl ester described in Preparation Example A+-3 (87mg,0.19mmol), and the solution was stirred for 25 minutes at 80°C. An aqueous solution of sodium bicarbonate and ethyl acetate were added to the reaction solution, which was then partitioned, the organic layer was washed with water twice, washed with brine, and the organic layer was dried over anhydrous magnesium sulfate. The solvent was evaporated, and the title compound (58mg, 0.162mmol, 85.5%) was obtained as a white solid.
¹H-NMR Spectrum (DMSO-d₆) δ (ppm) : 2.78 (3H, d, J=4.8Hz), 4.47 (2H, d, J=5.6Hz), 6.40 (1H, d, J=8.0Hz), 6.55 (1H, d, J=3.6Hz), 6.76 (1H, d, J=3.6Hz), 6.86-7.06 (4H, m), 7.36-7.44 (1H, m), 7.79 (1H, dd, J=2.0, 8.0Hz), 8.50 (1H, d, J=2.0Hz), 8.77 (1 H, t, J=5.6Hz).

### Reference Example A-166. N-(5-Benzyl-thiophen-2-ylmethyl)-6-methylaminonicotinamide

To a solution of N-(5-benzyl-thiophen-2-ylmethyl)-6-(methoxymethyl-amino)-nicotinamide described in Reference Example A-159 (183mg, 0.52mmol) in dimethylsulfoxide (3mL) was added sodium borohydride (120mg, 3.12mmol), and the solution was stirred at 140°C for 5 hours. Water and ethyl acetate were added to the reaction solution, which was then partitioned, and the organic layer was washed with water twice. Silica gel was added to the organic layer, the solvent was evaporated *in vacuo* for adsorption, purification was carried out by silica gel column chromatography (ethyl acetate, then ethyl acetate : methanol = 10 : 1), and the title compound (68mg, 0.20mmol, 38.8%) was obtained as a green solid.
¹H-NMR Spectrum (DMSO-d₆) δ (ppm) : 2.77 (3H, d, J=4.8Hz), 4.03 (2H, s), 4.46 (2H, d, J=5.6Hz), 6.39 (1 H, d, J=8.8Hz), 6.68 (1 H, d, J=3.6Hz), 6.76 (1 H, d, J=3.6Hz), 6.96-7.04 (1 H, m), 7.15-7.32 (5H, m), 7.77 (1 H, dd, J=2.0, 8.8Hz), 8.49 (1 H, d, J=2.0Hz), 8.70 (1 H, t, J=5.6Hz).

### Reference Example A-167. 6-Methylamino-N-(5-m-tolyloxy-thiophen-2-ylmethyl)-nicotinamide

To a solution of 6-(ethoxymethyl-amino)-N-(5-*m*-tolyloxy-thiophen-2-ylmethyl)-nicotinamide described in Reference Example A-155 (70mg, 0.17mmol) in dimethylsulfoxide (5mL) was added sodium borohydride (20mg, 0.51mmol), and the solution was stirred for 20 minutes at 120°C. Water and ethyl acetate were added to the reaction solution, which was then partitioned, the organic layer was washed with brine once. The organic layer was passed through a glass filter lined with NH silica gel, and eluted thoroughly with ethyl acetate. The solvent was evaporated *in vacuo,* and the title compound (53mg, 0.15mmol, 88.2%) was obtained as a light brown solid.
¹H-NMR Spectrum (Dmso-d₆) δ (ppm) : 2.25 (3H, s), 2.77 (3H, d, J=4.4Hz), 4.45 (2H, d, J=5.6Hz), 6.40 (1 H, d, J=8.8Hz), 6.45 (1 H, d, J=3.6Hz), 6.72 (1 H, d, J=3.6Hz), 6.83-6.96 (3H, m), 7.02 (1H, q, J=4.4Hz), 7.22 (1H, dd, J=8.0, 8.0Hz), 7.78 (1H, dd, J=2.4, 8.8Hz), 8.50 (1 H, d, J=2.4Hz), 8.75 (1 H, t, J=5.6Hz).

### Reference Example A-168. 2-Amino-N-(5-phenoxy-thiophen-2-ylmethyl)-6-vinylnicotinamide

To a mixture of 2-amino-6-chloro-N-(5-phenoxy-thiophen-2-ylmethyl)-nicotinamide described in Reference Example A-101 (30mg, 0.083mmol), tetrakis(triphenylphosphine)palladium(0) (19mg, 0.017mmol) and xylene (1.5mL) was added vinyl(tri-butyl)tin (0.073mL, 0.25mmol), and the solution was stirred at 130°C for 3 hours. After cooling, water and ethyl acetate were added to the reaction solution for extraction, which was then washed with brine. The solvent was evaporated *in vacuo,* then, the residue was purified by NH silica gel column chromatography (hexane : ethyl acetate = 2 : 1), and the title compound (19mg, 0.054mmol, 65%) was obtained as a white solid.
¹H-NMR Spectrum (CDCl₃) δ (ppm) : 4.65 (2H, d, J=5.5Hz), 5.49 (1H, dd, J=1.4, 10.7Hz), 6.22 (1H, dd, J=1.4, 17.3Hz), 6.27 (1H, brs), 6.36 (2H, brs), 6.39 (1H, d, J=3.8Hz), 6.60-6.67 (2H, m), 6.74 (1 H, d, J=3.7Hz), 7.08-7.13 (3H, m), 7.30-7.35 (2H, m), 7.56 (1 H, d, J=8.1 Hz).

### Reference Example A-169. 2-Amino-N-(5-bromo-4-phenoxy-thiophen-2-ylmethyl)-nicotinamide

The title compound was obtained from C-(5-bromo-4-phenoxy-thiophen-2-yl)-methylamine described in Preparation Example 129 (500mg, 1.76mmol) and 2-amino-nicotinic acid (267mg, 1.94mmol) according to an analogous method to Reference Example A-26. Trifluoroacetic acid salt of the title compound was obtained by reverse phase high performance liquid chromatography (acetonitrile-water mobile phase (containing 0.1% trifluoroacetic acid) was used).
MS m/e (ESI) 405.94(MH⁺)

### Reference Example A-170. 2-Amino-N-(5-methyl-4-phenoxy-thiophen-2-ylmethyl)-nicotinamide

To a mixture of 2-amino-N-(5-bromo-4-phenoxy-thiophen-2-ylmethyl)-nicotinamide described in Reference Example A-169 (30mg, 0.074mmol), dichloro(1,1'-bis(diphenylphosphino)ferrocene) nickel(II) (10mg, 0.015mmol) and tetrahydrofuran (1mL) was added methylmagnesium bromide (638µl, 0.592mmol) at room temperature, the solution was stirred for 2 hours at room temperature, and was further stirred for 2 hours at 50°C. After cooling, water and ethyl acetate were added for extraction, and the solution was washed with brine, then, the solvent was evaporated *in vacuo.* The residue was purified by reverse phase high performance liquid chromatography (acetonitrile-water mobile phase (containing 0.1% trifluoroacetic acid) was used), and the title compound was obtained as a trifluoroacetic acid salt.
MS m/e (ESI) 340.09(MH⁺)

### Reference Example A-171. 2-Amino-N-(4-chloro-5-phenoxy-thiophen-2-ylmethyl)-nicotinamide

To a mixture of 2-amino-N-(5-phenoxy-thiophen-2-ylmethyl)-nicotinamide described in Reference Example A-67 (21 mg, 0.064mmol) and N,N-dimethylformamide (1mL) was added N-chlorosuccinimide (13mg, 0.096mmol), and the solution was stirred overnight at room temperature. The reaction solution was purified by reverse phase high performance liquid chromatography (acetonitrile-water mobile phase (containing 0.1% trifluoroacetic acid) was used), and trifluoroacetic acid salt of the title compound (7.4mg, 0.016mmol, 24%) was obtained. MS m/e (ESI) 360.1 (MH⁺)

### Reference Example A-172. 2-Amino-5-chloro-N-(4-chloro-5-phenoxy-thiophen-2-ylmethyl)-nicotinamide

Trifluoroacetic acid salt of the title compound (1.5mg, 0.0030mmol, 4.6%) was obtained as a by-product of Reference Example A-171.
MS m/e (ESI) 394.0 (MH⁺)

### Reference Example A-173. 2,6-Diamino-N-(4-(3-chloro-benzyloxy)-benzyl)-nicotinamide

MS m/e (ESI) 383.246 (MH⁺)

### Reference Example A-174. 2,6-Diamino-N-(5-benzyloxy-pyridin-2-ylmethyl)-nicotinamide

¹H-NMR Spectrum (DMSO-d₆) δ (ppm) : 4.39(2H, d, J=5.9Hz), 5.16(2H, s), 5.68(1H, d, J=8.6Hz), 6.09(2H, s), 6.94(2H, s), 7.20(1H, d, J=8.8Hz), 7.31-7.46(6H, m), 7.69(1 H, d, J=8.6Hz), 8.25(1H, d, J=2.9Hz), 8.35(1 H, t, J=5.9Hz).

### Reference Example A-175. 2,6-Diamino-N-(5-phenoxymethyl-pyridin-2-ylmethyl)-nicotinamide

¹H-NMR Spectrum (CDCl₃) δ (ppm) : 4.64(2H, s), 4.68(2H, d, J=4.9Hz), 5.07(2H, s), 5.80(1H, d, J=8.4Hz), 6.50(2H, s), 6.96-7.01(3H, m), 7.26-7.35(4H, m), 7.56(1H, d, J=8.4Hz), 7.76(1H, dd, J=2.2Hz, 8.0Hz), 8.61(1H, d, J=1.7Hz).

### Reference Example A-176. 2-Amino-6-(2-cyano-ethyl)-N-(4-(6-fluoro-pyridin-2-yloxymethyl)-benzyl))-nicotinamide

¹H-NMR Spectrum (CDCl₃) δ (ppm) : 2.78(2H, t, J=7.2Hz), 2.94(2H, t, J=7.2Hz), 4.60(2H, d, J=5.6Hz), 5.33(2H, s), 6.24(1H, brs), 6.39(2H, brs), 6.47-6.48(1H, m), 6.49-6.50(1H, m), 6.64-6.67(1H, m), 7.35(2H, d, J=8.4Hz), 7.46(2H, d, J=8.4Hz), 7.53(1H, d, J=8.0Hz), 7.66(1H, q, J=8.0Hz).

### Reference Example A-177. 2-Amino-6-(2-fluoro-ethyl)-N-(5-phenoxy-thiophen-2-ylmethyl)-nicotinamide

¹H-NMR Spectrum (DMSO-d₆) δ (ppm) : 2.93 (2H, td, J=6.0, 25.6 Hz), 4.47 (2H, d, J=5.6 Hz), 4.75 (2H, td, J=6.0, 47.2 Hz), 6.50 (1 H, d, J=4.0 Hz), 6.53 (1 H, d, J=8.0 Hz), 6.77 (1 H, d, J=4.0 Hz), 7.07-7.18 (5H, m), 7.38 (2H, t, J=8.0 Hz), 7.85 (1 H, d, J=8.0 Hz), 8.99 (1 H, t, J=5.6 Hz).

### Reference Example A-178. 2-Amino-6-ethoxymethyl-N-(4-(pyridin-2-yloxymethyl)-benzyl)-nicotinamide

¹H-NMR Spectrum (CDCl₃) δ (ppm) :1.27(3H, t, J=7.0Hz), 3.60(2H, q, J=7.2Hz), 4.44(2H, s), 4.60(2H, d, 5.6Hz), 5.38(2H, s), 6.24(1 H, brs), 6.38(2H, brs), 6.73(1 H, d, J=8.0Hz), 6.80(1H, dd, J=0.8, 7.6Hz), 6.88-6.91(1H, m), 7.36(2H, d, J=8.0Hz), 7.46(2H, d, J=8.0Hz), 7.56-7.61 (2H, m), 8.17-8.18(1 H, m).

### Reference Example A-179. 2-Amino-N-(5-isopropoxymethyl-thiophen-2-ylmethyl)-6-methoxymethyl-nicotinamide

MS m/e (ESI) 350 (MH⁺)

### Reference Example A-180. 2-Amino-N-(4-(3-methoxy-benzyloxy)-benzyl)-6-methoxymethyl-nicotinamide

MS m/e (ESI) 408.27 (MH⁺)
¹H-NMR Spectrum (CD₃OD) δ (ppm) : 3.50(3H, s), 3.77(3H, s), 4.48(2H, d, J=4Hz), 4.57(2H, s), 5.04(2H, s), 6.84-6.88(2H, m), 6.94-6.98(4H, m), 7.23-7.29(3H, m), 8.27-8.29(1 H, m).

### Reference Example A-181. 2-Amino-N-(4-(3-chloro-benzyloxy)-benzyl)-6-methoxymethyl-nicotinamide

MS m/e (ESI) 412.26(MH⁺)

### Reference Example A-182. 2-Amino-N-(4-butoxymethyl-benzyl)-6-methoxymethyl-nicotinamide

MS m/e (ESI) 358 (MH⁺)

### Reference Example A-183. 2-Amino-6-methoxymethyl-N-(4-propoxymethyl-benzyl)-nicotinamide

MS m/e (ESI) 344 (MH⁺)

### Reference Example A-184. 2-Amino-N-(3-cyclopropylmethoxy-benzyl)-6-methoxymethyl-nicotinamide

MS m/e (ESI) 342 (MH⁺)

### Reference Example A-185. 2-Amino-N-(4-(6-fluoro-Ryridin-2-yloxymethyl)-benzyl)-6-methoxymethyl-nicotinamide

¹H-NMR Spectrum (CDCl₃) δ (ppm) : 3.46(3H, s), 4.40(2H, s), 4.61(2H, d, J=5.6Hz), 5.33(2H, s), 6.26(1H, brs), 6.40(2H, s), 6.49(1H, dd, J=2.4, 7.6Hz), 6.66(1H, dd, J=1.1, 7.6Hz), 6.70(1H, d, J=8.0Hz), 7.36(2H, d, J=8.0Hz), 7.46(2H, d, J=8.0Hz), 7.60(1 H, d, J=8.0Hz), 7.66(1 H, dd, J=8.0, 8.0Hz).

### Reference Example A-186. 2-Amino-6-methoxymethyl-N-(4-(4-methyl-pyridin-2-yloxymethyl)-benzyl)-nicotinamide

¹H-NMR Spectrum (CDCl₃) δ (ppm) : 2.30(3H, s), 3.45(3H, s), 4.39(2H, s), 4.60(2H, d, J=5.6Hz), 5.36(2H, s), 6.25(1H, brs), 6.40(2H, brs), 6.62(1H, s), 6.65(1H, d, J=8.0Hz), 6.72(1 H, d, J=5.2Hz), 7.35(2H, d, J=8.4Hz), 7.45(2H, d, J=8.4Hz), 7.59(1 H, d, J=8.0Hz), 8.02(1 H, d, J=5.2Hz).

### Reference Example A-187. 2-Amino-N-(4-(6-fluoro-pyridin-2-ylmethoxy)-benzyl)-6-methoxymethyl-nicotinamide

¹H-NMR Spectrum (CDCl₃) δ (ppm) : 3.45(3H, s), 4.39(2H, s), 4.53(2H, d, J=5.6Hz), 5.13(2H, s), 6.22(1H, brs), 6.39(2H, brs), 6.69(1H, d, J=7.6Hz), 6.87(1H, dd, J=2.8, 8.4Hz), 6.96(2H, d, J=8.4Hz), 7.28(2H, d, J=8.4Hz), 7.40-7.42(1H, m), 7.58(1H, d, J=8.0Hz), 7.81 (1 H, dd, J=8.0, 8.0Hz).

### Reference Example A-188. 2-Amino-N-(4-butoxy-benzyl)-6-methoxymethyl-nicotinamide

MS m/e (ESI) 344 (MH⁺)

### Reference Example A-189. 2-Amino-N-(4-(2-ethoxy-ethyl)-benzyl))-6-methoxymethyl-nicotinamide

MS m/e (ESI) 344 (MH⁺)

### Reference Example A-190. 2-Amino-6-methoxymethyl-N-(4-(5-methyl-pyridin-2-yloxymethyl)-benzyl)-nicotinamide

¹H-NMR Spectrum (CDCl₃) δ (ppm) : 2.25(3H, s), 3.45(3H, s),4.40(2H, s), 4.60(2H, d, J=5.6Hz), 5.34(2H, s), 6.24(1 H, brs), 6.40(2H, brs), 6.69(1H, d, J=8.0Hz), 6.71 (1 H, d, J=8.0Hz), 7.35(2H, d, J=8.0Hz), 7.41(1H, dd, J=2.4, 8.4Hz), 7.46(2H, d, J=8.0Hz), 7,59(1 H, d, J=8.4Hz), 7.96(1 H, s).

### Reference Example A-191. 2-Amino-N-(6-benzyl-pyridin-3-ylmethyl)-6-methoxymethyl-nicotinamide

¹H-NMR Spectrum (CDCl₃) δ (ppm) : 3.45(3H, s), 4.15(2H, s), 4.39(2H, s), 4.57(2H, d, J=5.6Hz), 6.32(1H, brs), 6.38(2H, brs), 6.69(1 H, d, J=8.0Hz), 7.12(1 H, d, J=8.4Hz), 7.20-7.32(5H, m), 7.58(7.61(2H, m), 8.52(1 H, d, J=2.0Hz).

### Reference Example A-192. 2-Amino-6-methoxymethyl-N-(5-phenoxymethyl-pyridin-2-ylmethyl)-nicotinamide

¹H-NMR Spectrum (CDCl₃) δ (ppm) : 3:46(3H, s), 4.41 (2H, s), 4.72(2H, d, J=4.8Hz), 5.09(2H, s), 6.45(2H, s), 6.74(1H, d, J=7.9Hz), 6.96-7.02(3H, m), 7.29-7.36(3H, m), 7.53(1 H, s), 7.79(2H, d, J=7.9Hz), 8.63(1 H, d, J=1.7Hz).

### Reference Example A-193. 2-Amino-6-methoxymethyl-N-(5-phenoxy-pyridin-2-ylmethyl)-nicotinamide

¹H-NMR Spectrum (DMSO-d₆) δ (ppm) : 3.34 (3H, s), 4.30 (2H, s), 4.52 (2H, d, J=6.0 Hz), 6.62 (1H, d, J=8.0 Hz), 7.02-7.07 (2H, m), 7.10-7.20 (3H, m), 7.33-7.46 (4H, m), 8.02 (1 H, d, J=8.0 Hz), 8.31 (1 H, d, J=2.8 Hz), 9.02 (1 H, t, J=6.0 Hz).

### Reference Example A-194. 2-Amino-6-methoxymethyl-N-(4-(2-propoxy-ethyl)-benzyl)-nicotinamide

MS m/e (ESI) 358 (MH⁺)

### Reference Example AA-1. 3-Amino-pyrazine-2-carboxylic acid (5-phenoxy-thiophen-2-ylmethyl)-amide

¹H-NMR Spectrum (DMSO-d₆) δ (ppm) : 4.47 (2H, d, J=6.4 Hz), 6.47 (1H, d, J=4.0 Hz), 6.74 (1 H, d, J=4.0 Hz), 7.05-7.15 (3H, m), 7.24-7.40 (2H, m), 7.50 (2H, brs), 7.80 (1 H, d, J=2.4 Hz), 8.18 (1 H, d, J=2.4 Hz), 9.28 (1 H, t, J=6.4 Hz).

### Reference Example AA-2. 3,5-Diamino-pyrazine-2-carboxylic acid 4-(pyridin-2-yloxymethyl)-benzylamide

¹H-NMR Spectrum (DMSO-d₆) δ (ppm) : 4.38(2H, d, J=6.4Hz), 5.3(2H, s), 6.67(2H, s), 6.84(1H, d, J=8.2Hz), 6.98(1H, t, J=7.2Hz), 7.13(1H, s), 7.28(2H, d, J=8.1 Hz), 7.38(2H, d, J=8.1 Hz), 7.71(1H, dt, J=2.0Hz, 7.8Hz), 8.16(1H, dd, J=2.0Hz, 4.8Hz), 8.54(1 H, t, J=6.4Hz).

### Reference Example B-1. 4-Amino-pyrimidine-5-carboxylic acid (5-phenoxy-thiophen-2-ylmethyl)-amide

The title compound (7mg, 0.021 mmol, 4.4 %) was obtained as a white solid from 4-amino-pyrimidine-5-carboxylic acid (68mg, 0.49mmol) and C-(5-phenoxy-thiophen-2-yl)-methylamine described in Preparation Example 26 (100mg, 0.49mmol) according to an analogous technique to Reference Example Q-6. ¹H-NMR Spectrum (DMSO-d₆) δ (ppm) : 4.47 (2H, d, J=5.2Hz), 6.47-6.50 (1 H, m), 6.76-6.78 (1 H, m), 7.04-7.15 (3H, m), 7.34-7.40 (2H, m), 7.77 (2H, brs), 8.40 (1 H, d, J=1.6Hz), 8.59 (1 H, d, J=1.6Hz), 9.19 (1 H, t, J=5.2Hz).

### Reference Example B-2. 4-Amino-2-propylamino-pyrimidine-5-carboxylic acid 4-(pyridin-2-yloxymethyl)-benzylamide

¹H-NMR Spectrum (DMSO-d₆) δ (ppm) : 0.86 (3H, t, J=7.2 Hz), 1.44-1.55 (2H, m), 3.14-3.24 (2H, m), 4.38 (2H, d, J=6.4 Hz), 5.32 (2H, s), 6.83-6.87 (1 H, m), 6.96-7.01 (1 H, m), 7.28 (2H, d, J=8.0 Hz), 7.38 (2H, d, J=8.0 Hz), 7.69-7.75 (1 H, m), 8.15-8.19 (1 H, m), 8.42 (1 H, s), 8.60 (1 H, brs).

### Reference Example C-1. (2-Amino-6,7-dihydro-5H-cyclopenta[b]pyridine-3-carboxylic acid (5-phenoxy-thiophen-2-ylmethyl)-amide

2-Amino-6,7-dihydro-5*H*-cyclopenta[b]pyridine-3-carboxylic acid described in Preparation Example C-5 (100mg, 0.56mmol), triethylamine (0.188mL, 1.35mmol) and benzotriazol-1-yloxytris(dimethylamino)phosphonium hexafluorophosphate (298mg, 0.67mmol) were dissolved in N,N-dimethylformamide (3mL), C-(5-phenoxy-thiophen-2-yl)-methylamine described in Preparation Example 26 (138mg, 0.67mmol) was added, followed by stirring for 15 hours 20 minutes at room temperature. After the reaction was completed, the reaction solution was poured into brine, and the solution was extracted with ethyl acetate. The organic layer was dried over anhydrous magnesium sulfate, then concentrated, the obtained residue was purified by silica gel column chromatography (ethyl acetate : hexane = 1:1), and the title compound (77mg, 0.21 mmol, 38%) was obtained.
¹H-NMR Spectrum (DMSO-d₆) δ (ppm) :1.95-2.03 (2H, m), 2.68-2.76 (4H, m), 4.46 (2H, d, J=5.6Hz), 6.45 (1 H, d, J=4.0Hz), 6.76 (1 H, d, J=4.0Hz), 6.97 (2H, brs), 7.07-7.17(3H, m), 7.36-7.41 (2H, m), 7.76 (1 H, s), 8.88-8.93 (1 H, m).

### Reference Example D-1. [1,5]Naphthylidine-2-carboxylic acid 3-phenoxybenzylamide

The title compound (14mg, 76%) was obtained as a colorless oil from [1,5]naphthylidine-2-carboxylic acid described in Preparation Example D-1 (9mg, 0.0517mmol) and 3-phenoxybenzylamine described in Preparation Example 4 (6mg, 0.0517mmol) according to an analogous method to Reference Example L-4.
¹H-NMR Spectrum (CDCl₃) δ (ppm) : 4.73(2H, d, J=6.0Hz), 6.92-6.94(1H, m), 7.01-7.03(2H, m), 7.07-7.17(3H, m), 7.26-7.36(3H, m), 7.70(1H, dd, J=4.4, 8.8Hz), 8.39(1 H, dd, J=1.6, 8.8Hz), 8.51 (1 H, brs), 8.57(2H, s), 9.06(1 H, dd, J=1.6, 4.4Hz).

### Reference Example D-2. [1,5]Naphthylidine-2-carboxylic acid 4-benzyloxybenzylamide

The title compound (11mg, 52%) was obtained as a white solid from [1,5]naphthylidine-2-carboxylic acid described in Preparation Example D-1 (10mg, 0.0574mmol) and 4-benzyloxybenzylamine described in Preparation Example 1 (12mg, 0.0574mmol) according to an analogous method to Reference Example L-4
¹H-NMR Spectrum (CDCl₃) δ(ppm) : 4.68(2H, d, J=6.4Hz), 5.08(2H, s), 6.97-6.99(2H, m), 7.32-7.45(7H, m), 7.69(1 H, dd, J=4.4, 8.8Hz), 8.36-8.39(1 H, m), 8.43(1 H, brs), 8.54-8.60(2H, m), 9.04-9.06(1 H, m).

### Reference Example D-3. [1.5]Naphthylidine-2-carboxylic acid 4-phenoxymethyl-benzylamide

¹H-NMR Spectrum (CDCl₃) 6 (ppm) : 4.76(2H, d, J=6Hz), 5.07(2H, s), 6.94-6.99(3H, m), 7.27-7.31 (3H, m), 7.43-7.45(4H, m), 7.68-7.71 (1 H, m), 8.37-8.40(1 H, m), 8.50(1 H, brs), 8.55-8.60(1 H, m), 9.05-9.06(1 H, m).

### Reference Example D-4. [1,5]Naphthylidine-2-carboxylic acid (1-(3-fluoro-benzyl)-1H-pyrrol-3-ylmethyl)-amide

To a solution of C-(1-(3-fluoro-benzyl)-1*H*-pyrrol-3-yl)-methylamine described in Preparation Example 59 (59mg, 0.287mmol) and [1,5]naphthylidine-2-carboxylic acid described in Preparation Example D-1 (50mg, 0.287mmol) in N,N-dimethylformamide (3mL) were added benzotriazol-1-yloxytris(dimethylamino)phosphonium hexafluorophosphate (152mg, 0.344mmol) and triethylamine (80µL, 0.574mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and the title compound (49mg, 47%) was obtained as a pale yellow solid.
¹H-NMR Spectrum (CDCl₃) δ (ppm) : 4.59(2H, d, J=5.6Hz), 5.03(2H, s), 6.26-6.27(1H, m), 6.66-6.67(1H, m), 6.74-6.75(1H, m), 6.81-6.84(1H, m), 6.92-6.94(1H, m), 6 96-7.01 (1H, m), 7.28-7.33(1H, m), 7.67-7.70(1H, m), 8.31 (1H, brs), 8.37-8.40(1 H, m), 8.53-8.59(2H, m), 9.03-9.05(1 H, m).

### Reference Example D-5. [1,5]Naphthylidine-2-carboxylic acid (5-(3-fluorophenoxy)thiophen-2-ylmethyl) amide

The title compound (66mg, 0.17mmol, 72.5%) was obtained as a brown oil from [1,5]-naphthylidine-2-carboxylic acid (42mg, 0.24mmol) and C-(5-(3-fluorophenoxy)thiophen-2-yl)methylamine described in Preparation Example 23 (54mg,0.24mmol) according to an analogous method to Reference Example Q-6.
¹H-NMR Spectrum (DMSO-d₆) δ (ppm) : 4.61 (2H, d, J=6.4Hz), 6.57 (1H, d, J=3.6Hz), 6.83 (1H, d, J=3.6Hz), 6.87-7.00 (3H, m), 7.36-7.42 (1H, m), 7.87 (1H, dd, J=4.0, 8.8Hz), 8.37 (1H, d, J=8.8Hz), 8.49 (1H, d, J=8.8Hz), 8.59 (1 H, d, J=8.8Hz), 9.09 (1 H, d, J=4.0Hz), 9.58 (1 H, t, J=6.4Hz).

### Reference Example D-6. [1,5]Naphthylidine-2-carboxylic acid (5-phenoxy-thiophen-2-ylmethyl)-amide

The title compound (15mg, 0.042mmol, 17.3 %) was obtained as a light brown solid from [1,5]-naphthylidine-2-carboxylic acid (42mg,0.24mmol) and C-(5-phenoxy-thiophen-2-yl)methylamine described in Preparation Example 26 (49mg,0.24mmol) according to an analogous technique to Reference Example Q-6.
¹H-NMR Spectrum (DMSO-d₆) δ (ppm) : 4.59 (2H, d, J=5.6Hz), 6.49 (1H, d, J=3.6Hz), 6.81 (1H, d, J=3.6Hz), 7.03-7.14 (3H, m), 7.30-7.40 (2H, m), 7.88 (1 H, dd, J=4.0, 8.8Hz), 8.36 (1H, d, J=8.8Hz), 8.48 (1H, d, J=8.8Hz), 8.58 (1H, d, J=8.8Hz), 9.09 (1 H, d, J=4.0Hz), 9.56 (1 H, t, J=5.6Hz).

### Reference Example D-7. [1,5]Naphthylidine-2-carboxylic acid (5-(4-fluoro-phenoxy)-thiophen-2-ylmethyl)-amide

To a solution of [1,5]naphthylidine-2-carboxylic acid described in Preparation Example D-1 (20mg, 0.115mmol) and described in Preparation Example 28C-(5-(4-fluorophenoxy)thiophen-2-yl)methylamine) (51 mg, 0.23mmol) in dimethylsulfoxide (9mL) were added benzotriazol-1-yloxytris(dimethylamino)phosphonium hexafluorophosphate (102mg,0.23mmol) and triethylamine (56µL, 0.43mmol), and the solution was stirred at 60°C for 30 minutes. Ethyl acetate and water were added to the reaction solution, which was then partitioned, the organic layer was sequentially washed with an aqueous solution of saturated sodium bicarbonate, water and brine, and then, dried over anhydrous sodium sulfate. The solvent was evaporated, the residue was sequentially purified by silica gel column chromatography (hexane : ethyl acetate), silica gel column chromatography (dichloromethane : ethyl acetate), reverse phase high performance liquid chromatography (acetonitrile-water mobile phase (containing 0.1% trifluoroacetic acid) was used), and the title compound (8.6mg, 0.017mmol, 15%) was obtained.
MS m/e (ESI) 379.76(MH⁺)
¹H-NMR Spectrum (CD₃OD) δ (ppm) : 4.71(2H, d, J=6.4Hz), 6.38(1H, d, J=4.0Hz), 6.83 (1H, d, J=4.0Hz), 7.02-7.09 (4H, m), 7.85 (1H, dd, J=4.0, 8.8Hz), 8.46 (1H, d, J=8.8Hz), 8.56-8.61 (2H, m), 9.04(1 H, dd, J=1.6, 4.0Hz), 9.55-9.64 (1 H, m).

### Reference Example D-8. [1,5]Naphthylidine-2-carboxylic acid (5-(3-chloro-phenoxy)-thiophen-2-ylmethyl)-amide

MS m/e (ESI) 396.28 (MH⁺)

### Reference Example D-9. [1,5]Naphthylidine-2-carboxylic acid (5-benzofuran-2-ylmethyl-thiophen-2-ylmethyl)-amide

MS m/e (ESI) 400.51 (MH⁺)

### Reference Example D-10. [1,5]Naphthylidine-2-carboxylic acid 4-(6-fluoro-pyridin-2-yloxymethyl)-benzylamide

¹H-NMR Spectrum (CDCl₃) δ (ppm) : 4.75(2H, d, J=6.0Hz), 5.34(2H, s), 6.47-6.49(1H, m), 6.64-6.66(1H, m), 7.43-7.48(4H, m), 7.62-7.70(2H, m), 8.36-8.39(1 H, m), 8.50(1 H, brs), 8.55-8.60(2H, m), 9.04-9.06(1 H, m).

### Reference Example E-1. Quinoline-6-carboxylic acid (5-benzyl-furan-2-yl)-amide

The title compound (200mg, 5.8mmol, 54.6%) was obtained as a slightly yellow solid from 5-benzylfuran-2-carbaldehyde described in Preparation Example 39 and 6-quinolinecarboxylic acid according to an analogous method to Reference Example Q-1.
¹H-NMR Spectrum (DMSO-d₆) δ (ppm) : 3.92 (2H, s), 4.45 (2H, d, J=5.6Hz), 6.02 (1H, d, J=2.8Hz), 6.20 (1H, d, J=2.8Hz), 7.16-7.30 (5H, m), 7.59 (1H, dd, J=4.4, 8.0Hz), 8.05 (1 H, d, J=8.8Hz), 8.16 (1 H, dd, J=2.0, 8.8Hz), 8.44 (1 H, dd, J=1.6, 8.0Hz), 8.50 (1 H, d, J=2.0Hz), 8.97 (1 H, dd, J=1.6, 4.4Hz), 9.16 (1 H, t, J=5.6Hz).

### Reference Example E-2. Quinoline-6-carboxylic acid (5-phenoxy-furan-2-ylmethyl)-amide

The title compound (68mg, 0.197mmol, 17.2%) was obtained as a white solid from quinoline-6-carboxylic acid (200mg, 1.15mmol) and C-(5-phenoxy-furan-2-yl)-methylamine described in Preparation Example 49 (200mg, 1.12mmol) according to an analogous method to Reference Reference Example Q-6.
¹H-NMR Spectrum (DMSO-d₆) δ (ppm) : 4.45 (2H, d, J=5.6Hz), 5.72 (1H, d, J=3.2Hz), 6.34 (1 H, d, J=3.2Hz), 7.02-7.08 (2H, m), 7.11-7.17 (1 H, m), 7.14-7.40 (2H, m), 7.60 (1 H, dd, J=2.0, 8.0Hz), 8.06 (1 H, d, J=8.8Hz), 8.16 (1 H, d, J=8.8Hz), 8.45 (1 H, d, J=8.0Hz), 8.51 (1 H, s), 8.97 (1 H, d, J=4.0Hz), 9.18 (1 H, t, J=5.6Hz).

### Reference Example E-3. Quinoline-6-carboxylic acid (5-(3-fluoro-phenoxy)-furan-2-ylmethyl)-amide

To a solution of (5-(3-fluoro-phenoxy)-furan-2-yl)-methanol described in Preparation Example 51 (1.5g, 7.2mmol), phthalimide (1.1g, 7.2mmol) and triphenyl phosphine (1.9g, 7.2mmol) in tetrahydrofuran (10mL) was added diethyl azodicarboxylate (3.5g, 7.9mmol) dropwise at 0°C, and the solution was stirred at room temperature for 30 minutes. Silica gel was added to the reaction solution, the solvent was evaporated *in vacuo* for adsorption, purification was carried out by silica gel column chromatography (hexane : ethyl acetate = 2 : 1), and a white solid (700mg) was obtained. Ethanol (10mL) and hydrazine monohydrate (0.3mL) were added to this solid (700mg), and stirred for 15 minutes at 90°C. The solution was allowed to room temperature, the solid was removed by filtration, and a pale yellow oil (360mg) containing C-(5-(3-fluoro-phenoxy)-furan-2-yl)-methylamine was obtained. The title compound (17mg, 0.046mmol, 2.7%) was obtained as a brown solid from this oil (360mg) and quinoline-6-carboxylic acid (300mg, 1.7mmol) according to an analogous method to Example Q-6.
¹H-NMR Spectrum (DMSO-d₆) δ (ppm) : 4.46 (2H, d, J=5.2Hz), 5.83 (1H, d, J=3.2Hz), 6.36(1H, d, J=3.2Hz), 6.87-7.03 (3H, m), 7.38-7.45 (1H, m), 7.60 (1H, d, J=4.0, 8.4Hz), 8.06 (1 H, d, J=8.8Hz), 8.16 (1 H, dd, J=2.0, 8.8Hz), 8.45 (1 H, dd, J=1.6, 8.4Hz), 8.51 (1 H, d, J=2.0Hz), 8.97(1 H, dd, J=1.6, 4.0Hz), 9.19(1 H, t, J=5.2Hz).

### Reference Example E-4. Quinoline-6-carboxylic acid (5-phenyl-furan-2-ylmethyl)-amide

To a solution of quinoline-6-carboxylic acid (5-bromo-furan-2-ylmethyl)-amide described in Preparation Example E+-2 (200mg,0.60mmol) in 1,4-dioxane (5mL) were added phenylboronic acid (150mg, 1.2mmol), tetrakis(triphenylphosphine)palladium(0) (55mg, 0.047mmol) and an aqueous solution of potassium carbonate (2mol), and the solution was stirred at 110°C for 2 hours. Water and ethyl acetate were added to the reaction solution, which was then partitioned, NH silica gel was added to the organic layer, the solvent was evaporated *in vacuo* for adsorption, purification was carried out by NH silica gel column chromatography (hexane : ethyl acetate = 2 : 1), and the title compound (65mg, 0.198mmol, 33.0%) was obtained as a white solid.
¹H-NMR Spectrum (DMSO-d₆) δ (ppm) : 4.59 (2H, d, J=6.0Hz), 6.44 (1H, d, J=3.2Hz), 6.88 (1 H, d, J=3.2Hz), 7.23-7.28 (1 H, m), 7.37-7.42 (2H, m), 7.60 (1 H, dd, J=4.0, 8.4Hz), 7.64-7.70 (2H, m), 8.07 (1 H, d, J=8.8Hz), 8.20 (1 H, dd, J=2.0, 8.8Hz), 8.45-8.50 (1H, m), 8.55 (1H, d, J=2.0Hz), 8.97 (1H, dd, J=1.6, 4.0Hz), 9.26 (1H, t, J=6.0Hz).

### Reference Example E-5. Quinoline-6-carboxylic acid (5-(2,4-difluoro-phenoxy)-furan-2-ylmethyl)-amide

The title compound (71mg, 0.187mmol, 18%) was obtained from quinoline-6-carboxylic acid (180mg, 1.04mmol) and C-(5-(2,4-difluoro-phenoxy)-furan-2-yl)-methylamine described in Preparation Example 77 (258mg, 1.15mmol) according to an analogous method to Reference Example H-1.
¹H-NMR Spectrum (DMSO-d₆) δ (ppm) : 4.44 (2H, d, J=5.6Hz), 5.59 (1 H, d, J=3.6Hz), 6.31 (1 H, d, J=3.6Hz), 7.06-7.13 (1 H, m), 7.27-7.34 (1 H, m), 7.44-7.51 (1 H, m), 7.59(1H, dd, J=4.0, 8.8Hz), 8.06 (1H, d, J=9.2 Hz), 8.16 (1H, dd, J=1.2, 8.8Hz), 8.45 (1H, d, J=9.2Hz), 8.51 (1H, s), 8.97 (1H, dd, J=1.2, 4.0Hz), 9.19 (1H, t, J=5.6Hz).

### Reference Example E-6. Quinoline-6-carboxylic acid (5-(2,5-difluoro-phenoxy)-furan-2-ylmethyl)-amide

The title compound (194mg, 0.51mmol, 32%) was obtained from quinoline-6-carboxylic acid (275mg, 1.59mmol) and C-(5-(2,5-difluoro-phenoxy)-furan-2-yl)-methylamine described in Preparation Example 79 (357mg, 1.59mmol) according to an analogous method to Reference Example H-1.
¹H-NMR Spectrum (DMSO-d₆) δ (ppm) : 4.46 (2H, d, J=5.2Hz), 5.78 (1H, d, J=3.2Hz), 6.35 (1 H, d, J=3.2Hz), 7.04-7.18 (2H, m), 7.42-7.50 (1 H, m), 7.60 (1 H, dd, J=4.0, 8.0Hz), 8.06 (1H, d, J=8.8Hz), 8.17 (1H, dd, J=1.2, 8.0Hz), 8.45 (1H, d, J=8.8Hz), 8.52(1H, s), 8.97 (1 H, dd, J=1.2, 4.0Hz), 9.19 (1 H, t, J=5.2Hz).

### Reference Example E-7. Quinoline-6-carboxylic acid (5-(3-fluoro-benzyl)-furan-2-ylmethyl)-amide

Title compound (301 mg, 0.835mmol, 80%) was obtained from quinoline-6-carboxylic acid (188mg, 1.16mmol) and C-(5-(3-fluoro-benzyl)-furan-2-yl)-methylamine described in Preparation Example 84 (279mg, 1.36mmol) according to an analogous method to Reference Example H-1.
¹H-NMR Spectrum (DMSO-d₆) δ (ppm) : 3.96 (2H, s), 4.46 (2H, d, J=5.6Hz), 6.07 (1 H, d, J=3.2Hz), 6.21 (1 H, d, J=3.2Hz), 7.00-7.09 (3H, m), 7.28-7.35 (1 H, m), 7.59 (1 H, dd, J=4.4, 8.4Hz), 8.05 (1 H, d, J=8.8Hz), 8.16 (1 H, d, J=2.0, 8.4Hz), 8.44 (1 H, dd, J=1.2, 8.8Hz), 8.50 (1 H, d, J=1.2Hz), 8.97 (1 H, dd, J=2.0, 4.4Hz), 9.17 (1 H, t, J=5.6Hz).

### Reference Example E-8. Quinoline-6-carboxylic acid 4-benzyloxybenzylamide

To a solution of 6-quinoline carboxylic acid (100mg, 0.577mmol) in tetrahydrofuran (50mL) was added N,N'-dicyclohexylcarbodiimide (1.90g, 11.7mmol), and the solution was stirred at room temperature for 1 hour. Then, to this solution was added a solution of 4-benzyloxybenzylamine described in Preparation Example 1 (2.49g, 11.7mmol) in tetrahydrofuran (5mL), and the solution was stirred overnight at room temperature. The solvent was evaporated, the residue was purified by NH silica gel column chromatography (hexane : ethyl acetate), and the title compound (4.31 g, quantitatively) was obtained as a white solid.
¹H-NMR Spectrum (CDCl₃) δ (ppm) : 4.65 (2H, d, J=5.6Hz), 5.08(2H, s), 6.48(1H, brs), 6.97-7.00(2H, m), 7.31-7.35(3H, m), 7.37-7.45(4H, m), 7.47(1H, dd, J=4.4, 8.4Hz), 8.05(1 H, dd, J=2.0, 8.8Hz), 8.15(1 H, d, J=8.8Hz), 8.22-8.25(1 H, m), 8.32(1 H, d, J=2.0Hz), 8.98-9.00(1H, m).

### Reference Example E-9. Quinoline-6-carboxylic acid 3-benzyloxybenzylamide

The title compound (102mg, 48%) was obtained as a colorless oil from 6-quinolinecarboxylic acid (100mg, 0.58mmol) and 3-benzyloxybenzylamine described in Preparation Example 2 (126mg, 0.58mmol) according to an analogous method to Reference Example E-8.
¹H-NMR Spectrum (CDCl₃) δ (ppm) : 4.69 (2H, d, J=5.6Hz), 5.07(2H, s), 6.57(1H, brs), 6.92-6.95(1H, m), 6.98-7.02(2H, m), 7.27-7.32(2H, m), 7.34-7.38(2H, m), 7.41-7.43(2H, m), 7.47(1H, dd, J=4.4, 8.4Hz), 8.05(1H, dd, J=2.0, 8.8Hz), 8.16(1H, d, J=8.8Hz), 8.22-8.25(1 H, m), 8.32(1 H, d, J=2.0Hz), 8.98-9.00(1H, m).

### Reference Example E-10. Quinoline-6-carboxylic acid 4-phenoxybenzylamide

The title compound (63mg, 31%) was obtained as a colorless solid from 6-quinolinecarboxylic acid (100mg, 0.58mmol) and 4-phenoxybenzylamine described in Preparation Example 3 (115mg, 0.58mmol) according to an analogous method to Reference Example E-8.
¹H-NMR Spectrum (CDCl₃) δ (ppm) : 4.69 (2H, d, J=5.6Hz), 6.59(1H, brs), 7.00-7.03(4H, m), 7.10-7.14(1H, m), 7.32-7.38(4H, m), 7.46-7.49(1H, m), 8.05-8.08(1 H, m), 8.15-8.17(1 H, m), 8.23-8.25(1 H, m), 8.34(1 H, s), 8.98-9.00(1 H, m).

### Reference Example E-11. Quinoline-6-carboxylic acid 3-phenoxybenzylamide

The title compound (140mg, 69%) was obtained as a colorless oil from 6-quinolinecarboxylic acid (100mg, 0.58mmol) and 4-phenoxybenzylamine described in Preparation Example 3 (115mg, 0.58mmol) according to an analogous method to Reference Example E-8.
¹H-NMR Spectrum (CDCl₃) δ (ppm) : 4.69 (2H, d, J=6.0Hz), 6.63(1H, brs), 6.92-6.95(1 H, m), 7.01-7.05(3H, m), 7.09-7.14(2H, m), 7.30-7.36(3H, m), 7.47(1H, dd, J=4.0, 8.0Hz), 8.05(1H, dd, J=2.0, 8.8Hz), 8.15(1H, d, J=8.8Hz), 8.21-8.24(1H, m), 8.32(1H, d, J=2.0Hz), 8.98-8.99(1H, m).

### Reference Example E-12. Quinoline-6-carboxylic acid 4-(pyridin-2-ylmethoxy)-benzylamide

To a solution of quinoline-6-carboxylic acid 4-hydroxybenzylamide described in Preparation Example E+-1 (20mg, 0.0719mmol) and 2-chloromethyl-pyridine hydrochloride (12mg, 0.0719mmol) in N,N-dimethyl formamide (1.0mL) was added potassium carbonate (298mg, 2.16mmol), and the solution was stirred at room temperature for 12 hours. Ethyl acetate and water were added to the reaction solution, which was then partitioned, and the organic layer was dried over anhydrous magnesium sulfate. The solvent was evaporated, the residue was purified by reverse phase high performance liquid chromatography (acetonitrile-water mobile phase (containing 0.1% trifluoroacetic acid) was used), and trifluoroacetic acid salt of the title compound was obtained.
MS m/e (ESI) 369.2 (MH⁺)
¹H-NMR Spectrum (CD₃OD) δ (ppm) ; 4.60(2H, s), 5.37(2H, s), 7:35(2H, d, J=8.8Hz), 7.40(2H, d, J=8.8Hz), 7.74-7.77(1H, m), 7.82-7.85(1H, m), 7.92-7.94(1H, m), 8.18-8.20(1H, m), 8.29-8.35(2H, m), 8.59-8.60(1H, m), 8.70-8.71 (1H, m), 8.79-8.81 (1 H, m), 9.08-9.09(1 H, m).

### Reference Example E-13. Quinoline-6-carboxylic acid (biphenyl-3-ylmethyl)-amide

The title compound (20mg, 21%) was obtained as a colorless oil from 6-quinolinecarboxylic acid (50mg, 0.289mmol) and C-biphenyl-3-yl-methylamine described in Preparation Example 5 (48mg, 0.263mmol) according to an analogous method to Reference Example E-8.
¹H-NMR Spectrum (CDCl₃) δ (ppm) : 4.79(2H, d, J=5.6Hz), 6.62(1H, brs), 7.34-7.49(6H, m), 7.55-7.62(4H, m), 8.08(1H, dd, J=2.0, 8.8Hz), 8.16(1H, d, J=8.8Hz), 8.23-8.25(1 H, m), 8.35(1 H, d, J=2.0Hz), 8.98-9.00(1 H, m).

### Reference Example E-14. Quinoline-6-carboxylic acid 4-(3-methyl-benzyloxy)-benzylamide

The title compound (2.5mg, 18%) was obtained as a white solid from quinoline-6-carboxylic acid 4-hydroxybenzylamide obtained in Preparation Example E+-1 (10mg, 0.0359mmol) and 3-methylbenzyl chloride (5mg, 0.0359mmol) according to an analogous method to Reference Example E-12.
¹H-NMR Spectrum (CDCl₃) δ (ppm) : 2.38(3H, s), 4.66(2H, d, J=5.2Hz), 5.04(2H, s), 6.50(1H, brs), 6.97-7.00(2H, m), 7.14-7.16(1H, m), 7.22-7.35(5H, m), 7.46-7.50(1H, m), 8.06(1H, dd, J=2.0, 8.8Hz), 8.17(1H, d, J=8.8Hz), 8.23-8.26(1H, m), 8.33(1H, d, J=2.0Hz), 8.99-9.00(1 H, m).

### Reference Example E-15. Quinoline-6-carboxylic acid 3-(4-fluoro-phenoxy)-benzylamide

The title compound (28mg, 25%) was obtained as a colorless oil from 6-quinolinecarboxylic acid (52mg, 0.30mmol) and 3-(4-fluorophenoxy)-benzylamine described in Preparation Example 8 (65mg, 0.30mmol) according to an analogous method to Reference Example L-4.
¹H-NMR Spectrum (CDCl₃) δ (ppm) : 4.70(2H, d, J=5.6Hz), 6.64(1H, brs), 6.88-6.91 (1H, m), 6.99-7.06(5H, m), 7.12-7.14(1H, m), 7.31-7.35(1H, m), 7.47-7.50(1 H, m), 8.04-8.07(1 H, m), 8.15-8.17(1 H, m), 8.23-8.25(1 H, m), 8.33(1 H, m), 9.00-9.01 (1H, m).

### Reference Example E-16. Quinoline-6-carboxylic acid 3-(4-methoxy-phenoxy)-benzylamide

The title compound (28mg, 25%) was obtained as a colorless oil from 6-quinolinecarboxylic acid (50mg, 0.29mmol) and 3-(4-methoxyphenoxy) benzylamine described in Preparation Example 9 (66mg, 0.30mmol) according to an analogous method to Reference Example L-4.
¹H-NMR Spectrum (CDCl₃) δ (ppm) : 3.79(3H, s), 4.66(2H, d, J=5.6Hz), 6.55(1H, brs), 6.84-6.89(3H, m), 6.95-6.99(3H, m), 7.05-7.07(1H, m), 7.46(2H, dd, J=4.4, 8.4Hz), 8.03(1H, dd, J=2.4, 8.8Hz), 8.14(1H, d, J=8.8Hz), 8.21-8.23(1H, m), 8.29(1H, d, J=2.0Hz), 8.98(1 H, dd, J=2.0, 4.4Hz).

### Reference Example E-17. Quinoline-6-carboxylic acid 3-(3-trifluoromethyl-phenoxy)-benzylamide

The title compound (39mg, 32%) was obtained as a colorless oil from 6-quinolinecarboxylic acid (50mg, 0.29mmol) and 3-(3-trifluoromethyl-phenoxy)-benzylamine described in Preparation Example 10 (77mg, 0.29mmol) according to an analogous method to Reference Example L-4.
¹H-NMR Spectrum (CDCl₃) δ (ppm) : 4.70(2H, d, J=5.6Hz), 6.68(1H, brs), 6.93-6.95(1 H, m), 7.05-7.06(1 H, m), 7.14-7.25(3H, m), 7.31-7.47(4H, m), 8.04(1 H, dd, J=1.6, 8.8Hz), 8.13(1H, d, J=8.8Hz), 8.20-8.2(1H, m), 8.31 (1 H, d, J=2.0Hz), 8.97(1H, dd, J=1.6, 4.4Hz).

### Reference Example E-18. Quinoline-6-carboxylic acid 3-(3-fluoro-phenoxy)-benzylamide

The title compound (70mg, 65%) was obtained as a colorless oil from 6-quinolinecarboxylic acid (50mg, 0.29mmol) and 3-(3-fluoro-phenoxy)-benzylamine described in Preparation Example 11 (63mg, 0.29mmol) according to an analogous method to Reference Example L-4.
¹H-NMR Spectrum (CDCl₃) δ (ppm) : 4.71 (2H, d, J=6.0Hz), 6.63(1H, brs), 6.69-6.73(1H, m), 6.78-6.83(2H, m), 6.96-6.98(1H, m), 7.07(1H, s), 7.18(1H, d, J=7.6Hz), 7.24-7.30(1H, m), 7.34-7.38(1H, m), 7.48(1H, dd, J=4.4, 8.4Hz), 8.06(1H, dd, J=2.0, 8.8Hz), 8.16(1H, d, J=8.8Hz), 8.23-8.25(1H, m), 8.33(1H, d, J=1.6Hz), 8.99(1 H, dd, J=1.6, 4.4Hz).

### Reference Example E-19. 2-(3-Phenoxy-phenyl)-N-puinolin-6-yl-acetamide

The title compound (116mg, 94%) was obtained as a colorless oil from 6-aminoquinoline (50mg, 3.47mmol) and 3-phenoxyphenylacetic acid (79mg, 3.47mmol) according to an analogous method to Reference Example L-4.
¹H-NMR Spectrum (CDCl₃) δ (ppm) : 3.77(2H, s), 6.98-7.06(4H, m), 7.10-7.16(2H, m), 7.34-7.44(6H, m), 8.01 (1 H, d, J=8.8Hz), 8.09-8.16(1 H, m), 8.32(1 H, d, J=2.0Hz), 8.83(1H, dd, J=1.6, 4.0Hz).

### Reference Example E-20. Quinoline-6-carboxylic acid 4-(furan-2-ylmethoxy)-benzylamide

The title compound (0.7mg, 4%) was obtained as a colorless oil from 6-quinolinecarboxylic acid (10mg, 0.0577mmol) and 4-(furan-2-ylmethoxy)-benzylamine described in Preparation Example 12 (12mg, 0.0577mmol) according to an analogous method to Reference Example L-4.
¹H-NMR Spectrum (CDCl₃) δ (ppm) : 4.66(2H, d, J=5.6Hz), 5.02(2H, s), 6.48(1H, brs), 6.99-7.02(2H, m), 7.24-7.27(2H, m), 7.35-7.36(2H, m), 7.47-7.50(2H, m), 8.05-8.08(1 H, m), 8.16-8.18(1 H, m), 8.24-8.27(1 H, m), 8.33(1 H, s), 9.00(1 H, s).

### Reference Example E-21. Quinoline-6-carboxylic acid 4-(thiophen-2-ylmethoxy)-benzylamide

The title compound (11mg, 53%) was obtained as a colorless solid from 6-quinolinecarboxylic acid (10mg, 0.0577mmol) and 4-(thiophen-2-ylmethoxy)-benzylamine described in Preparation Example 13 (13mg, 0.0577mmol) according to an analogous method to Reference Example L-4.
¹H-NMR Spectrum (CDCl₃) δ (ppm) : 4.65(2H, d, J=5.6Hz), 5.23(2H, s), 6.60(1H, brs), 6.97-7.03(3H, m), 7.11-7.12(1H, m), 7.32-7.34(3H, m), 7.45-7.48(1H, m), 8.05-8.07(1H, m), 8.13-8.16(1 H, m), 8.21-8.23(1 H, m), 8.32(1H, s), 8.97-8.99(1 H, m).

### Reference Example E-22. Quinoline-6-carboxylic acid 4-(thiophen-3-ylmethoxy)-benzylamide

The title compound (78mg, 72%) was obtained as a white solid from 6-quinolinecarboxylic acid (50mg, 0.289mmol) and 4-(thiophen-3-ylmethyl)-benzylamine described in Preparation Example 14 (63mg, 0.289mmol) according to an analogous method to Reference Example L-4.
¹H-NMR Spectrum (CDCl₃) δ (ppm) : 4.65(2H, d, J=5.6Hz), 5.08(2H, s), 6.51 (1 H, brs), 6.96-6.99(2H, m), 7.14-7.16(1H, m), 7.32-7.36(4H, m), 7.46-7.49(1H, m), 8.04-8.07(1 H, m), 8.15(1H, d, J=8.8Hz), 8.22-8.24(1 H, m), 8.32(1 H, d, J=2.0Hz), 8.99(1 H, dd, J=2.0, 4.4Hz).

### Reference Example E-23. Quinoline-6-carboxylic acid 4-((S)-1-phenyl-ethoxy)-benzylamide

The title compound (219mg, 80%) was obtained as a colorless oil from 6-quinolinecarboxylic acid (123mg, 0.712mmol) and 4-((S)-1-phenyl-ethoxy)-benzylamine described in Preparation Example 15 (172mg, 0.712mmol) according to an analogous method to Reference Example L-4.
¹H-NMR Spectrum (CDCl₃) δ (ppm) :1.64(3H, d, J=6.4Hz), 4.58(2H, d, J=5.2Hz), 5.31(1H, q, J=6.4Hz), 6.47(1H, brs), 6.86(2H, d, J=8.8Hz), 7.22(2H, d, J=8.8Hz), 7.26-7.27(2H, m), 7.32-7.39(3H, m), 7.46(1 H, dd, J=4.0, 8.0Hz), 8.03(1H, dd, J=2.0, 8.8Hz), 8.13(1H, d, J=8.8Hz), 8.22(1H, d, J=8.0Hz), 8.30(1H, d, J=2.0Hz), 8.97-8.98(1 H, m).

### Reference Example E-24. Quinoline-6-carboxylic acid 3-benzylamino-benzylamide

3-Benzylamino-benzonitrile described in Preparation Example 87 (57mg, 0.27mmol) was dissolved at 0°C in tetrahydrofuran (0.5mL), and lithium aluminum hydride (52mg,1.35mmol) was added. After stirring overnight at room temperature, water (52µl), an aqueous solution of 5N sodium hydroxide (52µl) and water (156µl) were sequentially added at 0°C. The reaction solution was filtered through Celite pad, then, the solvent was evaporated *in vacuo,* and (3-aminomethyl-phenyl)-benzyl-amine (62mg, 0.29mmol) was obtained as an oil.
(3-Aminomethyl-phenyl)-benzylamine (62mg,0.29mmol), quinoline-6-carboxylic acid (52mg,0.30mmol), benzotriazol-1-yl-tris(dimethylamino)phosphonium hexafluorophosphate (182mg,0.41mmol) and triethylamine (114µl,0.81mmol) were dissolved in N,N-dimethylformamide (0.5mL), and the solution was stirred at room temperature for 2 hours. Water was added to the reaction solution, which was then extracted with ethyl acetate, the organic layer was washed with water and brine, and dried over anhydrous magnesium sulfate. The solvent was evaporated *in vacuo,* the residue was purified by NH silica gel column chromatography (hexane : ethyl acetate = 1 : 1), and the title compound (73mg,0.198mmol,73%) was obtained as a white solid.
¹H-NMR Spectrum (DMSO-d₆) δ (ppm) : 4.22 (2H, d, J=6.0Hz), 4.39 (2H, d, J=6.0Hz), 6.25 (1H, t, J=6.0Hz), 6.40 (1H, dd, J=1.6, 7.7Hz), 6.50 (1H, d, J=7.7Hz), 6.60 (1 H, s), 6.97 (1 H, t, J=7.8Hz), 7.14 (1 H, t, J=7.3Hz), 7.23 (2H, t, J=7.8Hz), 7.30 (2H, d, J=7.1Hz), 7.60 (1 H, dd, J=4.4, 8.4Hz), 8.07 (1 H, d, J=8.8Hz), 8.19 (1 H, dd, J=2.0, 8.8Hz), 8.45 (1H, d, J=7.6Hz), 8.52 (1H, d, J=1.6Hz), 8.97 (1H, dd, J=1.6, 4.4Hz), 9.14 (1 H, t, J=6.0Hz).

### Reference Example E-25. Quinoline-6-carboxylic acid 4-phenylamino-benzylamide

(4-Aminomethyl-phenyl)-phenylamine (98mg, 0.494mmol) was obtained as an oil from 4-phenylamino-benzonitrile described in Preparation Example 88 (110mg, 0.566mmol) according to an analogous method to Reference Example E-24.
Then, the title compound (52mg, 0.147mmol, 26%) was obtained from the resulting (4-aminomethyl-phenyl)-phenylamine and quinoline-6-carboxylic acid (108mg,0.623mmol).
¹H-NMR Spectrum (CDCl₃) δ (ppm) : 4.66 (2H, d, J=5.5Hz), 5.76 (1 H, s), 6.50 (1 H, brs), 6.96 (1 H, t, J=7.4Hz), 7.07-7.10 (4H, m), 7.29-7.32 (4H, m), 7.49 (1 H, dd, J=4.2, 8.4Hz), 8.08 (1H, dd, J=2.0, 8.6Hz), 8.17 (1H, d, J=8.8Hz), 8.25 (1H, d, J=7.5Hz), 8.34 (1 H, d, J=1.8Hz), 9.00 (1 H, dd, J=1.7, 4.2Hz).

### Reference Example E-26. Quinoline-6-carboxylic acid 4-(benzyl-methyl-amino)-benzylamide

Quinoline-6-carboxylic acid 4-benzylamino-benzylamide described in Preparation Example E+-3 (30mg, 82µmol), formalin (9µl, 115µmol), triacetoxy sodium borohydride (25mg,115µmol) and acetic acid (several drops) were suspended in tetrahydrofuran (1mL), and the solution was stirred at room temperature for 4 hours. An aqueous solution of saturated sodium bicarbonate was added to the reaction suspension at 0°C, which was then extracted with ethyl acetate, the organic layer was washed with brine and dried over anhydrous magnesium sulfate. The solvent was evaporated *in vacuo,* purification was carried out using thin layer NH silica gel chromatography (hexane : ethyl acetate = 1 : 1), and the title compound (11mg,28µmol,35%) was obtained as a white solid.
¹H-NMR Spectrum (CDCl₃) δ (ppm) : 3.05 (3H, s), 4.55 (2H, s), 4.59 (2H, d, J=5.1 Hz), 6.38 (1H, brs), 6.74 (2H, d, J=8.8Hz), 7.21-7.26 (4H, m), 7.32 (2H, t, J=7.2Hz), 7.4 (1H, d, J=7.0Hz), 7.47 (1H, dd, J=4.3, 8.2Hz), 8.05 (1H, dd, J=2.0, 8.8Hz), 8.14 (1H, d, J=8.8Hz), 8.23 (1H, d, J=7.1Hz), 8.30 (1H, d, J=1.7Hz), 8.98 (1H, dd, J=1.7, 4.2Hz).

### Reference Example E-27. Quinoline-6-carboxylic acid 3-phenylsulfanyl-benzylamide

The title compound (50mg, 0.14mmol, 75%) was obtained as a white solid from 3-phenylsulfanyl-benzylamine described in Preparation Example 95 (38mg, 0.18mmol) and quinoline-6-carboxylic acid (31 mg, 0.18mmol) according to an analogous method to Reference Example A-26.
¹H-NMR Spectrum (CDCl₃) δ (ppm) : 4.67 (2H, d, J=5.7Hz), 6.52 (1H, brs), 7.23-7.26 (3H, m), 7.31 (4H, t, J=7.4Hz), 7.38 (2H, d, J=6.8Hz), 7.49 (1H, dd, J=4.2, 8.4Hz), 8.03 (1H, dd, J=1.8, 8.8Hz), 8.16 (1H, d, J=8.8Hz), 8.24 (1H, d, J=7.5Hz), 8.31 (1H, d, J=1.8Hz), 9.00 (1H, dd, J=1.7, 4.2Hz).

### Reference Example E-28. Quinoline-6-carboxylic acid 4-benzylsulfanyl-benzylamide

The title compound (54mg, 0.14mmol, 38%) was obtained as a white solid from 4-benzylsulfanyl-benzylamine described in Preparation Example 101 (84mg, 0.37mmol) and quinoline-6-carboxylic acid (70mg, 0.40mmol) according to an analogous method to Reference Example A-26.
¹H-NMR Spectrum (DMSO-d₆) δ (ppm) : 4.20 (2H, s), 4.47 (2H, d, J=5.9Hz), 7.18-7.34 (9H, m), 7.59 (1 H, dd, J=4.2, 8.4Hz), 8.07 (1 H, d, J=9.0Hz), 8.18 (1H, dd, J=2.0, 8.8Hz), 8.45 (1H, d, J=7.9Hz), 8.52 (1H, d, J=2.0Hz), 8.97 (1H, dd, J=1.8, 4.2Hz), 9.24 (1 H, t, J=5.9Hz).

### Reference Example E-29. Quinoline-6-carboxylic acid 3-(3-methylbutoxy)-benzylamide

To a mixture solution of quinoline-6-carboxylic acid 3-hydroxybenzylamide described in Preparation Example E+-4 (13mg, 0.048mmol) and N,N-dimethylformamide (0.5mL) were added potassium carbonate (13mg,0.096mmol) and 1-iodine-3-methylbutane (0.013mL, 0.096mmol), and the solution was stirred overnight at room temperature. Water and dichloromethane was added to the reaction solution for extraction, which was then washed with brine, then, the solvent was evaporated *in vacuo.* The residue was purified by silica gel column chromatography (ethyl acetate), and the title compound (12mg, 0.033mmol, 69%) was obtained as a colorless oil.
¹H-NMR Spectrum (CDCl₃) δ (ppm) : 0.96 (6H, d, J=6.6Hz), 1.65-1.70 (2H, m), 1.80-1.87 (1H, m), 3.99 (2H, t, J=6.7Hz), 4.68 (2H, d, J=5.7Hz), 6.56 (1H, brs), 6.84-6.87 (1H, m), 6.93-6.98 (2H, m), 7.26-7.31 (1 H, m), 7.48 (1 H, dd, J=4.2, 8.2Hz), 8.07 (1 H, dd, J=2.0, 8.8Hz), 8.16 (1H, d, J=8.8Hz), 8.23-8.25 (1H, m), 8.33 (1 H, d, J=2.0Hz), 8.99 (1 H, dd, J=1.8, 4.2Hz).

### Reference Example E-30. Quinoline-6-carboxylic acid (Z)-4-styryl-benzylamide

To a mixture of quinoline-6-carboxylic acid 4-phenylethynyl-benzylamide described in Preparation Example E+-5 (48mg, 0.13mmol), quinoline (26mg, 0.20mmol) and tetrahydrofuran (2mL) was added Lindlar catalyst (5.0mg), and the solution was stirred under hydrogen atmosphere at room temperature for 1 hour. The interior of the reaction system was changed to nitrogen atmosphere, then, filtration was carried out through Celite pad. The filtrate was evaporated *in vacuo,* the residue was purified by silica gel column chromatography (hexane : ethyl acetate =1:4), and the title compound (45mg, 0.12mmol, 92%) was obtained as a colorless oil.
¹H-NMR Spectrum (DMSO-d₆) δ (ppm) : 4.57 (2H, d, J=6.0Hz), 6.58-6.60 (2H, m), 7.16-7.27 (9H, m), 7.59 (1H, dd, J=4.2, 8.2Hz), 8.06 (1H, d, J=8.8Hz), 8.19 (1H, dd, J=2.0, 8.8Hz), 8.45-8.47 (1 H, m), 8.54 (1 H, d, J=1,7Hz), 8.97 (1 H, dd, J=1.7, 4.2Hz), 9.24 (1 H, t, J=5.7Hz).

### Reference Example E-31. Quinoline-6-carboxylic acid 4-phenylaminomethyl-benzylamide

The title compound (13.8mg) was obtained according to an analogous method to Reference Example E-26 using quinoline-6-carboxylic acid 4-formyl-benzylamide described in Preparation Example E+-6 (50mg, 0.172mmol) instead of formalin, and phenylamine (31µl, 0.34mmol) instead of quinoline-6-carboxylic acid 4-benzyl amino-benzylamide. Trifluoroacetic acid salt of the title compound was obtained by reverse phase high performance liquid chromatography (acetonitrile-water mobile phase (containing 0.1% trifluoroacetic acid) was used).
MS m/e (ESI) 368.5(MH⁺)

### Reference Example E-32. Quinoline-6-carboxylic acid 4-((methyl-phenyl-amino)-methyl)-benzylamide

The title compound (4.25mg) was obtained from quinoline-6-carboxylic acid 4-phenylaminomethyl-benzylamide described in Reference Example E-31 (30mg, 82µmol) according to an analogous method to Reference Example E-26. Trifluoroacetic acid salt of the title compound was obtained by reverse phase high performance liquid chromatography (acetonitrile-water mobile phase (containing 0.1% trifluoroacetic acid) was used).
MS m/e (ESI) 382.3 (MH⁺)

### Reference Example E-33. Quinoline-6-carboxylic acid 3-(4-nitro phenoxy)-benzylamide

To a mixture solution of quinoline-6-carboxylic acid 3-hydroxybenzylamide described in Preparation Example E+-4 (3.0mg, 0.011mmol), copper(II) acetate (2.9mg, 0.016mmol), molecular sieves 4A (50mg) and dichloromethane (2mL) were added triethylamine (0.0077mL, 0.055mmol) and 4-nitrophenylboronic acid (1.8mg, 0.011 mmol), and the solution was stirred in the presence of air at room temperature for 10 days. The reaction solution was filtered, then, water, ethyl acetate and an aqueous solution of 29% ammonia were added for extraction, the solution was washed with brine, then, the solvent was evaporated *in vacuo.* The residue was purified by reverse phase high performance liquid chromatography (acetonitrile-water mobile phase (containing 0.1 % trifluoroacetic acid) was used), and trifluoroacetic acid salt of the title compound (1.14mg, 0.0022mmol, 20%) was obtained.
MS m/e (ESI) 400.2 (MH⁺)

### Reference Example E-34. Quinoline-6-carboxylic acid 3-(4-methanesulfonyl phenoxy)-benzylamide

Trifluoroacetic acid salt of the title compound (0.21 mg, 0.00038mmol, 3.5%) was obtained from quinoline-6-carboxylic acid 3-hydroxybenzylamide described in Preparation Example E+-4 (3.0mg, 0.011 mmol) and 4-methanesulfonylphenylboronic acid (2.2mg, 0.011mmol) according to an analogous method to Reference Example E-33.
MS m/e (ESI) 433.2 (MH⁺)

### Reference Example E-35. 4-(3-(((Quinoline-6-carbonyl)amino)methyl)phenoxy)benzoic acid methyl ester

Trifluoroacetic acid salt of the title compound (0.14mg, 0.00027mmol, 2.4%) was obtained from quinoline-6-carboxylic acid 3-hydroxybenzylamide described in Preparation Example E+-4 (3.0mg, 0.011mmol) and 4-methoxy-carbonyl phenylboronic acid (1.8mg, 0.011mmol) according to an analogous method to Reference Example E-33.
MS m/e (ESI) 413.3 (MH⁺)

### Reference Example E-36. Quinoline-6-carboxylic acid 3-(3-cyanoahenoxy)-benzylamide

Trifluoroacetic acid salt of the title compound (0.25mg, 0.00051mmol, 4.6%) was obtained from quinoline-6-carboxylic acid 3-hydroxybenzylamide described in Preparation Example E+-4 (5.0mg, 0.011mmol) and 3-cyanophenylboronic acid (2.6mg, 0.018mmol) according to an analogous method to Reference Example E-33.
MS m/e (ESI) 380.1 (MH⁺)

### Reference Example E-37. Quinoline-6-carboxylic acid 3-(3-acetylphenoxy)-benzylamide

Trifluoroacetic acid salt of the title compound (0.17mg, 0.00033mmol, 3.0%) was obtained from quinoline-6-carboxylic acid 3-hydroxybenzylamide described in Preparation Example E+-4 (5.0mg, 0.011mmol) and 3-acetylphenylboronic acid (3.0mg, 0.018mmol) according to an analogous method to Reference Example E-33.
MS m/e (ESI) 397.0 (MH⁺)

### Reference Example E-38. Quinoline-6-carboxylic acid 3-(3-trifluoromethoxy phenoxy)-benzylamide

Trifluoroacetic acid salt of the title compound (0.15mg, 0.00027mmol, 2.5%) was obtained from quinoline-6-carboxylic acid 3-hydroxybenzylamide described in Preparation Example E+-4 (5.0mg, 0.011mmol) and 3-trifluoromethoxyphenylboronic acid (3.7mg, 0.018mmol) according to an analogous method to Reference Example E-330.
MS m/e (ESI) 439.0 (MH⁺)

### Reference Example E-39. Quinoline-6-carboxylic acid (3'-fluorobiphenyl-3-ylmethyl)-amide

To a mixture of quinoline-6-carboxylic acid 3-bromobenzylamide described in Preparation Example E+-8 (4.0mg, 0.012mmol), toluene (1 mL) and methanol (0.25mL) were added an aqueous solution of 2M sodium carbonate (0.5mL), tetrakis(triphenylphosphine)palladium(0) (1.4mg,0.0012mmol) and 3-fluorophenylboronic acid (1.7mg, 0.012mmol), and the solution was stirred at 70°C for 4 hours. After cooling, water, ethyl acetate and acetic acid were added for extraction, the solution was washed with brine, then, the solvent was evaporated in *vacuo.* The residue was purified by reverse phase high performance liquid chromatography (acetonitrile-water mobile phase (containing 0.1 % trifluoroacetic acid) was used), and trifluoroacetic acid salt of the title compound (0.47mg, 0.0010mmol, 8.3%) was obtained.
MS m/e (ESI) 357.2 (MH⁺)

### Reference Example E-40. Quinoline-6-carboxylic acid 3-benzyl-benzylamide

To a mixture of quinoline-6-carboxylic acid 3-bromobenzylamide described in Preparation Example E+-8 (8.0mg, 0.023mmol), dichloro(1,1'-bis(diphenylphosphino)ferrocene) nickel(II) (3.2mg,0.0047mmol) and tetrahydrofuran (1mL) was added benzylmagnesium chloride (1.1M tetrahydrofuran solution, 0.088mL, 0.094mmol) at room temperature, and the solution was stirred for 30 minutes at 50°C. After cooling, water and ethyl acetate were added for extraction, the solution was washed with brine, the solvent was then evaporated *in vacuo.* The residue was purified by reverse phase high performance liquid chromatography (acetonitrile-water mobile phase (containing 0.1% trifluoroacetic acid) was used), and trifluoroacetic acid salt of the title compound (2.1mg, 0.0045mmol, 19%) was obtained.
MS m/e (ESI) 353.2 (MH⁺)

### Reference Example E-41. Quinoline-6-carboxylic acid 4-benzyl-benzylamide

Trifluoroacetic acid salt of the title compound (1.5mg, 0.0032mmol, 14%) was obtained from quinoline-6-carboxylic acid 4-bromobenzylamide described in Preparation Example E+-9 (8.0mg, 0.023mmol) according to an analogous method to Reference Example E-40.
MS m/e (ESI) 353.3 (MH⁺)

### Reference Example E-42. Quinoline-6-carboxylic acid 4-phenethyl-benzylamide

Trifluoroacetic acid salt of the title compound (0.39mg, 0.00081 mmol, 3.5%) was obtained from quinoline-6-carboxylic acid 4-bromobenzylamide described in Preparation Example E+-9 (8.0mg, 0.023mmol) and phenethylmagnesium chloride (1.0M tetrahydrofuran solution, 0.094mL, 0.094mmol) according to an analogous method to Reference Example E-40.
MS m/e (ESI) 367.3 (MH⁺)

### Reference Example E-43. Quinoline-6-carboxylic acid 3-cyclopropylmethoxy-benzylamide

To a mixture of quinoline-6-carboxylic acid 3-hydroxybenzylamide described in Preparation Example E+-4 (87mg, 0.31mmol) and tetrahydrofuran (2mL) was added an aqueous solution of 1 N sodium hydroxide (0.31 mL, 0.31mmol), and the solvent was evaporated *in vacuo.* To a mixture of a portion (5.0mg) of the residue (93mg) and N,N-dimethylformamide (1 mL) were added cyclopropylmethyl bromide (2.7mg, 0.020mmol) and catalytic amount of sodium iodide at room temperature, followed by stirring for 3 hours at the same temperature. The reaction solution was purified by reverse phase high performance liquid chromatography (acetonitrile-water mobile phase (containing 0.1% trifluoroacetic acid) was used), and trifluoroacetic acid salt of the title compound (1.50mg, 0.0034mmol, 20%) was obtained.
MS m/e (ESI) 333.0 (MH⁺)

### Reference Example E-44. N-(4-Benzyloxy-benzyl)-N'-methoxy-quinoline-6-carboxamidine

To a mixture of quinoline-6-carbothioic acid 4-benzyloxy-benzylamide described in Preparation Example E+-10 (57mg, 0.15mmol) and acetonitrile (3mL) was added 2-(bromomethyl)naphthalene (200mg, 0.94mmol), which was then refluxed for 2 hours. After cooling, the solvent was evaporated *in vacuo,* and the residue was washed with diethyl ether three times. A mixture of a portion (29mg) of the resulting crude product (57mg), methoxylamine hydrochloride (2.9mg, 0.035mmol), an aqueous solution of 1 N sodium hydroxide (0.035mL, 0.035mmol) and N-methylpyrrolidinone (1 mL) was stirred at room temperature for 25 minutes. The reaction solution was directly purified by reverse phase high performance liquid chromatography (acetonitrile-water mobile phase (containing 0.1 % trifluoroacetic acid) was used), and ditrifluoroacetic acid salt of the title compound (1.9mg, 0.0030mmol, 6.4%) was obtained.
MS m/e (ESI) 398.5 (MH⁺)

### Reference Example E-45. N-(4-Benzyloxy-benzyl)-N'-cyano-quinoline-6-carboxamidine

To a mixture of quinoline-6-carbothioic acid 4-benzyloxy-behzylamide described in Preparation Example E+-10 (57mg, 0.15mmol) and toluene (2mL) was added benzyl bromide (0.089mL, 0.74mmol), which was then stirred under reflux for 90 minutes. After cooling, the solvent was evaporated *in vacuo,* and the residue was washed with diethyl ether twice. A mixture of a portion (16mg) of the resulting crude product (72mg), cyanamide (20mg, 0.48mmol) and N-methylpyrrolidinone (1 mL) was stirred for 2.5 hours at 120°C. After cooling, the reaction solution was filtered through a membrane filter, the filtrate was directly purified by reverse phase high performance liquid chromatography (acetonitrile-water mobile phase (containing 0.1 % trifluoroacetic acid) was used), and trifluoroacetic acid salt of the title compound (0.76mg, 0.0015mmol, 4.5%) was obtained.
MS m/e (ESI) 393.5 (MH⁺)

### Reference Example E-46. Quinoline-6-carboxylic acid 4-(3-chloro-benzyloxy)-benzylamide

Trifluoroacetic acid salt of the title compound was obtained from quinoline-6-carboxylic acid 4-hydroxybenzylamine described in Preparation Example E+-1 and 3-chlorobenzyl chloride according to an analogous method to Reference Example E-12.
MS m/e (ESI) 403 (MH⁺)

### Reference Example E-47. Quinoline-6-carboxylic acid 4-(3-fluoro-benzyloxy)-benzylamide

Trifluoroacetic acid salt of the title compound was obtained from quinoline-6-carboxylic acid 4-hydroxybenzylamine described in Preparation Example E+-1 and 3-fluorobenzyl bromide according to an analogous method to Reference Example E-12.
MS m/e (ESI) 387 (MH⁺)

### Reference Example E-48. Quinoline-6-carboxylic acid 4-(benzo[1.3]dioxol-5-ylmethoxy)-benzylamide

Trifluoroacetic acid salt of the title compound was obtained from quinoline-6-carboxylic acid 4-hydroxybenzylamine described in Preparation Example E+-1 and methanesulfonic acid benzo[1,3]dioxol-5-ylmethyl ester according to an analogous method to Reference Example E-12.
MS m/e (ESI) 413 (MH⁺)

### Reference Example E-49. 6-Quinolinecarboxylic acid 3-(3-methyl-2-butenyloxy)-benzylamide

6-Quinolinecarboxylic acid (100mg, 0.577mmol), 3-(3-methyl-2-butenyloxy)-benzylamine described in Preparation Example 134 (112mg, 0.635mmol), benzotriazol-1-yl-tris(dimethylamino)phosphonium hexafluorophosphate (306mg, 0.693mmol) and triethylamine (0.12mL, 0.87mmol) were dissolved in tetrahydrofuran (5mL), and the solution was stirred at room temperature for 3 hours. The solvent was evaporated *in vacuo,* the obtained residue was purified by NH silica gel column chromatography (hexane : ethyl acetate = 4 : 1), and the title compound (153mg,80.1%) was obtained as a white solid.
¹H-NMR Spectrum (CDCl₃) δ (ppm) :1.73(3H, s), 1.78(3H, s), 4.51(2H, d, J=6.8Hz), 4.68(2H, d, J=5.6Hz), 5.48(1H, t, J=6.8Hz), 6.62(1H, t, J=5.6Hz), 6.87(1 H, dd, J=2.4, 8.4Hz), 6.95(1H, d, J=2.4Hz), 6.96(1H, d, J=8.4Hz), 7.28(1H, t, J=8.4Hz), 7.47(1H, dd, J=4.0, 8.0Hz), 8.05(1H, dd, J=2.0, 8.8Hz), 8.13(1H, d, J=8.8Hz), 8.23(1H, dd, J=1.2, 8.0Hz), 8.32(1 H, d, J=2.0Hz), 8,97(1 H, dd, J=1.2, 4.0Hz).

### Reference Example E-50. 6-Quinolinecarboxylic acid 3-(2-methylpropenyl)-benzylamide

The title compound (150mg, 0.475mmol, 82.2%) was obtained as a white solid from 6-quinolinecarboxylic acid (100mg, 0.577mmol) and 3-(2-methyl-propenyl)-benzylamine described in Preparation Example 137 (93mg, 0.577mmol) according to an analogous method to Reference Example E-49.
¹H-NMR Spectrum (CDCl₃) δ (ppm) :1.87(3H, s), 1.90(3H, s), 4.70(2H, d, J=5.6Hz), 6.27(1H, s), 6.62(1H, t, J=5.6Hz), 7.20-7.46(4H, m), 7.47(1H, dd, J=4.0, 8.0Hz), 8.05(1H, dd, J=2.0, 8.8Hz), 8.13(1H, d, J=8.8Hz), 8.23(1H, dd, J=1.2, 8.0Hz), 8.32(1H, d, J=2.0Hz), 8,97(1H, dd, J=1.2, 4.0Hz).

### Reference Example E-51. 6-Quinolinecarboxylic acid 3-cyclopentylydenemethylbenzylamide

The title compound (150mg, 0.457mol, 79.3%) was obtained as a white solid from 6-quinolinecarboxylic acid (100mg, 0.577mmol) and 3-cyclopentylydenemethyl-benzylamine described in Preparation Example 139 (108mg, 0.577mmol) according to an analogous method to Reference Example E-49.
¹H-NMR Spectrum (CDCl₃) δ (ppm) :1.65-1.79(4H, m), 2.47-2.58(4H, m), 4.70(2H, d, J=5.6Hz), 6.36(1H,s), 6.54(1H, t, J=5.6Hz), 7.20-7.35(4H, m), 7.47(1H, dd, J=4.0, 8.0Hz), 8.05(1 H, dd, J=2.0, 8.8Hz), 8.13(1 H, d, J=8.8Hz), 8.23(1 H, dd, J=1.2, 8.0Hz), 8.32(1 H, d, J=2.0Hz), 8.97(1 H, dd, J=1.2, 4.0Hz).

### Reference Example E-52. 6-Quinolinecarboxylic acid 3-isobutylbenzylamide

The title compound (75mg, 0.236mol, 66.2%) was obtained as a white solid from 6-quinolinecarboxylic acid (60mg, 0.356mmol) and 3-isobutylbenzylamine described in Preparation Example 146 (58mg, 0.356mmol) according to an analogous method to Reference Example E-49.
¹H-NMR Spectrum (CDCl₃) δ (ppm) : 0.90(6H, d, J=6.8Hz), 1.87(1H, dq, J=7.6Hz, 6.8Hz), 2.48(2H, d, J=7.6Hz), 4.69(2H, d, J=6.0Hz), 6.52(1H, t, J=6.0Hz), 7.10-7.30(4H, m), 7.47(1 H, dd, J=4.0, 8.0Hz), 8.05(1 H, dd, J=2.0, 8.8Hz), 8.13(1H, d, J=8.8Hz), 8.23(1H, dd, J=1.2, 8.0Hz), 8.32(1H, d, J=2.0Hz), 8,97(1H, dd, J=1.2, 4.0Hz).

### Reference Example E-53. Quinoline-6-carboxylic acid 4-benzyloxy-2-fluoro-benzylamide

To a mixture of lithium aluminum hydride (84mg, 2.2mmol) and tetrahydrofuran (2mL) was added 4-benzyloxy-2-fluoro-benzonitrile described in Preparation Example 118 (100mg, 0.44mmol) on an ice bath, and the solution was stirred at room temperature for 1 hour. Water (0.084mL), an aqueous solution of 5N sodium hydroxide (0.084mL) and water (0.25mL) were added sequentially on an ice bath, and the solution was stirred at room temperature for 90 minutes. The reaction solution was filtered through Celite pad, then, the solvent was evaporated *in vacuo.* The title compound (140mg, 0.35mmol, 90%) was obtained as a white solid from the obtained residue (91 mg) and quinoline-6-carboxylic acid (68mg, 0.39mmol) according to an analogous method to Reference Example Q-6.
¹H-NMR Spectrum (CDCl₃) δ (ppm) : 4.68 (2H, d, J=5.7Hz), 5.05 (2H, s), 6.62 (1H,br s), 6.71-6.78 (2H, m), 7.34-7.43 (6H, m), 7.47 (1 H, dd, J=4.3, 8.3Hz), 8.04 (1 H, dd, J=2.0, 8.8Hz), 8.15 (1H, d, J=8.8Hz), 8.23-8.25 (1 H, m), 8.31 (1H, d, J=2.0Hz), 8.98 (1 H, dd, J=1.8, 4.3Hz).

### Reference Example E-54. Quinoline-6-carboxylic acid 4-benzyloxy-3-chloro-benzylamide

Trifluoroacetic acid salt of the title compound (29mg, 0.057mmol, 10%) was obtained from quinoline-6-carboxylic acid 4-benzyloxybenzylamide described in Reference Example E-8 (200mg, 0.54mmol) according to an analogous method to Reference Example A-171.
MS m/e (ESI) 403.1 (MH⁺)

### Reference Example E-55. Quinoline-6-carboxylic acid (4-phenoxy-pyridine-2-ylmethyl)-amide

The title compound (9mg, 8%) was obtained as a colorless oil from 6-quinolinecarboxylic acid (52mg, 0.30mmol) and C-(4-phenoxy-pyridine-2-yl)-methylamine described in Preparation Example 7 (60mg, 0.30mmol) according to an analogous method to Reference Example L-4.
¹H-NMR Spectrum (CDCl₃) δ (ppm) : 4.74(2H, d, J=4.4Hz), 6.79(1H, dd, J=2.4Hz, 5.6Hz), 6.88(1H, d, J=2.4Hz), 7.10-7.12(2H, m), 7.43-7.49(4H, m), 7.81(1H, brs), 8.14-8.19(2H, m), 8.26-8.28(1 H, m), 8.40-8.44(2H, m), 8.99-9.0(1 H, m).

### Reference Example E-56. Quinoline-6-carboxylic acid (6-phenoxy-pyridin-2-ylmethyl)-benzylamide

The title compound (32mg, 28%) was obtained as a colorless oil from 6-quinolinecarboxylic acid (56mg, 0.325mmol) and C-(6-phenoxy-pyridin-2-yl)-methylamine described in Preparation Example 16 (65mg, 0.325mmol) according to an analogous method to Reference Example L-4.
¹H-NMR Spectrum (CDCl₃) δ (ppm) : 4.73(2H, d, J=4.4Hz), 6.89-6.91(1H, m), 7.04-7.06(1H, m), 7.21-7.23(2H, m), 7.30-7.34(1H, m), 7.44-7.52(3H, m), 7.64(1H, brs), 7.65-7.68(1 H, m), 7.73-7.77(1 H, m), 8.11 (1 H, d, J=8.4Hz), 8.26(1 H, d, J=8.0Hz), 8.32(1 H, d, J=1.6Hz), 9.01-9.03(1 H, m).

### Reference Example E-57. Quinoline-6-carboxylic acid (1-benzyl-1H-pyrrol-3-ylmethyl)-amide

7N Ammonia/methanol (80mL) and Raney nickel (2g) were added to 1-benzyl-1*H*-pyrrole-3-carbaldehyde described in Preparation Example 57 (800mg, 4.3mmol), and the solution was stirred at room temperature for 22 hours under hydrogen atmosphere at ordinary pressure. The catalyst was removed by filtrating through Celite pad, then, the solvent was evaporated *in vacuo* and C-(1-benzyl-1*H*-pyrrolo-3-yl)methylamine was quantitatively obtained as a brown oil. The title compound (110mg, 0.32mmol, 24.8%) was obtained as a white solid from the resulting C-(1-benzyl-1*H*-pyrrolo-3-yl)methylamine (240mg, 1.3mmol) and 6-quinolinecarboxylic acid (180mg, 1.04mmol) according to an analogous method to Reference Example Q-6.
¹H-NMR Spectrum (DMSO-d₆) δ (ppm) : 4.32 (2H, d, 5.6Hz), 5.02 (2H, s), 6.02 (1H, s), 6.74 (1H, s), 6.76 (1 H, s), 6.18-7.34 (5H, m), 7.58 (1H, dd, J=4.0, 8.4Hz), 8.03 (1 H, d, J=8.8Hz), 8.16 (1H, dd, J=2.0, 8.8Hz), 8.43 (1H, dd, J=1.6, 8.4Hz), 8.49 (1H, d, J=2.0Hz), 8.97 (1 H, t, J=5.6Hz), 8.95 (1 H, dd, J=1.6, 4.0Hz).

### Reference Example E-58. Quinoline-6-carboxylic acid (1-benzo[1,3]dioxol-5-ylmethyl-1H-pyrrol-3-ylmethyl)-amide

The title compound (30mg, 0.077mmol, 18.1%) was obtained as a white solid from C-(1-benzo[1,3]dioxol-5-ylmethyl-1*H*-pyrrol-3-yl)-methylamine described in Preparation Example 61 (100mg, 0.43mmol) and 6-quinolinecarboxylic acid (75mg, 0.43mmol) according to an analogous method to Reference Example Q-6.
¹H-NMR Spectrum (DMSO-d₆) δ (ppm) : 4.28-4.34 (2H, m), 4.90 (2H, s), 5.95 (2H, s), 6.00 (1 H, s), 6.70-6.86 (5H, m), 7.55-7.60 (1H, m), 8.01-8.05 (1 H, m), 8.14-8.20 (1 H, m), 8.40-8.46 (1 H, m), 8.48-8.51 (1 H, m), 8.39-8.98 (2H, m).

### Reference Example E-59. Quinoline-6-carboxylic acid (1-phenethyl-1H-pyrrol-3-ylmethyl)-amide

The title compound (108mg, 0.304mmol, 30.4%) was obtained as a slightly yellow solid from 1-phenethyl-1*H*-pyrrole-3-carbaldehyde described in Preparation Example 62 and quinoline-6-carboxylic acid (173mg, 1.0mmol) according to an analogous method to Reference Example E-57.
¹H-NMR Spectrum (DMSO-d₆) δ (ppm) : 2.96 (2H, t, J=7.6Hz), 4.03 (2H, t, J=7.6Hz), 4.31 (2H, d, J=5.6Hz), 5.96 (1 H, s), 6.64 (1 H, s), 6.73 (1 H, s), 7.14-7.28 (5H, m), 7.58 (1H, dd, J=4.0, 8.0Hz), 8.04 (1H, d, J=8.8Hz), 8.17 (1H, d, J=8.8Hz), 8.43 (1H, d, J=8.0Hz), 8.50 (1H, s), 8.90 (1H, t, J=5.6Hz), 8.95-8.99 (1H, m).

### Reference Example E-60. Quinoline-6-carboxylic acid (1-benzyloxy-1H-pyrrol-3-ylmethyl)-amide

Diethyl azodicarboxylate (154mg, 0.869mmol) was added dropwise to a solution of (1-benzyloxy-1H-pyrrol-3-yl)-methanol described in Preparation Example 64 (168mg, 0.828mmol), phthalimide (130mg, 0.869mmol) and triphenylphosphine (230mg, 0.869mmol) in dichloromethane at 0°C, and then, the solution was stirred at room temperature for 8 minutes. NH silica gel was added to the reaction solution, the solvent was evaporated *in vacuo* for adsorption, purification was carried out by NH silica gel column chromatography (hexane : ethyl acetate = 4 : 1), and a colorless oil (100mg) was obtained. Ethanol (5mL) and hydrazine monohydrate (0.1mL) were added to this oil (100mg), and the solution was stirred under reflux for 15 minutes. The reaction solution was allowed to room temperature, solid was eliminated by filtration, then, the solvent was evaporated, and an oil containing C-(1-benzyloxy-1*H*-pyrrol-3-yl)methylamine was obtained (80mg). The title compound (31 mg, 0.086mmol) was obtained as a colorless oil from this oil (80mg) and quinoline-6-carboxylic acid according to an analogous method to Reference Example Q-6.
¹H-NMR Spectrum (DMSO-d₆) δ (ppm) : 4.28 (2H, d, J=5.6Hz), 5.11 (2H, s), 5.86-5.89 (1 H, m), 6.78-6.81 (1 H, m), 6.88-6.91 (1 H, m), 7.34-7.42 (5H, m), 7.59 (1 H, dd, J=4.0, 8.0Hz), 8.04 (1 H, d, J=8.8Hz), 8.16 (1 H, d, J=8.8Hz), 8.44 (1 H, d, J=8.0Hz), 8.49 (1 H, s), 8.92-8.98 (2H, m).

### Reference Example E-61. Quinoline-6-carboxylic acid (1-phenyl-1H-pyrrol-3-ylmethyl)-amide

The title compound (136mg, 0.415mmol, 47.8%) was obtained as a white solid from C-(1-phenyl-1*H*-pyrrol-3-yl)-methylamine described in Preparation Example 74 (150mg, 0.87mmol) and quinoline-6-carboxylic acid (150mg, 0.87mmol) according to an analogous method to Reference Example Q-6.
¹H-NMR Spectrum (DMSO-d₆) δ (ppm) : 4.42 (2H, d, J=5.6Hz), 6.28-6.31 (1 H, m), 7.18-7.24 (1 H, m), 7.29-7.35 (2H, m), 7.40-7.45 (2H, m), 7.50-7.65 (2H, m), 7.59 (1 H, dd, J=4.0, 8.0Hz), 8.05 (1 H, d, J=8.8Hz), 8.20 (1 H, dd, J=2.0, 8.8Hz), 8.43-8.48 (1 H, m), 8.53 (1 H, d, J=2.0Hz), 8.96 (1 H, dd, J=1.6, 4.0Hz), 9.01 (1 H, t, J=5.6Hz).

### Reference Example E-62. Quinoline-6-carboxylic acid (2-benzyl-2H-tetrazol-5-ylmethyl)-amide

Sodium azide (260mg,4.0mmol) and ammonium chloride (210mg,4.0mmol) were suspended in a solution of quinoline-6-carboxylic acid cyanomethyl-amide described in Preparation Example E-1 (420mg,2.0mmol) in N,N-dimethylformamide (15mL), and the solution was stirred at 100°C for 12 hours. Benzyl bromide (0.12mL, 1.0mmol) and potassium carbonate (400mg, 3.0mmol) were added to a solution having half the amount of the solution containing quinoline-6-carboxylic acid (2H-tetrazol-5-ylmethyl)amide obtained, which was then stirred for 20 minutes at 50°C. Water and ethyl acetate were added to the reaction solution, which was then partitioned, NH silica gel was added to the organic layer, and the solvent was evaporated *in vacuo* for adsorption, purification was carried out by NH silica gel column chromatography, and the title compound (20mg, 0.058mmol) was obtained as a white solid.
¹H-NMR Spectrum (DMSO-d₆) δ (ppm) : 4.78 (2H, d, J=5.6Hz), 5.92 (2H, s), 7.34-7.43 (5H, m), 7.62 (1 H, dd, J=4.0, 8.4Hz), 8.09 (1 H, d, J=8.8Hz), 8.19 (1 H, dd, J=2.0, 8.8Hz), 8.48 (1 H, dd, J=1.2, 8.4Hz), 8.54 (1 H, d, J=2.0Hz), 9.00 (1 H, dd, J=1.2, 4.0Hz), 9.44 (1H, t, J=5.6Hz).

### Reference Example E-63. Quinoline-6-carboxylic acid (2-phenoxy-thiazol-5-ylmethyl)-amide

The title compound (17mg, 46µmol, 91%) was obtained as a white solid from C-(2-phenoxy-thiazol-5-yl)-methylamine described in Preparation Example 117 (10mg, 50µmol) and quinoline-6-carboxylic acid (11mg, 60µmol) according to an analogous method to Reference Example A-26.
¹H-NMR Spectrum (DMSO-d₆) δ (ppm) : 4.55 (2H, d, J=5.7Hz), 7.23 (1H, s), 7.27-7.31 (3H, m), 7.45 (2H, dd, J=7.2, 8.8Hz), 7.59 (1 H, dd, J=4.2, 8.8Hz), 8.06 (1 H, d, J=8.8Hz), 8.14 (1 H, dd, J=1.8, 8.8Hz), 8.46 (1 H, d, J=7.1Hz), 8.5 (1H, d, J=1.8Hz), 8.97 (1H, dd, J=1.7, 4.2Hz), 9.39 (1H, t, J=5.7Hz).

### Reference Example E-64. Quinoline-6-carboxylic acid (5-(3-cyano-phenoxy)-thiophen-2-ylmethyl)-amide

To a solution of C-(5-(3-bromophenoxy)-thiophen-2-yl)-methylamine described in Preparation Example 17 (200mg, 0.703mmol) and 6-quinolinecarboxylic acid (123mg, 0.703mmol) in tetrahydrofuran (5mL) were added benzotriazol-1-yloxytris(dimethylamino)phosphonium hexafluorophosphate (373mg, 0.844mmol) and triethylamine (0.2mL, 1.41mmol), and the solution was stirred at room temperature for 2 hours. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and a mixture of quinoline-6-carboxylic acid (5-(3-bromophenoxy)-thiophene-2ylmethyl)-amide and debrominated compound (170mg, 55%) was obtained as a colorless oil.
Next, to a solution of the mixture of quinoline-6-carboxylic acid (5-(3-bromophenoxy)-thiophene-2ylmethyl)-amide and debrominated compound (130mg, 0.303mmol) in N,N-dimethylformamide (5.0mL) were added zinc cyanide (71 mg, 0.605mmol) and tetrakis(triphenylphosphine)palladium(0) (70mg, 0.061mmol) under nitrogen atmosphere, the solution was stirred at 100°C for 1 hour, and the solution was stirred at 140°C for 3 hours. The reaction solution was allowed to room temperature, ethyl acetate and water were added to the reaction solution, which was then partitioned, the organic layer was washed with water and dried over anhydrous magnesium sulfate. The solvent was evaporated, then, the residue was purified by NH silica gel column chromatography (hexane : ethyl acetate), and title compound (25mg, 21 %) was obtained as a colorless oil.
¹H-NMR Spectrum (CDCl₃) δ (ppm) : 4.80(2H, d, J=5.2Hz), 6.49(1H, d, J=4.0Hz), 6.68(1H, brs), 6.85(1 H, d, J=3.6Hz), 7.30-7.45(4H, m), 7.48(1H, dd, J=4.4, 8.4Hz), 8.07(1H, dd, J=2.0, 8.8Hz), 8.17(1H, d, J=8.8Hz), 8.24-8.27(1H, m), 8.35(1H, d, J=2.0Hz), 8.99-9.01(1H, m).

### Reference Example E-65. Quinoline-6-carboxylic acid (5-(3-fluorophenoxy)thiophen-2-ylmethyl) amide

The title compound (100mg, 0.265mmol, 29.4%) was obtained as a reddish brown oil from C-(5-(3-fluorophenoxy)thiophen-2-yl)methylamine described in Preparation Example 23 and 6-quinolinecarboxylic acid according to an analogous method to Reference Example Q-6.
¹H-NMR Spectrum (DMSO-d₆) δ (ppm) : 4.59 (2H, d, J=5.6Hz), 6.59 (1H, d, J=3.6Hz), 6.84 (1 H, d, J=3.6Hz), 6.90-7.7.02 (3H, m), 7.39 (1 H, ddd, J=8.0, 8.0, 8.0Hz), 7.60(1 H, dd, J=4.0, 8.0Hz), 8.06(1 H, d, J=8.8Hz), 8.16 (1 H, dd, J=1.6, 8.8Hz), 8.45 (1 H, d, J=8.0Hz), 8.51 (1 H, s), 8.97(1 H, d, J=4.0Hz), 9.37(1 H, t, J=5.6Hz).

### Reference Example E-66. Quinoline-6-carboxylic acid (5-phenoxythiophen-2-ylmethyl) amide

Sodium hydride (3g, 74mmol, 60% in oil) was added to a solution of phenol (7g,74mmol) in dimethylsulfoxide (40mL), which was then stirred at room temperature for 10 minutes, and 5-nitrothiophene-2-carbaldehyde (10g, 64mmol) was further added, followed by stirring for 15 minutes. Water and ethyl acetate were added to the reaction solution, which was then partitioned, the organic layer was washed with an aqueous solution of 2N sodium hydroxide twice and with water three times, then, passed through a glass filter lined with silica gel, and eluted with ethyl acetate. The solvent was evaporated *in vacuo,* and a yellow oil containing 5-phenoxy thiophene-2-carbaldehyde was obtained (500mg). This oil (500mg) was dissolved in 7N ammonia/methanol solution (30mL), Raney nickel (1.5g) was added thereto, and the solution was stirred overnight under hydrogen atmosphere. Raney nickel was removed by filtering through Celite pad, then, the filtrate was concentrated *in vacuo,* the residue was purified by silica gel chromatography (ethyl acetate, then ethyl acetate : methanol = 4 : 1) and a brown oil containing C-(5-phenoxythiophen-2-yl)methylamine was obtained (40mg).
Then, to a solution of the obtained oil (40mg, 0.195mmol) and 6-quinolinecarboxylic acid (41mg, 0.234mmol) in N,N-dimethylformamide (5mL) were added benzotriazol-1-yl-tris(dimethylamino)phosphonium hexafluorophosphate (100mg, 0.234mmol) and triethylamine (0.054mL, 0.39mmol), and the solution was stirred for 30 minutes at 60°C. Water and ethyl acetate were added to the reaction solution, which was then partitioned, the organic layer was washed with water twice, NH silica gel was added to this organic layer, the solvent was evaporated *in vacuo* for adsorption, and purification was carried out by NH silica gel column chromatography (hexane : ethyl acetate = 3 : 1, then 1:1, then ethyl acetate). The solvent was evaporated *in vacuo,* then, the solid generated by adding diethyl ether to the residue was collected by filtration, and the title compound (40mg, 0.111 mmol;- 56.9%) was obtained as a pale yellow solid.
¹H-NMR Spectrum (DMSO-d₆) δ (ppm) : 4.57 (2H, d, J=5.6Hz), 6.51 (1 H, d, J=3.6Hz), 6.81 (1 H, d, J=3.6Hz), 7.06-7.15 (3H, m), 7.23-7.40 (2H, m), 7.59 (1 H, dd, J=4.0, 8.0Hz), 8.06 (1 H, d, J=8.8Hz), 8.16 (1 H, dd, J=2.0, 8.8Hz), 8.45 (1 H, dd, J=1.6, J=8.0Hz), 8.51 (1 H, d, J=2.0Hz), 8.97 (1 H, dd, J=1.6, 4.0Hz), 9.36 (1 H, t, J=5.6Hz).

### Reference Example E-67. Quinoline-6-carboxylic acid (5-(4-fluorophenoxy)thiophen-2-yl)methyl amide

The title compound (38mg, 0.100mmol, 27.8%) was obtained as a white solid from C-(5-(4-fluorophenoxy)thiophen-2-yl)methylamine described in Preparation Example 28 and 6-quinolinecarboxylic acid according to an analogous method to Reference Example Q-6.
¹H-NMR Spectrum (DMSO-d₆) δ (ppm) : 4.57 (2H, d, J=6.0Hz), 6.49 (1H, d, J=3.6Hz), 6.80 (1 H, d, J=3.6Hz), 7.10-7.17 (2H, m), 7.17-7.24 (2H, m), 7.59 (1 H, dd, J=4.0, 8.0Hz), 8.06 (1H, d, J=8.8Hz), 8.16 (1H, dd, J=2.0, J=8.8Hz), 8.45 (1H, dd, J=1.6, 8.0Hz), 8.51 (1H, d, J=2.0Hz), 8.97 (1H, dd, J=1.6, 4.0Hz), 9.36 (1H, t, J=6.0Hz).

### Reference Example E-68. Quinoline-6-carboxylic acid (5-(4-chloro-phenoxy)-thiophen-2-ylmethyl)-amide

The title compound (87mg, 0.22mmol, 76.1 %) was obtained as a brown oil from the resulting C-(5-(4-chlorophenoxy)thiophen-2-yl)methylamine (70mg, 0.29mmol) and 6-quinolinecarboxylic acid (51mg, 0.29mmol) according to an analogous method to Reference Example E-66.
¹H-NMR Spectrum (DMSO-d₆) δ (ppm) : 4.58 (2H, d, 5.6Hz), 6.55 (1 H, d, J=4.0Hz), 6.83 (1H, d, J=4.0Hz), 7.08-7.14 (2H, m), 7.38-7.45 (2H, m), 7.59 (1H, dd, J=4.4, 8.0Hz), 8.06 (1H, d, J=8.8Hz), 8.16 (1H, dd, J=2.0, 8.8Hz), 8.46 (1H, dd, J=1.6, 8.0Hz), 8.51 (1 H, d, J=2.0Hz), 8.97 (1 H, dd, J=1.6, 4.4Hz), 9.36 (1 H, t, J=5.6Hz).

### Reference Example E-69. Quinoline-6-carboxylic acid (4-(3-fluoro-phenoxy)-thiophen-2-ylmethyl)-amide

The title compound (24mg, 0.063mmol, 39.7%) was obtained as a white solid from C-(4-(3-fluorophenoxy)thiophen-2-yl)methylamine described in Preparation Example 35 (35mg, 0.16mmol) and 6-quinolinecarboxylic acid (33mg, 0.19mmol) according to an analogous method to Reference Example Q-6.
¹H-NMR Spectrum (DMSO-d₆) δ (ppm) : 4.63 (2H, d, J=6.0Hz), 6.83-6.97 (5H, m), 7.38 (1 H, ddd, J=8.0, 8.0, 8.0Hz), 7.60 (1 H, dd, J=4.4, 8.0Hz), 8.07 (1 H, d, J=8.8Hz), 8.17 (1 H, dd, J=2.0, 8.8Hz), 8.47 (1 H, dd, J=2.0, 8.0Hz), 8.53 (1 H, d, J=2.0Hz), 8.97 (1 H, dd, J=2.0, 4.4Hz), 9.39 (1 H, t, J=6.0Hz).

### Reference Example E-70. Quinoline-6-carboxylic acid (5-benzyl-thiophen-2-ylmethyl)-amide

The title compound (40mg, 0.111mmol, 41.3 %) was obtained as a white solid from quinoline-6-carboxylic acid (46mg, 0.27mmol) and C-(5-benzyl-thiophen-2-yl)-methylamine described in Preparation Example 42 (54mg, 0.27mmol) according to an analogous method to Reference Example Q-6.
¹H-NMR Spectrum (DMSO-d₆) δ (ppm) : 4.04 (2H, s), 4.57 (2H, d, J=5.6Hz), 6.71 (1H, d, J=3.6Hz), 6.84 (1H, d, J=3.6Hz), 7.14-7.30 (5H, m), 7.58 (1H, dd, J=4.0, 8.4Hz), 8.04 (1H, d, J=8.8Hz), 8.14 (1H, dd, J=2.0, 8.8Hz), 8.44 (1H, dd, J=2.0, 8.4Hz), 8.49 (1 H, d, J=2.0Hz), 8.96 (1 H, dd, J=2.0, 4.0Hz), 9.29 (1 H, t, J=5.6Hz).

### Reference Example E-71. Quinoline-6-carboxylic acid (5-(3-chloro-benzyl)-thiophen-2ylmethyl)-amide

The title compound (73mg, 0.18mmol, 85.7%) was obtained as a white solid from C-(5-(3-chloro-benzyl)-2-yl)-methylamine described in Preparation Example 45 (50mg, 0.21 mmol) and 6-quinolinecarboxylic acid (40mg, 0.23mmol) according to an analogous method to Reference Example Q-6.
¹H-NMR Spectrum (DMSO-d₆) δ (ppm) : 4.07 (2H, s), 4.58 (2H, d, J=5.2Hz), 6.74 (1H, d, J=2.4Hz), 6.84 (1H, d, J=2.4Hz), 7.17-7.34 (4H, m), 7.59 (1H, dd, J=4.0, 8.4Hz), 8.05 (1 H, d, J=8.4Hz), 8.14 (1 H, d, J=8.4Hz), 8.44 (1 H, d, J=8.4Hz), 8.49 (1 H, s), 8.95 (1 H, d, J=4.0Hz), 9.30(1 H, t, J=5.2Hz).

### Reference Example E-72. Quinoline-6-carboxylic acid (5-(3-fluoro-benzyl)-thiophen-2-ylmethyl)-amide

The title compound (75mg, 0.199mmol, 80.2%) was obtained as a light brown solid from quinoline-6-carboxylic acid (43mg, 0.248mmol) and 5-(3-fluoro-benzyl)-thiophene-2-carbaldehyde described in Preparation Example 53 (50mg, 0.226mmol) according to an analogous method to Reference Example A-146.
¹H-NMR Spectrum (DMSO-d₆) δ (ppm) : 4.08 (2H, s), 4.58 (2H, d, J=5.6Hz), 6.74 (1H, d, J=3.2Hz), 6.85 (1H, d, J=3.2Hz), 6.98-7.10 (3H, m), 7.28-7.35 (1H, m), 7.59 (1 H, dd, J=4.0, 8.0Hz), 8.05 (1 H, d, J=8.8Hz), 8.15 (1 H, dd, J=1.6, 8.8Hz), 8.44 (1 H, d, J=8.0Hz), 8.49 (1 H, d, J=1.6Hz), 8.95-8.99 (1 H, m), 9.30 (1 H, t, J=5.6Hz).

### Reference Example E-73. Quinoline-6-carboxylic acid (5-(5-methyl-thiophen-2-ylmethyl)-thiophen-2-ylmethyl)-amide

To a solution of (5-(5-methyl-thiophen-2-ylmethyl)-thiophen-2-yl)-methanol described in Preparation Example 67 (640mg, 2.86mmol), phthalimide (420mg, 2.86mmol) and triphenylphosphine (750mg, 2.86mmol) in tetrahydrofuran (7mL) was added diethyl azodicarboxylate (500mg, 2.86mmol) dropwise at 0°C, which was then stirred at room temperature for 15 minutes. Water and ethyl acetate were added to the reaction solution, which was then partitioned, silica gel was added, the solvent was concentrated *in vacuo* for adsorption, and purification was carried out by silica gel column chromatography (hexane : ethyl acetate = 8 : 1). The solvent was evaporated *in vacuo,* a brown solid (360mg, 1.02mmol, 35.6%) was obtained.
Ethanol (5mL) and hydrazine monohydrate (180mg, 3.06mmol) were added to the resulting solid (360mg), and the solution was stirred for 20 minutes at 90°C. The solution was allowed to room temperature, the solid was eliminated by filtration, and a pale yellow oil containing C-5-(5-methyl-thiophen-2-ylmethyl)-thiophen-2-yl)-methylamine was obtained (200mg, 0.896mmol, 89.6%). The title compound (101mg, 0.267mmol, 29.8%) was obtained as a white solid from this oil (200mg, 0.896mmol) and quinoline-6-carboxylic acid (160mg, 0.896mmol) according to an analogous method to Reference Example Q-6.
¹H-NMR Spectrum (DMSO-d₆) δ (ppm) : 2.35 (3H, s), 4.20 (2H, s), 4.61 (2H, d, J=5.6Hz), 6.58-6.62 (1 H, m), 6.69 (1H, d, J=3.2Hz), 6.75 (1 H, d, J=3.2Hz), 6.86 (1 H, d, J=3.2Hz), 7.61 (1 H, dd, J=4.0, 8.4Hz), 8.08 (1 H, d, J=8.8Hz), 8.18 (1 H, dd, J=2.0, 8.8Hz), 8.47 (1H, d, J=8.4Hz), 8.52 (1H; d, J=2.0Hz), 8.97-9.01 (1H, m), 9.34 (1H, t, J=5.6Hz).

### Reference Example E-74. Quinoline-6-carboxylic acid (5-(5-methyl-furan-2-ylmethyl)-thiophen-2-ylmethyl)-amide

The title compound (3.0mg, 0.008mmol) was obtained as a brown oil from (5-(5-methyl-furan-2-ylmethyl)-thiophen-2-yl)-methanol described in Preparation Example 70 (210mg, 1.0mmol) according to an analogous method to Reference Example E-73.
¹H-NMR Spectrum (DMSO-d₆) δ (ppm) : 2.18 (3H, s), 4.04 (2H, s), 4.61 (2H, d, J=5.6Hz), 5.94 (1 H, d, J=3.2Hz), 6.03 (1 H, d, J=3.2Hz), 6.74 (1 H, d, J=3.2Hz), 6.87 (1 H, d, J=3.2Hz), 7.61 (1H, dd, J=4.0, 8.0Hz), 8.07 (1H, d, J=8.4Hz), 8.17 (1H, dd, J=1.6, 8.4Hz), 8.47 (1 H, dd, J=2.0, 4.0Hz), 8.52 (1 H, d, J=1.6Hz), 8.98 (1 H, dd, J=2.0, 4.0Hz), 9.33 (1 H, t, J=5.6Hz).

### Reference Example E-75. Quinoline-6-carboxylic acid (5-benzofuran-2-ylmethyl-thiophen-2-ylmethyl)-amide

The title compound (55mg, 0.13mmol) was obtained as a white solid from (5-benzofuran-2-ylmethyl-thiophene)-methanol described in Preparation Example 72 according to an analogous method to Reference Example E-73.
¹H-NMR Spectrum (DMSO-d₆) δ (ppm) : 4.31 (2H, s), 4.62 (2H, d, J=5.6Hz), 6.68 (1 H, s), 6.85 (1 H, d, J=3.6Hz), 6.91 (1 H, d, J=3.6Hz), 7.16-7.26 (2H, m), 7.46-7.64 (3H, m), 8.07 (1 H, d, J=8.8Hz), 8.17 (1 H, dd, J=2.0, 8.8Hz), 8.44-8.48 (1 H, m), 8.52 (1 H, d, J=2.0Hz), 8.96-9.00 (1 H, m), 9.34 (1 H, t, J=5.6Hz).

### Reference Example E-76. Quinoline-6-carboxylic acid (5-benzyloxy-thiophen-2-ylmethyl)-amide

To a solution of 5-benzyloxy-thiophene-2-carbonitrile described in Preparation Example 81 (30mg, 0.14mmol) in tetrahydrofuran (3mL) was added lithium aluminum hydride (21 mg, 0.557mmol), which was then stirred for 1.5 hours at room temperature. Sodium fluoride (240mg, 5.72mmol) was added to the reaction mixture, which was stirred for 2 hours, then, on an ice bath, 10% hydrous tetrahydrofuran (2mL) was added. The reaction mixture was filtered through Celite pad, the filtrate was concentrated and C-(5-benzyloxy-thiophen-2-yl)methylamine (32mg, 0.147mmol) was obtained as a crude product. The title compound (3mg, 0.008mmol, 5.4%) was obtained from this and quinoline-6-carboxylic acid (26mg, 0.15mmol) according to an analogous method to Reference Example H-1.
¹H-NMR Spectrum (CDCl₃) δ (ppm) : 4.74 (2H, d, J=4.4Hz), 5.09 (2H, s), 6.15 (1H, d, J=4.0Hz), 6.52-6.62 (1 H, m), 6.71 (1 H, d, J=4.0Hz), 7.32-7.47 (5H, m), 7.50 (1 H, dd, J=4.0, 8.4Hz), 8.07 (1 H, dd, J=2.0, 8.8Hz), 8.18 (1 H, d, J=8.8Hz), 8.27 (1 H, dd, J=1.6, 8.4Hz), 8.34 (1 H, d, J=2.0Hz), 9.02 (1 H, dd, J=1.6, 4.0Hz).

### Reference Example E-77. Quinoline-6-carboxylic acid (5-(3-chloro-phenoxy)-thiophen-2-ylmethyl)-amide

The title compound (9.53mg) was obtained from C-(5-(3-chloro-phenoxy)-thiophen-2-yl)-methylamine described in Reference Example A-73 (30mg, 0.13mmol) and quinoline-6-carboxylic acid (22mg, 0.13mmol) according to an analogous method to Reference Example E-24. Trifluoroacetic acid salt of the title compound was obtained by reverse phase high performance liquid chromatography (acetonitrile-water mobile phase (containing 0.1% trifluoroacetic acid) was used).
MS m/e (ESI) 395.35(MH⁺)

### Reference Example E-78. 6-Quinolinecarboxylic acid (5-(2-methylpropenyl)thiophen-2-ylmethyl) amide

The title compound (15mg, 0.0466mmol, 56.1 %) was obtained as a white solid from 6-quinolinecarboxylic acid (15mg, 0.083mmol) and C-(5-(2-methylpropenyl)-thiophen-2-yl)-methylamine described in Preparation Example 145 (14mg, 0.083mmol) according to an analogous method to Reference Example E-49.
¹H-NMR Spectrum (CDCl₃) δ (ppm) :1.91(3H, s), 1.96(3H, s), 4.83(2H, d, J=5.2Hz), 6.33(1H, s), 6.62(1H, t, J=5.2Hz), 6.75(1H, d, J=3.6Hz), 6.96(1H, d, J=3.6Hz), 7.47(1H, dd, J=4.0, 8.0Hz), 8.05(1H, dd, J=2.0, 8.8Hz), 8.13(1H, d, J=8.8Hz), 8.23(1H, dd, J=1.2, 8.0Hz), 8.32(1 H, d, J=2.0Hz), 8,97(1H, dd, J=1.2, 4.0Hz).

### Reference Example E-79. Quinoline-6-carboxylic acid (5-(2-fluoro-phenoxy)-thiophen-2-ylmethyl)-amide

¹H-NMR Spectrum (CDCl₃) δ (ppm) : 4.55 (2H, d, J=6.0Hz), 6.47 (1 H, d, J=4.0Hz), 6.78 (1H, d, J=3.6Hz), 7.16-7.26 (3H, m), 7.32-7.39 (1H, m), 7.59 (1H, dd, J=4.0, J=8.0Hz), 8.06 (1H, d, J=8.8Hz), 8.15 (1H, dd, J=2.0, J=8.8Hz), 8.45 (1H, d, J=8.0Hz), 8.51 (1 H, d, J=2.0Hz), 8.95-8.98 (1 H, m), 9.35 (1 H, t, J=6.0Hz).

### Reference Example E-80 Quinoline-6-carboxylic acid (5-pyridin-2-ylmethyl-thiophen-2-ylmethyl)-amide

¹H-NMR Spectrum (CDCl₃) δ (ppm) : 4.20 (2H, s), 4.60 (2H, d, J=5.6Hz), 6.77 (1 H, d, J=3.2Hz), 6.86 (1 H, d, J=3.2Hz), 7.22 (1 H, dd, J=5.2, J=7.6Hz), 7.31 (1 H, d, J=7.6Hz), 7.61 (1H, dd, J=4.0, 8.0Hz), 7.71 (1H, ddd, J=1.6, J=7.6, J=7.6Hz), 8.07 (1H, d, J=8.0Hz), 8.17 (1H, dd, J=2.0, J=8.0Hz), 8.42-8.51 (2H, m), 8.52 (1H, d, J=2.0Hz), 8.98 (1 H, dd, J=1.2, J=4.0Hz), 9.32 (1 H, t, J=5.6Hz).

### Reference Example E-81. Quinoline-6-carboxylic acid (5-benzo[1,3]dioxol-5-ylmethyl-thiophen-2-ylmethyl)-amide

¹H-NMR Spectrum (CDCl₃) δ (ppm) : 3.97 (2H, s), 4.59 (2H, d, J=6.0Hz), 5.95 (2H, s), 6.69-6.75 (2H, m), 6.78-6.87 (3H, m), 7.61 (1H, dd, J=4.4, J=8.4Hz), 8.07 (1H, d, J=8.8Hz), 8.17 (1H, dd, J=2.0, J=8.8Hz), 8.46 (1H, dd, J=1.6, J=8.4Hz), 8.51 (1H, d, J=2.0Hz), 8.98 (1 H, dd, J=1.6, J=4.4Hz), 9.31 (1 H, t, J=6.0Hz).

### Reference Example E-82. Quinoline-6-carboxylic acid (5-(3-hydroxy-phenoxy)-thiophen-2-ylmethyl)-amide

To a solution of C-(5-(3-benzyloxy-phenoxy)-thiophen-2-yl)-methylamine described in Preparation Example 18 (180mg, 0.578mmol) and 6-quinolinecarboxylic acid (100mg, 0.578mmol) in tetrahydrofuran (5mL) were added benzotriazol-1-yloxytris(dimethylamino)phosphonium hexafluorophosphate (307mg, 0.694mmol) and triethylamine (0.16mL, 1.16mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and quinoline-6-carboxylic acid (5-(3-benzyloxy-phenoxy)-thiophen-2-ylmethyl)-amide (73mg, 27%) was obtained as a pale yellow solid.
Trifluoroacetic acid (1.0mL) and thioanisole (100µl) were added to the resulting quinoline-6-carboxylic acid (5-(3-benzyloxy-phenoxy)-thiophene-2-ylmethyl)-amide (73mg, 0.156mmol), and the solution was stirred for 30 minutes at room temperature. The reaction solution was neutralized with an aqueous solution of saturated sodium bicarbonate, then, extracted with ethyl acetate and dried over anhydrous magnesium sulfate. The solvent was evaporated, the residue was purified by NH silica gel column chromatography (ethyl acetate : methanol), and the title compound (47mg, 80%) was obtained as a colorless solid.
¹H-NMR Spectrum (CDCl₃) δ (ppm) : 4.73(2H, d, J=5.6Hz), 6.39(1H, d, J=3.6Hz), 6.59-6.62(2H, m), 6.64-6.67(1H, m), 6.74(1H, d, J=3.6Hz), 6.83(1H, brs), 7.17(1H, t, J=8.4Hz), 7.49(1H, dd, J=4.4, 8.4Hz), 8.05(1H, dd, J=2.0, 8.4Hz), 8.14(1H, d, J=8.4Hz), 8.24-8.26(1H, m), 8.33(1 H, d, J=2.0Hz), 8.98-8.99(1H, m).

### Reference Example F-1. Cinnoline-6-carboxylic acid 3-phenoxy-benzylamide

To a solution of cinnoline-6-carboxylic acid methyl ester described in Preparation Example F-4 (16mg, 0.085mmol) in ethanol (1mL) was added an aqueous solution of 1 N sodium hydroxide (0.7mL), and the solution was stirred at room temperature for 2 hours. 1 N Hydrochloric acid was added to the reaction mixture to adjust the pH to 4, toluene was added, and the solution was concentrated *in vacuo.* To a solution of the obtained residue in N,N-dimethylformamide (2mL) were added 4-phenoxybenzylamine described in Preparation Example 3 (17mg, 0.085mmol), benzotriazot-1-yloxytris(dimethylamino)phosphonium hexafluorophosphate (48mg, 0.108mmol) and triethylamine (24µl, 0.172mmol), and the solution was stirred at room temperature for 14 hours. Water was added to the reaction mixture, which was extracted with ethyl acetate, the organic layer was washed with an aqueous solution of saturated sodium bicarbonate, and then concentrated. The residue was purified by reverse phase high performance liquid chromatography (acetonitrile-water mobile phase (containing 0.1% trifluoroacetic acid) was used), and the title compound (4.4mg, 0.0093mmol, 11%) was obtained as a trifluoroacetic acid salt.
MS m/e(ESI) 356.44 (MH⁺)

### Reference Example G-1. Isoquinoline-6-carboxylic acid 3-phenoxybenzylamide

The title compound (3.4mg, 33%) was obtained as a colorless oil from isoquinoline-6-carboxylic acid described in Preparation Example G-1 (5mg, 0.0289mmol) and 4-phenoxybenzylamine described in Preparation Example 3 (6mg, 0.0289mmol) according to an analogous method to Reference Example L-4.
¹H-NMR Spectrum (CDCl₃) δ (ppm) : 4.70(2H, d, J=5.6Hz), 6.57(1H, brs), 6.94-6.96(1H, m), 7.02-7.04(3H, m), 7.11-7.15(2H, m), 7.32-7.37(3H, m), 7.72-7.74(1 H, m), 7.96-7.98(1 H, m), 8.05-8.07(1 H, m), 8.26(1H, s), 8.61-8.62(1 H, m), 9.32-9.33(1 H, m).

### Reference Example H-1 Quinazoline-6-carboxylic acid 3-phenoxybenzylamide

To a solution of quinazoline-6-carboxylic acid obtained in Preparation Example H-3 (9mg, 0.052mmol) in N,N-dimethylformamide (3mL) were added 3-phenoxybenzylamine described in Preparation Example 4 (11 mg, 0.052mmol), benzotriazol-1-yloxytris(dimethylamino)phosphonium hexafluorophosphate (28mg, 0.062mmol) and triethylamine (17µl, 0.125mmol), and the solution was stirred for 2 days at room temperature. Water was added to the reaction mixture, which was extracted with ethyl acetate, and concentrated. The residue was purified by NH silica gel column chromatography (hexane-ethyl acetate), and the title compound (11 mg, 0.031 mmol, 50%) was obtained.
¹H-NMR Spectrum (CD₃OD) δ (ppm) : 4.62 (2H, s), 6.88 (1H, dd, J=8.0, 1.2Hz), 6.98 (2H, dd, J=1.2, 8.0Hz), 7.09 (1H, s), 7.07 (1H, dd, J=7.6, 8.0Hz), 7.15 (1H, d, J=7.6Hz), 7.29-7.35 (3H, m), 8.10 (1H, d, J=8.8Hz), 8.40 (1H, dd, J=2.0, 8.8Hz), 8.57 (1 H, d, J=2.0Hz), 9.32 (1 H, s), 9.61 (1 H, s).

### Reference Example I-1. Quinoxaline-6-carboxylic acid 3-phenoxybenzylamide

To a solution of quinoxaline-6-carboxylic acid described in Preparation Example I-1 (15mg, 0.063mmol) and 4-phenoxybenzylamine described in Preparation Example 3 (13mg, 0.063mmol) in N,N-dimethylformamide (2mL) were added benzotriazol-1-yloxytris(pyrrolidino)phosphonium hexafluorophosphate (36mg, 0.069mmol) and triethylamine (19µl, 0.14mmol), which was then stirred at room temperature for 24 hours. The reaction mixture was concentrated, the residue was purified by reverse phase high performance liquid chromatography (acetonitrile-water mobile phase (containing 0.1% trifluoroacetic acid) was used), and the title compound (12mg, 0.025mmol, 40%) was obtained as a trifluoroacetic acid salt.
MS m/e(ESI) 356.37(MH⁺)

### Reference Example J-1. [1.8]Naphthylidine-3-carboxylic acid 3-phenoxybenzylamide

Tetrahydrofuran (1mL), methanol (0.1mL) and water (0.1mL) were added to [1,8]naphthylidine-3-carboxylic acid ethyl ester described in Preparation Example J-7 (8.1mg, 0.040mmol) and lithium hydroxide monohydrate (3.4mg, 0.080mmol), and the solution was stirred at 50°C for 1 hour. The solvent was evaporated *in vacuo,* then, the obtained residue and 3-phenoxybenzylamine (5.0mg, 0.025mmol) were reacted according to an analogous method to Reference Example Q-6, and trifluoroacetic acid salt of the title compound (3.7mg, 0.0079mmol, 20%) was obtained.
MS m/e (ESI) 356.3 (MH⁺)

### Reference Example K-1. 2-Methyl-benzoxazole-6-carboxylic acid 3-phenoxybenzylamide

The title compound (22mg, 0.061 mmol, 72%) was obtained from 2-methyl-benzoxazole-6-carboxylic acid described in Preparation Example K-2 (15mg, 0.085mmol) and 4-phenoxybenzylamine described in Preparation Example 3 (17mg, 0.085mmol) according to an analogous method to Reference Example H-1.
¹H-NMR Spectrum (CD₃OD) δ (ppm) : 2.66 (3H, s), 4.56 (2H, s), 6.80-7.15 (6H, m), 7.22-7.78 (3H, m), 7.62-7.68 (1 H, m), 7.81-7.86 (1 H, m), 8.00-8.04 (1 H, m), 9.08(1H, brs).

### Reference Example L-1. Benzothiazole-6-carboxylic acid (5-(3-fluoro-benzyl)-furan-2-ylmethyl)-amide

The title compound (290mg, 0.791mmol, 76%) was obtained from benzothiazole-6-carboxylic acid (188mg, 1.05mmol) and C-(5-(3-fluoro-benzyl)-furan-2-yl)-methylamine described in Preparation Example 84 (236mg, 1.15mmol) according to an analogous method to Reference Example H-1 (with the proviso that only the reaction temperature was changed to 60°C).
¹H-NMR Spectrum (DMSO-d₆) δ (ppm) : 3.96 (2H, s), 4.43 (2H, d, J=5.2Hz), 6.06 (1H, d, J=3.2Hz), 6.20 (1H, d, J=3.2Hz), 7.00-7.09 (3H, m), 7.29-7.36 (1H, m), 7.99 (1 H, dd, J=1.6, 8.4Hz), 8.12 (1 H, d, J=8.4Hz), 8.64 (1 H, d, J=1.6Hz), 9.07 (1 H, t, J=5.2Hz), 9.51 (1H, s).

### Reference Example L-2. Benzothiazole-6-carboxylic acid 4-benzyloxybenzylamide

The title compound (41 mg, 47%) was obtained as a white solid from benzothiazole-6-carboxylic acid (42mg, 0.234mmol) and 4-benzyloxybenzylamine described in Preparation Example 1 (50mg, 0.234mmol) according to an analogous method to Reference Example E-8.
¹H-NMR Spectrum (CDCl₃) δ (ppm) : 4.62(2H, d, J=5.2Hz), 5.08(2H, s), 6.40(1 H, brs), 6.98(2H, d, J=8.8Hz), 7.30-7.35(3H, m), 7.37-7.45(4H, m), 7.85-7.88(1H, m), 8.16(1H, d, J=8.4Hz), 8.49(1H, d, J=1.6Hz), 9.11(1H, s).,

### Reference Example L-3. Benzothiazole-6-carboxylic acid 3-phenoxybenzylamide

To a solution of 3-phenoxybenzylamine described in Preparation Example 4 (33mg, 0.167mmol) and benzothiazole-6-carboxylic acid (30mg, 0.167mmol) in tetrahydrofuran (1 mL) were added benzotriazol-1-yloxytris(dimethylamino)phosphonium hexafluorophosphate (89mg, 0.20mmol) and triethylamine (28µl, 0.20mmol), and the solution was stirred at room temperature for 17 hours. The solvent was evaporated, the residue was purified by NH silica gel column chromatography (hexane : ethyl acetate), and the title compound (37mg, 62%) was obtained as a colorless oil.
¹H-NMR Spectrum (CDCl₃) δ (ppm) : 4.68(2H, d, J=6.0Hz), 6.50(1 H, brs), 6.94(1 H, dd, J=2.0, 8.0Hz), 7.02-7.04(3H, m), 7.11-7.15(2H, m), 7.31-7.37(3H, m), 7.88(1 H, dd, J=1.6, 8.8Hz), 8.18(1 H, d, J=8.8Hz), 8.49(1 H, d, J=1.6Hz), 9.13(1 H, s).

### Reference Example L-4. Benzothiazole-6-carboxylic acid 4-(3-fluoro-benzyloxy)-benzylamide

To a solution of 4-(3-fluorobenzyloxy)-benzylamine described in Preparation Example 6 (129mg, 0.558mmol) and benzothiazole-6-carboxylic acid (100mg, 0.558mmol) in tetrahydrofuran (5mL) were added benzotriazol-1-yloxytris(dimethylamino)phosphonium hexafluorophosphate (296mg, 0.670mmol) and triethylamine (93µl, 0.670mmol), and the solution was stirred overnight at room temperature. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and the title compound (148mg, 68%) was obtained as a white solid.
¹H-NMR Spectrum (CDCl₃) δ (ppm) : 4.64(2H, d, J=5.6Hz), 5.08(2H, s), 6.42(1H, brs), 6.97(2H, d, J=8.8Hz), 7.02(1H, td, J=2.8, 8.4Hz), 7.15-7.21 (2H, m), 7.31-7.38(3H, m), 7.88(1H, dd, J=1.6Hz, 8.4Hz), 8.17(1H, d, J=8.8Hz), 8.50(1H, d, J=1.6Hz), 9.12(1H, s).

### Reference Example L-5. N-Benzothiazol-6-yl-2-(3-phenoxy-phenyl)-acetamide

The title compound (118mg, 95%) was obtained as a colorless oil from 6-aminobenzothiazole (50mg, 3.33mmol) and 3-phenoxyphenylacetic acid (76mg, 3.33mmol) according to an analogous method to Reference Example L-4.
¹H-NMR Spectrum (CDCl₃) δ (ppm) : 3.75(2H, s), 6.97-7.22(6H, m), 7.31 (1 H, brs), 7.34-7.40(4H, m), 8.01 (1 H, d, J=8.8Hz), 8.50(1 H, d, J=2.0Hz), 8.91 (1 H, s).

### Reference Example L-6. Benzothiazole-6-carboxylic acid (5-(3-fluorophenoxy)thiophene-2-ylmethyl) amide

The title compound (100mg, 0.26mmol, 48.2%) was obtained as a white solid from benzotriazole-6-carboxylic acid (96mg, 0.54mmol) and C-(5-(3-fluorophenoxy)thiophen-2-yl)methylamine described in Preparation Example 23 (120mg, 0.54mmol) according to an analogous method to Reference Example Q-6.
¹H-NMR Spectrum (DMSO-d₆) δ (ppm) : 4.57 (2H, d, 5.6Hz), 6.58 (1H, d, J=3.6Hz), 6.83 (1 H, d, J=3.6Hz), 6.89-7.00 (3H, m), 7.39 (1 H, ddd, J=8.0, 8.0, 8.0Hz), 8.00 (1 H, dd, J=1.6, 8.8Hz), 8.14 (1 H, d, J=8.8Hz), 8.66 (1 H, d, J=1.6Hz), 9.27 (1 H, t, J=5.6Hz), 9.51 (1H, s).

### Reference Example L-7. Benzothiazole-6-carboxylic acid (5-phenoxythiophen-2-ylmethyl)-amide

The title compound (97mg, 0.265mmol, 54.0 %) was obtained as a light brown solid from benzothiazole-6-carboxylic acid (87mg, 0.49mmol) and C-(5-phenoxy-thiophen-2-yl)-methylamine described in Preparation Example 26 (100mg, 0.49mmol) according to an analogous method to Reference Example Q-6.
¹H-NMR Spectrum (DMSO-d₆) δ (ppm) : 4.55 (2H, d, J=5.6Hz), 6.49 (1H, d, J=3.6Hz), 6.79 (1 H, d, J=3.6Hz), 7.05-7.15 (3H, m), 7.30-7.40 (2H, m), 7.99 (1 H, dd, J=1.6, 8.8Hz), 8.13 (1H, d, J=8.8Hz), 8.66 (1H, d, J=1.6Hz), 9.25 (1H, t, J=5.6Hz), 9.51 (1H, s).

### Reference Example L-8. Benzothiazole-6-carboxylic acid (5-(3-chloro-benzyl)-thiophen-2-ylmethyl)-amide

The title compound (64mg, 0.16mmol, 47.2%) was obtained as a white solid from C-(5-(3-chloro-benzyl-2-yl)-methylamine described in Preparation Example 45 (80mg,0.34mmol) and benzothiazole-6-carboxylic acid (66mg ,0.37mmol) according to an analogous method to Reference Example Q-6.
¹H-NMR Spectrum (DMSO-d₆) δ (ppm) : 4.06 (2H, s), 4.56 (2H, d, J=5.6Hz), 6.74 (1H, d, J=3.6Hz), 6.84 (1H, d, J=3.6Hz), 7.18-7.34 (4H, m), 7.98 (1H, dd, J=2.0, 8.8Hz), 8.12 (1H, d, J=8.8Hz), 8.64 (1H, d, J=2.0Hz), 9.21 (1H, t, J=5.6Hz), 9.51 (1H, s).

### Reference Example L-9. Benzothiazole-6-carboxylic acid (5-(3-chloro-phenoxy)-thiophen-2-ylmethyl) amide

The title compound (7.28mg) was obtained from C-(5-(3-chloro-phenoxy)-thiophen-2-yl)-methylamine described in Reference Example A-73 (30mg, 0.13mmol) and benzothiazole-6-carboxylic acid (22mg, 0.13mmol) according to an analogous method to Reference Example E-24. Trifluoroacetic acid salt of the title compound (7.28mg) was obtained by reverse phase high performance liquid chromatography (acetonitrile-water mobile phase (containing 0.1% trifluoroacetic acid) was used).
MS m/e (ESI) 401.32(MH⁺)

### Reference Example L-10. Benzothiazole-6-carboxylic acid (5-(2-fluoro-phenoxy)-thiophen-2-ylmethyl)-amide

Trifluoroacetic acid salt of the title compound (10.7mg, 0.021mmol, 14.3%) was obtained as a brown oil from benzothiazole-6-carboxylic acid (27.4mg, 0.15mmol) and C-(5-(2-fluoro-phenoxy)-thiophen-2-yl)-methylamine described in Preparation Example 161 (33.5mg, 0.15mmol) according to an analogous method to Reference Example A-75.
MS m/e (ESI) 385(MH⁺)

### Reference Example L-11. Benzothiazole-6-carboxylic acid ((5-(3-cyano-phenoxy)-thiophen-2-ylmethyl)-amide

To a solution of C-(5-(3-bromophenoxy)-thiophen-2-yl)-methylamine described in Preparation Example 17 (141 mg, 0.496mmol) and benzothiazole-6-carboxylic acid (89mg, 0.496mmol) in tetrahydrofuran (5mL) were added benzotriazol-1-yloxytris(dimethylamino)phosphonium hexafluorophosphate (263mg, 0.595mmol) and triethylamine (0.14mL, 0.992mmol), and the solution was stirred at room temperature for 3 hours. Ethyl acetate and water were added to the reaction solution, which was then partitioned, the organic layer was washed with water, and then, dried over anhydrous magnesium sulfate. The solvent was evaporated, the residue was purified by NH silica gel column chromatography (hexane : ethyl acetate), and a mixture of benzothiazole-6-carboxylic acid (5-(3-bromophenoxy)-thiophene-2-ylmethyl)-amide and debrominated compound (120mg, 53%) was obtained as a yellow oil.
Next, to a solution of the mixture of benzothiazole-6-carboxylic acid (5-(3-bromophenoxy)-thiophene-2ylmethyl)-amide and debrominated compound (120mg, 0.269mmol) in N,N-dimethylformamide (3.0mL) were added zinc cyanide (63mg, 0.538mmol) and tetrakis(triphenylphosphine)palladium(0) (62mg, 0.054mmol) under nitrogen atmosphere, which was then stirred at 140°C for 14 hours, and the solution was stirred at 140°C for 3 hours. The reaction solution was allowed to room temperature, ethyl acetate and water were added to the reaction solution, which was then partitioned, the organic layer was washed with water and dried over anhydrous magnesium sulfate. After removing the solvent, the residue was purified by NH silica gel column chromatography (hexane : ethyl acetate), and the title compound (6.2mg, 6%) was obtained as a colorless oil.
¹H-NMR Spectrum (CDCl₃) δ (ppm) : 4.78(2H, d, J=6.0Hz), 6.48(1H, d, J=4.0Hz), 6.57(1H, brs), 6.83(1H, d, J=4.0Hz), 7.30(1 H, s), 7.32-7.35(1 H, m), 7.39-7.43(2H, m), 7.90(1 H, dd, J=1.6, 8.8Hz), 8.19(1H, d, J=8.8Hz), 8.51 (1 H, d, J=1.6Hz), 9.13(1 H, s).

### Reference Example M-1. Benzo[1,2,5]thiadiazole-5-carboxylic acid 3-phenoxybenzylamide

The title compound (43mg, 71%) was obtained as a colorless solid from 2,1,3-benzothiadiazole-5-carboxylic acid (30mg, 0.167mmol) obtained by hydrolysis of benzo-2,1,3-thiadiazole-5-carboxylic acid methyl ester with sodium hydroxide and 4-phenoxybenzylamine described in Preparation Example 3 (33mg, 0.167mmol) according to an analogous method to Reference Example L-4.
¹H-NMR Spectrum (CDCl₃) δ (ppm) : 4.67(2H, d, J=6.0Hz), 6.65(1H, brs), 6.94(1H, dd, J=2.4Hz, 8.4Hz), 7.01-7.03(3H, m), 7.10-7.14(2H, m), 7.30-7.36(3H, m), 8.01-8.07(2H, m), 8.37(1 H, s).

### Reference Example O-1. 2.3-Dihydro-1H-pyrrolo[2.3-b]pyridine-5-carboxylic acid (5-phenoxy-thiophen-2-ylmethyl)-amide

The title compound (15mg, 43µmol, 47%) was obtained as a white solid from 2,3-dihydro-1H-pyrrolo[2,3-b]pyridine-5-carboxylic acid described in Preparation Example 0-2 (15mg, 91µmol) and C-(5-phenoxy-thiophen-2-yl)-methylamine described in Preparation Example 26 (19mg, 91µmol) according to an analogous method to Reference Example A-26.
¹H-NMR Spectrum (DMSO-d₆) δ (ppm) : 2.99 (2H, t, J=9.0Hz), 3.53 (2H, t, J=8.6Hz), 4.46 (2H, d, J=6.0Hz), 6.49 (1H, d, J=3.8Hz), 6.74 (1H, d, J=3.7Hz), 7.00 (1H, s), 7.09 (2H, d, J=8.6Hz), 7.14 (1 H, t, J=7.5Hz), 7.38 (2H, t, J=7.5Hz), 7.63 (1 H, s), 8.25 (1 H, s), 8.71-8.77 (1H, m).

### Reference Example P-1. Furo[2,3-b]pyridine-5-carboxylic acid (5-phenoxy-thiophen-2-ylmethyl)-amide

Furo[2,3-b]pyridine-5-carboxylic acid (31mg) was obtained as a lithium salt from furo[2,3-b]pyridine-5-carboxylic acid ethyl ester described in Preparation Example P-4 (33mg, 0.17mmol) according to an analogous method to Reference Example T-2.
The title compound (28mg, 80µmol, 79%) was obtained as a white solid from the lithium salt (17mg) of the resulting furo[2,3-b]pyridine-5-carboxylic acid (31 mg) and C-(5-phenoxy-thiophen-2-yl)-methylamine described in Preparation Example 26 (23mg, 0.11 mmol).
¹H-NMR Spectrum (DMSO-d₆) δ (ppm) : 4.57 (2H, d, J=5.9Hz), 6.52 (1H, d, J=3.8Hz), 6.82 (1 H, d, J=3.7Hz), 7.09-7.17 (4H, m), 7.39 (2H, t, J=8.4Hz), 8.21 (1 H, d, J=2.6Hz), 8.58 (1 H, d, J=2.2Hz), 8.79 (1 H, d, J=2.2Hz), 9.34 (1 H, t, J=6.0Hz).

### Reference Example Q-1. Imidazo[1,2-a]pyridine-6-carboxylic acid (5-benzyl-furan-2-ylmethyl)-amide

7N Ammonia/methanol(40mL) and Raney nickel(3g) were added to 5-benzyl-furan-2-carbaldehyde described in Preparation Example 39 (2.5g, 13mmol), and the solution was stirred for 22 hours under hydrogen atmosphere at room temperature. After removing the catalyst by filtering through Celite pad, the solvent was evaporated *in vacuo* and C-(5-benzyl-furan-2-yl)methylamine (1.6g, 8.6mmol,65.8%) was obtained.
The title compound (150mg, 0.45mmol, 45.3%) was obtained as a white solid from the resulting C-(5-benzylfuran-2-yl)methylamine (200mg, 1.07mmol) and imidazo[1,2-a]pyridine-6-carboxylic acid (170mg, 1.07mmol) according to an analogous method to Reference Example Q-6.
¹H-NMR Spectrum (DMSO-d₆) δ (ppm) : 3.92 (2H, s), 4.41 (2H, d, J=5.2Hz), 6.01 (1H, d, J=2.4Hz), 6.19 (1H, d, J=2.4Hz), 7.16-7.30 (5H, m), 7.56-7.66 (3H, m), 8.03 (1 H, s), 9.00 (1 H, t, J=5.2Hz), 9.10 (1 H, s).

### Reference Example Q-2. Imidazo[1,2-a]pyridine-6-carboxylic acid (5-(3-fluoro-benzyl)-furan-2 ylmethyl)-amide

The title compound (363mg, 1.04mmol, 90%) was obtained from imidazo[1,2-a]pyridine-6-carboxylic acid (188mg, 1.16mmol) and C-(5-(3-fluoro-benzyl)-furan-2-yl)-methylamine described in Preparation Example 84 (286mg, 1.39mmol) according to an analogous technique to Reference Example H-1 (with the proviso that only the reaction temperature was changed to 60°C).
¹H-NMR Spectrum (DMSO-d₆) δ (ppm) : 3.97 (2H, s), 4.42 (2H, d, J=5.2Hz), 6.07 (1H, d, J=3.2Hz), 6.21 (1H, d, J=3.2Hz), 7.00-7.08 (3H, m), 7.29-7.37 (1H, m), 7.56-7.66 (3H, m), 8.04 (1 H, s), 9.01 (1 H, t, J=5.2Hz), 9.10 (1 H, dd, J=1.2, 1.6Hz).

### Reference Example Q-3. Imidazo[1,2-a]pyridine-6-carboxylic acid 4-benzyloxybenzylamide

The title compound (121mg, 55%) was obtained as a white solid from imidazo[1,2-a]pyridine-6-carboxylic acid (100mg, 0.617mmol) and 4-benzyloxybenzylamine described in Preparation Example 1 (132mg, 0.617mmol) according to an analogous method to Reference Example E-8.
¹H-NMR Spectrum (CDCl₃) δ (ppm) : 4.59(2H, d, J=5,6Hz), 5.07(2H, s), 6.44(1H, brs), 6.96-6.98(2H, m), 7.27-7.30(3H, m), 7.33-7.44(5H, m), 7.59-7.61(1H, m), 7.65-7.66(1 H, m), 7.69-7.70(1 H, m), 8.83-8.84(1 H, m).

### Reference Example Q-4. Imidazo[1,2-a]pyridine-6-carboxylic acid 3-phenoxybenzylamide

The title compound (22mg, 35%) was obtained as a colorless oil from imidazo[1,2-a]pyridine-6-carboxylic acid (30mg, 0.185mmol) and 4-phenoxybenzylamine described in Preparation Example 3 (37mg, 0.185mmol) according to an analogous method to Reference Example L-3.
¹H-NMR Spectrum (CDCl₃) δ (ppm) : 4.64(2H, d, J=5.6Hz), 6.60(1H, brs), 6.92-6.94(1H, m), 7.00-7.02(3H, m), 7.08-7.14(2H, m), 7.30-7.41 (4H, m), 7.59-7.61 (1 H, m), 7.69(1 H, s), 7.69-7.70(1 H, m), 8.83(1 H, s).

### Reference Example Q-5. Imidazo[1,2-a]pyridine-6-carboxylic acid 4-(3-fluoro-benzyloxy)-benzylamide

The title compound (64mg, 45%) was obtained as a white solid from 4-(3-fluorobenzyloxy)-benzylamine described in Preparation Example 6 (87mg, 0.376mmol) and imidazo[1,2-a]pyridine-6-carboxylic acid (61mg, 0.376mmol) according to an analogous method to Reference Example L-4.
¹H-NMR Spectrum (CDCl₃) δ (ppm) : 4.60(2H, d, J=5.6Hz), 5.07(2H, s), 6.44(1H, brs), 6.96(2H, d, J=8.8Hz), 7.02(1H, dt, J=2.4, 8.4Hz), 7.15(1 H, d, J=9.6Hz), 7.19(1 H, d, J=8.4Hz), 7.30(2H, d, J=8.4Hz), 7.32-7.40(2H, m), 7.61(1H, d, J=9.6Hz), 7.67(1H, s), 7.70(1 H, s), 8.84(1H, s).

### Reference Example Q-6. Imidazo[1,2-a]pyridine-6-carboxylic acid (5-(3-fluorophenoxy)thiophen-2-ylmethyl) amide

To a solution of imidazo[1,2-a]pyridine-6-carboxylic acid (87mg, 0.54mmol) and C-(5-(3-fluorophenoxy)thiophen-2-yl)methylamine described in Preparation Example 23 (120mg, 0.54mmol) in N,N-dimethylformamide (5mL) were added benzotriazol-1-yl-tris(dimethylamino)phosphonium hexafluorophosphate (240mg, 0.54mmol) and triethylamine (0.15mL, 1.08mmol), and the solution was stirred for 40 minutes at 80°C. Water and ethyl acetate were added to the reaction solution, which was then partitioned, and the organic layer was washed with water twice. Silica gel was added to the organic layer, the solvent was evaporated *in vacuo* for adsorption, purification was carried out by silica gel column chromatography (hexane : ethyl acetate = 1 : 1, then ethyl acetate), and the title compound (90mg, 0.25mmol, 45.4%) was obtained as a light brown oil.
¹H-NMR Spectrum (DMSO-d₆) δ (ppm) : 4.55 (2H, d, J=5.6Hz), 6.58 (1H, d, J=4.0Hz), 6.83 (1 H, d, J=4.0Hz), 6.90-7.00 (3H, m), 7.40 (1 H, ddd, J=8.0, 8.0, 8.0Hz), 7.57-7.66 (3H, m), 8.04 (1 H, s), 9.12 (1 H, d, J=0.8Hz), 9.20 (1 H, t, J=5.6Hz).

### Reference Example Q-7. Imidazo[1,2-a]pyridine-6-carboxylic acid (5-phenoxy-thiophen-2-ylmethyl)-amide

The title compound (160mg, 0.458mmol, 93.5 %) was obtained as a light brown oil from imidazo[1,2-a]pyridine-6-carboxylic acid (80mg, 0.49mmol) and C-(5-phenoxythiophen-2-yl)methylamine described in Preparation Example 26 (100mg, 0.49mmol) according to an analogous method to Reference Example Q-6.
¹H-NMR Spectrum (DMSO-d₆) δ (ppm) : 4.53 (2H, d, J=5.6Hz), 6.50 (1H, d, J=4.0Hz), 6.79 (1 H, d, J=4.0Hz), 7.04-7.15 (3H, m), 7.32-7.40 (2H, m), 7.56-7.66 (3H, m), 8.03 (1 H, s), 9.08-9.13 (1 H, m), 9.19 (1 H, t, J=5.6Hz).

### Reference Example Q-8. Imidazo[1,2-a]pyridine-6-carboxylic acid (5-(3-chloro-phenoxy)-thiophen-2-ylmethyl)-amide

To a solution of C-(5-(3-chloro-phenoxy)-thiophen-2-yl)-methylamine described in Preparation Example 167 (104mg, 0.434mmol) and imidazo[1,2-a]pyridine-6-carboxylic acid (77mg, 0.477mmol) in N,N-dimethylformamide (3mL) were added benzotriazol-1-yloxy tris(dimethylamino)phosphonium hexafluorophosphate (250mg, 0.564mmol) and triethylamine (181µL) at room temperature, and the solution was stirred at room temperature for 4 hours. Water was added to the reaction solution, which was then extracted with ethyl acetate, and the organic layer was washed with water and brine. Anhydrous magnesium sulfate was added to the organic layer for drying, filtration was carried out, then, the solvent was evaporated *in vacuo,* the residue was purified by NH silica gel column chromatography (ethyl acetate / methanol =40/1), and the title compound (149mg, 89%) was obtained as a white solid.
¹H-NMR Spectrum (DMSO-d₆) δ (ppm) : 4.55(2H, d, J=5.3Hz), 6.59(1 H, dd, J=1.1, 3.7Hz), 6.83(1H, d, J=3.7Hz), 7.06(1H, dd, J=1.1, 8.2Hz), 7.13(1H, s), 7.20(1H, d, J=8.1 Hz), 7.40(1H, t, 8.1Hz), 7.58-7.64(3H, m), 8.05(1H, s), 9.12(1H, s), 9.23(1H, t, J=5.5Hz).

### Reference Example R-1. 1 H-Pyrrolo[2,3-b]pyridine-5-carboxylic acid (5-phenoxy-thiophen-2-ylmethyl)-amide

The title compound (22mg, 63µmol,68%) was obtained from 1*H*-pyrrolo[2,3-b]pyridine-5-carboxylic acid described in Preparation Example R-7 (15mg, 93µmol) and C-(5-phenoxy-thiophen-2-yl)-methylamine described in Preparation Example 26 (19mg, 93µmol) according to an analogous method to Reference Example A-26. Trifluoroacetic acid salt of the title compound was obtained by reverse phase high performance liquid chromatography (acetonitrile-water mobile phase (containing 0.1 % trifluoroacetic acid) was used).
MS m/e (ESI) 350.26(MH⁺)

### Example R-2. 6-Amino-1H-pyrrolo[2,3-b]pyridine-5-carboxylic acid (5-phenoxy-thiophen-2-ylmethyl)-amide

To a solution of 6-amino-1*H*-pyrrolo[2,3-b]pyridine-5-carboxylic acid ethyl ester described in Preparation Example R-2 (95mg, 0.46mmol) in ethanol (10mL) was added an aqueous solution of 1N sodium hydroxide (5mL, 5mmol), which was then heated for 3 hours in an oil bath at 98°C. After cooling the reaction solution, the reaction solution was concentrated until it reached 1/3 of its volume, neutralized with 1 N hydrochloric acid, and further concentrated. The resulting crude product was suspended in N,N-dimethylformamide (5mL), triethylamine (0.096mL, 0.69mmol), benzotriazol-1-yloxytris(dimethylamino)phosphonium hexafluorophosphate (153mg, 0.35mmol) and C-(5-phenoxy-thiophen-2-yl)-methylamine described in Preparation Example 26 (71 mg, 0.35mmol) were added thereto, followed by stirring at room temperature for 15 hours. After the reaction was completed, the reaction solution was poured into brine, and extracted with ethyl acetate. The organic layer was dried over anhydrous magnesium sulfate, then concentrated, the obtained residue was purified by silica gel column chromatography (ethyl acetate : hexane = 2 : 1), and the title compound (31 mg, 0.085mmol, 18.5%) was obtained.
¹H-NMR Spectrum (DMSO-d₆) δ (ppm) : 4.49 (2H, d, J=5.6Hz), 6.24 (1H, dd, J=2.0, 3.6Hz), 6.50 (1H, d, J=4.0Hz), 6.78 (1H, d, J=4.0Hz), 6.80 (2H, brs), 7.04 (1H, dd, J=2.0, 3.2Hz), 7.07-7.16 (3H, m), 7.35-7.41 (2H, m), 8.14 (1 H, s), 8.91-8.95 (1 H, m), 11.0(1H, brs).

### Reference Example S-1. Pyrrolo[3,2-b]pyridine-1-carboxylic acid 3-phenoxy-benzylamide

1*H*-Pyrrolo[3,2-b]pyridine described in Preparation Example S-4 (44mg, 0.37mmol) was dissolved in N,N-dimethylformamide (3mL), sodium hydride (18mg, 0.45mmol, 60% in oil) was added thereto, followed by stirring at room temperature for 30 minutes. Next, (4-phenoxy-benzyl)-carbamic acid phenyl ester described in Preparation Example 75 (143mg, 0.45mmol) was added, and the solution was stirred at room temperature for 2 hours. After the reaction was completed, the reaction solution was poured into brine, which was then extracted with ethyl acetate, and concentrated. The obtained residue was purified by silica gel column chromatography (ethyl acetate : hexane = 1 : 1), and the title compound (11mg, 0.032mmol, 8.7%) was obtained.
¹H-NMR Spectrum (DMSO-d₆) δ (ppm) : 4.16 (2H, d, J=6.0 Hz), 6.49 (1H, d, J=3.6 Hz), 6.56 (1 H, dd, J=2.0, 8.0 Hz), 6.65-6.73 (3H, m), 6.77-6.85 (2H, m), 6.93 (1 H, dd, J=4.4, 8.0 Hz), 7.01-7.06 (3H, m), 7.85 (1 H, d, J=3.6 Hz), 8.10-8.17 (2H, m), 8.58 (1 H, t, J=6.0 Hz).

### Reference Example T-1. 6-Amino-thieno[2,3-b]pyridine-5-carboxylic acid (5-phenoxy-thiophen-2-ylmethyl)-amide

The title compound (30mg,78µmol,76%) was obtained as a white solid from 6-amino-thieno[2,3-b]pyridine-5-carboxylic acid described in Preparation Example T-6 (20mg, 0.10mmol) and C-(5-phenoxy-thiophen-2-yl)-methylamine described in Preparation Example 26 (21 mg, 0.10mmol) according to an analogous method to Reference Example A-26.
MS m/e (ESI) 382.35(MH⁺)
¹H-NMR Spectrum (DMSO-d₆) δ (ppm) : 4.52 (2H, d, J=6.0Hz), 6.52 (1H, d, J=3.7Hz), 6.81 (1H, d, J=3.7Hz), 7.09-7.19 (6H, m), 7.33 (1H, dd, J=1.1,5.9Hz), 7.39 (2H, t, J=8.6Hz), 8.34 (1 H, s), 9.21 (1 H, m).

### Reference Example T-2. Thieno[2,3-b]pyridine-5-carboxylic acid (5-phenoxy-thiophen-2-ylmethyl)-amide

Thieno[2,3-b]pyridine-5-carboxylic acid methyl ester described in Preparation Example T-10 (4.0mg, 21µmol) and lithium hydroxide monohydrate (0.9mg, 21µmol) were dissolved in a mixture solvent of tetrahydrofuran (0.5mL), methanol (50µl) and water (50µl), and the solution was heated under reflux for 1 hour. The reaction solution was cooled to room temperature, then, removal *in vacuo* was carried out, and thieno[2,3-b]pyridine-5-carboxylic acid was obtained as a lithium salt.
Then, the lithium salt of the resulting thieno[2,3-b]pyridine-5-carboxylic acid, C-(5-phenoxy-thiophen-2-yl)-methylamine described in Preparation Example 26 (4.7mg, 23µmol), benzotriazol-1-yl-tris(dimethylamino)phosphonium hexafluorophosphate (14mg, 32µmol) and triethylamine (9µl,63µmol) were dissolved in N,N-dimethylformamide (0.5mL), and the solution was stirred at room temperature for 2 hours. Water and ethyl acetate were added to the reaction solution, the organic layer was partitioned, and washed with brine. The solvent was evaporated *in vacuo,* the residue was purified by silica gel column chromatography (hexane : ethyl acetate = 1 : 1), and the title compound (3.0mg, 8.2µmol, 40%) was obtained as a white solid.
¹H-NMR Spectrum (CDCl₃) δ (ppm) : 4.75 (2H, d, J=5.2Hz), 6.40 (1H, d, J=3.6Hz), 6.77 (1H, s), 6.93 (1 H, d, J=3.2Hz), 7.08-7.12 (3H, m), 7.30-7.34 (3H, m), 7.62 (1H, d, J=6.4Hz), 8.51 (1 H, d, J=2.0Hz), 8.92 (1 H, d, J=2.0Hz).

### Reference Example U-1. 5-Amino-thieno[3,2-b]pyridine-6-carboxylic acid (5-phenoxy-thiophen-2-ylmethyl)-amide

The title compound (85mg, 0.22mmol, 87%) was obtained as a white solid from 5-amino-thieno[3,2-b]pyridine-6-carboxylic acid described in Preparation Example U-4 (50mg, 0.26mmol) and C-(5-phenoxy-thiophen-2-yl)-methylamine described in Preparation Example 26 (53mg, 0.26mmol) according to an analogous method to Reference Example A-26.
¹H-NMR Spectrum (DMSO-d₆) δ (ppm) : 4.52 (2H, d, J=5.7Hz), 6.52 (1 H, d, J=3.7Hz), 6.82 (1 H, d, J=3.8Hz), 6.99 (2H, s), 7.10 (2H, d, J=7.5Hz), 7.15 (1 H, t, J=7.3Hz), 7.18 (1 H, d, J=5.5Hz), 7.39 (2H, dd, J=7.3, 8.8Hz), 8.08 (1 H , d, J=5.5Hz), 8.49 (1 H, s), 9.17 (1 H, t, J=5.5Hz).

### Reference Example U-2. Thieno[3,2-b]pyridine-6-carboxylic acid (5-phenoxy-thiophen-2-ylmethyl)-amide

The title compound (7mg, 19µmol, 89%) was obtained as a white solid from trifluoromethanesulfonic acid 6-((5-phenoxy-thiophen-2-ylmethyl)-carbamoyl)-thieno[3,2-b]pyridin-5-yl ester described in Preparation Example U+-2 (11mg,21µmol) according to an analogous method to Preparation Example T-10.
¹H-NMR Spectrum (DMSO-d₆) δ (ppm) : 4.57 (2H, d, J=5.7Hz), 6.50 (1H, d, J=3.7Hz), 6.81 (1H, d, J=3.7Hz), 7.08 (2H, d, J=8.1Hz), 7.12 (1H, t, J=7.5Hz), 7.36 (2H, t, J=7.7Hz), 7.62 (1 H, d, J=5.7Hz), 8.31 (1 H, d, J=5.5Hz), 8.90 (1 H, s), 9.08 (1 H, d, J=2.0Hz), 9.32-9.38 (1 H, m).

### Reference Example V-1. 1H-Indole-5-carboxylic acid 3-phenoxybenzylamide

The title compound was obtained from 1*H*-indole-5-carboxylic acid and 4-phenoxybenzylamine described in Preparation Example 4 according to an analogous method to Reference Example H-1.
MS m/e(ESI) 343.15(MH⁺)

The structural formulae of the compounds obtained in the above Reference Examples and examples are shown in the following Table 2 to Table 5.

**[Table 2]**

| Reference Example A-4 | |
|---|---|
| 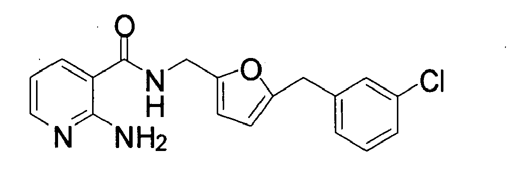 | |

| Reference Example A-5 | Reference Example A-64 |
|---|---|
| 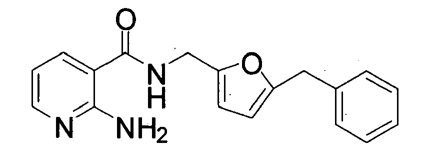 | 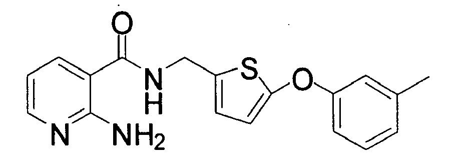 |

| Reference Example A-6 | Reference Example A-65 |
|---|---|
| 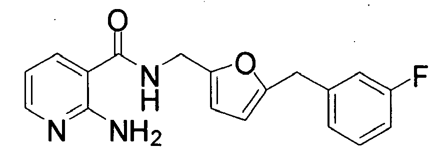 | 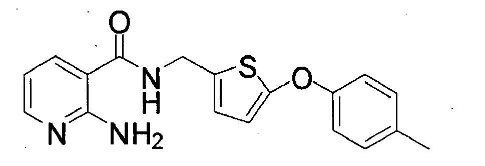 |

| Reference Example A-53 | Reference Example A-66 |
|---|---|
| 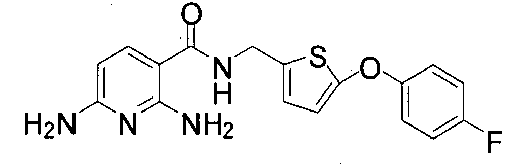 | 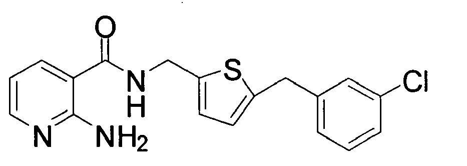 |

| Reference Example A-54 | Reference Example A-67 |
|---|---|
| 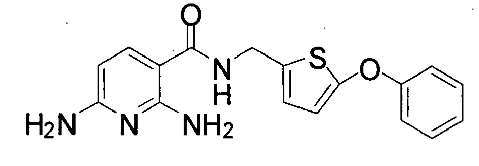 | 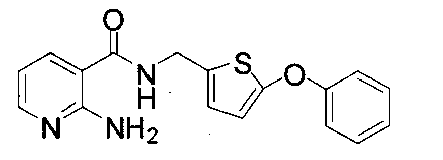 |

| Reference Example A-62 | Reference Example A-68 |
|---|---|
| 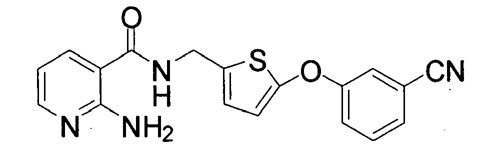 | 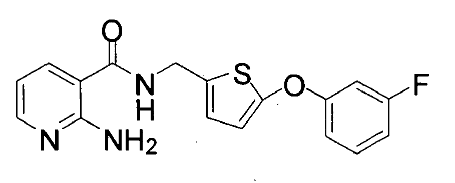 |

**[Table 3]**

| Reference Example A-69 | Reference Example A-75 |
|---|---|
| 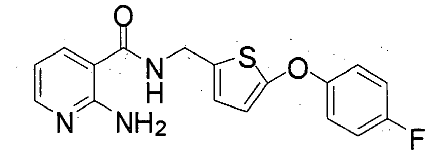 | 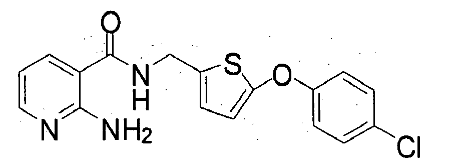 |

| Reference Example A-70 | Reference Example A-76 |
|---|---|
| 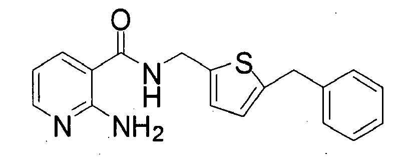 | 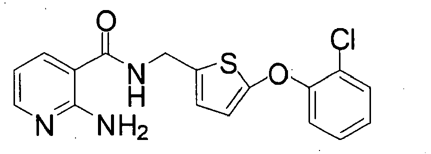 |

| Reference Example A-71 | Reference Example A-98 |
|---|---|
| 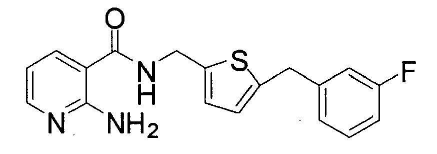 | 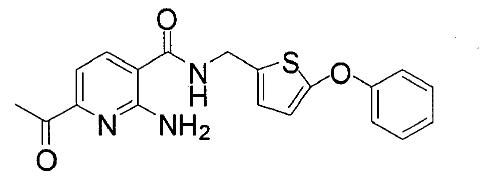 |

| Reference Example A-72 | Reference Example A-105 |
|---|---|
| 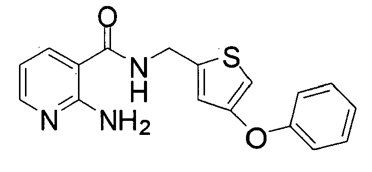 | 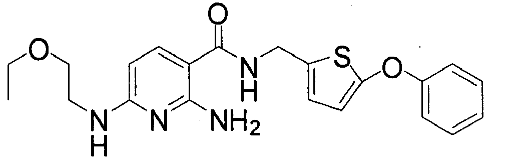 |

| Reference Example A-73 | Reference Example A-119 |
|---|---|
| 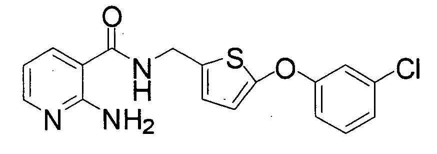 | 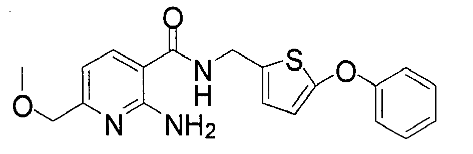 |

| Reference Example A-74 | Reference Example A-122 |
|---|---|
| 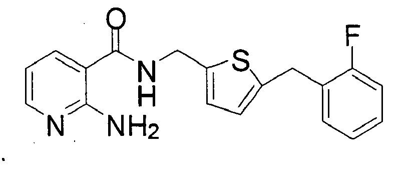 | 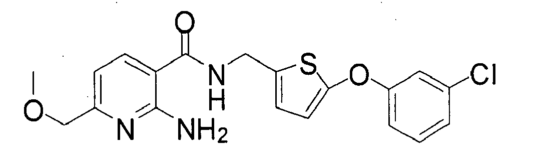 |

**[Table 4]**

| Reference Example A-123 | Reference Example A-183 |
|---|---|
| 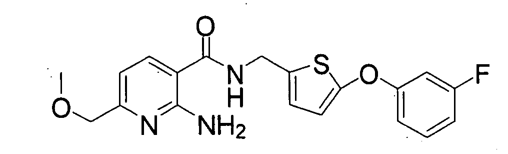 | 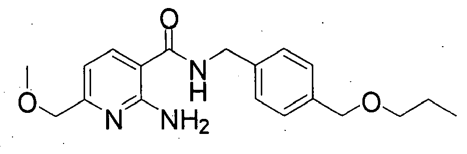 |

| Reference Example A-168 | Reference Example A-188 |
|---|---|
| 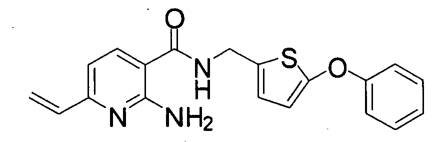 | 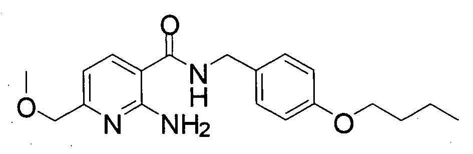 |

| Reference Example A-173 | |
|---|---|
| 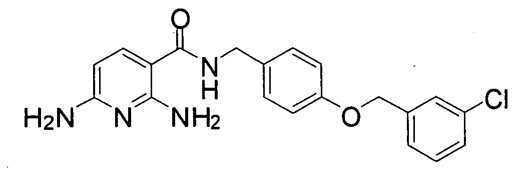 | |

| Reference Example A-177 | |
|---|---|
| 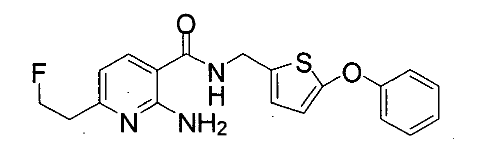 | |

| Reference Example A-182 | |
|---|---|
| 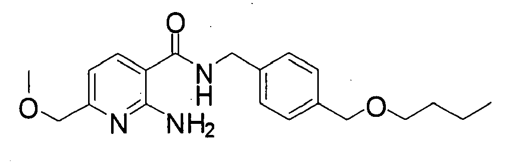 | |

**[Table 5]**

| Reference Example E-10 | Reference Example E-68 |
|---|---|
| 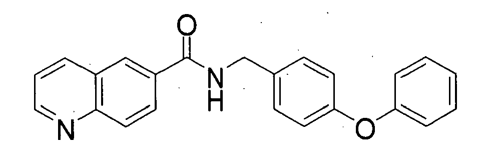 | 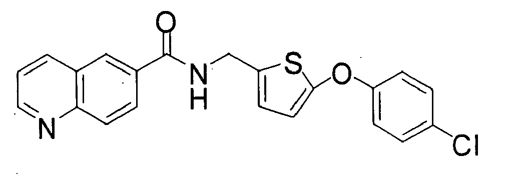 |

| Reference Example E-11 | |
|---|---|
| 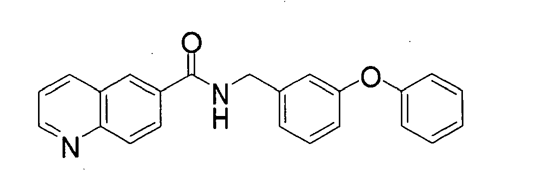 | |

| Reference Example E-64 | |
|---|---|
| 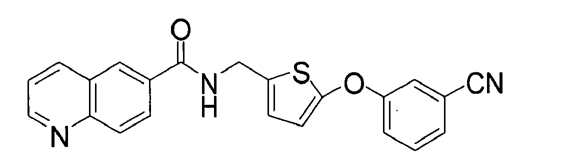 | |

| Reference Example E-65 | |
|---|---|
| 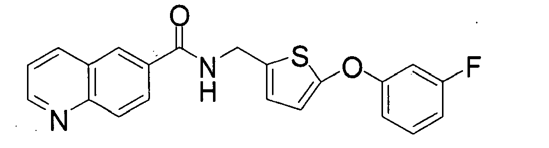 | |

| Reference Example E-66 | |
|---|---|
| 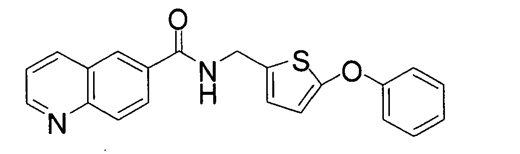 | |

| Reference Example E-67 | |
|---|---|
| 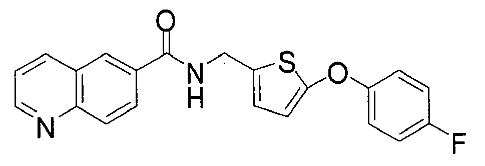 | |

### Industrial Applicability

The compounds represented by the formula (I-a) or salts thereof, or hydrates thereof are very useful for the antimalarial agents.

## Claims

1. An antimalarial agent containing a compound represented by the formula: 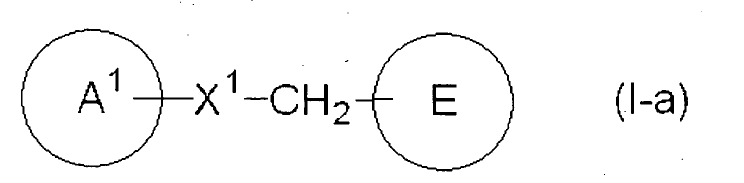 [wherein A¹ represents a 3-pyridyl group or a 6-quinolyl group;
X¹ represents a group represented by the formula -C(=Y¹)-NH-;
Y¹ represents an oxygen atom;
E represents a furyl group, a thienyl group or a phenyl group; with the proviso that A¹ may have one to three substituents selected from the following Substituent Groups a-1 and a-2, and E has one or two substituents selected from the following Substituent Groups a-1 and a-2] or a salt thereof, or hydrates thereof,
[Substituent Group a-1]
a halogen atom, a hydroxyl group, a mercapto group, a cyano group, a carboxyl group, an amino group, a carbamoyl group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₃₋₈ cycloalkylydene C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkoxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₁₋₆ alkylthio group, a C₂₋₆ alkenylthio group, a C₂₋₆ alkynylthio group, a C₃₋₈ cycloalkylthio group, a C₆₋₁₀ arylthio group, a C₃₋₈ cycloalkyl C₁₋₆ alkylthio group, a C₆₋₁₀ aryl C₁₋₆ alkylthio group, a 5 to 10-membered heterocyclic C₁₋₆ alkylthio group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a di-C₁₋₆ alkylamino group, a N-C₂₋₆ alkenyl-N-C₁₋₆ alkylamino group, a N-C₂₋₆ alkynyl-N-C₁₋₆ alkylamino group, a N-C₃₋₈ cycloalkyl-N-C₁₋₆ alkylamino group, a N-C₆₋₁₀ aryl-N-C₁₋₆ alkylamino group, a N-C₃₋₈ cycloalkyl C₁₋₆ alkyl-N-C₁₋₆ alkylamino group, a N-C₆₋₁₀ aryl C₁₋₆ alkyl-N-C₁₋₆ alkylamino group, a N-5 to 10-membered heterocyclic C₁₋₆ alkyl-N-C₁₋₆ alkylamino group, a C₁₋₆ alkylcarbonyl group, a C₁₋₆ alkoxycarbonyl group, a C₁₋₆ alkylsulfonyl group and a group represented by the formula -C(=N-R^{a1})R^{a2} (wherein R^{a1} represents a hydroxyl group or a C₁₋₆ alkoxy group; and R^{a2} represents a C₁₋₆ alkyl group);
[Substituent Group a-2]
a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkoxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₁₋₆ alkylthio group, a C₂₋₆ alkenylthio group, a C₂₋₆ alkynylthio group, a C₃₋₈ cycloalkylthio group, a C₆₋₁₀ arylthio group, a C₃₋₈ cycloalkyl C₁₋₆ alkylthio group, a C₆₋₁₀ aryl C₁₋₆ alkylthio group, a 5 to 10-membered heterocyclic C₁₋₆ alkylthio group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a di-C₁₋₆ alkylamino group, a N-C₂₋₆ alkenyl-N-C₁₋₆ alkylamino group, a N-C₂₋₆ alkynyl-N-C₁₋₆ alkylamino group, a N-C₃₋₈ cycloalkyl-N-C₁₋₆ alkylamino group, a N-C₆₋₁₀ aryl-N-C₁₋₆ alkylamino group, a N-C₃₋₈ cycloalkyl C₁₋₆ alkyl-N-C₁₋₆ alkylamino group, a N-C₆₋₁₀ aryl C₁₋₆ alkyl-N-C₁₋₆ alkylamino group and a N-5 to 10-membered heterocyclic C₁₋₆ alkyl-N-C₁₋₆ alkylamino group;
with the proviso that each group mentioned in Substituent Group a-2 has one to three substituents selected from the following Substituent Group b;
[Substituent Group b]
a halogen atom, a hydroxyl group, a mercapto group, a cyano group, a carboxyl group, an amino group, a carbamoyl group, a nitro group, a C₁₋₆ alkyl group, a C₃₋₈ cycloalkyl group, a C₆₋₁₀ aryl group, a 5 to 10-membered heterocyclic group, a C₁₋₆ alkoxy group, a C₆₋₁₀ aryloxy group, a 5 to 10-membered heterocyclic oxy group, a C₁₋₆ alkylcarbonyl group, a C₁₋₆ alkoxycarbonyl group, a C₁₋₆ alkylsulfonyl group, a trifluoromethyl group, a trifluoromethoxy group, a mono-C₁₋₆ alkylamino group, a di-C₁₋₆ alkylamino group, a mono-C₆₋₁₀ arylamino group that may have one amino group, a N-C₆₋₁₀ aryl C₁₋₆ alkyl-N-C₁₋₆ alkylamino group that may have one amino group, a trifluoromethyl group, a trifluoromethoxy group and a C₁₋₆ alkyl group.

2. The antimalarial agent according to Claim 1, wherein A¹ represents a 3-pyridyl group (with the proviso that A¹ may have one to three substituents selected from the following Substituent Groups c-1 and c-2),
[Substituent Group c-1]
a halogen atom, an amino group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a C₁₋₆ alkylcarbonyl group and a group represented by the formula -C(=N-OH)R^{a2} (wherein R^{a2} has the same meaning as defined above);
[Substituent Group c-2]
a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group and a mono-5 to 10-membered heterocyclic C₁₋₆ alkylamino group;
with the proviso that each group mentioned in Substituent Group c-2 has one to three substituents selected from the following Substituent Group d;
[Substituent Group d]
a halogen atom, a hydroxyl group, a carboxyl group, an amino group, a carbamoyl group, a C₁₋₆ alkoxy group, a mono-C₁₋₆ alkylamino group, a di-C₁₋₆ alkylamino group, a mono-C₆₋₁₀ arylamino group that may have one amino group and a N-C₆₋₁₀ aryl C₁₋₆ alkyl-N-C₁₋₆ alkylamino group that may have one amino group.

3. The antimalarial agent according to Claim 1, wherein A¹ represents a 3-pyridyl group (with the proviso that A¹ may have one to three substituents selected from the following Substituent Groups c'-1 and c'-2),
[Substituent Group c'-1]
a halogen atom, an amino group, a C₁₋₆ alkyl group, a C₂₋₆ alkenyl group, a C₂₋₆alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group, a mono-C₂₋₆ alkynylamino group and a C₁₋₆ alkylcarbonyl group;
[Substituent Group c'-2]
a C₁₋₆alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group and a mono-C₂₋₆ alkynylamino group;
with the proviso that each group mentioned in Substituent Group c'-2 has one to three substituents selected from the following Substituent Group d';
[Substituent Group d']
a halogen atom, a hydroxyl group, a carboxyl group, an amino group, a carbamoyl group and a C₁₋₆ alkoxy group.

4. The antimalarial agent according to Claim 1, wherein A¹ represents a group represented by the formula: 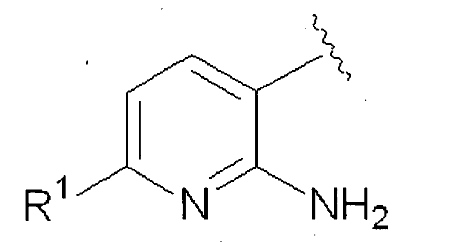 (wherein R¹ represents a hydrogen atom, a substituent selected from the above Substituent Group c-1 or a substituent selected from the above Substituent Group c-2).

5. The antimalarial agent according to Claim 1, wherein A¹ represents a group represented by the formula: 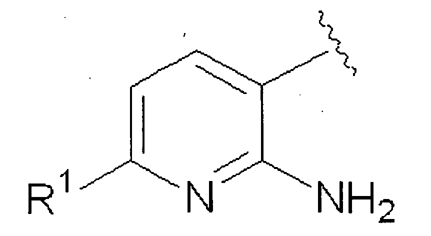 (wherein R¹ represents a hydrogen atom, a substituent selected from the above Substituent Group c'-1 or a substituent selected from the above Substituent Group c'-2).

6. The antimalarial agent according to Claim 1, wherein A¹ represents a group represented by the formula: 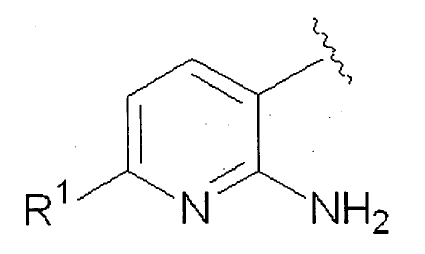 (wherein R¹ represents a hydrogen atom, an amino group, a C₁₋₆ alkyl group that may have one C₁₋₆ alkoxy group, a C₂₋₆ alkenyl group, a mono-C₁₋₆ alkylamino group that may have one C₁₋₆ alkoxy group or a C₁₋₆ alkylcarbonyl group).

7. The antimalarial agent according to Claim 1, wherein A¹ represents a group represented by the formula: 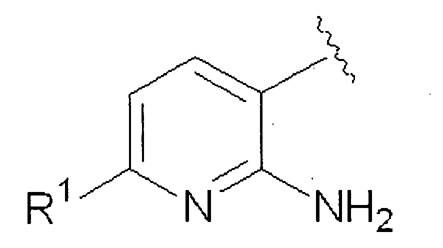 (wherein R¹ represents a C₁₋₆ alkyl group having one halogen atom).

8. The antimalarial agent according to Claim 1, wherein A¹ represents a group represented by the formula: 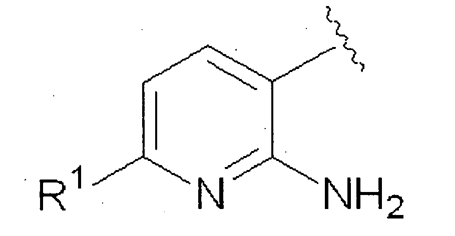 (wherein R¹ represents a hydrogen atom, an amino group, a methoxymethyl group, an ethenyl group, an ethoxyethylamino group or a methylcarbonyl group).

9. The antimalarial agent according to Claim 1, wherein A¹ represents a group represented by the formula: 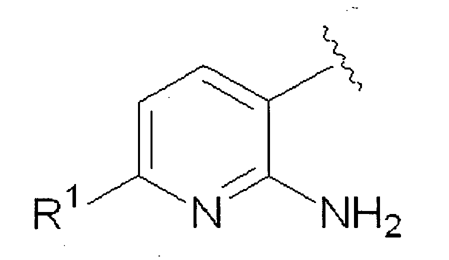 (wherein R¹ represents a fluoroethyl group).

10. The antimalarial agent according to Claim 1, wherein A¹ represents a 6-quinolyl group (with the proviso that A¹ may have one to three substituents selected from the following Substituent Groups c-1 and c-2),
[Substituent Group c-1]
a halogen atom, an amino group, a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkyl amino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group, a mono-5 to 10-membered heterocyclic C₁₋₆ alkylamino group, a C₁₋₆ alkylcarbonyl group and a group represented by the formula -C(=N-OH)R^{a2} (wherein R^{a2} has the same meaning as defined above);
[Substituent Group c-2]
a C₁₋₆ alkyl group, a C₂₋₆ alkenyl group, a C₂₋₆ alkynyl group, a C₃₋₈ cycloalkyl group, a C₆₋₁₀ aryl group, a 5 to 10-membered heterocyclic group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a mono-C₁₋₆ alkylamino group, a mono-C₂₋₆ alkenylamino group, a mono-C₂₋₆ alkynylamino group, a mono-C₃₋₈ cycloalkylamino group, a mono-C₆₋₁₀ arylamino group, a mono-C₃₋₈ cycloalkyl C₁₋₆ alkylamino group, a mono-C₆₋₁₀ aryl C₁₋₆ alkylamino group and a mono-5 to 10-membered heterocyclic C₁₋₆ alkylamino group;
with the proviso that each group mentioned in Substituent Group c-2 has one to three substituents selected from the following Substituent Group d;
[Substituent Group d]
a halogen atom, a hydroxyl group, a carboxyl group, an amino group, a carbamoyl group, a C₁₋₆ alkoxy group, a mono-C₁₋₆ alkylamino group, a di-C₁₋₆ alkylamino group, a mono-C₆₋₁₀ arylamino group that may have one amino group and a N-C₆₋₁₀ aryl C₁₋₆ alkyl-N-C₁₋₆ alkylamino group that may have one amino group.

11. The antimalarial agent according to Claim 1, wherein A¹ represents a 6-quinolyl group (with the proviso that A¹ may have one to three substituents selected from the following Substituent Groups c'-1 and c'-2),
[Substituent Group c'-1]
a halogen atom, an amino group, a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group, a mono-C₂₋₆ alkynylamino group and a C₁₋₆ alkylcarbonyl group;
[Substituent Group c'-2]
a C₁₋₆ alkyl group, a C₂₋₆ alkenyl group, a C₂₋₆ alkynyl group, a C₁₋₆ alkoxy group, a C₂₋₆ alkenyloxy group, a C₂₋₆ alkynyloxy group, a mono-C₁₋₆ alkylamino group, a mono-C₂₋₆ alkenylamino group and a mono-C₂₋₆ alkynylamino group;
with the proviso that each group mentioned in Substituent Group c'-2 has one to three substituents selected from the following Substituent Group d';
[Substituent Group d']
a halogen atom, a hydroxyl group, a carboxyl group, an amino group, a carbamoyl group and a C₁₋₆ alkoxy group.

12. The antimalarial agent according to Claim 1, wherein A¹ represents a 6-quinolyl group.

13. The antimalarial agent according to any one of Claims 1 to 12, wherein E represents a furyl group, a thienyl group or a phenyl group (with the proviso that E has one or two substituents selected from the following Substituent Groups e-1 and e-2),
[Substituent Group e-1]
a halogen atom, a hydroxyl group, a C₁₋₆ alkyl group, a C₂₋₆ alkenyl group, a C₂₋₆ alkynyl group, a C₆₋₁₀ aryl group, a C₃₋₈ cycloalkyl C₁₋₆ alkyl group, a C₃₋₈ cycloalkylydene C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₆₋₁₀ arylthio group, a C₆₋₁₀ aryl C₁₋₆ alkylthio group, a mono-C₆₋₁₀ arylamino group, a mono-C₆₋₁₀ aryl C₁₋₆ alkylamino group, a N-C₆₋₁₀ aryl-N-C₁₋₆ alkylamino group and a N-C₆₋₁₀ aryl C₁₋₆ alkyl-N-C₁₋₆ alkylamino group;
[Substituent Group e-2]
a C₁₋₆ alkyl group, a C₂₋₆ alkenyl group, a C₂₋₆ alkynyl group, a C₆₋₁₀ aryl group, a C₃₋₈ cycloalkyl C₁₋₆ alkyl group, a C₆₋₁₀ aryl C₁₋₆ alkyl group, a 5 to 10-membered heterocyclic C₁₋₆ alkyl group, a C₁₋₆ alkoxy group, a C₂₋₆ alkenyloxy group, a C₂₋₆ alkynyloxy group, a C₆₋₁₀ aryloxy group, a C₃₋₈ cycloalkyl C₁₋₆ alkoxy group, a C₆₋₁₀ aryl C₁₋₆ alkoxy group, a 5 to 10-membered heterocyclic C₁₋₆ alkoxy group, a C₆₋₁₀ arylthio group, a C₆₋₁₀ aryl C₁₋₆ alkylthio group, a mono-C₆₋₁₀ arylamino group, a mono-C₆₋₁₀ aryl C₁₋₆ alkylamino group, a N-C₆₋₁₀ aryl-N-C₁₋₆ alkylamino group and a N-C₆₋₁₀ aryl C₁₋₆ alkyl-N-C₁₋₆ alkylamino group;
with the proviso that each group mentioned in Substituent Group e-2 has one to three substituents selected from the following Substituent Group f;
[Substituent Group f]
a halogen atom, a hydroxyl group, a cyano group, an amino group, a nitro group, a C₃₋₈ cycloalkyl group, a C₁₋₆ alkoxy group, a C₆₋₁₀ aryloxy group, a 5 to 10-membered heterocyclic oxy group, a C₁₋₆ alkylcarbonyl group, a C₁₋₆ alkoxycarbonyl group, a C₁₋₆ alkylsulfonyl group, a mono-C₆₋₁₀ arylamino group, a trifluoromethyl group, a trifluoromethoxy group and a C₁₋₆ alkyl group.

14. The antimalarial agent according to any one of Claims 1 to 12, wherein E represents a furyl group, a thienyl group or a phenyl group (with the proviso that E has one substituent selected from the following Substituent Groups g-1 and g-2)
[Substituent Group g-1]
a C₃₋₈ cycloalkyl C₁₋₆ alkyl group, a phenyl C₁₋₆ alkyl group, a furyl C₁₋₆ alkyl group, a thienyl C₁₋₆ alkyl group, a benzofuryl C₁₋₆ alkyl group, a benzothienyl C₁₋₆ alkyl group, a phenoxy group, a C₃₋₈ cycloalkyl C₁₋₆ alkoxy group, a phenyl C₁₋₆ alkoxy group, a furyl C₁₋₆ alkoxy group, a thienyl C₁₋₆ alkoxy group and a pyridyl C₁₋₆ alkoxy group;
[Substituent Group g-2]
a C₃₋₈ cycloalkyl C₁₋₆ alkyl group, a phenyl C₁₋₆ alkyl group, a furyl C₁₋₆ alkyl group, a thienyl C₁₋₆ alkyl group, a benzofuryl C₁₋₆ alkyl group, a benzothienyl C₁₋₆ alkyl group, a C₁₋₆ alkoxy group, a phenoxy group, a C₃₋₈ cycloalkyl C₁₋₆ alkoxy group, a phenyl C₁₋₆ alkoxy group, a furyl C₁₋₆ alkoxy group, a thienyl C₁₋₆ alkoxy group and a pyridyl C₁₋₆ alkoxy group;
with the proviso that each group mentioned in Substituent Group g-2 has one to three substituents selected from the following Substituent Group h;
[Substituent Group h]
a halogen atom, a hydroxyl group, a cyano group and a C₁₋₆ alkyl group.

15. The antimalarial agent according to any one of Claims 1 to 12, wherein E represents a furyl group, a thienyl group or a phenyl group (with the proviso that E has one C₁₋₆ alkoxy C₁₋₆ alkyl group or one C₁₋₆ alkoxy group).

16. The antimalarial agent according to any one of Claims 1 to 12, wherein E represents a 2-furyl group, a 2-thienyl group or a phenyl group (with the proviso that E has one substituent selected from the above Substituent Group g-1 and g-2).

17. The antimalarial agent according to any one of Claims 1 to 12, wherein E represents a 2-furyl group, a 2-thienyl group or a phenyl group (with the proviso that E has one phenyl C₁₋₆ alkyl group that may have one halogen atom or one C₁₋₆ alkyl group, or one phenoxy group that may have one halogen atom or one C₁₋₆ alkyl group).

18. The antimalarial agent according to any one of Claims 1 to 12, wherein E represents a 2-furyl group, a 2-thienyl group or a phenyl group (with the proviso that E has one C₁₋₆ alkoxy C₁₋₆ alkyl group or one C₁₋₆ alkoxy group).

19. The antimalarial agent according to any one of Claims 1 to 12, wherein E represents a 2-furyl group, a 2-thienyl group or a phenyl group (with the proviso that E has one benzyl group that may have one fluorine atom, one chlorine atom or one methyl group, or one phenoxy group that may have one fluorine atom, one chlorine atom or one methyl group).

20. The antimalarial agent according to any one of Claims 1 to 12, wherein E represents a 2-furyl group, a 2-thienyl group or a phenyl group (with the proviso that E has one *n*-butoxy methyl group or one *n*-butoxy group).

21. The antimalarial agent according to any one of Claims 1 to 12, wherein E represents a 2-furyl group or a 2-thienyl group (with the proviso that E has one phenyl C₁₋₆ alkyl group that may have one halogen atom or one C₁₋₆ alkyl group, or one phenoxy group that may have one halogen atom or one C₁₋₆ alkyl group).

22. The antimalarial agent according to any one of Claims 1 to 12, wherein E represents a 2-furyl group or a 2-thienyl group (with the proviso that E has one benzyl group that may have one fluorine atom, one chlorine atom or one methyl group, or one phenoxy group that may have one fluorine atom, one chlorine atom or one methyl group).

23. The antimalarial agent according to any one of Claims 1 to 12, wherein E represents a phenyl group (with the proviso that E has one *n*-butoxymethyl group or one *n*-butoxy group).
